# EUROPEAN PATENT SPECIFICATION

(11) **EP 3 119 425 B1**
(45) Date of publication and mention of the grant of the patent: **23.09.2020**
(21) Application number: 15764851.0
(22) Date of filing: 13.03.2015
(51) Int. Cl.: A61K 39/00, A61K 38/00

(54) **REGULATABLE CHIMERIC ANTIGEN RECEPTOR**
REGULIERBARER CHIMÄRER ANTIGENREZEPTOR
RÉCEPTEUR D'ANTIGÈNE CHIMÈRIQUE RÉGULABLE

(30) Priority: 15.03.2014 US 201461953822 P
(43) Date of publication of application: 25.01.2017
(60) Divisional application: 20191324.1
(73) Proprietor: Novartis AG, 4056 Basel (CH); The Trustees of The University of Pennsylvania, Philadelphia, PA 19104 (US); Engels, Boris, Cambridge, MA 02139 (US)
(72) Inventor: ENGELS, Boris, Cambridge, MA 02139 (US); LOEW, Andreas, Cambridge, MA 02139 (US); MILONE, Michael C., Cherry Hill, NJ 08002 (US); ZHOU, Li, Cambridge, MA 02139 (US)
(74) Representative: Wilding, James Roger
(86) International application number: PCT/US2015/020533
(87) International publication number: WO 2015/142661

(56) References cited:
- WO-A1-2014/127261
- WO-A2-2014/145252
- US-A1- 2008 274 475
- US-A1- 2011 003 385
- US-A1- 2012 029 063

### SEQUENCE LISTING

<110> NOVARTIS AG
   THE TRUSTEES OF THE UNIVERSITY OF PENNSYLVANIA
<120> REGULATABLE CHIMERIC ANTIGEN RECEPTOR
<130> N2067-7087WO
<140> PCT/US2015/020533
   <141> 2015-03-13
<150> 61/953,822
   <151> 2014-03-15
<160> 242
<170> PatentIn version 3.5
<210> 1
   <211> 132
   <212> PRT
   <213> Artificial Sequence
<220>
   <223> Description of Artificial Sequence: Synthetic polypeptide
<400> 1
   Asp Val Pro Asp Tyr Ala Ser Leu Gly Gly Pro Ser Ser Pro Lys Lys
   1               5                   10                  15
   Lys Arg Lys Val Ser Arg Gly Val Gln Val Glu Thr Ile Ser Pro Gly
               20                  25                  30
   Asp Gly Arg Thr Phe Pro Lys Arg Gly Gln Thr Cys Val Val His Tyr
           35                  40                  45
   Thr Gly Met Leu Glu Asp Gly Lys Lys Phe Asp Ser Ser Arg Asp Arg
       50                  55                  60
   Asn Lys Pro Phe Lys Phe Met Leu Gly Lys Gln Glu Val Ile Arg Gly
   65                  70                  75                  80
   Trp Glu Glu Gly Val Ala Gln Met Ser Val Gly Gln Arg Ala Lys Leu
                   85                  90                  95
   Thr Ile Ser Pro Asp Tyr Ala Tyr Gly Ala Thr Gly His Pro Gly Ile
               100                 105                 110
   Ile Pro Pro His Ala Thr Leu Val Phe Asp Val Glu Leu Leu Lys Leu
           115                 120                 125
   Glu Thr Ser Tyr
       130
<210> 2
   <211> 93
   <212> PRT
   <213> Artificial Sequence
<220>
   <223> Description of Artificial Sequence: Synthetic polypeptide
<400> 2
<210> 3
   <211> 446
   <212> PRT
   <213> Artificial Sequence
<220>
   <223> Description of Artificial Sequence: Synthetic polypeptide
<400> 3
   Met Ala Leu Pro Val Thr Ala Leu Leu Leu Pro Leu Ala Leu Leu Leu
   1 5 10 15
   His Ala Ala Arg Pro Asp Ile Gln Met Thr Gln Ser Pro Ser Ser Leu
   20 25 30
   Ser Ala Ser Val Gly Asp Arg Val Thr Ile Thr Cys Arg Ala Ser Gln
   35 40 45
   Gly Ile Arg Asn Asn Leu Ala Trp Tyr Gln Gln Lys Pro Gly Lys Ala
   50 55 60
   Pro Lys Arg Leu Ile Tyr Ala Ala Ser Asn Leu Gln Ser Gly Val Pro
   65 70 75 80
   Ser Arg Phe Thr Gly Ser Gly Ser Gly Thr Glu Phe Thr Leu Ile Val
   85 90 95
   Ser Ser Leu Gln Pro Glu Asp Phe Ala Thr Tyr Tyr Cys Leu Gln His
   100 105 110
   His Ser Tyr Pro Leu Thr Ser Gly Gly Gly Thr Lys Val Glu Ile Lys
   115 120 125
   Arg Thr Gly Ser Thr Ser Gly Ser Gly Lys Pro Gly Ser Gly Glu Gly
   130 135 140
   Ser Glu Val Gln Val Leu Glu Ser Gly Gly Gly Leu Val Gln Pro Gly
   145 150 155 160
   Gly Ser Leu Arg Leu Ser Cys Ala Ala Ser Gly Phe Thr Phe Ser Ser
   165 170 175
   Tyr Ala Met Ser Trp Val Arg Gln Ala Pro Gly Lys Gly Leu Glu Trp
   180 185 190
   Val Ser Ala Ile Ser Gly Ser Gly Gly Ser Thr Asn Tyr Ala Asp Ser
   195 200 205
   Val Lys Gly Arg Phe Thr Ile Ser Arg Asp Asn Ser Lys Asn Thr Leu
   210 215 220
   Tyr Leu Gln Met Asn Ser Leu Arg Ala Glu Asp Thr Ala Val Tyr Tyr
   225 230 235 240
   Cys Ala Gly Ser Ser Gly Trp Ser Glu Tyr Trp Gly Gln Gly Thr Leu
   245 250 255
   Val Thr Val Ser Ser Thr Thr Thr Pro Ala Pro Arg Pro Pro Thr Pro
   260 265 270
   Ala Pro Thr Ile Ala Ser Gln Pro Leu Ser Leu Arg Pro Glu Ala Cys
   275 280 285
   Arg Pro Ala Ala Gly Gly Ala Val His Thr Arg Gly Leu Asp Phe Ala
   290 295 300
   Cys Asp Ile Tyr Ile Trp Ala Pro Leu Ala Gly Thr Cys Gly Val Leu
   305 310 315 320
   Leu Leu Ser Leu Val Ile Thr Leu Tyr Cys Lys Arg Gly Arg Lys Lys
   325 330 335
   Leu Leu Gly Gly Gly Gly Ser Gly Gly Gly Gly Ser Ala Ser Arg Ile
   340 345 350
   Leu Trp His Glu Met Trp His Glu Gly Leu Glu Glu Ala Ser Arg Leu
   355 360 365
   Tyr Phe Gly Glu Arg Asn Val Lys Gly Met Phe Glu Val Leu Glu Pro
   370 375 380
   Leu His Ala Met Met Glu Arg Gly Pro Gln Thr Leu Lys Glu Thr Ser
   385 390 395 400
   Phe Asn Gln Ala Tyr Gly Arg Asp Leu Met Glu Ala Gln Glu Trp Cys
   405 410 415
   Arg Lys Tyr Met Lys Ser Gly Asn Val Lys Asp Leu Leu Gln Ala Trp
   420 425 430
   Asp Leu Tyr Tyr His Val Phe Arg Arg Ile Ser Lys Thr Ser
   435 440 445
<210> 4
   <211> 274
   <212> PRT
   <213> Artificial Sequence
<220>
   <223> Description of Artificial Sequence: Synthetic polypeptide
<400> 4
   Met Gly Val Gln Val Glu Thr Ile Ser Pro Gly Asp Gly Arg Thr Phe
   1               5                   10                  15
   Pro Lys Arg Gly Gln Thr Cys Val Val His Tyr Thr Gly Met Leu Glu
               20                  25                  30
   Asp Gly Lys Lys Phe Asp Ser Ser Arg Asp Arg Asn Lys Pro Phe Lys
           35                  40                  45
   Phe Met Leu Gly Lys Gln Glu Val Ile Arg Gly Trp Glu Glu Gly Val
       50                  55                  60
   Ala Gln Met Ser Val Gly Gln Arg Ala Lys Leu Thr Ile Ser Pro Asp
   65                  70                  75                  80
   Tyr Ala Tyr Gly Ala Thr Gly His Pro Gly Ile Ile Pro Pro His Ala
   85 90 95
   Thr Leu Val Phe Asp Val Glu Leu Leu Lys Leu Glu Thr Ser Gly Gly
   100 105 110
   Gly Gly Ser Gly Gly Gly Gly Ser Lys Arg Gly Arg Lys Lys Leu Leu
   115 120 125
   Tyr Ile Phe Lys Gln Pro Phe Met Arg Pro Val Gln Thr Thr Gln Glu
   130 135 140
   Glu Asp Gly Cys Ser Cys Arg Phe Pro Glu Glu Glu Glu Gly Gly Cys
   145 150 155 160
   Glu Leu Arg Val Lys Phe Ser Arg Ser Ala Asp Ala Pro Ala Tyr Lys
   165 170 175
   Gln Gly Gln Asn Gln Leu Tyr Asn Glu Leu Asn Leu Gly Arg Arg Glu
   180 185 190
   Glu Tyr Asp Val Leu Asp Lys Arg Arg Gly Arg Asp Pro Glu Met Gly
   195 200 205
   Gly Lys Pro Arg Arg Lys Asn Pro Gln Glu Gly Leu Tyr Asn Glu Leu
   210 215 220
   Gln Lys Asp Lys Met Ala Glu Ala Tyr Ser Glu Ile Gly Met Lys Gly
   225 230 235 240
   Glu Arg Arg Arg Gly Lys Gly His Asp Gly Leu Tyr Gln Gly Leu Ser
   245 250 255
   Thr Ala Thr Lys Asp Thr Tyr Asp Ala Leu His Met Gln Ala Leu Pro
   260 265 270
   Pro Arg
<210> 5
   <211> 457
   <212> PRT
   <213> Artificial Sequence
<220>
   <223> Description of Artificial Sequence: Synthetic polypeptide
<400> 5
   Met Ala Leu Pro Val Thr Ala Leu Leu Leu Pro Leu Ala Leu Leu Leu
   1 5 10 15
   His Ala Ala Arg Pro Asp Ile Gln Met Thr Gln Ser Pro Ser Ser Leu
   20 25 30
   Ser Ala Ser Val Gly Asp Arg Val Thr Ile Thr Cys Arg Ala Ser Gln
   35 40 45
   Gly Ile Arg Asn Asn Leu Ala Trp Tyr Gln Gln Lys Pro Gly Lys Ala
   50 55 60
   Pro Lys Arg Leu Ile Tyr Ala Ala Ser Asn Leu Gln Ser Gly Val Pro
   65 70 75 80
   Ser Arg Phe Thr Gly Ser Gly Ser Gly Thr Glu Phe Thr Leu Ile Val
   85 90 95
   Ser Ser Leu Gln Pro Glu Asp Phe Ala Thr Tyr Tyr Cys Leu Gln His
   100 105 110
   His Ser Tyr Pro Leu Thr Ser Gly Gly Gly Thr Lys Val Glu Ile Lys
   115 120 125
   Arg Thr Gly Ser Thr Ser Gly Ser Gly Lys Pro Gly Ser Gly Glu Gly
   130 135 140
   Ser Glu Val Gln Val Leu Glu Ser Gly Gly Gly Leu Val Gln Pro Gly
   145 150 155 160
   Gly Ser Leu Arg Leu Ser Cys Ala Ala Ser Gly Phe Thr Phe Ser Ser
   165 170 175
   Tyr Ala Met Ser Trp Val Arg Gln Ala Pro Gly Lys Gly Leu Glu Trp
   180 185 190
   Val Ser Ala Ile Ser Gly Ser Gly Gly Ser Thr Asn Tyr Ala Asp Ser
   195 200 205
   Val Lys Gly Arg Phe Thr Ile Ser Arg Asp Asn Ser Lys Asn Thr Leu
   210 215 220
   Tyr Leu Gln Met Asn Ser Leu Arg Ala Glu Asp Thr Ala Val Tyr Tyr
   225 230 235 240
   Cys Ala Gly Ser Ser Gly Trp Ser Glu Tyr Trp Gly Gln Gly Thr Leu
   245 250 255
   Val Thr Val Ser Ser Thr Thr Thr Pro Ala Pro Arg Pro Pro Thr Pro
   260 265 270
   Ala Pro Thr Ile Ala Ser Gln Pro Leu Ser Leu Arg Pro Glu Ala Cys
   275 280 285
   Arg Pro Ala Ala Gly Gly Ala Val His Thr Arg Gly Leu Asp Phe Ala
   290 295 300
   Cys Asp Ile Tyr Ile Trp Ala Pro Leu Ala Gly Thr Cys Gly Val Leu
   305 310 315 320
   Leu Leu Ser Leu Val Ile Thr Leu Tyr Cys Lys Arg Gly Arg Lys Lys
   325 330 335
   Leu Leu Gly Gly Gly Gly Ser Gly Gly Gly Gly Ser Gly Val Gln Val
   340 345 350
   Glu Thr Ile Ser Pro Gly Asp Gly Arg Thr Phe Pro Lys Arg Gly Gln
   355 360 365
   Thr Cys Val Val His Tyr Thr Gly Met Leu Glu Asp Gly Lys Lys Phe
   370 375 380
   Asp Ser Ser Arg Asp Arg Asn Lys Pro Phe Lys Phe Met Leu Gly Lys
   385 390 395 400
   Gln Glu Val Ile Arg Gly Trp Glu Glu Gly Val Ala Gln Met Ser Val
   405 410 415
   Gly Gln Arg Ala Lys Leu Thr Ile Ser Pro Asp Tyr Ala Tyr Gly Ala
   420 425 430
   Thr Gly His Pro Gly Ile Ile Pro Pro His Ala Thr Leu Val Phe Asp
   435 440 445
   Val Glu Leu Leu Lys Leu Glu Thr Ser
   450 455
<210> 6
   <211> 263
   <212> PRT
   <213> Artificial Sequence
<220>
   <223> Description of Artificial Sequence: Synthetic polypeptide
<400> 6
   Met Ala Ser Arg Ile Leu Trp His Glu Met Trp His Glu Gly Leu Glu
   1               5                   10                  15
   Glu Ala Ser Arg Leu Tyr Phe Gly Glu Arg Asn Val Lys Gly Met Phe
   20 25 30
   Glu Val Leu Glu Pro Leu His Ala Met Met Glu Arg Gly Pro Gln Thr
   35 40 45
   Leu Lys Glu Thr Ser Phe Asn Gln Ala Tyr Gly Arg Asp Leu Met Glu
   50 55 60
   Ala Gln Glu Trp Cys Arg Lys Tyr Met Lys Ser Gly Asn Val Lys Asp
   65 70 75 80
   Leu Leu Gln Ala Trp Asp Leu Tyr Tyr His Val Phe Arg Arg Ile Ser
   85 90 95
   Lys Thr Ser Gly Gly Gly Gly Ser Gly Gly Gly Gly Ser Lys Arg Gly
   100 105 110
   Arg Lys Lys Leu Leu Tyr Ile Phe Lys Gln Pro Phe Met Arg Pro Val
   115 120 125
   Gln Thr Thr Gln Glu Glu Asp Gly Cys Ser Cys Arg Phe Pro Glu Glu
   130 135 140
   Glu Glu Gly Gly Cys Glu Leu Arg Val Lys Phe Ser Arg Ser Ala Asp
   145 150 155 160
   Ala Pro Ala Tyr Lys Gln Gly Gln Asn Gln Leu Tyr Asn Glu Leu Asn
   165 170 175
   Leu Gly Arg Arg Glu Glu Tyr Asp Val Leu Asp Lys Arg Arg Gly Arg
   180 185 190
   Asp Pro Glu Met Gly Gly Lys Pro Arg Arg Lys Asn Pro Gln Glu Gly
   195 200 205
   Leu Tyr Asn Glu Leu Gln Lys Asp Lys Met Ala Glu Ala Tyr Ser Glu
   210 215 220
   Ile Gly Met Lys Gly Glu Arg Arg Arg Gly Lys Gly His Asp Gly Leu
   225 230 235 240
   Tyr Gln Gly Leu Ser Thr Ala Thr Lys Asp Thr Tyr Asp Ala Leu His
   245 250 255
   Met Gln Ala Leu Pro Pro Arg
   260
<210> 7
   <211> 488
   <212> PRT
   <213> Artificial Sequence
<220>
   <223> Description of Artificial Sequence: Synthetic polypeptide
<400> 7
   Met Ala Leu Pro Val Thr Ala Leu Leu Leu Pro Leu Ala Leu Leu Leu
   1               5                   10                  15
   His Ala Ala Arg Pro Gly Ser Asp Ile Gln Met Thr Gln Ser Pro Ser
               20                  25                  30
   Ser Leu Ser Ala Ser Val Gly Asp Arg Val Thr Ile Thr Cys Arg Ala
           35                  40                  45
   Ser Gln Gly Ile Arg Asn Asn Leu Ala Trp Tyr Gln Gln Lys Pro Gly
       50                  55                  60
   Lys Ala Pro Lys Arg Leu Ile Tyr Ala Ala Ser Asn Leu Gln Ser Gly
   65                  70                  75                  80
   Val Pro Ser Arg Phe Thr Gly Ser Gly Ser Gly Thr Glu Phe Thr Leu
                   85                  90                  95
   Ile Val Ser Ser Leu Gln Pro Glu Asp Phe Ala Thr Tyr Tyr Cys Leu
               100                 105                 110
   Gln His His Ser Tyr Pro Leu Thr Ser Gly Gly Gly Thr Lys Val Glu
           115                 120                 125
   Ile Lys Arg Thr Gly Ser Thr Ser Gly Ser Gly Lys Pro Gly Ser Gly
       130                 135                 140
   Glu Gly Ser Glu Val Gln Val Leu Glu Ser Gly Gly Gly Leu Val Gln
   145                 150                 155                 160
   Pro Gly Gly Ser Leu Arg Leu Ser Cys Ala Ala Ser Gly Phe Thr Phe
                   165                 170                 175
   Ser Ser Tyr Ala Met Ser Trp Val Arg Gln Ala Pro Gly Lys Gly Leu
               180                 185                 190
   Glu Trp Val Ser Ala Ile Ser Gly Ser Gly Gly Ser Thr Asn Tyr Ala
           195                 200                 205
   Asp Ser Val Lys Gly Arg Phe Thr Ile Ser Arg Asp Asn Ser Lys Asn
   210 215 220
   Thr Leu Tyr Leu Gln Met Asn Ser Leu Arg Ala Glu Asp Thr Ala Val
   225 230 235 240
   Tyr Tyr Cys Ala Gly Ser Ser Gly Trp Ser Glu Tyr Trp Gly Gln Gly
   245 250 255
   Thr Leu Val Thr Val Ser Ser Ala Ser Thr Thr Thr Pro Ala Pro Arg
   260 265 270
   Pro Pro Thr Pro Ala Pro Thr Ile Ala Ser Gln Pro Leu Ser Leu Arg
   275 280 285
   Pro Glu Ala Cys Arg Pro Ala Ala Gly Gly Ala Val His Thr Arg Gly
   290 295 300
   Leu Asp Phe Ala Cys Asp Ile Tyr Ile Trp Ala Pro Leu Ala Gly Thr
   305 310 315 320
   Cys Gly Val Leu Leu Leu Ser Leu Val Ile Thr Leu Tyr Cys Lys Arg
   325 330 335
   Gly Arg Lys Lys Leu Leu Tyr Ile Phe Lys Gln Pro Phe Met Arg Pro
   340 345 350
   Val Gln Thr Thr Gln Glu Glu Asp Gly Cys Ser Cys Arg Phe Pro Glu
   355 360 365
   Glu Glu Glu Gly Gly Cys Glu Leu Arg Val Lys Phe Ser Arg Ser Ala
   370 375 380
   Asp Ala Pro Ala Tyr Lys Gln Gly Gln Asn Gln Leu Tyr Asn Glu Leu
   385 390 395 400
   Asn Leu Gly Arg Arg Glu Glu Tyr Asp Val Leu Asp Lys Arg Arg Gly
   405 410 415
   Arg Asp Pro Glu Met Gly Gly Lys Pro Arg Arg Lys Asn Pro Gln Glu
   420 425 430
   Gly Leu Tyr Asn Glu Leu Gln Lys Asp Lys Met Ala Glu Ala Tyr Ser
   435 440 445
   Glu Ile Gly Met Lys Gly Glu Arg Arg Arg Gly Lys Gly His Asp Gly
   450 455 460
   Leu Tyr Gln Gly Leu Ser Thr Ala Thr Lys Asp Thr Tyr Asp Ala Leu
   465 470 475 480
   His Met Gln Ala Leu Pro Pro Arg
   485
<210> 8
   <211> 567
   <212> PRT
   <213> Artificial Sequence
<220>
   <223> Description of Artificial Sequence: Synthetic polypeptide
<400> 8
   Met Ala Leu Pro Val Thr Ala Leu Leu Leu Pro Leu Ala Leu Leu Leu
   1 5 10 15
   His Ala Ala Arg Pro Asp Ile Gln Met Thr Gln Ser Pro Ser Ser Leu
   20 25 30
   Ser Ala Ser Val Gly Asp Arg Val Thr Ile Thr Cys Arg Ala Ser Gln
   35 40 45
   Gly Ile Arg Asn Asn Leu Ala Trp Tyr Gln Gln Lys Pro Gly Lys Ala
   50 55 60
   Pro Lys Arg Leu Ile Tyr Ala Ala Ser Asn Leu Gln Ser Gly Val Pro
   65 70 75 80
   Ser Arg Phe Thr Gly Ser Gly Ser Gly Thr Glu Phe Thr Leu Ile Val
   85 90 95
   Ser Ser Leu Gln Pro Glu Asp Phe Ala Thr Tyr Tyr Cys Leu Gln His
   100 105 110
   His Ser Tyr Pro Leu Thr Ser Gly Gly Gly Thr Lys Val Glu Ile Lys
   115 120 125
   Arg Thr Gly Ser Thr Ser Gly Ser Gly Lys Pro Gly Ser Gly Glu Gly
   130 135 140
   Ser Glu Val Gln Val Leu Glu Ser Gly Gly Gly Leu Val Gln Pro Gly
   145 150 155 160
   Gly Ser Leu Arg Leu Ser Cys Ala Ala Ser Gly Phe Thr Phe Ser Ser
   165 170 175
   Tyr Ala Met Ser Trp Val Arg Gln Ala Pro Gly Lys Gly Leu Glu Trp
   180 185 190
   Val Ser Ala Ile Ser Gly Ser Gly Gly Ser Thr Asn Tyr Ala Asp Ser
   195 200 205
   Val Lys Gly Arg Phe Thr Ile Ser Arg Asp Asn Ser Lys Asn Thr Leu
   210 215 220
   Tyr Leu Gln Met Asn Ser Leu Arg Ala Glu Asp Thr Ala Val Tyr Tyr
   225 230 235 240
   Cys Ala Gly Ser Ser Gly Trp Ser Glu Tyr Trp Gly Gln Gly Thr Leu
   245 250 255
   Val Thr Val Ser Ser Thr Thr Thr Pro Ala Pro Arg Pro Pro Thr Pro
   260 265 270
   Ala Pro Thr Ile Ala Ser Gln Pro Leu Ser Leu Arg Pro Glu Ala Cys
   275 280 285
   Arg Pro Ala Ala Gly Gly Ala Val His Thr Arg Gly Leu Asp Phe Ala
   290 295 300
   Cys Asp Ile Tyr Ile Trp Ala Pro Leu Ala Gly Thr Cys Gly Val Leu
   305 310 315 320
   Leu Leu Ser Leu Val Ile Thr Leu Tyr Cys Lys Arg Gly Arg Lys Lys
   325 330 335
   Leu Leu Gly Gly Gly Gly Ser Gly Gly Gly Gly Ser Ser Asn Ser Tyr
   340 345 350
   Asp Ser Ser Ser Ile Lys Val Leu Lys Gly Leu Asp Ala Val Arg Lys
   355 360 365
   Arg Pro Gly Met Tyr Ile Gly Asp Thr Asp Asp Gly Thr Gly Leu His
   370 375 380
   His Met Val Phe Glu Val Val Asp Asn Ala Ile Asp Glu Ala Leu Ala
   385 390 395 400
   Gly His Cys Lys Glu Ile Ile Val Thr Ile His Ala Asp Asn Ser Val
   405 410 415
   Ser Val Gln Asp Asp Gly Arg Gly Ile Pro Thr Gly Ile His Pro Glu
   420 425 430
   Glu Gly Val Ser Ala Ala Glu Val Ile Met Thr Val Leu His Ala Gly
   435 440 445
   Gly Lys Phe Asp Asp Asn Ser Tyr Lys Val Ser Gly Gly Leu His Gly
   450 455 460
   Val Gly Val Ser Val Val Asn Ala Leu Ser Gln Lys Leu Glu Leu Val
   465 470 475 480
   Ile Gln Arg Glu Gly Lys Ile His Arg Gln Ile Tyr Glu His Gly Val
   485 490 495
   Pro Gln Ala Pro Leu Ala Val Thr Gly Glu Thr Glu Lys Thr Gly Thr
   500 505 510
   Met Val Arg Phe Trp Pro Ser Leu Glu Thr Phe Thr Asn Val Thr Glu
   515 520 525
   Phe Glu Tyr Glu Ile Leu Ala Lys Arg Leu Arg Glu Leu Ser Phe Leu
   530 535 540
   Asn Ser Gly Val Ser Ile Arg Leu Arg Asp Lys Arg Asp Gly Lys Glu
   545 550 555 560
   Asp His Phe His Tyr Glu Gly
   565
<210> 9
   <211> 384
   <212> PRT
   <213> Artificial Sequence
<220>
   <223> Description of Artificial Sequence: Synthetic polypeptide
<400> 9
   Met Ser Asn Ser Tyr Asp Ser Ser Ser Ile Lys Val Leu Lys Gly Leu
   1 5 10 15
   Asp Ala Val Arg Lys Arg Pro Gly Met Tyr Ile Gly Asp Thr Asp Asp
   20 25 30
   Gly Thr Gly Leu His His Met Val Phe Glu Val Val Asp Asn Ala Ile
   35 40 45
   Asp Glu Ala Leu Ala Gly His Cys Lys Glu Ile Ile Val Thr Ile His
   50 55 60
   Ala Asp Asn Ser Val Ser Val Gln Asp Asp Gly Arg Gly Ile Pro Thr
   65 70 75 80
   Gly Ile His Pro Glu Glu Gly Val Ser Ala Ala Glu Val Ile Met Thr
   85 90 95
   Val Leu His Ala Gly Gly Lys Phe Asp Asp Asn Ser Tyr Lys Val Ser
   100 105 110
   Gly Gly Leu His Gly Val Gly Val Ser Val Val Asn Ala Leu Ser Gln
   115 120 125
   Lys Leu Glu Leu Val Ile Gln Arg Glu Gly Lys Ile His Arg Gln Ile
   130 135 140
   Tyr Glu His Gly Val Pro Gln Ala Pro Leu Ala Val Thr Gly Glu Thr
   145 150 155 160
   Glu Lys Thr Gly Thr Met Val Arg Phe Trp Pro Ser Leu Glu Thr Phe
   165 170 175
   Thr Asn Val Thr Glu Phe Glu Tyr Glu Ile Leu Ala Lys Arg Leu Arg
   180 185 190
   Glu Leu Ser Phe Leu Asn Ser Gly Val Ser Ile Arg Leu Arg Asp Lys
   195 200 205
   Arg Asp Gly Lys Glu Asp His Phe His Tyr Glu Gly Gly Gly Gly Gly
   210 215 220
   Ser Gly Gly Gly Gly Ser Lys Arg Gly Arg Lys Lys Leu Leu Tyr Ile
   225 230 235 240
   Phe Lys Gln Pro Phe Met Arg Pro Val Gln Thr Thr Gln Glu Glu Asp
   245 250 255
   Gly Cys Ser Cys Arg Phe Pro Glu Glu Glu Glu Gly Gly Cys Glu Leu
   260 265 270
   Arg Val Lys Phe Ser Arg Ser Ala Asp Ala Pro Ala Tyr Lys Gln Gly
   275 280 285
   Gln Asn Gln Leu Tyr Asn Glu Leu Asn Leu Gly Arg Arg Glu Glu Tyr
   290 295 300
   Asp Val Leu Asp Lys Arg Arg Gly Arg Asp Pro Glu Met Gly Gly Lys
   305 310 315 320
   Pro Arg Arg Lys Asn Pro Gln Glu Gly Leu Tyr Asn Glu Leu Gln Lys
   325 330 335
   Asp Lys Met Ala Glu Ala Tyr Ser Glu Ile Gly Met Lys Gly Glu Arg
   340 345 350
   Arg Arg Gly Lys Gly His Asp Gly Leu Tyr Gln Gly Leu Ser Thr Ala
   355 360 365
   Thr Lys Asp Thr Tyr Asp Ala Leu His Met Gln Ala Leu Pro Pro Arg
   370 375 380
<210> 10
   <211> 439
   <212> PRT
   <213> Artificial Sequence
<220>
   <223> Description of Artificial Sequence: Synthetic polypeptide
<400> 10
   Met Ala Leu Pro Val Thr Ala Leu Leu Leu Pro Leu Ala Leu Leu Leu
   1 5 10 15
   His Ala Ala Arg Pro Asp Ile Gln Met Thr Gln Ser Pro Ser Ser Leu
   20 25 30
   Ser Ala Ser Val Gly Asp Arg Val Thr Ile Thr Cys Arg Ala Ser Gln
   35 40 45
   Gly Ile Arg Asn Asn Leu Ala Trp Tyr Gln Gln Lys Pro Gly Lys Ala
   50 55 60
   Pro Lys Arg Leu Ile Tyr Ala Ala Ser Asn Leu Gln Ser Gly Val Pro
   65 70 75 80
   Ser Arg Phe Thr Gly Ser Gly Ser Gly Thr Glu Phe Thr Leu Ile Val
   85 90 95
   Ser Ser Leu Gln Pro Glu Asp Phe Ala Thr Tyr Tyr Cys Leu Gln His
   100 105 110
   His Ser Tyr Pro Leu Thr Ser Gly Gly Gly Thr Lys Val Glu Ile Lys
   115 120 125
   Arg Thr Gly Ser Thr Ser Gly Ser Gly Lys Pro Gly Ser Gly Glu Gly
   130 135 140
   Ser Glu Val Gln Val Leu Glu Ser Gly Gly Gly Leu Val Gln Pro Gly
   145 150 155 160
   Gly Ser Leu Arg Leu Ser Cys Ala Ala Ser Gly Phe Thr Phe Ser Ser
   165 170 175
   Tyr Ala Met Ser Trp Val Arg Gln Ala Pro Gly Lys Gly Leu Glu Trp
   180 185 190
   Val Ser Ala Ile Ser Gly Ser Gly Gly Ser Thr Asn Tyr Ala Asp Ser
   195 200 205
   Val Lys Gly Arg Phe Thr Ile Ser Arg Asp Asn Ser Lys Asn Thr Leu
   210 215 220
   Tyr Leu Gln Met Asn Ser Leu Arg Ala Glu Asp Thr Ala Val Tyr Tyr
   225 230 235 240
   Cys Ala Gly Ser Ser Gly Trp Ser Glu Tyr Trp Gly Gln Gly Thr Leu
   245 250 255
   Val Thr Val Ser Ser Thr Thr Thr Pro Ala Pro Arg Pro Pro Thr Pro
   260 265 270
   Ala Pro Thr Ile Ala Ser Gln Pro Leu Ser Leu Arg Pro Glu Ala Cys
   275 280 285
   Arg Pro Ala Ala Gly Gly Ala Val His Thr Arg Gly Leu Asp Phe Ala
   290 295 300
   Cys Asp Ile Tyr Ile Trp Ala Pro Leu Ala Gly Thr Cys Gly Val Leu
   305 310 315 320
   Leu Leu Ser Leu Val Ile Thr Leu Tyr Cys Lys Arg Gly Arg Lys Lys
   325 330 335
   Leu Leu Gly Gly Gly Gly Ser Gly Gly Gly Gly Ser Lys Arg Asp His
   340 345 350
   His His His His His Gln Asp Lys Lys Thr Met Met Met Asn Glu Glu
   355 360 365
   Asp Asp Gly Asn Gly Met Asp Glu Leu Leu Ala Val Leu Gly Tyr Lys
   370 375 380
   Val Arg Ser Ser Glu Met Ala Asp Val Ala Gln Lys Leu Glu Gln Leu
   385 390 395 400
   Glu Val Met Met Ser Asn Val Gln Glu Asp Asp Leu Ser Gln Leu Ala
   405 410 415
   Thr Glu Thr Val His Tyr Asn Pro Ala Glu Leu Tyr Thr Trp Leu Asp
   420 425 430
   Ser Met Leu Thr Asp Leu Asn
   435
<210> 11
   <211> 509
   <212> PRT
   <213> Artificial Sequence
<220>
   <223> Description of Artificial Sequence: Synthetic polypeptide
<400> 11
   Met Ala Ala Ser Asp Glu Val Asn Leu Ile Glu Ser Arg Thr Val Val
   1 5 10 15
   Pro Leu Asn Thr Trp Val Leu Ile Ser Asn Phe Lys Val Ala Tyr Asn
   20 25 30
   Ile Leu Arg Arg Pro Asp Gly Thr Phe Asn Arg His Leu Ala Glu Tyr
   35 40 45
   Leu Asp Arg Lys Val Thr Ala Asn Ala Asn Pro Val Asp Gly Val Phe
   50 55 60
   Ser Phe Asp Val Leu Ile Asp Arg Arg Ile Asn Leu Leu Ser Arg Val
   65 70 75 80
   Tyr Arg Pro Ala Tyr Ala Asp Gln Glu Gln Pro Pro Ser Ile Leu Asp
   85 90 95
   Leu Glu Lys Pro Val Asp Gly Asp Ile Val Pro Val Ile Leu Phe Phe
   100 105 110
   His Gly Gly Ser Phe Ala His Ser Ser Ala Asn Ser Ala Ile Tyr Asp
   115 120 125
   Thr Leu Cys Arg Arg Leu Val Gly Leu Cys Lys Cys Val Val Val Ser
   130 135 140
   Val Asn Tyr Arg Arg Ala Pro Glu Asn Pro Tyr Pro Cys Ala Tyr Asp
   145 150 155 160
   Asp Gly Trp Ile Ala Leu Asn Trp Val Asn Ser Arg Ser Trp Leu Lys
   165 170 175
   Ser Lys Lys Asp Ser Lys Val His Ile Phe Leu Ala Gly Asp Ser Ser
   180 185 190
   Gly Gly Asn Ile Ala His Asn Val Ala Leu Arg Ala Gly Glu Ser Gly
   195 200 205
   Ile Asp Val Leu Gly Asn Ile Leu Leu Asn Pro Met Phe Gly Gly Asn
   210 215 220
   Glu Arg Thr Glu Ser Glu Lys Ser Leu Asp Gly Lys Tyr Phe Val Thr
   225 230 235 240
   Val Arg Asp Arg Asp Trp Tyr Trp Lys Ala Phe Leu Pro Glu Gly Glu
   245 250 255
   Asp Arg Glu His Pro Ala Cys Asn Pro Phe Ser Pro Arg Gly Lys Ser
   260 265 270
   Leu Glu Gly Val Ser Phe Pro Lys Ser Leu Val Val Val Ala Gly Leu
   275 280 285
   Asp Leu Ile Arg Asp Trp Gln Leu Ala Tyr Ala Glu Gly Leu Lys Lys
   290 295 300
   Ala Gly Gln Glu Val Lys Leu Met His Leu Glu Lys Ala Thr Val Gly
   305 310 315 320
   Phe Tyr Leu Leu Pro Asn Asn Asn His Phe His Asn Val Met Asp Glu
   325 330 335
   Ile Ser Ala Phe Val Asn Ala Glu Cys Gly Gly Gly Gly Ser Gly Gly
   340 345 350
   Gly Gly Ser Lys Arg Gly Arg Lys Lys Leu Leu Tyr Ile Phe Lys Gln
   355 360 365
   Pro Phe Met Arg Pro Val Gln Thr Thr Gln Glu Glu Asp Gly Cys Ser
   370 375 380
   Cys Arg Phe Pro Glu Glu Glu Glu Gly Gly Cys Glu Leu Arg Val Lys
   385 390 395 400
   Phe Ser Arg Ser Ala Asp Ala Pro Ala Tyr Lys Gln Gly Gln Asn Gln
   405 410 415
   Leu Tyr Asn Glu Leu Asn Leu Gly Arg Arg Glu Glu Tyr Asp Val Leu
   420 425 430
   Asp Lys Arg Arg Gly Arg Asp Pro Glu Met Gly Gly Lys Pro Arg Arg
   435 440 445
   Lys Asn Pro Gln Glu Gly Leu Tyr Asn Glu Leu Gln Lys Asp Lys Met
   450 455 460
   Ala Glu Ala Tyr Ser Glu Ile Gly Met Lys Gly Glu Arg Arg Arg Gly
   465 470 475 480
   Lys Gly His Asp Gly Leu Tyr Gln Gly Leu Ser Thr Ala Thr Lys Asp
   485 490 495
   Thr Tyr Asp Ala Leu His Met Gln Ala Leu Pro Pro Arg
   500 505
<210> 12
   <211> 692
   <212> PRT
   <213> Artificial Sequence
<220>
   <223> Description of Artificial Sequence: Synthetic polypeptide
<400> 12
   Met Ala Leu Pro Val Thr Ala Leu Leu Leu Pro Leu Ala Leu Leu Leu
   1 5 10 15
   His Ala Ala Arg Pro Asp Ile Gln Met Thr Gln Ser Pro Ser Ser Leu
   20 25 30
   Ser Ala Ser Val Gly Asp Arg Val Thr Ile Thr Cys Arg Ala Ser Gln
   35 40 45
   Gly Ile Arg Asn Asn Leu Ala Trp Tyr Gln Gln Lys Pro Gly Lys Ala
   50 55 60
   Pro Lys Arg Leu Ile Tyr Ala Ala Ser Asn Leu Gln Ser Gly Val Pro
   65 70 75 80
   Ser Arg Phe Thr Gly Ser Gly Ser Gly Thr Glu Phe Thr Leu Ile Val
   85 90 95
   Ser Ser Leu Gln Pro Glu Asp Phe Ala Thr Tyr Tyr Cys Leu Gln His
   100 105 110
   His Ser Tyr Pro Leu Thr Ser Gly Gly Gly Thr Lys Val Glu Ile Lys
   115 120 125
   Arg Thr Gly Ser Thr Ser Gly Ser Gly Lys Pro Gly Ser Gly Glu Gly
   130 135 140
   Ser Glu Val Gln Val Leu Glu Ser Gly Gly Gly Leu Val Gln Pro Gly
   145 150 155 160
   Gly Ser Leu Arg Leu Ser Cys Ala Ala Ser Gly Phe Thr Phe Ser Ser
   165 170 175
   Tyr Ala Met Ser Trp Val Arg Gln Ala Pro Gly Lys Gly Leu Glu Trp
   180 185 190
   Val Ser Ala Ile Ser Gly Ser Gly Gly Ser Thr Asn Tyr Ala Asp Ser
   195 200 205
   Val Lys Gly Arg Phe Thr Ile Ser Arg Asp Asn Ser Lys Asn Thr Leu
   210 215 220
   Tyr Leu Gln Met Asn Ser Leu Arg Ala Glu Asp Thr Ala Val Tyr Tyr
   225 230 235 240
   Cys Ala Gly Ser Ser Gly Trp Ser Glu Tyr Trp Gly Gln Gly Thr Leu
   245 250 255
   Val Thr Val Ser Ser Thr Thr Thr Pro Ala Pro Arg Pro Pro Thr Pro
   260 265 270
   Ala Pro Thr Ile Ala Ser Gln Pro Leu Ser Leu Arg Pro Glu Ala Cys
   275 280 285
   Arg Pro Ala Ala Gly Gly Ala Val His Thr Arg Gly Leu Asp Phe Ala
   290 295 300
   Cys Asp Ile Tyr Ile Trp Ala Pro Leu Ala Gly Thr Cys Gly Val Leu
   305 310 315 320
   Leu Leu Ser Leu Val Ile Thr Leu Tyr Cys Lys Arg Gly Arg Lys Lys
   325 330 335
   Leu Leu Gly Gly Gly Gly Ser Gly Gly Gly Gly Ser Ala Ala Ser Asp
   340 345 350
   Glu Val Asn Leu Ile Glu Ser Arg Thr Val Val Pro Leu Asn Thr Trp
   355 360 365
   Val Leu Ile Ser Asn Phe Lys Val Ala Tyr Asn Ile Leu Arg Arg Pro
   370 375 380
   Asp Gly Thr Phe Asn Arg His Leu Ala Glu Tyr Leu Asp Arg Lys Val
   385 390 395 400
   Thr Ala Asn Ala Asn Pro Val Asp Gly Val Phe Ser Phe Asp Val Leu
   405 410 415
   Ile Asp Arg Arg Ile Asn Leu Leu Ser Arg Val Tyr Arg Pro Ala Tyr
   420 425 430
   Ala Asp Gln Glu Gln Pro Pro Ser Ile Leu Asp Leu Glu Lys Pro Val
   435 440 445
   Asp Gly Asp Ile Val Pro Val Ile Leu Phe Phe His Gly Gly Ser Phe
   450 455 460
   Ala His Ser Ser Ala Asn Ser Ala Ile Tyr Asp Thr Leu Cys Arg Arg
   465 470 475 480
   Leu Val Gly Leu Cys Lys Cys Val Val Val Ser Val Asn Tyr Arg Arg
   485 490 495
   Ala Pro Glu Asn Pro Tyr Pro Cys Ala Tyr Asp Asp Gly Trp Ile Ala
   500 505 510
   Leu Asn Trp Val Asn Ser Arg Ser Trp Leu Lys Ser Lys Lys Asp Ser
   515 520 525
   Lys Val His Ile Phe Leu Ala Gly Asp Ser Ser Gly Gly Asn Ile Ala
   530 535 540
   His Asn Val Ala Leu Arg Ala Gly Glu Ser Gly Ile Asp Val Leu Gly
   545 550 555 560
   Asn Ile Leu Leu Asn Pro Met Phe Gly Gly Asn Glu Arg Thr Glu Ser
   565 570 575
   Glu Lys Ser Leu Asp Gly Lys Tyr Phe Val Thr Val Arg Asp Arg Asp
   580 585 590
   Trp Tyr Trp Lys Ala Phe Leu Pro Glu Gly Glu Asp Arg Glu His Pro
   595 600 605
   Ala Cys Asn Pro Phe Ser Pro Arg Gly Lys Ser Leu Glu Gly Val Ser
   610 615 620
   Phe Pro Lys Ser Leu Val Val Val Ala Gly Leu Asp Leu Ile Arg Asp
   625 630 635 640
   Trp Gln Leu Ala Tyr Ala Glu Gly Leu Lys Lys Ala Gly Gln Glu Val
   645 650 655
   Lys Leu Met His Leu Glu Lys Ala Thr Val Gly Phe Tyr Leu Leu Pro
   660 665 670
   Asn Asn Asn His Phe His Asn Val Met Asp Glu Ile Ser Ala Phe Val
   675 680 685
   Asn Ala Glu Cys
   690
<210> 13
   <211> 256
   <212> PRT
   <213> Artificial Sequence
<220>
   <223> Description of Artificial Sequence: Synthetic polypeptide
<400> 13
   Met Lys Arg Asp His His His His His His Gln Asp Lys Lys Thr Met
   1               5                   10                  15
   Met Met Asn Glu Glu Asp Asp Gly Asn Gly Met Asp Glu Leu Leu Ala
               20                  25                  30
   Val Leu Gly Tyr Lys Val Arg Ser Ser Glu Met Ala Asp Val Ala Gln
           35                  40                  45
   Lys Leu Glu Gln Leu Glu Val Met Met Ser Asn Val Gln Glu Asp Asp
       50                  55                  60
   Leu Ser Gln Leu Ala Thr Glu Thr Val His Tyr Asn Pro Ala Glu Leu
   65                  70                  75                  80
   Tyr Thr Trp Leu Asp Ser Met Leu Thr Asp Leu Asn Gly Gly Gly Gly
                   85                  90                  95
   Ser Gly Gly Gly Gly Ser Lys Arg Gly Arg Lys Lys Leu Leu Tyr Ile
               100                 105                 110
   Phe Lys Gln Pro Phe Met Arg Pro Val Gln Thr Thr Gln Glu Glu Asp
           115                 120                 125
   Gly Cys Ser Cys Arg Phe Pro Glu Glu Glu Glu Gly Gly Cys Glu Leu
       130                 135                 140
   Arg Val Lys Phe Ser Arg Ser Ala Asp Ala Pro Ala Tyr Lys Gln Gly
   145                 150                 155                 160
   Gln Asn Gln Leu Tyr Asn Glu Leu Asn Leu Gly Arg Arg Glu Glu Tyr
                   165                 170                 175
   Asp Val Leu Asp Lys Arg Arg Gly Arg Asp Pro Glu Met Gly Gly Lys
               180                 185                 190
   Pro Arg Arg Lys Asn Pro Gln Glu Gly Leu Tyr Asn Glu Leu Gln Lys
           195                 200                 205
   Asp Lys Met Ala Glu Ala Tyr Ser Glu Ile Gly Met Lys Gly Glu Arg
       210                 215                 220
   Arg Arg Gly Lys Gly His Asp Gly Leu Tyr Gln Gly Leu Ser Thr Ala
   225                 230                 235                 240
   Thr Lys Asp Thr Tyr Asp Ala Leu His Met Gln Ala Leu Pro Pro Arg
                   245                 250                 255
<210> 14
   <211> 297
   <212> PRT
   <213> Artificial Sequence
<220>
   <223> Description of Artificial Sequence: Synthetic polypeptide
<400> 14
   Gly Ser Glu Ile Gly Thr Gly Phe Pro Phe Asp Pro His Tyr Val Glu
   1               5                   10                  15
   Val Leu Gly Glu Arg Met His Tyr Val Asp Val Gly Pro Arg Asp Gly
               20                  25                  30
   Thr Pro Val Leu Phe Leu His Gly Asn Pro Thr Ser Ser Tyr Val Trp
           35                  40                  45
   Arg Asn Ile Ile Pro His Val Ala Pro Thr His Arg Cys Ile Ala Pro
       50                  55                  60
   Asp Leu Ile Gly Met Gly Lys Ser Asp Lys Pro Asp Leu Gly Tyr Phe
   65                  70                  75                  80
   Phe Asp Asp His Val Arg Phe Met Asp Ala Phe Ile Glu Ala Leu Gly
   85 90 95
   Leu Glu Glu Val Val Leu Val Ile His Asp Trp Gly Ser Ala Leu Gly
   100 105 110
   Phe His Trp Ala Lys Arg Asn Pro Glu Arg Val Lys Gly Ile Ala Phe
   115 120 125
   Met Glu Phe Ile Arg Pro Ile Pro Thr Trp Asp Glu Trp Pro Glu Phe
   130 135 140
   Ala Arg Glu Thr Phe Gln Ala Phe Arg Thr Thr Asp Val Gly Arg Lys
   145 150 155 160
   Leu Ile Ile Asp Gln Asn Val Phe Ile Glu Gly Thr Leu Pro Met Gly
   165 170 175
   Val Val Arg Pro Leu Thr Glu Val Glu Met Asp His Tyr Arg Glu Pro
   180 185 190
   Phe Leu Asn Pro Val Asp Arg Glu Pro Leu Trp Arg Phe Pro Asn Glu
   195 200 205
   Leu Pro Ile Ala Gly Glu Pro Ala Asn Ile Val Ala Leu Val Glu Glu
   210 215 220
   Tyr Met Asp Trp Leu His Gln Ser Pro Val Pro Lys Leu Leu Phe Trp
   225 230 235 240
   Gly Thr Pro Gly Val Leu Ile Pro Pro Ala Glu Ala Ala Arg Leu Ala
   245 250 255
   Lys Ser Leu Pro Asn Cys Lys Ala Val Asp Ile Gly Pro Gly Leu Asn
   260 265 270
   Leu Leu Gln Glu Asp Asn Pro Asp Leu Ile Gly Ser Glu Ile Ala Arg
   275 280 285
   Trp Leu Ser Thr Leu Glu Ile Ser Gly
   290 295
<210> 15
   <211> 182
   <212> PRT
   <213> Artificial Sequence
<220>
   <223> Description of Artificial Sequence: Synthetic polypeptide
<400> 15
   Met Asp Lys Asp Cys Glu Met Lys Arg Thr Thr Leu Asp Ser Pro Leu
   1               5                   10                  15
   Gly Lys Leu Glu Leu Ser Gly Cys Glu Gln Gly Leu His Arg Ile Ile
               20                  25                  30
   Phe Leu Gly Lys Gly Thr Ser Ala Ala Asp Ala Val Glu Val Pro Ala
           35                  40                  45
   Pro Ala Ala Val Leu Gly Gly Pro Glu Pro Leu Met Gln Ala Thr Ala
       50                  55                  60
   Trp Leu Asn Ala Tyr Phe His Gln Pro Glu Ala Ile Glu Glu Phe Pro
   65                  70                  75                  80
   Val Pro Ala Leu His His Pro Val Phe Gln Gln Glu Ser Phe Thr Arg
                   85                  90                  95
   Gln Val Leu Trp Lys Leu Leu Lys Val Val Lys Phe Gly Glu Val Ile
               100                 105                 110
   Ser Tyr Ser His Leu Ala Ala Leu Ala Gly Asn Pro Ala Ala Thr Ala
           115                 120                 125
   Ala Val Lys Thr Ala Leu Ser Gly Asn Pro Val Pro Ile Leu Ile Pro
       130                 135                 140
   Cys His Arg Val Val Gln Gly Asp Leu Asp Val Gly Gly Tyr Glu Gly
   145                 150                 155                 160
   Gly Leu Ala Val Lys Glu Trp Leu Leu Ala His Glu Gly His Arg Leu
                   165                 170                 175
   Gly Lys Pro Gly Leu Gly
               180
<210> 16
   <211> 529
   <212> PRT
   <213> Artificial Sequence
<220>
   <223> Description of Artificial Sequence: Synthetic polypeptide
<400> 16
   Met Ala Leu Pro Val Thr Ala Leu Leu Leu Pro Leu Ala Leu Leu Leu
   1               5                   10                  15
   His Ala Ala Arg Pro Asp Ile Gln Met Thr Gln Ser Pro Ser Ser Leu
               20                  25                  30
   Ser Ala Ser Val Gly Asp Arg Val Thr Ile Thr Cys Arg Ala Ser Gln
           35                  40                  45
   Gly Ile Arg Asn Asn Leu Ala Trp Tyr Gln Gln Lys Pro Gly Lys Ala
       50                  55                  60
   Pro Lys Arg Leu Ile Tyr Ala Ala Ser Asn Leu Gln Ser Gly Val Pro
   65                  70                  75                  80
   Ser Arg Phe Thr Gly Ser Gly Ser Gly Thr Glu Phe Thr Leu Ile Val
                   85                  90                  95
   Ser Ser Leu Gln Pro Glu Asp Phe Ala Thr Tyr Tyr Cys Leu Gln His
               100                 105                 110
   His Ser Tyr Pro Leu Thr Ser Gly Gly Gly Thr Lys Val Glu Ile Lys
           115                 120                 125
   Arg Thr Gly Ser Thr Ser Gly Ser Gly Lys Pro Gly Ser Gly Glu Gly
       130                 135                 140
   Ser Glu Val Gln Val Leu Glu Ser Gly Gly Gly Leu Val Gln Pro Gly
   145                 150                 155                 160
   Gly Ser Leu Arg Leu Ser Cys Ala Ala Ser Gly Phe Thr Phe Ser Ser
                   165                 170                 175
   Tyr Ala Met Ser Trp Val Arg Gln Ala Pro Gly Lys Gly Leu Glu Trp
               180                 185                 190
   Val Ser Ala Ile Ser Gly Ser Gly Gly Ser Thr Asn Tyr Ala Asp Ser
           195                 200                 205
   Val Lys Gly Arg Phe Thr Ile Ser Arg Asp Asn Ser Lys Asn Thr Leu
       210                 215                 220
   Tyr Leu Gln Met Asn Ser Leu Arg Ala Glu Asp Thr Ala Val Tyr Tyr
   225                 230                 235                 240
   Cys Ala Gly Ser Ser Gly Trp Ser Glu Tyr Trp Gly Gln Gly Thr Leu
                   245                 250                 255
   Val Thr Val Ser Ser Thr Thr Thr Pro Ala Pro Arg Pro Pro Thr Pro
   260 265 270
   Ala Pro Thr Ile Ala Ser Gln Pro Leu Ser Leu Arg Pro Glu Ala Cys
   275 280 285
   Arg Pro Ala Ala Gly Gly Ala Val His Thr Arg Gly Leu Asp Phe Ala
   290 295 300
   Cys Asp Ile Tyr Ile Trp Ala Pro Leu Ala Gly Thr Cys Gly Val Leu
   305 310 315 320
   Leu Leu Ser Leu Val Ile Thr Leu Tyr Cys Lys Arg Gly Arg Lys Lys
   325 330 335
   Leu Leu Gly Gly Gly Gly Ser Gly Gly Gly Gly Ser Asp Lys Asp Cys
   340 345 350
   Glu Met Lys Arg Thr Thr Leu Asp Ser Pro Leu Gly Lys Leu Glu Leu
   355 360 365
   Ser Gly Cys Glu Gln Gly Leu His Arg Ile Ile Phe Leu Gly Lys Gly
   370 375 380
   Thr Ser Ala Ala Asp Ala Val Glu Val Pro Ala Pro Ala Ala Val Leu
   385 390 395 400
   Gly Gly Pro Glu Pro Leu Met Gln Ala Thr Ala Trp Leu Asn Ala Tyr
   405 410 415
   Phe His Gln Pro Glu Ala Ile Glu Glu Phe Pro Val Pro Ala Leu His
   420 425 430
   His Pro Val Phe Gln Gln Glu Ser Phe Thr Arg Gln Val Leu Trp Lys
   435 440 445
   Leu Leu Lys Val Val Lys Phe Gly Glu Val Ile Ser Tyr Ser His Leu
   450 455 460
   Ala Ala Leu Ala Gly Asn Pro Ala Ala Thr Ala Ala Val Lys Thr Ala
   465 470 475 480
   Leu Ser Gly Asn Pro Val Pro Ile Leu Ile Pro Cys His Arg Val Val
   485 490 495
   Gln Gly Asp Leu Asp Val Gly Gly Tyr Glu Gly Gly Leu Ala Val Lys
   500 505 510
   Glu Trp Leu Leu Ala His Glu Gly His Arg Leu Gly Lys Pro Gly Leu
           515                 520                 525
   Gly
<210> 17
   <211> 462
   <212> PRT
   <213> Artificial Sequence
<220>
   <223> Description of Artificial Sequence: Synthetic polypeptide
<400> 17
   Met Gly Ser Glu Ile Gly Thr Gly Phe Pro Phe Asp Pro His Tyr Val
   1               5                   10                  15
   Glu Val Leu Gly Glu Arg Met His Tyr Val Asp Val Gly Pro Arg Asp
               20                  25                  30
   Gly Thr Pro Val Leu Phe Leu His Gly Asn Pro Thr Ser Ser Tyr Val
           35                  40                  45
   Trp Arg Asn Ile Ile Pro His Val Ala Pro Thr His Arg Cys Ile Ala
       50                  55                  60
   Pro Asp Leu Ile Gly Met Gly Lys Ser Asp Lys Pro Asp Leu Gly Tyr
   65                  70                  75                  80
   Phe Phe Asp Asp His Val Arg Phe Met Asp Ala Phe Ile Glu Ala Leu
                   85                  90                  95
   Gly Leu Glu Glu Val Val Leu Val Ile His Asp Trp Gly Ser Ala Leu
               100                 105                 110
   Gly Phe His Trp Ala Lys Arg Asn Pro Glu Arg Val Lys Gly Ile Ala
           115                 120                 125
   Phe Met Glu Phe Ile Arg Pro Ile Pro Thr Trp Asp Glu Trp Pro Glu
       130                 135                 140
   Phe Ala Arg Glu Thr Phe Gln Ala Phe Arg Thr Thr Asp Val Gly Arg
   145                 150                 155                 160
   Lys Leu Ile Ile Asp Gln Asn Val Phe Ile Glu Gly Thr Leu Pro Met
                   165                 170                 175
   Gly Val Val Arg Pro Leu Thr Glu Val Glu Met Asp His Tyr Arg Glu
               180                 185                 190
   Pro Phe Leu Asn Pro Val Asp Arg Glu Pro Leu Trp Arg Phe Pro Asn
           195                 200                 205
   Glu Leu Pro Ile Ala Gly Glu Pro Ala Asn Ile Val Ala Leu Val Glu
       210                 215                 220
   Glu Tyr Met Asp Trp Leu His Gln Ser Pro Val Pro Lys Leu Leu Phe
   225                 230                 235                     240
   Trp Gly Thr Pro Gly Val Leu Ile Pro Pro Ala Glu Ala Ala Arg Leu
                   245                 250                 255
   Ala Lys Ser Leu Pro Asn Cys Lys Ala Val Asp Ile Gly Pro Gly Leu
               260                 265                 270
   Asn Leu Leu Gln Glu Asp Asn Pro Asp Leu Ile Gly Ser Glu Ile Ala
           275                 280                 285
   Arg Trp Leu Ser Thr Leu Glu Ile Ser Gly Gly Gly Gly Gly Ser Gly
       290                 295                 300
   Gly Gly Gly Ser Lys Arg Gly Arg Lys Lys Leu Leu Tyr Ile Phe Lys
   305                 310                 315                     320
   Gln Pro Phe Met Arg Pro Val Gln Thr Thr Gln Glu Glu Asp Gly Cys
                   325                 330                 335
   Ser Cys Arg Phe Pro Glu Glu Glu Glu Gly Gly Cys Glu Leu Arg Val
               340                 345                 350
   Lys Phe Ser Arg Ser Ala Asp Ala Pro Ala Tyr Lys Gln Gly Gln Asn
           355                 360                 365
   Gln Leu Tyr Asn Glu Leu Asn Leu Gly Arg Arg Glu Glu Tyr Asp Val
       370                 375                 380
   Leu Asp Lys Arg Arg Gly Arg Asp Pro Glu Met Gly Gly Lys Pro Arg
   385                 390                 395                     400
   Arg Lys Asn Pro Gln Glu Gly Leu Tyr Asn Glu Leu Gln Lys Asp Lys
                   405                 410                 415
   Met Ala Glu Ala Tyr Ser Glu Ile Gly Met Lys Gly Glu Arg Arg Arg
               420                 425                 430
   Gly Lys Gly His Asp Gly Leu Tyr Gln Gly Leu Ser Thr Ala Thr Lys
   435 440 445
   Asp Thr Tyr Asp Ala Leu His Met Gln Ala Leu Pro Pro Arg
   450 455 460
<210> 18
   <211> 645
   <212> PRT
   <213> Artificial Sequence
<220>
   <223> Description of Artificial Sequence: Synthetic polypeptide
<400> 18
   Met Ala Leu Pro Val Thr Ala Leu Leu Leu Pro Leu Ala Leu Leu Leu
   1 5 10 15
   His Ala Ala Arg Pro Asp Ile Gln Met Thr Gln Ser Pro Ser Ser Leu
   20 25 30
   Ser Ala Ser Val Gly Asp Arg Val Thr Ile Thr Cys Arg Ala Ser Gln
   35 40 45
   Gly Ile Arg Asn Asn Leu Ala Trp Tyr Gln Gln Lys Pro Gly Lys Ala
   50 55 60
   Pro Lys Arg Leu Ile Tyr Ala Ala Ser Asn Leu Gln Ser Gly Val Pro
   65 70 75 80
   Ser Arg Phe Thr Gly Ser Gly Ser Gly Thr Glu Phe Thr Leu Ile Val
   85 90 95
   Ser Ser Leu Gln Pro Glu Asp Phe Ala Thr Tyr Tyr Cys Leu Gln His
   100 105 110
   His Ser Tyr Pro Leu Thr Ser Gly Gly Gly Thr Lys Val Glu Ile Lys
   115 120 125
   Arg Thr Gly Ser Thr Ser Gly Ser Gly Lys Pro Gly Ser Gly Glu Gly
   130 135 140
   Ser Glu Val Gln Val Leu Glu Ser Gly Gly Gly Leu Val Gln Pro Gly
   145 150 155 160
   Gly Ser Leu Arg Leu Ser Cys Ala Ala Ser Gly Phe Thr Phe Ser Ser
   165 170 175
   Tyr Ala Met Ser Trp Val Arg Gln Ala Pro Gly Lys Gly Leu Glu Trp
   180 185 190
   Val Ser Ala Ile Ser Gly Ser Gly Gly Ser Thr Asn Tyr Ala Asp Ser
   195 200 205
   Val Lys Gly Arg Phe Thr Ile Ser Arg Asp Asn Ser Lys Asn Thr Leu
   210 215 220
   Tyr Leu Gln Met Asn Ser Leu Arg Ala Glu Asp Thr Ala Val Tyr Tyr
   225 230 235 240
   Cys Ala Gly Ser Ser Gly Trp Ser Glu Tyr Trp Gly Gln Gly Thr Leu
   245 250 255
   Val Thr Val Ser Ser Thr Thr Thr Pro Ala Pro Arg Pro Pro Thr Pro
   260 265 270
   Ala Pro Thr Ile Ala Ser Gln Pro Leu Ser Leu Arg Pro Glu Ala Cys
   275 280 285
   Arg Pro Ala Ala Gly Gly Ala Val His Thr Arg Gly Leu Asp Phe Ala
   290 295 300
   Cys Asp Ile Tyr Ile Trp Ala Pro Leu Ala Gly Thr Cys Gly Val Leu
   305 310 315 320
   Leu Leu Ser Leu Val Ile Thr Leu Tyr Cys Lys Arg Gly Arg Lys Lys
   325 330 335
   Leu Leu Gly Gly Gly Gly Ser Gly Gly Gly Gly Ser Gly Ser Glu Ile
   340 345 350
   Gly Thr Gly Phe Pro Phe Asp Pro His Tyr Val Glu Val Leu Gly Glu
   355 360 365
   Arg Met His Tyr Val Asp Val Gly Pro Arg Asp Gly Thr Pro Val Leu
   370 375 380
   Phe Leu His Gly Asn Pro Thr Ser Ser Tyr Val Trp Arg Asn Ile Ile
   385 390 395 400
   Pro His Val Ala Pro Thr His Arg Cys Ile Ala Pro Asp Leu Ile Gly
   405 410 415
   Met Gly Lys Ser Asp Lys Pro Asp Leu Gly Tyr Phe Phe Asp Asp His
   420 425 430
   Val Arg Phe Met Asp Ala Phe Ile Glu Ala Leu Gly Leu Glu Glu Val
   435 440 445
   Val Leu Val Ile His Asp Trp Gly Ser Ala Leu Gly Phe His Trp Ala
   450 455 460
   Lys Arg Asn Pro Glu Arg Val Lys Gly Ile Ala Phe Met Glu Phe Ile
   465 470 475 480
   Arg Pro Ile Pro Thr Trp Asp Glu Trp Pro Glu Phe Ala Arg Glu Thr
   485 490 495
   Phe Gln Ala Phe Arg Thr Thr Asp Val Gly Arg Lys Leu Ile Ile Asp
   500 505 510
   Gln Asn Val Phe Ile Glu Gly Thr Leu Pro Met Gly Val Val Arg Pro
   515 520 525
   Leu Thr Glu Val Glu Met Asp His Tyr Arg Glu Pro Phe Leu Asn Pro
   530 535 540
   Val Asp Arg Glu Pro Leu Trp Arg Phe Pro Asn Glu Leu Pro Ile Ala
   545 550 555 560
   Gly Glu Pro Ala Asn Ile Val Ala Leu Val Glu Glu Tyr Met Asp Trp
   565 570 575
   Leu His Gln Ser Pro Val Pro Lys Leu Leu Phe Trp Gly Thr Pro Gly
   580 585 590
   Val Leu Ile Pro Pro Ala Glu Ala Ala Arg Leu Ala Lys Ser Leu Pro
   595 600 605
   Asn Cys Lys Ala Val Asp Ile Gly Pro Gly Leu Asn Leu Leu Gln Glu
   610 615 620
   Asp Asn Pro Asp Leu Ile Gly Ser Glu Ile Ala Arg Trp Leu Ser Thr
   625 630 635 640
   Leu Glu Ile Ser Gly
   645
<210> 19
   <211> 346
   <212> PRT
   <213> Artificial Sequence
<220>
   <223> Description of Artificial Sequence: Synthetic polypeptide
<400> 19
   Met Asp Lys Asp Cys Glu Met Lys Arg Thr Thr Leu Asp Ser Pro Leu
   1               5                   10                  15
   Gly Lys Leu Glu Leu Ser Gly Cys Glu Gln Gly Leu His Arg Ile Ile
               20                  25                  30
   Phe Leu Gly Lys Gly Thr Ser Ala Ala Asp Ala Val Glu Val Pro Ala
           35                  40                  45
   Pro Ala Ala Val Leu Gly Gly Pro Glu Pro Leu Met Gln Ala Thr Ala
       50                  55                  60
   Trp Leu Asn Ala Tyr Phe His Gln Pro Glu Ala Ile Glu Glu Phe Pro
   65                  70                  75                  80
   Val Pro Ala Leu His His Pro Val Phe Gln Gln Glu Ser Phe Thr Arg
                   85                  90                  95
   Gln Val Leu Trp Lys Leu Leu Lys Val Val Lys Phe Gly Glu Val Ile
               100                 105                 110
   Ser Tyr Ser His Leu Ala Ala Leu Ala Gly Asn Pro Ala Ala Thr Ala
           115                 120                 125
   Ala Val Lys Thr Ala Leu Ser Gly Asn Pro Val Pro Ile Leu Ile Pro
       130                 135                 140
   Cys His Arg Val Val Gln Gly Asp Leu Asp Val Gly Gly Tyr Glu Gly
   145                 150                 155                 160
   Gly Leu Ala Val Lys Glu Trp Leu Leu Ala His Glu Gly His Arg Leu
                   165                 170                 175
   Gly Lys Pro Gly Leu Gly Gly Gly Gly Gly Ser Gly Gly Gly Gly Ser
               180                 185                 190
   Lys Arg Gly Arg Lys Lys Leu Leu Tyr Ile Phe Lys Gln Pro Phe Met
           195                 200                 205
   Arg Pro Val Gln Thr Thr Gln Glu Glu Asp Gly Cys Ser Cys Arg Phe
       210                 215                 220
   Pro Glu Glu Glu Glu Gly Gly Cys Glu Leu Arg Val Lys Phe Ser Arg
   225                 230                 235                 240
   Ser Ala Asp Ala Pro Ala Tyr Lys Gln Gly Gln Asn Gln Leu Tyr Asn
   245 250 255
   Glu Leu Asn Leu Gly Arg Arg Glu Glu Tyr Asp Val Leu Asp Lys Arg
   260 265 270
   Arg Gly Arg Asp Pro Glu Met Gly Gly Lys Pro Arg Arg Lys Asn Pro
   275 280 285
   Gln Glu Gly Leu Tyr Asn Glu Leu Gln Lys Asp Lys Met Ala Glu Ala
   290 295 300
   Tyr Ser Glu Ile Gly Met Lys Gly Glu Arg Arg Arg Gly Lys Gly His
   305 310 315 320
   Asp Gly Leu Tyr Gln Gly Leu Ser Thr Ala Thr Lys Asp Thr Tyr Asp
   325 330 335
   Ala Leu His Met Gln Ala Leu Pro Pro Arg
   340 345
<210> 20
   <211> 264
   <212> PRT
   <213> Artificial Sequence
<220>
   <223> Description of Artificial Sequence: Synthetic polypeptide
<400> 20
   Met Ala Leu Pro Val Thr Ala Leu Leu Leu Pro Leu Ala Leu Leu Leu
   1 5 10 15
   His Ala Ala Arg Pro Gly Ser Asp Tyr Lys Asp Asp Asp Asp Lys Gly
   20 25 30
   Gly Gly Gly Ser Gly Gly Gly Gly Ser Thr Thr Thr Pro Ala Pro Arg
   35 40 45
   Pro Pro Thr Pro Ala Pro Thr Ile Ala Ser Gln Pro Leu Ser Leu Arg
   50 55 60
   Pro Glu Ala Cys Arg Pro Ala Ala Gly Gly Ala Val His Thr Arg Gly
   65 70 75 80
   Leu Asp Phe Ala Cys Asp Ile Tyr Ile Trp Ala Pro Leu Ala Gly Thr
   85 90 95
   Cys Gly Val Leu Leu Leu Ser Leu Val Ile Thr Leu Tyr Cys Lys Arg
   100 105 110
   Gly Arg Lys Lys Leu Leu Tyr Ile Phe Lys Gln Pro Phe Met Arg Pro
   115 120 125
   Val Gln Thr Thr Gln Glu Glu Asp Gly Cys Ser Cys Arg Phe Pro Glu
   130 135 140
   Glu Glu Glu Gly Gly Cys Glu Leu Arg Val Lys Phe Ser Arg Ser Ala
   145 150 155 160
   Asp Ala Pro Ala Tyr Lys Gln Gly Gln Asn Gln Leu Tyr Asn Glu Leu
   165 170 175
   Asn Leu Gly Arg Arg Glu Glu Tyr Asp Val Leu Asp Lys Arg Arg Gly
   180 185 190
   Arg Asp Pro Glu Met Gly Gly Lys Pro Arg Arg Lys Asn Pro Gln Glu
   195 200 205
   Gly Leu Tyr Asn Glu Leu Gln Lys Asp Lys Met Ala Glu Ala Tyr Ser
   210 215 220
   Glu Ile Gly Met Lys Gly Glu Arg Arg Arg Gly Lys Gly His Asp Gly
   225 230 235 240
   Leu Tyr Gln Gly Leu Ser Thr Ala Thr Lys Asp Thr Tyr Asp Ala Leu
   245 250 255
   His Met Gln Ala Leu Pro Pro Arg
   260
<210> 21
   <211> 266
   <212> PRT
   <213> Artificial Sequence
<220>
   <223> Description of Artificial Sequence: Synthetic polypeptide
<400> 21
   Met Ala Leu Pro Val Thr Ala Leu Leu Leu Pro Leu Ala Leu Leu Leu
   1 5 10 15
   His Ala Ala Arg Pro Gly Ser Glu Gln Lys Leu Ile Ser Glu Glu Asp
   20 25 30
   Leu Gly Gly Gly Gly Ser Gly Gly Gly Gly Ser Thr Thr Thr Pro Ala
   35 40 45
   Pro Arg Pro Pro Thr Pro Ala Pro Thr Ile Ala Ser Gln Pro Leu Ser
   50 55 60
   Leu Arg Pro Glu Ala Cys Arg Pro Ala Ala Gly Gly Ala Val His Thr
   65 70 75 80
   Arg Gly Leu Asp Phe Ala Cys Asp Ile Tyr Ile Trp Ala Pro Leu Ala
   85 90 95
   Gly Thr Cys Gly Val Leu Leu Leu Ser Leu Val Ile Thr Leu Tyr Cys
   100 105 110
   Lys Arg Gly Arg Lys Lys Leu Leu Tyr Ile Phe Lys Gln Pro Phe Met
   115 120 125
   Arg Pro Val Gln Thr Thr Gln Glu Glu Asp Gly Cys Ser Cys Arg Phe
   130 135 140
   Pro Glu Glu Glu Glu Gly Gly Cys Glu Leu Arg Val Lys Phe Ser Arg
   145 150 155 160
   Ser Ala Asp Ala Pro Ala Tyr Lys Gln Gly Gln Asn Gln Leu Tyr Asn
   165 170 175
   Glu Leu Asn Leu Gly Arg Arg Glu Glu Tyr Asp Val Leu Asp Lys Arg
   180 185 190
   Arg Gly Arg Asp Pro Glu Met Gly Gly Lys Pro Arg Arg Lys Asn Pro
   195 200 205
   Gln Glu Gly Leu Tyr Asn Glu Leu Gln Lys Asp Lys Met Ala Glu Ala
   210 215 220
   Tyr Ser Glu Ile Gly Met Lys Gly Glu Arg Arg Arg Gly Lys Gly His
   225 230 235 240
   Asp Gly Leu Tyr Gln Gly Leu Ser Thr Ala Thr Lys Asp Thr Tyr Asp
   245 250 255
   Ala Leu His Met Gln Ala Leu Pro Pro Arg
   260 265
<210> 22
   <211> 11
   <212> PRT
   <213> Artificial Sequence
<220>
   <223> Description of Artificial Sequence: Synthetic peptide
<400> 22
   Glu Glu Gln Lys Leu Ile Ser Glu Glu Asp Leu
   1 5 10
<210> 23
   <211> 37
   <212> PRT
   <213> Artificial Sequence
<220>
   <223> Description of Artificial Sequence: Synthetic polypeptide
<400> 23
   Glu Glu Gln Lys Leu Ile Ser Glu Glu Asp Leu Gly Gly Gly Gly Ser
   1 5 10 15
   Gly Gly Gly Gly Ser Gly Gly Gly Gly Ser Glu Glu Gln Lys Leu Ile
   20 25 30
   Ser Glu Glu Asp Leu
   35
<210> 24
   <211> 63
   <212> PRT
   <213> Artificial Sequence
<220>
   <223> Description of Artificial Sequence: Synthetic polypeptide
<400> 24
   Glu Glu Gln Lys Leu Ile Ser Glu Glu Asp Leu Gly Gly Gly Gly Ser
   1 5 10 15
   Gly Gly Gly Gly Ser Gly Gly Gly Gly Ser Glu Glu Gln Lys Leu Ile
   20 25 30
   Ser Glu Glu Asp Leu Gly Gly Gly Gly Ser Gly Gly Gly Gly Ser Gly
   35 40 45
   Gly Gly Gly Ser Glu Glu Gln Lys Leu Ile Ser Glu Glu Asp Leu
   50 55 60
<210> 25
   <211> 89
   <212> PRT
   <213> Artificial Sequence
<220>
   <223> Description of Artificial Sequence: Synthetic polypeptide
<400> 25
   Glu Glu Gln Lys Leu Ile Ser Glu Glu Asp Leu Gly Gly Gly Gly Ser
   1               5                   10                  15
   Gly Gly Gly Gly Ser Gly Gly Gly Gly Ser Glu Glu Gln Lys Leu Ile
               20                  25                  30
   Ser Glu Glu Asp Leu Gly Gly Gly Gly Ser Gly Gly Gly Gly Ser Gly
           35                  40                  45
   Gly Gly Gly Ser Glu Glu Gln Lys Leu Ile Ser Glu Glu Asp Leu Gly
       50                  55                  60
   Gly Gly Gly Ser Gly Gly Gly Gly Ser Gly Gly Gly Gly Ser Glu Glu
   65                  70                  75                  80
   Gln Lys Leu Ile Ser Glu Glu Asp Leu
                   85
<210> 26
   <211> 338
   <212> PRT
   <213> Artificial Sequence
<220>
   <223> Description of Artificial Sequence: Synthetic polypeptide
<400> 26
   Met Ala Leu Pro Val Thr Ala Leu Leu Leu Pro Leu Ala Leu Leu Leu
   1               5                   10                  15
   His Ala Ala Arg Pro Asp Ile Gln Met Thr Gln Ser Pro Ser Ser Leu
               20                  25                  30
   Ser Ala Ser Val Gly Asp Arg Val Thr Ile Thr Cys Arg Ala Ser Gln
           35                  40                  45
   Gly Ile Arg Asn Asn Leu Ala Trp Tyr Gln Gln Lys Pro Gly Lys Ala
       50                  55                  60
   Pro Lys Arg Leu Ile Tyr Ala Ala Ser Asn Leu Gln Ser Gly Val Pro
   65                  70                  75                  80
   Ser Arg Phe Thr Gly Ser Gly Ser Gly Thr Glu Phe Thr Leu Ile Val
                   85                  90                  95
   Ser Ser Leu Gln Pro Glu Asp Phe Ala Thr Tyr Tyr Cys Leu Gln His
               100                 105                 110
   His Ser Tyr Pro Leu Thr Ser Gly Gly Gly Thr Lys Val Glu Ile Lys
           115                 120                 125
   Arg Thr Gly Ser Thr Ser Gly Ser Gly Lys Pro Gly Ser Gly Glu Gly
       130                 135                 140
   Ser Glu Val Gln Val Leu Glu Ser Gly Gly Gly Leu Val Gln Pro Gly
   145                 150                 155                 160
   Gly Ser Leu Arg Leu Ser Cys Ala Ala Ser Gly Phe Thr Phe Ser Ser
                   165                 170                 175
   Tyr Ala Met Ser Trp Val Arg Gln Ala Pro Gly Lys Gly Leu Glu Trp
               180                 185                 190
   Val Ser Ala Ile Ser Gly Ser Gly Gly Ser Thr Asn Tyr Ala Asp Ser
           195                 200                 205
   Val Lys Gly Arg Phe Thr Ile Ser Arg Asp Asn Ser Lys Asn Thr Leu
       210                 215                 220
   Tyr Leu Gln Met Asn Ser Leu Arg Ala Glu Asp Thr Ala Val Tyr Tyr
   225                 230                 235                 240
   Cys Ala Gly Ser Ser Gly Trp Ser Glu Tyr Trp Gly Gln Gly Thr Leu
                   245                 250                 255
   Val Thr Val Ser Ser Thr Thr Thr Pro Ala Pro Arg Pro Pro Thr Pro
               260                 265                 270
   Ala Pro Thr Ile Ala Ser Gln Pro Leu Ser Leu Arg Pro Glu Ala Cys
           275                 280                 285
   Arg Pro Ala Ala Gly Gly Ala Val His Thr Arg Gly Leu Asp Phe Ala
       290                 295                 300
   Cys Asp Ile Tyr Ile Trp Ala Pro Leu Ala Gly Thr Cys Gly Val Leu
   305                 310                 315                 320
   Leu Leu Ser Leu Val Ile Thr Leu Tyr Cys Lys Arg Gly Arg Lys Lys
                   325                 330                 335
   Leu Leu
<210> 27
   <211> 501
   <212> PRT
   <213> Artificial Sequence
<220>
   <223> Description of Artificial Sequence: Synthetic polypeptide
<400> 27
   Met Ala Leu Pro Val Thr Ala Leu Leu Leu Pro Leu Ala Leu Leu Leu
   1               5                   10                  15
   His Ala Ala Arg Pro Gly Ser Asp Ile Val Leu Thr Gln Ser Pro Ala
               20                  25                  30
   Ser Leu Ala Val Ser Leu Gly Gln Arg Ala Thr Ile Ser Cys Arg Ala
           35                  40                  45
   Ser Glu Ser Val Asp Asn Tyr Gly Phe Ser Phe Met Asn Trp Phe Gln
       50                  55                  60
   Gln Lys Pro Gly Gln Pro Pro Lys Leu Leu Ile Tyr Ala Ile Ser Asn
   65                  70                  75                  80
   Arg Gly Ser Gly Val Pro Ala Arg Phe Ser Gly Ser Gly Ser Gly Thr
                   85                  90                  95
   Asp Phe Ser Leu Asn Ile His Pro Val Glu Glu Asp Asp Pro Ala Met
               100                 105                 110
   Tyr Phe Cys Gln Gln Thr Lys Glu Val Pro Trp Thr Phe Gly Gly Gly
           115                 120                 125
   Thr Lys Leu Glu Ile Lys Gly Gly Gly Gly Ser Gly Gly Gly Gly Ser
       130                 135                 140
   Gly Gly Gly Gly Ser Glu Val His Leu Val Glu Ser Gly Gly Asp Leu
   145                 150                 155                 160
   Val Lys Pro Gly Gly Ser Leu Lys Leu Ser Cys Ala Ala Ser Gly Phe
                   165                 170                 175
   Thr Phe Ser His Tyr Gly Met Ser Trp Val Arg Gln Thr Pro Asp Lys
               180                 185                 190
   Arg Leu Glu Trp Val Ala Thr Ile Gly Ser Arg Gly Thr Tyr Thr His
           195                 200                 205
   Tyr Pro Asp Ser Val Lys Gly Arg Phe Thr Ile Ser Arg Asp Asn Asp
   210 215 220
   Lys Asn Ala Leu Tyr Leu Gln Met Asn Ser Leu Lys Ser Glu Asp Thr
   225 230 235 240
   Ala Met Tyr Tyr Cys Ala Arg Arg Ser Glu Phe Tyr Tyr Tyr Gly Asn
   245 250 255
   Thr Tyr Tyr Tyr Ser Ala Met Asp Tyr Trp Gly Gln Gly Ala Ser Val
   260 265 270
   Thr Val Ser Ser Ala Ser Thr Thr Thr Pro Ala Pro Arg Pro Pro Thr
   275 280 285
   Pro Ala Pro Thr Ile Ala Ser Gln Pro Leu Ser Leu Arg Pro Glu Ala
   290 295 300
   Cys Arg Pro Ala Ala Gly Gly Ala Val His Thr Arg Gly Leu Asp Phe
   305 310 315 320
   Ala Cys Asp Ile Tyr Ile Trp Ala Pro Leu Ala Gly Thr Cys Gly Val
   325 330 335
   Leu Leu Leu Ser Leu Val Ile Thr Leu Tyr Cys Lys Arg Gly Arg Lys
   340 345 350
   Lys Leu Leu Tyr Ile Phe Lys Gln Pro Phe Met Arg Pro Val Gln Thr
   355 360 365
   Thr Gln Glu Glu Asp Gly Cys Ser Cys Arg Phe Pro Glu Glu Glu Glu
   370 375 380
   Gly Gly Cys Glu Leu Arg Val Lys Phe Ser Arg Ser Ala Asp Ala Pro
   385 390 395 400
   Ala Tyr Lys Gln Gly Gln Asn Gln Leu Tyr Asn Glu Leu Asn Leu Gly
   405 410 415
   Arg Arg Glu Glu Tyr Asp Val Leu Asp Lys Arg Arg Gly Arg Asp Pro
   420 425 430
   Glu Met Gly Gly Lys Pro Arg Arg Lys Asn Pro Gln Glu Gly Leu Tyr
   435 440 445
   Asn Glu Leu Gln Lys Asp Lys Met Ala Glu Ala Tyr Ser Glu Ile Gly
   450 455 460
   Met Lys Gly Glu Arg Arg Arg Gly Lys Gly His Asp Gly Leu Tyr Gln
   465 470 475 480
   Gly Leu Ser Thr Ala Thr Lys Asp Thr Tyr Asp Ala Leu His Met Gln
   485 490 495
   Ala Leu Pro Pro Arg
   500
<210> 28
   <211> 367
   <212> PRT
   <213> Homo sapiens
<400> 28
   Met Ala Leu Pro Val Thr Ala Leu Leu Leu Pro Leu Ala Leu Leu Leu
   1 5 10 15
   His Ala Ala Arg Pro Pro Gly Trp Phe Leu Asp Ser Pro Asp Arg Pro
   20 25 30
   Trp Asn Pro Pro Thr Phe Ser Pro Ala Leu Leu Val Val Thr Glu Gly
   35 40 45
   Asp Asn Ala Thr Phe Thr Cys Ser Phe Ser Asn Thr Ser Glu Ser Phe
   50 55 60
   Val Leu Asn Trp Tyr Arg Met Ser Pro Ser Asn Gln Thr Asp Lys Leu
   65 70 75 80
   Ala Ala Phe Pro Glu Asp Arg Ser Gln Pro Gly Gln Asp Cys Arg Phe
   85 90 95
   Arg Val Thr Gln Leu Pro Asn Gly Arg Asp Phe His Met Ser Val Val
   100 105 110
   Arg Ala Arg Arg Asn Asp Ser Gly Thr Tyr Leu Cys Gly Ala Ile Ser
   115 120 125
   Leu Ala Pro Lys Ala Gln Ile Lys Glu Ser Leu Arg Ala Glu Leu Arg
   130 135 140
   Val Thr Glu Arg Arg Ala Glu Val Pro Thr Ala His Pro Ser Pro Ser
   145 150 155 160
   Pro Arg Pro Ala Gly Gln Phe Gln Thr Leu Val Thr Thr Thr Pro Ala
   165 170 175
   Pro Arg Pro Pro Thr Pro Ala Pro Thr Ile Ala Ser Gln Pro Leu Ser
   180 185 190
   Leu Arg Pro Glu Ala Cys Arg Pro Ala Ala Gly Gly Ala Val His Thr
   195 200 205
   Arg Gly Leu Asp Phe Ala Cys Asp Ile Tyr Ile Trp Ala Pro Leu Ala
   210 215 220
   Gly Thr Cys Gly Val Leu Leu Leu Ser Leu Val Ile Thr Leu Tyr Cys
   225 230 235 240
   Lys Arg Gly Arg Lys Lys Leu Leu Gly Gly Gly Gly Ser Gly Gly Gly
   245 250 255
   Gly Ser Gly Val Gln Val Glu Thr Ile Ser Pro Gly Asp Gly Arg Thr
   260 265 270
   Phe Pro Lys Arg Gly Gln Thr Cys Val Val His Tyr Thr Gly Met Leu
   275 280 285
   Glu Asp Gly Lys Lys Phe Asp Ser Ser Arg Asp Arg Asn Lys Pro Phe
   290 295 300
   Lys Phe Met Leu Gly Lys Gln Glu Val Ile Arg Gly Trp Glu Glu Gly
   305 310 315 320
   Val Ala Gln Met Ser Val Gly Gln Arg Ala Lys Leu Thr Ile Ser Pro
   325 330 335
   Asp Tyr Ala Tyr Gly Ala Thr Gly His Pro Gly Ile Ile Pro Pro His
   340 345 350
   Ala Thr Leu Val Phe Asp Val Glu Leu Leu Lys Leu Glu Thr Ser
   355 360 365
<210> 29
   <211> 356
   <212> PRT
   <213> Homo sapiens
<400> 29
   Met Ala Leu Pro Val Thr Ala Leu Leu Leu Pro Leu Ala Leu Leu Leu
   1 5 10 15
   His Ala Ala Arg Pro Pro Gly Trp Phe Leu Asp Ser Pro Asp Arg Pro
   20 25 30
   Trp Asn Pro Pro Thr Phe Ser Pro Ala Leu Leu Val Val Thr Glu Gly
   35 40 45
   Asp Asn Ala Thr Phe Thr Cys Ser Phe Ser Asn Thr Ser Glu Ser Phe
   50 55 60
   Val Leu Asn Trp Tyr Arg Met Ser Pro Ser Asn Gln Thr Asp Lys Leu
   65 70 75 80
   Ala Ala Phe Pro Glu Asp Arg Ser Gln Pro Gly Gln Asp Cys Arg Phe
   85 90 95
   Arg Val Thr Gln Leu Pro Asn Gly Arg Asp Phe His Met Ser Val Val
   100 105 110
   Arg Ala Arg Arg Asn Asp Ser Gly Thr Tyr Leu Cys Gly Ala Ile Ser
   115 120 125
   Leu Ala Pro Lys Ala Gln Ile Lys Glu Ser Leu Arg Ala Glu Leu Arg
   130 135 140
   Val Thr Glu Arg Arg Ala Glu Val Pro Thr Ala His Pro Ser Pro Ser
   145 150 155 160
   Pro Arg Pro Ala Gly Gln Phe Gln Thr Leu Val Thr Thr Thr Pro Ala
   165 170 175
   Pro Arg Pro Pro Thr Pro Ala Pro Thr Ile Ala Ser Gln Pro Leu Ser
   180 185 190
   Leu Arg Pro Glu Ala Cys Arg Pro Ala Ala Gly Gly Ala Val His Thr
   195 200 205
   Arg Gly Leu Asp Phe Ala Cys Asp Ile Tyr Ile Trp Ala Pro Leu Ala
   210 215 220
   Gly Thr Cys Gly Val Leu Leu Leu Ser Leu Val Ile Thr Leu Tyr Cys
   225 230 235 240
   Lys Arg Gly Arg Lys Lys Leu Leu Gly Gly Gly Gly Ser Gly Gly Gly
   245 250 255
   Gly Ser Ala Ser Arg Ile Leu Trp His Glu Met Trp His Glu Gly Leu
   260 265 270
   Glu Glu Ala Ser Arg Leu Tyr Phe Gly Glu Arg Asn Val Lys Gly Met
   275 280 285
   Phe Glu Val Leu Glu Pro Leu His Ala Met Met Glu Arg Gly Pro Gln
       290                 295                 300
   Thr Leu Lys Glu Thr Ser Phe Asn Gln Ala Tyr Gly Arg Asp Leu Met
   305                 310                 315                 320
   Glu Ala Gln Glu Trp Cys Arg Lys Tyr Met Lys Ser Gly Asn Val Lys
                   325                 330                 335
   Asp Leu Leu Gln Ala Trp Asp Leu Tyr Tyr His Val Phe Arg Arg Ile
               340                 345                 350
   Ser Lys Thr Ser
           355
<210> 30
   <211> 446
   <212> PRT
   <213> Artificial Sequence
<220>
   <223> Description of Artificial Sequence: Synthetic polypeptide
<400> 30
   Met Ala Leu Pro Val Thr Ala Leu Leu Leu Pro Leu Ala Leu Leu Leu
   1               5                   10                  15
   His Ala Ala Arg Pro Asp Ile Gln Met Thr Gln Ser Pro Ser Ser Leu
               20                  25                  30
   Ser Ala Ser Val Gly Asp Arg Val Thr Ile Thr Cys Arg Ala Ser Gln
           35                  40                  45
   Gly Ile Arg Asn Asn Leu Ala Trp Tyr Gln Gln Lys Pro Gly Lys Ala
       50                  55                  60
   Pro Lys Arg Leu Ile Tyr Ala Ala Ser Asn Leu Gln Ser Gly Val Pro
   65                  70                  75                  80
   Ser Arg Phe Thr Gly Ser Gly Ser Gly Thr Glu Phe Thr Leu Ile Val
                   85                  90                  95
   Ser Ser Leu Gln Pro Glu Asp Phe Ala Thr Tyr Tyr Cys Leu Gln His
               100                 105                 110
   His Ser Tyr Pro Leu Thr Ser Gly Gly Gly Thr Lys Val Glu Ile Lys
           115                 120                 125
   Arg Thr Gly Ser Thr Ser Gly Ser Gly Lys Pro Gly Ser Gly Glu Gly
   130 135 140
   Ser Glu Val Gln Val Leu Glu Ser Gly Gly Gly Leu Val Gln Pro Gly
   145 150 155 160
   Gly Ser Leu Arg Leu Ser Cys Ala Ala Ser Gly Phe Thr Phe Ser Ser
   165 170 175
   Tyr Ala Met Ser Trp Val Arg Gln Ala Pro Gly Lys Gly Leu Glu Trp
   180 185 190
   Val Ser Ala Ile Ser Gly Ser Gly Gly Ser Thr Asn Tyr Ala Asp Ser
   195 200 205
   Val Lys Gly Arg Phe Thr Ile Ser Arg Asp Asn Ser Lys Asn Thr Leu
   210 215 220
   Tyr Leu Gln Met Asn Ser Leu Arg Ala Glu Asp Thr Ala Val Tyr Tyr
   225 230 235 240
   Cys Ala Gly Ser Ser Gly Trp Ser Glu Tyr Trp Gly Gln Gly Thr Leu
   245 250 255
   Val Thr Val Ser Ser Lys Arg Gly Arg Lys Lys Leu Leu Gly Gly Gly
   260 265 270
   Gly Ser Gly Gly Gly Gly Ser Ala Ser Arg Ile Leu Trp His Glu Met
   275 280 285
   Trp His Glu Gly Leu Glu Glu Ala Ser Arg Leu Tyr Phe Gly Glu Arg
   290 295 300
   Asn Val Lys Gly Met Phe Glu Val Leu Glu Pro Leu His Ala Met Met
   305 310 315 320
   Glu Arg Gly Pro Gln Thr Leu Lys Glu Thr Ser Phe Asn Gln Ala Tyr
   325 330 335
   Gly Arg Asp Leu Met Glu Ala Gln Glu Trp Cys Arg Lys Tyr Met Lys
   340 345 350
   Ser Gly Asn Val Lys Asp Leu Leu Gln Ala Trp Asp Leu Tyr Tyr His
   355 360 365
   Val Phe Arg Arg Ile Ser Lys Thr Ser Thr Thr Thr Pro Ala Pro Arg
   370 375 380
   Pro Pro Thr Pro Ala Pro Thr Ile Ala Ser Gln Pro Leu Ser Leu Arg
   385 390 395 400
   Pro Glu Ala Cys Arg Pro Ala Ala Gly Gly Ala Val His Thr Arg Gly
   405 410 415
   Leu Asp Phe Ala Cys Asp Ile Tyr Ile Trp Ala Pro Leu Ala Gly Thr
   420 425 430
   Cys Gly Val Leu Leu Leu Ser Leu Val Ile Thr Leu Tyr Cys
   435 440 445
<210> 31
   <211> 457
   <212> PRT
   <213> Artificial Sequence
<220>
   <223> Description of Artificial Sequence: Synthetic polypeptide
<400> 31
   Met Ala Leu Pro Val Thr Ala Leu Leu Leu Pro Leu Ala Leu Leu Leu
   1 5 10 15
   His Ala Ala Arg Pro Asp Ile Gln Met Thr Gln Ser Pro Ser Ser Leu
   20 25 30
   Ser Ala Ser Val Gly Asp Arg Val Thr Ile Thr Cys Arg Ala Ser Gln
   35 40 45
   Gly Ile Arg Asn Asn Leu Ala Trp Tyr Gln Gln Lys Pro Gly Lys Ala
   50 55 60
   Pro Lys Arg Leu Ile Tyr Ala Ala Ser Asn Leu Gln Ser Gly Val Pro
   65 70 75 80
   Ser Arg Phe Thr Gly Ser Gly Ser Gly Thr Glu Phe Thr Leu Ile Val
   85 90 95
   Ser Ser Leu Gln Pro Glu Asp Phe Ala Thr Tyr Tyr Cys Leu Gln His
   100 105 110
   His Ser Tyr Pro Leu Thr Ser Gly Gly Gly Thr Lys Val Glu Ile Lys
   115 120 125
   Arg Thr Gly Ser Thr Ser Gly Ser Gly Lys Pro Gly Ser Gly Glu Gly
   130 135 140
   Ser Glu Val Gln Val Leu Glu Ser Gly Gly Gly Leu Val Gln Pro Gly
   145                 150                 155                 160
   Gly Ser Leu Arg Leu Ser Cys Ala Ala Ser Gly Phe Thr Phe Ser Ser
                   165                 170                 175
   Tyr Ala Met Ser Trp Val Arg Gln Ala Pro Gly Lys Gly Leu Glu Trp
               180                 185                 190
   Val Ser Ala Ile Ser Gly Ser Gly Gly Ser Thr Asn Tyr Ala Asp Ser
           195                 200                 205
   Val Lys Gly Arg Phe Thr Ile Ser Arg Asp Asn Ser Lys Asn Thr Leu
       210                 215                 220
   Tyr Leu Gln Met Asn Ser Leu Arg Ala Glu Asp Thr Ala Val Tyr Tyr
   225                 230                 235                 240
   Cys Ala Gly Ser Ser Gly Trp Ser Glu Tyr Trp Gly Gln Gly Thr Leu
                   245                 250                 255
   Val Thr Val Ser Ser Lys Arg Gly Arg Lys Lys Leu Leu Gly Gly Gly
               260                 265                 270
   Gly Ser Gly Gly Gly Gly Ser Gly Val Gln Val Glu Thr Ile Ser Pro
           275                 280                 285
   Gly Asp Gly Arg Thr Phe Pro Lys Arg Gly Gln Thr Cys Val Val His
       290                 295                 300
   Tyr Thr Gly Met Leu Glu Asp Gly Lys Lys Phe Asp Ser Ser Arg Asp
   305                 310                 315                 320
   Arg Asn Lys Pro Phe Lys Phe Met Leu Gly Lys Gln Glu Val Ile Arg
                   325                 330                 335
   Gly Trp Glu Glu Gly Val Ala Gln Met Ser Val Gly Gln Arg Ala Lys
               340                 345                 350
   Leu Thr Ile Ser Pro Asp Tyr Ala Tyr Gly Ala Thr Gly His Pro Gly
           355                 360                 365
   Ile Ile Pro Pro His Ala Thr Leu Val Phe Asp Val Glu Leu Leu Lys
       370                 375                 380
   Leu Glu Thr Ser Thr Thr Thr Pro Ala Pro Arg Pro Pro Thr Pro Ala
   385                 390                 395                 400
   Pro Thr Ile Ala Ser Gln Pro Leu Ser Leu Arg Pro Glu Ala Cys Arg
   405 410 415
   Pro Ala Ala Gly Gly Ala Val His Thr Arg Gly Leu Asp Phe Ala Cys
   420 425 430
   Asp Ile Tyr Ile Trp Ala Pro Leu Ala Gly Thr Cys Gly Val Leu Leu
   435 440 445
   Leu Ser Leu Val Ile Thr Leu Tyr Cys
   450 455
<210> 32
   <211> 351
   <212> PRT
   <213> Artificial Sequence
<220>
   <223> Description of Artificial Sequence: Synthetic polypeptide
<400> 32
   Met Ala Leu Pro Val Thr Ala Leu Leu Leu Pro Leu Ala Leu Leu Leu
   1 5 10 15
   His Ala Ala Arg Ala Ser Arg Ile Leu Trp His Glu Met Trp His Glu
   20 25 30
   Gly Leu Glu Glu Ala Ser Arg Leu Tyr Phe Gly Glu Arg Asn Val Lys
   35 40 45
   Gly Met Phe Glu Val Leu Glu Pro Leu His Ala Met Met Glu Arg Gly
   50 55 60
   Pro Gln Thr Leu Lys Glu Thr Ser Phe Asn Gln Ala Tyr Gly Arg Asp
   65 70 75 80
   Leu Met Glu Ala Gln Glu Trp Cys Arg Lys Tyr Met Lys Ser Gly Asn
   85 90 95
   Val Lys Asp Leu Leu Gln Ala Trp Asp Leu Tyr Tyr His Val Phe Arg
   100 105 110
   Arg Ile Ser Lys Thr Ser Thr Thr Thr Pro Ala Pro Arg Pro Pro Thr
   115 120 125
   Pro Ala Pro Thr Ile Ala Ser Gln Pro Leu Ser Leu Arg Pro Glu Ala
   130 135 140
   Cys Arg Pro Ala Ala Gly Gly Ala Val His Thr Arg Gly Leu Asp Phe
   145                 150                 155                 160
   Ala Cys Asp Ile Tyr Ile Trp Ala Pro Leu Ala Gly Thr Cys Gly Val
                   165                 170                 175
   Leu Leu Leu Ser Leu Val Ile Thr Leu Tyr Cys Gly Gly Gly Gly Ser
               180                 185                 190
   Gly Gly Gly Gly Ser Lys Arg Gly Arg Lys Lys Leu Leu Tyr Ile Phe
           195                 200                 205
   Lys Gln Pro Phe Met Arg Pro Val Gln Thr Thr Gln Glu Glu Asp Gly
       210                 215                 220
   Cys Ser Cys Arg Phe Pro Glu Glu Glu Glu Gly Gly Cys Glu Leu Arg
   225                 230                 235                 240
   Val Lys Phe Ser Arg Ser Ala Asp Ala Pro Ala Tyr Lys Gln Gly Gln
                   245                 250                 255
   Asn Gln Leu Tyr Asn Glu Leu Asn Leu Gly Arg Arg Glu Glu Tyr Asp
               260                 265                 270
   Val Leu Asp Lys Arg Arg Gly Arg Asp Pro Glu Met Gly Gly Lys Pro
           275                 280                 285
   Arg Arg Lys Asn Pro Gln Glu Gly Leu Tyr Asn Glu Leu Gln Lys Asp
       290                 295                 300
   Lys Met Ala Glu Ala Tyr Ser Glu Ile Gly Met Lys Gly Glu Arg Arg
   305                 310                 315                 320
   Arg Gly Lys Gly His Asp Gly Leu Tyr Gln Gly Leu Ser Thr Ala Thr
                   325                 330                 335
   Lys Asp Thr Tyr Asp Ala Leu His Met Gln Ala Leu Pro Pro Arg
               340                 345                 350
<210> 33
   <211> 362
   <212> PRT
   <213> Artificial Sequence
<220>
   <223> Description of Artificial Sequence: Synthetic polypeptide
<400> 33
   Met Ala Leu Pro Val Thr Ala Leu Leu Leu Pro Leu Ala Leu Leu Leu
   1 5 10 15
   His Ala Ala Arg Gly Val Gln Val Glu Thr Ile Ser Pro Gly Asp Gly
   20 25 30
   Arg Thr Phe Pro Lys Arg Gly Gln Thr Cys Val Val His Tyr Thr Gly
   35 40 45
   Met Leu Glu Asp Gly Lys Lys Phe Asp Ser Ser Arg Asp Arg Asn Lys
   50 55 60
   Pro Phe Lys Phe Met Leu Gly Lys Gln Glu Val Ile Arg Gly Trp Glu
   65 70 75 80
   Glu Gly Val Ala Gln Met Ser Val Gly Gln Arg Ala Lys Leu Thr Ile
   85 90 95
   Ser Pro Asp Tyr Ala Tyr Gly Ala Thr Gly His Pro Gly Ile Ile Pro
   100 105 110
   Pro His Ala Thr Leu Val Phe Asp Val Glu Leu Leu Lys Leu Glu Thr
   115 120 125
   Ser Thr Thr Thr Pro Ala Pro Arg Pro Pro Thr Pro Ala Pro Thr Ile
   130 135 140
   Ala Ser Gln Pro Leu Ser Leu Arg Pro Glu Ala Cys Arg Pro Ala Ala
   145 150 155 160
   Gly Gly Ala Val His Thr Arg Gly Leu Asp Phe Ala Cys Asp Ile Tyr
   165 170 175
   Ile Trp Ala Pro Leu Ala Gly Thr Cys Gly Val Leu Leu Leu Ser Leu
   180 185 190
   Val Ile Thr Leu Tyr Cys Gly Gly Gly Gly Ser Gly Gly Gly Gly Ser
   195 200 205
   Lys Arg Gly Arg Lys Lys Leu Leu Tyr Ile Phe Lys Gln Pro Phe Met
   210 215 220
   Arg Pro Val Gln Thr Thr Gln Glu Glu Asp Gly Cys Ser Cys Arg Phe
   225 230 235 240
   Pro Glu Glu Glu Glu Gly Gly Cys Glu Leu Arg Val Lys Phe Ser Arg
   245 250 255
   Ser Ala Asp Ala Pro Ala Tyr Lys Gln Gly Gln Asn Gln Leu Tyr Asn
   260 265 270
   Glu Leu Asn Leu Gly Arg Arg Glu Glu Tyr Asp Val Leu Asp Lys Arg
   275 280 285
   Arg Gly Arg Asp Pro Glu Met Gly Gly Lys Pro Arg Arg Lys Asn Pro
   290 295 300
   Gln Glu Gly Leu Tyr Asn Glu Leu Gln Lys Asp Lys Met Ala Glu Ala
   305 310 315 320
   Tyr Ser Glu Ile Gly Met Lys Gly Glu Arg Arg Arg Gly Lys Gly His
   325 330 335
   Asp Gly Leu Tyr Gln Gly Leu Ser Thr Ala Thr Lys Asp Thr Tyr Asp
   340 345 350
   Ala Leu His Met Gln Ala Leu Pro Pro Arg
   355 360
<210> 34
   <211> 19
   <212> DNA
   <213> Mus musculus
<400> 34
   ggaggtccct caccttcta 19
<210> 35
   <211> 19
   <212> DNA
   <213> Mus musculus
<400> 35
   cggaggatct tatgctgaa 19
<210> 36
   <211> 19
   <212> DNA
   <213> Mus musculus
<400> 36
   cccgcttcca gatcataca 19
<210> 37
   <211> 19
   <212> DNA
   <213> Mus musculus
<400> 37
   ggagacctca acaagatat 19
<210> 38
   <211> 19
   <212> DNA
   <213> Mus musculus
<400> 38
   aaggcatggt cattggtat 19
<210> 39
   <211> 19
   <212> DNA
   <213> Mus musculus
<400> 39
   gcatggtcat tggtatcat 19
<210> 40
   <211> 19
   <212> DNA
   <213> Mus musculus
<400> 40
   ggtcattggt atcatgagt 19
<210> 41
   <211> 19
   <212> DNA
   <213> Mus musculus
<400> 41
   cctagtgggt atccctgta 19
<210> 42
   <211> 19
   <212> DNA
   <213> Mus musculus
<400> 42
   gaggatggac attgttctt 19
<210> 43
   <211> 19
   <212> DNA
   <213> Mus musculus
<400> 43
   gcatgcaggc tacagttca 19
<210> 44
   <211> 19
   <212> DNA
   <213> Mus musculus
<400> 44
   ccagcacatg cactgttga 19
<210> 45
   <211> 19
   <212> DNA
   <213> Mus musculus
<400> 45
   cacatgcact gttgagtga 19
<210> 46
   <211> 21
   <212> DNA
   <213> Mus musculus
<400> 46
   ctggaggtcc ctcaccttct a 21
<210> 47
   <211> 21
   <212> DNA
   <213> Mus musculus
<400> 47
   gtcggaggat cttatgctga a 21
<210> 48
   <211> 21
   <212> DNA
   <213> Mus musculus
<400> 48
   tgcccgcttc cagatcatac a 21
<210> 49
   <211> 21
   <212> DNA
   <213> Mus musculus
<400> 49
   ctggagacct caacaagata t 21
<210> 50
   <211> 21
   <212> DNA
   <213> Mus musculus
<400> 50
   tcaaggcatg gtcattggta t 21
<210> 51
   <211> 21
   <212> DNA
   <213> Mus musculus
<400> 51
   aggcatggtc attggtatca t 21
<210> 52
   <211> 21
   <212> DNA
   <213> Mus musculus
<400> 52
   atggtcattg gtatcatgag t 21
<210> 53
   <211> 21
   <212> DNA
   <213> Mus musculus
<400> 53
   gccctagtgg gtatccctgt a 21
<210> 54
   <211> 21
   <212> DNA
   <213> Mus musculus
<400> 54
   atgaggatgg acattgttct t 21
<210> 55
   <211> 21
   <212> DNA
   <213> Mus musculus
<400> 55
   gagcatgcag gctacagttc a 21
<210> 56
   <211> 21
   <212> DNA
   <213> Mus musculus
<400> 56
   ttccagcaca tgcactgttg a 21
<210> 57
   <211> 21
   <212> DNA
   <213> Mus musculus
<400> 57
   agcacatgca ctgttgagtg a 21
<210> 58
   <211> 21
   <212> DNA
   <213> Mus musculus
<400> 58
   tagaaggtga gggacctcca g 21
<210> 59
   <211> 21
   <212> DNA
   <213> Mus musculus
<400> 59
   ttcagcataa gatcctccga c 21
<210> 60
   <211> 21
   <212> DNA
   <213> Mus musculus
<400> 60
   tgtatgatct ggaagcgggc a 21
<210> 61
   <211> 21
   <212> DNA
   <213> Mus musculus
<400> 61
   atatcttgtt gaggtctcca g 21
<210> 62
   <211> 21
   <212> DNA
   <213> Mus musculus
<400> 62
   ataccaatga ccatgccttg a 21
<210> 63
   <211> 21
   <212> DNA
   <213> Mus musculus
<400> 63
   atgataccaa tgaccatgcc t 21
<210> 64
   <211> 21
   <212> DNA
   <213> Mus musculus
<400> 64
   atggtcattg gtatcatgag t 21
<210> 65
   <211> 21
   <212> DNA
   <213> Mus musculus
<400> 65
   gccctagtgg gtatccctgt a 21
<210> 66
   <211> 21
   <212> DNA
   <213> Mus musculus
<400> 66
   atgaggatgg acattgttct t 21
<210> 67
   <211> 21
   <212> DNA
   <213> Mus musculus
<400> 67
   gagcatgcag gctacagttc a 21
<210> 68
   <211> 21
   <212> DNA
   <213> Mus musculus
<400> 68
   ttccagcaca tgcactgttg a 21
<210> 69
   <211> 21
   <212> DNA
   <213> Mus musculus
<400> 69
   agcacatgca ctgttgagtg a 21
<210> 70
   <211> 19
   <212> DNA
   <213> Mus musculus
<400> 70
   tagaaggtga gggacctcc 19
<210> 71
   <211> 19
   <212> DNA
   <213> Mus musculus
<400> 71
   ttcagcataa gatcctccg 19
<210> 72
   <211> 19
   <212> DNA
   <213> Mus musculus
<400> 72
   tgtatgatct ggaagcggg 19
<210> 73
   <211> 19
   <212> DNA
   <213> Mus musculus
<400> 73
   atatcttgtt gaggtctcc 19
<210> 74
   <211> 19
   <212> DNA
   <213> Mus musculus
<400> 74
   ataccaatga ccatgcctt 19
<210> 75
   <211> 19
   <212> DNA
   <213> Mus musculus
<400> 75
   atgataccaa tgaccatgc 19
<210> 76
   <211> 19
   <212> DNA
   <213> Mus musculus
<400> 76
   atggtcattg gtatcatga 19
<210> 77
   <211> 19
   <212> DNA
   <213> Mus musculus
<400> 77
   gccctagtgg gtatccctg 19
<210> 78
   <211> 19
   <212> DNA
   <213> Mus musculus
<400> 78
   atgaggatgg acattgttc 19
<210> 79
   <211> 19
   <212> DNA
   <213> Mus musculus
<400> 79
   gagcatgcag gctacagtt 19
<210> 80
   <211> 19
   <212> DNA
   <213> Mus musculus
<400> 80
   ttccagcaca tgcactgtt 19
<210> 81
   <211> 19
   <212> DNA
   <213> Mus musculus
<400> 81
   agcacatgca ctgttgagt 19
<210> 82
   <211> 19
   <212> DNA
   <213> Homo sapiens
<400> 82
   ggccaggatg gttcttaga 19
<210> 83
   <211> 19
   <212> DNA
   <213> Homo sapiens
<400> 83
   gcttcgtgct aaactggta 19
<210> 84
   <211> 19
   <212> DNA
   <213> Homo sapiens
<400> 84
   gggcgtgact tccacatga 19
<210> 85
   <211> 19
   <212> DNA
   <213> Homo sapiens
<400> 85
   caggcctaga gaagtttca 19
<210> 86
   <211> 19
   <212> DNA
   <213> Homo sapiens
<400> 86
   cttggaaccc attcctgaa 19
<210> 87
   <211> 19
   <212> DNA
   <213> Homo sapiens
<400> 87
   ggaacccatt cctgaaatt 19
<210> 88
   <211> 19
   <212> DNA
   <213> Homo sapiens
<400> 88
   gaacccattc ctgaaatta 19
<210> 89
   <211> 19
   <212> DNA
   <213> Homo sapiens
<400> 89
   aacccattcc tgaaattat 19
<210> 90
   <211> 19
   <212> DNA
   <213> Homo sapiens
<400> 90
   acccattcct gaaattatt 19
<210> 91
   <211> 19
   <212> DNA
   <213> Homo sapiens
<400> 91
   cccattcctg aaattattt 19
<210> 92
   <211> 19
   <212> DNA
   <213> Homo sapiens
<400> 92
   ctgtggttct attatatta 19
<210> 93
   <211> 19
   <212> DNA
   <213> Homo sapiens
<400> 93
   aaatatgaga gcatgctaa 19
<210> 94
   <211> 19
   <212> DNA
   <213> Homo sapiens
<400> 94
   tctaagaacc atcctggcc 19
<210> 95
   <211> 19
   <212> DNA
   <213> Homo sapiens
<400> 95
   taccagttta gcacgaagc 19
<210> 96
   <211> 19
   <212> DNA
   <213> Homo sapiens
<400> 96
   tcatgtggaa gtcacgccc 19
<210> 97
   <211> 19
   <212> DNA
   <213> Homo sapiens
<400> 97
   tgaaacttct ctaggcctg 19
<210> 98
   <211> 19
   <212> DNA
   <213> Homo sapiens
<400> 98
   ttcaggaatg ggttccaag 19
<210> 99
   <211> 19
   <212> DNA
   <213> Homo sapiens
<400> 99
   aatttcagga atgggttcc 19
<210> 100
   <211> 19
   <212> DNA
   <213> Homo sapiens
<400> 100
   taatttcagg aatgggttc 19
<210> 101
   <211> 19
   <212> DNA
   <213> Homo sapiens
<400> 101
   ataatttcag gaatgggtt 19
<210> 102
   <211> 19
   <212> DNA
   <213> Homo sapiens
<400> 102
   aataatttca ggaatgggt 19
<210> 103
   <211> 19
   <212> DNA
   <213> Homo sapiens
<400> 103
   aaataatttc aggaatggg 19
<210> 104
   <211> 19
   <212> DNA
   <213> Homo sapiens
<400> 104
   taatataata gaaccacag 19
<210> 105
   <211> 19
   <212> DNA
   <213> Homo sapiens
<400> 105
   ttagcatgct ctcatattt 19
<210> 106
   <211> 21
   <212> DNA
   <213> Homo sapiens
<400> 106
   gcggccagga tggttcttag a 21
<210> 107
   <211> 21
   <212> DNA
   <213> Homo sapiens
<400> 107
   gagcttcgtg ctaaactggt a 21
<210> 108
   <211> 21
   <212> DNA
   <213> Homo sapiens
<400> 108
   acgggcgtga cttccacatg a 21
<210> 109
   <211> 21
   <212> DNA
   <213> Homo sapiens
<400> 109
   tgcaggccta gagaagtttc a 21
<210> 110
   <211> 21
   <212> DNA
   <213> Homo sapiens
<400> 110
   tccttggaac ccattcctga a 21
<210> 111
   <211> 21
   <212> DNA
   <213> Homo sapiens
<400> 111
   ttggaaccca ttcctgaaat t 21
<210> 112
   <211> 21
   <212> DNA
   <213> Homo sapiens
<400> 112
   tggaacccat tcctgaaatt a 21
<210> 113
   <211> 21
   <212> DNA
   <213> Homo sapiens
<400> 113
   ggaacccatt cctgaaatta t 21
<210> 114
   <211> 21
   <212> DNA
   <213> Homo sapiens
<400> 114
   gaacccattc ctgaaattat t 21
<210> 115
   <211> 21
   <212> DNA
   <213> Homo sapiens
<400> 115
   aacccattcc tgaaattatt t 21
<210> 116
   <211> 21
   <212> DNA
   <213> Homo sapiens
<400> 116
   ccctgtggtt ctattatatt a 21
<210> 117
   <211> 21
   <212> DNA
   <213> Homo sapiens
<400> 117
   ttaaatatga gagcatgcta a 21
<210> 118
   <211> 21
   <212> DNA
   <213> Homo sapiens
<400> 118
   tctaagaacc atcctggccg c 21
<210> 119
   <211> 21
   <212> DNA
   <213> Homo sapiens
<400> 119
   taccagttta gcacgaagct c 21
<210> 120
   <211> 21
   <212> DNA
   <213> Homo sapiens
<400> 120
   tcatgtggaa gtcacgcccg t 21
<210> 121
   <211> 21
   <212> DNA
   <213> Homo sapiens
<400> 121
   tgaaacttct ctaggcctgc a 21
<210> 122
   <211> 21
   <212> DNA
   <213> Homo sapiens
<400> 122
   ttcaggaatg ggttccaagg a 21
<210> 123
   <211> 21
   <212> DNA
   <213> Homo sapiens
<400> 123
   aatttcagga atgggttcca a 21
<210> 124
   <211> 21
   <212> DNA
   <213> Homo sapiens
<400> 124
   taatttcagg aatgggttcc a 21
<210> 125
   <211> 21
   <212> DNA
   <213> Homo sapiens
<400> 125
   ataatttcag gaatgggttc c 21
<210> 126
   <211> 21
   <212> DNA
   <213> Homo sapiens
<400> 126
   aataatttca ggaatgggtt c 21
<210> 127
   <211> 21
   <212> DNA
   <213> Homo sapiens
<400> 127
   aaataatttc aggaatgggt t 21
<210> 128
   <211> 21
   <212> DNA
   <213> Homo sapiens
<400> 128
   taatataata gaaccacagg g 21
<210> 129
   <211> 21
   <212> DNA
   <213> Homo sapiens
<400> 129
   ttagcatgct ctcatattta a 21
<210> 130
   <211> 63
   <212> DNA
   <213> Artificial Sequence
<220>
   <223> Description of Artificial Sequence: Synthetic oligonucleotide
<400> 130
   atggccttac cagtgaccgc cttgctcctg ccgctggcct tgctgctcca cgccgccagg 60
   ccg 63
<210> 131
   <211> 135
   <212> DNA
   <213> Artificial Sequence
<220>
   <223> Description of Artificial Sequence: Synthetic polynucleotide
<400> 131
   accacgacgc cagcgccgcg accaccaaca ccggcgccca ccatcgcgtc gcagcccctg 60
   tccctgcgcc cagaggcgtg ccggccagcg gcggggggcg cagtgcacac gagggggctg 120
   gacttcgcct gtgat 135
<210> 132
   <211> 72
   <212> DNA
   <213> Artificial Sequence
<220>
   <223> Description of Artificial Sequence: Synthetic oligonucleotide
<400> 132
   atctacatct gggcgccctt ggccgggact tgtggggtcc ttctcctgtc actggttatc 60
   accctttact gc 72
<210> 133
   <211> 126
   <212> DNA
   <213> Artificial Sequence
<220>
   <223> Description of Artificial Sequence: Synthetic polynucleotide
<400> 133
   aaacggggca gaaagaaact cctgtatata ttcaaacaac catttatgag accagtacaa 60
   actactcaag aggaagatgg ctgtagctgc cgatttccag aagaagaaga aggaggatgt 120
   gaactg 126
<210> 134
   <211> 336
   <212> DNA
   <213> Artificial Sequence
<220>
   <223> Description of Artificial Sequence: Synthetic polynucleotide
<400> 134
   agagtgaagt tcagcaggag cgcagacgcc cccgcgtaca agcagggcca gaaccagctc 60
   tataacgagc tcaatctagg acgaagagag gagtacgatg ttttggacaa gagacgtggc 120
   cgggaccctg agatgggggg aaagccgaga aggaagaacc ctcaggaagg cctgtacaat 180
   gaactgcaga aagataagat ggcggaggcc tacagtgaga ttgggatgaa aggcgagcgc 240
   cggaggggca aggggcacga tggcctttac cagggtctca gtacagccac caaggacacc 300
   tacgacgccc ttcacatgca ggccctgccc cctcgc 336
<210> 135
   <211> 21
   <212> PRT
   <213> Artificial Sequence
<220>
   <223> Description of Artificial Sequence: Synthetic peptide
<400> 135
   Met Ala Leu Pro Val Thr Ala Leu Leu Leu Pro Leu Ala Leu Leu Leu
   1               5                   10                  15
   His Ala Ala Arg Pro
               20
<210> 136
   <211> 45
   <212> PRT
   <213> Artificial Sequence
<220>
   <223> Description of Artificial Sequence: Synthetic polypeptide
<400> 136
   Thr Thr Thr Pro Ala Pro Arg Pro Pro Thr Pro Ala Pro Thr Ile Ala
   1               5                   10                  15
   Ser Gln Pro Leu Ser Leu Arg Pro Glu Ala Cys Arg Pro Ala Ala Gly
               20                  25                  30
   Gly Ala Val His Thr Arg Gly Leu Asp Phe Ala Cys Asp
           35                  40                  45
<210> 137
   <211> 24
   <212> PRT
   <213> Artificial Sequence
<220>
   <223> Description of Artificial Sequence: Synthetic peptide
<400> 137
   Ile Tyr Ile Trp Ala Pro Leu Ala Gly Thr Cys Gly Val Leu Leu Leu
   1               5                   10                  15
   Ser Leu Val Ile Thr Leu Tyr Cys
               20
<210> 138
   <211> 42
   <212> PRT
   <213> Artificial Sequence
<220>
   <223> Description of Artificial Sequence: Synthetic polypeptide
<400> 138
   Lys Arg Gly Arg Lys Lys Leu Leu Tyr Ile Phe Lys Gln Pro Phe Met
   1               5                   10                  15
   Arg Pro Val Gln Thr Thr Gln Glu Glu Asp Gly Cys Ser Cys Arg Phe
               20                  25                  30
   Pro Glu Glu Glu Glu Gly Gly Cys Glu Leu
           35                  40
<210> 139
   <211> 112
   <212> PRT
   <213> Artificial Sequence
<220>
   <223> Description of Artificial Sequence: Synthetic polypeptide
<400> 139
   Arg Val Lys Phe Ser Arg Ser Ala Asp Ala Pro Ala Tyr Lys Gln Gly
   1               5                   10                  15
   Gln Asn Gln Leu Tyr Asn Glu Leu Asn Leu Gly Arg Arg Glu Glu Tyr
               20                  25                  30
   Asp Val Leu Asp Lys Arg Arg Gly Arg Asp Pro Glu Met Gly Gly Lys
           35                  40                  45
   Pro Arg Arg Lys Asn Pro Gln Glu Gly Leu Tyr Asn Glu Leu Gln Lys
       50                  55                  60
   Asp Lys Met Ala Glu Ala Tyr Ser Glu Ile Gly Met Lys Gly Glu Arg
   65                  70                  75                  80
   Arg Arg Gly Lys Gly His Asp Gly Leu Tyr Gln Gly Leu Ser Thr Ala
                   85                  90                  95
   Thr Lys Asp Thr Tyr Asp Ala Leu His Met Gln Ala Leu Pro Pro Arg
               100                 105                 110
<210> 140
   <211> 1184
   <212> DNA
   <213> Artificial Sequence
<220>
   <223> Description of Artificial Sequence: Synthetic polynucleotide
<400> 140
   cgtgaggctc cggtgcccgt cagtgggcag agcgcacatc gcccacagtc cccgagaagt      60
   tggggggagg ggtcggcaat tgaaccggtg cctagagaag gtggcgcggg gtaaactggg     120
   aaagtgatgt cgtgtactgg ctccgccttt ttcccgaggg tgggggagaa ccgtatataa     180
   gtgcagtagt cgccgtgaac gttctttttc gcaacgggtt tgccgccaga acacaggtaa     240
   gtgccgtgtg tggttcccgc gggcctggcc tctttacggg ttatggccct tgcgtgcctt     300
   gaattacttc cacctggctg cagtacgtga ttcttgatcc cgagcttcgg gttggaagtg     360
   ggtgggagag ttcgaggcct tgcgcttaag gagccccttc gcctcgtgct tgagttgagg     420
   cctggcctgg gcgctggggc cgccgcgtgc gaatctggtg gcaccttcgc gcctgtctcg     480
   ctgctttcga taagtctcta gccatttaaa atttttgatg acctgctgcg acgctttttt     540
   tctggcaaga tagtcttgta aatgcgggcc aagatctgca cactggtatt tcggtttttg     600
   gggccgcggg cggcgacggg gcccgtgcgt cccagcgcac atgttcggcg aggcggggcc     660
   tgcgagcgcg gccaccgaga atcggacggg ggtagtctca agctggccgg cctgctctgg     720
   tgcctggcct cgcgccgccg tgtatcgccc cgccctgggc ggcaaggctg gcccggtcgg     780
   caccagttgc gtgagcggaa agatggccgc ttcccggccc tgctgcaggg agctcaaaat     840
   ggaggacgcg gcgctcggga gagcgggcgg gtgagtcacc cacacaaagg aaaagggcct     900
   ttccgtcctc agccgtcgct tcatgtgact ccacggagta ccgggcgccg tccaggcacc     960
   tcgattagtt ctcgagcttt tggagtacgt cgtctttagg ttggggggag gggttttatg    1020
   cgatggagtt tccccacact gagtgggtgg agactgaagt taggccagct tggcacttga    1080
   tgtaattctc cttggaattt gccctttttg agtttggatc ttggttcatt ctcaagcctc    1140
   agacagtggt tcaaagtttt tttcttccat ttcaggtgtc gtga                     1184
<210> 141
   <211> 108
   <212> PRT
   <213> Artificial Sequence
<220>
   <223> Description of Artificial Sequence: Synthetic polypeptide
<400> 141
   Val Gln Val Glu Thr Ile Ser Pro Gly Asp Gly Arg Thr Phe Pro Lys
   1               5                   10                  15
   Arg Gly Gln Thr Cys Val Val His Tyr Thr Gly Met Leu Glu Asp Gly
               20                  25                  30
   Lys Lys Phe Asp Ser Ser Arg Asp Arg Asn Lys Pro Phe Lys Phe Met
           35                  40                  45
   Leu Gly Lys Gln Glu Val Ile Arg Gly Trp Glu Glu Gly Val Ala Gln
       50                  55                  60
   Met Ser Val Gly Gln Arg Ala Lys Leu Thr Ile Ser Pro Asp Tyr Ala
   65                  70                  75                  80
   Tyr Gly Ala Thr Gly His Pro Gly Ile Ile Pro Pro His Ala Thr Leu
                   85                  90                  95
   Val Phe Asp Val Glu Leu Leu Lys Leu Glu Thr Ser
               100                 105
<210> 142
   <211> 99
   <212> PRT
   <213> Artificial Sequence
<220>
   <223> Description of Artificial Sequence: Synthetic polypeptide
<400> 142
   Met Ala Ser Arg Ile Leu Trp His Glu Met Trp His Glu Gly Leu Glu
   1 5 10 15
   Glu Ala Ser Arg Leu Tyr Phe Gly Glu Arg Asn Val Lys Gly Met Phe
   20 25 30
   Glu Val Leu Glu Pro Leu His Ala Met Met Glu Arg Gly Pro Gln Thr
   35 40 45
   Leu Lys Glu Thr Ser Phe Asn Gln Ala Tyr Gly Arg Asp Leu Met Glu
   50 55 60
   Ala Gln Glu Trp Cys Arg Lys Tyr Met Lys Ser Gly Asn Val Lys Asp
   65 70 75 80
   Leu Leu Gln Ala Trp Asp Leu Tyr Tyr His Val Phe Arg Arg Ile Ser
   85 90 95
   Lys Thr Ser
<210> 143
   <211> 66
   <212> DNA
   <213> Artificial Sequence
<220>
   <223> Description of Artificial Sequence: Synthetic oligonucleotide
<400> 143
   ggcagcggcg ccaccaactt cagcctgctg aagcaggccg gcgacgtgga ggaaaaccct 60
   ggcccc 66
<210> 144
   <211> 22
   <212> PRT
   <213> Artificial Sequence
<220>
   <223> Description of Artificial Sequence: Synthetic peptide
<400> 144
   Gly Ser Gly Ala Thr Asn Phe Ser Leu Leu Lys Gln Ala Gly Asp Val
   1 5 10 15
   Glu Glu Asn Pro Gly Pro
   20
<210> 145
   <211> 66
   <212> DNA
   <213> Artificial Sequence
<220>
   <223> Description of Artificial Sequence: Synthetic oligonucleotide
<400> 145
   gtgaagcaga ccctgaactt cgacctgctg aaactggccg gcgacgtgga gagcaatccc 60
   ggccct 66
<210> 146
   <211> 22
   <212> PRT
   <213> Artificial Sequence
<220>
   <223> Description of Artificial Sequence: Synthetic peptide
<400> 146
   Val Lys Gln Thr Leu Asn Phe Asp Leu Leu Lys Leu Ala Gly Asp Val
   1               5                   10                  15
   Glu Ser Asn Pro Gly Pro
               20
<210> 147
   <211> 394
   <212> PRT
   <213> Artificial Sequence
<220>
   <223> Description of Artificial Sequence: Synthetic polypeptide
<400> 147
   Met Ala Leu Pro Val Thr Ala Leu Leu Leu Pro Leu Ala Leu Leu Leu
   1               5                   10                  15
   His Ala Ala Arg Pro Pro Gly Trp Phe Leu Asp Ser Pro Asp Arg Pro
               20                  25                  30
   Trp Asn Pro Pro Thr Phe Ser Pro Ala Leu Leu Val Val Thr Glu Gly
           35                  40                  45
   Asp Asn Ala Thr Phe Thr Cys Ser Phe Ser Asn Thr Ser Glu Ser Phe
       50                  55                  60
   Val Leu Asn Trp Tyr Arg Met Ser Pro Ser Asn Gln Thr Asp Lys Leu
   65                  70                  75                  80
   Ala Ala Phe Pro Glu Asp Arg Ser Gln Pro Gly Gln Asp Cys Arg Phe
                   85                  90                  95
   Arg Val Thr Gln Leu Pro Asn Gly Arg Asp Phe His Met Ser Val Val
               100                 105                 110
   Arg Ala Arg Arg Asn Asp Ser Gly Thr Tyr Leu Cys Gly Ala Ile Ser
           115                 120                 125
   Leu Ala Pro Lys Ala Gln Ile Lys Glu Ser Leu Arg Ala Glu Leu Arg
       130                 135                 140
   Val Thr Glu Arg Arg Ala Glu Val Pro Thr Ala His Pro Ser Pro Ser
   145                 150                 155                 160
   Pro Arg Pro Ala Gly Gln Phe Gln Thr Leu Val Thr Thr Thr Pro Ala
                   165                 170                 175
   Pro Arg Pro Pro Thr Pro Ala Pro Thr Ile Ala Ser Gln Pro Leu Ser
               180                 185                 190
   Leu Arg Pro Glu Ala Cys Arg Pro Ala Ala Gly Gly Ala Val His Thr
           195                 200                 205
   Arg Gly Leu Asp Phe Ala Cys Asp Ile Tyr Ile Trp Ala Pro Leu Ala
       210                 215                 220
   Gly Thr Cys Gly Val Leu Leu Leu Ser Leu Val Ile Thr Leu Tyr Cys
   225                 230                 235                 240
   Lys Arg Gly Arg Lys Lys Leu Leu Tyr Ile Phe Lys Gln Pro Phe Met
                   245                 250                 255
   Arg Pro Val Gln Thr Thr Gln Glu Glu Asp Gly Cys Ser Cys Arg Phe
               260                 265                 270
   Pro Glu Glu Glu Glu Gly Gly Cys Glu Leu Arg Val Lys Phe Ser Arg
           275                 280                 285
   Ser Ala Asp Ala Pro Ala Tyr Lys Gln Gly Gln Asn Gln Leu Tyr Asn
       290                 295                 300
   Glu Leu Asn Leu Gly Arg Arg Glu Glu Tyr Asp Val Leu Asp Lys Arg
   305                 310                 315                 320
   Arg Gly Arg Asp Pro Glu Met Gly Gly Lys Pro Arg Arg Lys Asn Pro
                   325                 330                 335
   Gln Glu Gly Leu Tyr Asn Glu Leu Gln Lys Asp Lys Met Ala Glu Ala
               340                 345                 350
   Tyr Ser Glu Ile Gly Met Lys Gly Glu Arg Arg Arg Gly Lys Gly His
   355 360 365
   Asp Gly Leu Tyr Gln Gly Leu Ser Thr Ala Thr Lys Asp Thr Tyr Asp
   370 375 380
   Ala Leu His Met Gln Ala Leu Pro Pro Arg
   385 390
<210> 148
   <211> 373
   <212> PRT
   <213> Artificial Sequence
<220>
   <223> Description of Artificial Sequence: Synthetic polypeptide
<400> 148
   Pro Gly Trp Phe Leu Asp Ser Pro Asp Arg Pro Trp Asn Pro Pro Thr
   1 5 10 15
   Phe Ser Pro Ala Leu Leu Val Val Thr Glu Gly Asp Asn Ala Thr Phe
   20 25 30
   Thr Cys Ser Phe Ser Asn Thr Ser Glu Ser Phe Val Leu Asn Trp Tyr
   35 40 45
   Arg Met Ser Pro Ser Asn Gln Thr Asp Lys Leu Ala Ala Phe Pro Glu
   50 55 60
   Asp Arg Ser Gln Pro Gly Gln Asp Cys Arg Phe Arg Val Thr Gln Leu
   65 70 75 80
   Pro Asn Gly Arg Asp Phe His Met Ser Val Val Arg Ala Arg Arg Asn
   85 90 95
   Asp Ser Gly Thr Tyr Leu Cys Gly Ala Ile Ser Leu Ala Pro Lys Ala
   100 105 110
   Gln Ile Lys Glu Ser Leu Arg Ala Glu Leu Arg Val Thr Glu Arg Arg
   115 120 125
   Ala Glu Val Pro Thr Ala His Pro Ser Pro Ser Pro Arg Pro Ala Gly
   130 135 140
   Gln Phe Gln Thr Leu Val Thr Thr Thr Pro Ala Pro Arg Pro Pro Thr
   145 150 155 160
   Pro Ala Pro Thr Ile Ala Ser Gln Pro Leu Ser Leu Arg Pro Glu Ala
                   165                 170                 175
   Cys Arg Pro Ala Ala Gly Gly Ala Val His Thr Arg Gly Leu Asp Phe
               180                 185                 190
   Ala Cys Asp Ile Tyr Ile Trp Ala Pro Leu Ala Gly Thr Cys Gly Val
           195                 200                 205
   Leu Leu Leu Ser Leu Val Ile Thr Leu Tyr Cys Lys Arg Gly Arg Lys
       210                 215                 220
   Lys Leu Leu Tyr Ile Phe Lys Gln Pro Phe Met Arg Pro Val Gln Thr
   225                 230                 235                 240
   Thr Gln Glu Glu Asp Gly Cys Ser Cys Arg Phe Pro Glu Glu Glu Glu
                   245                 250                 255
   Gly Gly Cys Glu Leu Arg Val Lys Phe Ser Arg Ser Ala Asp Ala Pro
               260                 265                 270
   Ala Tyr Lys Gln Gly Gln Asn Gln Leu Tyr Asn Glu Leu Asn Leu Gly
           275                 280                 285
   Arg Arg Glu Glu Tyr Asp Val Leu Asp Lys Arg Arg Gly Arg Asp Pro
       290                 295                 300
   Glu Met Gly Gly Lys Pro Arg Arg Lys Asn Pro Gln Glu Gly Leu Tyr
   305                 310                 315                 320
   Asn Glu Leu Gln Lys Asp Lys Met Ala Glu Ala Tyr Ser Glu Ile Gly
                   325                 330                 335
   Met Lys Gly Glu Arg Arg Arg Gly Lys Gly His Asp Gly Leu Tyr Gln
               340                 345                 350
   Gly Leu Ser Thr Ala Thr Lys Asp Thr Tyr Asp Ala Leu His Met Gln
           355                 360                 365
   Ala Leu Pro Pro Arg
       370
<210> 149
   <211> 1182
   <212> DNA
   <213> Artificial Sequence
<220>
   <223> Description of Artificial Sequence: Synthetic polynucleotide
<400> 149
   atggccctcc ctgtcactgc cctgcttctc cccctcgcac tcctgctcca cgccgctaga      60
   ccacccggat ggtttctgga ctctccggat cgcccgtgga atcccccaac cttctcaccg     120
   gcactcttgg ttgtgactga gggcgataat gcgaccttca cgtgctcgtt ctccaacacc     180
   tccgaatcat tcgtgctgaa ctggtaccgc atgagcccgt caaaccagac cgacaagctc     240
   gccgcgtttc cggaagatcg gtcgcaaccg ggacaggatt gtcggttccg cgtgactcaa     300
   ctgccgaatg gcagagactt ccacatgagc gtggtccgcg ctaggcgaaa cgactccggg     360
   acctacctgt gcggagccat ctcgctggcg cctaaggccc aaatcaaaga gagcttgagg     420
   gccgaactga gagtgaccga gcgcagagct gaggtgccaa ctgcacatcc atccccatcg     480
   cctcggcctg cggggcagtt tcagaccctg gtcacgacca ctccggcgcc gcgcccaccg     540
   actccggccc caactatcgc gagccagccc ctgtcgctga ggccggaagc atgccgccct     600
   gccgccggag gtgctgtgca tacccgggga ttggacttcg catgcgacat ctacatttgg     660
   gctcctctcg ccggaacttg tggcgtgctc cttctgtccc tggtcatcac cctgtactgc     720
   aagcggggtc ggaaaaagct tctgtacatt ttcaagcagc ccttcatgag gcccgtgcaa     780
   accacccagg aggaggacgg ttgctcctgc cggttccccg aagaggaaga aggaggttgc     840
   gagctgcgcg tgaagttctc ccggagcgcc gacgcccccg cctataagca gggccagaac     900
   cagctgtaca acgaactgaa cctgggacgg cgggaagagt acgatgtgct ggacaagcgg     960
   cgcggccggg accccgaaat gggcgggaag cctagaagaa agaaccctca ggaaggcctg    1020
   tataacgagc tgcagaagga caagatggcc gaggcctact ccgaaattgg gatgaaggga    1080
   gagcggcgga ggggaaaggg gcacgacggc ctgtaccaag gactgtccac cgccaccaag    1140
   gacacatacg atgccctgca catgcaggcc cttccccctc gc                       1182
<210> 150
   <211> 40
   <212> PRT
   <213> Artificial Sequence
<220>
   <223> Description of Artificial Sequence: Synthetic polypeptide
<220>
   <221> misc_feature
   <222> (1)..(40)
   <223> This sequence may encompass 1-10 repeating "Gly Gly Gly Ser" units
<400> 150
   Gly Gly Gly Ser Gly Gly Gly Ser Gly Gly Gly Ser Gly Gly Gly Ser
   1               5                   10                  15
   Gly Gly Gly Ser Gly Gly Gly Ser Gly Gly Gly Ser Gly Gly Gly Ser
               20                  25                  30
   Gly Gly Gly Ser Gly Gly Gly Ser
           35                  40
<210> 151
   <211> 58
   <212> DNA
   <213> Artificial Sequence
<220>
   <223> Description of Artificial Sequence: Synthetic oligonucleotide
<220>
   <221> modified_base
   <222> (2)..(22)
   <223> a, c, t, g, unknown or other
<220>
   <221> modified_base
   <222> (32)..(52)
   <223> a, c, t, g, unknown or other
<220>
   <223> See specification as filed for detailed description of substitutions and preferred embodiments
<400> 151
   gnnnnnnnnn nnnnnnnnnn nnttcaagag annnnnnnnn nnnnnnnnnn nntttttt 58
<210> 152
   <211> 9
   <212> DNA
   <213> Artificial Sequence
<220>
   <223> Description of Artificial Sequence: Synthetic oligonucleotide
<400> 152
   ttcaagaga 9
<210> 153
   <211> 9810
   <212> DNA
   <213> Artificial Sequence
<220>
   <223> Description of Artificial Sequence: Synthetic polynucleotide
<400> 153
   gtgcacgagt gggttacatc gaactggatc tcaacagcgg taagatcctt gagagttttc 60
<210> 154
   <211> 9165
   <212> DNA
   <213> Artificial Sequence
<220>
   <223> Description of Artificial Sequence: Synthetic polynucleotide
<400> 154
   gtgcacgagt gggttacatc gaactggatc tcaacagcgg taagatcctt gagagttttc 60
   gccccgaaga acgttttcca atgatgagca cttttaaagt tctgctatgt ggcgcggtat 120
   tatcccgtat tgacgccggg caagagcaac tcggtcgccg catacactat tctcagaatg 180
   acttggttga gtactcacca gtcacagaaa agcatcttac ggatggcatg acagtaagag 240
   aattatgcag tgctgccata accatgagtg ataacactgc ggccaactta cttctgacaa 300
   cgatcggagg accgaaggag ctaaccgctt ttttgcacaa catgggggat catgtaactc 360
   gccttgatcg ttgggaaccg gagctgaatg aagccatacc aaacgacgag cgtgacacca 420
   cgatgcctgt agcaatggca acaacgttgc gcaaactatt aactggcgaa ctacttactc 480
   tagcttcccg gcaacaatta atagactgga tggaggcgga taaagttgca ggaccacttc 540
   tgcgctcggc ccttccggct ggctggttta ttgctgataa atctggagcc ggtgagcgtg 600
   ggtctcgcgg tatcattgca gcactggggc cagatggtaa gccctcccgt atcgtagtta 660
   tctacacgac ggggagtcag gcaactatgg atgaacgaaa tagacagatc gctgagatag 720
   gtgcctcact gattaagcat tggtaactgt cagaccaagt ttactcatat atactttaga 780
   ttgatttaaa acttcatttt taatttaaaa ggatctaggt gaagatcctt tttgataatc 840
   tcatgaccaa aatcccttaa cgtgagtttt cgttccactg agcgtcagac cccgtagaaa 900
   agatcaaagg atcttcttga gatccttttt ttctgcgcgt aatctgctgc ttgcaaacaa 960
   aaaaaccacc gctaccagcg gtggtttgtt tgccggatca agagctacca actctttttc 1020
   cgaaggtaac tggcttcagc agagcgcaga taccaaatac tgtccttcta gtgtagccgt 1080
   agttaggcca ccacttcaag aactctgtag caccgcctac atacctcgct ctgctaatcc 1140
   tgttaccagt ggctgctgcc agtggcgata agtcgtgtct taccgggttg gactcaagac 1200
   gatagttacc ggataaggcg cagcggtcgg gctgaacggg gggttcgtgc acacagccca 1260
   gcttggagcg aacgacctac accgaactga gatacctaca gcgtgagcta tgagaaagcg 1320
   ccacgcttcc cgaagggaga aaggcggaca ggtatccggt aagcggcagg gtcggaacag 1380
   gagagcgcac gagggagctt ccagggggaa acgcctggta tctttatagt cctgtcgggt 1440
   ttcgccacct ctgacttgag cgtcgatttt tgtgatgctc gtcagggggg cggagcctat 1500
   ggaaaaacgc cagcaacgcg gcctttttac ggttcctggc cttttgctgg ccttttgctc 1560
   acatgttctt tcctgcgtta tcccctgatt ctgtggataa ccgtattacc gcctttgagt 1620
   gagctgatac cgctcgccgc agccgaacga ccgagcgcag cgagtcagtg agcgaggaag 1680
   cggaagagcg cccaatacgc aaaccgcctc tccccgcgcg ttggccgatt cattaatgca 1740
   gctggcacga caggtttccc gactggaaag cgggcagtga gcgcaacgca attaatgtga 1800
   gttagctcac tcattaggca ccccaggctt tacactttat gcttccggct cgtatgttgt 1860
   gtggaattgt gagcggataa caatttcaca caggaaacag ctatgaccat gattacgcca 1920
   agcgcgcaat taaccctcac taaagggaac aaaagctgga gctgcaagct taatgtagtc 1980
   ttatgcaata ctcttgtagt cttgcaacat ggtaacgatg agttagcaac atgccttaca 2040
   aggagagaaa aagcaccgtg catgccgatt ggtggaagta aggtggtacg atcgtgcctt 2100
   attaggaagg caacagacgg gtctgacatg gattggacga accactgaat tgccgcattg 2160
   cagagatatt gtatttaagt gcctagctcg atacaataaa cgggtctctc tggttagacc 2220
   agatctgagc ctgggagctc tctggctaac tagggaaccc actgcttaag cctcaataaa 2280
   gcttgccttg agtgcttcaa gtagtgtgtg cccgtctgtt gtgtgactct ggtaactaga 2340
   gatccctcag acccttttag tcagtgtgga aaatctctag cagtggcgcc cgaacaggga 2400
   cctgaaagcg aaagggaaac cagagctctc tcgacgcagg actcggcttg ctgaagcgcg 2460
   cacggcaaga ggcgaggggc ggcgactggt gagtacgcca aaaattttga ctagcggagg 2520
   ctagaaggag agagatgggt gcgagagcgt cagtattaag cgggggagaa ttagatcgcg 2580
   atgggaaaaa attcggttaa ggccaggggg aaagaaaaaa tataaattaa aacatatagt 2640
   atgggcaagc agggagctag aacgattcgc agttaatcct ggcctgttag aaacatcaga 2700
   aggctgtaga caaatactgg gacagctaca accatccctt cagacaggat cagaagaact 2760
   tagatcatta tataatacag tagcaaccct ctattgtgtg catcaaagga tagagataaa 2820
   agacaccaag gaagctttag acaagataga ggaagagcaa aacaaaagta agaccaccgc 2880
   acagcaagcg gccgctgatc ttcagacctg gaggaggaga tatgagggac aattggagaa 2940
   gtgaattata taaatataaa gtagtaaaaa ttgaaccatt aggagtagca cccaccaagg 3000
   caaagagaag agtggtgcag agagaaaaaa gagcagtggg aataggagct ttgttccttg 3060
   ggttcttggg agcagcagga agcactatgg gcgcagcctc aatgacgctg acggtacagg 3120
   ccagacaatt attgtctggt atagtgcagc agcagaacaa tttgctgagg gctattgagg 3180
   cgcaacagca tctgttgcaa ctcacagtct ggggcatcaa gcagctccag gcaagaatcc 3240
   tggctgtgga aagataccta aaggatcaac agctcctggg gatttggggt tgctctggaa 3300
   aactcatttg caccactgct gtgccttgga atgctagttg gagtaataaa tctctggaac 3360
   agattggaat cacacgacct ggatggagtg ggacagagaa attaacaatt acacaagctt 3420
   aatacactcc ttaattgaag aatcgcaaaa ccagcaagaa aagaatgaac aagaattatt 3480
   ggaattagat aaatgggcaa gtttgtggaa ttggtttaac ataacaaatt ggctgtggta 3540
   tataaaatta ttcataatga tagtaggagg cttggtaggt ttaagaatag tttttgctgt 3600
   actttctata gtgaatagag ttaggcaggg atattcacca ttatcgtttc agacccacct 3660
   cccaaccccg aggggacccg acaggcccga aggaatagaa gaagaaggtg gagagagaga 3720
   cagagacaga tccattcgat tagtgaacgg atctcgacgg tatcgattag actgtagccc 3780
   aggaatatgg cagctagatt gtacacattt agaaggaaaa gttatcttgg tagcagttca 3840
   tgtagccagt ggatatatag aagcagaagt aattccagca gagacagggc aagaaacagc 3900
   atacttcctc ttaaaattag caggaagatg gccagtaaaa acagtacata cagacaatgg 3960
   cagcaatttc accagtacta cagttaaggc cgcctgttgg tgggcgggga tcaagcagga 4020
   atttggcatt ccctacaatc cccaaagtca aggagtaata gaatctatga ataaagaatt 4080
   aaagaaaatt ataggacagg taagagatca ggctgaacat cttaagacag cagtacaaat 4140
   ggcagtattc atccacaatt ttaaaagaaa aggggggatt ggggggtaca gtgcagggga 4200
   aagaatagta gacataatag caacagacat acaaactaaa gaattacaaa aacaaattac 4260
   aaaaattcaa aattttcggg tttattacag ggacagcaga gatccagttt ggctgcatac 4320
   gcgtcgtgag gctccggtgc ccgtcagtgg gcagagcgca catcgcccac agtccccgag 4380
   aagttggggg gaggggtcgg caattgaacc ggtgcctaga gaaggtggcg cggggtaaac 4440
   tgggaaagtg atgtcgtgta ctggctccgc ctttttcccg agggtggggg agaaccgtat 4500
   ataagtgcag tagtcgccgt gaacgttctt tttcgcaacg ggtttgccgc cagaacacag 4560
   gtaagtgccg tgtgtggttc ccgcgggcct ggcctcttta cgggttatgg cccttgcgtg 4620
   ccttgaatta cttccacctg gctgcagtac gtgattcttg atcccgagct tcgggttgga 4680
   agtgggtggg agagttcgag gccttgcgct taaggagccc cttcgcctcg tgcttgagtt 4740
   gaggcctggc ctgggcgctg gggccgccgc gtgcgaatct ggtggcacct tcgcgcctgt 4800
   ctcgctgctt tcgataagtc tctagccatt taaaattttt gatgacctgc tgcgacgctt 4860
   tttttctggc aagatagtct tgtaaatgcg ggccaagatc tgcacactgg tatttcggtt 4920
   tttggggccg cgggcggcga cggggcccgt gcgtcccagc gcacatgttc ggcgaggcgg 4980
   ggcctgcgag cgcggccacc gagaatcgga cgggggtagt ctcaagctgg ccggcctgct 5040
   ctggtgcctg gcctcgcgcc gccgtgtatc gccccgccct gggcggcaag gctggcccgg 5100
   tcggcaccag ttgcgtgagc ggaaagatgg ccgcttcccg gccctgctgc agggagctca 5160
   aaatggagga cgcggcgctc gggagagcgg gcgggtgagt cacccacaca aaggaaaagg 5220
   gcctttccgt cctcagccgt cgcttcatgt gactccacgg agtaccgggc gccgtccagg 5280
   cacctcgatt agttctcgtg cttttggagt acgtcgtctt taggttgggg ggaggggttt 5340
   tatgcgatgg agtttcccca cactgagtgg gtggagactg aagttaggcc agcttggcac 5400
   ttgatgtaat tctccttgga atttgccctt tttgagtttg gatcttggtt cattctcaag 5460
   cctcagacag tggttcaaag tttttttctt ccatttcagg tgtcgtgagc tagaatgggg 5520
   ggacttgaac cctgcagcag gctcctgctc ctgcctctcc tgctggctgt aagtggtctc 5580
   cgtcctgtcc aggcccaggc ccagagcgat tgcagttgct ctacggtgag cccgggcgtg 5640
   ctggcaggga tcgtgatggg agacctggtg ctgacagtgc tcattgccct ggccgtgtac 5700
   ttcctgggcc ggctggtccc tcgggggcga ggggctgcgg aggcagcgac ccggaaacag 5760
   cgtatcactg agaccgagtc gccttatcag gagctccagg gtcagaggtc ggatgtctac 5820
   agcgacctca acacacagag gccgtattac aaagtcgagg gcggcggaga gggcagagga 5880
   agtcttctaa catgcggtga cgtggaggag aatcccggcc ctaggatggc cttaccagtg 5940
   accgccttgc tcctgccgct ggccttgctg ctccacgccg ccaggccggg atcccaggta 6000
   caactgcagc agtctgggcc tgagctggag aagcctggcg cttcagtgaa gatatcctgc 6060
   aaggcttctg gttactcatt cactggctac accatgaact gggtgaagca gagccatgga 6120
   aagagccttg agtggattgg acttattact ccttacaatg gtgcttctag ctacaaccag 6180
   aagttcaggg gcaaggccac attaactgta gacaagtcat ccagcacagc ctacatggac 6240
   ctcctcagtc tgacatctga agactctgca gtctatttct gtgcaagggg gggttacgac 6300
   gggaggggtt ttgactactg gggccaaggg accacggtca ccgtctcctc aggtggaggc 6360
   ggttcaggcg gcggtggctc tagcggtggt ggatcggaca tcgagctcac tcagtctcca 6420
   gcaatcatgt ctgcatctcc aggggagaag gtcaccatga cctgcagtgc cagctcaagt 6480
   gtaagttaca tgcactggta ccagcagaag tcaggcacct cccccaaaag atggatttat 6540
   gacacatcca aactggcttc tggagtccca ggtcgcttca gtggcagtgg gtctggaaac 6600
   tcttactctc tcacaatcag cagcgtggag gctgaagatg atgcaactta ttactgccag 6660
   cagtggagta agcaccctct cacgtacggt gctgggacaa agttggaaat caaagctagc 6720
   ggtggcggag gttctggagg tgggggttcc tcacccactg aaccaagctc caaaaccggt 6780
   aaccccagac acctgcatgt tctgattggg acctcagtgg tcaaaatccc tttcaccatc 6840
   ctcctcttct ttctccttca tcgctggtgc tccaacaaaa aaaatgctgc tgtaatggac 6900
   caagagcctg cagggaacag aacagtgaac agcgaggatt ctgatgaaca agaccatcag 6960
   gaggtgtcat acgcataagt cgacaatcaa cctctggatt acaaaatttg tgaaagattg 7020
   actggtattc ttaactatgt tgctcctttt acgctatgtg gatacgctgc tttaatgcct 7080
   ttgtatcatg ctattgcttc ccgtatggct ttcattttct cctccttgta taaatcctgg 7140
   ttgctgtctc tttatgagga gttgtggccc gttgtcaggc aacgtggcgt ggtgtgcact 7200
   gtgtttgctg acgcaacccc cactggttgg ggcattgcca ccacctgtca gctcctttcc 7260
   gggactttcg ctttccccct ccctattgcc acggcggaac tcatcgccgc ctgccttgcc 7320
   cgctgctgga caggggctcg gctgttgggc actgacaatt ccgtggtgtt gtcggggaag 7380
   ctgacgtcct ttccatggct gctcgcctgt gttgccacct ggattctgcg cgggacgtcc 7440
   ttctgctacg tcccttcggc cctcaatcca gcggaccttc cttcccgcgg cctgctgccg 7500
   gctctgcggc ctcttccgcg tcttcgcctt cgccctcaga cgagtcggat ctccctttgg 7560
   gccgcctccc cgcctggaat tcgagctcgg tacctttaag accaatgact tacaaggcag 7620
   ctgtagatct tagccacttt ttaaaagaaa aggggggact ggaagggcta attcactccc 7680
   aacgaagaca agatctgctt tttgcttgta ctgggtctct ctggttagac cagatctgag 7740
   cctgggagct ctctggctaa ctagggaacc cactgcttaa gcctcaataa agcttgcctt 7800
   gagtgcttca agtagtgtgt gcccgtctgt tgtgtgactc tggtaactag agatccctca     7860
   gaccctttta gtcagtgtgg aaaatctcta gcagtagtag ttcatgtcat cttattattc     7920
   agtatttata acttgcaaag aaatgaatat cagagagtga gaggaacttg tttattgcag 7980
   cttataatgg ttacaaataa agcaatagca tcacaaattt cacaaataaa gcattttttt 8040
   cactgcattc tagttgtggt ttgtccaaac tcatcaatgt atcttatcat gtctggctct 8100
   agctatcccg cccctaactc cgcccagttc cgcccattct ccgccccatg gctgactaat 8160
   tttttttatt tatgcagagg ccgaggccgc ctcggcctct gagctattcc agaagtagtg 8220
   aggaggcttt tttggaggcc tacgcttttg cgtcgagacg tacccaattc gccctatagt 8280
   gagtcgtatt acgcgcgctc actggccgtc gttttacaac gtcgtgactg ggaaaaccct 8340
   ggcgttaccc aacttaatcg ccttgcagca catccccctt tcgccagctg gcgtaatagc 8400
   gaagaggccc gcaccgatcg cccttcccaa cagttgcgca gcctgaatgg cgaatggcgc 8460
   gacgcgccct gtagcggcgc attaagcgcg gcgggtgtgg tggttacgcg cagcgtgacc 8520
   gctacacttg ccagcgccct agcgcccgct cctttcgctt tcttcccttc ctttctcgcc 8580
   acgttcgccg gctttccccg tcaagctcta aatcgggggc tccctttagg gttccgattt 8640
   agtgctttac ggcacctcga ccccaaaaaa cttgattagg gtgatggttc acgtagtggg 8700
   ccatcgccct gatagacggt ttttcgccct ttgacgttgg agtccacgtt ctttaatagt 8760
   ggactcttgt tccaaactgg aacaacactc aaccctatct cggtctattc ttttgattta 8820
   taagggattt tgccgatttc ggcctattgg ttaaaaaatg agctgattta acaaaaattt 8880
   aacgcgaatt ttaacaaaat attaacgttt acaatttccc aggtggcact tttcggggaa 8940
   atgtgcgcgg aacccctatt tgtttatttt tctaaataca ttcaaatatg tatccgctca 9000
   tgagacaata accctgataa atgcttcaat aatattgaaa aaggaagagt atgagtattc 9060
   aacatttccg tgtcgccctt attccctttt ttgcggcatt ttgccttcct gtttttgctc 9120
   acccagaaac gctggtgaaa gtaaaagatg ctgaagatca gttgg 9165
<210> 155
   <211> 9915
   <212> DNA
   <213> Artificial Sequence
<220>
   <223> Description of Artificial Sequence: Synthetic polynucleotide
<400> 155
   gtgcacgagt gggttacatc gaactggatc tcaacagcgg taagatcctt gagagttttc 60
   gccccgaaga acgttttcca atgatgagca cttttaaagt tctgctatgt ggcgcggtat 120
   tatcccgtat tgacgccggg caagagcaac tcggtcgccg catacactat tctcagaatg 180
   acttggttga gtactcacca gtcacagaaa agcatcttac ggatggcatg acagtaagag 240
   aattatgcag tgctgccata accatgagtg ataacactgc ggccaactta cttctgacaa 300
   cgatcggagg accgaaggag ctaaccgctt ttttgcacaa catgggggat catgtaactc 360
   gccttgatcg ttgggaaccg gagctgaatg aagccatacc aaacgacgag cgtgacacca 420
   cgatgcctgt agcaatggca acaacgttgc gcaaactatt aactggcgaa ctacttactc 480
   tagcttcccg gcaacaatta atagactgga tggaggcgga taaagttgca ggaccacttc 540
   tgcgctcggc ccttccggct ggctggttta ttgctgataa atctggagcc ggtgagcgtg 600
   ggtctcgcgg tatcattgca gcactggggc cagatggtaa gccctcccgt atcgtagtta 660
   tctacacgac ggggagtcag gcaactatgg atgaacgaaa tagacagatc gctgagatag 720
   gtgcctcact gattaagcat tggtaactgt cagaccaagt ttactcatat atactttaga 780
   ttgatttaaa acttcatttt taatttaaaa ggatctaggt gaagatcctt tttgataatc 840
   tcatgaccaa aatcccttaa cgtgagtttt cgttccactg agcgtcagac cccgtagaaa 900
   agatcaaagg atcttcttga gatccttttt ttctgcgcgt aatctgctgc ttgcaaacaa 960
   aaaaaccacc gctaccagcg gtggtttgtt tgccggatca agagctacca actctttttc 1020
   cgaaggtaac tggcttcagc agagcgcaga taccaaatac tgtccttcta gtgtagccgt 1080
   agttaggcca ccacttcaag aactctgtag caccgcctac atacctcgct ctgctaatcc 1140
   tgttaccagt ggctgctgcc agtggcgata agtcgtgtct taccgggttg gactcaagac 1200
   gatagttacc ggataaggcg cagcggtcgg gctgaacggg gggttcgtgc acacagccca 1260
   gcttggagcg aacgacctac accgaactga gatacctaca gcgtgagcta tgagaaagcg 1320
   ccacgcttcc cgaagggaga aaggcggaca ggtatccggt aagcggcagg gtcggaacag 1380
   gagagcgcac gagggagctt ccagggggaa acgcctggta tctttatagt cctgtcgggt 1440
   ttcgccacct ctgacttgag cgtcgatttt tgtgatgctc gtcagggggg cggagcctat 1500
   ggaaaaacgc cagcaacgcg gcctttttac ggttcctggc cttttgctgg ccttttgctc 1560
   acatgttctt tcctgcgtta tcccctgatt ctgtggataa ccgtattacc gcctttgagt 1620
   gagctgatac cgctcgccgc agccgaacga ccgagcgcag cgagtcagtg agcgaggaag 1680
   cggaagagcg cccaatacgc aaaccgcctc tccccgcgcg ttggccgatt cattaatgca 1740
   gctggcacga caggtttccc gactggaaag cgggcagtga gcgcaacgca attaatgtga 1800
   gttagctcac tcattaggca ccccaggctt tacactttat gcttccggct cgtatgttgt 1860
   gtggaattgt gagcggataa caatttcaca caggaaacag ctatgaccat gattacgcca 1920
   agcgcgcaat taaccctcac taaagggaac aaaagctgga gctgcaagct taatgtagtc 1980
   ttatgcaata ctcttgtagt cttgcaacat ggtaacgatg agttagcaac atgccttaca 2040
   aggagagaaa aagcaccgtg catgccgatt ggtggaagta aggtggtacg atcgtgcctt 2100
   attaggaagg caacagacgg gtctgacatg gattggacga accactgaat tgccgcattg 2160
   cagagatatt gtatttaagt gcctagctcg atacaataaa cgggtctctc tggttagacc 2220
   agatctgagc ctgggagctc tctggctaac tagggaaccc actgcttaag cctcaataaa 2280
   gcttgccttg agtgcttcaa gtagtgtgtg cccgtctgtt gtgtgactct ggtaactaga 2340
   gatccctcag acccttttag tcagtgtgga aaatctctag cagtggcgcc cgaacaggga 2400
   cctgaaagcg aaagggaaac cagagctctc tcgacgcagg actcggcttg ctgaagcgcg 2460
   cacggcaaga ggcgaggggc ggcgactggt gagtacgcca aaaattttga ctagcggagg 2520
   ctagaaggag agagatgggt gcgagagcgt cagtattaag cgggggagaa ttagatcgcg 2580
   atgggaaaaa attcggttaa ggccaggggg aaagaaaaaa tataaattaa aacatatagt 2640
   atgggcaagc agggagctag aacgattcgc agttaatcct ggcctgttag aaacatcaga 2700
   aggctgtaga caaatactgg gacagctaca accatccctt cagacaggat cagaagaact 2760
   tagatcatta tataatacag tagcaaccct ctattgtgtg catcaaagga tagagataaa 2820
   agacaccaag gaagctttag acaagataga ggaagagcaa aacaaaagta agaccaccgc 2880
   acagcaagcg gccgctgatc ttcagacctg gaggaggaga tatgagggac aattggagaa 2940
   gtgaattata taaatataaa gtagtaaaaa ttgaaccatt aggagtagca cccaccaagg 3000
   caaagagaag agtggtgcag agagaaaaaa gagcagtggg aataggagct ttgttccttg 3060
   ggttcttggg agcagcagga agcactatgg gcgcagcctc aatgacgctg acggtacagg 3120
   ccagacaatt attgtctggt atagtgcagc agcagaacaa tttgctgagg gctattgagg 3180
   cgcaacagca tctgttgcaa ctcacagtct ggggcatcaa gcagctccag gcaagaatcc 3240
   tggctgtgga aagataccta aaggatcaac agctcctggg gatttggggt tgctctggaa 3300
   aactcatttg caccactgct gtgccttgga atgctagttg gagtaataaa tctctggaac 3360
   agattggaat cacacgacct ggatggagtg ggacagagaa attaacaatt acacaagctt 3420
   aatacactcc ttaattgaag aatcgcaaaa ccagcaagaa aagaatgaac aagaattatt 3480
   ggaattagat aaatgggcaa gtttgtggaa ttggtttaac ataacaaatt ggctgtggta 3540
   tataaaatta ttcataatga tagtaggagg cttggtaggt ttaagaatag tttttgctgt 3600
   actttctata gtgaatagag ttaggcaggg atattcacca ttatcgtttc agacccacct 3660
   cccaaccccg aggggacccg acaggcccga aggaatagaa gaagaaggtg gagagagaga 3720
   cagagacaga tccattcgat tagtgaacgg atctcgacgg tatcgattag actgtagccc 3780
   aggaatatgg cagctagatt gtacacattt agaaggaaaa gttatcttgg tagcagttca 3840
   tgtagccagt ggatatatag aagcagaagt aattccagca gagacagggc aagaaacagc 3900
   atacttcctc ttaaaattag caggaagatg gccagtaaaa acagtacata cagacaatgg 3960
   cagcaatttc accagtacta cagttaaggc cgcctgttgg tgggcgggga tcaagcagga 4020
   atttggcatt ccctacaatc cccaaagtca aggagtaata gaatctatga ataaagaatt 4080
   aaagaaaatt ataggacagg taagagatca ggctgaacat cttaagacag cagtacaaat 4140
   ggcagtattc atccacaatt ttaaaagaaa aggggggatt ggggggtaca gtgcagggga 4200
   aagaatagta gacataatag caacagacat acaaactaaa gaattacaaa aacaaattac 4260
   aaaaattcaa aattttcggg tttattacag ggacagcaga gatccagttt ggctgcatac 4320
   gcgtcgtgag gctccggtgc ccgtcagtgg gcagagcgca catcgcccac agtccccgag 4380
   aagttggggg gaggggtcgg caattgaacc ggtgcctaga gaaggtggcg cggggtaaac 4440
   tgggaaagtg atgtcgtgta ctggctccgc ctttttcccg agggtggggg agaaccgtat 4500
   ataagtgcag tagtcgccgt gaacgttctt tttcgcaacg ggtttgccgc cagaacacag 4560
   gtaagtgccg tgtgtggttc ccgcgggcct ggcctcttta cgggttatgg cccttgcgtg 4620
   ccttgaatta cttccacctg gctgcagtac gtgattcttg atcccgagct tcgggttgga 4680
   agtgggtggg agagttcgag gccttgcgct taaggagccc cttcgcctcg tgcttgagtt 4740
   gaggcctggc ctgggcgctg gggccgccgc gtgcgaatct ggtggcacct tcgcgcctgt 4800
   ctcgctgctt tcgataagtc tctagccatt taaaattttt gatgacctgc tgcgacgctt 4860
   tttttctggc aagatagtct tgtaaatgcg ggccaagatc tgcacactgg tatttcggtt 4920
   tttggggccg cgggcggcga cggggcccgt gcgtcccagc gcacatgttc ggcgaggcgg 4980
   ggcctgcgag cgcggccacc gagaatcgga cgggggtagt ctcaagctgg ccggcctgct 5040
   ctggtgcctg gcctcgcgcc gccgtgtatc gccccgccct gggcggcaag gctggcccgg 5100
   tcggcaccag ttgcgtgagc ggaaagatgg ccgcttcccg gccctgctgc agggagctca 5160
   aaatggagga cgcggcgctc gggagagcgg gcgggtgagt cacccacaca aaggaaaagg 5220
   gcctttccgt cctcagccgt cgcttcatgt gactccacgg agtaccgggc gccgtccagg 5280
   cacctcgatt agttctcgtg cttttggagt acgtcgtctt taggttgggg ggaggggttt 5340
   tatgcgatgg agtttcccca cactgagtgg gtggagactg aagttaggcc agcttggcac 5400
   ttgatgtaat tctccttgga atttgccctt tttgagtttg gatcttggtt cattctcaag 5460
   cctcagacag tggttcaaag tttttttctt ccatttcagg tgtcgtgagc tagaatgggg 5520
   ggacttgaac cctgcagcag gctcctgctc ctgcctctcc tgctggctgt aagtggtctc 5580
   cgtcctgtcc aggcccaggc ccagagcgat tgcagttgct ctacggtgag cccgggcgtg 5640
   ctggcaggga tcgtgatggg agacctggtg ctgacagtgc tcattgccct ggccgtgtac 5700
   ttcctgggcc ggctggtccc tcgggggcga ggggctgcgg aggcagcgac ccggaaacag 5760
   cgtatcactg agaccgagtc gccttatcag gagctccagg gtcagaggtc ggatgtctac 5820
   agcgacctca acacacagag gccgtattac aaagtcgagg gcggcggaga gggcagagga 5880
   agtcttctaa catgcggtga cgtggaggag aatcccggcc ctaggatggc cttaccagtg 5940
   accgccttgc tcctgccgct ggccttgctg ctccacgccg ccaggccggg atcccaggta 6000
   caactgcagc agtctgggcc tgagctggag aagcctggcg cttcagtgaa gatatcctgc 6060
   aaggcttctg gttactcatt cactggctac accatgaact gggtgaagca gagccatgga 6120
   aagagccttg agtggattgg acttattact ccttacaatg gtgcttctag ctacaaccag 6180
   aagttcaggg gcaaggccac attaactgta gacaagtcat ccagcacagc ctacatggac 6240
   ctcctcagtc tgacatctga agactctgca gtctatttct gtgcaagggg gggttacgac 6300
   gggaggggtt ttgactactg gggccaaggg accacggtca ccgtctcctc aggtggaggc 6360
   ggttcaggcg gcggtggctc tagcggtggt ggatcggaca tcgagctcac tcagtctcca 6420
   gcaatcatgt ctgcatctcc aggggagaag gtcaccatga cctgcagtgc cagctcaagt 6480
   gtaagttaca tgcactggta ccagcagaag tcaggcacct cccccaaaag atggatttat 6540
   gacacatcca aactggcttc tggagtccca ggtcgcttca gtggcagtgg gtctggaaac 6600
   tcttactctc tcacaatcag cagcgtggag gctgaagatg atgcaactta ttactgccag 6660
   cagtggagta agcaccctct cacgtacggt gctgggacaa agttggaaat caaagctagc 6720
   ggtggcggag gttctggagg tgggggttcc cagggggcct ggccacatga gggagtccac 6780
   agaaaacctt ccctcctggc ccacccaggt cccctggtga aatcagaaga gacagtcatc 6840
   ctgcaatgtt ggtcagatgt caggtttcag cacttccttc tgcacagaga agggaagttt 6900
   aaggacactt tgcacctcat tggagagcac catgatgggg tctccaaggc caacttctcc 6960
   atcggtccca tgatgcaaga ccttgcaggg acctacagat gctacggttc tgttactcac 7020
   tccccctatc agttgtcagc tcccagtgac cctctggaca tcgtcatcac aggtctatat 7080
   gagaaacctt ctctctcagc ccagccgggc cccacggttc tggcaggaga gagcgtgacc 7140
   ttgtcctgca gctcccggag ctcctatgac atgtaccatc tatccaggga gggggaggcc 7200
   catgaacgta ggttctctgc agggcccaag gtcaacggaa cattccaggc cgactttcct 7260
   ctgggccctg ccacccacgg aggaacctac agatgcttcg gctctttccg tgactctcca 7320
   tacgagtggt caaactcgag tgacccactg cttgtttctg tcacaggaaa cccttcaaat 7380
   agttggcttt cacccactga accaagctcc gaaaccggta accccagaca cctgcatgtt 7440
   ctgattggga cctcagtggt catcatcctc ttcatcctcc tcctcttctt tctccttcat 7500
   cgctggtgct gcaacaaaaa aaatgctgtt gtaatggacc aagagcctgc agggaacaga 7560
   acagtgaaca gggaggactc tgatgaacaa gaccctcagg aggtgacata tgcacagttg 7620
   aatcactgcg ttttcacaca gagaaaaatc actcaccctt ctcagaggcc caagacaccc 7680
   ccaacagata tcatcgtgta cacggaactt ccaaatgctg agccctgagt cgacaatcaa 7740
   cctctggatt acaaaatttg tgaaagattg actggtattc ttaactatgt tgctcctttt 7800
   acgctatgtg gatacgctgc tttaatgcct ttgtatcatg ctattgcttc ccgtatggct 7860
   ttcattttct cctccttgta taaatcctgg ttgctgtctc tttatgagga gttgtggccc 7920
   gttgtcaggc aacgtggcgt ggtgtgcact gtgtttgctg acgcaacccc cactggttgg 7980
   ggcattgcca ccacctgtca gctcctttcc gggactttcg ctttccccct ccctattgcc 8040
   acggcggaac tcatcgccgc ctgccttgcc cgctgctgga caggggctcg gctgttgggc 8100
   actgacaatt ccgtggtgtt gtcggggaag ctgacgtcct ttccatggct gctcgcctgt 8160
   gttgccacct ggattctgcg cgggacgtcc ttctgctacg tcccttcggc cctcaatcca 8220
   gcggaccttc cttcccgcgg cctgctgccg gctctgcggc ctcttccgcg tcttcgcctt 8280
   cgccctcaga cgagtcggat ctccctttgg gccgcctccc cgcctggaat tcgagctcgg 8340
   tacctttaag accaatgact tacaaggcag ctgtagatct tagccacttt ttaaaagaaa 8400
   aggggggact ggaagggcta attcactccc aacgaagaca agatctgctt tttgcttgta 8460
   ctgggtctct ctggttagac cagatctgag cctgggagct ctctggctaa ctagggaacc 8520
   cactgcttaa gcctcaataa agcttgcctt gagtgcttca agtagtgtgt gcccgtctgt 8580
   tgtgtgactc tggtaactag agatccctca gaccctttta gtcagtgtgg aaaatctcta 8640
   gcagtagtag ttcatgtcat cttattattc agtatttata acttgcaaag aaatgaatat 8700
   cagagagtga gaggaacttg tttattgcag cttataatgg ttacaaataa agcaatagca 8760
   tcacaaattt cacaaataaa gcattttttt cactgcattc tagttgtggt ttgtccaaac 8820
   tcatcaatgt atcttatcat gtctggctct agctatcccg cccctaactc cgcccagttc 8880
   cgcccattct ccgccccatg gctgactaat tttttttatt tatgcagagg ccgaggccgc 8940
   ctcggcctct gagctattcc agaagtagtg aggaggcttt tttggaggcc tacgcttttg 9000
   cgtcgagacg tacccaattc gccctatagt gagtcgtatt acgcgcgctc actggccgtc 9060
   gttttacaac gtcgtgactg ggaaaaccct ggcgttaccc aacttaatcg ccttgcagca 9120
   catccccctt tcgccagctg gcgtaatagc gaagaggccc gcaccgatcg cccttcccaa 9180
   cagttgcgca gcctgaatgg cgaatggcgc gacgcgccct gtagcggcgc attaagcgcg 9240
   gcgggtgtgg tggttacgcg cagcgtgacc gctacacttg ccagcgccct agcgcccgct 9300
   cctttcgctt tcttcccttc ctttctcgcc acgttcgccg gctttccccg tcaagctcta 9360
   aatcgggggc tccctttagg gttccgattt agtgctttac ggcacctcga ccccaaaaaa 9420
   cttgattagg gtgatggttc acgtagtggg ccatcgccct gatagacggt ttttcgccct 9480
   ttgacgttgg agtccacgtt ctttaatagt ggactcttgt tccaaactgg aacaacactc 9540
   aaccctatct cggtctattc ttttgattta taagggattt tgccgatttc ggcctattgg 9600
   ttaaaaaatg agctgattta acaaaaattt aacgcgaatt ttaacaaaat attaacgttt 9660
   acaatttccc aggtggcact tttcggggaa atgtgcgcgg aacccctatt tgtttatttt 9720
   tctaaataca ttcaaatatg tatccgctca tgagacaata accctgataa atgcttcaat 9780
   aatattgaaa aaggaagagt atgagtattc aacatttccg tgtcgccctt attccctttt 9840
   ttgcggcatt ttgccttcct gtttttgctc acccagaaac gctggtgaaa gtaaaagatg 9900
   ctgaagatca gttgg 9915
<210> 156
   <211> 9816
   <212> DNA
   <213> Artificial Sequence
<220>
   <223> Description of Artificial Sequence: Synthetic polynucleotide
<400> 156
   gtgcacgagt gggttacatc gaactggatc tcaacagcgg taagatcctt gagagttttc 60
   gccccgaaga acgttttcca atgatgagca cttttaaagt tctgctatgt ggcgcggtat 120
   tatcccgtat tgacgccggg caagagcaac tcggtcgccg catacactat tctcagaatg 180
   acttggttga gtactcacca gtcacagaaa agcatcttac ggatggcatg acagtaagag 240
   aattatgcag tgctgccata accatgagtg ataacactgc ggccaactta cttctgacaa 300
   cgatcggagg accgaaggag ctaaccgctt ttttgcacaa catgggggat catgtaactc 360
   gccttgatcg ttgggaaccg gagctgaatg aagccatacc aaacgacgag cgtgacacca 420
   cgatgcctgt agcaatggca acaacgttgc gcaaactatt aactggcgaa ctacttactc 480
   tagcttcccg gcaacaatta atagactgga tggaggcgga taaagttgca ggaccacttc 540
   tgcgctcggc ccttccggct ggctggttta ttgctgataa atctggagcc ggtgagcgtg 600
   ggtctcgcgg tatcattgca gcactggggc cagatggtaa gccctcccgt atcgtagtta 660
   tctacacgac ggggagtcag gcaactatgg atgaacgaaa tagacagatc gctgagatag 720
   gtgcctcact gattaagcat tggtaactgt cagaccaagt ttactcatat atactttaga 780
   ttgatttaaa acttcatttt taatttaaaa ggatctaggt gaagatcctt tttgataatc 840
   tcatgaccaa aatcccttaa cgtgagtttt cgttccactg agcgtcagac cccgtagaaa 900
   agatcaaagg atcttcttga gatccttttt ttctgcgcgt aatctgctgc ttgcaaacaa 960
   aaaaaccacc gctaccagcg gtggtttgtt tgccggatca agagctacca actctttttc 1020
   cgaaggtaac tggcttcagc agagcgcaga taccaaatac tgtccttcta gtgtagccgt 1080
   agttaggcca ccacttcaag aactctgtag caccgcctac atacctcgct ctgctaatcc 1140
   tgttaccagt ggctgctgcc agtggcgata agtcgtgtct taccgggttg gactcaagac 1200
   gatagttacc ggataaggcg cagcggtcgg gctgaacggg gggttcgtgc acacagccca 1260
   gcttggagcg aacgacctac accgaactga gatacctaca gcgtgagcta tgagaaagcg 1320
   ccacgcttcc cgaagggaga aaggcggaca ggtatccggt aagcggcagg gtcggaacag 1380
   gagagcgcac gagggagctt ccagggggaa acgcctggta tctttatagt cctgtcgggt 1440
   ttcgccacct ctgacttgag cgtcgatttt tgtgatgctc gtcagggggg cggagcctat 1500
   ggaaaaacgc cagcaacgcg gcctttttac ggttcctggc cttttgctgg ccttttgctc 1560
   acatgttctt tcctgcgtta tcccctgatt ctgtggataa ccgtattacc gcctttgagt 1620
   gagctgatac cgctcgccgc agccgaacga ccgagcgcag cgagtcagtg agcgaggaag 1680
   cggaagagcg cccaatacgc aaaccgcctc tccccgcgcg ttggccgatt cattaatgca 1740
   gctggcacga caggtttccc gactggaaag cgggcagtga gcgcaacgca attaatgtga 1800
   gttagctcac tcattaggca ccccaggctt tacactttat gcttccggct cgtatgttgt 1860
   gtggaattgt gagcggataa caatttcaca caggaaacag ctatgaccat gattacgcca 1920
   agcgcgcaat taaccctcac taaagggaac aaaagctgga gctgcaagct taatgtagtc 1980
   ttatgcaata ctcttgtagt cttgcaacat ggtaacgatg agttagcaac atgccttaca 2040
   aggagagaaa aagcaccgtg catgccgatt ggtggaagta aggtggtacg atcgtgcctt 2100
   attaggaagg caacagacgg gtctgacatg gattggacga accactgaat tgccgcattg 2160
   cagagatatt gtatttaagt gcctagctcg atacaataaa cgggtctctc tggttagacc 2220
   agatctgagc ctgggagctc tctggctaac tagggaaccc actgcttaag cctcaataaa 2280
   gcttgccttg agtgcttcaa gtagtgtgtg cccgtctgtt gtgtgactct ggtaactaga 2340
   gatccctcag acccttttag tcagtgtgga aaatctctag cagtggcgcc cgaacaggga 2400
   cctgaaagcg aaagggaaac cagagctctc tcgacgcagg actcggcttg ctgaagcgcg 2460
   cacggcaaga ggcgaggggc ggcgactggt gagtacgcca aaaattttga ctagcggagg 2520
   ctagaaggag agagatgggt gcgagagcgt cagtattaag cgggggagaa ttagatcgcg 2580
   atgggaaaaa attcggttaa ggccaggggg aaagaaaaaa tataaattaa aacatatagt 2640
   atgggcaagc agggagctag aacgattcgc agttaatcct ggcctgttag aaacatcaga 2700
   aggctgtaga caaatactgg gacagctaca accatccctt cagacaggat cagaagaact 2760
   tagatcatta tataatacag tagcaaccct ctattgtgtg catcaaagga tagagataaa 2820
   agacaccaag gaagctttag acaagataga ggaagagcaa aacaaaagta agaccaccgc 2880
   acagcaagcg gccgctgatc ttcagacctg gaggaggaga tatgagggac aattggagaa 2940
   gtgaattata taaatataaa gtagtaaaaa ttgaaccatt aggagtagca cccaccaagg 3000
   caaagagaag agtggtgcag agagaaaaaa gagcagtggg aataggagct ttgttccttg 3060
   ggttcttggg agcagcagga agcactatgg gcgcagcctc aatgacgctg acggtacagg 3120
   ccagacaatt attgtctggt atagtgcagc agcagaacaa tttgctgagg gctattgagg 3180
   cgcaacagca tctgttgcaa ctcacagtct ggggcatcaa gcagctccag gcaagaatcc 3240
   tggctgtgga aagataccta aaggatcaac agctcctggg gatttggggt tgctctggaa 3300
   aactcatttg caccactgct gtgccttgga atgctagttg gagtaataaa tctctggaac 3360
   agattggaat cacacgacct ggatggagtg ggacagagaa attaacaatt acacaagctt 3420
   aatacactcc ttaattgaag aatcgcaaaa ccagcaagaa aagaatgaac aagaattatt 3480
   ggaattagat aaatgggcaa gtttgtggaa ttggtttaac ataacaaatt ggctgtggta 3540
   tataaaatta ttcataatga tagtaggagg cttggtaggt ttaagaatag tttttgctgt 3600
   actttctata gtgaatagag ttaggcaggg atattcacca ttatcgtttc agacccacct 3660
   cccaaccccg aggggacccg acaggcccga aggaatagaa gaagaaggtg gagagagaga 3720
   cagagacaga tccattcgat tagtgaacgg atctcgacgg tatcgattag actgtagccc 3780
   aggaatatgg cagctagatt gtacacattt agaaggaaaa gttatcttgg tagcagttca 3840
   tgtagccagt ggatatatag aagcagaagt aattccagca gagacagggc aagaaacagc 3900
   atacttcctc ttaaaattag caggaagatg gccagtaaaa acagtacata cagacaatgg 3960
   cagcaatttc accagtacta cagttaaggc cgcctgttgg tgggcgggga tcaagcagga 4020
   atttggcatt ccctacaatc cccaaagtca aggagtaata gaatctatga ataaagaatt 4080
   aaagaaaatt ataggacagg taagagatca ggctgaacat cttaagacag cagtacaaat 4140
   ggcagtattc atccacaatt ttaaaagaaa aggggggatt ggggggtaca gtgcagggga 4200
   aagaatagta gacataatag caacagacat acaaactaaa gaattacaaa aacaaattac 4260
   aaaaattcaa aattttcggg tttattacag ggacagcaga gatccagttt ggctgcatac 4320
   gcgtcgtgag gctccggtgc ccgtcagtgg gcagagcgca catcgcccac agtccccgag 4380
   aagttggggg gaggggtcgg caattgaacc ggtgcctaga gaaggtggcg cggggtaaac 4440
   tgggaaagtg atgtcgtgta ctggctccgc ctttttcccg agggtggggg agaaccgtat 4500
   ataagtgcag tagtcgccgt gaacgttctt tttcgcaacg ggtttgccgc cagaacacag 4560
   gtaagtgccg tgtgtggttc ccgcgggcct ggcctcttta cgggttatgg cccttgcgtg 4620
   ccttgaatta cttccacctg gctgcagtac gtgattcttg atcccgagct tcgggttgga 4680
   agtgggtggg agagttcgag gccttgcgct taaggagccc cttcgcctcg tgcttgagtt 4740
   gaggcctggc ctgggcgctg gggccgccgc gtgcgaatct ggtggcacct tcgcgcctgt 4800
   ctcgctgctt tcgataagtc tctagccatt taaaattttt gatgacctgc tgcgacgctt 4860
   tttttctggc aagatagtct tgtaaatgcg ggccaagatc tgcacactgg tatttcggtt 4920
   tttggggccg cgggcggcga cggggcccgt gcgtcccagc gcacatgttc ggcgaggcgg 4980
   ggcctgcgag cgcggccacc gagaatcgga cgggggtagt ctcaagctgg ccggcctgct 5040
   ctggtgcctg gcctcgcgcc gccgtgtatc gccccgccct gggcggcaag gctggcccgg 5100
   tcggcaccag ttgcgtgagc ggaaagatgg ccgcttcccg gccctgctgc agggagctca 5160
   aaatggagga cgcggcgctc gggagagcgg gcgggtgagt cacccacaca aaggaaaagg 5220
   gcctttccgt cctcagccgt cgcttcatgt gactccacgg agtaccgggc gccgtccagg 5280
   cacctcgatt agttctcgtg cttttggagt acgtcgtctt taggttgggg ggaggggttt 5340
   tatgcgatgg agtttcccca cactgagtgg gtggagactg aagttaggcc agcttggcac 5400
   ttgatgtaat tctccttgga atttgccctt tttgagtttg gatcttggtt cattctcaag 5460
   cctcagacag tggttcaaag tttttttctt ccatttcagg tgtcgtgagc tagaatgggg 5520
   ggacttgaac cctgcagcag gctcctgctc ctgcctctcc tgctggctgt aagtggtctc 5580
   cgtcctgtcc aggcccaggc ccagagcgat tgcagttgct ctacggtgag cccgggcgtg 5640
   ctggcaggga tcgtgatggg agacctggtg ctgacagtgc tcattgccct ggccgtgtac 5700
   ttcctgggcc ggctggtccc tcgggggcga ggggctgcgg aggcagcgac ccggaaacag 5760
   cgtatcactg agaccgagtc gccttatcag gagctccagg gtcagaggtc ggatgtctac 5820
   agcgacctca acacacagag gccgtattac aaagtcgagg gcggcggaga gggcagagga 5880
   agtcttctaa catgcggtga cgtggaggag aatcccggcc ctaggatggc cttaccagtg 5940
   accgccttgc tcctgccgct ggccttgctg ctccacgccg ccaggccggg atccgacatc 6000
   cagatgacac agactacatc ctccctgtct gcctctctgg gagacagagt caccatcagt 6060
   tgcagggcaa gtcaggacat tagtaaatat ttaaattggt atcagcagaa accagatgga 6120
   actgttaaac tcctgatcta ccatacatca agattacact caggagtccc atcaaggttc 6180
   agtggcagtg ggtctggaac agattattct ctcaccatta gcaacctgga gcaagaagat 6240
   attgccactt acttttgcca acagggtaat acgcttccgt acacgttcgg aggggggact 6300
   aagttggaaa taacaggtgg cggtggctcg ggcggtggtg ggtcgggtgg cggcggatct 6360
   gaggtgaaac tgcaggagtc aggacctggc ctggtggcgc cctcacagag cctgtccgtc 6420
   acatgcactg tctcaggggt ctcattaccc gactatggtg taagctggat tcgccagcct 6480
   ccacgaaagg gtctggagtg gctgggagta atatggggta gtgaaaccac atactataat 6540
   tcagctctca aatccagact gaccatcatc aaggacaact ccaagagcca agttttctta 6600
   aaaatgaaca gtctgcaaac tgatgacaca gccatttact actgtgccaa acattattac 6660
   tacggtggta gctatgctat ggactactgg ggtcaaggaa cctcagtcac cgtctcctca 6720
   gctagcacgc gtggtggcgg aggttctgga ggtgggggtt cccagggggc ctggccacat 6780
   gagggagtcc acagaaaacc ttccctcctg gcccacccag gtcccctggt gaaatcagaa 6840
   gagacagtca tcctgcaatg ttggtcagat gtcaggtttg agcacttcct tctgcacaga 6900
   gaggggaagt ataaggacac tttgcacctc attggagagc accatgatgg ggtctccaag 6960
   gccaacttct ccatcggtcc catgatgcaa gaccttgcag ggacctacag atgctacggt 7020
   tctgttactc actcccccta tcagttgtca gctcccagtg accctctgga catcgtcatc 7080
   acaggtctat atgagaaacc ttctctctca gcccagccgg gccccacggt tttggcagga 7140
   gagagcgtga ccttgtcctg cagctcccgg agctcctatg acatgtacca tctatccagg 7200
   gagggggagg cccatgaacg taggttctct gcagggccca aggtcaacgg aacattccag 7260
   gccgactttc ctctgggccc tgccacccac ggaggaacct acagatgctt cggctctttc 7320
   cgtgactctc cctatgagtg gtcaaactcg agtgacccac tgcttgtttc tgtcacagga 7380
   aacccttcaa atagttggcc ttcacccact gaaccaagct ccaaaaccgg taaccccaga 7440
   cacctgcatg ttctgattgg gacctcagtg gtcaaaatcc ctttcaccat cctcctcttc 7500
   tttctccttc atcgctggtg ctccaacaaa aaaaatgctg ctgtaatgga ccaagagcct 7560
   gcagggaaca gaacagtgaa cagcgaggat tctgatgaac aagaccatca ggaggtgtca 7620
   tacgcataag tcgacaatca acctctggat tacaaaattt gtgaaagatt gactggtatt 7680
   cttaactatg ttgctccttt tacgctatgt ggatacgctg ctttaatgcc tttgtatcat 7740
   gctattgctt cccgtatggc tttcattttc tcctccttgt ataaatcctg gttgctgtct 7800
   ctttatgagg agttgtggcc cgttgtcagg caacgtggcg tggtgtgcac tgtgtttgct 7860
   gacgcaaccc ccactggttg gggcattgcc accacctgtc agctcctttc cgggactttc 7920
   gctttccccc tccctattgc cacggcggaa ctcatcgccg cctgccttgc ccgctgctgg 7980
   acaggggctc ggctgttggg cactgacaat tccgtggtgt tgtcggggaa gctgacgtcc 8040
   tttccatggc tgctcgcctg tgttgccacc tggattctgc gcgggacgtc cttctgctac 8100
   gtcccttcgg ccctcaatcc agcggacctt ccttcccgcg gcctgctgcc ggctctgcgg 8160
   cctcttccgc gtcttcgcct tcgccctcag acgagtcgga tctccctttg ggccgcctcc 8220
   ccgcctggaa ttcgagctcg gtacctttaa gaccaatgac ttacaaggca gctgtagatc 8280
   ttagccactt tttaaaagaa aaggggggac tggaagggct aattcactcc caacgaagac 8340
   aagatctgct ttttgcttgt actgggtctc tctggttaga ccagatctga gcctgggagc 8400
   tctctggcta actagggaac ccactgctta agcctcaata aagcttgcct tgagtgcttc 8460
   aagtagtgtg tgcccgtctg ttgtgtgact ctggtaacta gagatccctc agaccctttt 8520
   agtcagtgtg gaaaatctct agcagtagta gttcatgtca tcttattatt cagtatttat 8580
   aacttgcaaa gaaatgaata tcagagagtg agaggaactt gtttattgca gcttataatg 8640
   gttacaaata aagcaatagc atcacaaatt tcacaaataa agcatttttt tcactgcatt 8700
   ctagttgtgg tttgtccaaa ctcatcaatg tatcttatca tgtctggctc tagctatccc 8760
   gcccctaact ccgcccagtt ccgcccattc tccgccccat ggctgactaa ttttttttat 8820
   ttatgcagag gccgaggccg cctcggcctc tgagctattc cagaagtagt gaggaggctt 8880
   ttttggaggc ctacgctttt gcgtcgagac gtacccaatt cgccctatag tgagtcgtat 8940
   tacgcgcgct cactggccgt cgttttacaa cgtcgtgact gggaaaaccc tggcgttacc 9000
   caacttaatc gccttgcagc acatccccct ttcgccagct ggcgtaatag cgaagaggcc 9060
   cgcaccgatc gcccttccca acagttgcgc agcctgaatg gcgaatggcg cgacgcgccc 9120
   tgtagcggcg cattaagcgc ggcgggtgtg gtggttacgc gcagcgtgac cgctacactt 9180
   gccagcgccc tagcgcccgc tcctttcgct ttcttccctt cctttctcgc cacgttcgcc 9240
   ggctttcccc gtcaagctct aaatcggggg ctccctttag ggttccgatt tagtgcttta 9300
   cggcacctcg accccaaaaa acttgattag ggtgatggtt cacgtagtgg gccatcgccc 9360
   tgatagacgg tttttcgccc tttgacgttg gagtccacgt tctttaatag tggactcttg 9420
   ttccaaactg gaacaacact caaccctatc tcggtctatt cttttgattt ataagggatt 9480
   ttgccgattt cggcctattg gttaaaaaat gagctgattt aacaaaaatt taacgcgaat 9540
   tttaacaaaa tattaacgtt tacaatttcc caggtggcac ttttcgggga aatgtgcgcg 9600
   gaacccctat ttgtttattt ttctaaatac attcaaatat gtatccgctc atgagacaat 9660
   aaccctgata aatgcttcaa taatattgaa aaaggaagag tatgagtatt caacatttcc 9720
   gtgtcgccct tattcccttt tttgcggcat tttgccttcc tgtttttgct cacccagaaa 9780
   cgctggtgaa agtaaaagat gctgaagatc agttgg 9816
<210> 157
   <211> 3821
   <212> DNA
   <213> Artificial Sequence
<220>
   <223> Description of Artificial Sequence: Synthetic polynucleotide
<400> 157
   tgatgcggta ttttctcctt acgcatctgt gcggtatttc acaccgcata tggtgcactc 60
   tcagtacaat ctgctctgat gccgcatagt taagccagcc ccgacacccg ccaacacccg 120
   ctgacgcgcc ctgacgggct tgtctgctcc cggcatccgc ttacagacaa gctgtgaccg 180
   tctccgggag ctgcatgtgt cagaggtttt caccgtcatc accgaaacgc gcgagacgaa 240
   agggcctcgt gatacgccta tttttatagg ttaatgtcat gataataatg gtttcttaga 300
   cgtcaggtgg cacttttcgg ggaaatgtgc gcggaacccc tatttgttta tttttctaaa 360
   tacattcaaa tatgtatccg ctcatgagac aataaccctg ataaatgctt caataatatt 420
   gaaaaaggaa gagtatgagt attcaacatt tccgtgtcgc ccttattccc ttttttgcgg 480
   cattttgcct tcctgttttt gctcacccag aaacgctggt gaaagtaaaa gatgctgaag 540
   atcagttggg tgcacgagtg ggttacatcg aactggatct caacagcggt aagatccttg 600
   agagttttcg ccccgaagaa cgttttccaa tgatgagcac ttttaaagtt ctgctatgtg 660
   gcgcggtatt atcccgtatt gacgccgggc aagagcaact cggtcgccgc atacactatt 720
   ctcagaatga cttggttgag tactcaccag tcacagaaaa gcatcttacg gatggcatga 780
   cagtaagaga attatgcagt gctgccataa ccatgagtga taacactgcg gccaacttac 840
   ttctgacaac gatcggagga ccgaaggagc taaccgcttt tttgcacaac atgggggatc 900
   atgtaactcg ccttgatcgt tgggaaccgg agctgaatga agccatacca aacgacgagc 960
   gtgacaccac gatgcctgta gcaatggcaa caacgttgcg caaactatta actggcgaac 1020
   tacttactct agcttcccgg caacaattaa tagactggat ggaggcggat aaagttgcag 1080
   gaccacttct gcgctcggcc cttccggctg gctggtttat tgctgataaa tctggagccg 1140
   gtgagcgtgg gtctcgcggt atcattgcag cactggggcc agatggtaag ccctcccgta 1200
   tcgtagttat ctacacgacg gggagtcagg caactatgga tgaacgaaat agacagatcg 1260
   ctgagatagg tgcctcactg attaagcatt ggtaactgtc agaccaagtt tactcatata 1320
   tactttagat tgatttaaaa cttcattttt aatttaaaag gatctaggtg aagatccttt 1380
   ttgataatct catgaccaaa atcccttaac gtgagttttc gttccactga gcgtcagacc 1440
   ccgtagaaaa gatcaaagga tcttcttgag atcctttttt tctgcgcgta atctgctgct 1500
   tgcaaacaaa aaaaccaccg ctaccagcgg tggtttgttt gccggatcaa gagctaccaa 1560
   ctctttttcc gaaggtaact ggcttcagca gagcgcagat accaaatact gttcttctag 1620
   tgtagccgta gttaggccac cacttcaaga actctgtagc accgcctaca tacctcgctc 1680
   tgctaatcct gttaccagtg gctgctgcca gtggcgataa gtcgtgtctt accgggttgg 1740
   actcaagacg atagttaccg gataaggcgc agcggtcggg ctgaacgggg ggttcgtgca 1800
   cacagcccag cttggagcga acgacctaca ccgaactgag atacctacag cgtgagctat 1860
   gagaaagcgc cacgcttccc gaagggagaa aggcggacag gtatccggta agcggcaggg 1920
   tcggaacagg agagcgcacg agggagcttc cagggggaaa cgcctggtat ctttatagtc 1980
   ctgtcgggtt tcgccacctc tgacttgagc gtcgattttt gtgatgctcg tcaggggggc 2040
   ggagcctatg gaaaaacgcc agcaacgcgg cctttttacg gttcctggcc ttttgctggc 2100
   cttttgctca catgttcttt cctgcgttat cccctgattc tgtggataac cgtattaccg 2160
   cctttgagtg agctgatacc gctcgccgca gccgaacgac cgagcgcagc gagtcagtga 2220
   gcgaggaagc ggaagagcgc ccaatacgca aaccgcctct ccccgcgcgt tggccgattc 2280
   attaatgcag ctggcacgac aggtttcccg actggaaagc gggcagtgag cgcaacgcaa 2340
   ttaatgtgag ttagctcact cattaggcac cccaggcttt acactttatg cttccggctc 2400
   gtatgttgtg tggaattgtg agcggataac aatttcacac aggaaacagc tatgaccatg 2460
   attacgccaa gctctaatac gactcactat agggagacaa gcttgcatgc ctgcaggtcg 2520
   acatggcctt accagtgacc gccttgctcc tgccgctggc cttgctgctc cacgccgcca 2580
   ggccggacat ccagatgaca cagactacat cctccctgtc tgcctctctg ggagacagag 2640
   tcaccatcag ttgcagggca agtcaggaca ttagtaaata tttaaattgg tatcagcaga 2700
   aaccagatgg aactgttaaa ctcctgatct accatacatc aagattacac tcaggagtcc 2760
   catcaaggtt cagtggcagt gggtctggaa cagattattc tctcaccatt agcaacctgg 2820
   agcaagaaga tattgccact tacttttgcc aacagggtaa tacgcttccg tacacgttcg 2880
   gaggggggac taagttggaa ataacaggtg gcggtggctc gggcggtggt gggtcgggtg 2940
   gcggcggatc tgaggtgaaa ctgcaggagt caggacctgg cctggtggcg ccctcacaga 3000
   gcctgtccgt cacatgcact gtctcagggg tctcattacc cgactatggt gtaagctgga 3060
   ttcgccagcc tccacgaaag ggtctggagt ggctgggagt aatatggggt agtgaaacca 3120
   catactataa ttcagctctc aaatccagac tgaccatcat caaggacaac tccaagagcc 3180
   aagttttctt aaaaatgaac agtctgcaaa ctgatgacac agccatttac tactgtgcca 3240
   aacattatta ctacggtggt agctatgcta tggactactg gggtcaagga acctcagtca 3300
   ccgtctcctc agctagcacg cgtggtggcg gaggttctgg aggtgggggt tccaccctgg 3360
   tggttggtgt cgtgggcggc ctgctgggca gcctggtgct gctagtctgg gtcctggccg 3420
   tcatctgctc ccgggccgca cgagggacaa taggagccag gcgcaccggc cagcccctga 3480
   aggaggaccc ctcagccgtg cctgtgttct ctgtggacta tggggagctg gatttccagt 3540
   ggcgagagaa gaccccggag ccccccgtgc cctgtgtccc tgagcagacg gagtatgcca 3600
   ccattgtctt tcctagcgga atgggcacct catcccccgc ccgcaggggc tcagctgacg 3660
   gccctcggag tgcccagcca ctgaggcctg aggatggaca ctgctcttgg cccctctgag 3720
   gatccccggg taccgagctc gaattcaaaa aaaaaaaaaa aaaaaaaaaa aaaaaaaaaa 3780
   aaaaaaaaaa aaaaaaaaaa aaaaaaaaaa ctagtggcgc c 3821
<210> 158
   <211> 1464
   <212> DNA
   <213> Artificial Sequence
<220>
   <223> Description of Artificial Sequence: Synthetic polynucleotide
<400> 158
   atggggggac ttgaaccctg cagcaggttc ctgctcctgc ctctcctgct ggctgtaagt 60
   ggtctccgtc ctgtccaggt ccaggcccag agcgattgca gttgctctac ggtgagcccg 120
   ggcgtgctgg cagggatcgt gatgggagac ctggtgctga cagtgctcat tgccctggcc 180
   gtgtacttcc tgggccggct ggtccctcgg gggcgagggg ctgcggaggc agcgacccgg 240
   aaacagcgta tcactgagac cgagtcgcct tatcaggagc tccagggtca gaggtcggat 300
   gtctacagcg acctcaacac acagaggccg tattacaaag tcgagggcgg cggagagggc 360
   agaggaagtc ttctaacatg cggtgacgtg gaggagaatc ccggccctag gatggcctta 420
   ccagtgaccg ccttgctcct gccgctggcc ttgctgctcc acgccgccag gccgggatcc 480
   caggtacaac tgcagcagtc tgggcctgag ctggagaagc ctggcgcttc agtgaagata 540
   tcctgcaagg cttctggtta ctcattcact ggctacacca tgaactgggt gaagcagagc 600
   catggaaaga gccttgagtg gattggactt attactcctt acaatggtgc ttctagctac 660
   aaccagaagt tcaggggcaa ggccacatta actgtagaca agtcatccag cacagcctac 720
   atggacctcc tcagtctgac atctgaagac tctgcagtct atttctgtgc aagggggggt 780
   tacgacggga ggggttttga ctactggggc caagggacca cggtcaccgt ctcctcaggt 840
   ggaggcggtt caggcggcgg tggctctagc ggtggtggat cggacatcga gctcactcag 900
   tctccagcaa tcatgtctgc atctccaggg gagaaggtca ccatgacctg cagtgccagc 960
   tcaagtgtaa gttacatgca ctggtaccag cagaagtcag gcacctcccc caaaagatgg 1020
   atttatgaca catccaaact ggcttctgga gtcccaggtc gcttcagtgg cagtgggtct 1080
   ggaaactctt actctctcac aatcagcagc gtggaggctg aagatgatgc aacttattac 1140
   tgccagcagt ggagtaagca ccctctcacg tacggtgctg ggacaaagtt ggaaatcaaa 1200
   gctagcggtg gcggaggttc tggaggtggg ggttcctcac ccactgaacc aagctccaaa 1260
   accggtaacc ccagacacct gcatgttctg attgggacct cagtggtcaa aatccctttc 1320
   accatcctcc tcttctttct ccttcatcgc tggtgctcca acaaaaaaaa tgctgctgta 1380
   atggaccaag agcctgcagg gaacagaaca gtgaacagcg aggattctga tgaacaagac 1440
   catcaggagg tgtcatacgc ataa 1464
<210> 159
   <211> 487
   <212> PRT
   <213> Artificial Sequence
<220>
   <223> Description of Artificial Sequence: Synthetic polypeptide
<400> 159
   Met Gly Gly Leu Glu Pro Cys Ser Arg Phe Leu Leu Leu Pro Leu Leu
   1               5                   10                  15
   Leu Ala Val Ser Gly Leu Arg Pro Val Gln Val Gln Ala Gln Ser Asp
               20                  25                  30
   Cys Ser Cys Ser Thr Val Ser Pro Gly Val Leu Ala Gly Ile Val Met
           35                  40                  45
   Gly Asp Leu Val Leu Thr Val Leu Ile Ala Leu Ala Val Tyr Phe Leu
       50                  55                  60
   Gly Arg Leu Val Pro Arg Gly Arg Gly Ala Ala Glu Ala Ala Thr Arg
   65                  70                  75                  80
   Lys Gln Arg Ile Thr Glu Thr Glu Ser Pro Tyr Gln Glu Leu Gln Gly
                   85                  90                  95
   Gln Arg Ser Asp Val Tyr Ser Asp Leu Asn Thr Gln Arg Pro Tyr Tyr
               100                 105                 110
   Lys Val Glu Gly Gly Gly Glu Gly Arg Gly Ser Leu Leu Thr Cys Gly
           115                 120                 125
   Asp Val Glu Glu Asn Pro Gly Pro Arg Met Ala Leu Pro Val Thr Ala
       130                 135                 140
   Leu Leu Leu Pro Leu Ala Leu Leu Leu His Ala Ala Arg Pro Gly Ser
   145                 150                 155                 160
   Gln Val Gln Leu Gln Gln Ser Gly Pro Glu Leu Glu Lys Pro Gly Ala
   165 170 175
   Ser Val Lys Ile Ser Cys Lys Ala Ser Gly Tyr Ser Phe Thr Gly Tyr
   180 185 190
   Thr Met Asn Trp Val Lys Gln Ser His Gly Lys Ser Leu Glu Trp Ile
   195 200 205
   Gly Leu Ile Thr Pro Tyr Asn Gly Ala Ser Ser Tyr Asn Gln Lys Phe
   210 215 220
   Arg Gly Lys Ala Thr Leu Thr Val Asp Lys Ser Ser Ser Thr Ala Tyr
   225 230 235 240
   Met Asp Leu Leu Ser Leu Thr Ser Glu Asp Ser Ala Val Tyr Phe Cys
   245 250 255
   Ala Arg Gly Gly Tyr Asp Gly Arg Gly Phe Asp Tyr Trp Gly Gln Gly
   260 265 270
   Thr Thr Val Thr Val Ser Ser Gly Gly Gly Gly Ser Gly Gly Gly Gly
   275 280 285
   Ser Ser Gly Gly Gly Ser Asp Ile Glu Leu Thr Gln Ser Pro Ala Ile
   290 295 300
   Met Ser Ala Ser Pro Gly Glu Lys Val Thr Met Thr Cys Ser Ala Ser
   305 310 315 320
   Ser Ser Val Ser Tyr Met His Trp Tyr Gln Gln Lys Ser Gly Thr Ser
   325 330 335
   Pro Lys Arg Trp Ile Tyr Asp Thr Ser Lys Leu Ala Ser Gly Val Pro
   340 345 350
   Gly Arg Phe Ser Gly Ser Gly Ser Gly Asn Ser Tyr Ser Leu Thr Ile
   355 360 365
   Ser Ser Val Glu Ala Glu Asp Asp Ala Thr Tyr Tyr Cys Gln Gln Trp
   370 375 380
   Ser Lys His Pro Leu Thr Tyr Gly Ala Gly Thr Lys Leu Glu Ile Lys
   385 390 395 400
   Ala Ser Gly Gly Gly Gly Ser Gly Gly Gly Gly Ser Ser Pro Thr Glu
                   405                 410                 415
   Pro Ser Ser Lys Thr Gly Asn Pro Arg His Leu His Val Leu Ile Gly
               420                 425                 430
   Thr Ser Val Val Lys Ile Pro Phe Thr Ile Leu Leu Phe Phe Leu Leu
           435                 440                 445
   His Arg Trp Cys Ser Asn Lys Lys Asn Ala Ala Val Met Asp Gln Glu
       450                 455                 460
   Pro Ala Gly Asn Arg Thr Val Asn Ser Glu Asp Ser Asp Glu Gln Asp
   465                 470                 475                 480
   His Gln Glu Val Ser Tyr Ala
                   485
<210> 160
   <211> 1365
   <212> DNA
   <213> Artificial Sequence
<220>
   <223> Description of Artificial Sequence: Synthetic polynucleotide
<400> 160
   atgattccag cagtggtctt gctcttactc cttttggttg aacaagcagc ggccctggga      60
   gagcctcagc tctgctatat cctggatgcc atcctgtttc tgtatggaat tgtcctcacc     120
   ctcctctact gccgactgaa gatccaagtg cgaaaggcag ctataaccag ctatgagaaa     180
   tcagatggtg tttacacggg cctgagcacc aggaaccagg agacttacga gactctgaag     240
   catgagaaac caccacagtc cggaggcggc ggagagggca gaggaagtct tctaacatgc     300
   ggtgacgtgg aggagaatcc cggccctagg atggccttac cagtgaccgc cttgctcctg     360
   ccgctggcct tgctgctcca cgccgccagg ccgggatccc aggtacaact gcagcagtct     420
   gggcctgagc tggagaagcc tggcgcttca gtgaagatat cctgcaaggc ttctggttac     480
   tcattcactg gctacaccat gaactgggtg aagcagagcc atggaaagag ccttgagtgg     540
   attggactta ttactcctta caatggtgct tctagctaca accagaagtt caggggcaag     600
   gccacattaa ctgtagacaa gtcatccagc acagcctaca tggacctcct cagtctgaca     660
   tctgaagact ctgcagtcta tttctgtgca agggggggtt acgacgggag gggttttgac     720
   tactggggcc aagggaccac ggtcaccgtc tcctcaggtg gaggcggttc aggcggcggt     780
   ggctctagcg gtggtggatc ggacatcgag ctcactcagt ctccagcaat catgtctgca     840
   tctccagggg agaaggtcac catgacctgc agtgccagct caagtgtaag ttacatgcac     900
   tggtaccagc agaagtcagg cacctccccc aaaagatgga tttatgacac atccaaactg 960
   gcttctggag tcccaggtcg cttcagtggc agtgggtctg gaaactctta ctctctcaca 1020
   atcagcagcg tggaggctga agatgatgca acttattact gccagcagtg gagtaagcac 1080
   cctctcacgt acggtgctgg gacaaagttg gaaatcaaag ctagcggtgg cggaggttct 1140
   ggaggtgggg gttccttaac cacagagacg ggactccaga aagaccatgc cctctgggat 1200
   cacactgccc agaatctcct tcggatgggc ctggcctttc tagtcctggt ggctctagtg 1260
   tggttcctgg ttgaagactg gctcagcagg aagaggacta gagagcgagc cagcagagct 1320
   tccacttggg aaggcaggag aaggctgaac acacagactc tttga 1365
<210> 161
   <211> 454
   <212> PRT
   <213> Artificial Sequence
<220>
   <223> Description of Artificial Sequence: Synthetic polypeptide
<400> 161
   Met Ile Pro Ala Val Val Leu Leu Leu Leu Leu Leu Val Glu Gln Ala
   1               5                   10                  15
   Ala Ala Leu Gly Glu Pro Gln Leu Cys Tyr Ile Leu Asp Ala Ile Leu
               20                  25                  30
   Phe Leu Tyr Gly Ile Val Leu Thr Leu Leu Tyr Cys Arg Leu Lys Ile
           35                  40                  45
   Gln Val Arg Lys Ala Ala Ile Thr Ser Tyr Glu Lys Ser Asp Gly Val
       50                  55                  60
   Tyr Thr Gly Leu Ser Thr Arg Asn Gln Glu Thr Tyr Glu Thr Leu Lys
   65                  70                  75                  80
   His Glu Lys Pro Pro Gln Ser Gly Gly Gly Gly Glu Gly Arg Gly Ser
                   85                  90                  95
   Leu Leu Thr Cys Gly Asp Val Glu Glu Asn Pro Gly Pro Arg Met Ala
               100                 105                 110
   Leu Pro Val Thr Ala Leu Leu Leu Pro Leu Ala Leu Leu Leu His Ala
           115                 120                 125
   Ala Arg Pro Gly Ser Gln Val Gln Leu Gln Gln Ser Gly Pro Glu Leu
       130                 135                 140
   Glu Lys Pro Gly Ala Ser Val Lys Ile Ser Cys Lys Ala Ser Gly Tyr
   145 150 155 160
   Ser Phe Thr Gly Tyr Thr Met Asn Trp Val Lys Gln Ser His Gly Lys
   165 170 175
   Ser Leu Glu Trp Ile Gly Leu Ile Thr Pro Tyr Asn Gly Ala Ser Ser
   180 185 190
   Tyr Asn Gln Lys Phe Arg Gly Lys Ala Thr Leu Thr Val Asp Lys Ser
   195 200 205
   Ser Ser Thr Ala Tyr Met Asp Leu Leu Ser Leu Thr Ser Glu Asp Ser
   210 215 220
   Ala Val Tyr Phe Cys Ala Arg Gly Gly Tyr Asp Gly Arg Gly Phe Asp
   225 230 235 240
   Tyr Trp Gly Gln Gly Thr Thr Val Thr Val Ser Ser Gly Gly Gly Gly
   245 250 255
   Ser Gly Gly Gly Gly Ser Ser Gly Gly Gly Ser Asp Ile Glu Leu Thr
   260 265 270
   Gln Ser Pro Ala Ile Met Ser Ala Ser Pro Gly Glu Lys Val Thr Met
   275 280 285
   Thr Cys Ser Ala Ser Ser Ser Val Ser Tyr Met His Trp Tyr Gln Gln
   290 295 300
   Lys Ser Gly Thr Ser Pro Lys Arg Trp Ile Tyr Asp Thr Ser Lys Leu
   305 310 315 320
   Ala Ser Gly Val Pro Gly Arg Phe Ser Gly Ser Gly Ser Gly Asn Ser
   325 330 335
   Tyr Ser Leu Thr Ile Ser Ser Val Glu Ala Glu Asp Asp Ala Thr Tyr
   340 345 350
   Tyr Cys Gln Gln Trp Ser Lys His Pro Leu Thr Tyr Gly Ala Gly Thr
   355 360 365
   Lys Leu Glu Ile Lys Ala Ser Gly Gly Gly Gly Ser Gly Gly Gly Gly
   370 375 380
   Ser Leu Thr Thr Glu Thr Gly Leu Gln Lys Asp His Ala Leu Trp Asp
   385 390 395 400
   His Thr Ala Gln Asn Leu Leu Arg Met Gly Leu Ala Phe Leu Val Leu
   405 410 415
   Val Ala Leu Val Trp Phe Leu Val Glu Asp Trp Leu Ser Arg Lys Arg
   420 425 430
   Thr Arg Glu Arg Ala Ser Arg Ala Ser Thr Trp Glu Gly Arg Arg Arg
   435 440 445
   Leu Asn Thr Gln Thr Leu
   450
<210> 162
   <211> 1470
   <212> DNA
   <213> Artificial Sequence
<220>
   <223> Description of Artificial Sequence: Synthetic polynucleotide
<400> 162
   atggggggac ttgaaccctg cagcaggttc ctgctcctgc ctctcctgct ggctgtaagt 60
   ggtctccgtc ctgtccaggt ccaggcccag agcgattgca gttgctctac ggtgagcccg 120
   ggcgtgctgg cagggatcgt gatgggagac ctggtgctga cagtgctcat tgccctggcc 180
   gtgtacttcc tgggccggct ggtccctcgg gggcgagggg ctgcggaggc agcgacccgg 240
   aaacagcgta tcactgagac cgagtcgcct tatcaggagc tccagggtca gaggtcggat 300
   gtctacagcg acctcaacac acagaggccg tattacaaag tcgagggcgg cggagagggc 360
   agaggaagtc ttctaacatg cggtgacgtg gaggagaatc ccggccctag gatggcctta 420
   ccagtgaccg ccttgctcct gccgctggcc ttgctgctcc acgccgccag gccgggatcc 480
   gacatccaga tgacacagac tacatcctcc ctgtctgcct ctctgggaga cagagtcacc 540
   atcagttgca gggcaagtca ggacattagt aaatatttaa attggtatca gcagaaacca 600
   gatggaactg ttaaactcct gatctaccat acatcaagat tacactcagg agtcccatca 660
   aggttcagtg gcagtgggtc tggaacagat tattctctca ccattagcaa cctggagcaa 720
   gaagatattg ccacttactt ttgccaacag ggtaatacgc ttccgtacac gttcggaggg 780
   gggactaagt tggaaataac aggtggcggt ggctcgggcg gtggtgggtc gggtggcggc 840
   ggatctgagg tgaaactgca ggagtcagga cctggcctgg tggcgccctc acagagcctg 900
   tccgtcacat gcactgtctc aggggtctca ttacccgact atggtgtaag ctggattcgc 960
   cagcctccac gaaagggtct ggagtggctg ggagtaatat ggggtagtga aaccacatac 1020
   tataattcag ctctcaaatc cagactgacc atcatcaagg acaactccaa gagccaagtt 1080
   ttcttaaaaa tgaacagtct gcaaactgat gacacagcca tttactactg tgccaaacat 1140
   tattactacg gtggtagcta tgctatggac tactggggtc aaggaacctc agtcaccgtc 1200
   tcctcagcta gcggtggcgg aggttctgga ggtgggggtt cctcacccac tgaaccaagc 1260
   tccaaaaccg gtaaccccag acacctgcat gttctgattg ggacctcagt ggtcaaaatc 1320
   cctttcacca tcctcctctt ctttctcctt catcgctggt gctccaacaa aaaaaatgct 1380
   gctgtaatgg accaagagcc tgcagggaac agaacagtga acagcgagga ttctgatgaa 1440
   caagaccatc aggaggtgtc atacgcataa 1470
<210> 163
   <211> 489
   <212> PRT
   <213> Artificial Sequence
<220>
   <223> Description of Artificial Sequence: Synthetic polypeptide
<400> 163
   Met Gly Gly Leu Glu Pro Cys Ser Arg Phe Leu Leu Leu Pro Leu Leu
   1               5                   10                  15
   Leu Ala Val Ser Gly Leu Arg Pro Val Gln Val Gln Ala Gln Ser Asp
               20                  25                  30
   Cys Ser Cys Ser Thr Val Ser Pro Gly Val Leu Ala Gly Ile Val Met
           35                  40                  45
   Gly Asp Leu Val Leu Thr Val Leu Ile Ala Leu Ala Val Tyr Phe Leu
       50                  55                  60
   Gly Arg Leu Val Pro Arg Gly Arg Gly Ala Ala Glu Ala Ala Thr Arg
   65                  70                  75                  80
   Lys Gln Arg Ile Thr Glu Thr Glu Ser Pro Tyr Gln Glu Leu Gln Gly
                   85                  90                  95
   Gln Arg Ser Asp Val Tyr Ser Asp Leu Asn Thr Gln Arg Pro Tyr Tyr
               100                 105                 110
   Lys Val Glu Gly Gly Gly Glu Gly Arg Gly Ser Leu Leu Thr Cys Gly
           115                 120                 125
   Asp Val Glu Glu Asn Pro Gly Pro Arg Met Ala Leu Pro Val Thr Ala
       130                 135                 140
   Leu Leu Leu Pro Leu Ala Leu Leu Leu His Ala Ala Arg Pro Gly Ser
   145                 150                 155                 160
   Asp Ile Gln Met Thr Gln Thr Thr Ser Ser Leu Ser Ala Ser Leu Gly
   165 170 175
   Asp Arg Val Thr Ile Ser Cys Arg Ala Ser Gln Asp Ile Ser Lys Tyr
   180 185 190
   Leu Asn Trp Tyr Gln Gln Lys Pro Asp Gly Thr Val Lys Leu Leu Ile
   195 200 205
   Tyr His Thr Ser Arg Leu His Ser Gly Val Pro Ser Arg Phe Ser Gly
   210 215 220
   Ser Gly Ser Gly Thr Asp Tyr Ser Leu Thr Ile Ser Asn Leu Glu Gln
   225 230 235 240
   Glu Asp Ile Ala Thr Tyr Phe Cys Gln Gln Gly Asn Thr Leu Pro Tyr
   245 250 255
   Thr Phe Gly Gly Gly Thr Lys Leu Glu Ile Thr Gly Gly Gly Gly Ser
   260 265 270
   Gly Gly Gly Gly Ser Gly Gly Gly Gly Ser Glu Val Lys Leu Gln Glu
   275 280 285
   Ser Gly Pro Gly Leu Val Ala Pro Ser Gln Ser Leu Ser Val Thr Cys
   290 295 300
   Thr Val Ser Gly Val Ser Leu Pro Asp Tyr Gly Val Ser Trp Ile Arg
   305 310 315 320
   Gln Pro Pro Arg Lys Gly Leu Glu Trp Leu Gly Val Ile Trp Gly Ser
   325 330 335
   Glu Thr Thr Tyr Tyr Asn Ser Ala Leu Lys Ser Arg Leu Thr Ile Ile
   340 345 350
   Lys Asp Asn Ser Lys Ser Gln Val Phe Leu Lys Met Asn Ser Leu Gln
   355 360 365
   Thr Asp Asp Thr Ala Ile Tyr Tyr Cys Ala Lys His Tyr Tyr Tyr Gly
   370 375 380
   Gly Ser Tyr Ala Met Asp Tyr Trp Gly Gln Gly Thr Ser Val Thr Val
   385 390 395 400
   Ser Ser Ala Ser Gly Gly Gly Gly Ser Gly Gly Gly Gly Ser Ser Pro
   405 410 415
   Thr Glu Pro Ser Ser Lys Thr Gly Asn Pro Arg His Leu His Val Leu
   420 425 430
   Ile Gly Thr Ser Val Val Lys Ile Pro Phe Thr Ile Leu Leu Phe Phe
   435 440 445
   Leu Leu His Arg Trp Cys Ser Asn Lys Lys Asn Ala Ala Val Met Asp
   450 455 460
   Gln Glu Pro Ala Gly Asn Arg Thr Val Asn Ser Glu Asp Ser Asp Glu
   465 470 475 480
   Gln Asp His Gln Glu Val Ser Tyr Ala
   485
<210> 164
   <211> 97
   <212> PRT
   <213> Artificial Sequence
<220>
   <223> Description of Artificial Sequence: Synthetic polypeptide
<400> 164
   Cys Ser Arg Ala Ala Arg Gly Thr Ile Gly Ala Arg Arg Thr Gly Gln
   1 5 10 15
   Pro Leu Lys Glu Asp Pro Ser Ala Val Pro Val Phe Ser Val Asp Tyr
   20 25 30
   Gly Glu Leu Asp Phe Gln Trp Arg Glu Lys Thr Pro Glu Pro Pro Val
   35 40 45
   Pro Cys Val Pro Glu Gln Thr Glu Tyr Ala Thr Ile Val Phe Pro Ser
   50 55 60
   Gly Met Gly Thr Ser Ser Pro Ala Arg Arg Gly Ser Ala Asp Gly Pro
   65 70 75 80
   Arg Ser Ala Gln Pro Leu Arg Pro Glu Asp Gly His Cys Ser Trp Pro
   85 90 95
   Leu
<210> 165
   <211> 41
   <212> PRT
   <213> Artificial Sequence
<220>
   <223> Description of Artificial Sequence: Synthetic polypeptide
<400> 165
   Ala Val Ser Leu Ser Lys Met Leu Lys Lys Arg Ser Pro Leu Thr Thr
   1               5                   10                  15
   Gly Val Tyr Val Lys Met Pro Pro Thr Glu Pro Glu Cys Glu Lys Gln
               20                  25                  30
   Phe Gln Pro Tyr Phe Ile Pro Ile Asn
           35                  40
<210> 166
   <211> 230
   <212> PRT
   <213> Artificial Sequence
<220>
   <223> Description of Artificial Sequence: Synthetic polypeptide
<400> 166
   Glu Ser Lys Tyr Gly Pro Pro Cys Pro Pro Cys Pro Ala Pro Glu Phe
   1               5                   10                  15
   Leu Gly Gly Pro Ser Val Phe Leu Phe Pro Pro Lys Pro Lys Asp Thr
               20                  25                  30
   Leu Met Ile Ser Arg Thr Pro Glu Val Thr Cys Val Val Val Asp Val
           35                  40                  45
   Ser Gln Glu Asp Pro Glu Val Gln Phe Asn Trp Tyr Val Asp Gly Val
       50                  55                  60
   Glu Val His Asn Ala Lys Thr Lys Pro Arg Glu Glu Gln Phe Asn Ser
   65                  70                  75                  80
   Thr Tyr Arg Val Val Ser Val Leu Thr Val Leu His Gln Asp Trp Leu
                   85                  90                  95
   Asn Gly Lys Glu Tyr Lys Cys Lys Val Ser Asn Lys Gly Leu Pro Ser
               100                 105                 110
   Ser Ile Glu Lys Thr Ile Ser Lys Ala Lys Gly Gln Pro Arg Glu Pro
           115                 120                 125
   Gln Val Tyr Thr Leu Pro Pro Ser Gln Glu Glu Met Thr Lys Asn Gln
   130 135 140
   Val Ser Leu Thr Cys Leu Val Lys Gly Phe Tyr Pro Ser Asp Ile Ala
   145 150 155 160
   Val Glu Trp Glu Ser Asn Gly Gln Pro Glu Asn Asn Tyr Lys Thr Thr
   165 170 175
   Pro Pro Val Leu Asp Ser Asp Gly Ser Phe Phe Leu Tyr Ser Arg Leu
   180 185 190
   Thr Val Asp Lys Ser Arg Trp Gln Glu Gly Asn Val Phe Ser Cys Ser
   195 200 205
   Val Met His Glu Ala Leu His Asn His Tyr Thr Gln Lys Ser Leu Ser
   210 215 220
   Leu Ser Leu Gly Lys Met
   225 230
<210> 167
   <211> 282
   <212> PRT
   <213> Artificial Sequence
<220>
   <223> Description of Artificial Sequence: Synthetic polypeptide
<400> 167
   Arg Trp Pro Glu Ser Pro Lys Ala Gln Ala Ser Ser Val Pro Thr Ala
   1 5 10 15
   Gln Pro Gln Ala Glu Gly Ser Leu Ala Lys Ala Thr Thr Ala Pro Ala
   20 25 30
   Thr Thr Arg Asn Thr Gly Arg Gly Gly Glu Glu Lys Lys Lys Glu Lys
   35 40 45
   Glu Lys Glu Glu Gln Glu Glu Arg Glu Thr Lys Thr Pro Glu Cys Pro
   50 55 60
   Ser His Thr Gln Pro Leu Gly Val Tyr Leu Leu Thr Pro Ala Val Gln
   65 70 75 80
   Asp Leu Trp Leu Arg Asp Lys Ala Thr Phe Thr Cys Phe Val Val Gly
   85 90 95
   Ser Asp Leu Lys Asp Ala His Leu Thr Trp Glu Val Ala Gly Lys Val
   100 105 110
   Pro Thr Gly Gly Val Glu Glu Gly Leu Leu Glu Arg His Ser Asn Gly
   115 120 125
   Ser Gln Ser Gln His Ser Arg Leu Thr Leu Pro Arg Ser Leu Trp Asn
   130 135 140
   Ala Gly Thr Ser Val Thr Cys Thr Leu Asn His Pro Ser Leu Pro Pro
   145 150 155 160
   Gln Arg Leu Met Ala Leu Arg Glu Pro Ala Ala Gln Ala Pro Val Lys
   165 170 175
   Leu Ser Leu Asn Leu Leu Ala Ser Ser Asp Pro Pro Glu Ala Ala Ser
   180 185 190
   Trp Leu Leu Cys Glu Val Ser Gly Phe Ser Pro Pro Asn Ile Leu Leu
   195 200 205
   Met Trp Leu Glu Asp Gln Arg Glu Val Asn Thr Ser Gly Phe Ala Pro
   210 215 220
   Ala Arg Pro Pro Pro Gln Pro Gly Ser Thr Thr Phe Trp Ala Trp Ser
   225 230 235 240
   Val Leu Arg Val Pro Ala Pro Pro Ser Pro Gln Pro Ala Thr Tyr Thr
   245 250 255
   Cys Val Val Ser His Glu Asp Ser Arg Thr Leu Leu Asn Ala Ser Arg
   260 265 270
   Ser Leu Glu Val Ser Tyr Val Thr Asp His
   275 280
<210> 168
   <211> 43
   <212> PRT
   <213> Homo sapiens
<400> 168
   Thr Thr Thr Pro Ala Pro Arg Pro Pro Thr Pro Ala Pro Thr Ile Ala
   1 5 10 15
   Ser Gln Pro Leu Ser Leu Arg Pro Glu Ala Cys Arg Pro Ala Ala Gly
   20 25 30
   Gly Ala Val His Thr Arg Gly Leu Asp Phe Ala
   35 40
<210> 169
   <211> 5000
   <212> DNA
   <213> Artificial Sequence
<220>
   <223> Description of Artificial Sequence: Synthetic polynucleotide
<220>
   <221> misc_feature
   <222> (1)..(5000)
   <223> This sequence may encompass 50-5000 nucleotides
<400> 169
   tttttttttt tttttttttt tttttttttt tttttttttt tttttttttt tttttttttt 60
   tttttttttt tttttttttt tttttttttt tttttttttt tttttttttt tttttttttt 120
   tttttttttt tttttttttt tttttttttt tttttttttt tttttttttt tttttttttt 180
   tttttttttt tttttttttt tttttttttt tttttttttt tttttttttt tttttttttt 240
   tttttttttt tttttttttt tttttttttt tttttttttt tttttttttt tttttttttt 300
   tttttttttt tttttttttt tttttttttt tttttttttt tttttttttt tttttttttt 360
   tttttttttt tttttttttt tttttttttt tttttttttt tttttttttt tttttttttt 420
   tttttttttt tttttttttt tttttttttt tttttttttt tttttttttt tttttttttt 480
   tttttttttt tttttttttt tttttttttt tttttttttt tttttttttt tttttttttt 540
   tttttttttt tttttttttt tttttttttt tttttttttt tttttttttt tttttttttt 600
   tttttttttt tttttttttt tttttttttt tttttttttt tttttttttt tttttttttt 660
   tttttttttt tttttttttt tttttttttt tttttttttt tttttttttt tttttttttt 720
   tttttttttt tttttttttt tttttttttt tttttttttt tttttttttt tttttttttt 780
   tttttttttt tttttttttt tttttttttt tttttttttt tttttttttt tttttttttt 840
   tttttttttt tttttttttt tttttttttt tttttttttt tttttttttt tttttttttt 900
   tttttttttt tttttttttt tttttttttt tttttttttt tttttttttt tttttttttt 960
   tttttttttt tttttttttt tttttttttt tttttttttt tttttttttt tttttttttt 1020
   tttttttttt tttttttttt tttttttttt tttttttttt tttttttttt tttttttttt 1080
   tttttttttt tttttttttt tttttttttt tttttttttt tttttttttt tttttttttt 1140
   tttttttttt tttttttttt tttttttttt tttttttttt tttttttttt tttttttttt 1200
   tttttttttt tttttttttt tttttttttt tttttttttt tttttttttt tttttttttt 1260
   tttttttttt tttttttttt tttttttttt tttttttttt tttttttttt tttttttttt 1320
   tttttttttt tttttttttt tttttttttt tttttttttt tttttttttt tttttttttt 1380
   tttttttttt tttttttttt tttttttttt tttttttttt tttttttttt tttttttttt 1440
   tttttttttt tttttttttt tttttttttt tttttttttt tttttttttt tttttttttt 1500
   tttttttttt tttttttttt tttttttttt tttttttttt tttttttttt tttttttttt 1560
   tttttttttt tttttttttt tttttttttt tttttttttt tttttttttt tttttttttt 1620
   tttttttttt tttttttttt tttttttttt tttttttttt tttttttttt tttttttttt 1680
   tttttttttt tttttttttt tttttttttt tttttttttt tttttttttt tttttttttt 1740
   tttttttttt tttttttttt tttttttttt tttttttttt tttttttttt tttttttttt 1800
   tttttttttt tttttttttt tttttttttt tttttttttt tttttttttt tttttttttt 1860
   tttttttttt tttttttttt tttttttttt tttttttttt tttttttttt tttttttttt 1920
   tttttttttt tttttttttt tttttttttt tttttttttt tttttttttt tttttttttt 1980
   tttttttttt tttttttttt tttttttttt tttttttttt tttttttttt tttttttttt 2040
   tttttttttt tttttttttt tttttttttt tttttttttt tttttttttt tttttttttt 2100
   tttttttttt tttttttttt tttttttttt tttttttttt tttttttttt tttttttttt 2160
   tttttttttt tttttttttt tttttttttt tttttttttt tttttttttt tttttttttt 2220
   tttttttttt tttttttttt tttttttttt tttttttttt tttttttttt tttttttttt 2280
   tttttttttt tttttttttt tttttttttt tttttttttt tttttttttt tttttttttt 2340
   tttttttttt tttttttttt tttttttttt tttttttttt tttttttttt tttttttttt 2400
   tttttttttt tttttttttt tttttttttt tttttttttt tttttttttt tttttttttt 2460
   tttttttttt tttttttttt tttttttttt tttttttttt tttttttttt tttttttttt 2520
   tttttttttt tttttttttt tttttttttt tttttttttt tttttttttt tttttttttt 2580
   tttttttttt tttttttttt tttttttttt tttttttttt tttttttttt tttttttttt 2640
   tttttttttt tttttttttt tttttttttt tttttttttt tttttttttt tttttttttt 2700
   tttttttttt tttttttttt tttttttttt tttttttttt tttttttttt tttttttttt 2760
   tttttttttt tttttttttt tttttttttt tttttttttt tttttttttt tttttttttt 2820
   tttttttttt tttttttttt tttttttttt tttttttttt tttttttttt tttttttttt 2880
   tttttttttt tttttttttt tttttttttt tttttttttt tttttttttt tttttttttt 2940
   tttttttttt tttttttttt tttttttttt tttttttttt tttttttttt tttttttttt 3000
   tttttttttt tttttttttt tttttttttt tttttttttt tttttttttt tttttttttt 3060
   tttttttttt tttttttttt tttttttttt tttttttttt tttttttttt tttttttttt 3120
   tttttttttt tttttttttt tttttttttt tttttttttt tttttttttt tttttttttt 3180
   tttttttttt tttttttttt tttttttttt tttttttttt tttttttttt tttttttttt 3240
   tttttttttt tttttttttt tttttttttt tttttttttt tttttttttt tttttttttt 3300
   tttttttttt tttttttttt tttttttttt tttttttttt tttttttttt tttttttttt 3360
   tttttttttt tttttttttt tttttttttt tttttttttt tttttttttt tttttttttt 3420
   tttttttttt tttttttttt tttttttttt tttttttttt tttttttttt tttttttttt 3480
   tttttttttt tttttttttt tttttttttt tttttttttt tttttttttt tttttttttt 3540
   tttttttttt tttttttttt tttttttttt tttttttttt tttttttttt tttttttttt 3600
   tttttttttt tttttttttt tttttttttt tttttttttt tttttttttt tttttttttt 3660
   tttttttttt tttttttttt tttttttttt tttttttttt tttttttttt tttttttttt 3720
   tttttttttt tttttttttt tttttttttt tttttttttt tttttttttt tttttttttt 3780
   tttttttttt tttttttttt tttttttttt tttttttttt tttttttttt tttttttttt 3840
   tttttttttt tttttttttt tttttttttt tttttttttt tttttttttt tttttttttt 3900
   tttttttttt tttttttttt tttttttttt tttttttttt tttttttttt tttttttttt 3960
   tttttttttt tttttttttt tttttttttt tttttttttt tttttttttt tttttttttt 4020
   tttttttttt tttttttttt tttttttttt tttttttttt tttttttttt tttttttttt 4080
   tttttttttt tttttttttt tttttttttt tttttttttt tttttttttt tttttttttt 4140
   tttttttttt tttttttttt tttttttttt tttttttttt tttttttttt tttttttttt 4200
   tttttttttt tttttttttt tttttttttt tttttttttt tttttttttt tttttttttt 4260
   tttttttttt tttttttttt tttttttttt tttttttttt tttttttttt tttttttttt 4320
   tttttttttt tttttttttt tttttttttt tttttttttt tttttttttt tttttttttt 4380
   tttttttttt tttttttttt tttttttttt tttttttttt tttttttttt tttttttttt 4440
   tttttttttt tttttttttt tttttttttt tttttttttt tttttttttt tttttttttt 4500
   tttttttttt tttttttttt tttttttttt tttttttttt tttttttttt tttttttttt 4560
   tttttttttt tttttttttt tttttttttt tttttttttt tttttttttt tttttttttt 4620
   tttttttttt tttttttttt tttttttttt tttttttttt tttttttttt tttttttttt 4680
   tttttttttt tttttttttt tttttttttt tttttttttt tttttttttt tttttttttt 4740
   tttttttttt tttttttttt tttttttttt tttttttttt tttttttttt tttttttttt 4800
   tttttttttt tttttttttt tttttttttt tttttttttt tttttttttt tttttttttt 4860
   tttttttttt tttttttttt tttttttttt tttttttttt tttttttttt tttttttttt 4920
   tttttttttt tttttttttt tttttttttt tttttttttt tttttttttt tttttttttt 4980
   tttttttttt tttttttttt 5000
<210> 170
   <211> 95
   <212> PRT
   <213> Artificial Sequence
<220>
   <223> Description of Artificial Sequence: Synthetic polypeptide
<220>
   <221> MOD_RES
   <222> (11)..(11)
   <223> Any naturally occurring amino acid
<400> 170
   Ile Leu Trp His Glu Met Trp His Glu Gly Xaa Glu Glu Ala Ser Arg
   1               5                   10                  15
   Leu Tyr Phe Gly Glu Arg Asn Val Lys Gly Met Phe Glu Val Leu Glu
               20                  25                  30
   Pro Leu His Ala Met Met Glu Arg Gly Pro Gln Thr Leu Lys Glu Thr
           35                  40                  45
   Ser Phe Asn Gln Ala Tyr Gly Arg Asp Leu Met Glu Ala Gln Glu Trp
       50                  55                  60
   Cys Arg Lys Tyr Met Lys Ser Gly Asn Val Lys Asp Leu Thr Gln Ala
   65                  70                  75                  80
   Trp Asp Leu Tyr Tyr His Val Phe Arg Arg Ile Ser Lys Thr Ser
                   85                  90                  95
<210> 171
   <211> 95
   <212> PRT
   <213> Artificial Sequence
<220>
   <223> Description of Artificial Sequence: Synthetic polypeptide
<220>
   <221> MOD_RES
   <222> (12)..(12)
   <223> Any naturally occurring amino acid
<400> 171
   Ile Leu Trp His Glu Met Trp His Glu Gly Leu Xaa Glu Ala Ser Arg
   1               5                   10                  15
   Leu Tyr Phe Gly Glu Arg Asn Val Lys Gly Met Phe Glu Val Leu Glu
               20                  25                  30
   Pro Leu His Ala Met Met Glu Arg Gly Pro Gln Thr Leu Lys Glu Thr
           35                  40                  45
   Ser Phe Asn Gln Ala Tyr Gly Arg Asp Leu Met Glu Ala Gln Glu Trp
       50                  55                  60
   Cys Arg Lys Tyr Met Lys Ser Gly Asn Val Lys Asp Leu Thr Gln Ala
   65                  70                  75                  80
   Trp Asp Leu Tyr Tyr His Val Phe Arg Arg Ile Ser Lys Thr Ser
                   85                  90                  95
<210> 172
   <211> 95
   <212> PRT
   <213> Artificial Sequence
<220>
   <223> Description of Artificial Sequence: Synthetic polypeptide
<220>
   <221> MOD_RES
   <222> (15)..(15)
   <223> Any naturally occurring amino acid
<400> 172
   Ile Leu Trp His Glu Met Trp His Glu Gly Leu Glu Glu Ala Xaa Arg
   1               5                   10                  15
   Leu Tyr Phe Gly Glu Arg Asn Val Lys Gly Met Phe Glu Val Leu Glu
               20                  25                  30
   Pro Leu His Ala Met Met Glu Arg Gly Pro Gln Thr Leu Lys Glu Thr
           35                  40                  45
   Ser Phe Asn Gln Ala Tyr Gly Arg Asp Leu Met Glu Ala Gln Glu Trp
       50                  55                  60
   Cys Arg Lys Tyr Met Lys Ser Gly Asn Val Lys Asp Leu Thr Gln Ala
   65                  70                  75                  80
   Trp Asp Leu Tyr Tyr His Val Phe Arg Arg Ile Ser Lys Thr Ser
                   85                  90                  95
<210> 173
   <211> 95
   <212> PRT
   <213> Artificial Sequence
<220>
   <223> Description of Artificial Sequence: Synthetic polypeptide
<220>
   <221> MOD_RES
   <222> (16)..(16)
   <223> Any naturally occurring amino acid
<400> 173
   Ile Leu Trp His Glu Met Trp His Glu Gly Leu Glu Glu Ala Ser Xaa
   1               5                   10                  15
   Leu Tyr Phe Gly Glu Arg Asn Val Lys Gly Met Phe Glu Val Leu Glu
               20                  25                  30
   Pro Leu His Ala Met Met Glu Arg Gly Pro Gln Thr Leu Lys Glu Thr
           35                  40                  45
   Ser Phe Asn Gln Ala Tyr Gly Arg Asp Leu Met Glu Ala Gln Glu Trp
       50                  55                  60
   Cys Arg Lys Tyr Met Lys Ser Gly Asn Val Lys Asp Leu Thr Gln Ala
   65                  70                  75                  80
   Trp Asp Leu Tyr Tyr His Val Phe Arg Arg Ile Ser Lys Thr Ser
                   85                  90                  95
<210> 174
   <211> 95
   <212> PRT
   <213> Artificial Sequence
<220>
   <223> Description of Artificial Sequence: Synthetic polypeptide
<220>
   <221> MOD_RES
   <222> (19)..(19)
   <223> Any naturally occurring amino acid
<400> 174
   Ile Leu Trp His Glu Met Trp His Glu Gly Leu Glu Glu Ala Ser Arg
   1               5                   10                  15
   Leu Tyr Xaa Gly Glu Arg Asn Val Lys Gly Met Phe Glu Val Leu Glu
               20                  25                  30
   Pro Leu His Ala Met Met Glu Arg Gly Pro Gln Thr Leu Lys Glu Thr
           35                  40                  45
   Ser Phe Asn Gln Ala Tyr Gly Arg Asp Leu Met Glu Ala Gln Glu Trp
       50                  55                  60
   Cys Arg Lys Tyr Met Lys Ser Gly Asn Val Lys Asp Leu Thr Gln Ala
   65 70 75 80
   Trp Asp Leu Tyr Tyr His Val Phe Arg Arg Ile Ser Lys Thr Ser
   85 90 95
<210> 175
   <211> 95
   <212> PRT
   <213> Artificial Sequence
<220>
   <223> Description of Artificial Sequence: Synthetic polypeptide
<220>
   <221> MOD_RES
   <222> (20)..(20)
   <223> Any naturally occurring amino acid
<400> 175
   Ile Leu Trp His Glu Met Trp His Glu Gly Leu Glu Glu Ala Ser Arg
   1 5 10 15
   Leu Tyr Phe Xaa Glu Arg Asn Val Lys Gly Met Phe Glu Val Leu Glu
   20 25 30
   Pro Leu His Ala Met Met Glu Arg Gly Pro Gln Thr Leu Lys Glu Thr
   35 40 45
   Ser Phe Asn Gln Ala Tyr Gly Arg Asp Leu Met Glu Ala Gln Glu Trp
   50 55 60
   Cys Arg Lys Tyr Met Lys Ser Gly Asn Val Lys Asp Leu Thr Gln Ala
   65 70 75 80
   Trp Asp Leu Tyr Tyr His Val Phe Arg Arg Ile Ser Lys Thr Ser
   85 90 95
<210> 176
   <211> 95
   <212> PRT
   <213> Artificial Sequence
<220>
   <223> Description of Artificial Sequence: Synthetic polypeptide
<220>
   <221> MOD_RES
   <222> (78)..(78)
   <223> Any naturally occurring amino acid
<400> 176
   Ile Leu Trp His Glu Met Trp His Glu Gly Leu Glu Glu Ala Ser Arg
   1               5                   10                  15
   Leu Tyr Phe Gly Glu Arg Asn Val Lys Gly Met Phe Glu Val Leu Glu
               20                  25                  30
   Pro Leu His Ala Met Met Glu Arg Gly Pro Gln Thr Leu Lys Glu Thr
           35                  40                  45
   Ser Phe Asn Gln Ala Tyr Gly Arg Asp Leu Met Glu Ala Gln Glu Trp
       50                  55                  60
   Cys Arg Lys Tyr Met Lys Ser Gly Asn Val Lys Asp Leu Xaa Gln Ala
   65                  70                  75                  80
   Trp Asp Leu Tyr Tyr His Val Phe Arg Arg Ile Ser Lys Thr Ser
                   85                  90                  95
<210> 177
   <211> 95
   <212> PRT
   <213> Artificial Sequence
<220>
   <223> Description of Artificial Sequence: Synthetic polypeptide
<220>
   <221> MOD_RES
   <222> (81)..(81)
   <223> Any naturally occurring amino acid
<400> 177
   Ile Leu Trp His Glu Met Trp His Glu Gly Leu Glu Glu Ala Ser Arg
   1               5                   10                  15
   Leu Tyr Phe Gly Glu Arg Asn Val Lys Gly Met Phe Glu Val Leu Glu
               20                  25                  30
   Pro Leu His Ala Met Met Glu Arg Gly Pro Gln Thr Leu Lys Glu Thr
           35                  40                  45
   Ser Phe Asn Gln Ala Tyr Gly Arg Asp Leu Met Glu Ala Gln Glu Trp
       50                  55                  60
   Cys Arg Lys Tyr Met Lys Ser Gly Asn Val Lys Asp Leu Thr Gln Ala
   65                  70                  75                  80
   Xaa Asp Leu Tyr Tyr His Val Phe Arg Arg Ile Ser Lys Thr Ser
                   85                  90                  95
<210> 178
   <211> 95
   <212> PRT
   <213> Homo sapiens
<220>
   <221> MOD_RES
   <222> (82)..(82)
   <223> Any naturally occurring amino acid
<400> 178
   Ile Leu Trp His Glu Met Trp His Glu Gly Leu Glu Glu Ala Ser Arg
   1               5                   10                  15
   Leu Tyr Phe Gly Glu Arg Asn Val Lys Gly Met Phe Glu Val Leu Glu
               20                  25                  30
   Pro Leu His Ala Met Met Glu Arg Gly Pro Gln Thr Leu Lys Glu Thr
           35                  40                  45
   Ser Phe Asn Gln Ala Tyr Gly Arg Asp Leu Met Glu Ala Gln Glu Trp
       50                  55                  60
   Cys Arg Lys Tyr Met Lys Ser Gly Asn Val Lys Asp Leu Thr Gln Ala
   65                  70                  75                  80
   Trp Xaa Leu Tyr Tyr His Val Phe Arg Arg Ile Ser Lys Thr Ser
                   85                  90                  95
<210> 179
   <211> 95
   <212> PRT
   <213> Artificial Sequence
<220>
   <223> Description of Artificial Sequence: Synthetic polypeptide
<220>
   <221> MOD_RES
   <222> (85)..(85)
   <223> Any naturally occurring amino acid
<400> 179
   Ile Leu Trp His Glu Met Trp His Glu Gly Leu Glu Glu Ala Ser Arg
   1               5                   10                  15
   Leu Tyr Phe Gly Glu Arg Asn Val Lys Gly Met Phe Glu Val Leu Glu
               20                  25                  30
   Pro Leu His Ala Met Met Glu Arg Gly Pro Gln Thr Leu Lys Glu Thr
   35 40 45
   Ser Phe Asn Gln Ala Tyr Gly Arg Asp Leu Met Glu Ala Gln Glu Trp
   50 55 60
   Cys Arg Lys Tyr Met Lys Ser Gly Asn Val Lys Asp Leu Thr Gln Ala
   65 70 75 80
   Trp Asp Leu Tyr Xaa His Val Phe Arg Arg Ile Ser Lys Thr Ser
   85 90 95
<210> 180
   <211> 95
   <212> PRT
   <213> Artificial Sequence
<220>
   <223> Description of Artificial Sequence: Synthetic polypeptide
<220>
   <221> MOD_RES
   <222> (88)..(88)
   <223> Any naturally occurring amino acid
<400> 180
   Ile Leu Trp His Glu Met Trp His Glu Gly Leu Glu Glu Ala Ser Arg
   1 5 10 15
   Leu Tyr Phe Gly Glu Arg Asn Val Lys Gly Met Phe Glu Val Leu Glu
   20 25 30
   Pro Leu His Ala Met Met Glu Arg Gly Pro Gln Thr Leu Lys Glu Thr
   35 40 45
   Ser Phe Asn Gln Ala Tyr Gly Arg Asp Leu Met Glu Ala Gln Glu Trp
   50 55 60
   Cys Arg Lys Tyr Met Lys Ser Gly Asn Val Lys Asp Leu Thr Gln Ala
   65 70 75 80
   Trp Asp Leu Tyr Tyr His Val Xaa Arg Arg Ile Ser Lys Thr Ser
   85 90 95
<210> 181
   <211> 95
   <212> PRT
   <213> Artificial Sequence
<220>
   <223> Description of Artificial Sequence: Synthetic polypeptide
<400> 181
   Ile Leu Trp His Glu Met Trp His Glu Gly Leu Ile Glu Ala Ser Arg
   1 5 10 15
   Leu Tyr Phe Gly Glu Arg Asn Val Lys Gly Met Phe Glu Val Leu Glu
   20 25 30
   Pro Leu His Ala Met Met Glu Arg Gly Pro Gln Thr Leu Lys Glu Thr
   35 40 45
   Ser Phe Asn Gln Ala Tyr Gly Arg Asp Leu Met Glu Ala Gln Glu Trp
   50 55 60
   Cys Arg Lys Tyr Met Lys Ser Gly Asn Val Lys Asp Leu Thr Gln Ala
   65 70 75 80
   Trp Asp Leu Tyr Tyr His Val Phe Arg Arg Ile Ser Lys Thr Ser
   85 90 95
<210> 182
   <211> 95
   <212> PRT
   <213> Artificial Sequence
<220>
   <223> Description of Artificial Sequence: Synthetic polypeptide
<400> 182
   Ile Leu Trp His Glu Met Trp His Glu Gly Leu Leu Glu Ala Ser Arg
   1 5 10 15
   Leu Tyr Phe Gly Glu Arg Asn Val Lys Gly Met Phe Glu Val Leu Glu
   20 25 30
   Pro Leu His Ala Met Met Glu Arg Gly Pro Gln Thr Leu Lys Glu Thr
   35 40 45
   Ser Phe Asn Gln Ala Tyr Gly Arg Asp Leu Met Glu Ala Gln Glu Trp
   50 55 60
   Cys Arg Lys Tyr Met Lys Ser Gly Asn Val Lys Asp Leu Thr Gln Ala
   65 70 75 80
   Trp Asp Leu Tyr Tyr His Val Phe Arg Arg Ile Ser Lys Thr Ser
   85 90 95
<210> 183
   <211> 95
   <212> PRT
   <213> Artificial Sequence
<220>
   <223> Description of Artificial Sequence: Synthetic polypeptide
<400> 183
   Ile Leu Trp His Glu Met Trp His Glu Gly Leu Glu Glu Ala Ser Arg
   1               5                   10                  15
   Leu Tyr Phe Gly Glu Arg Asn Val Lys Gly Met Phe Glu Val Leu Glu
               20                  25                  30
   Pro Leu His Ala Met Met Glu Arg Gly Pro Gln Thr Leu Lys Glu Thr
           35                  40                  45
   Ser Phe Asn Gln Ala Tyr Gly Arg Asp Leu Met Glu Ala Gln Glu Trp
       50                  55                  60
   Cys Arg Lys Tyr Met Lys Ser Gly Asn Val Lys Asp Leu Leu Gln Ala
   65                  70                  75                  80
   Trp Asp Leu Tyr Tyr His Val Phe Arg Arg Ile Ser Lys Thr Ser
                   85                  90                  95
<210> 184
   <211> 95
   <212> PRT
   <213> Artificial Sequence
<220>
   <223> Description of Artificial Sequence: Synthetic polypeptide
<220>
   <221> MOD_RES
   <222> (12)..(12)
   <223> Any naturally occurring amino acid
<220>
   <221> MOD_RES
   <222> (78)..(78)
   <223> Any naturally occurring amino acid
<400> 184
   Ile Leu Trp His Glu Met Trp His Glu Gly Leu Xaa Glu Ala Ser Arg
   1               5                   10                  15
   Leu Tyr Phe Gly Glu Arg Asn Val Lys Gly Met Phe Glu Val Leu Glu
               20                  25                  30
   Pro Leu His Ala Met Met Glu Arg Gly Pro Gln Thr Leu Lys Glu Thr
           35                  40                  45
   Ser Phe Asn Gln Ala Tyr Gly Arg Asp Leu Met Glu Ala Gln Glu Trp
   50 55 60
   Cys Arg Lys Tyr Met Lys Ser Gly Asn Val Lys Asp Leu Xaa Gln Ala
   65 70 75 80
   Trp Asp Leu Tyr Tyr His Val Phe Arg Arg Ile Ser Lys Thr Ser
   85 90 95
<210> 185
   <211> 95
   <212> PRT
   <213> Artificial Sequence
<220>
   <223> Description of Artificial Sequence: Synthetic polypeptide
<400> 185
   Ile Leu Trp His Glu Met Trp His Glu Gly Leu Ile Glu Ala Ser Arg
   1 5 10 15
   Leu Tyr Phe Gly Glu Arg Asn Val Lys Gly Met Phe Glu Val Leu Glu
   20 25 30
   Pro Leu His Ala Met Met Glu Arg Gly Pro Gln Thr Leu Lys Glu Thr
   35 40 45
   Ser Phe Asn Gln Ala Tyr Gly Arg Asp Leu Met Glu Ala Gln Glu Trp
   50 55 60
   Cys Arg Lys Tyr Met Lys Ser Gly Asn Val Lys Asp Leu Leu Gln Ala
   65 70 75 80
   Trp Asp Leu Tyr Tyr His Val Phe Arg Arg Ile Ser Lys Thr Ser
   85 90 95
<210> 186
   <211> 95
   <212> PRT
   <213> Artificial Sequence
<220>
   <223> Description of Artificial Sequence: Synthetic polypeptide
<400> 186
   Ile Leu Trp His Glu Met Trp His Glu Gly Leu Leu Glu Ala Ser Arg
   1 5 10 15
   Leu Tyr Phe Gly Glu Arg Asn Val Lys Gly Met Phe Glu Val Leu Glu
   20 25 30
   Pro Leu His Ala Met Met Glu Arg Gly Pro Gln Thr Leu Lys Glu Thr
   35 40 45
   Ser Phe Asn Gln Ala Tyr Gly Arg Asp Leu Met Glu Ala Gln Glu Trp
   50 55 60
   Cys Arg Lys Tyr Met Lys Ser Gly Asn Val Lys Asp Leu Leu Gln Ala
   65 70 75 80
   Trp Asp Leu Tyr Tyr His Val Phe Arg Arg Ile Ser Lys Thr Ser
   85 90 95
<210> 187
   <211> 522
   <212> PRT
   <213> Artificial Sequence
<220>
   <223> Description of Artificial Sequence: Synthetic polypeptide
<400> 187
   Met Ala Leu Pro Val Thr Ala Leu Leu Leu Pro Leu Ala Leu Leu Leu
   1 5 10 15
   His Ala Ala Arg Pro Glu Ile Val Met Thr Gln Ser Pro Ala Thr Leu
   20 25 30
   Ser Leu Ser Pro Gly Glu Arg Ala Thr Leu Ser Cys Arg Ala Ser Gln
   35 40 45
   Asp Ile Ser Lys Tyr Leu Asn Trp Tyr Gln Gln Lys Pro Gly Gln Ala
   50 55 60
   Pro Arg Leu Leu Ile Tyr His Thr Ser Arg Leu His Ser Gly Ile Pro
   65 70 75 80
   Ala Arg Phe Ser Gly Ser Gly Ser Gly Thr Asp Tyr Thr Leu Thr Ile
   85 90 95
   Ser Ser Leu Gln Pro Glu Asp Phe Ala Val Tyr Phe Cys Gln Gln Gly
   100 105 110
   Asn Thr Leu Pro Tyr Thr Phe Gly Gln Gly Thr Lys Leu Glu Ile Lys
   115 120 125
   Gly Gly Gly Gly Ser Gly Gly Gly Gly Ser Gly Gly Gly Gly Ser Gln
   130 135 140
   Val Gln Leu Gln Glu Ser Gly Pro Gly Leu Val Lys Pro Ser Glu Thr
   145 150 155 160
   Leu Ser Leu Thr Cys Thr Val Ser Gly Val Ser Leu Pro Asp Tyr Gly
   165 170 175
   Val Ser Trp Ile Arg Gln Pro Pro Gly Lys Gly Leu Glu Trp Ile Gly
   180 185 190
   Val Ile Trp Gly Ser Glu Thr Thr Tyr Tyr Ser Ser Ser Leu Lys Ser
   195 200 205
   Arg Val Thr Ile Ser Lys Asp Asn Ser Lys Asn Gln Val Ser Leu Lys
   210 215 220
   Leu Ser Ser Val Thr Ala Ala Asp Thr Ala Val Tyr Tyr Cys Ala Lys
   225 230 235 240
   His Tyr Tyr Tyr Gly Gly Ser Tyr Ala Met Asp Tyr Trp Gly Gln Gly
   245 250 255
   Thr Leu Val Thr Val Ser Ser Thr Thr Thr Pro Ala Pro Arg Pro Pro
   260 265 270
   Thr Pro Ala Pro Thr Ile Ala Ser Gln Pro Leu Ser Leu Arg Pro Glu
   275 280 285
   Ala Cys Arg Pro Ala Ala Gly Gly Ala Val His Thr Arg Gly Leu Asp
   290 295 300
   Phe Ala Cys Asp Ile Tyr Ile Trp Ala Pro Leu Ala Gly Thr Cys Gly
   305 310 315 320
   Val Leu Leu Leu Ser Leu Val Ile Thr Leu Tyr Cys Ala Leu Tyr Leu
   325 330 335
   Leu Arg Arg Asp Gln Arg Leu Pro Pro Asp Ala His Lys Pro Pro Gly
   340 345 350
   Gly Gly Ser Phe Arg Thr Pro Ile Gln Glu Glu Gln Ala Asp Ala His
   355 360 365
   Ser Thr Leu Ala Lys Ile Arg Ser Lys Arg Ser Arg Leu Leu His Ser
   370 375 380
   Asp Tyr Met Asn Met Thr Pro Arg Arg Pro Gly Pro Thr Arg Lys His
   385 390 395 400
   Tyr Gln Pro Tyr Ala Pro Pro Arg Asp Phe Ala Ala Tyr Arg Ser Gly
   405 410 415
   Val Gln Val Glu Thr Ile Ser Pro Gly Asp Gly Arg Thr Phe Pro Lys
   420 425 430
   Arg Gly Gln Thr Cys Val Val His Tyr Thr Gly Met Leu Glu Asp Gly
   435 440 445
   Lys Lys Phe Asp Ser Ser Arg Asp Arg Asn Lys Pro Phe Lys Phe Met
   450 455 460
   Leu Gly Lys Gln Glu Val Ile Arg Gly Trp Glu Glu Gly Val Ala Gln
   465 470 475 480
   Met Ser Val Gly Gln Arg Ala Lys Leu Thr Ile Ser Pro Asp Tyr Ala
   485 490 495
   Tyr Gly Ala Thr Gly His Pro Gly Ile Ile Pro Pro His Ala Thr Leu
   500 505 510
   Val Phe Asp Val Glu Leu Leu Lys Leu Glu
   515 520
<210> 188
   <211> 508
   <212> PRT
   <213> Artificial Sequence
<220>
   <223> Description of Artificial Sequence: Synthetic polypeptide
<400> 188
   Met Ala Leu Pro Val Thr Ala Leu Leu Leu Pro Leu Ala Leu Leu Leu
   1 5 10 15
   His Ala Ala Arg Pro Glu Ile Val Met Thr Gln Ser Pro Ala Thr Leu
   20 25 30
   Ser Leu Ser Pro Gly Glu Arg Ala Thr Leu Ser Cys Arg Ala Ser Gln
   35 40 45
   Asp Ile Ser Lys Tyr Leu Asn Trp Tyr Gln Gln Lys Pro Gly Gln Ala
   50 55 60
   Pro Arg Leu Leu Ile Tyr His Thr Ser Arg Leu His Ser Gly Ile Pro
   65 70 75 80
   Ala Arg Phe Ser Gly Ser Gly Ser Gly Thr Asp Tyr Thr Leu Thr Ile
                   85                  90                  95
   Ser Ser Leu Gln Pro Glu Asp Phe Ala Val Tyr Phe Cys Gln Gln Gly
               100                 105                 110
   Asn Thr Leu Pro Tyr Thr Phe Gly Gln Gly Thr Lys Leu Glu Ile Lys
           115                 120                 125
   Gly Gly Gly Gly Ser Gly Gly Gly Gly Ser Gly Gly Gly Gly Ser Gln
       130                 135                 140
   Val Gln Leu Gln Glu Ser Gly Pro Gly Leu Val Lys Pro Ser Glu Thr
   145                 150                 155                 160
   Leu Ser Leu Thr Cys Thr Val Ser Gly Val Ser Leu Pro Asp Tyr Gly
                   165                 170                 175
   Val Ser Trp Ile Arg Gln Pro Pro Gly Lys Gly Leu Glu Trp Ile Gly
               180                 185                 190
   Val Ile Trp Gly Ser Glu Thr Thr Tyr Tyr Ser Ser Ser Leu Lys Ser
           195                 200                 205
   Arg Val Thr Ile Ser Lys Asp Asn Ser Lys Asn Gln Val Ser Leu Lys
       210                 215                 220
   Leu Ser Ser Val Thr Ala Ala Asp Thr Ala Val Tyr Tyr Cys Ala Lys
   225                 230                 235                 240
   His Tyr Tyr Tyr Gly Gly Ser Tyr Ala Met Asp Tyr Trp Gly Gln Gly
                   245                 250                 255
   Thr Leu Val Thr Val Ser Ser Thr Thr Thr Pro Ala Pro Arg Pro Pro
               260                 265                 270
   Thr Pro Ala Pro Thr Ile Ala Ser Gln Pro Leu Ser Leu Arg Pro Glu
           275                 280                 285
   Ala Cys Arg Pro Ala Ala Gly Gly Ala Val His Thr Arg Gly Leu Asp
       290                 295                 300
   Phe Ala Cys Asp Ile Tyr Ile Trp Ala Pro Leu Ala Gly Thr Cys Gly
   305                 310                 315                 320
   Val Leu Leu Leu Ser Leu Val Ile Thr Leu Tyr Cys Ala Leu Tyr Leu
                   325                 330                 335
   Leu Arg Arg Asp Gln Arg Leu Pro Pro Asp Ala His Lys Pro Pro Gly
   340 345 350
   Gly Gly Ser Phe Arg Thr Pro Ile Gln Glu Glu Gln Ala Asp Ala His
   355 360 365
   Ser Thr Leu Ala Lys Ile Arg Ser Lys Arg Ser Arg Leu Leu His Ser
   370 375 380
   Asp Tyr Met Asn Met Thr Pro Arg Arg Pro Gly Pro Thr Arg Lys His
   385 390 395 400
   Tyr Gln Pro Tyr Ala Pro Pro Arg Asp Phe Ala Ala Tyr Arg Ser Ile
   405 410 415
   Leu Trp His Glu Met Trp His Glu Gly Leu Ile Glu Ala Ser Arg Leu
   420 425 430
   Tyr Phe Gly Glu Arg Asn Val Lys Gly Met Phe Glu Val Leu Glu Pro
   435 440 445
   Leu His Ala Met Met Glu Arg Gly Pro Gln Thr Leu Lys Glu Thr Ser
   450 455 460
   Phe Asn Gln Ala Tyr Gly Arg Asp Leu Met Glu Ala Gln Glu Trp Cys
   465 470 475 480
   Arg Lys Tyr Met Lys Ser Gly Asn Val Lys Asp Leu Leu Gln Ala Trp
   485 490 495
   Asp Leu Tyr Tyr His Val Phe Arg Arg Ile Ser Lys
   500 505
<210> 189
   <211> 528
   <212> PRT
   <213> Artificial Sequence
<220>
   <223> Description of Artificial Sequence: Synthetic polypeptide
<400> 189
   Met Ala Leu Pro Val Thr Ala Leu Leu Leu Pro Leu Ala Leu Leu Leu
   1 5 10 15
   His Ala Ala Arg Pro Glu Ile Val Met Thr Gln Ser Pro Ala Thr Leu
   20 25 30
   Ser Leu Ser Pro Gly Glu Arg Ala Thr Leu Ser Cys Arg Ala Ser Gln
   35 40 45
   Asp Ile Ser Lys Tyr Leu Asn Trp Tyr Gln Gln Lys Pro Gly Gln Ala
   50 55 60
   Pro Arg Leu Leu Ile Tyr His Thr Ser Arg Leu His Ser Gly Ile Pro
   65 70 75 80
   Ala Arg Phe Ser Gly Ser Gly Ser Gly Thr Asp Tyr Thr Leu Thr Ile
   85 90 95
   Ser Ser Leu Gln Pro Glu Asp Phe Ala Val Tyr Phe Cys Gln Gln Gly
   100 105 110
   Asn Thr Leu Pro Tyr Thr Phe Gly Gln Gly Thr Lys Leu Glu Ile Lys
   115 120 125
   Gly Gly Gly Gly Ser Gly Gly Gly Gly Ser Gly Gly Gly Gly Ser Gln
   130 135 140
   Val Gln Leu Gln Glu Ser Gly Pro Gly Leu Val Lys Pro Ser Glu Thr
   145 150 155 160
   Leu Ser Leu Thr Cys Thr Val Ser Gly Val Ser Leu Pro Asp Tyr Gly
   165 170 175
   Val Ser Trp Ile Arg Gln Pro Pro Gly Lys Gly Leu Glu Trp Ile Gly
   180 185 190
   Val Ile Trp Gly Ser Glu Thr Thr Tyr Tyr Ser Ser Ser Leu Lys Ser
   195 200 205
   Arg Val Thr Ile Ser Lys Asp Asn Ser Lys Asn Gln Val Ser Leu Lys
   210 215 220
   Leu Ser Ser Val Thr Ala Ala Asp Thr Ala Val Tyr Tyr Cys Ala Lys
   225 230 235 240
   His Tyr Tyr Tyr Gly Gly Ser Tyr Ala Met Asp Tyr Trp Gly Gln Gly
   245 250 255
   Thr Leu Val Thr Val Ser Ser Thr Thr Thr Pro Ala Pro Arg Pro Pro
   260 265 270
   Thr Pro Ala Pro Thr Ile Ala Ser Gln Pro Leu Ser Leu Arg Pro Glu
   275 280 285
   Ala Cys Arg Pro Ala Ala Gly Gly Ala Val His Thr Arg Gly Leu Asp
       290                 295                 300
   Phe Ala Cys Asp Ile Tyr Ile Trp Ala Pro Leu Ala Gly Thr Cys Gly
   305                 310                 315                 320
   Val Leu Leu Leu Ser Leu Val Ile Thr Leu Tyr Cys Gln Arg Arg Lys
                   325                 330                 335
   Tyr Arg Ser Asn Lys Gly Glu Ser Pro Val Glu Pro Ala Glu Pro Cys
               340                 345                 350
   His Tyr Ser Cys Pro Arg Glu Glu Glu Gly Ser Thr Ile Pro Ile Gln
           355                 360                 365
   Glu Asp Tyr Arg Lys Pro Glu Pro Ala Cys Ser Pro Arg Ser Lys Arg
       370                 375                 380
   Ser Arg Leu Leu His Ser Asp Tyr Met Asn Met Thr Pro Arg Arg Pro
   385                 390                 395                 400
   Gly Pro Thr Arg Lys His Tyr Gln Pro Tyr Ala Pro Pro Arg Asp Phe
                   405                 410                 415
   Ala Ala Tyr Arg Ser Gly Val Gln Val Glu Thr Ile Ser Pro Gly Asp
               420                 425                 430
   Gly Arg Thr Phe Pro Lys Arg Gly Gln Thr Cys Val Val His Tyr Thr
           435                 440                 445
   Gly Met Leu Glu Asp Gly Lys Lys Phe Asp Ser Ser Arg Asp Arg Asn
       450                 455                 460
   Lys Pro Phe Lys Phe Met Leu Gly Lys Gln Glu Val Ile Arg Gly Trp
   465                 470                 475                 480
   Glu Glu Gly Val Ala Gln Met Ser Val Gly Gln Arg Ala Lys Leu Thr
                   485                 490                 495
   Ile Ser Pro Asp Tyr Ala Tyr Gly Ala Thr Gly His Pro Gly Ile Ile
               500                 505                 510
   Pro Pro His Ala Thr Leu Val Phe Asp Val Glu Leu Leu Lys Leu Glu
           515                 520                 525
<210> 190
   <211> 514
   <212> PRT
   <213> Artificial Sequence
<220>
   <223> Description of Artificial Sequence: Synthetic polypeptide
<400> 190
   Met Ala Leu Pro Val Thr Ala Leu Leu Leu Pro Leu Ala Leu Leu Leu
   1               5                   10                  15
   His Ala Ala Arg Pro Glu Ile Val Met Thr Gln Ser Pro Ala Thr Leu
               20                  25                  30
   Ser Leu Ser Pro Gly Glu Arg Ala Thr Leu Ser Cys Arg Ala Ser Gln
           35                  40                  45
   Asp Ile Ser Lys Tyr Leu Asn Trp Tyr Gln Gln Lys Pro Gly Gln Ala
       50                  55                  60
   Pro Arg Leu Leu Ile Tyr His Thr Ser Arg Leu His Ser Gly Ile Pro
   65                  70                  75                  80
   Ala Arg Phe Ser Gly Ser Gly Ser Gly Thr Asp Tyr Thr Leu Thr Ile
                   85                  90                  95
   Ser Ser Leu Gln Pro Glu Asp Phe Ala Val Tyr Phe Cys Gln Gln Gly
               100                 105                 110
   Asn Thr Leu Pro Tyr Thr Phe Gly Gln Gly Thr Lys Leu Glu Ile Lys
           115                 120                 125
   Gly Gly Gly Gly Ser Gly Gly Gly Gly Ser Gly Gly Gly Gly Ser Gln
       130                 135                 140
   Val Gln Leu Gln Glu Ser Gly Pro Gly Leu Val Lys Pro Ser Glu Thr
   145                 150                 155                 160
   Leu Ser Leu Thr Cys Thr Val Ser Gly Val Ser Leu Pro Asp Tyr Gly
                   165                 170                 175
   Val Ser Trp Ile Arg Gln Pro Pro Gly Lys Gly Leu Glu Trp Ile Gly
               180                 185                 190
   Val Ile Trp Gly Ser Glu Thr Thr Tyr Tyr Ser Ser Ser Leu Lys Ser
           195                 200                 205
   Arg Val Thr Ile Ser Lys Asp Asn Ser Lys Asn Gln Val Ser Leu Lys
       210                 215                 220
   Leu Ser Ser Val Thr Ala Ala Asp Thr Ala Val Tyr Tyr Cys Ala Lys
   225 230 235 240
   His Tyr Tyr Tyr Gly Gly Ser Tyr Ala Met Asp Tyr Trp Gly Gln Gly
   245 250 255
   Thr Leu Val Thr Val Ser Ser Thr Thr Thr Pro Ala Pro Arg Pro Pro
   260 265 270
   Thr Pro Ala Pro Thr Ile Ala Ser Gln Pro Leu Ser Leu Arg Pro Glu
   275 280 285
   Ala Cys Arg Pro Ala Ala Gly Gly Ala Val His Thr Arg Gly Leu Asp
   290 295 300
   Phe Ala Cys Asp Ile Tyr Ile Trp Ala Pro Leu Ala Gly Thr Cys Gly
   305 310 315 320
   Val Leu Leu Leu Ser Leu Val Ile Thr Leu Tyr Cys Gln Arg Arg Lys
   325 330 335
   Tyr Arg Ser Asn Lys Gly Glu Ser Pro Val Glu Pro Ala Glu Pro Cys
   340 345 350
   His Tyr Ser Cys Pro Arg Glu Glu Glu Gly Ser Thr Ile Pro Ile Gln
   355 360 365
   Glu Asp Tyr Arg Lys Pro Glu Pro Ala Cys Ser Pro Arg Ser Lys Arg
   370 375 380
   Ser Arg Leu Leu His Ser Asp Tyr Met Asn Met Thr Pro Arg Arg Pro
   385 390 395 400
   Gly Pro Thr Arg Lys His Tyr Gln Pro Tyr Ala Pro Pro Arg Asp Phe
   405 410 415
   Ala Ala Tyr Arg Ser Ile Leu Trp His Glu Met Trp His Glu Gly Leu
   420 425 430
   Ile Glu Ala Ser Arg Leu Tyr Phe Gly Glu Arg Asn Val Lys Gly Met
   435 440 445
   Phe Glu Val Leu Glu Pro Leu His Ala Met Met Glu Arg Gly Pro Gln
   450 455 460
   Thr Leu Lys Glu Thr Ser Phe Asn Gln Ala Tyr Gly Arg Asp Leu Met
   465 470 475 480
   Glu Ala Gln Glu Trp Cys Arg Lys Tyr Met Lys Ser Gly Asn Val Lys
   485 490 495
   Asp Leu Leu Gln Ala Trp Asp Leu Tyr Tyr His Val Phe Arg Arg Ile
   500 505 510
   Ser Lys
<210> 191
   <211> 522
   <212> PRT
   <213> Artificial Sequence
<220>
   <223> Description of Artificial Sequence: Synthetic polypeptide
<400> 191
   Met Ala Leu Pro Val Thr Ala Leu Leu Leu Pro Leu Ala Leu Leu Leu
   1 5 10 15
   His Ala Ala Arg Pro Glu Ile Val Met Thr Gln Ser Pro Ala Thr Leu
   20 25 30
   Ser Leu Ser Pro Gly Glu Arg Ala Thr Leu Ser Cys Arg Ala Ser Gln
   35 40 45
   Asp Ile Ser Lys Tyr Leu Asn Trp Tyr Gln Gln Lys Pro Gly Gln Ala
   50 55 60
   Pro Arg Leu Leu Ile Tyr His Thr Ser Arg Leu His Ser Gly Ile Pro
   65 70 75 80
   Ala Arg Phe Ser Gly Ser Gly Ser Gly Thr Asp Tyr Thr Leu Thr Ile
   85 90 95
   Ser Ser Leu Gln Pro Glu Asp Phe Ala Val Tyr Phe Cys Gln Gln Gly
   100 105 110
   Asn Thr Leu Pro Tyr Thr Phe Gly Gln Gly Thr Lys Leu Glu Ile Lys
   115 120 125
   Gly Gly Gly Gly Ser Gly Gly Gly Gly Ser Gly Gly Gly Gly Ser Gln
   130 135 140
   Val Gln Leu Gln Glu Ser Gly Pro Gly Leu Val Lys Pro Ser Glu Thr
   145 150 155 160
   Leu Ser Leu Thr Cys Thr Val Ser Gly Val Ser Leu Pro Asp Tyr Gly
                   165                 170                 175
   Val Ser Trp Ile Arg Gln Pro Pro Gly Lys Gly Leu Glu Trp Ile Gly
               180                 185                 190
   Val Ile Trp Gly Ser Glu Thr Thr Tyr Tyr Ser Ser Ser Leu Lys Ser
           195                 200                 205
   Arg Val Thr Ile Ser Lys Asp Asn Ser Lys Asn Gln Val Ser Leu Lys
       210                 215                 220
   Leu Ser Ser Val Thr Ala Ala Asp Thr Ala Val Tyr Tyr Cys Ala Lys
   225                 230                 235                 240
   His Tyr Tyr Tyr Gly Gly Ser Tyr Ala Met Asp Tyr Trp Gly Gln Gly
                   245                 250                 255
   Thr Leu Val Thr Val Ser Ser Thr Thr Thr Pro Ala Pro Arg Pro Pro
               260                 265                 270
   Thr Pro Ala Pro Thr Ile Ala Ser Gln Pro Leu Ser Leu Arg Pro Glu
           275                 280                 285
   Ala Cys Arg Pro Ala Ala Gly Gly Ala Val His Thr Arg Gly Leu Asp
       290                 295                 300
   Phe Ala Cys Asp Ile Tyr Ile Trp Ala Pro Leu Ala Gly Thr Cys Gly
   305                 310                 315                 320
   Val Leu Leu Leu Ser Leu Val Ile Thr Leu Tyr Cys Lys Arg Gly Arg
                   325                 330                 335
   Lys Lys Leu Leu Tyr Ile Phe Lys Gln Pro Phe Met Arg Pro Val Gln
               340                 345                 350
   Thr Thr Gln Glu Glu Asp Gly Cys Ser Cys Arg Phe Pro Glu Glu Glu
           355                 360                 365
   Glu Gly Gly Cys Glu Leu Arg Ser Lys Arg Ser Arg Leu Leu His Ser
       370                 375                 380
   Asp Tyr Met Asn Met Thr Pro Arg Arg Pro Gly Pro Thr Arg Lys His
   385                 390                 395                 400
   Tyr Gln Pro Tyr Ala Pro Pro Arg Asp Phe Ala Ala Tyr Arg Ser Gly
                   405                 410                 415
   Val Gln Val Glu Thr Ile Ser Pro Gly Asp Gly Arg Thr Phe Pro Lys
               420                 425                 430
   Arg Gly Gln Thr Cys Val Val His Tyr Thr Gly Met Leu Glu Asp Gly
           435                 440                 445
   Lys Lys Phe Asp Ser Ser Arg Asp Arg Asn Lys Pro Phe Lys Phe Met
       450                 455                 460
   Leu Gly Lys Gln Glu Val Ile Arg Gly Trp Glu Glu Gly Val Ala Gln
   465                 470                 475                 480
   Met Ser Val Gly Gln Arg Ala Lys Leu Thr Ile Ser Pro Asp Tyr Ala
                   485                 490                 495
   Tyr Gly Ala Thr Gly His Pro Gly Ile Ile Pro Pro His Ala Thr Leu
               500                 505                 510
   Val Phe Asp Val Glu Leu Leu Lys Leu Glu
           515                 520
<210> 192
   <211> 508
   <212> PRT
   <213> Artificial Sequence
<220>
   <223> Description of Artificial Sequence: Synthetic polypeptide
<400> 192
   Met Ala Leu Pro Val Thr Ala Leu Leu Leu Pro Leu Ala Leu Leu Leu
   1               5                   10                  15
   His Ala Ala Arg Pro Glu Ile Val Met Thr Gln Ser Pro Ala Thr Leu
               20                  25                  30
   Ser Leu Ser Pro Gly Glu Arg Ala Thr Leu Ser Cys Arg Ala Ser Gln
           35                  40                  45
   Asp Ile Ser Lys Tyr Leu Asn Trp Tyr Gln Gln Lys Pro Gly Gln Ala
       50                  55                  60
   Pro Arg Leu Leu Ile Tyr His Thr Ser Arg Leu His Ser Gly Ile Pro
   65                  70                  75                  80
   Ala Arg Phe Ser Gly Ser Gly Ser Gly Thr Asp Tyr Thr Leu Thr Ile
                   85                  90                  95
   Ser Ser Leu Gln Pro Glu Asp Phe Ala Val Tyr Phe Cys Gln Gln Gly
   100 105 110
   Asn Thr Leu Pro Tyr Thr Phe Gly Gln Gly Thr Lys Leu Glu Ile Lys
   115 120 125
   Gly Gly Gly Gly Ser Gly Gly Gly Gly Ser Gly Gly Gly Gly Ser Gln
   130 135 140
   Val Gln Leu Gln Glu Ser Gly Pro Gly Leu Val Lys Pro Ser Glu Thr
   145 150 155 160
   Leu Ser Leu Thr Cys Thr Val Ser Gly Val Ser Leu Pro Asp Tyr Gly
   165 170 175
   Val Ser Trp Ile Arg Gln Pro Pro Gly Lys Gly Leu Glu Trp Ile Gly
   180 185 190
   Val Ile Trp Gly Ser Glu Thr Thr Tyr Tyr Ser Ser Ser Leu Lys Ser
   195 200 205
   Arg Val Thr Ile Ser Lys Asp Asn Ser Lys Asn Gln Val Ser Leu Lys
   210 215 220
   Leu Ser Ser Val Thr Ala Ala Asp Thr Ala Val Tyr Tyr Cys Ala Lys
   225 230 235 240
   His Tyr Tyr Tyr Gly Gly Ser Tyr Ala Met Asp Tyr Trp Gly Gln Gly
   245 250 255
   Thr Leu Val Thr Val Ser Ser Thr Thr Thr Pro Ala Pro Arg Pro Pro
   260 265 270
   Thr Pro Ala Pro Thr Ile Ala Ser Gln Pro Leu Ser Leu Arg Pro Glu
   275 280 285
   Ala Cys Arg Pro Ala Ala Gly Gly Ala Val His Thr Arg Gly Leu Asp
   290 295 300
   Phe Ala Cys Asp Ile Tyr Ile Trp Ala Pro Leu Ala Gly Thr Cys Gly
   305 310 315 320
   Val Leu Leu Leu Ser Leu Val Ile Thr Leu Tyr Cys Lys Arg Gly Arg
   325 330 335
   Lys Lys Leu Leu Tyr Ile Phe Lys Gln Pro Phe Met Arg Pro Val Gln
   340 345 350
   Thr Thr Gln Glu Glu Asp Gly Cys Ser Cys Arg Phe Pro Glu Glu Glu
   355 360 365
   Glu Gly Gly Cys Glu Leu Arg Ser Lys Arg Ser Arg Leu Leu His Ser
   370 375 380
   Asp Tyr Met Asn Met Thr Pro Arg Arg Pro Gly Pro Thr Arg Lys His
   385 390 395 400
   Tyr Gln Pro Tyr Ala Pro Pro Arg Asp Phe Ala Ala Tyr Arg Ser Ile
   405 410 415
   Leu Trp His Glu Met Trp His Glu Gly Leu Ile Glu Ala Ser Arg Leu
   420 425 430
   Tyr Phe Gly Glu Arg Asn Val Lys Gly Met Phe Glu Val Leu Glu Pro
   435 440 445
   Leu His Ala Met Met Glu Arg Gly Pro Gln Thr Leu Lys Glu Thr Ser
   450 455 460
   Phe Asn Gln Ala Tyr Gly Arg Asp Leu Met Glu Ala Gln Glu Trp Cys
   465 470 475 480
   Arg Lys Tyr Met Lys Ser Gly Asn Val Lys Asp Leu Leu Gln Ala Trp
   485 490 495
   Asp Leu Tyr Tyr His Val Phe Arg Arg Ile Ser Lys
   500 505
<210> 193
   <211> 206
   <212> PRT
   <213> Artificial Sequence
<220>
   <223> Description of Artificial Sequence: Synthetic polypeptide
<400> 193
   Met Ile Leu Trp His Glu Met Trp His Glu Gly Leu Ile Glu Ala Ser
   1               5                   10                  15
   Arg Leu Tyr Phe Gly Glu Arg Asn Val Lys Gly Met Phe Glu Val Leu
               20                  25                  30
   Glu Pro Leu His Ala Met Met Glu Arg Gly Pro Gln Thr Leu Lys Glu
           35                  40                  45
   Thr Ser Phe Asn Gln Ala Tyr Gly Arg Asp Leu Met Glu Ala Gln Glu
       50                  55                  60
   Trp Cys Arg Lys Tyr Met Lys Ser Gly Asn Val Lys Asp Leu Leu Gln
   65                  70                  75                  80
   Ala Trp Asp Leu Tyr Tyr His Val Phe Arg Arg Ile Ser Lys Arg Val
                   85                  90                  95
   Lys Phe Ser Arg Ser Ala Asp Ala Pro Ala Tyr Lys Gln Gly Gln Asn
               100                 105                 110
   Gln Leu Tyr Asn Glu Leu Asn Leu Gly Arg Arg Glu Glu Tyr Asp Val
           115                 120                 125
   Leu Asp Lys Arg Arg Gly Arg Asp Pro Glu Met Gly Gly Lys Pro Arg
       130                 135                 140
   Arg Lys Asn Pro Gln Glu Gly Leu Tyr Asn Glu Leu Gln Lys Asp Lys
   145                 150                 155                 160
   Met Ala Glu Ala Tyr Ser Glu Ile Gly Met Lys Gly Glu Arg Arg Arg
                   165                 170                 175
   Gly Lys Gly His Asp Gly Leu Tyr Gln Gly Leu Ser Thr Ala Thr Lys
               180                 185                 190
   Asp Thr Tyr Asp Ala Leu His Met Gln Ala Leu Pro Pro Arg
           195                 200                 205
<210> 194
   <211> 225
   <212> PRT
   <213> Artificial Sequence
<220>
   <223> Description of Artificial Sequence: Synthetic polypeptide
<400> 194
   Met Gly Val Gln Val Glu Thr Ile Ser Pro Gly Asp Gly Arg Thr Phe
   1               5                   10                  15
   Pro Lys Arg Gly Gln Thr Cys Val Val His Tyr Thr Gly Met Leu Glu
               20                  25                  30
   Asp Gly Lys Lys Phe Asp Ser Ser Arg Asp Arg Asn Lys Pro Phe Lys
           35                  40                  45
   Phe Met Leu Gly Lys Gln Glu Val Ile Arg Gly Trp Glu Glu Gly Val
       50                  55                  60
   Ala Gln Met Ser Val Gly Gln Arg Ala Lys Leu Thr Ile Ser Pro Asp
   65                  70                  75                  80
   Tyr Ala Tyr Gly Ala Thr Gly His Pro Gly Ile Ile Pro Pro His Ala
                   85                  90                  95
   Thr Leu Val Phe Asp Val Glu Leu Leu Lys Leu Glu Gly Gly Gly Gly
               100                 105                 110
   Ser Arg Val Lys Phe Ser Arg Ser Ala Asp Ala Pro Ala Tyr Lys Gln
           115                 120                 125
   Gly Gln Asn Gln Leu Tyr Asn Glu Leu Asn Leu Gly Arg Arg Glu Glu
       130                 135                 140
   Tyr Asp Val Leu Asp Lys Arg Arg Gly Arg Asp Pro Glu Met Gly Gly
   145                 150                 155                 160
   Lys Pro Arg Arg Lys Asn Pro Gln Glu Gly Leu Tyr Asn Glu Leu Gln
                   165                 170                 175
   Lys Asp Lys Met Ala Glu Ala Tyr Ser Glu Ile Gly Met Lys Gly Glu
               180                 185                 190
   Arg Arg Arg Gly Lys Gly His Asp Gly Leu Tyr Gln Gly Leu Ser Thr
           195                 200                 205
   Ala Thr Lys Asp Thr Tyr Asp Ala Leu His Met Gln Ala Leu Pro Pro
       210                 215                 220
   Arg
   225
<210> 195
   <211> 110
   <212> PRT
   <213> Artificial Sequence
<220>
   <223> Description of Artificial Sequence: Synthetic polypeptide
<220>
   <221> MOD_RES
   <222> (26)..(26)
   <223> Any amino acid
<400> 195
   Met Gly His His His His His His His His Gly Ser Ala Ser Arg Ile
   1               5                   10                  15
   Leu Trp His Glu Met Trp His Glu Gly Xaa Glu Glu Ala Ser Arg Leu
               20                  25                  30
   Tyr Phe Gly Glu Arg Asn Val Lys Gly Met Phe Glu Val Leu Glu Pro
           35                  40                  45
   Leu His Ala Met Met Glu Arg Gly Pro Gln Thr Leu Lys Glu Thr Ser
       50                  55                  60
   Phe Asn Gln Ala Tyr Gly Arg Asp Leu Met Glu Ala Gln Glu Trp Cys
   65                  70                  75                  80
   Arg Lys Tyr Met Lys Ser Gly Asn Val Lys Asp Leu Thr Gln Ala Trp
                   85                  90                  95
   Asp Leu Tyr Tyr His Val Phe Arg Arg Ile Ser Lys Thr Ser
               100                 105                 110
<210> 196
   <211> 110
   <212> PRT
   <213> Artificial Sequence
<220>
   <223> Description of Artificial Sequence: Synthetic polypeptide
<220>
   <221> MOD_RES
   <222> (27)..(27)
   <223> Any amino acid
<400> 196
   Met Gly His His His His His His His His Gly Ser Ala Ser Arg Ile
   1               5                   10                  15
   Leu Trp His Glu Met Trp His Glu Gly Leu Xaa Glu Ala Ser Arg Leu
               20                  25                  30
   Tyr Phe Gly Glu Arg Asn Val Lys Gly Met Phe Glu Val Leu Glu Pro
           35                  40                  45
   Leu His Ala Met Met Glu Arg Gly Pro Gln Thr Leu Lys Glu Thr Ser
       50                  55                  60
   Phe Asn Gln Ala Tyr Gly Arg Asp Leu Met Glu Ala Gln Glu Trp Cys
   65                  70                  75                  80
   Arg Lys Tyr Met Lys Ser Gly Asn Val Lys Asp Leu Thr Gln Ala Trp
                   85                  90                  95
   Asp Leu Tyr Tyr His Val Phe Arg Arg Ile Ser Lys Thr Ser
               100                 105                 110
<210> 197
   <211> 110
   <212> PRT
   <213> Artificial Sequence
<220>
   <223> Description of Artificial Sequence: Synthetic polypeptide
<220>
   <221> MOD_RES
   <222> (30)..(30)
   <223> Any amino acid
<400> 197
   Met Gly His His His His His His His His Gly Ser Ala Ser Arg Ile
   1               5                   10                  15
   Leu Trp His Glu Met Trp His Glu Gly Leu Glu Glu Ala Xaa Arg Leu
               20                  25                  30
   Tyr Phe Gly Glu Arg Asn Val Lys Gly Met Phe Glu Val Leu Glu Pro
           35                  40                  45
   Leu His Ala Met Met Glu Arg Gly Pro Gln Thr Leu Lys Glu Thr Ser
       50                  55                  60
   Phe Asn Gln Ala Tyr Gly Arg Asp Leu Met Glu Ala Gln Glu Trp Cys
   65                  70                  75                  80
   Arg Lys Tyr Met Lys Ser Gly Asn Val Lys Asp Leu Thr Gln Ala Trp
                   85                  90                  95
   Asp Leu Tyr Tyr His Val Phe Arg Arg Ile Ser Lys Thr Ser
               100                 105                 110
<210> 198
   <211> 110
   <212> PRT
   <213> Artificial Sequence
<220>
   <223> Description of Artificial Sequence: Synthetic polypeptide
<220>
   <221> MOD_RES
   <222> (31)..(31)
   <223> Any amino acid
<400> 198
   Met Gly His His His His His His His His Gly Ser Ala Ser Arg Ile
   1               5                   10                  15
   Leu Trp His Glu Met Trp His Glu Gly Leu Glu Glu Ala Ser Xaa Leu
               20                  25                  30
   Tyr Phe Gly Glu Arg Asn Val Lys Gly Met Phe Glu Val Leu Glu Pro
           35                  40                  45
   Leu His Ala Met Met Glu Arg Gly Pro Gln Thr Leu Lys Glu Thr Ser
       50                  55                  60
   Phe Asn Gln Ala Tyr Gly Arg Asp Leu Met Glu Ala Gln Glu Trp Cys
   65                  70                  75                  80
   Arg Lys Tyr Met Lys Ser Gly Asn Val Lys Asp Leu Thr Gln Ala Trp
                   85                  90                  95
   Asp Leu Tyr Tyr His Val Phe Arg Arg Ile Ser Lys Thr Ser
               100                 105                 110
<210> 199
   <211> 110
   <212> PRT
   <213> Artificial Sequence
<220>
   <223> Description of Artificial Sequence: Synthetic polypeptide
<220>
   <221> MOD_RES
   <222> (34)..(34)
   <223> Any amino acid
<400> 199
   Met Gly His His His His His His His His Gly Ser Ala Ser Arg Ile
   1               5                   10                  15
   Leu Trp His Glu Met Trp His Glu Gly Leu Glu Glu Ala Ser Arg Leu
               20                  25                  30
   Tyr Xaa Gly Glu Arg Asn Val Lys Gly Met Phe Glu Val Leu Glu Pro
           35                  40                  45
   Leu His Ala Met Met Glu Arg Gly Pro Gln Thr Leu Lys Glu Thr Ser
   50 55 60
   Phe Asn Gln Ala Tyr Gly Arg Asp Leu Met Glu Ala Gln Glu Trp Cys
   65 70 75 80
   Arg Lys Tyr Met Lys Ser Gly Asn Val Lys Asp Leu Thr Gln Ala Trp
   85 90 95
   Asp Leu Tyr Tyr His Val Phe Arg Arg Ile Ser Lys Thr Ser
   100 105 110
<210> 200
   <211> 110
   <212> PRT
   <213> Artificial Sequence
<220>
   <223> Description of Artificial Sequence: Synthetic polypeptide
<220>
   <221> MOD_RES
   <222> (35)..(35)
   <223> Any amino acid
<400> 200
   Met Gly His His His His His His His His Gly Ser Ala Ser Arg Ile
   1 5 10 15
   Leu Trp His Glu Met Trp His Glu Gly Leu Glu Glu Ala Ser Arg Leu
   20 25 30
   Tyr Phe Xaa Glu Arg Asn Val Lys Gly Met Phe Glu Val Leu Glu Pro
   35 40 45
   Leu His Ala Met Met Glu Arg Gly Pro Gln Thr Leu Lys Glu Thr Ser
   50 55 60
   Phe Asn Gln Ala Tyr Gly Arg Asp Leu Met Glu Ala Gln Glu Trp Cys
   65 70 75 80
   Arg Lys Tyr Met Lys Ser Gly Asn Val Lys Asp Leu Thr Gln Ala Trp
   85 90 95
   Asp Leu Tyr Tyr His Val Phe Arg Arg Ile Ser Lys Thr Ser
   100 105 110
<210> 201
   <211> 110
   <212> PRT
   <213> Artificial Sequence
<220>
   <223> Description of Artificial Sequence: Synthetic polypeptide
<220>
   <221> MOD_RES
   <222> (93)..(93)
   <223> Any amino acid
<400> 201
   Met Gly His His His His His His His His Gly Ser Ala Ser Arg Ile
   1               5                   10                  15
   Leu Trp His Glu Met Trp His Glu Gly Leu Glu Glu Ala Ser Arg Leu
               20                  25                  30
   Tyr Phe Gly Glu Arg Asn Val Lys Gly Met Phe Glu Val Leu Glu Pro
           35                  40                  45
   Leu His Ala Met Met Glu Arg Gly Pro Gln Thr Leu Lys Glu Thr Ser
       50                  55                  60
   Phe Asn Gln Ala Tyr Gly Arg Asp Leu Met Glu Ala Gln Glu Trp Cys
   65                  70                  75                  80
   Arg Lys Tyr Met Lys Ser Gly Asn Val Lys Asp Leu Xaa Gln Ala Trp
                   85                  90                  95
   Asp Leu Tyr Tyr His Val Phe Arg Arg Ile Ser Lys Thr Ser
               100                 105                 110
<210> 202
   <211> 110
   <212> PRT
   <213> Artificial Sequence
<220>
   <223> Description of Artificial Sequence: Synthetic polypeptide
<220>
   <221> MOD_RES
   <222> (96)..(96)
   <223> Any amino acid
<400> 202
   Met Gly His His His His His His His His Gly Ser Ala Ser Arg Ile
   1               5                   10                  15
   Leu Trp His Glu Met Trp His Glu Gly Leu Glu Glu Ala Ser Arg Leu
                   20                  25                  30
   Tyr Phe Gly Glu Arg Asn Val Lys Gly Met Phe Glu Val Leu Glu Pro
           35                  40                  45
   Leu His Ala Met Met Glu Arg Gly Pro Gln Thr Leu Lys Glu Thr Ser
       50                  55                  60
   Phe Asn Gln Ala Tyr Gly Arg Asp Leu Met Glu Ala Gln Glu Trp Cys
   65                  70                  75                  80
   Arg Lys Tyr Met Lys Ser Gly Asn Val Lys Asp Leu Thr Gln Ala Xaa
                   85                  90                  95
   Asp Leu Tyr Tyr His Val Phe Arg Arg Ile Ser Lys Thr Ser
               100                 105                 110
<210> 203
   <211> 110
   <212> PRT
   <213> Artificial Sequence
<220>
   <223> Description of Artificial Sequence: Synthetic polypeptide
<220>
   <221> MOD_RES
   <222> (97)..(97)
   <223> Any amino acid
<400> 203
   Met Gly His His His His His His His His Gly Ser Ala Ser Arg Ile
   1               5                   10                  15
   Leu Trp His Glu Met Trp His Glu Gly Leu Glu Glu Ala Ser Arg Leu
               20                  25                  30
   Tyr Phe Gly Glu Arg Asn Val Lys Gly Met Phe Glu Val Leu Glu Pro
           35                  40                  45
   Leu His Ala Met Met Glu Arg Gly Pro Gln Thr Leu Lys Glu Thr Ser
       50                  55                  60
   Phe Asn Gln Ala Tyr Gly Arg Asp Leu Met Glu Ala Gln Glu Trp Cys
   65                  70                  75                  80
   Arg Lys Tyr Met Lys Ser Gly Asn Val Lys Asp Leu Thr Gln Ala Trp
                   85                  90                  95
   Xaa Leu Tyr Tyr His Val Phe Arg Arg Ile Ser Lys Thr Ser
   100 105 110
<210> 204
   <211> 110
   <212> PRT
   <213> Artificial Sequence
<220>
   <223> Description of Artificial Sequence: Synthetic polypeptide
<220>
   <221> MOD_RES
   <222> (100)..(100)
   <223> Any amino acid
<400> 204
   Met Gly His His His His His His His His Gly Ser Ala Ser Arg Ile
   1 5 10 15
   Leu Trp His Glu Met Trp His Glu Gly Leu Glu Glu Ala Ser Arg Leu
   20 25 30
   Tyr Phe Gly Glu Arg Asn Val Lys Gly Met Phe Glu Val Leu Glu Pro
   35 40 45
   Leu His Ala Met Met Glu Arg Gly Pro Gln Thr Leu Lys Glu Thr Ser
   50 55 60
   Phe Asn Gln Ala Tyr Gly Arg Asp Leu Met Glu Ala Gln Glu Trp Cys
   65 70 75 80
   Arg Lys Tyr Met Lys Ser Gly Asn Val Lys Asp Leu Thr Gln Ala Trp
   85 90 95
   Asp Leu Tyr Xaa His Val Phe Arg Arg Ile Ser Lys Thr Ser
   100 105 110
<210> 205
   <211> 110
   <212> PRT
   <213> Artificial Sequence
<220>
   <223> Description of Artificial Sequence: Synthetic polypeptide
<220>
   <221> MOD_RES
   <222> (103)..(103)
   <223> Any amino acid
<400> 205
   Met Gly His His His His His His His His Gly Ser Ala Ser Arg Ile
   1 5 10 15
   Leu Trp His Glu Met Trp His Glu Gly Leu Glu Glu Ala Ser Arg Leu
   20 25 30
   Tyr Phe Gly Glu Arg Asn Val Lys Gly Met Phe Glu Val Leu Glu Pro
   35 40 45
   Leu His Ala Met Met Glu Arg Gly Pro Gln Thr Leu Lys Glu Thr Ser
   50 55 60
   Phe Asn Gln Ala Tyr Gly Arg Asp Leu Met Glu Ala Gln Glu Trp Cys
   65 70 75 80
   Arg Lys Tyr Met Lys Ser Gly Asn Val Lys Asp Leu Thr Gln Ala Trp
   85 90 95
   Asp Leu Tyr Tyr His Val Xaa Arg Arg Ile Ser Lys Thr Ser
   100 105 110
<210> 206
   <211> 110
   <212> PRT
   <213> Artificial Sequence
<220>
   <223> Description of Artificial Sequence: Synthetic polypeptide
<400> 206
   Met Gly His His His His His His His His Gly Ser Ala Ser Arg Ile
   1 5 10 15
   Leu Trp His Glu Met Trp His Glu Gly Leu Glu Glu Ala Ser Arg Leu
   20 25 30
   Tyr Phe Gly Glu Arg Asn Val Lys Gly Met Phe Glu Val Leu Glu Pro
   35 40 45
   Leu His Ala Met Met Glu Arg Gly Pro Gln Thr Leu Lys Glu Thr Ser
   50 55 60
   Phe Asn Gln Ala Tyr Gly Arg Asp Leu Met Glu Ala Gln Glu Trp Cys
   65 70 75 80
   Arg Lys Tyr Met Lys Ser Gly Asn Val Lys Asp Leu Thr Gln Ala Trp
   85 90 95
   Asp Leu Tyr Tyr His Val Phe Arg Arg Ile Ser Lys Thr Ser
   100 105 110
<210> 207
   <211> 110
   <212> PRT
   <213> Artificial Sequence
<220>
   <223> Description of Artificial Sequence: Synthetic polypeptide
<400> 207
   Met Gly His His His His His His His His Gly Ser Ala Ser Arg Ile
   1 5 10 15
   Leu Trp His Glu Met Trp His Glu Gly Leu Ile Glu Ala Ser Arg Leu
   20 25 30
   Tyr Phe Gly Glu Arg Asn Val Lys Gly Met Phe Glu Val Leu Glu Pro
   35 40 45
   Leu His Ala Met Met Glu Arg Gly Pro Gln Thr Leu Lys Glu Thr Ser
   50 55 60
   Phe Asn Gln Ala Tyr Gly Arg Asp Leu Met Glu Ala Gln Glu Trp Cys
   65 70 75 80
   Arg Lys Tyr Met Lys Ser Gly Asn Val Lys Asp Leu Thr Gln Ala Trp
   85 90 95
   Asp Leu Tyr Tyr His Val Phe Arg Arg Ile Ser Lys Thr Ser
   100 105 110
<210> 208
   <211> 110
   <212> PRT
   <213> Artificial Sequence
<220>
   <223> Description of Artificial Sequence: Synthetic polypeptide
<400> 208
   Met Gly His His His His His His His His Gly Ser Ala Ser Arg Ile
   1 5 10 15
   Leu Trp His Glu Met Trp His Glu Gly Leu Leu Glu Ala Ser Arg Leu
   20 25 30
   Tyr Phe Gly Glu Arg Asn Val Lys Gly Met Phe Glu Val Leu Glu Pro
   35 40 45
   Leu His Ala Met Met Glu Arg Gly Pro Gln Thr Leu Lys Glu Thr Ser
   50 55 60
   Phe Asn Gln Ala Tyr Gly Arg Asp Leu Met Glu Ala Gln Glu Trp Cys
   65 70 75 80
   Arg Lys Tyr Met Lys Ser Gly Asn Val Lys Asp Leu Thr Gln Ala Trp
   85 90 95
   Asp Leu Tyr Tyr His Val Phe Arg Arg Ile Ser Lys Thr Ser
   100 105 110
<210> 209
   <211> 110
   <212> PRT
   <213> Artificial Sequence
<220>
   <223> Description of Artificial Sequence: Synthetic polypeptide
<400> 209
   Met Gly His His His His His His His His Gly Ser Ala Ser Arg Ile
   1 5 10 15
   Leu Trp His Glu Met Trp His Glu Gly Leu Glu Glu Ala Ser Arg Leu
   20 25 30
   Tyr Phe Gly Glu Arg Asn Val Lys Gly Met Phe Glu Val Leu Glu Pro
   35 40 45
   Leu His Ala Met Met Glu Arg Gly Pro Gln Thr Leu Lys Glu Thr Ser
   50 55 60
   Phe Asn Gln Ala Tyr Gly Arg Asp Leu Met Glu Ala Gln Glu Trp Cys
   65 70 75 80
   Arg Lys Tyr Met Lys Ser Gly Asn Val Lys Asp Leu Leu Gln Ala Trp
   85 90 95
   Asp Leu Tyr Tyr His Val Phe Arg Arg Ile Ser Lys Thr Ser
   100 105 110
<210> 210
   <211> 110
   <212> PRT
   <213> Artificial Sequence
<220>
   <223> Description of Artificial Sequence: Synthetic polypeptide
<400> 210
   Met Gly His His His His His His His His Gly Ser Ala Ser Arg Ile
   1               5                   10                  15
   Leu Trp His Glu Met Trp His Glu Gly Leu Ile Glu Ala Ser Arg Leu
               20                  25                  30
   Tyr Phe Gly Glu Arg Asn Val Lys Gly Met Phe Glu Val Leu Glu Pro
           35                  40                  45
   Leu His Ala Met Met Glu Arg Gly Pro Gln Thr Leu Lys Glu Thr Ser
       50                  55                  60
   Phe Asn Gln Ala Tyr Gly Arg Asp Leu Met Glu Ala Gln Glu Trp Cys
   65                  70                  75                  80
   Arg Lys Tyr Met Lys Ser Gly Asn Val Lys Asp Leu Leu Gln Ala Trp
                   85                  90                  95
   Asp Leu Tyr Tyr His Val Phe Arg Arg Ile Ser Lys Thr Ser
               100                 105                 110
<210> 211
   <211> 110
   <212> PRT
   <213> Artificial Sequence
<220>
   <223> Description of Artificial Sequence: Synthetic polypeptide
<400> 211
   Met Gly His His His His His His His His Gly Ser Ala Ser Arg Ile
   1               5                   10                  15
   Leu Trp His Glu Met Trp His Glu Gly Leu Leu Glu Ala Ser Arg Leu
               20                  25                  30
   Tyr Phe Gly Glu Arg Asn Val Lys Gly Met Phe Glu Val Leu Glu Pro
           35                  40                  45
   Leu His Ala Met Met Glu Arg Gly Pro Gln Thr Leu Lys Glu Thr Ser
       50                  55                  60
   Phe Asn Gln Ala Tyr Gly Arg Asp Leu Met Glu Ala Gln Glu Trp Cys
   65                  70                  75                  80
   Arg Lys Tyr Met Lys Ser Gly Asn Val Lys Asp Leu Leu Gln Ala Trp
                   85                  90                  95
   Asp Leu Tyr Tyr His Val Phe Arg Arg Ile Ser Lys Thr Ser
   100 105 110
<210> 212
   <211> 120
   <212> PRT
   <213> Artificial Sequence
<220>
   <223> Description of Artificial Sequence: Synthetic polypeptide
<400> 212
   Met Gly His His His His His His His His Gly Ser Asp Tyr Lys Asp
   1 5 10 15
   Asp Asp Asp Lys Gly Ser Ala Ser Arg Ile Leu Trp His Glu Met Trp
   20 25 30
   His Glu Gly Leu Glu Glu Ala Ser Arg Leu Tyr Phe Gly Glu Arg Asn
   35 40 45
   Val Lys Gly Met Phe Glu Val Leu Glu Pro Leu His Ala Met Met Glu
   50 55 60
   Arg Gly Pro Gln Thr Leu Lys Glu Thr Ser Phe Asn Gln Ala Tyr Gly
   65 70 75 80
   Arg Asp Leu Met Glu Ala Gln Glu Trp Cys Arg Lys Tyr Met Lys Ser
   85 90 95
   Gly Asn Val Lys Asp Leu Thr Gln Ala Trp Asp Leu Tyr Tyr His Val
   100 105 110
   Phe Arg Arg Ile Ser Lys Thr Ser
   115 120
<210> 213
   <211> 120
   <212> PRT
   <213> Artificial Sequence
<220>
   <223> Description of Artificial Sequence: Synthetic polypeptide
<400> 213
   Met Gly His His His His His His His His Gly Ser Asp Tyr Lys Asp
   1               5                   10                  15
   Asp Asp Asp Lys Gly Ser Ala Ser Arg Ile Leu Trp His Glu Met Trp
               20                  25                  30
   His Glu Gly Leu Ile Glu Ala Ser Arg Leu Tyr Phe Gly Glu Arg Asn
           35                  40                  45
   Val Lys Gly Met Phe Glu Val Leu Glu Pro Leu His Ala Met Met Glu
       50                  55                  60
   Arg Gly Pro Gln Thr Leu Lys Glu Thr Ser Phe Asn Gln Ala Tyr Gly
   65                  70                  75                  80
   Arg Asp Leu Met Glu Ala Gln Glu Trp Cys Arg Lys Tyr Met Lys Ser
                   85                  90                  95
   Gly Asn Val Lys Asp Leu Thr Gln Ala Trp Asp Leu Tyr Tyr His Val
               100                 105                 110
   Phe Arg Arg Ile Ser Lys Thr Ser
           115                 120
<210> 214
   <211> 120
   <212> PRT
   <213> Artificial Sequence
<220>
   <223> Description of Artificial Sequence: Synthetic polypeptide
<400> 214
   Met Gly His His His His His His His His Gly Ser Asp Tyr Lys Asp
   1               5                   10                  15
   Asp Asp Asp Lys Gly Ser Ala Ser Arg Ile Leu Trp His Glu Met Trp
               20                  25                  30
   His Glu Gly Leu Leu Glu Ala Ser Arg Leu Tyr Phe Gly Glu Arg Asn
           35                  40                  45
   Val Lys Gly Met Phe Glu Val Leu Glu Pro Leu His Ala Met Met Glu
       50                  55                  60
   Arg Gly Pro Gln Thr Leu Lys Glu Thr Ser Phe Asn Gln Ala Tyr Gly
   65                  70                  75                  80
   Arg Asp Leu Met Glu Ala Gln Glu Trp Cys Arg Lys Tyr Met Lys Ser
                   85                  90                  95
   Gly Asn Val Lys Asp Leu Thr Gln Ala Trp Asp Leu Tyr Tyr His Val
               100                 105                 110
   Phe Arg Arg Ile Ser Lys Thr Ser
           115                 120
<210> 215
   <211> 120
   <212> PRT
   <213> Artificial Sequence
<220>
   <223> Description of Artificial Sequence: Synthetic polypeptide
<400> 215
   Met Gly His His His His His His His His Gly Ser Asp Tyr Lys Asp
   1               5                   10                  15
   Asp Asp Asp Lys Gly Ser Ala Ser Arg Ile Leu Trp His Glu Met Trp
               20                  25                  30
   His Glu Gly Leu Glu Glu Ala Ser Arg Leu Tyr Phe Gly Glu Arg Asn
           35                  40                  45
   Val Lys Gly Met Phe Glu Val Leu Glu Pro Leu His Ala Met Met Glu
       50                  55                  60
   Arg Gly Pro Gln Thr Leu Lys Glu Thr Ser Phe Asn Gln Ala Tyr Gly
   65                  70                  75                  80
   Arg Asp Leu Met Glu Ala Gln Glu Trp Cys Arg Lys Tyr Met Lys Ser
                   85                  90                  95
   Gly Asn Val Lys Asp Leu Leu Gln Ala Trp Asp Leu Tyr Tyr His Val
               100                 105                 110
   Phe Arg Arg Ile Ser Lys Thr Ser
           115                 120
<210> 216
   <211> 120
   <212> PRT
   <213> Artificial Sequence
<220>
   <223> Description of Artificial Sequence: Synthetic polypeptide
<400> 216
   Met Gly His His His His His His His His Gly Ser Asp Tyr Lys Asp
   1               5                   10                  15
   Asp Asp Asp Lys Gly Ser Ala Ser Arg Ile Leu Trp His Glu Met Trp
               20                  25                  30
   His Glu Gly Leu Ile Glu Ala Ser Arg Leu Tyr Phe Gly Glu Arg Asn
           35                  40                  45
   Val Lys Gly Met Phe Glu Val Leu Glu Pro Leu His Ala Met Met Glu
       50                  55                  60
   Arg Gly Pro Gln Thr Leu Lys Glu Thr Ser Phe Asn Gln Ala Tyr Gly
   65                  70                  75                  80
   Arg Asp Leu Met Glu Ala Gln Glu Trp Cys Arg Lys Tyr Met Lys Ser
                   85                  90                  95
   Gly Asn Val Lys Asp Leu Leu Gln Ala Trp Asp Leu Tyr Tyr His Val
               100                 105                 110
   Phe Arg Arg Ile Ser Lys Thr Ser
           115                 120
<210> 217
   <211> 120
   <212> PRT
   <213> Artificial Sequence
<220>
   <223> Description of Artificial Sequence: Synthetic polypeptide
<400> 217
   Met Gly His His His His His His His His Gly Ser Asp Tyr Lys Asp
   1               5                   10                  15
   Asp Asp Asp Lys Gly Ser Ala Ser Arg Ile Leu Trp His Glu Met Trp
               20                  25                  30
   His Glu Gly Leu Leu Glu Ala Ser Arg Leu Tyr Phe Gly Glu Arg Asn
           35                  40                  45
   Val Lys Gly Met Phe Glu Val Leu Glu Pro Leu His Ala Met Met Glu
       50                  55                  60
   Arg Gly Pro Gln Thr Leu Lys Glu Thr Ser Phe Asn Gln Ala Tyr Gly
   65                  70                  75                  80
   Arg Asp Leu Met Glu Ala Gln Glu Trp Cys Arg Lys Tyr Met Lys Ser
                   85                  90                  95
   Gly Asn Val Lys Asp Leu Leu Gln Ala Trp Asp Leu Tyr Tyr His Val
               100                 105                 110
   Phe Arg Arg Ile Ser Lys Thr Ser
           115                 120
<210> 218
   <211> 136
   <212> PRT
   <213> Artificial Sequence
<220>
   <223> Description of Artificial Sequence: Synthetic polypeptide
<400> 218
   Met Gly His His His His His His His His Gly Ser Gly Leu Asn Asp
   1               5                   10                  15
   Ile Phe Glu Ala Gln Lys Ile Glu Trp His Glu Gly Ser Gly Val Gln
               20                  25                  30
   Val Glu Thr Ile Ser Pro Gly Asp Gly Arg Thr Phe Pro Lys Arg Gly
           35                  40                  45
   Gln Thr Cys Val Val His Tyr Thr Gly Met Leu Glu Asp Gly Lys Lys
       50                  55                  60
   Phe Asp Ser Ser Arg Asp Arg Asn Lys Pro Phe Lys Phe Met Leu Gly
   65                  70                  75                  80
   Lys Gln Glu Val Ile Arg Gly Trp Glu Glu Gly Val Ala Gln Met Ser
                   85                  90                  95
   Val Gly Gln Arg Ala Lys Leu Thr Ile Ser Pro Asp Tyr Ala Tyr Gly
               100                 105                 110
   Ala Thr Gly His Pro Gly Ile Ile Pro Pro His Ala Thr Leu Val Phe
           115                 120                 125
   Asp Val Glu Leu Leu Lys Leu Glu
       130                 135
<210> 219
   <211> 1578
   <212> DNA
   <213> Artificial Sequence
<220>
   <223> Description of Artificial Sequence: Synthetic polynucleotide
<220>
   <221> CDS
   <222> (36)..(947)
<400> 219
   ctggggcgcg gccgcctgtc tgcacagaca gcacc atg tcg ctc atg gtc gtc        53
                                          Met Ser Leu Met Val Val
                                          1               5
   agc atg gcg tgt gtt ggg ttc ttc ttg ctg cag ggg gcc tgg cca cat      101
   Ser Met Ala Cys Val Gly Phe Phe Leu Leu Gln Gly Ala Trp Pro His
               10                  15                  20
   gag gga gtc cac aga aaa cct tcc ctc ctg gcc cac cca ggt ccc ctg      149
   Glu Gly Val His Arg Lys Pro Ser Leu Leu Ala His Pro Gly Pro Leu
           25                  30                  35
   gtg aaa tca gaa gag aca gtc atc ctg caa tgt tgg tca gat gtc agg      197
   Val Lys Ser Glu Glu Thr Val Ile Leu Gln Cys Trp Ser Asp Val Arg
       40                  45                  50
   ttt gag cac ttc ctt ctg cac aga gag ggg aag tat aag gac act ttg      245
   Phe Glu His Phe Leu Leu His Arg Glu Gly Lys Tyr Lys Asp Thr Leu
   55                  60                  65                  70
   cac ctc att gga gag cac cat gat ggg gtc tcc aag gcc aac ttc tcc      293
   His Leu Ile Gly Glu His His Asp Gly Val Ser Lys Ala Asn Phe Ser
                   75                  80                  85
   atc ggt ccc atg atg caa gac ctt gca ggg acc tac aga tgc tac ggt      341
   Ile Gly Pro Met Met Gln Asp Leu Ala Gly Thr Tyr Arg Cys Tyr Gly
               90                  95                  100
   tct gtt act cac tcc ccc tat cag ttg tca gct ccc agt gac cct ctg      389
   Ser Val Thr His Ser Pro Tyr Gln Leu Ser Ala Pro Ser Asp Pro Leu
           105                 110                 115
   gac atc gtc atc aca ggt cta tat gag aaa cct tct ctc tca gcc cag      437
   Asp Ile Val Ile Thr Gly Leu Tyr Glu Lys Pro Ser Leu Ser Ala Gln
       120                 125                 130
   ccg ggc ccc acg gtt ttg gca gga gag agc gtg acc ttg tcc tgc agc      485
   Pro Gly Pro Thr Val Leu Ala Gly Glu Ser Val Thr Leu Ser Cys Ser
   135                 140                 145                 150
   tcc cgg agc tcc tat gac atg tac cat cta tcc agg gag ggg gag gcc      533
   Ser Arg Ser Ser Tyr Asp Met Tyr His Leu Ser Arg Glu Gly Glu Ala
                   155                 160                 165
   cat gaa cgt agg ttc tct gca ggg ccc aag gtc aac gga aca ttc cag      581
   His Glu Arg Arg Phe Ser Ala Gly Pro Lys Val Asn Gly Thr Phe Gln
               170                 175                 180
   gcc gac ttt cct ctg ggc cct gcc acc cac gga gga acc tac aga tgc      629
   Ala Asp Phe Pro Leu Gly Pro Ala Thr His Gly Gly Thr Tyr Arg Cys
           185                 190                 195
   ttc ggc tct ttc cgt gac tct ccc tat gag tgg tca aac tcg agt gac      677
   Phe Gly Ser Phe Arg Asp Ser Pro Tyr Glu Trp Ser Asn Ser Ser Asp
       200                 205                 210
   cca ctg ctt gtt tct gtc aca gga aac cct tca aat agt tgg cct tca      725
   Pro Leu Leu Val Ser Val Thr Gly Asn Pro Ser Asn Ser Trp Pro Ser
   215                 220                 225                 230
   ccc act gaa cca agc tcc aaa acc ggt aac ccc aga cac ctg cat gtt      773
   Pro Thr Glu Pro Ser Ser Lys Thr Gly Asn Pro Arg His Leu His Val
                   235                     240                     245
   ctg att ggg acc tca gtg gtc aaa atc cct ttc acc atc ctc ctc ttc      821
   Leu Ile Gly Thr Ser Val Val Lys Ile Pro Phe Thr Ile Leu Leu Phe
               250                     255                     260
   ttt ctc ctt cat cgc tgg tgc tcc aac aaa aaa aat gct gct gta atg      869
   Phe Leu Leu His Arg Trp Cys Ser Asn Lys Lys Asn Ala Ala Val Met
           265                     270                     275
   gac caa gag cct gca ggg aac aga aca gtg aac agc gag gat tct gat      917
   Asp Gln Glu Pro Ala Gly Asn Arg Thr Val Asn Ser Glu Asp Ser Asp
       280                     285                     290
   gaa caa gac cat cag gag gtg tca tac gca taattggatc actgtgtttt        967
   Glu Gln Asp His Gln Glu Val Ser Tyr Ala
   295                     300
   cacacagaga aaaatcactc gcccttctga gaggcccaag acacccccaa cagataccag    1027
   catgtacata gaacttccaa atgctgagcc cagatccaaa gttgtcttct gtccacgagc    1087
   accacagtca ggccttgagg ggatcttcta gggagacaac agccctgtct caaaaccggg    1147
   ttgccagctc ccatgtacca gcagctggaa tctgaaggca tcagtcttca tcttagggca    1207
   tcgctcttcc tcacaccacg aatctgaaca tgcctctctc ttgcttacaa atgtctaagg    1267
   tccccactgc ctgctggaga gaaaacacac tcctttgctt agcccacaat tctccatttc    1327
   acttgacccc tgcccacctc tccaacctaa ctggcttact tcctagtcta cctgaggctg    1387
   caatcacact gaggaactca caattccaaa catacaagag gctgcctctt aacacagcac    1447
   ttagacacgt gctgttccac ctcccttcag actatctttc agccttctgc cagcagtaaa    1507
   acttataaat tttttaaata atttcaatgt agttttcccg ccttcaaata aacatgtctg    1567
   ccctcatgaa a                                                         1578
<210> 220
   <211> 304
   <212> PRT
   <213> Artificial Sequence
<220>
   <223> Description of Artificial Sequence: Synthetic polypeptide
<400> 220
   Met Ser Leu Met Val Val Ser Met Ala Cys Val Gly Phe Phe Leu Leu
   1               5                   10                  15
   Gln Gly Ala Trp Pro His Glu Gly Val His Arg Lys Pro Ser Leu Leu
               20                  25                  30
   Ala His Pro Gly Pro Leu Val Lys Ser Glu Glu Thr Val Ile Leu Gln
           35                  40                  45
   Cys Trp Ser Asp Val Arg Phe Glu His Phe Leu Leu His Arg Glu Gly
   50 55 60
   Lys Tyr Lys Asp Thr Leu His Leu Ile Gly Glu His His Asp Gly Val
   65 70 75 80
   Ser Lys Ala Asn Phe Ser Ile Gly Pro Met Met Gln Asp Leu Ala Gly
   85 90 95
   Thr Tyr Arg Cys Tyr Gly Ser Val Thr His Ser Pro Tyr Gln Leu Ser
   100 105 110
   Ala Pro Ser Asp Pro Leu Asp Ile Val Ile Thr Gly Leu Tyr Glu Lys
   115 120 125
   Pro Ser Leu Ser Ala Gln Pro Gly Pro Thr Val Leu Ala Gly Glu Ser
   130 135 140
   Val Thr Leu Ser Cys Ser Ser Arg Ser Ser Tyr Asp Met Tyr His Leu
   145 150 155 160
   Ser Arg Glu Gly Glu Ala His Glu Arg Arg Phe Ser Ala Gly Pro Lys
   165 170 175
   Val Asn Gly Thr Phe Gln Ala Asp Phe Pro Leu Gly Pro Ala Thr His
   180 185 190
   Gly Gly Thr Tyr Arg Cys Phe Gly Ser Phe Arg Asp Ser Pro Tyr Glu
   195 200 205
   Trp Ser Asn Ser Ser Asp Pro Leu Leu Val Ser Val Thr Gly Asn Pro
   210 215 220
   Ser Asn Ser Trp Pro Ser Pro Thr Glu Pro Ser Ser Lys Thr Gly Asn
   225 230 235 240
   Pro Arg His Leu His Val Leu Ile Gly Thr Ser Val Val Lys Ile Pro
   245 250 255
   Phe Thr Ile Leu Leu Phe Phe Leu Leu His Arg Trp Cys Ser Asn Lys
   260 265 270
   Lys Asn Ala Ala Val Met Asp Gln Glu Pro Ala Gly Asn Arg Thr Val
   275 280 285
   Asn Ser Glu Asp Ser Asp Glu Gln Asp His Gln Glu Val Ser Tyr Ala
   290 295 300
<210> 221
   <211> 1596
   <212> DNA
   <213> Homo sapiens
<220>
   <221> CDS
   <222> (38)..(1060)
<400> 221
   agctggggcg cggccgcctg tctgcacaga cagcacc atg tcg ctc atg gtc gtc      55
                                            Met Ser Leu Met Val Val
                                            1               5
   agc atg gtg tgt gtt ggg ttc ttc ttg ctg cag ggg gcc tgg cca cat      103
   Ser Met Val Cys Val Gly Phe Phe Leu Leu Gln Gly Ala Trp Pro His
               10                  15                  20
   gag gga gtc cac aga aaa cct tcc ctc ctg gcc cac cca ggt ccc ctg      151
   Glu Gly Val His Arg Lys Pro Ser Leu Leu Ala His Pro Gly Pro Leu
           25                  30                  35
   gtg aaa tca gaa gag aca gtc atc ctg caa tgt tgg tca gat gtc agg      199
   Val Lys Ser Glu Glu Thr Val Ile Leu Gln Cys Trp Ser Asp Val Arg
       40                  45                  50
   ttt cag cac ttc ctt ctg cac aga gaa ggg aag ttt aag gac act ttg      247
   Phe Gln His Phe Leu Leu His Arg Glu Gly Lys Phe Lys Asp Thr Leu
   55                  60                  65                  70
   cac ctc att gga gag cac cat gat ggg gtc tcc aag gcc aac ttc tcc      295
   His Leu Ile Gly Glu His His Asp Gly Val Ser Lys Ala Asn Phe Ser
                   75                  80                  85
   atc ggt ccc atg atg caa gac ctt gca ggg acc tac aga tgc tac ggt      343
   Ile Gly Pro Met Met Gln Asp Leu Ala Gly Thr Tyr Arg Cys Tyr Gly
               90                  95                  100
   tct gtt act cac tcc ccc tat cag ttg tca gct ccc agt gac cct ctg      391
   Ser Val Thr His Ser Pro Tyr Gln Leu Ser Ala Pro Ser Asp Pro Leu
           105                 110                 115
   gac atc gtc atc aca ggt cta tat gag aaa cct tct ctc tca gcc cag      439
   Asp Ile Val Ile Thr Gly Leu Tyr Glu Lys Pro Ser Leu Ser Ala Gln
       120                 125                 130
   ccg ggc ccc acg gtt ctg gca gga gag agc gtg acc ttg tcc tgc agc      487
   Pro Gly Pro Thr Val Leu Ala Gly Glu Ser Val Thr Leu Ser Cys Ser
   135                 140                 145                 150
   tcc cgg agc tcc tat gac atg tac cat cta tcc agg gag ggg gag gcc      535
   Ser Arg Ser Ser Tyr Asp Met Tyr His Leu Ser Arg Glu Gly Glu Ala
                   155                 160                 165
   cat gaa cgt agg ttc tct gca ggg ccc aag gtc aac gga aca ttc cag      583
   His Glu Arg Arg Phe Ser Ala Gly Pro Lys Val Asn Gly Thr Phe Gln
               170                 175                 180
   gcc gac ttt cct ctg ggc cct gcc acc cac gga gga acc tac aga tgc      631
   Ala Asp Phe Pro Leu Gly Pro Ala Thr His Gly Gly Thr Tyr Arg Cys
           185                 190                 195
   ttc ggc tct ttc cgt gac tct cca tac gag tgg tca aac tcg agt gac      679
   Phe Gly Ser Phe Arg Asp Ser Pro Tyr Glu Trp Ser Asn Ser Ser Asp
       200                 205                 210
   cca ctg ctt gtt tct gtc aca gga aac cct tca aat agt tgg cct tca      727
   Pro Leu Leu Val Ser Val Thr Gly Asn Pro Ser Asn Ser Trp Pro Ser
   215                 220                 225                 230
   ccc act gaa cca agc tcc gaa acc ggt aac ccc aga cac ctg cat gtt      775
   Pro Thr Glu Pro Ser Ser Glu Thr Gly Asn Pro Arg His Leu His Val
                   235                 240                 245
   ctg att ggg acc tca gtg gtc atc atc ctc ttc atc ctc ctc ctc ttc      823
   Leu Ile Gly Thr Ser Val Val Ile Ile Leu Phe Ile Leu Leu Leu Phe
               250                 255                 260
   ttt ctc ctt cat cgc tgg tgc tgc aac aaa aaa aat gct gtt gta atg      871
   Phe Leu Leu His Arg Trp Cys Cys Asn Lys Lys Asn Ala Val Val Met
           265                 270                 275
   gac caa gag cct gca ggg aac aga aca gtg aac agg gag gac tct gat      919
   Asp Gln Glu Pro Ala Gly Asn Arg Thr Val Asn Arg Glu Asp Ser Asp
       280                 285                 290
   gaa caa gac cct cag gag gtg aca tat gca cag ttg aat cac tgc gtt      967
   Glu Gln Asp Pro Gln Glu Val Thr Tyr Ala Gln Leu Asn His Cys Val
   295                 300                 305                 310
   ttc aca cag aga aaa atc act cgc cct tct cag agg ccc aag aca ccc     1015
   Phe Thr Gln Arg Lys Ile Thr Arg Pro Ser Gln Arg Pro Lys Thr Pro
                   315                 320                 325
   cca aca gat atc atc gtg tac acg gaa ctt cca aat gct gag ccc         1060
   Pro Thr Asp Ile Ile Val Tyr Thr Glu Leu Pro Asn Ala Glu Pro
               330                 335                 340
   tgatccaaag ttgtctcctg cccatgagca ccacagtcag gccttgaggg gatcttctag   1120
   ggagacaaca gccctgtctc aaaactgggt tgccagctcc aatgtaccag cagctggaat   1180
   ctgaaggcgt gagtctgcat cttagggcat cgctcttcct cacaccacaa atctgaacgt   1240
   gcctctccct tgcttacaaa tgtctaaggt ccccactgcc tgctggagag aaaacacact   1300
   cctttgctta gcccacaatt ctccatttca cttgacccct gcccacctct ccaacctaac   1360
   tggcttactt cctagtctac ttgaggctgc aatcacactg aggaactcac aattccaaac   1420
   atacaagagg ctccctctta acacggcact tagacacgtg ctgttccacc ttccctcatg   1480
   ctgttccacc tcccctcaga ctagctttca gccttctgtc agcagtaaaa cttatatatt   1540
   ttttaaaata atttcaatgt agttttccct ccttcaaata aacatgtctg ccctca       1596
<210> 222
   <211> 341
   <212> PRT
   <213> Homo sapiens
<400> 222
   Met Ser Leu Met Val Val Ser Met Val Cys Val Gly Phe Phe Leu Leu
   1               5                   10                  15
   Gln Gly Ala Trp Pro His Glu Gly Val His Arg Lys Pro Ser Leu Leu
               20                  25                  30
   Ala His Pro Gly Pro Leu Val Lys Ser Glu Glu Thr Val Ile Leu Gln
           35                  40                  45
   Cys Trp Ser Asp Val Arg Phe Gln His Phe Leu Leu His Arg Glu Gly
       50                  55                  60
   Lys Phe Lys Asp Thr Leu His Leu Ile Gly Glu His His Asp Gly Val
   65                  70                  75                  80
   Ser Lys Ala Asn Phe Ser Ile Gly Pro Met Met Gln Asp Leu Ala Gly
                   85                  90                  95
   Thr Tyr Arg Cys Tyr Gly Ser Val Thr His Ser Pro Tyr Gln Leu Ser
               100                 105                 110
   Ala Pro Ser Asp Pro Leu Asp Ile Val Ile Thr Gly Leu Tyr Glu Lys
           115                 120                 125
   Pro Ser Leu Ser Ala Gln Pro Gly Pro Thr Val Leu Ala Gly Glu Ser
       130                 135                 140
   Val Thr Leu Ser Cys Ser Ser Arg Ser Ser Tyr Asp Met Tyr His Leu
   145                 150                 155                 160
   Ser Arg Glu Gly Glu Ala His Glu Arg Arg Phe Ser Ala Gly Pro Lys
                   165                 170                 175
   Val Asn Gly Thr Phe Gln Ala Asp Phe Pro Leu Gly Pro Ala Thr His
               180                 185                 190
   Gly Gly Thr Tyr Arg Cys Phe Gly Ser Phe Arg Asp Ser Pro Tyr Glu
           195                 200                 205
   Trp Ser Asn Ser Ser Asp Pro Leu Leu Val Ser Val Thr Gly Asn Pro
       210                 215                 220
   Ser Asn Ser Trp Pro Ser Pro Thr Glu Pro Ser Ser Glu Thr Gly Asn
   225                 230                 235                 240
   Pro Arg His Leu His Val Leu Ile Gly Thr Ser Val Val Ile Ile Leu
                   245                 250                 255
   Phe Ile Leu Leu Leu Phe Phe Leu Leu His Arg Trp Cys Cys Asn Lys
                   260                 265                 270
   Lys Asn Ala Val Val Met Asp Gln Glu Pro Ala Gly Asn Arg Thr Val
               275                 280                 285
   Asn Arg Glu Asp Ser Asp Glu Gln Asp Pro Gln Glu Val Thr Tyr Ala
       290                 295                 300
   Gln Leu Asn His Cys Val Phe Thr Gln Arg Lys Ile Thr Arg Pro Ser
   305                 310                 315                 320
   Gln Arg Pro Lys Thr Pro Pro Thr Asp Ile Ile Val Tyr Thr Glu Leu
                   325                 330                 335
   Pro Asn Ala Glu Pro
               340
<210> 223
   <211> 1153
   <212> DNA
   <213> Artificial Sequence
<220>
   <223> Description of Artificial Sequence: Synthetic polynucleotide
<220>
   <221> CDS
   <222> (39)..(950)
<400> 223
   tccccactgc tcagcactag gccggcagaa tctgagcg atg tct tcc aca ctc cct       56
                                             Met Ser Ser Thr Leu Pro
                                             1               5
   gcc ctg ctc tgc gtc ggg ctg tgt ctg agt cag agg atc agc gcc cag        104
   Ala Leu Leu Cys Val Gly Leu Cys Leu Ser Gln Arg Ile Ser Ala Gln
               10                  15                  20
   cag cag act ctc cca aaa ccg ttc atc tgg gcc gag ccc cat ttc atg        152
   Gln Gln Thr Leu Pro Lys Pro Phe Ile Trp Ala Glu Pro His Phe Met
           25                  30                  35
   gtt cca aag gaa aag caa gtg acc atc tgt tgc cag gga aat tat ggg        200
   Val Pro Lys Glu Lys Gln Val Thr Ile Cys Cys Gln Gly Asn Tyr Gly
       40                  45                  50
   gct gtt gaa tac cag ctg cac ttt gaa gga agc ctt ttt gcc gtg gac        248
   Ala Val Glu Tyr Gln Leu His Phe Glu Gly Ser Leu Phe Ala Val Asp
   55                  60                  65                  70
   aga cca aaa ccc cct gag cgg att aac aaa gtc caa ttc tac atc ccg        296
   Arg Pro Lys Pro Pro Glu Arg Ile Asn Lys Val Gln Phe Tyr Ile Pro
                   75                  80                  85
   gac atg aac tcc cgc atg gca ggg caa tac agc tgc atc tat cgg gtt      344
   Asp Met Asn Ser Arg Met Ala Gly Gln Tyr Ser Cys Ile Tyr Arg Val
               90                  95                  100
   ggg gag ctc tgg tca gag ccc agc aac ttg ctg gat ctg gtg gta aca      392
   Gly Glu Leu Trp Ser Glu Pro Ser Asn Leu Leu Asp Leu Val Val Thr
           105                 110                 115
   gaa atg tat gac aca ccc acc ctc tcg gtt cat cct gga ccc gaa gtg      440
   Glu Met Tyr Asp Thr Pro Thr Leu Ser Val His Pro Gly Pro Glu Val
       120                 125                 130
   atc tcg gga gag aag gtg acc ttc tac tgc cgt cta gac act gca aca      488
   Ile Ser Gly Glu Lys Val Thr Phe Tyr Cys Arg Leu Asp Thr Ala Thr
   135                 140                 145                 150
   agc atg ttc tta ctg ctc aag gag gga aga tcc agc cac gta cag cgc      536
   Ser Met Phe Leu Leu Leu Lys Glu Gly Arg Ser Ser His Val Gln Arg
                   155                 160                 165
   gga tac ggg aag gtc cag gcg gag ttc ccc ctg ggc cct gtg acc aca      584
   Gly Tyr Gly Lys Val Gln Ala Glu Phe Pro Leu Gly Pro Val Thr Thr
               170                 175                 180
   gcc cac aga ggg aca tac cga tgt ttt ggc tcc tat aac aac cat gcc      632
   Ala His Arg Gly Thr Tyr Arg Cys Phe Gly Ser Tyr Asn Asn His Ala
           185                 190                 195
   tgg tct ttc ccc agt gag cca gtg aag ctc ctg gtc aca ggc gac att      680
   Trp Ser Phe Pro Ser Glu Pro Val Lys Leu Leu Val Thr Gly Asp Ile
       200                 205                 210
   gag aac acc agc ctt gca cct gaa gac ccc acc ttt cct gca gac act      728
   Glu Asn Thr Ser Leu Ala Pro Glu Asp Pro Thr Phe Pro Ala Asp Thr
   215                 220                 225                 230
   tgg ggc acc tac ctt tta acc aca gag acg gga ctc cag aaa gac cat      776
   Trp Gly Thr Tyr Leu Leu Thr Thr Glu Thr Gly Leu Gln Lys Asp His
                   235                 240                 245
   gcc ctc tgg gat cac act gcc cag aat ctc ctt cgg atg ggc ctg gcc      824
   Ala Leu Trp Asp His Thr Ala Gln Asn Leu Leu Arg Met Gly Leu Ala
               250                 255                 260
   ttt cta gtc ctg gtg gct cta gtg tgg ttc ctg gtt gaa gac tgg ctc      872
   Phe Leu Val Leu Val Ala Leu Val Trp Phe Leu Val Glu Asp Trp Leu
           265                 270                 275
   agc agg aag agg act aga gag cga gcc agc aga gct tcc act tgg gaa      920
   Ser Arg Lys Arg Thr Arg Glu Arg Ala Ser Arg Ala Ser Thr Trp Glu
       280                 285                 290
   ggc agg aga agg ctg aac aca cag act ctt tgaagaatga ccatgagaca        970
   Gly Arg Arg Arg Leu Asn Thr Gln Thr Leu
   295                 300
   cagtggccat gggtggatct gaaagctggt gttgagcctg ggcggcgtga gctctgtgtt    1030
   ggacccacgg aggagggagt cactgcaggg aaagagggac actggcattc catttgtcag    1090
   agcatcccgg acgatgcaga gggtgggaga actacatgct aaatttcttt tttttttttt    1150
   ttg 1153
<210> 224
   <211> 304
   <212> PRT
   <213> Artificial Sequence
<220>
   <223> Description of Artificial Sequence: Synthetic polypeptide
<400> 224
   Met Ser Ser Thr Leu Pro Ala Leu Leu Cys Val Gly Leu Cys Leu Ser
   1               5                   10                  15
   Gln Arg Ile Ser Ala Gln Gln Gln Thr Leu Pro Lys Pro Phe Ile Trp
               20                  25                  30
   Ala Glu Pro His Phe Met Val Pro Lys Glu Lys Gln Val Thr Ile Cys
           35                  40                  45
   Cys Gln Gly Asn Tyr Gly Ala Val Glu Tyr Gln Leu His Phe Glu Gly
       50                  55                  60
   Ser Leu Phe Ala Val Asp Arg Pro Lys Pro Pro Glu Arg Ile Asn Lys
   65                  70                  75                  80
   Val Gln Phe Tyr Ile Pro Asp Met Asn Ser Arg Met Ala Gly Gln Tyr
                   85                  90                  95
   Ser Cys Ile Tyr Arg Val Gly Glu Leu Trp Ser Glu Pro Ser Asn Leu
               100                 105                 110
   Leu Asp Leu Val Val Thr Glu Met Tyr Asp Thr Pro Thr Leu Ser Val
           115                 120                 125
   His Pro Gly Pro Glu Val Ile Ser Gly Glu Lys Val Thr Phe Tyr Cys
       130                 135                 140
   Arg Leu Asp Thr Ala Thr Ser Met Phe Leu Leu Leu Lys Glu Gly Arg
   145                 150                 155                 160
   Ser Ser His Val Gln Arg Gly Tyr Gly Lys Val Gln Ala Glu Phe Pro
                   165                 170                 175
   Leu Gly Pro Val Thr Thr Ala His Arg Gly Thr Tyr Arg Cys Phe Gly
               180                 185                 190
   Ser Tyr Asn Asn His Ala Trp Ser Phe Pro Ser Glu Pro Val Lys Leu
                   195                 200                 205
   Leu Val Thr Gly Asp Ile Glu Asn Thr Ser Leu Ala Pro Glu Asp Pro
       210                 215                 220
   Thr Phe Pro Ala Asp Thr Trp Gly Thr Tyr Leu Leu Thr Thr Glu Thr
   225                 230                 235                 240
   Gly Leu Gln Lys Asp His Ala Leu Trp Asp His Thr Ala Gln Asn Leu
                   245                 250                 255
   Leu Arg Met Gly Leu Ala Phe Leu Val Leu Val Ala Leu Val Trp Phe
               260                 265                 270
   Leu Val Glu Asp Trp Leu Ser Arg Lys Arg Thr Arg Glu Arg Ala Ser
           275                 280                 285
   Arg Ala Ser Thr Trp Glu Gly Arg Arg Arg Leu Asn Thr Gln Thr Leu
       290                 295                 300
<210> 225
   <211> 1053
   <212> DNA
   <213> Artificial Sequence
<220>
   <223> Description of Artificial Sequence: Synthetic polynucleotide
<220>
   <221> CDS
   <222> (1)..(1050)
<400> 225
   atg gcc tta cca gtg acc gcc ttg ctc ctg ccg ctg gcc ttg ctg ctc        48
   Met Ala Leu Pro Val Thr Ala Leu Leu Leu Pro Leu Ala Leu Leu Leu
   1               5                   10                  15
   cac gcc gcc agg ccg gga tcc cag gta caa ctg cag cag tct ggg cct        96
   His Ala Ala Arg Pro Gly Ser Gln Val Gln Leu Gln Gln Ser Gly Pro
               20                  25                  30
   gag ctg gag aag cct ggc gct tca gtg aag ata tcc tgc aag gct tct       144
   Glu Leu Glu Lys Pro Gly Ala Ser Val Lys Ile Ser Cys Lys Ala Ser
           35                  40                  45
   ggt tac tca ttc act ggc tac acc atg aac tgg gtg aag cag agc cat       192
   Gly Tyr Ser Phe Thr Gly Tyr Thr Met Asn Trp Val Lys Gln Ser His
       50                  55                  60
   gga aag agc ctt gag tgg att gga ctt att act cct tac aat ggt gct       240
   Gly Lys Ser Leu Glu Trp Ile Gly Leu Ile Thr Pro Tyr Asn Gly Ala
   65                  70                  75                  80
   tct agc tac aac cag aag ttc agg ggc aag gcc aca tta act gta gac       288
   Ser Ser Tyr Asn Gln Lys Phe Arg Gly Lys Ala Thr Leu Thr Val Asp
                   85                  90                  95
   aag tca tcc agc aca gcc tac atg gac ctc ctc agt ctg aca tct gaa       336
   Lys Ser Ser Ser Thr Ala Tyr Met Asp Leu Leu Ser Leu Thr Ser Glu
               100                 105                 110
   gac tct gca gtc tat ttc tgt gca agg ggg ggt tac gac ggg agg ggt       384
   Asp Ser Ala Val Tyr Phe Cys Ala Arg Gly Gly Tyr Asp Gly Arg Gly
           115                 120                 125
   ttt gac tac tgg ggc caa ggg acc acg gtc acc gtc tcc tca ggt gga       432
   Phe Asp Tyr Trp Gly Gln Gly Thr Thr Val Thr Val Ser Ser Gly Gly
       130                 135                 140
   ggc ggt tca ggc ggc ggt ggc tct agc ggt ggt gga tcg gac atc gag       480
   Gly Gly Ser Gly Gly Gly Gly Ser Ser Gly Gly Gly Ser Asp Ile Glu
   145                 150                 155                 160
   ctc act cag tct cca gca atc atg tct gca tct cca ggg gag aag gtc       528
   Leu Thr Gln Ser Pro Ala Ile Met Ser Ala Ser Pro Gly Glu Lys Val
                   165                 170                 175
   acc atg acc tgc agt gcc agc tca agt gta agt tac atg cac tgg tac       576
   Thr Met Thr Cys Ser Ala Ser Ser Ser Val Ser Tyr Met His Trp Tyr
               180                 185                 190
   cag cag aag tca ggc acc tcc ccc aaa aga tgg att tat gac aca tcc       624
   Gln Gln Lys Ser Gly Thr Ser Pro Lys Arg Trp Ile Tyr Asp Thr Ser
           195                 200                 205
   aaa ctg gct tct gga gtc cca ggt cgc ttc agt ggc agt ggg tct gga       672
   Lys Leu Ala Ser Gly Val Pro Gly Arg Phe Ser Gly Ser Gly Ser Gly
       210                 215                 220
   aac tct tac tct ctc aca atc agc agc gtg gag gct gaa gat gat gca       720
   Asn Ser Tyr Ser Leu Thr Ile Ser Ser Val Glu Ala Glu Asp Asp Ala
   225                 230                 235                 240
   act tat tac tgc cag cag tgg agt aag cac cct ctc acg tac ggt gct       768
   Thr Tyr Tyr Cys Gln Gln Trp Ser Lys His Pro Leu Thr Tyr Gly Ala
                   245                 250                 255
   ggg aca aag ttg gaa atc aaa gct agc ggt ggc gga ggt tct gga ggt       816
   Gly Thr Lys Leu Glu Ile Lys Ala Ser Gly Gly Gly Gly Ser Gly Gly
               260                 265                 270
   ggg ggt tcc tca ccc act gaa cca agc tcc aaa acc ggt aac ccc aga       864
   Gly Gly Ser Ser Pro Thr Glu Pro Ser Ser Lys Thr Gly Asn Pro Arg
           275                 280                 285
   cac ctg cat gtt ctg att ggg acc tca gtg gtc aaa atc cct ttc acc       912
   His Leu His Val Leu Ile Gly Thr Ser Val Val Lys Ile Pro Phe Thr
       290                 295                 300
   atc ctc ctc ttc ttt ctc ctt cat cgc tgg tgc tcc aac aaa aaa aat       960
   Ile Leu Leu Phe Phe Leu Leu His Arg Trp Cys Ser Asn Lys Lys Asn
   305                 310                 315                 320
   gct gct gta atg gac caa gag cct gca ggg aac aga aca gtg aac agc      1008
   Ala Ala Val Met Asp Gln Glu Pro Ala Gly Asn Arg Thr Val Asn Ser
                   325                 330                 335
   gag gat tct gat gaa caa gac cat cag gag gtg tca tac gca taa          1053
   Glu Asp Ser Asp Glu Gln Asp His Gln Glu Val Ser Tyr Ala
               340                 345                 350
<210> 226
   <211> 350
   <212> PRT
   <213> Artificial Sequence
<220>
   <223> Description of Artificial Sequence: Synthetic polypeptide
<400> 226
   Met Ala Leu Pro Val Thr Ala Leu Leu Leu Pro Leu Ala Leu Leu Leu
   1               5                   10                  15
   His Ala Ala Arg Pro Gly Ser Gln Val Gln Leu Gln Gln Ser Gly Pro
               20                  25                  30
   Glu Leu Glu Lys Pro Gly Ala Ser Val Lys Ile Ser Cys Lys Ala Ser
           35                  40                  45
   Gly Tyr Ser Phe Thr Gly Tyr Thr Met Asn Trp Val Lys Gln Ser His
       50                  55                  60
   Gly Lys Ser Leu Glu Trp Ile Gly Leu Ile Thr Pro Tyr Asn Gly Ala
   65                  70                  75                  80
   Ser Ser Tyr Asn Gln Lys Phe Arg Gly Lys Ala Thr Leu Thr Val Asp
                   85                  90                  95
   Lys Ser Ser Ser Thr Ala Tyr Met Asp Leu Leu Ser Leu Thr Ser Glu
               100                 105                 110
   Asp Ser Ala Val Tyr Phe Cys Ala Arg Gly Gly Tyr Asp Gly Arg Gly
           115                 120                 125
   Phe Asp Tyr Trp Gly Gln Gly Thr Thr Val Thr Val Ser Ser Gly Gly
       130                 135                 140
   Gly Gly Ser Gly Gly Gly Gly Ser Ser Gly Gly Gly Ser Asp Ile Glu
   145                 150                 155                 160
   Leu Thr Gln Ser Pro Ala Ile Met Ser Ala Ser Pro Gly Glu Lys Val
                   165                 170                 175
   Thr Met Thr Cys Ser Ala Ser Ser Ser Val Ser Tyr Met His Trp Tyr
               180                 185                 190
   Gln Gln Lys Ser Gly Thr Ser Pro Lys Arg Trp Ile Tyr Asp Thr Ser
           195                 200                 205
   Lys Leu Ala Ser Gly Val Pro Gly Arg Phe Ser Gly Ser Gly Ser Gly
       210                 215                 220
   Asn Ser Tyr Ser Leu Thr Ile Ser Ser Val Glu Ala Glu Asp Asp Ala
   225                 230                 235                 240
   Thr Tyr Tyr Cys Gln Gln Trp Ser Lys His Pro Leu Thr Tyr Gly Ala
                   245                 250                 255
   Gly Thr Lys Leu Glu Ile Lys Ala Ser Gly Gly Gly Gly Ser Gly Gly
               260                 265                 270
   Gly Gly Ser Ser Pro Thr Glu Pro Ser Ser Lys Thr Gly Asn Pro Arg
           275                 280                 285
   His Leu His Val Leu Ile Gly Thr Ser Val Val Lys Ile Pro Phe Thr
       290                 295                 300
   Ile Leu Leu Phe Phe Leu Leu His Arg Trp Cys Ser Asn Lys Lys Asn
   305                 310                 315                 320
   Ala Ala Val Met Asp Gln Glu Pro Ala Gly Asn Arg Thr Val Asn Ser
                   325                 330                 335
   Glu Asp Ser Asp Glu Gln Asp His Gln Glu Val Ser Tyr Ala
               340                 345                 350
<210> 227
   <211> 1698
   <212> DNA
   <213> Artificial Sequence
<220>
   <223> Description of Artificial Sequence: Synthetic polynucleotide
<220>
   <221> CDS
   <222> (1)..(1695)
<400> 227
   atg gcc tta cca gtg acc gcc ttg ctc ctg ccg ctg gcc ttg ctg ctc      48
   Met Ala Leu Pro Val Thr Ala Leu Leu Leu Pro Leu Ala Leu Leu Leu
   1               5                   10                  15
   cac gcc gcc agg ccg gga tcc cag gta caa ctg cag cag tct ggg cct      96
   His Ala Ala Arg Pro Gly Ser Gln Val Gln Leu Gln Gln Ser Gly Pro
               20                  25                  30
   gag ctg gag aag cct ggc gct tca gtg aag ata tcc tgc aag gct tct      144
   Glu Leu Glu Lys Pro Gly Ala Ser Val Lys Ile Ser Cys Lys Ala Ser
           35                  40                  45
   ggt tac tca ttc act ggc tac acc atg aac tgg gtg aag cag agc cat      192
   Gly Tyr Ser Phe Thr Gly Tyr Thr Met Asn Trp Val Lys Gln Ser His
       50                  55                  60
   gga aag agc ctt gag tgg att gga ctt att act cct tac aat ggt gct      240
   Gly Lys Ser Leu Glu Trp Ile Gly Leu Ile Thr Pro Tyr Asn Gly Ala
   65                  70                  75                  80
   tct agc tac aac cag aag ttc agg ggc aag gcc aca tta act gta gac      288
   Ser Ser Tyr Asn Gln Lys Phe Arg Gly Lys Ala Thr Leu Thr Val Asp
                   85                  90                  95
   aag tca tcc agc aca gcc tac atg gac ctc ctc agt ctg aca tct gaa      336
   Lys Ser Ser Ser Thr Ala Tyr Met Asp Leu Leu Ser Leu Thr Ser Glu
               100                 105                 110
   gac tct gca gtc tat ttc tgt gca agg ggg ggt tac gac ggg agg ggt      384
   Asp Ser Ala Val Tyr Phe Cys Ala Arg Gly Gly Tyr Asp Gly Arg Gly
           115                 120                 125
   ttt gac tac tgg ggc caa ggg acc acg gtc acc gtc tcc tca ggt gga      432
   Phe Asp Tyr Trp Gly Gln Gly Thr Thr Val Thr Val Ser Ser Gly Gly
       130                 135                 140
   ggc ggt tca ggc ggc ggt ggc tct agc ggt ggt gga tcg gac atc gag      480
   Gly Gly Ser Gly Gly Gly Gly Ser Ser Gly Gly Gly Ser Asp Ile Glu
   145                 150                 155                 160
   ctc act cag tct cca gca atc atg tct gca tct cca ggg gag aag gtc      528
   Leu Thr Gln Ser Pro Ala Ile Met Ser Ala Ser Pro Gly Glu Lys Val
                   165                 170                 175
   acc atg acc tgc agt gcc agc tca agt gta agt tac atg cac tgg tac      576
   Thr Met Thr Cys Ser Ala Ser Ser Ser Val Ser Tyr Met His Trp Tyr
               180                 185                 190
   cag cag aag tca ggc acc tcc ccc aaa aga tgg att tat gac aca tcc      624
   Gln Gln Lys Ser Gly Thr Ser Pro Lys Arg Trp Ile Tyr Asp Thr Ser
           195                 200                 205
   aaa ctg gct tct gga gtc cca ggt cgc ttc agt ggc agt ggg tct gga      672
   Lys Leu Ala Ser Gly Val Pro Gly Arg Phe Ser Gly Ser Gly Ser Gly
       210                 215                 220
   aac tct tac tct ctc aca atc agc agc gtg gag gct gaa gat gat gca      720
   Asn Ser Tyr Ser Leu Thr Ile Ser Ser Val Glu Ala Glu Asp Asp Ala
   225                 230                 235                 240
   act tat tac tgc cag cag tgg agt aag cac cct ctc acg tac ggt gct      768
   Thr Tyr Tyr Cys Gln Gln Trp Ser Lys His Pro Leu Thr Tyr Gly Ala
                   245                 250                 255
   ggg aca aag ttg gaa atc aaa gct agc acg cgt ggt ggc gga ggt tct      816
   Gly Thr Lys Leu Glu Ile Lys Ala Ser Thr Arg Gly Gly Gly Gly Ser
               260                 265                 270
   gga ggt ggg ggt tcc cag ggg gcc tgg cca cat gag gga gtc cac aga      864
   Gly Gly Gly Gly Ser Gln Gly Ala Trp Pro His Glu Gly Val His Arg
           275                 280                 285
   aaa cct tcc ctc ctg gcc cac cca ggt ccc ctg gtg aaa tca gaa gag       912
   Lys Pro Ser Leu Leu Ala His Pro Gly Pro Leu Val Lys Ser Glu Glu
       290                 295                 300
   aca gtc atc ctg caa tgt tgg tca gat gtc agg ttt gag cac ttc ctt       960
   Thr Val Ile Leu Gln Cys Trp Ser Asp Val Arg Phe Glu His Phe Leu
   305                 310                 315                 320
   ctg cac aga gag ggg aag tat aag gac act ttg cac ctc att gga gag      1008
   Leu His Arg Glu Gly Lys Tyr Lys Asp Thr Leu His Leu Ile Gly Glu
                   325                 330                 335
   cac cat gat ggg gtc tcc aag gcc aac ttc tcc atc ggt ccc atg atg      1056
   His His Asp Gly Val Ser Lys Ala Asn Phe Ser Ile Gly Pro Met Met
               340                 345                 350
   caa gac ctt gca ggg acc tac aga tgc tac ggt tct gtt act cac tcc      1104
   Gln Asp Leu Ala Gly Thr Tyr Arg Cys Tyr Gly Ser Val Thr His Ser
           355                 360                 365
   ccc tat cag ttg tca gct ccc agt gac cct ctg gac atc gtc atc aca      1152
   Pro Tyr Gln Leu Ser Ala Pro Ser Asp Pro Leu Asp Ile Val Ile Thr
       370                 375                 380
   ggt cta tat gag aaa cct tct ctc tca gcc cag ccg ggc ccc acg gtt      1200
   Gly Leu Tyr Glu Lys Pro Ser Leu Ser Ala Gln Pro Gly Pro Thr Val
   385                 390                 395                 400
   ttg gca gga gag agc gtg acc ttg tcc tgc agc tcc cgg agc tcc tat      1248
   Leu Ala Gly Glu Ser Val Thr Leu Ser Cys Ser Ser Arg Ser Ser Tyr
                   405                 410                 415
   gac atg tac cat cta tcc agg gag ggg gag gcc cat gaa cgt agg ttc      1296
   Asp Met Tyr His Leu Ser Arg Glu Gly Glu Ala His Glu Arg Arg Phe
               420                 425                 430
   tct gca ggg ccc aag gtc aac gga aca ttc cag gcc gac ttt cct ctg      1344
   Ser Ala Gly Pro Lys Val Asn Gly Thr Phe Gln Ala Asp Phe Pro Leu
           435                 440                 445
   ggc cct gcc acc cac gga gga acc tac aga tgc ttc ggc tct ttc cgt      1392
   Gly Pro Ala Thr His Gly Gly Thr Tyr Arg Cys Phe Gly Ser Phe Arg
       450                 455                 460
   gac tct ccc tat gag tgg tca aac tcg agt gac cca ctg ctt gtt tct      1440
   Asp Ser Pro Tyr Glu Trp Ser Asn Ser Ser Asp Pro Leu Leu Val Ser
   465                 470                 475                 480
   gtc aca gga aac cct tca aat agt tgg cct tca ccc act gaa cca agc      1488
   Val Thr Gly Asn Pro Ser Asn Ser Trp Pro Ser Pro Thr Glu Pro Ser
                   485                 490                 495
   tcc aaa acc ggt aac ccc aga cac ctg cat gtt ctg att ggg acc tca      1536
   Ser Lys Thr Gly Asn Pro Arg His Leu His Val Leu Ile Gly Thr Ser
               500                 505                 510
   gtg gtc aaa atc cct ttc acc atc ctc ctc ttc ttt ctc ctt cat cgc      1584
   Val Val Lys Ile Pro Phe Thr Ile Leu Leu Phe Phe Leu Leu His Arg
           515                 520                 525
   tgg tgc tcc aac aaa aaa aat gct gct gta atg gac caa gag cct gca      1632
   Trp Cys Ser Asn Lys Lys Asn Ala Ala Val Met Asp Gln Glu Pro Ala
       530                 535                 540
   ggg aac aga aca gtg aac agc gag gat tct gat gaa caa gac cat cag        1680
   Gly Asn Arg Thr Val Asn Ser Glu Asp Ser Asp Glu Gln Asp His Gln
   545                 550                 555                 560
   gag gtg tca tac gca taa                                                1698
   Glu Val Ser Tyr Ala
                   565
<210> 228
   <211> 565
   <212> PRT
   <213> Artificial Sequence
<220>
   <223> Description of Artificial Sequence: Synthetic polypeptide
<400> 228
   Met Ala Leu Pro Val Thr Ala Leu Leu Leu Pro Leu Ala Leu Leu Leu
   1               5                   10                  15
   His Ala Ala Arg Pro Gly Ser Gln Val Gln Leu Gln Gln Ser Gly Pro
               20                  25                  30
   Glu Leu Glu Lys Pro Gly Ala Ser Val Lys Ile Ser Cys Lys Ala Ser
           35                  40                  45
   Gly Tyr Ser Phe Thr Gly Tyr Thr Met Asn Trp Val Lys Gln Ser His
       50                  55                  60
   Gly Lys Ser Leu Glu Trp Ile Gly Leu Ile Thr Pro Tyr Asn Gly Ala
   65                  70                  75                  80
   Ser Ser Tyr Asn Gln Lys Phe Arg Gly Lys Ala Thr Leu Thr Val Asp
                   85                  90                  95
   Lys Ser Ser Ser Thr Ala Tyr Met Asp Leu Leu Ser Leu Thr Ser Glu
               100                 105                 110
   Asp Ser Ala Val Tyr Phe Cys Ala Arg Gly Gly Tyr Asp Gly Arg Gly
           115                 120                 125
   Phe Asp Tyr Trp Gly Gln Gly Thr Thr Val Thr Val Ser Ser Gly Gly
       130                 135                 140
   Gly Gly Ser Gly Gly Gly Gly Ser Ser Gly Gly Gly Ser Asp Ile Glu
   145                 150                 155                 160
   Leu Thr Gln Ser Pro Ala Ile Met Ser Ala Ser Pro Gly Glu Lys Val
                   165                 170                 175
   Thr Met Thr Cys Ser Ala Ser Ser Ser Val Ser Tyr Met His Trp Tyr
   180 185 190
   Gln Gln Lys Ser Gly Thr Ser Pro Lys Arg Trp Ile Tyr Asp Thr Ser
   195 200 205
   Lys Leu Ala Ser Gly Val Pro Gly Arg Phe Ser Gly Ser Gly Ser Gly
   210 215 220
   Asn Ser Tyr Ser Leu Thr Ile Ser Ser Val Glu Ala Glu Asp Asp Ala
   225 230 235 240
   Thr Tyr Tyr Cys Gln Gln Trp Ser Lys His Pro Leu Thr Tyr Gly Ala
   245 250 255
   Gly Thr Lys Leu Glu Ile Lys Ala Ser Thr Arg Gly Gly Gly Gly Ser
   260 265 270
   Gly Gly Gly Gly Ser Gln Gly Ala Trp Pro His Glu Gly Val His Arg
   275 280 285
   Lys Pro Ser Leu Leu Ala His Pro Gly Pro Leu Val Lys Ser Glu Glu
   290 295 300
   Thr Val Ile Leu Gln Cys Trp Ser Asp Val Arg Phe Glu His Phe Leu
   305 310 315 320
   Leu His Arg Glu Gly Lys Tyr Lys Asp Thr Leu His Leu Ile Gly Glu
   325 330 335
   His His Asp Gly Val Ser Lys Ala Asn Phe Ser Ile Gly Pro Met Met
   340 345 350
   Gln Asp Leu Ala Gly Thr Tyr Arg Cys Tyr Gly Ser Val Thr His Ser
   355 360 365
   Pro Tyr Gln Leu Ser Ala Pro Ser Asp Pro Leu Asp Ile Val Ile Thr
   370 375 380
   Gly Leu Tyr Glu Lys Pro Ser Leu Ser Ala Gln Pro Gly Pro Thr Val
   385 390 395 400
   Leu Ala Gly Glu Ser Val Thr Leu Ser Cys Ser Ser Arg Ser Ser Tyr
   405 410 415
   Asp Met Tyr His Leu Ser Arg Glu Gly Glu Ala His Glu Arg Arg Phe
                   420                 425                 430
   Ser Ala Gly Pro Lys Val Asn Gly Thr Phe Gln Ala Asp Phe Pro Leu
           435                 440                 445
   Gly Pro Ala Thr His Gly Gly Thr Tyr Arg Cys Phe Gly Ser Phe Arg
       450                 455                 460
   Asp Ser Pro Tyr Glu Trp Ser Asn Ser Ser Asp Pro Leu Leu Val Ser
   465                 470                 475                 480
   Val Thr Gly Asn Pro Ser Asn Ser Trp Pro Ser Pro Thr Glu Pro Ser
                   485                 490                 495
   Ser Lys Thr Gly Asn Pro Arg His Leu His Val Leu Ile Gly Thr Ser
               500                 505                 510
   Val Val Lys Ile Pro Phe Thr Ile Leu Leu Phe Phe Leu Leu His Arg
           515                 520                 525
   Trp Cys Ser Asn Lys Lys Asn Ala Ala Val Met Asp Gln Glu Pro Ala
       530                 535                 540
   Gly Asn Arg Thr Val Asn Ser Glu Asp Ser Asp Glu Gln Asp His Gln
   545                 550                 555                 560
   Glu Val Ser Tyr Ala
                   565
<210> 229
   <211> 1035
   <212> DNA
   <213> Artificial Sequence
<220>
   <223> Description of Artificial Sequence: Synthetic polynucleotide
<220>
   <221> CDS
   <222> (1)..(1032)
<400> 229
   atg gcc tta cca gtg acc gcc ttg ctc ctg ccg ctg gcc ttg ctg ctc       48
   Met Ala Leu Pro Val Thr Ala Leu Leu Leu Pro Leu Ala Leu Leu Leu
   1               5                   10                  15
   cac gcc gcc agg ccg gga tcc cag gta caa ctg cag cag tct ggg cct       96
   His Ala Ala Arg Pro Gly Ser Gln Val Gln Leu Gln Gln Ser Gly Pro
               20                  25                  30
   gag ctg gag aag cct ggc gct tca gtg aag ata tcc tgc aag gct tct      144
   Glu Leu Glu Lys Pro Gly Ala Ser Val Lys Ile Ser Cys Lys Ala Ser
           35                  40                  45
   ggt tac tca ttc act ggc tac acc atg aac tgg gtg aag cag agc cat      192
   Gly Tyr Ser Phe Thr Gly Tyr Thr Met Asn Trp Val Lys Gln Ser His
       50                  55                  60
   gga aag agc ctt gag tgg att gga ctt att act cct tac aat ggt gct      240
   Gly Lys Ser Leu Glu Trp Ile Gly Leu Ile Thr Pro Tyr Asn Gly Ala
   65                  70                  75                  80
   tct agc tac aac cag aag ttc agg ggc aag gcc aca tta act gta gac      288
   Ser Ser Tyr Asn Gln Lys Phe Arg Gly Lys Ala Thr Leu Thr Val Asp
                   85                  90                  95
   aag tca tcc agc aca gcc tac atg gac ctc ctc agt ctg aca tct gaa      336
   Lys Ser Ser Ser Thr Ala Tyr Met Asp Leu Leu Ser Leu Thr Ser Glu
               100                 105                 110
   gac tct gca gtc tat ttc tgt gca agg ggg ggt tac gac ggg agg ggt      384
   Asp Ser Ala Val Tyr Phe Cys Ala Arg Gly Gly Tyr Asp Gly Arg Gly
           115                 120                 125
   ttt gac tac tgg ggc caa ggg acc acg gtc acc gtc tcc tca ggt gga      432
   Phe Asp Tyr Trp Gly Gln Gly Thr Thr Val Thr Val Ser Ser Gly Gly
       130                 135                 140
   ggc ggt tca ggc ggc ggt ggc tct agc ggt ggt gga tcg gac atc gag      480
   Gly Gly Ser Gly Gly Gly Gly Ser Ser Gly Gly Gly Ser Asp Ile Glu
   145                 150                 155                 160
   ctc act cag tct cca gca atc atg tct gca tct cca ggg gag aag gtc      528
   Leu Thr Gln Ser Pro Ala Ile Met Ser Ala Ser Pro Gly Glu Lys Val
                   165                 170                 175
   acc atg acc tgc agt gcc agc tca agt gta agt tac atg cac tgg tac      576
   Thr Met Thr Cys Ser Ala Ser Ser Ser Val Ser Tyr Met His Trp Tyr
               180                 185                 190
   cag cag aag tca ggc acc tcc ccc aaa aga tgg att tat gac aca tcc      624
   Gln Gln Lys Ser Gly Thr Ser Pro Lys Arg Trp Ile Tyr Asp Thr Ser
           195                 200                 205
   aaa ctg gct tct gga gtc cca ggt cgc ttc agt ggc agt ggg tct gga      672
   Lys Leu Ala Ser Gly Val Pro Gly Arg Phe Ser Gly Ser Gly Ser Gly
       210                 215                 220
   aac tct tac tct ctc aca atc agc agc gtg gag gct gaa gat gat gca      720
   Asn Ser Tyr Ser Leu Thr Ile Ser Ser Val Glu Ala Glu Asp Asp Ala
   225                 230                 235                 240
   act tat tac tgc cag cag tgg agt aag cac cct ctc acg tac ggt gct      768
   Thr Tyr Tyr Cys Gln Gln Trp Ser Lys His Pro Leu Thr Tyr Gly Ala
                   245                 250                 255
   ggg aca aag ttg gaa atc aaa gct agc ggt ggc gga ggt tct gga ggt      816
   Gly Thr Lys Leu Glu Ile Lys Ala Ser Gly Gly Gly Gly Ser Gly Gly
               260                 265                 270
   ggg ggt tcc tta acc aca gag acg gga ctc cag aaa gac cat gcc ctc      864
   Gly Gly Ser Leu Thr Thr Glu Thr Gly Leu Gln Lys Asp His Ala Leu
           275                 280                 285
   tgg gat cac act gcc cag aat ctc ctt cgg atg ggc ctg gcc ttt cta        912
   Trp Asp His Thr Ala Gln Asn Leu Leu Arg Met Gly Leu Ala Phe Leu
       290                 295                 300
   gtc ctg gtg gct cta gtg tgg ttc ctg gtt gaa gac tgg ctc agc agg        960
   Val Leu Val Ala Leu Val Trp Phe Leu Val Glu Asp Trp Leu Ser Arg
   305                 310                 315                 320
   aag agg act aga gag cga gcc agc aga gct tcc act tgg gaa ggc agg       1008
   Lys Arg Thr Arg Glu Arg Ala Ser Arg Ala Ser Thr Trp Glu Gly Arg
                   325                 330                 335
   aga agg ctg aac aca cag act ctt tga                                   1035
   Arg Arg Leu Asn Thr Gln Thr Leu
               340
<210> 230
   <211> 344
   <212> PRT
   <213> Artificial Sequence
<220>
   <223> Description of Artificial Sequence: Synthetic polypeptide
<400> 230
   Met Ala Leu Pro Val Thr Ala Leu Leu Leu Pro Leu Ala Leu Leu Leu
   1               5                   10                  15
   His Ala Ala Arg Pro Gly Ser Gln Val Gln Leu Gln Gln Ser Gly Pro
               20                  25                  30
   Glu Leu Glu Lys Pro Gly Ala Ser Val Lys Ile Ser Cys Lys Ala Ser
           35                  40                  45
   Gly Tyr Ser Phe Thr Gly Tyr Thr Met Asn Trp Val Lys Gln Ser His
       50                  55                  60
   Gly Lys Ser Leu Glu Trp Ile Gly Leu Ile Thr Pro Tyr Asn Gly Ala
   65                  70                  75                  80
   Ser Ser Tyr Asn Gln Lys Phe Arg Gly Lys Ala Thr Leu Thr Val Asp
                   85                  90                  95
   Lys Ser Ser Ser Thr Ala Tyr Met Asp Leu Leu Ser Leu Thr Ser Glu
               100                 105                 110
   Asp Ser Ala Val Tyr Phe Cys Ala Arg Gly Gly Tyr Asp Gly Arg Gly
           115                 120                 125
   Phe Asp Tyr Trp Gly Gln Gly Thr Thr Val Thr Val Ser Ser Gly Gly
       130                 135                 140
   Gly Gly Ser Gly Gly Gly Gly Ser Ser Gly Gly Gly Ser Asp Ile Glu
   145 150 155 160
   Leu Thr Gln Ser Pro Ala Ile Met Ser Ala Ser Pro Gly Glu Lys Val
   165 170 175
   Thr Met Thr Cys Ser Ala Ser Ser Ser Val Ser Tyr Met His Trp Tyr
   180 185 190
   Gln Gln Lys Ser Gly Thr Ser Pro Lys Arg Trp Ile Tyr Asp Thr Ser
   195 200 205
   Lys Leu Ala Ser Gly Val Pro Gly Arg Phe Ser Gly Ser Gly Ser Gly
   210 215 220
   Asn Ser Tyr Ser Leu Thr Ile Ser Ser Val Glu Ala Glu Asp Asp Ala
   225 230 235 240
   Thr Tyr Tyr Cys Gln Gln Trp Ser Lys His Pro Leu Thr Tyr Gly Ala
   245 250 255
   Gly Thr Lys Leu Glu Ile Lys Ala Ser Gly Gly Gly Gly Ser Gly Gly
   260 265 270
   Gly Gly Ser Leu Thr Thr Glu Thr Gly Leu Gln Lys Asp His Ala Leu
   275 280 285
   Trp Asp His Thr Ala Gln Asn Leu Leu Arg Met Gly Leu Ala Phe Leu
   290 295 300
   Val Leu Val Ala Leu Val Trp Phe Leu Val Glu Asp Trp Leu Ser Arg
   305 310 315 320
   Lys Arg Thr Arg Glu Arg Ala Ser Arg Ala Ser Thr Trp Glu Gly Arg
   325 330 335
   Arg Arg Leu Asn Thr Gln Thr Leu
   340
<210> 231
   <211> 1194
   <212> DNA
   <213> Artificial Sequence
<220>
   <223> Description of Artificial Sequence: Synthetic polynucleotide
<220>
<210> 232
   <211> 397
   <212> PRT
   <213> Artificial Sequence
<220>
   <223> Description of Artificial Sequence: Synthetic polypeptide
<400> 232
   Met Ala Leu Pro Val Thr Ala Leu Leu Leu Pro Leu Ala Leu Leu Leu
   1               5                   10                  15
   His Ala Ala Arg Pro Gly Ser Gln Val Gln Leu Gln Gln Ser Gly Pro
               20                  25                  30
   Glu Leu Glu Lys Pro Gly Ala Ser Val Lys Ile Ser Cys Lys Ala Ser
           35                  40                  45
   Gly Tyr Ser Phe Thr Gly Tyr Thr Met Asn Trp Val Lys Gln Ser His
   50 55 60
   Gly Lys Ser Leu Glu Trp Ile Gly Leu Ile Thr Pro Tyr Asn Gly Ala
   65 70 75 80
   Ser Ser Tyr Asn Gln Lys Phe Arg Gly Lys Ala Thr Leu Thr Val Asp
   85 90 95
   Lys Ser Ser Ser Thr Ala Tyr Met Asp Leu Leu Ser Leu Thr Ser Glu
   100 105 110
   Asp Ser Ala Val Tyr Phe Cys Ala Arg Gly Gly Tyr Asp Gly Arg Gly
   115 120 125
   Phe Asp Tyr Trp Gly Gln Gly Thr Thr Val Thr Val Ser Ser Gly Gly
   130 135 140
   Gly Gly Ser Gly Gly Gly Gly Ser Gly Gly Gly Gly Ser Asp Ile Glu
   145 150 155 160
   Leu Thr Gln Ser Pro Ala Ile Met Ser Ala Ser Pro Gly Glu Lys Val
   165 170 175
   Thr Met Thr Cys Ser Ala Ser Ser Ser Val Ser Tyr Met His Trp Tyr
   180 185 190
   Gln Gln Lys Ser Gly Thr Ser Pro Lys Arg Trp Ile Tyr Asp Thr Ser
   195 200 205
   Lys Leu Ala Ser Gly Val Pro Gly Arg Phe Ser Gly Ser Gly Ser Gly
   210 215 220
   Asn Ser Tyr Ser Leu Thr Ile Ser Ser Val Glu Ala Glu Asp Asp Ala
   225 230 235 240
   Thr Tyr Tyr Cys Gln Gln Trp Ser Gly Tyr Pro Leu Thr Phe Gly Ala
   245 250 255
   Gly Thr Lys Leu Glu Ile Lys Gly Gly Asp Tyr Lys Asp Asp Asp Asp
   260 265 270
   Lys Ala Ser Gly Gly Gly Gly Ser Gly Gly Gly Gly Ser Ser Pro Thr
   275 280 285
   Glu Pro Ser Ser Glu Thr Gly Asn Pro Arg His Leu His Val Leu Ile
   290 295 300
   Gly Thr Ser Val Val Ile Ile Leu Phe Ile Leu Leu Leu Phe Phe Leu
   305 310 315 320
   Leu His Arg Trp Cys Cys Asn Lys Lys Asn Ala Val Val Met Asp Gln
   325 330 335
   Glu Pro Ala Gly Asn Arg Thr Val Asn Arg Glu Asp Ser Asp Glu Gln
   340 345 350
   Asp Pro Gln Glu Val Thr Tyr Ala Gln Leu Asn His Cys Val Phe Thr
   355 360 365
   Gln Arg Lys Ile Thr His Pro Ser Gln Arg Pro Lys Thr Pro Pro Thr
   370 375 380
   Asp Ile Ile Val Tyr Thr Glu Leu Pro Asn Ala Glu Pro
   385 390 395
<210> 233
   <211> 5000
   <212> DNA
   <213> Artificial Sequence
<220>
   <223> Description of Artificial Sequence: Synthetic polynucleotide
<220>
   <221> misc_feature
   <222> (1)..(5000)
   <223> This sequence may encompass 50-5000 nucleotides; See specification as filed for detailed description of substitutions and preferred embodiments
<400> 233
   aaaaaaaaaa aaaaaaaaaa aaaaaaaaaa aaaaaaaaaa aaaaaaaaaa aaaaaaaaaa 60
   aaaaaaaaaa aaaaaaaaaa aaaaaaaaaa aaaaaaaaaa aaaaaaaaaa aaaaaaaaaa 120
   aaaaaaaaaa aaaaaaaaaa aaaaaaaaaa aaaaaaaaaa aaaaaaaaaa aaaaaaaaaa 180
   aaaaaaaaaa aaaaaaaaaa aaaaaaaaaa aaaaaaaaaa aaaaaaaaaa aaaaaaaaaa 240
   aaaaaaaaaa aaaaaaaaaa aaaaaaaaaa aaaaaaaaaa aaaaaaaaaa aaaaaaaaaa 300
   aaaaaaaaaa aaaaaaaaaa aaaaaaaaaa aaaaaaaaaa aaaaaaaaaa aaaaaaaaaa 360
   aaaaaaaaaa aaaaaaaaaa aaaaaaaaaa aaaaaaaaaa aaaaaaaaaa aaaaaaaaaa 420
   aaaaaaaaaa aaaaaaaaaa aaaaaaaaaa aaaaaaaaaa aaaaaaaaaa aaaaaaaaaa 480
   aaaaaaaaaa aaaaaaaaaa aaaaaaaaaa aaaaaaaaaa aaaaaaaaaa aaaaaaaaaa 540
   aaaaaaaaaa aaaaaaaaaa aaaaaaaaaa aaaaaaaaaa aaaaaaaaaa aaaaaaaaaa 600
   aaaaaaaaaa aaaaaaaaaa aaaaaaaaaa aaaaaaaaaa aaaaaaaaaa aaaaaaaaaa 660
   aaaaaaaaaa aaaaaaaaaa aaaaaaaaaa aaaaaaaaaa aaaaaaaaaa aaaaaaaaaa 720
   aaaaaaaaaa aaaaaaaaaa aaaaaaaaaa aaaaaaaaaa aaaaaaaaaa aaaaaaaaaa 780
   aaaaaaaaaa aaaaaaaaaa aaaaaaaaaa aaaaaaaaaa aaaaaaaaaa aaaaaaaaaa 840
   aaaaaaaaaa aaaaaaaaaa aaaaaaaaaa aaaaaaaaaa aaaaaaaaaa aaaaaaaaaa 900
   aaaaaaaaaa aaaaaaaaaa aaaaaaaaaa aaaaaaaaaa aaaaaaaaaa aaaaaaaaaa 960
   aaaaaaaaaa aaaaaaaaaa aaaaaaaaaa aaaaaaaaaa aaaaaaaaaa aaaaaaaaaa 1020
   aaaaaaaaaa aaaaaaaaaa aaaaaaaaaa aaaaaaaaaa aaaaaaaaaa aaaaaaaaaa 1080
   aaaaaaaaaa aaaaaaaaaa aaaaaaaaaa aaaaaaaaaa aaaaaaaaaa aaaaaaaaaa 1140
   aaaaaaaaaa aaaaaaaaaa aaaaaaaaaa aaaaaaaaaa aaaaaaaaaa aaaaaaaaaa 1200
   aaaaaaaaaa aaaaaaaaaa aaaaaaaaaa aaaaaaaaaa aaaaaaaaaa aaaaaaaaaa 1260
   aaaaaaaaaa aaaaaaaaaa aaaaaaaaaa aaaaaaaaaa aaaaaaaaaa aaaaaaaaaa 1320
   aaaaaaaaaa aaaaaaaaaa aaaaaaaaaa aaaaaaaaaa aaaaaaaaaa aaaaaaaaaa 1380
   aaaaaaaaaa aaaaaaaaaa aaaaaaaaaa aaaaaaaaaa aaaaaaaaaa aaaaaaaaaa 1440
   aaaaaaaaaa aaaaaaaaaa aaaaaaaaaa aaaaaaaaaa aaaaaaaaaa aaaaaaaaaa 1500
   aaaaaaaaaa aaaaaaaaaa aaaaaaaaaa aaaaaaaaaa aaaaaaaaaa aaaaaaaaaa 1560
   aaaaaaaaaa aaaaaaaaaa aaaaaaaaaa aaaaaaaaaa aaaaaaaaaa aaaaaaaaaa 1620
   aaaaaaaaaa aaaaaaaaaa aaaaaaaaaa aaaaaaaaaa aaaaaaaaaa aaaaaaaaaa 1680
   aaaaaaaaaa aaaaaaaaaa aaaaaaaaaa aaaaaaaaaa aaaaaaaaaa aaaaaaaaaa 1740
   aaaaaaaaaa aaaaaaaaaa aaaaaaaaaa aaaaaaaaaa aaaaaaaaaa aaaaaaaaaa 1800
   aaaaaaaaaa aaaaaaaaaa aaaaaaaaaa aaaaaaaaaa aaaaaaaaaa aaaaaaaaaa 1860
   aaaaaaaaaa aaaaaaaaaa aaaaaaaaaa aaaaaaaaaa aaaaaaaaaa aaaaaaaaaa 1920
   aaaaaaaaaa aaaaaaaaaa aaaaaaaaaa aaaaaaaaaa aaaaaaaaaa aaaaaaaaaa 1980
   aaaaaaaaaa aaaaaaaaaa aaaaaaaaaa aaaaaaaaaa aaaaaaaaaa aaaaaaaaaa 2040
   aaaaaaaaaa aaaaaaaaaa aaaaaaaaaa aaaaaaaaaa aaaaaaaaaa aaaaaaaaaa 2100
   aaaaaaaaaa aaaaaaaaaa aaaaaaaaaa aaaaaaaaaa aaaaaaaaaa aaaaaaaaaa 2160
   aaaaaaaaaa aaaaaaaaaa aaaaaaaaaa aaaaaaaaaa aaaaaaaaaa aaaaaaaaaa 2220
   aaaaaaaaaa aaaaaaaaaa aaaaaaaaaa aaaaaaaaaa aaaaaaaaaa aaaaaaaaaa 2280
   aaaaaaaaaa aaaaaaaaaa aaaaaaaaaa aaaaaaaaaa aaaaaaaaaa aaaaaaaaaa 2340
   aaaaaaaaaa aaaaaaaaaa aaaaaaaaaa aaaaaaaaaa aaaaaaaaaa aaaaaaaaaa 2400
   aaaaaaaaaa aaaaaaaaaa aaaaaaaaaa aaaaaaaaaa aaaaaaaaaa aaaaaaaaaa 2460
   aaaaaaaaaa aaaaaaaaaa aaaaaaaaaa aaaaaaaaaa aaaaaaaaaa aaaaaaaaaa 2520
   aaaaaaaaaa aaaaaaaaaa aaaaaaaaaa aaaaaaaaaa aaaaaaaaaa aaaaaaaaaa 2580
   aaaaaaaaaa aaaaaaaaaa aaaaaaaaaa aaaaaaaaaa aaaaaaaaaa aaaaaaaaaa 2640
   aaaaaaaaaa aaaaaaaaaa aaaaaaaaaa aaaaaaaaaa aaaaaaaaaa aaaaaaaaaa 2700
   aaaaaaaaaa aaaaaaaaaa aaaaaaaaaa aaaaaaaaaa aaaaaaaaaa aaaaaaaaaa 2760
   aaaaaaaaaa aaaaaaaaaa aaaaaaaaaa aaaaaaaaaa aaaaaaaaaa aaaaaaaaaa 2820
   aaaaaaaaaa aaaaaaaaaa aaaaaaaaaa aaaaaaaaaa aaaaaaaaaa aaaaaaaaaa 2880
   aaaaaaaaaa aaaaaaaaaa aaaaaaaaaa aaaaaaaaaa aaaaaaaaaa aaaaaaaaaa 2940
   aaaaaaaaaa aaaaaaaaaa aaaaaaaaaa aaaaaaaaaa aaaaaaaaaa aaaaaaaaaa 3000
   aaaaaaaaaa aaaaaaaaaa aaaaaaaaaa aaaaaaaaaa aaaaaaaaaa aaaaaaaaaa 3060
   aaaaaaaaaa aaaaaaaaaa aaaaaaaaaa aaaaaaaaaa aaaaaaaaaa aaaaaaaaaa 3120
   aaaaaaaaaa aaaaaaaaaa aaaaaaaaaa aaaaaaaaaa aaaaaaaaaa aaaaaaaaaa 3180
   aaaaaaaaaa aaaaaaaaaa aaaaaaaaaa aaaaaaaaaa aaaaaaaaaa aaaaaaaaaa 3240
   aaaaaaaaaa aaaaaaaaaa aaaaaaaaaa aaaaaaaaaa aaaaaaaaaa aaaaaaaaaa 3300
   aaaaaaaaaa aaaaaaaaaa aaaaaaaaaa aaaaaaaaaa aaaaaaaaaa aaaaaaaaaa 3360
   aaaaaaaaaa aaaaaaaaaa aaaaaaaaaa aaaaaaaaaa aaaaaaaaaa aaaaaaaaaa 3420
   aaaaaaaaaa aaaaaaaaaa aaaaaaaaaa aaaaaaaaaa aaaaaaaaaa aaaaaaaaaa 3480
   aaaaaaaaaa aaaaaaaaaa aaaaaaaaaa aaaaaaaaaa aaaaaaaaaa aaaaaaaaaa 3540
   aaaaaaaaaa aaaaaaaaaa aaaaaaaaaa aaaaaaaaaa aaaaaaaaaa aaaaaaaaaa 3600
   aaaaaaaaaa aaaaaaaaaa aaaaaaaaaa aaaaaaaaaa aaaaaaaaaa aaaaaaaaaa 3660
   aaaaaaaaaa aaaaaaaaaa aaaaaaaaaa aaaaaaaaaa aaaaaaaaaa aaaaaaaaaa 3720
   aaaaaaaaaa aaaaaaaaaa aaaaaaaaaa aaaaaaaaaa aaaaaaaaaa aaaaaaaaaa 3780
   aaaaaaaaaa aaaaaaaaaa aaaaaaaaaa aaaaaaaaaa aaaaaaaaaa aaaaaaaaaa 3840
   aaaaaaaaaa aaaaaaaaaa aaaaaaaaaa aaaaaaaaaa aaaaaaaaaa aaaaaaaaaa 3900
   aaaaaaaaaa aaaaaaaaaa aaaaaaaaaa aaaaaaaaaa aaaaaaaaaa aaaaaaaaaa 3960
   aaaaaaaaaa aaaaaaaaaa aaaaaaaaaa aaaaaaaaaa aaaaaaaaaa aaaaaaaaaa 4020
   aaaaaaaaaa aaaaaaaaaa aaaaaaaaaa aaaaaaaaaa aaaaaaaaaa aaaaaaaaaa 4080
   aaaaaaaaaa aaaaaaaaaa aaaaaaaaaa aaaaaaaaaa aaaaaaaaaa aaaaaaaaaa 4140
   aaaaaaaaaa aaaaaaaaaa aaaaaaaaaa aaaaaaaaaa aaaaaaaaaa aaaaaaaaaa 4200
   aaaaaaaaaa aaaaaaaaaa aaaaaaaaaa aaaaaaaaaa aaaaaaaaaa aaaaaaaaaa 4260
   aaaaaaaaaa aaaaaaaaaa aaaaaaaaaa aaaaaaaaaa aaaaaaaaaa aaaaaaaaaa 4320
   aaaaaaaaaa aaaaaaaaaa aaaaaaaaaa aaaaaaaaaa aaaaaaaaaa aaaaaaaaaa 4380
   aaaaaaaaaa aaaaaaaaaa aaaaaaaaaa aaaaaaaaaa aaaaaaaaaa aaaaaaaaaa 4440
   aaaaaaaaaa aaaaaaaaaa aaaaaaaaaa aaaaaaaaaa aaaaaaaaaa aaaaaaaaaa 4500
   aaaaaaaaaa aaaaaaaaaa aaaaaaaaaa aaaaaaaaaa aaaaaaaaaa aaaaaaaaaa 4560
   aaaaaaaaaa aaaaaaaaaa aaaaaaaaaa aaaaaaaaaa aaaaaaaaaa aaaaaaaaaa 4620
   aaaaaaaaaa aaaaaaaaaa aaaaaaaaaa aaaaaaaaaa aaaaaaaaaa aaaaaaaaaa 4680
   aaaaaaaaaa aaaaaaaaaa aaaaaaaaaa aaaaaaaaaa aaaaaaaaaa aaaaaaaaaa 4740
   aaaaaaaaaa aaaaaaaaaa aaaaaaaaaa aaaaaaaaaa aaaaaaaaaa aaaaaaaaaa 4800
   aaaaaaaaaa aaaaaaaaaa aaaaaaaaaa aaaaaaaaaa aaaaaaaaaa aaaaaaaaaa 4860
   aaaaaaaaaa aaaaaaaaaa aaaaaaaaaa aaaaaaaaaa aaaaaaaaaa aaaaaaaaaa 4920
   aaaaaaaaaa aaaaaaaaaa aaaaaaaaaa aaaaaaaaaa aaaaaaaaaa aaaaaaaaaa 4980
   aaaaaaaaaa aaaaaaaaaa 5000
<210> 234
   <211> 2000
   <212> DNA
   <213> Artificial Sequence
<220>
   <223> Description of Artificial Sequence: Synthetic polynucleotide
<220>
   <221> misc_feature
   <222> (1)..(2000)
   <223> This sequence may encompass 50-2000 nucleotides
<400> 234
   aaaaaaaaaa aaaaaaaaaa aaaaaaaaaa aaaaaaaaaa aaaaaaaaaa aaaaaaaaaa 60
   aaaaaaaaaa aaaaaaaaaa aaaaaaaaaa aaaaaaaaaa aaaaaaaaaa aaaaaaaaaa 120
   aaaaaaaaaa aaaaaaaaaa aaaaaaaaaa aaaaaaaaaa aaaaaaaaaa aaaaaaaaaa 180
   aaaaaaaaaa aaaaaaaaaa aaaaaaaaaa aaaaaaaaaa aaaaaaaaaa aaaaaaaaaa 240
   aaaaaaaaaa aaaaaaaaaa aaaaaaaaaa aaaaaaaaaa aaaaaaaaaa aaaaaaaaaa 300
   aaaaaaaaaa aaaaaaaaaa aaaaaaaaaa aaaaaaaaaa aaaaaaaaaa aaaaaaaaaa 360
   aaaaaaaaaa aaaaaaaaaa aaaaaaaaaa aaaaaaaaaa aaaaaaaaaa aaaaaaaaaa 420
   aaaaaaaaaa aaaaaaaaaa aaaaaaaaaa aaaaaaaaaa aaaaaaaaaa aaaaaaaaaa 480
   aaaaaaaaaa aaaaaaaaaa aaaaaaaaaa aaaaaaaaaa aaaaaaaaaa aaaaaaaaaa 540
   aaaaaaaaaa aaaaaaaaaa aaaaaaaaaa aaaaaaaaaa aaaaaaaaaa aaaaaaaaaa 600
   aaaaaaaaaa aaaaaaaaaa aaaaaaaaaa aaaaaaaaaa aaaaaaaaaa aaaaaaaaaa 660
   aaaaaaaaaa aaaaaaaaaa aaaaaaaaaa aaaaaaaaaa aaaaaaaaaa aaaaaaaaaa 720
   aaaaaaaaaa aaaaaaaaaa aaaaaaaaaa aaaaaaaaaa aaaaaaaaaa aaaaaaaaaa 780
   aaaaaaaaaa aaaaaaaaaa aaaaaaaaaa aaaaaaaaaa aaaaaaaaaa aaaaaaaaaa 840
   aaaaaaaaaa aaaaaaaaaa aaaaaaaaaa aaaaaaaaaa aaaaaaaaaa aaaaaaaaaa 900
   aaaaaaaaaa aaaaaaaaaa aaaaaaaaaa aaaaaaaaaa aaaaaaaaaa aaaaaaaaaa 960
   aaaaaaaaaa aaaaaaaaaa aaaaaaaaaa aaaaaaaaaa aaaaaaaaaa aaaaaaaaaa 1020
   aaaaaaaaaa aaaaaaaaaa aaaaaaaaaa aaaaaaaaaa aaaaaaaaaa aaaaaaaaaa 1080
   aaaaaaaaaa aaaaaaaaaa aaaaaaaaaa aaaaaaaaaa aaaaaaaaaa aaaaaaaaaa 1140
   aaaaaaaaaa aaaaaaaaaa aaaaaaaaaa aaaaaaaaaa aaaaaaaaaa aaaaaaaaaa 1200
   aaaaaaaaaa aaaaaaaaaa aaaaaaaaaa aaaaaaaaaa aaaaaaaaaa aaaaaaaaaa 1260
   aaaaaaaaaa aaaaaaaaaa aaaaaaaaaa aaaaaaaaaa aaaaaaaaaa aaaaaaaaaa 1320
   aaaaaaaaaa aaaaaaaaaa aaaaaaaaaa aaaaaaaaaa aaaaaaaaaa aaaaaaaaaa 1380
   aaaaaaaaaa aaaaaaaaaa aaaaaaaaaa aaaaaaaaaa aaaaaaaaaa aaaaaaaaaa 1440
   aaaaaaaaaa aaaaaaaaaa aaaaaaaaaa aaaaaaaaaa aaaaaaaaaa aaaaaaaaaa 1500
   aaaaaaaaaa aaaaaaaaaa aaaaaaaaaa aaaaaaaaaa aaaaaaaaaa aaaaaaaaaa 1560
   aaaaaaaaaa aaaaaaaaaa aaaaaaaaaa aaaaaaaaaa aaaaaaaaaa aaaaaaaaaa 1620
   aaaaaaaaaa aaaaaaaaaa aaaaaaaaaa aaaaaaaaaa aaaaaaaaaa aaaaaaaaaa 1680
   aaaaaaaaaa aaaaaaaaaa aaaaaaaaaa aaaaaaaaaa aaaaaaaaaa aaaaaaaaaa 1740
   aaaaaaaaaa aaaaaaaaaa aaaaaaaaaa aaaaaaaaaa aaaaaaaaaa aaaaaaaaaa 1800
   aaaaaaaaaa aaaaaaaaaa aaaaaaaaaa aaaaaaaaaa aaaaaaaaaa aaaaaaaaaa 1860
   aaaaaaaaaa aaaaaaaaaa aaaaaaaaaa aaaaaaaaaa aaaaaaaaaa aaaaaaaaaa 1920
   aaaaaaaaaa aaaaaaaaaa aaaaaaaaaa aaaaaaaaaa aaaaaaaaaa aaaaaaaaaa 1980
   aaaaaaaaaa aaaaaaaaaa 2000
<210> 235
   <211> 20
   <212> PRT
   <213> Artificial Sequence
<220>
   <223> Description of Artificial Sequence: Synthetic peptide
<400> 235
   Gly Gly Gly Gly Ser Gly Gly Gly Gly Ser Gly Gly Gly Gly Ser Gly
   1 5 10 15
   Gly Gly Gly Ser
   20
<210> 236
   <211> 15
   <212> PRT
   <213> Artificial Sequence
<220>
   <223> Description of Artificial Sequence: Synthetic peptide
<400> 236
   Gly Gly Gly Gly Ser Gly Gly Gly Gly Ser Gly Gly Gly Gly Ser
   1 5 10 15
<210> 237
   <211> 4
   <212> PRT
   <213> Artificial Sequence
<220>
   <223> Description of Artificial Sequence: Synthetic peptide
<400> 237
   Gly Gly Gly Ser
   1
<210> 238
   <211> 100
   <212> DNA
   <213> Artificial Sequence
<220>
   <223> Description of Artificial Sequence: Synthetic polynucleotide
<400> 238
   tttttttttt tttttttttt tttttttttt tttttttttt tttttttttt tttttttttt 60
   tttttttttt tttttttttt tttttttttt tttttttttt 100
<210> 239
   <211> 400
   <212> DNA
   <213> Artificial Sequence
<220>
   <223> Description of Artificial Sequence: Synthetic polynucleotide
<220>
   <221> misc_feature
   <222> (1)..(400)
   <223> This sequence may encompass 100-400 nucleotides
<400> 239
   aaaaaaaaaa aaaaaaaaaa aaaaaaaaaa aaaaaaaaaa aaaaaaaaaa aaaaaaaaaa 60
   aaaaaaaaaa aaaaaaaaaa aaaaaaaaaa aaaaaaaaaa aaaaaaaaaa aaaaaaaaaa 120
   aaaaaaaaaa aaaaaaaaaa aaaaaaaaaa aaaaaaaaaa aaaaaaaaaa aaaaaaaaaa 180
   aaaaaaaaaa aaaaaaaaaa aaaaaaaaaa aaaaaaaaaa aaaaaaaaaa aaaaaaaaaa 240
   aaaaaaaaaa aaaaaaaaaa aaaaaaaaaa aaaaaaaaaa aaaaaaaaaa aaaaaaaaaa 300
   aaaaaaaaaa aaaaaaaaaa aaaaaaaaaa aaaaaaaaaa aaaaaaaaaa aaaaaaaaaa 360
   aaaaaaaaaa aaaaaaaaaa aaaaaaaaaa aaaaaaaaaa 400
<210> 240
   <211> 16
   <212> PRT
   <213> Artificial Sequence
<220>
   <223> Description of Artificial Sequence: Synthetic peptide
<220>
   <221> MOD_RES
   <222> (2)..(3)
   <223> Any amino acid
<220>
   <221> MOD_RES
   <222> (5)..(12)
   <223> Any amino acid and this region may encompass 6-8 residues wherein some positions may be absent
<220>
   <221> MOD_RES
   <222> (14)..(15)
   <223> Any amino acid
<400> 240
   Tyr Xaa Xaa Leu Xaa Xaa Xaa Xaa Xaa Xaa Xaa Xaa Tyr Xaa Xaa Leu
   1 5 10 15
<210> 241
   <211> 5000
   <212> DNA
   <213> Artificial Sequence
<220>
   <223> Description of Artificial Sequence: Synthetic polynucleotide
<220>
   <221> misc_feature
   <222> (1)..(5000)
   <223> This sequence may encompass 100-5000 nucleotides
<400> 241
   aaaaaaaaaa aaaaaaaaaa aaaaaaaaaa aaaaaaaaaa aaaaaaaaaa aaaaaaaaaa 60
   aaaaaaaaaa aaaaaaaaaa aaaaaaaaaa aaaaaaaaaa aaaaaaaaaa aaaaaaaaaa 120
   aaaaaaaaaa aaaaaaaaaa aaaaaaaaaa aaaaaaaaaa aaaaaaaaaa aaaaaaaaaa 180
   aaaaaaaaaa aaaaaaaaaa aaaaaaaaaa aaaaaaaaaa aaaaaaaaaa aaaaaaaaaa 240
   aaaaaaaaaa aaaaaaaaaa aaaaaaaaaa aaaaaaaaaa aaaaaaaaaa aaaaaaaaaa 300
   aaaaaaaaaa aaaaaaaaaa aaaaaaaaaa aaaaaaaaaa aaaaaaaaaa aaaaaaaaaa 360
   aaaaaaaaaa aaaaaaaaaa aaaaaaaaaa aaaaaaaaaa aaaaaaaaaa aaaaaaaaaa 420
   aaaaaaaaaa aaaaaaaaaa aaaaaaaaaa aaaaaaaaaa aaaaaaaaaa aaaaaaaaaa 480
   aaaaaaaaaa aaaaaaaaaa aaaaaaaaaa aaaaaaaaaa aaaaaaaaaa aaaaaaaaaa 540
   aaaaaaaaaa aaaaaaaaaa aaaaaaaaaa aaaaaaaaaa aaaaaaaaaa aaaaaaaaaa 600
   aaaaaaaaaa aaaaaaaaaa aaaaaaaaaa aaaaaaaaaa aaaaaaaaaa aaaaaaaaaa 660
   aaaaaaaaaa aaaaaaaaaa aaaaaaaaaa aaaaaaaaaa aaaaaaaaaa aaaaaaaaaa 720
   aaaaaaaaaa aaaaaaaaaa aaaaaaaaaa aaaaaaaaaa aaaaaaaaaa aaaaaaaaaa 780
   aaaaaaaaaa aaaaaaaaaa aaaaaaaaaa aaaaaaaaaa aaaaaaaaaa aaaaaaaaaa 840
   aaaaaaaaaa aaaaaaaaaa aaaaaaaaaa aaaaaaaaaa aaaaaaaaaa aaaaaaaaaa 900
   aaaaaaaaaa aaaaaaaaaa aaaaaaaaaa aaaaaaaaaa aaaaaaaaaa aaaaaaaaaa 960
   aaaaaaaaaa aaaaaaaaaa aaaaaaaaaa aaaaaaaaaa aaaaaaaaaa aaaaaaaaaa 1020
   aaaaaaaaaa aaaaaaaaaa aaaaaaaaaa aaaaaaaaaa aaaaaaaaaa aaaaaaaaaa 1080
   aaaaaaaaaa aaaaaaaaaa aaaaaaaaaa aaaaaaaaaa aaaaaaaaaa aaaaaaaaaa 1140
   aaaaaaaaaa aaaaaaaaaa aaaaaaaaaa aaaaaaaaaa aaaaaaaaaa aaaaaaaaaa 1200
   aaaaaaaaaa aaaaaaaaaa aaaaaaaaaa aaaaaaaaaa aaaaaaaaaa aaaaaaaaaa 1260
   aaaaaaaaaa aaaaaaaaaa aaaaaaaaaa aaaaaaaaaa aaaaaaaaaa aaaaaaaaaa 1320
   aaaaaaaaaa aaaaaaaaaa aaaaaaaaaa aaaaaaaaaa aaaaaaaaaa aaaaaaaaaa 1380
   aaaaaaaaaa aaaaaaaaaa aaaaaaaaaa aaaaaaaaaa aaaaaaaaaa aaaaaaaaaa 1440
   aaaaaaaaaa aaaaaaaaaa aaaaaaaaaa aaaaaaaaaa aaaaaaaaaa aaaaaaaaaa 1500
   aaaaaaaaaa aaaaaaaaaa aaaaaaaaaa aaaaaaaaaa aaaaaaaaaa aaaaaaaaaa 1560
   aaaaaaaaaa aaaaaaaaaa aaaaaaaaaa aaaaaaaaaa aaaaaaaaaa aaaaaaaaaa 1620
   aaaaaaaaaa aaaaaaaaaa aaaaaaaaaa aaaaaaaaaa aaaaaaaaaa aaaaaaaaaa 1680
   aaaaaaaaaa aaaaaaaaaa aaaaaaaaaa aaaaaaaaaa aaaaaaaaaa aaaaaaaaaa 1740
   aaaaaaaaaa aaaaaaaaaa aaaaaaaaaa aaaaaaaaaa aaaaaaaaaa aaaaaaaaaa 1800
   aaaaaaaaaa aaaaaaaaaa aaaaaaaaaa aaaaaaaaaa aaaaaaaaaa aaaaaaaaaa 1860
   aaaaaaaaaa aaaaaaaaaa aaaaaaaaaa aaaaaaaaaa aaaaaaaaaa aaaaaaaaaa 1920
   aaaaaaaaaa aaaaaaaaaa aaaaaaaaaa aaaaaaaaaa aaaaaaaaaa aaaaaaaaaa 1980
   aaaaaaaaaa aaaaaaaaaa aaaaaaaaaa aaaaaaaaaa aaaaaaaaaa aaaaaaaaaa 2040
   aaaaaaaaaa aaaaaaaaaa aaaaaaaaaa aaaaaaaaaa aaaaaaaaaa aaaaaaaaaa 2100
   aaaaaaaaaa aaaaaaaaaa aaaaaaaaaa aaaaaaaaaa aaaaaaaaaa aaaaaaaaaa 2160
   aaaaaaaaaa aaaaaaaaaa aaaaaaaaaa aaaaaaaaaa aaaaaaaaaa aaaaaaaaaa 2220
   aaaaaaaaaa aaaaaaaaaa aaaaaaaaaa aaaaaaaaaa aaaaaaaaaa aaaaaaaaaa 2280
   aaaaaaaaaa aaaaaaaaaa aaaaaaaaaa aaaaaaaaaa aaaaaaaaaa aaaaaaaaaa 2340
   aaaaaaaaaa aaaaaaaaaa aaaaaaaaaa aaaaaaaaaa aaaaaaaaaa aaaaaaaaaa 2400
   aaaaaaaaaa aaaaaaaaaa aaaaaaaaaa aaaaaaaaaa aaaaaaaaaa aaaaaaaaaa 2460
   aaaaaaaaaa aaaaaaaaaa aaaaaaaaaa aaaaaaaaaa aaaaaaaaaa aaaaaaaaaa 2520
   aaaaaaaaaa aaaaaaaaaa aaaaaaaaaa aaaaaaaaaa aaaaaaaaaa aaaaaaaaaa 2580
   aaaaaaaaaa aaaaaaaaaa aaaaaaaaaa aaaaaaaaaa aaaaaaaaaa aaaaaaaaaa 2640
   aaaaaaaaaa aaaaaaaaaa aaaaaaaaaa aaaaaaaaaa aaaaaaaaaa aaaaaaaaaa 2700
   aaaaaaaaaa aaaaaaaaaa aaaaaaaaaa aaaaaaaaaa aaaaaaaaaa aaaaaaaaaa 2760
   aaaaaaaaaa aaaaaaaaaa aaaaaaaaaa aaaaaaaaaa aaaaaaaaaa aaaaaaaaaa 2820
   aaaaaaaaaa aaaaaaaaaa aaaaaaaaaa aaaaaaaaaa aaaaaaaaaa aaaaaaaaaa 2880
   aaaaaaaaaa aaaaaaaaaa aaaaaaaaaa aaaaaaaaaa aaaaaaaaaa aaaaaaaaaa 2940
   aaaaaaaaaa aaaaaaaaaa aaaaaaaaaa aaaaaaaaaa aaaaaaaaaa aaaaaaaaaa 3000
   aaaaaaaaaa aaaaaaaaaa aaaaaaaaaa aaaaaaaaaa aaaaaaaaaa aaaaaaaaaa 3060
   aaaaaaaaaa aaaaaaaaaa aaaaaaaaaa aaaaaaaaaa aaaaaaaaaa aaaaaaaaaa 3120
   aaaaaaaaaa aaaaaaaaaa aaaaaaaaaa aaaaaaaaaa aaaaaaaaaa aaaaaaaaaa 3180
   aaaaaaaaaa aaaaaaaaaa aaaaaaaaaa aaaaaaaaaa aaaaaaaaaa aaaaaaaaaa 3240
   aaaaaaaaaa aaaaaaaaaa aaaaaaaaaa aaaaaaaaaa aaaaaaaaaa aaaaaaaaaa 3300
   aaaaaaaaaa aaaaaaaaaa aaaaaaaaaa aaaaaaaaaa aaaaaaaaaa aaaaaaaaaa 3360
   aaaaaaaaaa aaaaaaaaaa aaaaaaaaaa aaaaaaaaaa aaaaaaaaaa aaaaaaaaaa 3420
   aaaaaaaaaa aaaaaaaaaa aaaaaaaaaa aaaaaaaaaa aaaaaaaaaa aaaaaaaaaa 3480
   aaaaaaaaaa aaaaaaaaaa aaaaaaaaaa aaaaaaaaaa aaaaaaaaaa aaaaaaaaaa 3540
   aaaaaaaaaa aaaaaaaaaa aaaaaaaaaa aaaaaaaaaa aaaaaaaaaa aaaaaaaaaa 3600
   aaaaaaaaaa aaaaaaaaaa aaaaaaaaaa aaaaaaaaaa aaaaaaaaaa aaaaaaaaaa 3660
   aaaaaaaaaa aaaaaaaaaa aaaaaaaaaa aaaaaaaaaa aaaaaaaaaa aaaaaaaaaa 3720
   aaaaaaaaaa aaaaaaaaaa aaaaaaaaaa aaaaaaaaaa aaaaaaaaaa aaaaaaaaaa 3780
   aaaaaaaaaa aaaaaaaaaa aaaaaaaaaa aaaaaaaaaa aaaaaaaaaa aaaaaaaaaa 3840
   aaaaaaaaaa aaaaaaaaaa aaaaaaaaaa aaaaaaaaaa aaaaaaaaaa aaaaaaaaaa 3900
   aaaaaaaaaa aaaaaaaaaa aaaaaaaaaa aaaaaaaaaa aaaaaaaaaa aaaaaaaaaa    3960
   aaaaaaaaaa aaaaaaaaaa aaaaaaaaaa aaaaaaaaaa aaaaaaaaaa aaaaaaaaaa    4020
   aaaaaaaaaa aaaaaaaaaa aaaaaaaaaa aaaaaaaaaa aaaaaaaaaa aaaaaaaaaa    4080
   aaaaaaaaaa aaaaaaaaaa aaaaaaaaaa aaaaaaaaaa aaaaaaaaaa aaaaaaaaaa    4140
   aaaaaaaaaa aaaaaaaaaa aaaaaaaaaa aaaaaaaaaa aaaaaaaaaa aaaaaaaaaa    4200
   aaaaaaaaaa aaaaaaaaaa aaaaaaaaaa aaaaaaaaaa aaaaaaaaaa aaaaaaaaaa    4260
   aaaaaaaaaa aaaaaaaaaa aaaaaaaaaa aaaaaaaaaa aaaaaaaaaa aaaaaaaaaa    4320
   aaaaaaaaaa aaaaaaaaaa aaaaaaaaaa aaaaaaaaaa aaaaaaaaaa aaaaaaaaaa    4380
   aaaaaaaaaa aaaaaaaaaa aaaaaaaaaa aaaaaaaaaa aaaaaaaaaa aaaaaaaaaa    4440
   aaaaaaaaaa aaaaaaaaaa aaaaaaaaaa aaaaaaaaaa aaaaaaaaaa aaaaaaaaaa    4500
   aaaaaaaaaa aaaaaaaaaa aaaaaaaaaa aaaaaaaaaa aaaaaaaaaa aaaaaaaaaa    4560
   aaaaaaaaaa aaaaaaaaaa aaaaaaaaaa aaaaaaaaaa aaaaaaaaaa aaaaaaaaaa    4620
   aaaaaaaaaa aaaaaaaaaa aaaaaaaaaa aaaaaaaaaa aaaaaaaaaa aaaaaaaaaa    4680
   aaaaaaaaaa aaaaaaaaaa aaaaaaaaaa aaaaaaaaaa aaaaaaaaaa aaaaaaaaaa    4740
   aaaaaaaaaa aaaaaaaaaa aaaaaaaaaa aaaaaaaaaa aaaaaaaaaa aaaaaaaaaa    4800
   aaaaaaaaaa aaaaaaaaaa aaaaaaaaaa aaaaaaaaaa aaaaaaaaaa aaaaaaaaaa    4860
   aaaaaaaaaa aaaaaaaaaa aaaaaaaaaa aaaaaaaaaa aaaaaaaaaa aaaaaaaaaa    4920
   aaaaaaaaaa aaaaaaaaaa aaaaaaaaaa aaaaaaaaaa aaaaaaaaaa aaaaaaaaaa    4980
   aaaaaaaaaa aaaaaaaaaa                                                5000
<210> 242
   <211> 5
   <212> PRT
   <213> Artificial Sequence
<220>
   <223> Description of Artificial Sequence: Synthetic peptide
<400> 242
   Gly Gly Gly Gly Ser
   1               5

## Description

This application claims priority to U.S. Serial No. 61/953,822, filed March 15, 2014.

### FIELD OF THE INVENTION

The invention relates generally to a regulatable chimeric antigen receptors comprising components of a natural killer cell receptor (RNKR-CARs), and cells expressing such RNKR-CARs (RNKR-CARX cells) or cells expressing a combination of a regulatable chimeric antigen receptor (RCAR) and natural killer receptor chimeric antigen receptor (NKR-CAR) (RCAR/NKR-CARX cells), as well as methods of making and using the same, e.g., to target and inactivate or kill target cells, e.g., cancer cells.

### BACKGROUND

Adoptive cell transfer (ACT) therapy with autologous T-cells, especially with T-cells transduced with Chimeric Antigen Receptors (CARs), has shown promise in pilot hematologic cancer trials. WO 2014/127261 relates to heterodimeric, conditionally active CARs. WO 2014/145252 relates to CARs comprising components from a receptor found on natural killer (NK) cells. US 2011/003385 relates to regulated oligomerization and dimerization of intracellular proteins. US 2012/029063 relates to chimeric immune receptor molecules for reducing or eliminating tumors.

### SUMMARY

Embodiments of the invention address the optimization of safety and efficacy in the use of RNKR-CARX cells or RCAR/NKR-CARX cells to provide an immune response. Embodiments of the invention are based, in part, on the discovery that a CAR molecule can be partitioned such that a "binding domain" and a "signaling domain" are each linked to two separate "switch domains." In such embodiments, activation of signaling through the CAR only occurs when the switch domains, and hence the binding domain and the signaling domain, are brought together by a dimerization molecule, i.e. to switch "on" signaling through the CAR. Embodiments of the invention include, *inter alia,* the use of a dimerization switch that turns "on" the activation of a signal to allow external, e.g., temporal, control over the immune effector response mediated by a cell containing a RNKR-CAR or a cell containing a RCAR and a NKR-CAR. As discussed in more detail below, in embodiments, the RNKR-CAR or RCAR includes a dimerization switch that, upon the presence of a dimerization molecule, can couple an intracellular signaling domain to an extracellular recognition element, e.g., an antigen binding domain, an inhibitory counter ligand binding domain, or costimulatory ECD domain.

In some aspects, the disclosure features a purified, or non-naturally occurring, regulatable NKR-CAR (RNKR-CAR) comprising:
(a) an antigen binding member comprising: an binding domain element (e.g., an antigen binding domain, inhibitory extracellular domain, or costimulatory extracellular domain), a transmembrane domain, e.g., an NKR transmembrane domain, and a first switch domain, e.g., FKBP or FRB; and
(b) an intracellular signaling member comprising: a second switch domain, e.g., FKBP or FRB; and a cytoplasmic ITAM domain, e.g., a cytoplasmic DAP 12 signaling domain.

In one instance, said RNKR-CAR comprises a RKIR-CAR, e.g., an RactKIR-CAR, a RNCR-CAR, e.g., an RactNCR-CAR, a RFcR-CAR, e.g., an RactCD16-CAR, or an RactCD64-CAR, or an RLy49-CAR, e.g., an RactLy49-CAR.

In one instance, said RNKR-CAR comprises a RactKIR-CAR.

In another aspect, the disclosure features, a nucleic acid with comprises sequence that encodes: a RNKR-CAR, e.g., a RactKIR-CAR.

In an instance the nucleic acid further comprises a RCAR disclosed herein.

In another aspect, the disclosure features, a cytotoxic cell, e.g., a T cell, NK cell, or cultured NK cell, e.g., a NK92 cell, which comprises: a RNKR-CAR, e.g., a RactKIR-CAR.

In an instance the cell further comprises a RCAR disclosed herein.

In another aspect, the disclosure further comprises a method of treating a patient comprising:
administering to the patient a cytoxoic cell, e.g., a T cell, NK cell, or cultured NK cell, e.g., a NK92 cell, which comprises: a RNKR-CAR, e.g., a RactKIR-CAR.

In an instance the cell further comprises a RCAR disclosed herein.

In another aspect, the disclosure comprises a kit comprising a nucleic acid or cell described herein.

In an aspect, disclosed herein is a regulatable natural killer receptor CAR (RNKR-CAR), e.g., an isolated NKR-CAR, wherein the RNKR-CAR comprises:
a) an antigen binding member, comprising
   a binding domain element,
   a transmembrane domain,
   a first switch domain, and
   optionally, a NKR cytoplasmic domain,
      wherein the binding domain element comprises an antigen binding domain, an inhibitory extracellular domain, e.g., selected from Table 4, or a costimulatory extracellular domain, e.g., selected from Table 5; and
b) an intracellular signaling member comprising
   a second switch domain,
   a NKR cytoplasmic domain or an intracellular signaling domain, e.g., a primary signaling domain, e.g., a DAP12 signaling domain, or a CD3zeta signaling domain, and
   optionally, a transmembrane domain or a membrane tether.

More particularly, the present invention provides a regulatable natural killer receptor CAR (RNKR-CAR), wherein the RNKR-CAR comprises:
(a) an antigen binding member, comprising: a binding domain element that comprises an antigen binding domain derived from an antibody molecule, a transmembrane domain, and a first switch domain; and
(b) an intracellular signaling member comprising a second switch domain,
   wherein the first and second switch domains comprise a dimerization switch, further wherein either:
   (i) the antigen binding member further comprises a cytoplasmic domain, further wherein (1) the transmembrane and/or cytoplasmic domain comprises an NKR transmembrane or cytoplasmic domain, each of which is selected from a killer cell immunoglobulin-like receptor (KIR), a natural cytotoxicity receptor (NCR), the signaling lymphocyte activation molecule (SLAM) family of immune cell receptors, an Fc receptor (FcR), a Ly49 receptor, or an NKR of Table 24; and (2) the intracellular signaling member comprises a NKR cytoplasmic domain selected from Table 24, or a primary intracellular signaling domain selected from Table 1, or a cytoplasmic ITAM domain;
   (ii) the transmembrane domain comprises an NKR transmembrane domain from a killer cell immunoglobulin-like receptor (KIR), a natural cytotoxicity receptor (NCR), the signaling lymphocyte activation molecule (SLAM) family of immune cell receptors, an Fc receptor (FcR), a Ly49 receptor, or an NKR of Table 24, and the intracellular signaling member comprises a cytoplasmic ITAM domain; or
   (iii) the intracellular signaling member further comprises an NKR cytoplasmic domain selected from Table 24,

further wherein the dimerization switch comprises
a FKBP/FRB-based dimerization switch, optionally wherein the first and second switch domains are dimerized by a rapamycin or a rapamycin analogue;
a GyrB-GyrB based switch, optionally wherein the first and second switch domains are dimerized by coumermycin;
a GAI-GID1 based switch;
a gibberellin-based dimerization switch; or
a Halo-tag/SNAP-tag based switch.

In embodiments, the antigen binding member comprises a NKR cytoplasmic domain, e.g., selected from Table 24.

In an embodiment, a RNKR-CAR comprises an NKR cytoplasmic domain or an NKR transmembrane domain; and in an embodiment the NKR cytoplasmic domain is other than a FcR gamma (FCER1G), CD27, NKG2C, SLAMF7, NKP80 (KLRF1), CD160 (BY55), DNAM1 (CD226), SLAMF4 (CD244, 2B4), CD84, CD96 (Tactile), CEACAM1, CRTAM, Ly9 (CD229), PSGL1, CD100 (SEMA4D), SLAMF6 (NTB-A, Ly108), SLAM (SLAMF1, CD150, IPO-3), BLAME (SLAMF8), NKp44, NKp30, and/or NKp46 cytoplasmic domain. In an embodiment an RNKR-CAR comprises an NKR cytoplasmic domain and a primary signaling domain from an NK cell adaptor molecule, e.g., DAP12. In an embodiment an RNKR-CAR comprises an NKR transmembrane domain and a primary signaling domain from an NK cell adaptor molecule, e.g., DAP12. In an embodiment an RNKR-CAR comprises an NKR cytoplasmic domain (other than a FcR gamma (FCER1G), CD27, NKG2C, SLAMF7, NKP80 (KLRF1), CD160 (BY55), DNAM1 (CD226), SLAMF4 (CD244, 2B4), CD84, CD96 (Tactile), CEACAM1, CRTAM, Ly9 (CD229), PSGL1, CD100 (SEMA4D), SLAMF6 (NTB-A, Ly108), SLAM (SLAMF1, CD150, IPO-3), BLAME (SLAMF8), NKp44, NKp30, and/or NKp46 cytoplasmic domain) and a primary signaling domain from a T cell molecule, e.g., CD3zeta.

In embodiments, the antigen binding member comprises domains in the following orientation from N- to C- terminus: N-binding domain element---transmembrane domain-NKR cytoplasmic domain---first switch domain-C. In other embodiments, the antigen binding member comprises domains in the following orientation from N- to C- terminus: N-first switch domain---NKR cytoplasmic domain---transmembrane domain---binding domain element-C.
In embodiments, the antigen binding member comprises domains in the following orientation from N- to C- terminus: N-binding domain element---transmembrane domain-first switch domain--NKR cytoplasmic domain-C. In other embodiments, the antigen binding member comprises domains in the following orientation from N- to C- terminus: N-NKR cytoplasmic domain---first switch domain---transmembrane domain---binding domain element-C.

In some embodiments, the antigen binding member does not comprise a NKR cytoplasmic domain.

In embodiments, the antigen binding member comprises domains in the following orientation from N- to C- terminus of the polypeptide chain: N-binding domain element-transmembrane domain--- first switch domain-C. In embodiments, the antigen binding member comprises domains in the following orientation from N- to C- terminus of the polypeptide chain: N-first switch domain---transmembrane domain--- binding domain element-C. In embodiments, the intracellular signaling member comprises a NKR cytoplasmic domain, e.g., selected from Table 24. In embodiments, the intracellular signaling member comprises domains in the following orientation from N- to C- terminus: N-second switch domain---NKR cytoplasmic domain-C. In embodiments, the intracellular signaling member comprises domains in the following orientation from N- to C- terminus: N-NKR cytoplasmic domain---second switch domain-C. In embodiments, the intracellular signaling member does not comprise a NKR cytoplasmic domain.

In embodiments, the intracellular signaling member comprises an intracellular signaling domain, e.g., a primary signaling domain selected from Table 1. For example, the primary signaling domain comprises a CD3zeta domain. In an example, the primary signaling domain comprises a DAP12 domain.

In embodiments, the intracellular signaling member comprises domains in the following orientation from N- to C- terminus: N-intracellular signaling domain---second switch domain-C. In other embodiments, the intracellular signaling member comprises domains in the following orientation from N- to C- terminus: N-second switch domain-intracellular signaling domain-C. In embodiments, the intracellular signaling member comprises a NKR cytoplasmic domain and an intracellular signaling domain, e.g., a primary signaling domain selected from Table 1. In embodiments, the intracellular signaling member comprises domains in the following orientation from N- to C- terminus: N-second switch domain---NKR cytoplasmic domain---intracellular signaling domain-C. In embodiments, the intracellular signaling member comprises domains in the following orientation from N- to C- terminus: N-intracellular signaling domain---NKR cytoplasmic domain---second switch domain -C.

In embodiments, the intracellular signaling member comprises a transmembrane domain or a membrane tether. In embodiments, the transmembrane domain or the membrane tether is N-terminal to the second switch domain. In other cases, the transmembrane domain or the membrane tether is C-terminal to the second switch domain. In some cases, the transmembrane domain or the membrane tether is N-terminal to the intracellular signaling domain. In some cases, the transmembrane domain or the membrane tether is C-terminal to the intracellular signaling domain.

In certain embodiments, the antigen binding member does not comprise a NKR cytoplasmic domain and wherein the intracellular signaling member comprises a NKR cytoplasmic domain, e.g., selected from Table 24.

In embodiments, the antigen binding member does not comprise a NKR cytoplasmic domain, and wherein the intracellular signaling member comprises a NKR cytoplasmic domain, e.g., selected from Table 24, and a primary signaling domain, e.g., selected from Table 1.

In embodiments, the transmembrane domain of the antigen binding member and/or the intracellular signaling member comprises a NKR transmembrane domain, e.g., selected from Table 24. In embodiments, the NKR transmembrane domain can interact with, e.g., bind, an adaptor molecule or intracellular signaling molecule, e.g., DAP12. In embodiments, the NKR transmembrane domain can interact with, e.g., bind, the transmembrane domain of an adaptor molecule or intracellular signaling molecule, e.g., DAP12. In embodiments, the NKR transmembrane domain comprises a positively charged moiety, e.g., an amino acid residue comprising a positively charged moiety, e.g., side chain. In embodiments, the NKR transmembrane domain does not comprise a positively charged moiety, e.g., an amino acid residue comprising a positively charged moiety. In embodiments, the NKR transmembrane domain does not interact with (e.g., bind to) an adaptor molecule or intracellular signaling molecule, e.g., DAP12. In embodiments, the NKR transmembrane domain does not comprise a positively charged moiety, e.g., an amino acid residue comprising a positively charged moiety, that mediates binding of an NKR transmembrane to an adaptor molecule or intracellular signaling molecule, e.g., DAP12. In instances, a RNKR-CAR described herein comprises a mutated NKR transmembrane domain (e.g., a mutated KIR transmembrane domain, mutated NCR transmembrane domain, mutated FcR transmembrane domain, mutated Ly49 receptor transmembrane domain, mutated SLAMF receptor transmembrane domain). For example, a mutated NKR transmembrane domain comprises a mutation compared to a naturally occurring NKR transmembrane domain amino acid sequence, e.g., from a naturally occurring NKR described herein. For example, the NKR transmembrane domain comprises a mutation, e.g., where the mutated NKR transmembrane domain does not comprise a positively charged moiety, e.g., an amino acid residue comprising a positively charged moiety. For example, the mutated NKR transmembrane domain does not comprise one or more amino acids that mediate (e.g., that are necessary for) binding of an NKR transmembrane domain to an adaptor molecule or intracellular signaling molecule, e.g., DAP12. For example, the mutation in the mutated NKR transmembrane domain eliminates an amino acid comprising a positively charged moiety or an amino acid that mediates binding with an adaptor or intracellular signaling molecule from an endogenous or wildtype NKR transmembrane domain that normally has such an amino acid. For example, the mutated NKR transmembrane domain does not bind to (e.g., interact with) an adaptor molecule or intracellular signaling molecule.

In embodiments, the antigen binding member further comprises an extracellular hinge domain disposed between the transmembrane domain and the binding domain element.

In embodiments, the NKR transmembrane domain and the NKR cytoplasmic domain are from the same naturally-occurring NKR molecule.

In other embodiments, the transmembrane domain of the antigen binding member and/or the transmembrane domain of the intracellular signaling member is not derived from an NKR molecule. For example, the transmembrane domain of the antigen binding member and/or the transmembrane domain of the intracellular signaling member is derived from a T cell molecule. For example, the transmembrane domain of the antigen binding member and/or the transmembrane domain of the intracellular signaling member is derived from CD8alpha or CD3zeta.

In some embodiments, the antigen binding member comprises, from the extracellular to intracellular direction, an binding domain element, a transmembrane domain, a NKR cytoplasmic domain, and a first switch domain and the intracellular signaling member comprises, a primary signaling domain, e.g., a DAP12 domain, and a second switch domain. For example, the intracellular signaling member comprises the primary signaling domain and the second switch domain in the following orientation from N- to C- terminus: N-primary signaling domain---second switch domain-C. In other examples, the intracellular signaling member comprises the primary signaling domain and the second switch domain in the following orientation from N- to C- terminus: N-second switch domain---primary signaling domain-C.

In embodiments, the antigen binding member comprises, from the extracellular to intracellular direction, an binding domain element, a transmembrane domain, a first switch domain, and a NKR cytoplasmic domain and the intracellular signaling member comprises, a second switch domain and a primary signaling domain, e.g., a DAP12 domain.

In embodiments, the antigen binding member comprises, from the extracellular to intracellular direction, an binding domain element, a transmembrane domain, a NKR cytoplasmic domain, and a first switch domain and the intracellular signaling member comprises, a second switch domain and a primary signaling domain, e.g., a CD3zeta domain.

In embodiments, the antigen binding member comprises, from the extracellular to intracellular direction, an binding domain element, a transmembrane domain, and a first switch domain and the intracellular signaling member comprises, a second switch domain, a NKR cytoplasmic domain, and a primary signaling domain, e.g., a CD3zeta domain.

In embodiments, the antigen binding member comprises, from the extracellular to intracellular direction, an binding domain element, a transmembrane domain, and a first switch domain and the intracellular signaling member comprises, a second switch domain and a NKR cytoplasmic domain.

In embodiments, the RNKR-CAR comprises:
a regulatable killer immunoglobulin receptor-CAR (RKIR-CAR), e.g., an RactKIR-CAR;
a RNCR-CAR, e.g., an RactRNCR-CAR;
a RFcR-CAR, e.g., an RactCD16-CAR or an RactCD64-CAR; or
a RLy49-CAR, e.g., an RactLy49-CAR.

In embodiments, the antigen binding domain comprises an antibody, an antibody fragment, an scFv, a Fv, a Fab, a (Fab')2, a single domain antibody (SDAB), a VH or VL domain, or a camelid VHH domain. In embodiments, the antigen binding domain interacts with, e.g., binds, to a tumor antigen described herein. In embodiments, the tumor antigen is selected from selected from a group consisting of: CD19, CD123, CD22, CD30, CD171, CS-1, CLL-1, CD33, EGFRvIII, GD2, GD3, BCMA, Tn Ag, PSMA, ROR1, FLT3, FAP, TAG72, CD38, CD44v6, CEA, EPCAM, B7H3, KIT, IL-13Ra2, Mesothelin, IL-11Ra, PSCA, PRSS21, VEGFR2, LewisY, CD24, PDGFR-beta, SSEA-4, CD20, Folate receptor alpha, ERBB2 (Her2/neu), MUC1, EGFR, NCAM, Prostase, PAP, ELF2M, Ephrin B2, IGF-I receptor, CAIX, LMP2, gp100, bcr-abl, tyrosinase, EphA2, Fucosyl GM1, sLe, GM3, TGS5, HMWMAA, o-acetyl-GD2, Folate receptor beta, TEM1/CD248, TEM7R, CLDN6, TSHR, GPRC5D, CXORF61, CD97, CD179a, ALK, Plysialic acid, PLAC1, GloboH, NY-BR-1, UPK2, HAVCR1, ADRB3, PANX3, GPR20, LY6K, OR51E2, TARP, WT1, NY-ESO-1, LAGE-1a, MAGE-A1, legumain, HPV E6,E7, MAGE A1, ETV6-AML, sperm protein 17, XAGE1, Tie 2, MAD-CT-1, MAD-CT-2, Fos-related antigen 1, p53, p53 mutant, prostein, survivin and telomerase, PCTA-1/Galectin 8, MelanA/MART1, Ras mutant, hTERT, sarcoma translocation breakpoints, ML-IAP, ERG (TMPRSS2 ETS fusion gene), NA17, PAX3, Androgen receptor, Cyclin B1, MYCN, RhoC, TRP-2, CYP1B1, BORIS, SART3, PAX5, OY-TES1, LCK, AKAP-4, SSX2, RAGE-1, human telomerase reverse transcriptase, RU1, RU2, intestinal carboxyl esterase, mut hsp70-2, CD79a, CD79b, CD72, LAIR1, FCAR, LILRA2, CD300LF, CLEC12A, BST2, EMR2, LY75, GPC3, FCRL5, and IGLL1.

In some embodiments, the RNKR-CAR comprises a regulatable KIR-CAR (RKIR-CAR). In embodiments, the transmembrane domain comprises a KIR transmembrane domain selected from Table 24. For example, the transmembrane domain can interact with, e.g., bind, DAP12. In embodiments, the transmembrane domain of the antigen binding domain and/or the intracellular signaling domain does not comprise a KIR transmembrane domain or a mutated KIR transmembrane domain. In some cases, the NKR cytoplasmic domain comprises a KIR cytoplasmic domain selected from Table 24.

In embodiments, the RKIR-CAR is an activating RKIR-CAR (RactKIR-CAR). For example, the RactKIR-CAR comprises an activating KIR (actKIR) cytoplasmic domain. In some cases, the RactKIR-CAR can interact with and promote signaling from an ITAM-containing polypeptide or adaptor molecule, e.g., DAP12. In embodiments, the RactKIR-CAR comprises a KIR D domain, a KIR D1 domain, or a KIR D2 domain. In embodiments, the RactKIR-CAR comprises an actKIR cytoplasmic domain selected from KIR2DS1, KIR2DS2, KIR2DS3, KIR2DS4, or KIR2DS5. In embodiments, the RactKIR-CAR comprises an actKIR transmembrane domain selected from KIR2DS1, KIR2DS2, KIR2DS3, KIR2DS4, or KIR2DS5. In embodiments, the RactKIR-CAR comprises a KIR2DS2 transmembrane domain. In embodiments, the antigen binding domain binds to a tumor antigen.

In some embodiments, the RKIR-CAR is an inhibitory RKIR-CAR (RinhKIR-CAR). In embodiments, the RinhKIR-CAR comprises an inhKIR transmembrane domain. In embodiments, the RinhKIR-CAR comprises an ITIM-containing cytoplasmic domain, e.g, an inhKIR cytoplasmic domain. For example, the ITIM-containing cytoplasmic domain is selected from KIR2DL1, KIR2DL2/L3, KIR2DL5A, KIR2DL5B, KIR3DL2, or KIR3DL3.

In certain embodiments, the RinhKIR-CAR comprises a transmembrane domain other than a KIR transmembrane domain, e.g., a transmembrane domain from PD-1, CTLA4 or ITIM-containing receptors from ILT (CD85), Siglec, LMIR (CD300) and/ or SLAM gene families of receptors.

In embodiments, the RinhKIR-CAR comprises a cytoplasmic domain from an inhibitory receptor other than a KIR, e.g., from PD-1, CTLA4 or ITIM-containing receptors from ILT (CD85), Siglec, LMIR (CD300) and/ or SLAM gene families of receptors.

In embodiments, the RinhKIR-CAR comprises a transmembrane and cytoplasmic domain from an inhibitory receptor other than a KIR, e.g., transmembrane and cytoplasmic domain, independently, from e.g., PD-1, CTLA4 or ITIM-containing receptors from ILT (CD85), Siglec, LMIR (CD300) and/ or SLAM gene families of receptors.

In embodiments, the RinhKIR-CAR comprises a KIR D domain, a KIR D0 domain, a KIR D1 domain, or a KIR D2 domain.

In embodiments, the antigen binding domain of the RinhKIR-CAR binds an antigen that is more highly expressed on a non-target cell, e.g., a non-cancer cell, than a target cell, e.g., cancerous cell, e.g., a cancerous cell of the same type as the target cell.

In some embodiments, the RNKR-CAR comprises a regulatable NCR-CAR (RNCR-CAR). For example, the RNCR-CAR comprises a regulatable NKp30, NKp44, or NKp46-CAR. In some examples, the transmembrane domain is a NCR transmembrane domain selected from Table 24, e.g., a NKp30, NKp44, or NKp46 transmembrane domain. In embodiments, the NKR cytoplasmic domain is a NCR cytoplasmic domain selected from Table 24, e.g., a NKp30, NKp44, or NKp46 cytoplasmic domain.

In embodiments, the RNKR-CAR comprises a regulatable FcR-CAR (RFcR-CAR). In embodiments, the RFcR-CAR is a regulatable CD16-CAR. In embodiments, the RFcR-CAR is a regulatable CD64-CAR. In embodiments, the transmembrane domain is a FcR transmembrane domain selected from Table 24, e.g., a CD16 or CD64 transmembrane domain. In embodiments, the NKR cytoplasmic domain is a FcR cytoplasmic domain selected from Table 24, e.g., a CD16 or CD64 cytoplasmic domain.

In some embodiments, the RNKR-CAR comprises a regulatable Ly49-CAR (RLy49-CAR). In embodiments, the RLy49-CAR comprises a transmembrane domain and a Ly49 cytoplasmic domain. In embodiments, the RLy49-CAR is an activating Ly49-CAR, e.g., Ly49D or Ly49H. In embodiments, the RLy49-CAR comprises a positively charged transmembrane domain, e.g., a positively charged Ly49 transmembrane domain. In embodiments the RLy49-CAR is an inhibitory Ly49-CAR, e.g., Ly49A or Ly49C. In embodiments, the Rly49-CAR comprises an ITIM-containing cytoplasmic domain, e.g., a Ly49-cytoplasmic domain. In embodiments, the Rly49-CAR comprises a Ly49-transmembrane domain or a Ly49-cytoplasmic domain selected, independently from Ly49A-Ly49W. For example, the transmembrane domain is a Ly49 transmembrane domain selected from Table 24. For example, the NKR cytoplasmic domain is a Ly49 cytoplasmic domain selected from Table 24.

In the present invention, the first and second switch domains of the RNKR-CAR comprise a dimerization switch. In embodiments, the dimerization switch can be a homodimerization switch or a heterodimerization switch. In embodiments, the dimerization switch comprises a FKB-FRB based switch. For example, one of the first and second switch domains comprises an FKBP-based switch domain and the other comprises an FRB-based switch domain.

In embodiments, the switch domains are dimerized by a mTOR inhibitor, e.g., RAD001.

In embodiments, the FRB-based switch domain comprises an FKBP binding fragment or analog of FRB comprising any one of the following:
i) an E2032 mutation;
ii) an E2032I mutation or E2032L mutation;
iii) a T2098 mutation;
iv) a T2098L mutation;
v) an E2032 and a T2098 mutation;
vi) an E2032I and a T2098L mutation;
vii) or an E2032L and a T2098L mutation.

In certain embodiments, the dimerization switch comprises a GyrB-GyrB based switch. In embodiments, the dimerization molecule is a coumermycin.

In some embodiments, the dimerization switch comprises a GAI-GID1 based switch. In embodiments, the dimerization molecule is a GA₃-AM or GA₃.

In an embodiment, the dimerization switch comprises a Halo-tag/SNAP-tag based switch. In embodiments, the dimerization molecule comprises structure 5.

In other embodiments, the dimerization switch comprises switch domains that comprise tag molecules, e.g., a c-myc peptide tag, flag peptide tag, HA peptide tag or V5 peptide tag, and the dimerization switch comprises polypeptides with affinity for the switch domains, e.g., antibody molecules and non-antibody scaffold.

In embodiments, the dimerization molecule comprises three or more domains, e.g., protein tags, that bind a switch domain, e.g., a polypeptide, e.g., an antibody molecule or non-antibody scaffold, having affinity for the domain.

In an aspect, the disclosure features, a regulatable natural killer receptor CAR (RNKR-CAR) e.g., an isolated RNKR-CAR, wherein the RNKR-CAR, comprises:
a) an intracellular signaling member comprising:
   an intracellular signaling domain, e.g., a primary intracellular signaling domain, and
   a first switch domain;
b) an antigen binding member comprising:
   a binding domain element (e.g., antigen binding domain, inhibitory extracellular domain, or costimulatory extracellular domain),
   a second switch domain; and
   optionally, one or a plurality, of co-stimulatory signaling domains, and
c) optionally, a transmembrane domain.
(Unless otherwise indicated, when members or elements of an RNKR-CAR are described herein, the order can be as provided, but other orders are included as well. In other words, the order may be as set out in the text, or the order can be different.)

In an instance, the transmembrane domain can be disposed on the intracellular signaling member or the antigen binding member. In an instance, a transmembrane domain can be disposed on the intracellular signaling member and a transmembrane domain or membrane anchor (membrane anchor and membrane anchoring domain are used interchangeably herein) can be disposed on the antigen binding member.

In an instance, the first and second switch domains can form an intracellular or an extracellular dimerization switch.

In an instance, the dimerization switch can be a homodimerization switch or a heterodimerization switch.

As is discussed herein, instances of an RNKR-CAR can include a member, e.g., an intracellular signaling member, that comprises one or more intracellular signaling domains, as, e.g., is described above. In instances, an antigen binding member, can comprise an intracellular signaling domain, e.g., a costimulatory signaling domain.

In an embodiment, the intracellular signaling domain is a primary intracellular signaling domain, selected, e.g., from the list in Table 1.

In an embodiment, the primary intracellular signaling domain comprises a CD3zeta domain.

In an embodiment, the intracellular signaling domain is a costimulatory signaling domain, e.g., selected from the list in Table 2.

In an embodiment, the costimulatory signaling domain comprises a 4-1BB domain.

In an embodiment, the order of switch domain and the intracellular signaling domain (isd) or domains is as follows, beginning with the amino terminus:
switch/isd; or
isd/switch.

In an embodiment, the order of switch domain and the intracellular signaling domain (isd) or domains is as follows, beginning with the carboxy terminus:
switch/isd; or
isd/switch.

In an instance, the disclosure features, a RNKR-CAR, e.g., an isolated RNKR-CAR, wherein the RNKR-CAR comprises:
a) an antigen binding member comprising:
   a binding domain element (e.g., an antigen binding domain, inhibitory extracellular domain, or costimulatory extracellular domain),
   a first transmembrane domain, and
   a first switch domain; and
b) an intracellular signaling member comprising:
   a second transmembrane domain or membrane anchor,
   a second switch domain,
   and an intracellular signaling domain, e.g., a primary intracellular signaling domain.

In an instance, the antigen binding member optionally comprises one or more co-stimulatory signaling domains described herein. In an instance, the intracellular signaling domain further comprises one or more co-stimulatory signaling domains described herein.

In an instance, the first and second switch domains can form an intracellular or an extracellular dimerization switch.

In an instance, the dimerization switch can be a homodimerization switch or a heterodimerization switch.

In an instance, the first and/or second transmembrane domain comprises the transmembrane region(s) of e.g., the alpha, beta or zeta chain of the T-cell receptor, CD28, CD3 epsilon, CD45, CD4, CD5, CD8 (e.g., CD8 alpha, CD8 beta), CD9, CD16, CD22, CD33, CD37, CD64, CD80, CD86, CD134, CD137, CD154, KIRDS2, OX40, CD2, CD27, LFA-1 (CD11a, CD18), ICOS (CD278), 4-1BB (CD137), GITR, CD40, BAFFR, HVEM (LIGHTR), SLAMF7, NKp80 (KLRF1), CD160, CD19, IL2Rbeta, IL2Rgamma, IL7Rα, ITGA1, VLA1, CD49a, ITGA4, IA4, CD49D, ITGA6, VLA-6,CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, TNFR2, DNAM1 (CD226), SLAMF4 (CD244, 2B4), CD84, CD96 (Tactile), CEACAM1, CRTAM, Ly9 (CD229), CD160 (BY55),PSGL1, CD100 (SEMA4D), SLAMF6 (NTB-A, Ly108), SLAM (SLAMF1, CD150, IPO-3), BLAME (SLAMF8), SELPLG (CD162), LTBR, PAG/Cbp. In instances, the first and/or second transmembrane domain comprises the transmembrane region(s) of a natural killer receptor (NKR), e.g., a NKR described herein. The first transmembrane domain disposed on the antigen binding member and the second transmembrane domain disposed on the intracellular signaling member can be the same transmembrane domain, e.g., have the same sequence, or can be different transmembrane domains, e.g., have different sequences.

As is discussed herein, the RNKR-CAR can include any of a variety of dimerization switches, e.g., a dimerization switch described herein.

In an embodiment, the switch domains are components of a heterodimerization switch.

In an embodiment, the switch domains are components of a homodimerization switch.

In an embodiment, the dimerization switch is intracellular.

In an embodiment, the dimerization switch is extracellular.

In an embodiment, the transmembrane domain disposed on the antigen binding member and the dimerization switch, e.g., a heterodimerization switch or homodimerization switch, is intracellular.

In an embodiment, where the transmembrane domain disposed on the intracellular signaling member and the dimerization switch, e.g., heterodimerization or homodimerization switch, is extracellular.

In an embodiment, the dimerization switch comprises a FKBP-FRB based switch.

In an embodiment, the dimerization switch comprises:
a switch domain comprising a rapamycin analog binding sequence having at least 80, 85, 90, 95, 98, or 99 % identity with FKBP, and a switch domain comprising a rapamycin analog binding sequence binding sequence having at least 80, 85, 90, 95, 98, or 99% identity with FRB.

In an embodiment the dimerization switch comprises an FKBP-based switch domain and an FRB-based switch domain described herein.

In an embodiment, the dimerization switch comprises:
a switch domain comprising a rapamycin analog binding sequence that differs by no more than 10, 9, 8, 7, 6, 5, 4, 3, 2, or 1 amino acid residues from the corresponding sequence of FKBP, and a switch domain comprising a rapamycin analog binding sequence that differs by no more than 10, 9, 8, 7, 6, 5, 4, 3, 2, or 1 amino acid residues from the corresponding sequence of FRB.

In an embodiment, the dimerization switch comprises an FRB binding fragment or analog of FKBP and an FKBP binding fragment or analog of FRB, and the FKBP binding fragment or analog of FRB comprises one or more mutations which enhances the formation of a complex between an FKBP switch domain, an FRB switch domain, and the dimerization molecule, or a mutation described in the section herein entitled **MODIFIED FKBP/FRB-BASED DIMERIZATION SWITCHES.** E.g., the FKBP binding fragment or analog of FRB comprises: an E2032 mutation, e.g., an E2032I mutation or E2032L mutation; a T2098 mutation, e.g., a T2098L mutation; or an E2032 and a T2098 mutation, e.g., an E2032I and a T2098L or an E2032L and a T2098L mutation.

In an embodiment, wherein the switch is an FKBP-FRB based switch, the dimerization molecule is a mTOR inhibitor, e.g., an allosteric mTOR inhibitor, e.g., rapamycin or a rapalog, e.g., RAD001.

In an embodiment, any of the dosing regimes or formulations of an allosteric mTOR inhibitor, e.g., RAD001, described in the section here for a low, immune enhancing, dose of an allosteric mTOR inhibitor, e.g., RAD001, can be administered to dimerize an FKBP-FRB based switch.

In an embodiment, the switch is an FKBP-FRB based switch and the dimerization molecule is RAD001.

In an embodiment, 0.1 to 20, 0.5 to 10, 2.5 to 7.5, 3 to 6, or about 5, mgs of RAD001 per week, e.g., delivered once per week, is administered.

In an embodiment, 0.3 to 60, 1.5 to 30, 7.5 to 22.5, 9 to 18, or about 15 mgs of RAD001 in a sustained release formuation, per week, e.g., delivered once per week, is administered.

In an embodiment, 0.005 to 1.5, 0.01 to 1.5, 0.1 to 1.5, 0.2 to 1.5, 0.3 to 1.5, 0.4 to 1.5, 0.5 to 1.5, 0.6 to 1.5, 0.7 to 1.5, 0.8 to 1.5, 1.0 to 1.5, 0.3 to 0.6, or about 0.5 mgs of RAD001 per day, e.g., delivered once once per day, is administered.

In an embodiment, 0.015 to 4.5, 0.03 to 4.5, 0.3 to 4.5, 0.6 to 4.5, 0.9 to 4.5, 1.2 to 4.5, 1.5 to 4.5, 1.8 to 4.5, 2.1 to 4.5, 2.4 to 4.5, 3.0 to 4.5, 0.9 to 1.8, or about 1.5 mgs of RAD001 in a sustained release formulation, per day, e.g., delivered once once per day, is administered.

In an embodiment, 0.1 to 30, 0.2 to 30, 2 to 30, 4 to 30, 6 to 30, 8 to 30, 10 to 30, 1.2 to 30, 14 to 30, 16 to 30, 20 to 30, 6 to 12, or about 10 mgs of RAD001 in a sustained release formulation, per week, e.g., delivered once once per week, is administered.

In an embodiment, the dimerization switch comprises:
a switch domain comprising a rapamycin, or rapamycin analog, binding sequence from FKBP, and a switch domain comprising a rapamycin, or rapamycin analog, binding sequence from FRB, e.g., a sequence comprising a lysine at residue 2098.

In an embodiment, the dimerization switch comprises:
a switch domain comprising a rapamycin analog binding sequence from FKBP, and a switch domain comprising a rapamycin analog binding sequence from FRB, e.g., a sequence comprising a lysine at residue 2098.

In an embodiment, the dimerization switch comprises:
a switch domain comprising an AP21967 binding sequence from FKBP, and a switch domain comprising an AP21967 binding sequence from FRB, e.g., a sequence comprising a lysine at residue 2098.

In an embodiment:
the first switch domain comprises,
   a rapamycin, or rapamycin analog, binding sequence from FKBP;
   a rapamycin analog binding sequence from FKBP; or
   an AP21967 binding sequence from FKBP; and,
the second switch domain comprises,
   a rapamycin, or rapamycin analog, binding sequence from FRB;
   a rapamycin analog binding sequence from FRB; or
   an AP21967 binding sequence from FRB, e.g., a sequence comprising a lysine at residue 2098.

In an embodiment:
the first switch domain comprises,
   a rapamycin, or rapamycin analog, binding sequence from FRB;
   a rapamycin analog binding sequence from FRB; or
   an AP21967 binding sequence from FRB, e.g., a sequence comprising a lysine at residue 2098; and,
the second switch domain comprises,
   a rapamycin, or rapamycin analog, binding sequence from FKBP;
   a rapamycin analog binding sequence from FKBP; or an AP21967 binding sequence from FKBP.

In an embodiment:
the first switch domain comprises an AP21967 binding sequence from FKBP; and,
the second switch domain comprises an AP21967 binding sequence from FRB, e.g., a sequence comprising a lysine at residue 2098.

In an embodiment, the first switch domain comprises an AP21967 binding sequence from FRB, e.g., a sequence comprising a lysine at residue 2098; and,
the second switch domain comprises an AP21967 binding sequence from FKBP.

In an embodiment, the dimerizatio a molecule is a rapamycin analogue, e.g., AP21967.

In an embodiment, the dimerization switch comprises a GyrB-GyrB based switch.

In an embodiment, the dimerization switch comprises:
a switch domain comprising a coumermycin binding sequence having at least 80, 85, 90, 95, 98, or 99 % identity with the 24 K Da amino terminal sub-domain of GyrB.

In an embodiment the dimerization switch comprises:
a switch domain comprising a coumermycin binding sequence that differs by no more than 10, 9, 8, 7, 6, 5, 4, 3, 2, or 1 amino acid residues from the corresponding sequence of 24 K Da amino terminal sub-domain of GyrB.

In an embodiment, the dimerization switch comprises:
a switch domain comprising a coumermycin binding sequence from the 24 K Da amino terminal sub-domain of GyrB.

In an embodiment, the dimerization switch comprises:
the 24 K Da amino terminal sub-domain of GyrB.

In an embodiment, the dimerization molecule is a coumermycin.

In an embodiment, the dimerization switch comprises a GAI-GID1 based switch.

In an embodiment, the dimerization switch comprises:
a GID1 switch domain comprising a gibberellin, or gibberellin analog, e.g., GA₃, binding sequence having at least 80, 85, 90, 95, 98, or 99 % identity with GID1, and a switch domain comprising a GAI switch domain having at least 80, 85, 90, 95, 98, or 99 % identity with GAI.

In an embodiment, the dimerization switch comprises:
a GID1 switch domain comprising a gibberellin, or gibberellin analog, e.g., GA₃, binding sequence that differs by no more than 10, 9, 8, 7, 6, 5, 4, 3, 2, or 1 amino acid residues from the corresponding sequence of a GID1 described herein, and a GAI switch domain that differs by no more than 10, 9, 8, 7, 6, 5, 4, 3, 2, or 1 amino acid residues from the corresponding sequence of a GAI described herein.

In an embodiment:
the first switch domain comprises a GID1 switch domain; and,
the second switch domain comprises a GAI switch domain.

In an embodiment:
the first switch domain comprises a GAI switch domain; and,
the second switch domain comprises a GID1 switch domain.

In an embodiment, the dimerization molecule is GA₃-AM.

In an embodiment, the dimerization molecule is GA₃.

In an embodiment, the dimerization molecule is a small molecule, e.g., is other than a polypeptide.

In an embodiment, the dimerization molecule is a polypeptide, e.g., a polypeptide, e.g., an antibody molecule, or a non-antibody scaffold, e.g., a fibronectin or adnectin, having specific affinity for one or both of the first and second switch domains.

In an embodiment, the dimerization molecule, e.g. a polypeptide, is an antibody molecule.

In an embodiment, the dimerization switch comprises a Halo-tag/SNAP-tag based switch.

In an embodiment, the dimerization switch comprises:
a Halo-tag switch domain comprising having at least 80, 85, 90, 95, 98, or 99 % identity with SEQ ID NO: 14, and a SNAP-tag switch domain having at least 80, 85, 90, 95, 98, or 99 % identity with SEQ ID NO: 15.

In an embodiment, the dimerization switch comprises:
a Halo-tag switch domain comprising that differs by no more than 10, 9, 8, 7, 6, 5, 4, 3, 2, or 1 amino acid residues from SEQ ID NO: 14, and a SNAP-tag switch domain that differs by no more than 10, 9, 8, 7, 6, 5, 4, 3, 2, or 1 amino acid residues from SEQ ID: 15.

In an embodiment:
the first switch domain comprises a Halo-tag switch domain; and,
the second switch domain comprises a SNAP-tag switch domain.

In an embodiment:
the first switch domain comprises a SNAP-tag switch domain; and,
the second switch domain comprises a Halo-tag switch domain.

In an embodiment, the dimerization molecule comprises structure 5.

In an embodiment, the dimerization molecule comprises three or more domains, e.g., protein tags that bind a switch domain, e.g., a polypeptide, e.g., an antibody molecule or non-antibody scaffold, having affinity for the domain.

In an embodiment, the dimerization molecule is a non-covalent dimerization molecule.

In an embodiment, the dimerization molecule is covalent dimerization molecule.

In an instance, the dimerization switch, e.g., a homodimerization switch, e.g., an extracellular homodimerization switch, comprises switch domains that comprise tag molecules, e.g., a c-myc peptide tag, flag peptide tag, HA peptide tag or V5 peptide tag, and the dimerization switch comprises polypeptides with affinity for the switch domains, e.g., antibody molecules and non-antibody scaffold.

In an embodiment, the RNKR-CAR further comprises a second order dimerization switch.

In an embodiment, the dimerization molecule has a valency of greater than two, e.g., it is multi-valent, and binds, and thus clusters or dimerizes, more than two switch domains.

Embodiments of the dimerization switches described herein may feature multiple switch domains, sometimes referred to herein as a multi switch. A multi switch comprises plurality of, e.g., 2, 3, 4, 5, 6, 7, 8, 9, or 10, switch domains, independently, on a first member, e.g., an antigen binding member, and a second member, e.g., an intracellular signaling member, as described in the section herein entitled MULTIPLE SWITCH DOMAINS.

In an embodiment, the first member, e.g., an antigen binding member, comprises a plurality of first switch domains, e.g., FKBP-based switch domains, and the second member, e.g., an intracellular signaling member, comprises a plurality of second switch domains, e.g., FRB-based switch domains. In an embodiment, the first member comprises a first and a second switch domain, e.g., a FKBP-based switch domain and a FRB-based switch domain, and the second member comprises a first and a second switch domain, e.g., a FKBP-based switch domain and a FRB-based switch domain.

In an embodiment, the first member and the second member comprises a plurality of homodimerization switch domains, e.g., GyrB-based switch domains.

In embodiments, the RNKR-CAR comprises a multi switch comprising a plurality of, e.g., 2, 3, 4, 5, 6, 7, 8, 9, or 10, switch domains, independently, on a first member, e.g., an antigen binding member, and a the second member, e.g., an intracellular signaling member, as described in the section herein entitled MULTIPLE SWITCH DOMAINS. In an embodiment, the first member comprises a plurality of first switch domains, e.g., FKBP-based switch domains, and the second member comprises a plurality of second switch domains, e.g., FRB-based switch domains. In an embodiment, the first member comprises a first and a second switch domain, e.g., a FKBP-based switch domain and a FRB-based switch domain, and the second member comprises a first and a second switch domain, e.g., a FKBP-based switch domain and a FRB-based switch domain.

Also provided herein are RNKR-CARs wherein the antigen binding member comprises a plurality of antigen binding domains. In an embodiment, the antigen binding member comprises a plurality of, e.g., 2, 3, 4, or 5, antigen binding domains, e.g., scFvs, wherein each antigen binding domain binds to a target antigen. In an embodiment, two or more of the antigen binding domains can bind to different antigens. In an embodiment, two or more of the antigen binding domains can bind to the same antigen, e.g., the same or different epitopes on the same antigen. In embodiments, a linker or hinge region is optionally disposed between two or each of the antigen binding domains.

In an embodiment, dimerization of the switch domains results in clustering of intracellular signaling members.

In an embodiment, dimerization of the switch domains results in an increase in signaling by the intracellular signaling domains.

RNKR-CARs disclosed herein can include, e.g., in place of an scFv-based antigen binding domain, an extracellular domain of an inhibitory receptor, e.g., PD1. While not wishing to be bound by theory, it is believed that engagement of the inhibitory extracellular domain with its counter ligand (which normally down regulates the immune response), activates the immune response.

RNKR-CARs disclosed herein can include, e.g., in place of an scFv-based antigen binding domain, an extracellular domain of a costimulatory ECD domain. While not wishing to be bound by theory, it is believed that engagement of the ECD with its counter ligand activates the immune response via the RNKR-CAR.

In an embodiment, the RNKR-CAR is associated with, e.g., is provided in the same cell with:
an inhibitor of an inhibitory molecule, e.g., an inhibitor of an inhibitory molecule of Table 3.

In an embodiment, the RNKR-CAR is associated with, e.g., is provided in the same cell with, a nucleic acid inhibitor, e.g., an siRNA, an shRNA, or an antisense molecule, that targets a inhibitory molecule, e.g. a coinhibitory molecule from Table 3.

In an embodiment, the shRNA targets PD1.

In an embodiment, dimerization increases the level of proliferation or persistence of the RNKR-CAR expressing cell.

In an embodiment, the RNKR-CAR further comprises:
an inhibitory counter ligand binding member comprising,
an inhibitory counter ligand binding domain, selected e.g., from Table 4, and
a transmembrane domain or membrane anchor.

In another aspect, disclosed herein is a nucleic acid encoding a RNKR-CAR described herein. In particular, provided is a nucleic acid encoding a RNKR-CAR of the invention.

In embodiments, the nucleic acid comprises:
i) a sequence encoding (a) antigen binding member and (b) intracellular signaling member is disposed on a single nucleic acid molecule; or
ii) a sequence encoding (a) antigen binding member is disposed on a first nucleic acid molecule, and a sequence encoding (b) intracellular signaling member is disposed on a second nucleic acid molecule.

In embodiments, the nucleic acid further comprises a sequence encoding (c) an adaptor molecule or intracellular signaling molecule, e.g., DAP12 or Fcgamma R,
wherein
i) sequence encoding (a), (b) and (c), is provided on a single nucleic acid molecule;
ii) sequence encoding two of (a), (b), and (c), is provided on a first nucleic acid molecule and sequence encoding the other is provided on a second nucleic acid molecule; or
iii) sequence encoding (a) is provided on a first nucleic acid molecule, sequence encoding (b) is provided on a second nucleic acid molecule, and sequence encoding (c) is provided on a third nucleic acid molecule.

In embodiments, the nucleic acid further comprises a sequence encoding a second CAR, e.g., a standard CAR, RNKR-CAR, RCAR, or NKR-CAR.

In an embodiment, sequence encoding the antigen binding member is operatively linked to a first control region and sequence encoding the intracellular signaling member is operatively linked to a second control region.

In an embodiment, sequence encoding the antigen binding member is transcribed as a first RNA and sequence encoding intracellular signaling member is translated as a second RNA.

In an embodiment, the nucleic acid further comprises a sequence encoding a shRNA targeting a coinhibitory domain.

In an embodiment, sequence encoding the antigen binding member, the intracellular signaling member, and a sequence encoding a shRNA targeting a coinhibitory domain, are present in a single nucleic acid molecule.

In an embodiment, sequence encoding the antigen binding member is present on a first nucleic acid molecule and sequence encoding intracellular signaling member is present on a second nucleic acid molecule and a sequence encoding a shRNA targeting a coinhibitory domain is present on one or both of the first and second nucleic acid molecules.

In an embodiment, sequence encoding the antigen binding member is present on a first nucleic acid molecule, sequence encoding intracellular signaling member is present on a second nucleic acid molecule, and a sequence encoding a shRNA targeting a coinhibitory domain is present on a third nucleic acid molecule.

In an instance, the nucleic acid encodes a RNKR-CAR as described in any of Tables 6, 7, 8, 9, 10, or 11.

In an instance the nucleic acid encodes a RNKR-CAR which comprises:
a) an intracellular signaling member comprising:
   an intracellular signaling domain, e.g., a primary intracellular signaling domain, and
   a first switch domain;
b) an antigen binding member comprising:
   a binding domain element (e.g., an antigen binding domain, inhibitory extracellular domain, or costimulatory extracellular domain),
   a second switch domain; and
   optionally, an intracellular signaling domain, e.g., a costimulatory signaling domain, e.g., selected from Table 2, e.g., a 4-1BB domain; and
c) a transmembrane domain
   wherein:
   i) sequence encoding a) and b) is disposed on a single nucleic acid molecule, e.g., a viral vector, e.g., a lentivirus vector; or
   ii) sequence encoding a) is disposed on a first nucleic acid molecule, e.g., a viral vector, e.g., a lentivirus vector, and sequence encoding b) is disposed on a second nucleic acid molecule, e.g., a viral vector, e.g., a lentivirus vector.

In an instance the nucleic acid encodes a RNKR-CAR which comprises:
a) an intracellular signaling member comprising:
   an intracellular signaling domain, e.g., a primary intracellular signaling domain, and
   a first switch domain;
b) an antigen binding member comprising:
   an antigen binding domain,
   a second switch domain;
   a transmembrane domain in a) or b); and
c) an auxiliary antigen binding member comprising:
   an antigen binding domain that binds a second antigen; and
   a transmembrane domain or membrane anchoring domain,
   wherein:
   i) sequence encoding a), b), and c), is disposed on a single nucleic acid molecule, e.g., a viral vector, e.g., a lentivirus vector;
   ii) sequence encoding a) and b) is disposed on a first nucleic acid molecule, e.g., a viral vector, e.g., a lentivirus vector, and sequence encoding c is disposed on a second nucleic acid molecule, e.g., a viral vector, e.g., a lentivirus vector.
   iii) sequence encoding a) and c) is disposed on a first nucleic acid molecule, e.g., a viral vector, e.g., a lentivirus vector, and sequence encoding b is disposed on a second nucleic acid molecule, e.g., a viral vector, e.g., a lentivirus vector.
   iv) sequence encoding b) and c) is disposed on a first nucleic acid molecule, e.g., a viral vector, e.g., a lentivirus vector, and sequence encoding c) is disposed on a second nucleic acid molecule, e.g., a viral vector, e.g., a lentivirus vector; or
   v) sequence encoding each of a), b), and c) is provided on each of three a separate nucleic acid molecules, e.g., viral vectors, e.g., lentivirus vectors.

In an embodiment, the nucleic acid comprises:
a first nucleic acid molecule encoding a first transmembrane domain and a first intracellular signaling domain, e.g., a primary intracellular signaling domain, and
a second nucleic acid molecule encoding a second transmembrane domain and a second intracellular signaling domain, e.g., a primary intracellular signaling domain, and
a third nucleic acid molecule encoding an antigen binding domain tethered to a membrane anchor,
wherein the first and second transmembrane domains are separated from each other by a heterodimerization switch present on the outside of a cell,
wherein the heterodimerization switch comprises first switch domain and second switch domain, wherein the first and second switch domains of the heterodimerization switch interact together to form a complex in the presence of a heterodimerization molecule on the either the inside or outside of the cell.

In an embodiment, the nucleic acid comprises:
a first nucleic acid molecule encoding an antigen binding domain linked to a membrane anchor,
a second nucleic acid molecule encoding an inhibitory extracellular domain, and a transmembrane domain linked to first switch domain of a heterodimerization switch; and
a third nucleic acid molecule encoding second switch domain of a heterodimerization switch linked to an intracellular signaling domain, e.g., a primary intracellular signaling domain,
wherein the inhibitory extracellular domain is separated from the intracellular signaling domain by a heterodimerization switch, and
wherein the first and second switch domain interact together to form a complex in the presence of a heterodimerization molecule on the inside or outside, of the cell.

In an embodiment, the nucleic acid comprises:
a first nucleic acid molecule encoding an antigen binding domain that binds to first target, a transmembrane domain linked to first switch domain of a heterodimerization switch,
a second nucleic acid molecule encoding and an intracellular signaling domain, e.g., a primary intracellular signaling domain, wherein the intracellular signaling domain is linked to a second switch domain of a heterodimerization switch, and
a third nucleic acid molecule encoding an antigen binding domain that binds to a second target that is different from the first target and a transmembrane domain, wherein the heterodimerization switch is present on the inside of a cell, wherein first switch domain and second switch domain interact together to form a complex in the presence of a heterodimerization molecule on the inside of the cell.

In an embodiment, the nucleic acid encodes a RNKR-CAR comprising:
a) an intracellular signaling member;
b) an antigen binding member;
c) a second intracellular signaling member,
wherein
i) sequence encoding a), b) and c), is provided on a single nucleic acid molecule;
ii) sequence encoding two of a), b), and c), is provided on a first nucleic acid
   molecule and sequence encoding the other is provided on a second nucleic acid molecule; or
iii) sequence encoding a) is provided on a first nucleic acid molecule, sequence encoding b) is provided on a second nucleic acid molecule, and sequence encoding c) is provided on a third nucleic acid molecule.

In an embodiment the nucleic acid encode a RNKR-CAR comprising
a) an intracellular signaling member;
b) an antigen binding member;
c) a second intracellular signaling member,
wherein,
sequence encoding a) and b) is provided on a first nucleic acid molecule and sequence encoding c) is provided on a second nucleic acid molecule;
sequence encoding a) and c) is provided on a first nucleic acid molecule and sequence encoding b) is provided on a second nucleic acid molecule; or
sequence encoding b) and c) is provided on a first nucleic acid molecule and sequence encoding a) is provided on a second nucleic acid molecule.

In an embodiment, the nucleic acid encodes a RNKR-CAR comprising:
a) an intracellular signaling member;
b) an antigen binding member;
c) an auxiliary antigen binding member,
wherein
i) sequence encoding a), b) and c), is provided on a single nucleic acid molecule;
ii) sequence encoding two of a), b), and c), is provided on a first nucleic acid
   molecule and sequence encoding the other is provided on a second nucleic acid molecule; or
iii) sequence encoding a) is provided on a first nucleic acid molecule, sequence encoding b) is provided on a second nucleic acid molecule, and sequence encoding c) is provided on a third nucleic acid molecule.

In an embodiment, the nucleic acid encode a RNKR-CAR comprising
a) an intracellular signaling member;
b) an antigen binding member;
c) a second intracellular signaling member,
wherein
sequence encoding a) and b) is provided on a first nucleic acid molecule and sequence encoding c) is provided on a second nucleic acid molecule;
sequence encoding a) and c) is provided on a first nucleic acid molecule and sequence encoding b) is provided on a second nucleic acid molecule; or
sequence encoding b) and c) is provided on a first nucleic acid molecule and sequence encoding a is provided on a second nucleic acid molecule.

In an embodiment, the nucleic acid encode a RNKR-CAR in which the antigen binding domain is separated from the intracellular signaling domain by a dimerization switch comprising a first and a second switch domain, wherein the first switch domain is linked to the antigen binding domain and the second switch domain is linked to the intracellular signaling domain, wherein the first and second switch domains interact together to form a complex in the presence of a dimerization molecule.

In an aspect, provided herein is a vector system, e.g., one or more vectors, comprising a nucleic acid described herein.

In embodiments, all of the elements of a RNKR-CAR are encoded on a single vector. For example, the antigen binding member and the intracellular signaling member are encoded on a single vector.

In embodiments, an element of a RNKR-CAR is encoded on a first vector and another element of the RNKR-CAR is encoded on a second vector, of the vector system. For example, an antigen binding member is encoded on a first vector and an intracellular signaling member is encoded on a second vector of the vector system.

In embodiments, the vector system comprises a DNA, a RNA, a plasmid, a lentivirus vector, adenoviral vector, or a retrovirus vector.

In embodiments, the vector comprises a bi-cistronic or tri-cistronic lentivirus vector.

In an embodiment, the vector system comprises a bi-cistronic or tri-cistronic promoter.

In an aspect, disclosed herein is a cell comprising a RNKR-CAR described herein, a nucleic acid encoding a RNKR-CAR described herein, or a vector system described herein. Such a RNKR-CAR-containing (e.g., RNKR-CAR expressing) cell is also referred to as a RNKR-CARX cell. Provided is is a cell comprising a RNKR-CAR of the invention, a nucleic acid encoding a RNKR-CAR of the invention, or a vector system of the invention

In some embodiments, the cell further comprises a second RNKR-CAR, wherein the antigen binding domain of the second RNKR-CAR targets a different tumor antigen.

In some embodiments, the cell further comprises a standard CAR, RCAR, or NKR-CAR, wherein the antigen binding domain of the standard CAR, RCAR, or NKR-CAR is different from the antigen binding domain of the RNKR-CAR, e.g., binds a different antigen, e.g., a tumor antigen.

In embodiments, the cell is a human cell.

In embodiments, the cell is an immune effector cell. For example, the immune effector cell is a T cell or a NK cell.

In another aspect, provided herein is a method of making a cell described herein (e.g., a RNKR-CARX cell), comprising introducing a nucleic acid encoding a RNKR-CAR described herein, or a vector system described herein, into said cell.

In another aspect, disclosed herein is a method of treating a subject with a disease associated with a tumor antigen comprising administering to the subject an effective amount of a RNKR-CARX cell described herein.

In instances, the RNKR-CAR cell is an autologous T cell. For example, the RNKR-CAR cell is an allogeneic T cell. In instances, the RNKR-CAR cell is selected from: an autologous NK cell; and an allogeneic NK cell.

In instances, the subject is a human.

In instances, the method comprises treating the subject for cancer, e.g., a cancer described herein. In one instance, the cancer is a solid tumor, e.g., a solid tumor described herein, e.g., mesothelioma (e.g., malignant pleural mesothelioma), lung cancer (e.g., non-small cell lung cancer, small cell lung cancer, squamous cell lung cancer, or large cell lung cancer), pancreatic cancer (e.g., pancreatic ductal adenocarcinoma), ovarian cancer, colorectal cancer and bladder cancer or any combination thereof. In one instance, the disease is pancreatic cancer, e.g., metastatic pancreatic ductal adenocarcinoma (PDA), e.g., in a subject who has progressed on at least one prior standard therapy. In one instance, the disease is mesothelioma (e.g., malignant pleural mesothelioma), e.g., in a subject who has progressed on at least one prior standard therapy. In one instance, the disease is ovarian cancer, e.g., serous epithelial ovarian cancer, e.g., in a subject who has progressed after at least one prior regimen of standard therapy. In certain instances, the cancer is pancreatic carcinoma, mesothelioma, lung carcinoma, ovarian carcinoma, leukemia or lymphoma. In certain embodiments, the cancer is glioblastoma multiforme (GBM), anaplastic astrocytoma, giant cell glioblastoma, gliosarcoma, anaplastic oligodendroglioma, anaplastic ependymoma, choroid plexus carcinoma, anaplastic ganglioglioma, pineoblastoma, medulloepithelioma, ependymoblastoma, medulloblastoma, supratentorial primitive neuroectodermal tumor, and atypical teratoid/rhabdoid tumor, non-small cell lung carcinomas, lung, breast, prostate, ovarian, colorectal or bladder carcinoma.

In an instance, the cancer is selected from glioblastoma multiforme (GBM), anaplastic astrocytoma, giant cell glioblastoma, gliosarcoma, anaplastic oligodendroglioma, anaplastic ependymoma, choroid plexus carcinoma, anaplastic ganglioglioma, pineoblastoma, medulloepithelioma, ependymoblastoma, medulloblastoma, supratentorial primitive neuroectodermal tumor, and atypical teratoid/rhabdoid tumor, non-small cell lung carcinomas, lung, breast, prostate, ovarian, colorectal and bladder carcinoma.

In some instances, the cancer is B-cell acute lymphoid leukemia ("BALL"), T-cell acute lymphoid leukemia ("TALL"), acute lymphoid leukemia (ALL), acute myelogenous leukemia (AML); one or more chronic leukemias including but not limited to chronic myelogenous leukemia (CML), chronic lymphocytic leukemia (CLL); additional hematologic cancers or hematologic conditions including, but not limited to B cell prolymphocytic leukemia, blastic plasmacytoid dendritic cell neoplasm, Burkitt's lymphoma, diffuse large B cell lymphoma, follicular lymphoma, hairy cell leukemia, small cell- or a large cell-follicular lymphoma, malignant lymphoproliferative conditions, MALT lymphoma, mantle cell lymphoma, Marginal zone lymphoma, multiple myeloma, myelodysplasia and myelodysplastic syndrome, non-Hodgkin's lymphoma, plasmablastic lymphoma, plasmacytoid dendritic cell neoplasm, Waldenstrom macroglobulinemia.

In instances, the method comprises administering a dimerization molecule to the subject.

In instances, the RNKR-CAR comprises an FKBP-FRB based dimerization switch, and wherein the method comprises administering a dimerization molecule comprising an mTOR inhibitor, e.g., RAD001.

In an instance, disclosed herein is a method of providing a RNKR-CAR cell described herein, comprising:
providing an immune effector cell to a recipient entity; and
receiving from said entity, a RNKR-CAR cell derived from said immune effector cell, or a daughter cell thereof, wherein the RNKR-CAR comprises an RNKR-CAR described herein, or a nucleic acid or vector encoding the RNKR-CAR, e.g., as described herein.

In instances, the entity inserted a nucleic acid encoding the RNKR-CAR into said immune effector cell or a daughter cell thereof.

In instances, the method further comprises administering said RNKR-CAR to a subject.

In an instance, disclosed herein is a method of providing an RNKR-CAR cell comprising:
receiving from an entity an immune effector cell from a human; inserting a nucleic acid encoding an RNKR-CAR described herein into said immune effector cell, or a daughter cell thereof, to form an RNKR-CAR cell; and, optionally, providing said RNKR-CAR cell to said entity.

In instances, the entity is a laboratory, hospital, or a hospital provider.

In some instances, disclosed herein is a nucleic acid described herein, RNKR-CAR described herein, vector system described herein, or RNKR-CARX cell described herein for use as a medicament.

Methods and compositions described herein feature the combination of an RCAR and an NKR-CAR. In an embodiment a nucleic acid described herein can comprise, in addition to sequence encoding a RCAR, sequence encoding an NKR-CAR. In an embodiment a cell, e.g., a cytotoxic cell, e.g., a T cell or NK cell, described herein can comprise, in addition to a RCAR, an NKR-CAR. Such a cell is referred to herein as RCAR/NKR-CAR cell. Such "combination" nucleic acids and cells can be used in the method of the disclosure, e.g., methods of treating a patient, e.g., for cancer. In instances an inhNKR-CAR, e.g., an inhKIR-CAR, is used in combination with an RCAR.

In an aspect, disclosed herein is a RCAR/NKR-CAR cell comprising:
A) a regulatable CAR (RCAR) and a NKR-CAR;
B) a nucleic acid encoding a RCAR and a NKR-CAR; or
C) a vector system comprising a nucleic acid encoding a RCAR and a NKR-CAR.

In an instance, the cell comprises a regulatable CAR (RCAR) and a NKR-CAR.

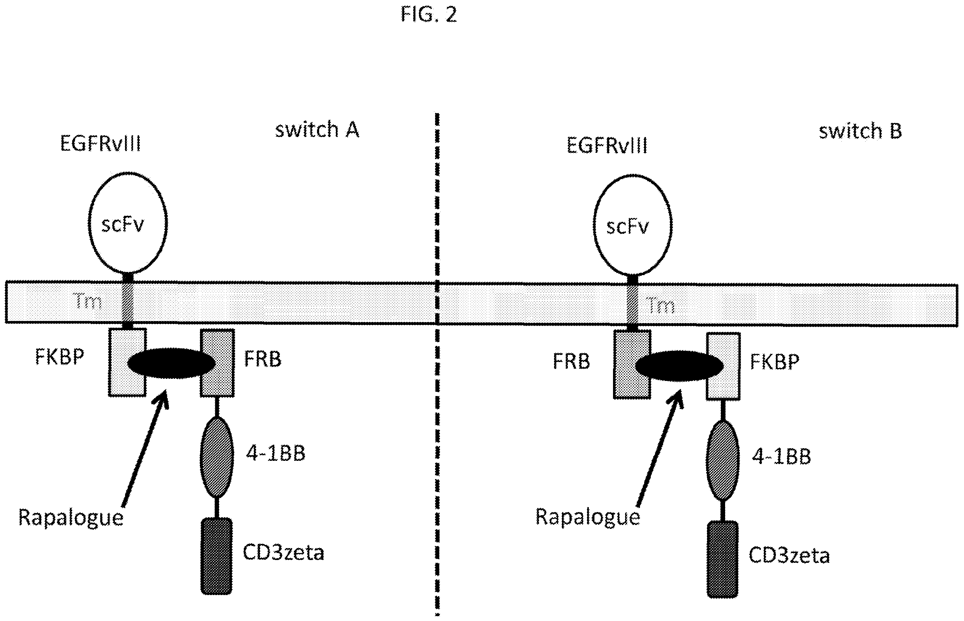

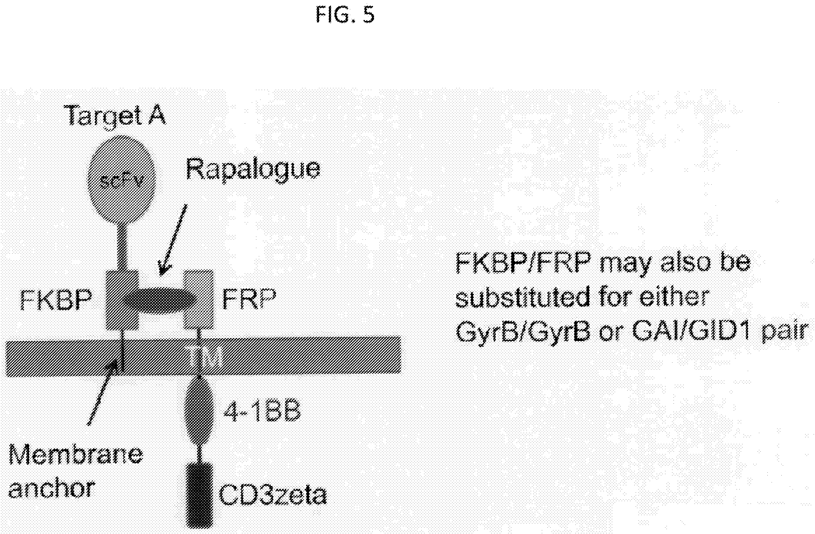

In an instance, e.g., in a RCAR/NKR-CAR cell, the RCAR comprises:
a) an intracellular signaling member comprising:
   an intracellular signaling domain, e.g., a primary intracellular signaling domain, and
   a first switch domain;
b) an antigen binding member comprising:
   an antigen binding domain,
   a second switch domain; and
   optionally, one or a plurality, of co-stimulatory signaling domains, and
c) optionally, a transmembrane domain. See, e.g., Figs. 2 and 5.
(Unless otherwise indicated, when members or elements of an RCAR are described herein, the order can be as provided, but other orders are included as well. In other words, the order may be as set out in the text, or the order can be different.)

More particularly, the invention provides a RCAR/NKR-CAR cell comprising: A) a regulatable CAR (RCAR) and a NKR-CAR; B) a nucleic acid encoding a RCAR and a NKR-CAR; or C) a vector system comprising a nucleic acid encoding a RCAR and a NKR-CAR,
wherein the RCAR comprises: a) an intracellular signaling member comprising: a primary intracellular signaling domain, and a first switch domain; b) an antigen binding member comprising: an antigen binding domain, a second switch domain; and optionally, one or a plurality, of co-stimulatory signaling domain, and c) a transmembrane domain,
wherein the first and second switch domains form a dimerization switch, comprising: 1) a FKBP/FRB-based dimerization switch, optionally wherein the first and second switch domains are dimerized by a rapamycin or a rapamycin analogue; 2) a GyrB-GyrB based switch, optionally wherein the first and second switch domains are dimerized by coumermycin; 3) a GAI-GID1 based switch; 4) a gibberellin-based dimerization switch; or 5) a Halo-tag/SNAP-tag based switch; and wherein the NKR-CAR comprises: a) an antigen binding domain derived from an antibody molecule, e.g. an antibody, an antibody fragment, an scFv, a Fv, a Fab, a (Fab')2, a single domain antibody (SDAB), a VH or VL domain, or a camelid VHH domain, b) a transmembrane domain, and c) a cytoplasmic domain,wherein the NKR-CAR comprises a NKR transmembrane domain and/or a NKR cytoplasmic domain.

In an embodiment, the transmembrane domain can be disposed on the intracellular signaling member or the antigen binding member. In an embodiment, a transmembrane domain can be disposed on the intracellular signaling member and a transmembrane domain or membrane anchor (membrane anchor and membrane anchoring domain are used interchangeably herein) can be disposed on the antigen binding member.

In an embodiment, the first and second switch domains can form an intracellular or an extracellular dimerization switch.

In an embodiment, the dimerization switch can be a homodimerization switch or a heterodimerization switch.

As is discussed herein, embodiments of an RCAR can include a member, e.g., an intracellular signaling member, that comprises one or more intracellular signaling domains, as, e.g., is described above. In embodiments, an antigen binding member, can comprise an intracellular signaling domain, e.g., a costimulatory signaling domain. Embodiments of such members, and intracellular signaling domains, are described in the section following immediately hereafter, sometimes referred to herein as the Intracellular Signaling domain Module.

In an embodiment, the intracellular signaling domain is a primary intracellular signaling domain, selected, e.g., from the list in Table 1.

In an embodiment, the primary intracellular signaling domain comprises a CD3zeta domain.

In an embodiment, the intracellular signaling domain is a costimulatory signaling domain, e.g., selected from the list in Table 2.

In an embodiment, the costimulatory signaling domain comprises a 4-1BB domain.

In an embodiment, the RCAR comprises a second intracellular signaling domain.

In an embodiment, the second intracellular signaling domain is a primary intracellular signaling domain, e.g., selected from the list in Table 1.

In an embodiment, the second intracellular signaling domain is a costimulatory signaling domain, e.g., selected from the list in Table 2.

In an embodiment, the first and second intracellular signaling domains comprise:
a 4-1BB domain and a CD3zeta domain; or
a CD28 domain and a 4-1BB domain.

In an embodiment, the RCAR comprises a third intracellular signaling domain.

In an embodiment, the third intracellular signaling domain is a primary intracellular signaling domain, e.g., selected from the list in Table 1.

In an embodiment, the third intracellular signaling domain is a costimulatory signaling domain, e.g., selected from the list in Table 2.

In an embodiment, one of the first, second and third intracellular signaling domain is a primary intracellular signaling domain, e.g., selected from the list in Table 1, and the other two are costimulatory signaling domains, e.g., selected from, Table 2.

In an embodiment, two of the first, second and third intracellular signaling domains are primary intracellular signaling domains, e.g., selected from the list in Table 1, and the other is a costimulatory signaling domain, e.g., selected from, Table 2.

In an embodiment each of the first, second and third intracellular signaling domains is a primary intracellular signaling domain, e.g., selected from the list in Table 1,

In an embodiment, each of the first, second and third intracellular signaling domains is a costimulatory signaling domain, e.g., selected from the list in Table 2.

In an embodiment, the first, second, and third intracellular signaling domains comprise: a CD28 domain; a 4-1BB domain, and a CD3zeta domain.

In an embodiment, the RCAR comprises a fourth intracellular signaling domain.

In an embodiment, the fourth intracellular signaling domain is a primary intracellular signaling domain, e.g., selected from the list in Table 1.

In an embodiment, the fourth intracellular signaling domain is a costimulatory signaling domain, e.g., selected from the list in Table 2.

In an embodiment, one of the first, second, third and fourth intracellular signaling domain is a primary intracellular signaling domain, e.g., selected from the list in Table 1 and the other three are costimulatory signaling domains, e.g., selected from the list in Table 2.

In an embodiment, two of the first, second, third, and fourth intracellular signaling domains are primary intracellular signaling domains, e.g., selected from the list in Table 1, and the other two are costimulatory signaling domain, e.g., selected from the list in Table 2.

In an embodiment, three of the first, second, third, and fourth intracellular signaling domains a In an embodiment re primary intracellular signaling domains, e.g., selected from the list in Table 1, and the other is a costimulatory signaling domain, e.g., selected from the list in Table 2.

In an embodiment, each of the first, second, third, and fourth intracellular signaling domains is a primary intracellular signaling domain, e.g., selected from the list in Table 1.

In an embodiment, each of the first, second, third, and fourth intracellular signaling domains is a costimulatory signaling domain, e.g., selected from the list in Table 2.

In an embodiment, the order of switch domain and the intracellular signaling domain (isd) or domains is as follows, beginning with the amino terminus:
switch/isd;
switch/isd1/isd2;
isd1/switch/isd2;
isd1/isd2/switch;
switch/isd1/isd2/isd3;
isd1/isd2/isd3/switch;
isd1/switch/isd2/isd3; and
isd1/isd2/switch/isd3.

In an embodiment, the order of switch domain and the intracellular signaling domain (isd) or domains is as follows, beginning with the carboxy terminus:
switch/isd;
switch/isd1/isd2;
isd1/switch/isd2;
isd1/isd2/switch;
switch/isd1/isd2/isd3;
isd1/isd2/isd3/switch;
isd1/switch/isd2/isd3; and
isd1/isd2/switch/isd3.

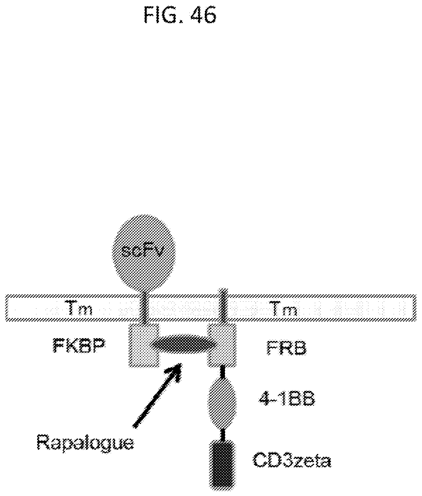

In an instance, the invention features, a RCAR, e.g., an isolated RCAR, wherein the RCAR comprises:
a) an antigen binding member comprising:
   an antigen binding domain,
   a first transmembrane domain, and
   a first switch domain; and
b) an intracellular signaling member comprising:
   a second transmembrane domain or membrane anchor,
   a second switch domain,
   and an intracellular signaling domain, e.g., a primary intracellular signaling domain.
See, e.g., Fig. 46.

In an instance, the antigen binding member optionally comprises one or more co-stimulatory signaling domains described herein. In an embodiment, the intracellular signaling domain further comprises one or more co-stimulatory signaling domains described herein.

In an instance, the first and second switch domains can form an intracellular or an extracellular dimerization switch.

In an instance, the dimerization switch can be a homodimerization switch or a heterodimerization switch.

In an instance, the first and/or second transmembrane domain comprises the transmembrane region(s) of e.g., the alpha, beta or zeta chain of the T-cell receptor, CD28, CD3 epsilon, CD45, CD4, CD5, CD8 (e.g., CD8 alpha, CD8 beta), CD9, CD16, CD22, CD33, CD37, CD64, CD80, CD86, CD134, CD137, CD154, KIRDS2, OX40, CD2, CD27, LFA-1 (CD11a, CD18), ICOS (CD278), 4-1BB (CD137), GITR, CD40, BAFFR, HVEM (LIGHTR), SLAMF7, NKp80 (KLRF1), CD160, CD19, IL2Rbeta, IL2Rgamma, IL7Rα, ITGA1, VLA1, CD49a, ITGA4, IA4, CD49D, ITGA6, VLA-6,CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, TNFR2, DNAM1 (CD226), SLAMF4 (CD244, 2B4), CD84, CD96 (Tactile), CEACAM1, CRTAM, Ly9 (CD229), CD160 (BY55),PSGL1, CD100 (SEMA4D), SLAMF6 (NTB-A, Ly108), SLAM (SLAMF1, CD150, IPO-3), BLAME (SLAMF8), SELPLG (CD162), LTBR, PAG/Cbp. The first transmembrane domain disposed on the antigen binding member and the second transmembrane domain disposed on the intracellular signaling member can be the same transmembrane domain, e.g., have the same sequence, or can be different transmembrane domains, e.g., have different sequences.

As is discussed herein, the RCAR can include any of a variety of dimerization switches, e.g., a dimerization switch described herein.

In an instance, the switch domains are components of a heterodimerization switch.

In an instance, the switch domains are components of a homodimerization switch.

In an instance, the dimerization switch is intracellular.

In an instance, the dimerization switch is extracellular.

In an instance, the transmembrane domain disposed on the antigen binding member and the dimerization switch, e.g., a heterodimerization switch or homodimerization switch, is intracellular.

In an instance, where the transmembrane domain disposed on the intracellular signaling member and the dimerization switch, e.g., heterodimerization or homodimerization switch, is extracellular.

In an instance, the dimerization switch comprises a FKBP-FRB based switch.

In an instance, the dimerization switch comprises:
a switch domain comprising a rapamycin analog binding sequence having at least 80, 85, 90, 95, 98, or 99 % identity with FKBP, and a switch domain comprising a rapamycin analog binding sequence binding sequence having at least 80, 85, 90, 95, 98, or 99% identity with FRB.

In an instance the dimerization switch comprises an FKBP-based switch domain and an FRB-based switch domain described herein.

In an instance, the dimerization switch comprises:
a switch domain comprising a rapamycin analog binding sequence that differs by no more than 10, 9, 8, 7, 6, 5, 4, 3, 2, or 1 amino acid residues from the corresponding sequence of FKBP, and a switch domain comprising a rapamycin analog binding sequence that differs by no more than 10, 9, 8, 7, 6, 5, 4, 3, 2, or 1 amino acid residues from the corresponding sequence of FRB.

In an instance, the dimerization switch comprises an FRB binding fragment or analog of FKBP and an FKBP binding fragment or analog of FRB, and the FKBP binding fragment or analog of FRB comprises one or more mutations which enhances the formation of a complex between an FKBP switch domain, an FRB switch domain, and the dimerization molecule, or a mutation described in the section herein entitled **MODIFIED FKBP/FRB-BASED DIMERIZATION SWITCHES.** E.g., the FKBP binding fragment or analog of FRB comprises: an E2032 mutation, e.g., an E2032I mutation or E2032L mutation; a T2098 mutation, e.g., a T2098L mutation; or an E2032 and a T2098 mutation, e.g., an E2032I and a T2098L or an E2032L and a T2098L mutation.

In an instance, wherein the switch is an FKBP-FRB based switch, the dimerization molecule is an mTOR inhibitor, e.g., an allosteric mTOR inhibitor, e.g., rapamycin or a rapalog, e.g., RAD001.

In an instance, any of the dosing regimes or formulations of an allosteric mTOR inhibitor, e.g., RAD001, described in the section here for a low, immune enhancing, dose of an allosteric mTOR inhibitor, e.g., RAD001, can be administered to dimerize an FKBP-FRB based switch.

In an instance, the switch is an FKBP-FRB based switch and the dimerization molecule is RAD001.

In an instance, 0.1 to 20, 0.5 to 10, 2.5 to 7.5, 3 to 6, or about 5, mgs of RAD001 per week, e.g., delivered once per week, is administered.

In an instance, 0.3 to 60, 1.5 to 30, 7.5 to 22.5, 9 to 18, or about 15 mgs of RAD001 in a sustained release formulation, per week, e.g., delivered once per week, is administered.

In an instance, 0.005 to 1.5, 0.01 to 1.5, 0.1 to 1.5, 0.2 to 1.5, 0.3 to 1.5,0.4 to 1.5, 0.5 to 1.5, 0.6 to 1.5, 0.7 to 1.5, 0.8 to 1.5, 1.0 to 1.5, 0.3 to 0.6, or about 0.5 mgs of RAD001 per day, e.g., delivered once per day, is administered.

In an instance, 0.015 to 4.5, 0.03 to 4.5, 0.3 to 4.5, 0.6 to 4.5, 0.9 to 4.5, 1.2 to 4.5, 1.5 to 4.5, 1.8 to 4.5, 2.1 to 4.5, 2.4 to 4.5, 3.0 to 4.5, 0.9 to 1.8, or about 1.5 mgs of RAD001 in a sustained release formulation, per day, e.g., delivered once per day, is administered.

In an instance, 0.1 to 30, 0.2 to 30, 2 to 30, 4 to 30, 6 to 30, 8 to 30, 10 to 30, 1.2 to 30, 14 to 30, 16 to 30, 20 to 30, 6 to 12, or about 10 mgs of RAD001 in a sustained release formulation, per week, e.g., delivered once per week, is administered.

In an instance, the dimerization switch comprises:
a switch domain comprising a rapamycin, or rapamycin analog, binding sequence from FKBP, and a switch domain comprising a rapamycin, or rapamycin analog, binding sequence from FRB, e.g., a sequence comprising a lysine at residue 2098.

In an instance, the dimerization switch comprises:
a switch domain comprising a rapamycin analog binding sequence from FKBP, and a switch domain comprising a rapamycin analog binding sequence from FRB, e.g., a sequence comprising a lysine at residue 2098.

In an instance, the dimerization switch comprises:
a switch domain comprising an AP21967 binding sequence from FKBP, and a switch domain comprising an AP21967 binding sequence from FRB, e.g., a sequence comprising a lysine at residue 2098.

In an instance:
the first switch domain comprises,
   a rapamycin, or rapamycin analog, binding sequence from FKBP;
   a rapamycin analog binding sequence from FKBP; or
   an AP21967 binding sequence from FKBP; and,
the second switch domain comprises,
   a rapamycin, or rapamycin analog, binding sequence from FRB;
   a rapamycin analog binding sequence from FRB; or
   an AP21967 binding sequence from FRB, e.g., a sequence comprising a lysine at residue 2098.

In an instance:
the first switch domain comprises,
   a rapamycin, or rapamycin analog, binding sequence from FRB;
   a rapamycin analog binding sequence from FRB; or
   an AP21967 binding sequence from FRB, e.g., a sequence comprising a lysine at residue 2098; and,
the second switch domain comprises,
   a rapamycin, or rapamycin analog, binding sequence from FKBP;
   a rapamycin analog binding sequence from FKBP; or
   an AP21967 binding sequence from FKBP.

In an instance:
the first switch domain comprises an AP21967 binding sequence from FKBP; and,
the second switch domain comprises an AP21967 binding sequence fromFRB, e.g., a sequence comprising a lysine at residue 2098.

In an instance, the first switch domain comprises an AP21967 binding sequence from FRB, e.g., a sequence comprising a lysine at residue 2098; and,
the second switch domain comprises an AP21967 binding sequence from FKBP.

In an instance, the dimerization molecule is a rapamycin analogue, e.g., AP21967.

In an instance, the dimerization switch comprises a GyrB-GyrB based switch.

In an instance, the dimerization switch comprises:
a switch domain comprising a coumermycin binding sequence having at least 80, 85, 90, 95, 98, or 99 % identity with the 24 K Da amino terminal sub-domain of GyrB.

In an instance the dimerization switch comprises:
a switch domain comprising a coumermycin binding sequence that differs by no more than 10, 9, 8, 7, 6, 5, 4, 3, 2, or 1 amino acid residues from the corresponding sequence of 24 K Da amino terminal sub-domain of GyrB.

In an instance, the dimerization switch comprises:
a switch domain comprising a coumermycin binding sequence from the 24 K Da amino terminal sub-domain of GyrB.

In an instance, the dimerization switch comprises:
the 24 K Da amino terminal sub-domain of GyrB.

In an instance, the dimerization molecule is a coumermycin.

In an instance, the dimerization switch comprises a GAI-GID1 based switch.

In an instance, the dimerization switch comprises:
a GID1 switch domain comprising a gibberellin, or gibberellin analog, e.g., GA₃, binding sequence having at least 80, 85, 90, 95, 98, or 99 % identity with GID1, and a switch domain comprising a GAI switch domain having at least 80, 85, 90, 95, 98, or 99 % identity with GAI.

In an instance, the dimerization switch comprises:
a GID1 switch domain comprising a gibberellin, or gibberellin analog, e.g., GA₃, binding sequence that differs by no more than 10, 9, 8, 7, 6, 5, 4, 3, 2, or 1 amino acid residues from the corresponding sequence of a GID1 described herein, and a GAI switch domain that differs by no more than 10, 9, 8, 7, 6, 5, 4, 3, 2, or 1 amino acid residues from the corresponding sequence of a GAI described herein.

In an instance:
the first switch domain comprises a GID1 switch domain; and,
the second switch domain comprises a GAI switch domain.

In an instance:
the first switch domain comprises a GAI switch domain; and,
the second switch domain comprises a GID1 switch domain.
In an instance, the dimerization molecule is GA₃-AM.

In an instance, the dimerization molecule is GA₃.

In an instance, the dimerization molecule is a small molecule, e.g., is other than a polypeptide.

In an instance, the dimerization molecule is a polypeptide, e.g., a polypeptide, e.g., an antibody molecule, or a non-antibody scaffold, e.g., a fibronectin or adnectin, having specific affinity for one or both of the first and second switch domains.

In an instance, the dimerization molecule, e.g. a polypeptide, is an antibody molecule.

In an instance, the dimerization switch comprises a Halo-tag/SNAP-tag based switch.

In an instance, the dimerization switch comprises:
a Halo-tag switch domain comprising having at least 80, 85, 90, 95, 98, or 99 % identity with SEQ ID NO: 14, and a SNAP-tag switch domain having at least 80, 85, 90, 95, 98, or 99 % identity with SEQ ID NO: 15.

In an instance, the dimerization switch comprises:
a Halo-tag switch domain comprising that differs by no more than 10, 9, 8, 7, 6, 5, 4, 3, 2, or 1 amino acid residues from SEQ ID NO: 14, and a SNAP-tag switch domain that differs by no more than 10, 9, 8, 7, 6, 5, 4, 3, 2, or 1 amino acid residues from SEQ ID: 15.

In an instance:
the first switch domain comprises a Halo-tag switch domain; and,
the second switch domain comprises a SNAP-tag switch domain.

In an instance:
the first switch domain comprises a SNAP-tag switch domain; and,
the second switch domain comprises a Halo-tag switch domain.

In an instance, the dimerization molecule comprises structure 5.

In an instance, the dimerization molecule comprises three or more domains, e.g., protein tags that bind a switch domain, e.g., a polypeptide, e.g., an antibody molecule or non-antibody scaffold, having affinity for the domain.

In an instance, the dimerization molecule is a non-covalent dimerization molecule.

In an instance, the dimerization molecule is covalent dimerization molecule.

In an instance, the dimerization switch, e.g., a homodimerization switch, e.g., an extracellular homodimerization switch, comprises switch domains that comprise tag molecules, e.g., a c-myc peptide tag, flag peptide tag, HA peptide tag or V5 peptide tag, and the dimerization switch comprises polypeptides with affinity for the switch domains, e.g., antibody molecules and non-antibody scaffold.

In an instance, the RCAR further comprises a second order dimerization switch.

In an instance, the dimerization molecule has a valency of greater than two, e.g., it is multi-valent, and binds, and thus clusters or dimerizes, more than two switch domains.

Instances of the dimerization switches described herein may feature multiple switch domains, sometimes referred to herein as a multi switch. A multi switch comprises plurality of, e.g., 2, 3, 4, 5, 6, 7, 8, 9, or 10, switch domains, independently, on a first member, e.g., an antigen binding member, and a second member, e.g., an intracellular signaling member, as described in the section herein entitled **MULTIPLE SWITCH DOMAINS.**

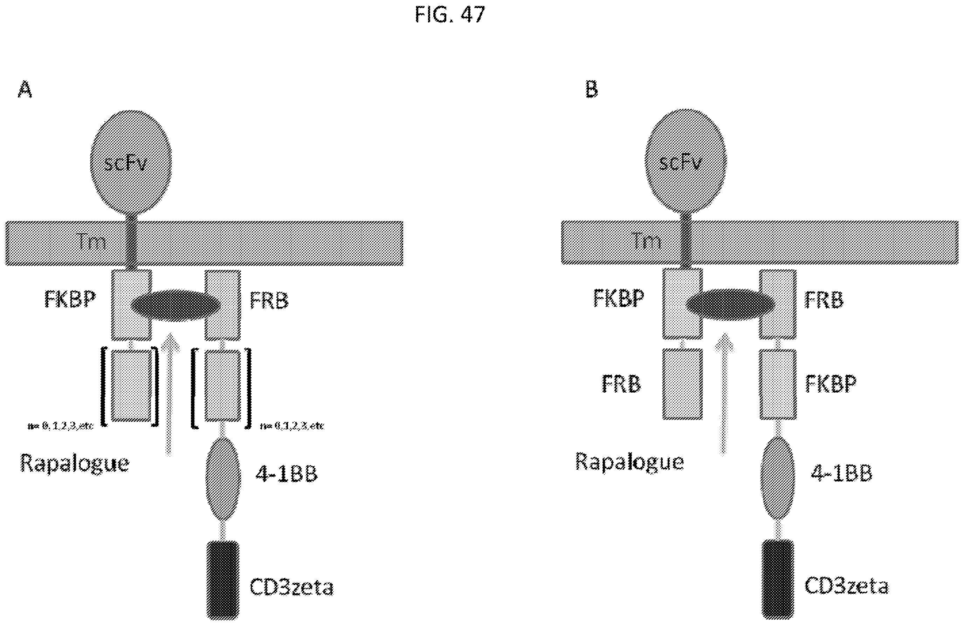

In an instance, the first member, e.g., an antigen binding member, comprises a plurality of first switch domains, e.g., FKBP-based switch domains, and the second member, e.g., an intracellular signaling member, comprises a plurality of second switch domains, e.g., FRB-based switch domains. See, e.g., Fig. 47A. In an instance, the first member comprises a first and a second switch domain, e.g., a FKBP-based switch domain and a FRB-based switch domain, and the second member comprises a first and a second switch domain, e.g., a FKBP-based switch domain and a FRB-based switch domain. See, e.g., Fig. 47B.

In an instance, the first member and the second member comprises a plurality of homodimerization switch domains, e.g., GyrB-based switch domains.

As is discussed herein, instances of an RCAR can include a member, e.g., an antigen binding member, comprising an intracellular signaling domain, e.g., a costimulatory signaling domain. While not wishing to be bound by theory, it is believed that the presence of such a domain promotes persistence of the member in a cell without significant activation in the absence of dimerization switch mediated association of members of the RCAR.

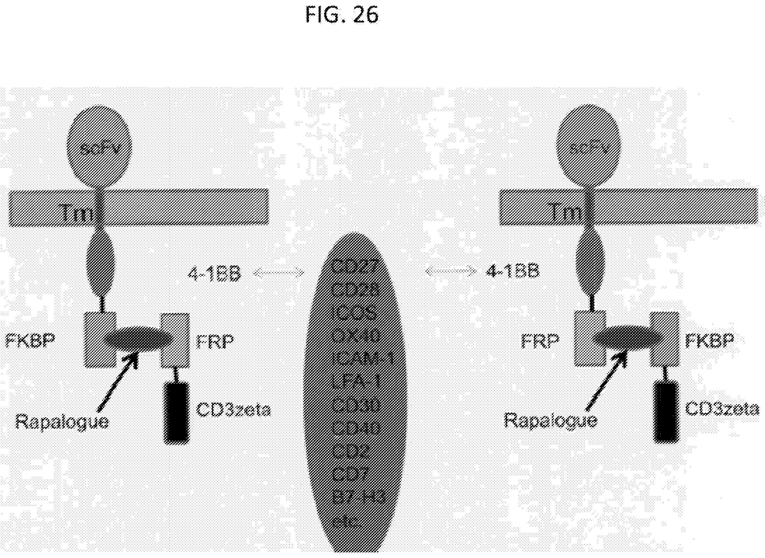

In an instance, the RCAR comprises:
a) an intracellular signaling member comprising:
   an intracellular signaling domain, e.g., a primary intracellular signaling domain, e.g., selected from Table 1, e.g., a CD3zeta domain, and
   a first switch domain, e.g., and FKBP switch domain; and
b) an antigen binding member comprising:
   an antigen binding domain,
   a transmembrane domain,
   an intracellular signaling domain, e.g., a costimulatory signaling domain, e.g.,
      selected from Table 2, e.g., a 4-1BB domain, and
   a second switch domain, e.g., a FRB switch domain.
   See, e.g., Figs. 26, 34A, and 36A.

In an instance, the antigen binding member comprises: a plurality, e.g., 2 or 3 costimulatory signaling domains, chosen e.g., from Table 2, and in instances, no primary intracellular signaling domain.

In an instance, the antigen binding member comprises: a plurality, e.g., 2 or 3, costimulatory signaling domains selected from 41BB, CD28, CD27, ICOS, and OX40.

In an instance, the two or more costimulatory domains can be the same costimulatory signaling domain or different costimulatory signaling domains.

In an instance, the antigen binding member comprises the following costimulatory signaling domains, from the extracellular to intracellular direction:
41BB-CD27;
CD27-41BB;
41BB-CD28;
CD28-41BB;
OX40-CD28;
CD28-OX40;
CD28-41BB; or
41BB-CD28.

In an instance, the antigen binding member comprises the following costimulatory signaling domains: CD28-41BB.

In an instance, the antigen binding member comprises the following costimulatory signaling domains: CD28-OX40.

In an instance, the antigen binding member comprises: a plurality, e.g., 2 or 3 costimulatory signaling domains, chosen e.g., from Table 2, e.g., a combination of costimulatory signaling domains described herein, and the intracellular binding domain comprises a CD3zeta domain.

In an instance, an antigen binding member having two or more costimulatory signaling domains does not comprise a primary intracellular signaling domain.

In an instance, the first and second switch domains comprise a FKBP-FRB based switch, which comprises a switch domain comprising a FRB binding fragment or analog of FKBP and a switch domain comprising an FKBP binding fragment or analog of FRB, and the FKBP binding fragment or analog of FRB comprises one or more mutations which enhances the formation of a complex between an FKBP switch domain, an FRB switch domain, and the dimerization molecule, or a mutation described in the section herein entitled **MODIFIED FKBP/FRB-BASED DIMERIZATION SWITCHES.** E.g., the FKBP binding fragment or analog of FRB comprises: an E2032 mutation, e.g., an E2032I mutation or E2032L mutation; a T2098 mutation, e.g., a T2098L mutation; or an E2032 and a T2098 mutation, e.g., an E2032I and a T2098L or an E2032L and a T2098L mutation.

In such instances, the RCAR comprises a multi switch comprising a plurality of, e.g., 2, 3, 4, 5, 6, 7, 8, 9, or 10, switch domains, independently, on a first member, e.g., an antigen binding member, and a the second member, e.g., an intracellular signaling member, as described in the section herein entitled **MULTIPLE SWITCH DOMAINS.** In an instance, the first member comprises a plurality of first switch domains, e.g., FKBP-based switch domains, and the second member comprises a plurality of second switch domains, e.g., FRB-based switch domains. In an instance, the first member comprises a first and a second switch domain, e.g., a FKBP-based switch domain and a FRB-based switch domain, and the second member comprises a first and a second switch domain, e.g., a FKBP-based switch domain and a FRB-based switch domain.

Also disclosed herein are RCARs wherein the antigen binding member comprises a plurality of antigen binding domains. In an instance, the antigen binding member comprises a plurality of, e.g., 2, 3, 4, or 5, antigen binding domains, e.g., scFvs, wherein each antigen binding domain binds to a target antigen. In an instance, two or more of the antigen binding domains can bind to different antigens. In an instance, two or more of the antigen binding domains can bind to the same antigen, e.g., the same or different epitopes on the same antigen. In instances, a linker or hinge region is optionally disposed between two or each of the antigen binding domains.

In an instance, the RCAR comprises
a) an intracellular signaling member comprising:
   an intracellular signaling domain, e.g., a primary intracellular signaling domain, e.g., selected from Table 1, e.g., a CD3zeta domain, and
   a FKBP switch domain; and
b) an antigen binding member comprising:
   (i) an antigen binding domain, e.g., an antigen binding domain that targets CD19, e.g., an anti-CD 19 antigen binding domain described herein,
   (ii) a transmembrane domain,
   (iii) one of:
      (A) a CD28 costimulatory signaling domain and a 4-1BB costimulatory signaling domain; or
      (B) a CD28 costimulatory signaling domain and an OX-40 costimulatory signaling domain; and
   (iv) a FRB switch domain comprising one or more mutations described in the section herein entitled, **MODIFIED FKBP/FRB-BASED DIMERIZATION SWITCHES,** e.g., an E2032I and a T2098L or an E2032L and a T2098L mutation.

In an instance, the order of elements on the antigen binding member is as follows, beginning with the amino terminus:
antigen binding domain/transmembrane domain/intracellular signaling domain, e.g., a costimulatory signaling domain, e.g., selected from Table 2, e.g., a 4-1BB domain /switch domain; or
antigen binding domain/transmembrane domain /switch domain/ intracellular signaling domain, e.g., a costimulatory signaling domain, e.g., selected from Table 2, e.g., a 4-1BB domain.

In an instance, the order of elements on the intracellular signaling member is as follows, with beginning with the amino terminus:
switch domain/ intracellular signaling domain, e.g., a primary intracellular signaling domain, e.g., selected from Table 1, e.g., a CD3zeta domain; or
intracellular signaling domain, e.g., a primary intracellular signaling domain, e.g., selected from Table 1, e.g., a CD3zeta domain/ switch domain.

In an instance, the order of elements on the antigen binding member is as follows, with beginning with the carboxy terminus:
antigen binding domain/transmembrane domain/intracellular signaling domain, e.g., a costimulatory signaling domain, e.g., selected from Table 2, e.g., a 4-1BB domain/switch domain; or
antigen binding domain/transmembrane domain /switch domain/ intracellular signaling domain, e.g., a costimulatory signaling domain, e.g., selected from Table 2, e.g., a 4-1BB domain.

In an instance, the order of elements on the intracellular signaling member is as follows, beginning with the carboxy terminus:
switch domain/ intracellular signaling domain, e.g., a primary intracellular signaling domain, e.g., selected from Table 1, e.g., a CD3zeta domain; or
intracellular signaling domain, e.g., a primary intracellular signaling domain, e.g., selected from Table 1, e.g., a CD3zeta domain/ switch domain.

In an instance, an RCAR comprises an auxiliary antigen binding member.

In an instance, the RCAR, e.g., comprising an antigen binding member and an intracellular signaling member, further comprises:
c) an auxiliary antigen binding member comprising:
an antigen binding domain that binds a second antigen; and
a transmembrane domain or membrane anchoring domain.

In an instance, the auxiliary antigen binding domain does not comprise a switch domain that can form a dimerization switch with a switch domain on the antigen binding member or the intracellular signaling member.

In an instance, the auxiliary antigen binding member does not comprise an intracellular signaling domain.

In an instance, said second antigen is a cancer cell surface antigen.

In an instance the RCAR, e.g., comprising an antigen binding member and an intracellular signaling member, further comprises:
d) a second auxiliary antigen binding member comprising
an antigen binding domain that binds a third antigen; and
a transmembrane domain or membrane anchoring domain.

In an instance, said third antigen is different from the antigen recognized by the antigen binding domain of the antigen binding member and different from the antigen recognized by the antigen binding domain of the auxiliary antigen binding member.

In an instance, the RCAR further comprises:
an antigen binding domain that binds to first target, a transmembrane domain linked to first switch domain of a heterodimerization switch,
an intracellular signaling domain, e.g., a primary intracellular signaling domain, wherein the intracellular signaling domain is linked to a second switch domain of a heterodimerization switch, and
an antigen binding domain that binds to a second target that is different from the first target and a transmembrane domain, wherein the heterodimerization switch is present on the inside of a cell, wherein first switch domain and second switch domain interact together to form a complex in the presence of a heterodimerization molecule on the inside of the cell.

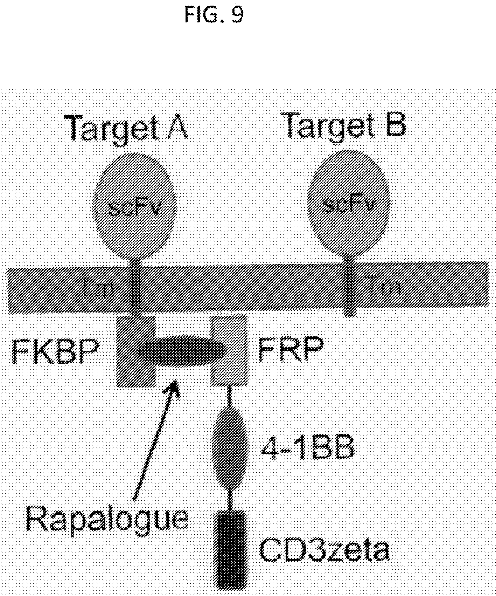

In an instance, the RCAR further comprises
an unswitched auxiliary antigen binding member comprising:
an antigen binding domain, e.g., which binds a second antigen,
a transmembrane domain, and
an intracellular signaling domain, e.g., a primary intracellular signaling domain.
See, e.g., Fig. 9.

In an instance, the unswitched auxiliary antigen binding member further comprises a costimulatory signaling domain.

In an instance, the intracellular signaling member unswitched auxiliary antigen binding member comprises a primary intracellular signaling domain and a costimulatory signaling domain.

In an instance, the unswitched auxiliary antigen binding member comprises a 4-1BB domain. In an instance, the unswitched auxiliary antigen binding member comprises a CD3zeta domain. In an instance, unswitched auxiliary antigen binding member comprises a CD3zeta domain and a 4-1BB domain.

In an instance, the RCAR is associated with, e.g., is provided in the same cell with:
an inhibitor of an inhibitory molecule, e.g., an inhibitor of an inhibitory molecule of Table 3.

In an instance, the RCAR is associated with, e.g., is provided in the same cell with, an shRNA that targets a inhibitory molecule, e.g. a coinhibitory molecule from Table 3.

In an instance, the shRNA targets PD1.

In an instance, the antigen binding domain binds to a target antigen on a cancer cell but does not activate the RCARX cell, e.g., a RCART cell, until a dimerization molecule is administered.

In an instance, the antigen binding domain binds to a target antigen on a target cell, e.g., a cancer cell, but does not promote an immune effector response, e.g., a T cell activation, until the dimerization molecule, e.g., a heterodimerization molecule or homodimerization molecule, is administered.

In an instance, the intracellular signaling member comprises a primary intracellular signaling domain and a costimulatory signaling domain.

In an instance, the intracellular signaling member comprises a 4-1BB domain. In an embodiment, the intracellular signaling member comprises a CD3zeta domain. In an embodiment, the intracellular signaling member comprises a CD3zeta domain and a 4-1BB domain.

In an instance, the RCAR further comprises
an inhibitory counter ligand binding member comprising,
an inhibitory counter ligand binding domain, and
a transmembrane domain or membrane anchor.

In an instance, the inhibitory counter ligand binding member comprises a switch domain that can form a dimerization switch with a switch domain on the intracellular signaling member.

In an instance, the inhibitory counter ligand binding member does not comprise a switch domain that can form a dimerization switch with a switch domain on the intracellular signaling member.

In an instance, the inhibitory counter ligand binding domain is selected from Table 4.

In an instance the RCAR comprises:
a) an intracellular signaling member comprising,
   an intracellular signaling domain, e.g., a primary intracellular signaling domain, and
   a first switch domain;
b) an antigen binding member comprising,
   an antigen binding domain,
   a second switch domain; and
   a transmembrane domain,
   wherein the first and second switch domains are intracellular.

In an instance, the RCAR comprises:
an antigen binding domain, a transmembrane domain, and a primary intracellular signaling domain,
wherein the antigen binding domain is separated from the primary intracellular signaling domain by a dimerization switch comprising the first switch domain and the second switch domain,
wherein the second switch domain is linked to the antigen binding domain and the first switch domain is linked to the intracellular signaling domain, wherein the first and second switch domain interact together to form a complex in the presence of a dimerization molecule.

In an instance, the RCAR comprises:
an antigen binding domain, a transmembrane domain and an intracellular signaling domain, e.g., a primary intracellular signaling domain,
wherein the antigen binding domain is separated from the intracellular signaling domain by a heterodimerization switch present on the inside of a cell,
wherein the heterodimerization switch comprises first switch domain and second switch domain, wherein the first switch domain is linked to the transmembrane domain and the second switch domain is linked to the intracellular signaling domain,
wherein the first switch domain and second switch domain interact together to form a complex in the presence of a heterodimerization molecule on the inside of the cell.

In an instance, the transmembrane domain is disposed between the second switch domain and the antigen binding domain.

In an instance, the intracellular signaling member does not comprise a transmembrane domain.

In an instance, the RCAR comprises:
a) an intracellular signaling member comprising
   an intracellular signaling domain, e.g., a primary intracellular signaling domain,
   a first switch domain,
   a transmembrane domain; and
b) an antigen binding member comprising
   an antigen binding domain, and
   a second switch domain,
wherein the first and second switch domains are extracellular.

In an instance, the RCAR comprises:
an antigen binding domain, a transmembrane domain, and an intracellular signaling domain, e.g., a primary intracellular signaling,
wherein the antigen binding domain is separated from the intracellular signaling domain by a dimerization switch present on the outside of a cell,
wherein the dimerization switch comprises the first switch domain and the second switch domain,
wherein the second switch domain is linked to the antigen binding domain tethered to a membrane anchor and the first switch domain is linked to the transmembrane domain,
wherein the first switch domain and second switch domain interact together to form a complex in the presence of a dimerization molecule on the outside of the cell.

In an instance, the RCAR comprises
an antigen binding domain, a transmembrane domain, and an intracellular signaling domain, e.g., a primary intracellular signaling,
wherein the antigen binding domain is separated from the intracellular signaling domain by a homodimerization switch present on the outside of a cell,
wherein the homodimerization switch comprises the first switch domain and the second switch domain,
wherein the second switch domain is linked to the antigen binding domain tethered to a membrane anchor and first switch domain is linked to the transmembrane domain,
wherein the first switch domain and second switch domain interact together to form a complex in the presence of a homodimerization molecule on the outside of the cell.

In an instance, the second switch domain is disposed between the antigen binding domain and a membrane anchor or transmembrane domain.

In an instance, the antigen binding member does not comprise a transmembrane domain.

In an instance the second switch domain is linked to the antigen binding domain tethered to a membrane anchor and the first switch domain is linked to the transmembrane domain.

In an instance, the dimerization molecule is selected from an antibody molecule, a dual-specific antibody, a monospecific antibody, a non-antibody scaffold, e.g., a fibronectin or adnectin, and a peptide.

In an instance, first switch domain and second switch domain are different and the heterodimerization molecule is a dual specific antibody molecule that binds to the first switch domain and the second switch domain.

In an instance, the RCAR comprises:
a) an intracellular signaling member comprising:
   an intracellular signaling domain, e.g., a primary intracellular signaling domain, and
   a first switch domain;
b) an antigen binding member comprising:
   an antigen binding domain,
   a second switch domain; and
c) and optionally, a transmembrane domain,
wherein said first and second switch domain form an FKBP-FRB based switch.

In an instance, the dimerization switch comprises a first and second FKBP-FRB based switch domain described herein, e.g., in the Switch Domain Module herein above.

In an instance, the FKBP-FRB based switch comprises a switch domain comprising a FRB binding fragment or analog of FKBP and a switch domain comprising an FKBP binding fragment or analog of FRB, and the FKBP binding fragment or analog of FRB comprises one or more mutations which enhances the formation of a complex between an FKBP switch domain, an FRB switch domain, and the dimerization molecule, or a mutation described in the section herein entitled **MODIFIED FKBP/FRB-BASED DIMERIZATION SWITCHES.** E.g., the FKBP binding fragment or analog of FRB comprises:
an E2032 mutation, e.g., an E2032I mutation or E2032L mutation; a T2098 mutation, e.g., a T2098L mutation; or an E2032 and a T2098 mutation, e.g., an E2032I and a T2098L or an E2032L and a T2098L mutation.

In an instance, the RCAR comprises an extracellular FKBP-FRB based switch, e.g., the RCAR comprises:
a) an intracellular signaling member comprising (in the direction of extracellular to cytoplasmic, when positioned in the membrane of a cell):
   a first switch domain,
   a transmembrane domain, and
   an intracellular signaling domain, e.g., a primary intracellular signaling domain;
b) an antigen binding member comprising (in the direction of extracellular to cytoplasmic, when positioned in the membrane of a cell):
   an antigen binding domain,
   a second switch domain, and
   a transmembrane domain or membrane anchoring domain,
   wherein said first and second switch domains form an extracellular FKPB-FRB based switch.

In an instance, the dimerization switch comprises a first and second FKBP-FRB based switch domain described herein, e.g., in the Switch Domain Module herein above.

In an instance, wherein the switch is an FKBP-FRB based switch, the dimerization molecule is an mTOR inhibitor, e.g., an allosteric mTOR inhibitor, e.g., rapamycin or a rapalog, e.g., RAD001.

In an instance, any of the dosing regimes or formulations of an allosteric mTOR inhibitor, e.g., RAD001, described in the section here for a low, immune enhancing, dose of an allosteric mTOR inhibitor, e.g., RAD001, can be administered to dimerize an FKBP-FRB based switch.

In an instance, the switch is an FKBP-FRB based switch and the dimerization molecule is RAD001.

In an instance, 0.1 to 20, 0.5 to 10, 2.5 to 7.5, 3 to 6, or about 5, mgs of RAD001 per week, e.g., delivered once per week, is administered.

In an instance, 0.3 to 60, 1.5 to 30, 7.5 to 22.5, 9 to 18, or about 15 mgs of RAD001 in a sustained release formuation, per week, e.g., delivered once per week,is administered.

In an instance, 0.005 to 1.5, 0.01 to 1.5, 0.1 to 1.5, 0.2 to 1.5, 0.3 to 1.5,0.4 to 1.5, 0.5 to 1.5, 0.6 to 1.5, 0.7 to 1.5, 0.8 to 1.5,1.0 to 1.5, 0.3 to 0.6, or about 0.5 mgs of RAD001 per day, e.g., delivered once once per day, is administered.

In an instance, 0.015 to 4.5, 0.03 to 4.5, 0.3 to 4.5, 0.6 to 4.5, 0.9 to 4.5, 1.2 to 4.5, 1.5 to 4.5, 1.8 to 4.5, 2.1 to 4.5, 2.4 to 4.5,3.0 to 4.5, 0.9 to 1.8, or about 1.5 mgs of RAD001 in a sustained release formulation, per day, e.g., delivered once once per day, is administered.

In an instance, 0.1 to 30, 0.2 to 30, 2 to 30, 4 to 30, 6 to 30, 8 to 30, 10 to 30, 1.2 to 30, 14 to 30, 16 to 30, 20 to 30, 6 to 12, or about 10 mgs of RAD001 in a sustained release formulation, per week, e.g., delivered once once per week, is administered.

In an instance, the RCAR comprises an intracelluar FKBP-FRB based switch, e.g., the RCAR comprises:
a) an intracellular signaling member comprising (e.g, in the direction of amino terminal to carboxy terminal):
   a first switch domain, and
   an intracellular signaling domain, e.g., a primary intracellular signaling domain;
b) an antigen binding member comprising (in the direction of extracellular to cytoplasmic, when positioned in the membrane of a cell):
   an antigen binding domain,
   a transmembrane domain, and
   a second switch domain
   wherein said first and second switch domains form an intracellular FKPB-FRB based switch.

In an instance, the dimerization switch comprises a first and second FKBP-FRB based switch domain described herein, e.g., in the Switch Domain Module herein above.

In an instance, wherein the switch is an FKBP-FRB based switch, the dimerization molecule is an mTOR inhibitor, e.g., an allosteric mTOR inhibitor, e.g., rapamycin or a rapalog, e.g., RAD001.

In an instance, any of the dosing regimes or formulations of an allosteric mTOR inhibitor, e.g., RAD001, described in the section here for a low, immune enhancing, dose of an allosteric mTOR inhibitor, e.g., RAD001, can be administered to dimerize an FKBP-FRB based switch.

In an instance, the switch is an FKBP-FRB based switch and the dimerization molecule is RAD001.

In an instance, 0.1 to 20, 0.5 to 10, 2.5 to 7.5, 3 to 6, or about 5, mgs of RAD001 per week, e.g., delivered once per week, is administered.

In an instance, 0.3 to 60, 1.5 to 30, 7.5 to 22.5, 9 to 18, or about 15 mgs of RAD001 in a sustained release formuation, per week, e.g., delivered once per week,is administered.

In an instance, 0.005 to 1.5, 0.01 to 1.5, 0.1 to 1.5, 0.2 to 1.5, 0.3 to 1.5,0.4 to 1.5, 0.5 to 1.5, 0.6 to 1.5, 0.7 to 1.5, 0.8 to 1.5, 1.0 to 1.5, 0.3 to 0.6, or about 0.5 mgs of RAD001 per day, e.g., delivered once once per day, is administered.

In an instance, 0.015 to 4.5, 0.03 to 4.5, 0.3 to 4.5, 0.6 to 4.5, 0.9 to 4.5, 1.2 to 4.5, 1.5 to 4.5, 1.8 to 4.5, 2.1 to 4.5, 2.4 to 4.5, 3.0to 4.5, 0.9 to 1.8, or about 1.5 mgs of RAD001 in a sustained release formulation, per day, e.g., delivered once once per day, is administered.

In an instance, 0.1 to 30, 0.2 to 30, 2 to 30, 4 to 30, 6 to 30, 8 to 30, 10 to 30, 1.2 to 30, 14 to 30, 16 to 30, 20 to 30, 6 to 12, or about 10 mgs of RAD001 in a sustained release formulation, per week, e.g., delivered once once per week, is administered.

In an instance the RCAR comprises:
a) an intracellular signaling member comprising (e.g., in the amino terminal to carboxy terminal direction):
   a first switch domain, and
   an intracellular signaling domain, e.g., a primary intracellular signaling domain;
b) an antigen binding member comprising (in the direction of extracellular to cytoplasmic, when positioned in the membrane of a cell):
   an antigen binding domain,
   a transmembrane domain, and
   a second switch domain.
   wherein said first and second switch domains form an intracellular switch.

In an instance, the switch domains are components of a heterodimerization switch.

In an instance, the switch domains are components of a homodimerization switch.

In an instance, the dimerization switch comprises a FKBP-FRB based switch, e.g., an FKBP-FRB based switch described herein, e.g., an FKBP-FRB based switch as described herein, e.g., in the Dimerization Switch Module.

In an instance, the dimerization switch comprises a GyrB-GyrB based switch, e.g., an GyrB-GyrB based switch described herein, e.g., an GyrB-GyrB based switch as described herein, e.g., in the Dimerization Switch Module.

In an instance, the dimerization switch comprises a GAI-GID1 based switch, e.g., an GAI-GID1 based switch described herein, e.g., an GAI-GID1 based switch as described herein, e.g., in the Dimerization Switch Module.

In an instance, the dimerization switch comprises a Halotag/SNAP-tag based switch, e.g., a Halotag/SNAP-tag based switch described herein, e.g., a Halotag/SNAP-tag based switch as described herein, e.g., in the Dimerization Switch Module.

In an instance, the RCAR comprises:
a) an extracellular signaling member comprising (in the direction of extracellular to cytoplasmic, when positioned in the membrane of a cell):
   a first switch domain, e.g., an FKBP switch domain,
   a transmembrane domain, and
   an intracellular signaling domain, e.g., a primary intracellular signaling domain;
b) an antigen binding member comprising (in the direction of extracellular to cytoplasmic, when inserted into the membrane of a cell):
   an antigen binding domain,
   a second switch domain, e.g., a FRB switch domain, and
   a transmembrane domain or membrane anchoring domain,
   wherein said first and second switch domains form an extracellular switch.

In an instance, the switch domains are components of a heterodimerization switch.

In an instance, the switch domains are components of a homodimerization switch.

In an instance, the dimerization switch comprises a FKBP-FRB based switch, e.g., an FKBP-FRB based switch described herein, e.g., an FKBP-FRB based switch as described herein, e.g., in the Dimerization Switch Module.

In an instance, the dimerization switch comprises a GyrB-GyrB based switch, e.g., an GyrB-GyrB based switch described herein, e.g., an GyrB-GyrB based switch as described herein, e.g., in the Dimerization Switch Module.

In an instance, the dimerization switch comprises a GAI-GID1 based switch, e.g., an GAI-GID1 based switch described herein, e.g., an GAI-GID1 based switch as described herein, e.g., in the Dimerization Switch Module.

In an instance, the dimerization switch comprises a Halotag/SNAP-tag based switch, e.g., a Halotag/SNAP-tag based switch described herein, e.g., a Halotag/SNAP-tag based switch as described herein, e.g., in the Dimerization Switch Module.

An instance provides RCARs wherein the antigen binding member is not tethered to the surface of the CAR cell. This allows a cell having an intracellular signaling member to be conveniently be paired with one or more antigen binding domains, without transforming the cell with sequence that encodes the antigen binding member, as is discussed in the section herein entitled, **UNIVERSAL RCARs.** These are sometimes referred to herein as universal RCARs.

In an instance, the RCAR comprises:
a) an intracellular signaling member comprising:
   a transmembraine domain,
   an intracellular signaling domain, e.g., a primary intracellular signaling domain, and
   a first switch domain, e.g., an FKPB switch domain; and
b) an antigen binding member comprising:
   an antigen binding domain, and
   a second switch domain, e.g., a FRB switch domain,
   wherein the antigen binding member does not comprise a transmembrane domain or membrane anchoring domain, and, optionally, does not comprise an intracellular signaling domain.

In an instance, the first and second switch domains comprises FKBP/FRB based switch.

In an instance, the first switch domain comprises an FRB binding fragment of FKBP.

In an instance, the second switch domain comprises an FKBP binding fragment of FRB.

In an instance, the FKBP-FRB based switch comprises a switch domain comprising a FRB binding fragment or analog of FKBP and a switch domain comprising an FKBP binding fragment or analog of FRB, and the FKBP binding fragment or analog of FRB comprises one or more mutations which enhances the formation of a complex between an FKBP switch domain, an FRB switch domain, and the dimerization molecule, or a mutation described in the section herein entitled **MODIFIED FKBP/FRB-BASED DIMERIZATION SWITCHES.** E.g., the FKBP binding fragment or analog of FRB comprises: an E2032 mutation, e.g., an E2032I mutation or E2032L mutation; a T2098 mutation, e.g., a T2098L mutation; or an E2032 and a T2098 mutation, e.g., an E2032I and a T2098L or an E2032L and a T2098L mutation.

In such instance, the RCAR comprises a multi switch comprising a plurality of, e.g., 2, 3, 4, 5, 6, 7, 8, 9, or 10, switch domains, independently, on a first member, e.g., an antigen binding member, and a the second member, e.g., an intracellular signaling member, as described in the section herein entitled **MULTIPLE SWITCH DOMAINS.** In an instance, the first member comprises a plurality of first switch domains, e.g., FKBP-based switch domains, and the second member comprises a plurality of second switch domains, e.g., FRB-based switch domains. In an instance, the first member comprises a first and a second switch domain, e.g., a FKBP-based switch domain and a FRB-based switch domain, and the second member comprises a first and a second switch domain, e.g., a FKBP-based switch domain and a FRB-based switch domain.

In an instance the intracellular signaling member comprises a primary signaling domain, e.g., form Table 1, and a costimulatory signaling domain, e.g., from Table 2.

In an instance the intracellular signaling member comprises a primary signaling domain, e.g., form Table 1, and plurality, e.g., 2 or 3, costimulatory signaling domain, e.g., from Table 2.

In an instance, the two or more costimulatory domains can be the same costimulatory signaling domain or different costimulatory signaling domains.

In an instance, the intracellular signaling member comprises CD3zeta.

In an instance, the RCAR further comprises:
c) a second antigen binding member comprising:
a second antigen binding domain, e.g., a second antigen binding domain that binds a different antigen than is bound by the antigen binding domain; and
a second switch domain.

In an instance, the antigen binding member comprises a plurality of, e.g., 2, 3, 4, or 5, antigen binding domains, e.g., scFvs, wherein each antigen binding domain binds to a target antigen. In an instance, two or more of the antigen binding domains can bind to different antigens. In an instance, two or more of the antigen binding domains can bind to the same antigen, e.g., the same or different epitopes on the same antigen. In instances, a linker or hinge region is optionally disposed between two or each of the antigen binding domains.

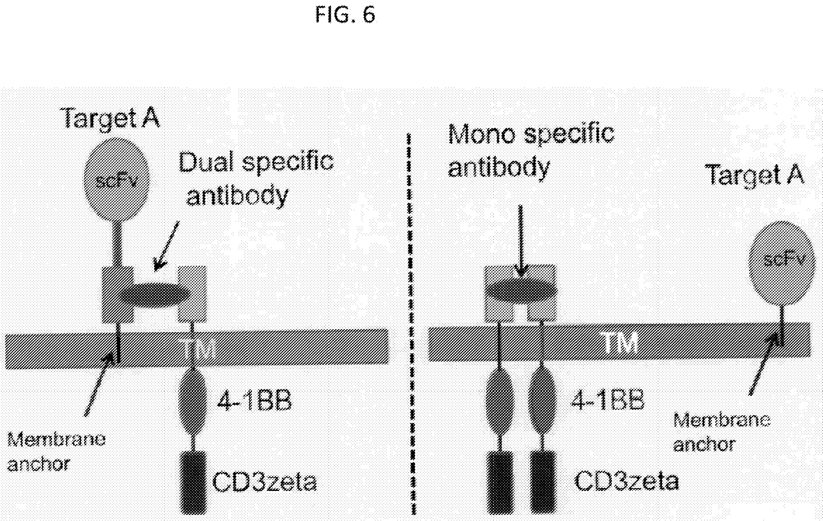

In a second aspect, the disclosure features, a RCAR, e.g., an isolated RCAR comprising:
a) an intracellular signaling member comprising,
   an intracellular signaling domain, e.g., a primary intracellular signaling domain,
   a first switch domain, and
   a transmembrane domain; and
b) an antigen binding member comprises
   an antigen binding domain, and
   a membrane anchor or a second transmembrane domain.
   See, e.g., Fig 6 right panel.

In an instance, the antigen binding member does not comprise a switch domain that forms a dimerization switch with an intracellular signaling member switch.

In an instance the antigen binding member does not comprise an intracellular signaling domain.

In an instance, two copies of the first switch domain are components of a homodimerization switch.

In an instance, the RCAR further comprises:
a second intracellular signaling member comprising
   an intracellular signaling domain, e.g., a primary intracellular signaling domain, and
   a second switch domain,
wherein the first switch domain and the second switch domain are components of a heterodimerization switch.

In an instance, dimerization of the switch domains results in clustering of intracellular signaling members.

In an instance, dimerization of the switch domains results in an increase in signaling by the intracellular signaling domains.

In an instance, the dimerization switch is extracellular.

In an instance, the dimerization switch is intracellular.

In an instance:
the dimerization switch is a an extracellular homodimerization switch, and
the antigen binding member does not comprise a switch domain that can dimerize with a switch domain on the intracellular signaling member.

In an instance:
the dimerization switch is an intracellular homodimerization switch, and
the antigen binding member does not comprise a switch domain that can dimerize with a switch domain on the intracellular signaling member.

In an instance, the RCAR comprises:
a second intracellular signaling member comprising an intracellular signaling domain and a second switch domain, which together with the first switch domain, forms an extracellular heterodimerization switch, and
the antigen binding member does not comprise a switch domain that can dimerize with a switch domain on an intracellular signaling member.

In an instance, the RCAR comprises:
a second intracellular signaling member comprising an intracellular signaling domain and a second switch domain, which together with the first switch domain, forms an intracellular heterodimerization switch, and
the antigen binding member does not comprise a switch domain that can dimerize with a switch domain on an intracellular signaling member.

In an instance, the RCAR comprises:
a second intracellular signaling member comprising an intracellular signaling domain and a second switch domain, which together with the first switch domain, forms an extracellular homodimerization switch, and
the antigen binding member does not comprise a switch domain that can dimerize with a switch domain on an intracellular signaling member.

In an instance, the RCAR comprises:
a second intracellular signaling member comprising an intracellular signaling domain and a second switch domain, which together with the first switch domain, forms an intracellular homodimerization switch, and
the antigen binding member does not comprise a switch domain that can dimerize with a switch domain on an intracellular signaling member.

In an instance, the intracellular signaling domain is a primary intracellular signaling domain, selected, e.g., from Table 1.

In an instance, the primary intracellular signaling domain comprises a CD3zeta domain.

In an instance, the intracellular signaling domain is a costimulatory signaling domain, e.g., selected from the list in Table 2.

In an instance, the costimulatory signaling domain comprises a 4-1BB domain.

In an instance, the RCAR comprises a second intracellular signaling domain.

In an instance, the second intracellular signaling domain is a primary intracellular signaling domain, e.g., selected from the list in Table 1.

In an instance, the second intracellular signaling domain is a costimulatory signaling domain, e.g., selected from the list in Table 2.

In an instance, the first and second intracellular signaling domains comprise:
a 4-1BB domain and a CD3zeta domain; or
a CD28 domain and a 4-1BB domain.

In an instance, the RCAR comprises a third intracellular signaling domain.

In an instance, the third intracellular signaling domain is a primary intracellular signaling domain, e.g., selected from the list in Table 1.

In an instance, the third intracellular signaling domain is a costimulatory signaling domain, e.g., selected from the list in Table 2.

In an instance, one of the first, second and third intracellular signaling domain is a primary intracellular signaling domain, e.g., selected from the list in Table 1, and the other two are costimulatory signaling domains, e.g., selected from the list in Table 2.

In an instance, two of the first, second and third intracellular signaling domains are primary intracellular signaling domains, e.g., selected from the list in Table 1, and the other is a costimulatory signaling domain, e.g., selected from the list in Table 2.

In an instance, each of the first, second and third intracellular signaling domains is a primary intracellular signaling domain, e.g., selected from the list in Table 1.

In an instance, each of the first, second and third intracellular signaling domains is a costimulatory signaling domain, e.g., selected from the list in Table 2.

In an instance, the first, second, and third intracellular signaling domains comprise: A CD28 domain; a 4-1BB domain, and a CD3zeta domain.

In an instance, the RCAR comprises a fourth intracellular signaling domain.

In an instance, the fourth intracellular signaling domain is a primary intracellular signaling domain, e.g., selected from the list in Table 1.

In an instance, the fourth intracellular signaling domain is a costimulatory signaling domain, e.g., selected from the list in Table 2.

In an instance, one of the first, second ,third and fourth intracellular signaling domain is a primary intracellular signaling domain, e.g., selected from the list in Table 1 and the other three are costimulatory signaling domains, e.g., selected from the list in Table 2.

In an instance, two of the first, second, third, and fourth intracellular signaling domains are primary intracellular signaling domains, e.g., selected from the list in Table 1, and the other two are costimulatory signaling domain, e.g., selected from the list in Table 2.

In an instance, three of the first, second, third, and fourth intracellular signaling domains are primary intracellular signaling domains, e.g., selected from the list in Table 1, and the other is a costimulatory signaling domain, e.g., selected from the list in Table 2.

In an instance, each of the first, second, third, and fourth intracellular signaling domains is a primary intracellular signaling domain, e.g., selected from the list in Table 1.

In an instance, each of the first, second, third, and fourth intracellular signaling domains is a costimulatory signaling domain, e.g., selected from the list in Table 2.

In an instance, the order of switch domain and the intracellular signaling domain (isd) or domains is as follows, beginning with the amino terminus:
switch/isd;
switch/isd1/isd2;
isd1/switch/isd2;
isd1/isd2/switch;
switch/isd1/isd2/isd3;
isd1/isd2/isd3/switch;
isd1/switch/isd2/isd3; and
isd1/isd2/switch/isd3.

In an instance, the order of switch domain and the intracellular signaling domain (isd) or domains is as follows, beginning with the carboxy terminus:
switch/isd;
switch/isd1/isd2;
isd1/switch/isd2;
isd1/isd2/switch;
switch/isd1/isd2/isd3;
isd1/isd2/isd3/switch;
isd1/switch/isd2/isd3; and
isd1/isd2/switch/isd3.

In an instance, the dimerization molecule, e.g., a polypeptide, e.g., an antibody molecule, comprises a first moiety, e.g., a first variable region, that specifically binds the first switch domain, and a second moiety, e.g., a second variable region, that specifically binds the second switch domain, wherein the first and second switch domains are components of a heterodimerization switch.

In an instance, the dimerization molecule is a polypeptide, e.g., an antibody molecule that binds the switch domains.

In an instance, the dimerization molecule, e.g., a polypeptide, e.g., an antibody molecule, specifically binds the first and second switch domain, wherein the first and second switch domains are components of a homodimerization switch.

In an instance, the heterodimermerization molecule is selected from the group consisting of an antibody molecule, a non-antibody scaffold, e.g., a fibronectin or adnectin, molecule switch, and a peptide.

In an instance, the homodimerization molecule is a monospecific antibody molecule.

In an instance, the dimerization molecule is a dual-specific antibody molecule.

In an instance, the antigen binding domain binds to a target antigen on a cancer cell but does not promote an immune effector response of a T cell, until the dimerization molecule is administered.

In an instance, the dimerization switch comprises a FKBP-FRB based switch.

In an instance, the dimerization switch comprises:
a switch domain comprising a rapamycin analog binding sequence having at least 80, 85, 90, 95, 98, or 99 % identity with FKBP, and a switch domain comprising a rapamycin analog binding sequence binding sequence having at least 80, 85, 90,95, 98, or 99 % identity with FRB.

In an instance, the FKBP-FRB based switch comprises a switch domain comprising a FRB binding fragment or analog of FKBP and a switch domain comprising an FKBP binding fragment or analog of FRB, and the FKBP binding fragment or analog of FRB comprises one or more mutations which enhances the formation of a complex between an FKBP switch domain, an FRB switch domain, and the dimerization molecule, or a mutation described in the section herein entitled **MODIFIED FKBP/FRB-BASEDDIMERIZATION SWITCHES.** E.g., the FKBP binding fragment or analog of FRB comprises: an E2032 mutation, e.g., an E2032I mutation or E2032L mutation; a T2098 mutation, e.g., a T2098L mutation; or an E2032 and a T2098 mutation, e.g., an E2032I and a T2098L or an E2032L and a T2098L mutation.

In an instance, the dimerization switch comprises:
a switch domain comprising a rapamycin analog binding sequence that differs by no more than 10, 9, 8, 7, 6, 5, 4, 3, 2, or 1 amino acid residues from the corresponding sequence of FKBP, and a switch domain comprising a rapamycin analog binding sequence that differs by no more than 10, 9, 8, 7, 6, 5, 4, 3, 2, or 1 amino acid residues from the corresponding sequence of FRB.

In an instance, wherein the switch is an FKBP-FRB based switch, the dimerization molecule is an mTOR inhibitor, e.g., an allosteric mTOR inhibitor, e.g., rapamycin or a rapalog, e.g., RAD001.

In an instance, any of the dosing regimes or formulations of an allosteric mTOR inhibitor, e.g., RAD001, described in the section here for a low, immune enhancing, dose of an allosteric mTOR inhibitor, e.g., RAD001, can be administered to dimerize an FKBP-FRB based switch.

In an instance, the switch is an FKBP-FRB based switch and the dimerization molecule is RAD001.
In an instance, 0.1 to 20, 0.5 to 10, 2.5 to 7.5, 3 to 6, or about 5, mgs of RAD001 per week, e.g., delivered once per week, is administered.

In an instance, 0.3 to 60, 1.5 to 30, 7.5 to 22.5, 9 to 18, or about 15 mgs of RAD001 in a sustained release formuation, per week, e.g., delivered once per week,is administered.

In an instance 0.005 to 1.5, 0.01 to 1.5, 0.1 to 1.5,0.2 to 1.5,0.3 to 1.5, 0.4 to 1.5, 0.5 to 1.5, 0.6 to 1.5, 0.7 to 1.5, 0.8 to 1.5, 1.0 to 1.5,0.3 to 0.6, or about 0.5 mgs of RAD001 per day, e.g., delivered once once per day, is administered.

In an instance, 0.015 to 4.5, 0.03 to 4.5, 0.3 to 4.5, 0.6 to 4.5, 0.9 to 4.5, 1.2 to 4.5, 1.5 to 4.5, 1.8 to 4.5, 2.1 to 4.5, 2.4 to 4.5, 3.0 to 4.5,0.9 to 1.8, or about 1.5 mgs of RAD001 in a sustained release formulation, per day, e.g., delivered once once per day, is administered.

In an instance, 0.1 to 30, 0.2 to 30, 2 to 30, 4 to 30, 6 to 30, 8 to 30, 10 to 30, 1.2 to 30, 14 to 30, 16 to 30, 20 to 30, 6 to 12, or about 10 mgs of RAD001 in a sustained release formulation, per week, e.g., delivered once once per week, is administered.

In an instance the dimerization switch comprises:
a switch domain comprising a rapamycin, or rapamycin analog, binding sequence from FKBP, and a switch domain comprising a rapamycin, or rapamycin analog, binding sequence from FRB, e.g., a sequence comprising a lysine at residue 2098.

In an instance, the dimerization switch comprises:
a switch domain comprising a rapamycin analog binding sequence from FKBP, and a switch domain comprising a rapamycin analog binding sequence from FRB, e.g., a sequence comprising a lysine at residue 2098.

In an instance, the dimerization switch comprises:
a switch domain comprising a AP21967 binding sequence from FKBP, and a switch domain comprising a AP21967 binding sequence from FRB, e.g., a sequence comprising a lysine at residue 2098.

In an instance:
the first switch domain comprises,
   a rapamycin, or rapamycin analog, binding sequence from FKBP;
   a rapamycin analog binding sequence from FKBP; or
   an AP21967 binding sequence from FKBP; and,
the second switch domain comprises,
   a rapamycin, or rapamycin analog, binding sequence from FRB;
   a rapamycin analog binding sequence from FRB; or
   an AP21967 binding sequence from FRB, e.g., a sequence comprising a lysine at residue 2098.

In an instance:
the first switch domain comprises,
   a rapamycin, or rapamycin analog, binding sequence from FRB;
   a rapamycin analog binding sequence from FRB; or
   an AP21967 binding sequence from FRB, e.g., a sequence comprising a lysine at residue 2098; and,
the second switch domain comprises,
   a rapamycin, or rapamycin analog, binding sequence from FKBP;
   a rapamycin analog binding sequence from FKBP; or
   an AP21967 binding sequence from FKBP.

In an instance:
the first switch domain comprises an AP21967 binding sequence from FKBP; and,
the second switch domain comprises an AP21967 binding sequence from FRB, e.g., a sequence comprising a lysine at residue 2098.

In an instance, the first switch domain comprises an AP21967 binding sequence from FRB, e.g., a sequence comprising a lysine at residue 2098; and,
the second switch domain comprises an AP21967 binding sequence from FKBP.

In an instance, the dimerization molecule is a rapamycin analogue, e.g., AP21967.

In an instance, the dimerization switch comprises a GyrB-GyrB based switch.

In an instance, the dimerization switch comprises:
a switch domain comprising a coumermycin binding sequence having at least 80, 85, 90, 95, 98, or 99 % identity with the 24 K Da amino terminal sub-domain of GyrB.

In an instance, the dimerization switch comprises:
a switch domain comprising a coumermycin binding sequence that differs by no more than 10, 9, 8, 7, 6, 5, 4, 3, 2, or 1 amino acid residues from the corresponding sequence of 24 K Da amino terminal sub-domain of GyrB.

In an instance, the dimerization switch comprises:
a switch domain comprising a coumermycin binding sequence from the 24 K Da amino terminal sub-domain of GyrB.

In an instance, the dimerization switch comprises:
the 24 K Da amino terminal sub-domain of GyrB.

In an instance, the dimerization molecule is a coumermycin.

In an instance, the dimerization switch comprises a GAI-GID1 based switch.

In an instance, the dimerization switch comprises:
a GID1 switch domain comprising a gibberellin, or gibberellin analog, e.g., GA₃, binding sequence having at least 80, 85, 90, 95, 98, or 99 % identity with GID1, and a switch domain comprising a GAI switch domain having at least 80, 85, 90, 95, 98, or 99 % identity with GAI.

In an instance, the dimerization switch comprises: a GID1 switch domain comprising a gibberellin, or gibberellin analog, e.g., GA₃, binding sequence that differs by no more than 10, 9, 8, 7, 6, 5, 4, 3, 2, or 1 amino acid residues from the corresponding sequence of FKBP, and a GAI switch domain that differs by no more than 10, 9, 8, 7, 6, 5, 4, 3, 2, or 1 amino acid residues from the corresponding sequence of FRB.

In an instance:
the first switch domain comprises a GID1 switch domain; and,
the second switch domain comprises a GAI switch domain.

In an instance:
the first switch domain comprises a GAI switch domain; and,
the second switch domain comprises a GID1 switch domain.
In an instance, the dimerization molecule is GA₃-AM.

In an instance, the dimerization molecule is GA₃.

In an instance, the dimerization molecule is a small molecule, e.g., is other than a polypeptide.

In an instance, the dimerization molecule is a polypeptide, e.g., a polypeptide, e.g., an antibody molecule, or a non-antibody scaffold, e.g., a fribronectin or adnectin, having specific affinity for one or both of the first and second switch domains.

In an instance, the dimerization molecule, e.g. a polypeptide, is an antibody molecule.

In an instance, the dimerization switch comprises a Halotag/SNAP-tag based switch.

In an instance, the dimerization switch comprises:
a Halotag switch domain comprising having at least 80, 85, 90, 95, 98, or 99 % identity with SEQ ID NO: 14, and a SNAP-tag switch domain having at least 80, 85, 90, 95,98, or 99 % identity with SEQ ID NO: 15.

In an instance, the dimerization switch comprises:
a Halotag switch domain comprising that differs by no more than 10, 9, 8, 7, 6, 5, 4, 3, 2, or 1 amino acid residues from SEQ ID NO: 14, and a SNAP-tag switch domain that differs by no more than 10, 9, 8, 7, 6, 5, 4, 3, 2, or 1 amino acid residues from SEQ ID NO: 15.

In an instance:
the first switch domain comprises a Halotag switch domain; and,
the second switch domain comprises a SNAP-tag switch domain.

In an instance:
the first switch domain comprises a SNAP-tag switch domain; and,
the second switch domain comprises a Halotag switch domain.

In an instance, the dimerization molecule comprises structure 5.

In an instance, the dimerization molecule comprises three or more domains, e.g., protein tags, that bind a switch domain, e.g., a polypeptide, e.g., an antibody molecule or non-antibody scaffold, having affinity for the domain.

In an instance, the dimerization molecule is a non-covalent dimerization molecule.

In an instance, the dimerization molecule is covalent dimerization molecule.

In an instance, the dimerization switch, e.g., a homodimerization switch, e.g., an extracellular homodimerization switch, comprises switch domains that comprise tag molecules, e.g., a c-myc peptide tag, flag peptide tag, HA peptide tag or V5 peptide tag, and the dimerization switch comprises polypeptides with affinity for the switch domains, e.g., antibody molecules and non-antibody scaffold.

In an instance, the RCAR further comprises a second order dimerization switch.

In an instance, the dimerization molecule has a valency of greater than two, e.g., it is multi-valent, and binds, and thus clusters or dimerizes, more than two switch domains.

In an instance, the RCAR comprises:
a first transmembrane domain and a first intracellular signaling domain, e.g., a primary intracellular signaling domain, and
a second transmembrane domain and a second intracellular signaling domain, e.g., a primary intracellular signaling domain, and
an antigen binding domain tethered to a membrane anchor,
wherein the first and second transmembrane domains are separated from each other by a heterodimerization switch present on the outside of a cell,
wherein the heterodimerization switch comprises first switch domain and second switch domain, wherein the first and second switch domains of the heterodimerization switch interact together to form a complex in the presence of a heterodimerization molecule on the either the inside or outside of the cell.

In an instance, the antigen binding member comprises
an antigen binding domain,
a second transmembrane domain, and
a costimulatory signaling domain, e.g., a costimulatory signaling domain from Table 2, e.g., a 4-1BB domain.

In an instance, the RCAR further comprises:
an unswitched auxiliary antigen binding member comprising:
an antigen binding domain, e.g., which binds a second antigen,
a transmembrane domain, and
an intracellular signaling domain, e.g., a primary intracellular signaling domain.

In an instance, the unswitched auxiliary antigen binding member further comprises a costimulatory signaling domain.

In an instance, the intracellular signaling member unswitched auxiliary antigen binding member comprises a primary intracellular signaling domain and a costimulatory signaling domain.

In an instance, the unswitched auxiliary antigen binding member comprises a 4-1BB domain.

In an instance, the unswitched auxiliary antigen binding member comprises a CD3zeta domain.

In an instance, the unswitched auxiliary antigen binding member comprises a CD3zeta domain and a 4-1BB domain.

In an instance, the RCAR is associated with, e.g., is provided in the same cell with an inhibitor of an inhibitory molecule, e.g., an inhibitor of a inhibitory molecule of Table 3.

In an instance, the RCAR is associated with, e.g., is provided in the same cell with, an shRNA that targets an inhibitory molecule, e.g. a coinhibitory molecule from Table 3.

In an instance, the shRNA targets PD1.

In an instance, the antigen binding domain binds to a target antigen on a cancer cell but does not activate the RCARX cell, e.g., a RCART cell, until a dimerization molecule is administered.

In an instance, the antigen binding domain binds to a target antigen on a target cell, e.g., a cancer cell, but does not promote an immune effector response, e.g., a T cell activation, until the dimerization molecule, e.g., a heterodimerization molecule or homodimerization molecule, is administered.

RCARs disclosed herein can include, e.g., in place of an scFv-based antigen binding domain, an extracelluar domain of an inhibitory receptor, e.g., PD1. While not wising to be bound by theory, it is believed that engagement of the inhibitory extracellular domain with its counter ligand (which normally down regulates the immune response), activates the immune response. This is discussed immediately below.

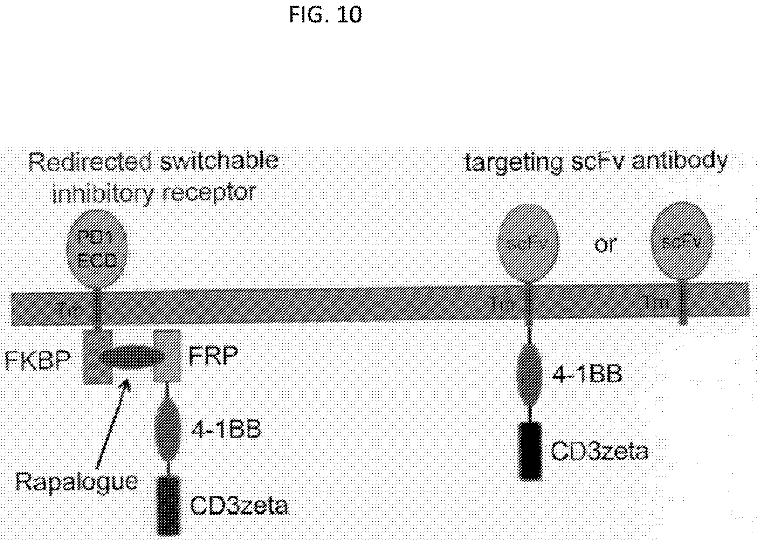

In a third aspect, the disclosure features, an RCAR, e.g., an isolated RCAR, comprising:
a) an inhibitory extracellular domain member comprising,
   an inhibitory extracellular domain,
   a transmembrane region, and
   a switch domain;
b) an intracellular signaling member comprising,
   an intracellular signaling domain, e.g., a primary intracellular signaling domain, and
   a switch domain; and optionally,
c) an antigen binding member comprising,
   an antigen binding domain, and
   a membrane anchoring domain or a transmembrane domain.
   See, e.g., Fig. 10.

In an instance:
the antigen binding member does not comprise an intracellular signaling domain and does not comprise a switch domain that forms a dimerization switch with a switch domain on the inhibitory extracellular domain member or the switch domain on the intracellular signaling member. See, e.g., Fig. 10, far right panel.

In an instance, the antigen binding member comprises
an antigen binding domain,
a second transmembrane domain, and
a costimulatory signaling domain, e.g., a costimulatory signaling domain from Table 2, e.g., a 4-1BB domain.

In an instance: the inhibitory extracellular domain is selected from Table 4.

In an instance: the first switch domain is linked to the intracellular signaling domain and second switch domain is linked to the transmembrane domain.

In an instance: the inhibitory extracellular domain binds to its ligand on the target cell and redirects signal activation in the presence of a heterodimerization molecule.

In an instance, the intracellular signaling domain is a primary intracellular signaling domain, selected, e.g., from Table 1.

In an instance, the primary intracellular signaling domain comprises a CD3zeta domain.

In an instance, the intracellular signaling domain is a costimulatory signaling domain, e.g., selected from the list in Table 2.

In an instance, the costimulatory signaling domain comprises a 4-1BB domain.

In an instance, the RCAR comprises a second intracellular signaling domain.

In an instance, the second intracellular signaling domain is a primary intracellular signaling domain, e.g., selected from the list in Table 1.

In an instance the second intracellular signaling domain is a costimulatory signaling domain, e.g., selected from the list in Table 2.

In an instance, the first and second intracellular signaling domains comprise:
a 4-1BB domain and a CD3zeta domain; or
a CD28 domain and a 4-1BB domain.

In an instance, the RCAR comprises a third intracellular signaling domain.

In an instance, the third intracellular signaling domain is a primary intracellular signaling domain, e.g., selected from the list in Table 1.

In an instance, the third intracellular signaling domain is a costimulatory signaling domain, e.g., selected from the list in Table 2.

In an instance, one of the first, second and third intracellular signaling domain is a primary intracellular signaling domain, e.g., selected from the list in Table 1, and the other two are costimulatory signaling domains, e.g., selected from, Table 2.

In an instance, two of the first, second and third intracellular signaling domains are primary intracellular signaling domains, e.g., selected from the list in Table 1, and the other is a costimulatory signaling domain, e.g., selected from, Table 2.

In an instance, each of the first, second and third intracellular signaling domains is a primary intracellular signaling domain, e.g., selected from the list in Table 1.

In an instance, each of the first, second, and third intracellular signaling domains is a costimulatory signaling domain, e.g., selected from the list in Table 2.

In an instance, the first, second, and third intracellular signaling domains comprise: A CD28 domain; a 4-1BB domain, and a CD3zeta domain.

In an instance, the RCAR comprises a fourth intracellular signaling domain.

In an instance, the fourth intracellular signaling domain is a primary intracellular signaling domain, e.g., selected from the list in Table 1.

In an instance, the fourth intracellular signaling domain is a costimulatory signaling domain, e.g., selected from the list in Table 2.

In an instance, one of the first, second, third and fourth intracellular signaling domain is a primary intracellular signaling domain, e.g., selected from the list in Table 1 and the other three are costimulatory signaling domains, e.g., selected from the list in Table 2.

In an instance, two of the first, second, third, and fourth intracellular signaling domains are primary intracellular signaling domains, e.g., selected from the list in Table 1, and the other two are costimulatory signaling domains, e.g., selected from the list in Table 2.

In an instance, three of the first, second, third, and fourth intracellular signaling domains are primary intracellular signaling domains, e.g., selected from the list in Table 1, and the other is a costimulatory signaling domain, e.g., selected from the list in Table 2.

In an instance, each of the first, second, third, and fourth intracellular signaling domains is a primary intracellular signaling domain, e.g., selected from the list in Table 1.

In an instance, each of the first, second, third, and fourth intracellular signaling domains is a costimulatory signaling domain, e.g., selected from the list in Table 2.

In an instance, the order of switch domain and the intracellular signaling domain (isd) or domains is as follows, beginning with amino terminus:
switch/isd;
switch/isd1/isd2;
isd1/switch/isd2;
isd1/isd2/switch;
switch/isd1/isd2/isd3;
isd1/isd2/isd3/switch;
isd1/switch/isd2/isd3; and
isd1/isd2/switch/isd3.

In an instance, the order of switch domain and the intracellular signaling domain (isd) or domains is as follows, beginning with carboxy terminus:
switch/isd;
switch/isd1/isd2;
isd1/switch/isd2;
isd1/isd2/switch;
switch/isd1/isd2/isd3;
isd1/isd2/isd3/switch;
isd1/switch/isd2/isd3; and
isd1/isd2/switch/isd3.

In an instance, the switch domains are components of a heterodimerization switch.

In an instance, the switch domains are components of a homodimerization switch.

In an instance, the dimerization switch is intracellular.

In an instance, the dimerization switch is extracellular.

In an instance, the transmembrane domain disposed on the antigen binding member and the dimerization switch, e.g., a heterodimerization switch or homodimerization switch, is intracellular.

In an instance, where the transmembrane domain disposed on the intracellular signaling member and the dimerization switch, e.g., heterodimerization or homodimerization switch, is extracellular.

In an instance, the dimerization switch comprises a FKBP-FRB based switch.

In an instance, the dimerization switch comprises:
a switch domain comprising a rapamycin analog binding sequence having at least 80, 85, 90, 95, 98, or 99 % identity with FKBP, and a switch domain comprising a rapamycin analog binding sequence binding sequence having at least 80, 85, 90, 95, 98, or 99 % identity with FRB.

In an instance, the FKBP-FRB based switch comprises a switch domain comprising a FRB binding fragment or analog of FKBP and a switch domain comprising an FKBP binding fragment or analog of FRB, and the FKBP binding fragment or analog of FRB comprises one or more mutations which enhances the formation of a complex between an FKBP switch domain, an FRB switch domain, and the dimerization molecule, or a mutation described in the section herein entitled **MODIFIED FKBP/FRB-BASED DIMERIZATION SWITCHES.** E.g., the FKBP binding fragment or analog of FRB comprises: an E2032 mutation, e.g., an E2032I mutation or E2032L mutation; a T2098 mutation, e.g., a T2098L mutation; or an E2032 and a T2098 mutation, e.g., an E2032I and a T2098L or an E2032L and a T2098L mutation.

In an instance, the dimerization switch comprises:
a switch domain comprising a rapamycin analog binding sequence that differs by no more than 10, 9, 8, 7, 6, 5, 4, 3, 2, or 1 amino acid residues from the corresponding sequence of FKBP, and a switch domain comprising a rapamycin analog binding sequence that differs by no more than 10, 9, 8, 7, 6, 5, 4, 3, 2, or 1 amino acid residues from the corresponding sequence of FRB.

In an instance, wherein the switch is an FKBP-FRB based switch, the dimerization molecule is an mTOR inhibitor, e.g., an allosteric mTOR inhibitor, e.g., rapamycin or a rapalog, e.g., RAD001.

In an instance, any of the dosing regimes or formulations of an allosteric mTOR inhibitor, e.g., RAD001, described in the section here for a low, immune enhancing, dose of an allosteric mTOR inhibitor, e.g., RAD001, can be administered to dimerize an FKBP-FRB based switch.

In an instance, the switch is an FKBP-FRB based switch and the dimerization molecule is RAD001.

In an instance, 0.1 to 20, 0.5 to 10, 2.5 to 7.5, 3 to 6, or about 5, mgs of RAD001 per week, e.g., delivered once per week, is administered.

In an instance, 0.3 to 60, 1.5 to 30, 7.5 to 22.5, 9 to 18, or about 15 mgs of RAD001 in a sustained release formuation, per week, e.g., delivered once per week,is administered.

In an instance, 0.005 to 1.5, 0.01 to 1.5, 0.1 to 1.5, 0.2 to 1.5, 0.3 to 1.5,0.4 to 1.5, 0.5 to 1.5, 0.6 to 1.5, 0.7 to 1.5, 0.8 to 1.5, 1.0 to 1.5, 0.3 to 0.6, or about 0.5 mgs of RAD001 per day, e.g., delivered once once per day, is administered.

In an instance, 0.015 to 4.5, 0.03 to 4.5, 0.3 to 4.5, 0.6 to 4.5, 0.9 to 4.5, 1.2 to 4.5, 1.5 to 4.5, 1.8 to 4.5, 2.1 to 4.5, 2.4 to 4.5, 3.0to 4.5, 0.9 to 1.8,or about 1.5 mgs of RAD001 in a sustained release formulation, per day, e.g., delivered once once per day, is administered.

In an instance, 0.1 to 30, 0.2 to 30, 2 to 30, 4 to 30, 6 to 30, 8 to 30, 10 to 30, 1.2 to 30, 14 to 30, 16 to 30, 20 to 30, 6 to 12, or about 10 mgs of RAD001 in a sustained release formulation, per week, e.g., delivered once once per week, is administered.

In an instance, the dimerization switch comprises:
a switch domain comprising a rapamycin, or rapamycin analog, binding sequence from FKBP, and a switch domain comprising a rapamycin, or rapamycin analog, binding sequence from FRB, e.g., a sequence comprising a lysine at residue 2098.

In an instance, the dimerization switch comprises:
a switch domain comprising a rapamycin analog binding sequence from FKBP, and a switch domain comprising a rapamycin analog binding sequence from FRB, e.g., a sequence comprising a lysine at residue 2098.

In an instance, the dimerization switch comprises:
a switch domain comprising a AP21967 binding sequence from FKBP, and a switch domain comprising a AP21967 binding sequence from FRB, e.g., a sequence comprising a lysine at residue 2098.

In an instance:
the first switch domain comprises,
   a rapamycin, or rapamycin analog, binding sequence from FKBP;
   a rapamycin analog binding sequence from FKBP; or
      an AP21967 binding sequence from FKBP; and,
the second switch domain comprises,
   a rapamycin, or rapamycin analog, binding sequence from FRB;
   a rapamycin analog binding sequence from FRB; or
   an AP21967 binding sequence from FRB, e.g., a sequence comprising a lysine at residue 2098.

In an instance:
the first switch domain comprises,
   a rapamycin, or rapamycin analog, binding sequence from FRB;
   a rapamycin analog binding sequence from FRB; or
   an AP21967 binding sequence from FRB, e.g., a sequence comprising a lysine at residue 2098; and,
the second switch domain comprises,
   a rapamycin, or rapamycin analog, binding sequence from FKBP;
   a rapamycin analog binding sequence from FKBP; or
   an AP21967 binding sequence from FKBP.

In an instance:
the first switch domain comprises an AP21967 binding sequence from FKBP; and,
the second switch domain comprises an AP21967 binding sequence from FRB, e.g., a sequence comprising a lysine at residue 2098.

In an instance the first switch domain comprises an AP21967 binding sequence from FRB, e.g., a sequence comprising a lysine at residue 2098; and,
the second switch domain comprises an AP21967 binding sequence from FKBP.

In an instance, the dimerization molecule is a rapamycin analogue, e.g., AP21967.

In an instance, the dimerization switch comprises a GyrB-GyrB based switch.

In an instance, the dimerization switch comprises:
a switch domain comprising a coumermycin binding sequence having at least 80, 85, 90, 95, 98, or 99 % identity with the 24 K Da amino terminal sub-domain of GyrB.

In an instance, the dimerization switch comprises:
a switch domain comprising a coumermycin binding sequence that differs by no more than 10, 9, 8, 7, 6, 5, 4, 3, 2, or 1 amino acid residues from the corresponding sequence of 24 K Da amino terminal sub-domain of GyrB.

In an instance, the dimerization switch comprises:
a switch domain comprising a coumermycin binding sequence from the 24 K Da amino terminal sub-domain of GyrB.

In an instance, the dimerization switch comprises:
the 24 K Da amino terminal sub-domain of GyrB.

In an instance, the dimerization molecule is a coumermycin.

In an instance, the dimerization switch comprises a GAI-GID1 based switch.

In an instance, the dimerization switch comprises:
a GID1 switch domain comprising a gibberellin, or gibberellin analog, e.g., GA₃, binding sequence having at least 80, 85, 90, 95, 98, or 99 % identity with GID1, and a switch domain comprising a GAI switch domain having at least 80, 85, 90, 95, 98, or 99 % identity with GAI.

In an instance, the dimerization switch comprises:
a GID1 switch domain comprising a gibberellin, or gibberellin analog, e.g., GA₃, binding sequence that differs by no more than 10, 9, 8, 7, 6, 5, 4, 3, 2, or 1 amino acid residues from the corresponding sequence of GID1, and a GAI switch domain that differs by no more than 10, 9, 8, 7, 6, 5, 4, 3, 2, or 1 amino acid residues from the corresponding sequence of GAI.

In an instance:
the first switch domain comprises a GID1 switch domain; and,
the second switch domain comprises a GAI switch domain.

In an instance:
the first switch domain comprises a GAI switch domain; and,
the second switch domain comprises a GID1 switch domain.
In an instance, the dimerization molecule is GA₃-AM.

In an instance, the dimerization molecule is GA₃.

In an instance, the dimerization molecule is a small molecule, e.g., is other than a polypeptide.

In an instance, the dimerization molecule is a polypeptide, e.g., a polypeptide, e.g., an antibody molecule, or a non-antibody scaffold, e.g., a fribronectin or adnectin, having specific affinity for one or both of the first and second switch domains.

In an instance, the dimerization molecule, e.g. a polypeptide, is an antibody molecule.

In an instance, the dimerization switch comprises a Halotag/SNAP-tag based switch.

In an instance, the dimerization switch comprises:
a Halotag switch domain comprising having at least 80, 85, 90, 95, 98, or 99 % identity with SEQ ID NO 14, and a SNAP-tag switch domain having at least 80, 85, 90, 95, 98, or 99 % identity with SEQ ID NO 15.

In an instance, the dimerization switch comprises:
a Halotag switch domain comprising that differs by no more than 10, 9, 8, 7, 6, 5, 4, 3, 2, or 1 amino acid residues from SEQ ID NO 14, and a SNAP-tag switch domain that differs by no more than 10, 9, 8, 7, 6, 5, 4, 3, 2, or 1 amino acid residues from SEQ ID NO: 15.

In an instance:
the first switch domain comprises a Halotag switch domain; and,
the second switch domain comprises a SNAP-tag switch domain.

In an instance:
the first switch domain comprises a SNAP-tag switch domain; and,
the second switch domain comprises a Halotag switch domain.

In an instance, the dimerization molecule comprises structure 5.

In an instance the dimerization molecule comprises three or more domains, e.g., protein tags, that bind a switch domain, e.g., a polypeptide, e.g., an antibody molecule or non-antibody scaffold, having affinity for the domain.

In an instance, the dimerization molecule is a non-covalent dimerization molecule.

In an instance, the dimerization molecule is covalent dimerization molecule.

In an instance, the dimerization switch, e.g., a homodimerization switch, e.g., an extracellular homodimerization switch, comprises switch domains that comprise tag molecules, e.g., a c-myc peptide tag, flag peptide tag, HA peptide tag or V5 peptide tag, and the dimerization switch comprises polypeptides with affinity for the switch domains, e.g., antibody molecules and non-antibody scaffold.

In an instance, the RCAR further comprises a second order dimerization switch.

In an instance the dimerization molecule has a valency of greater than two, e.g., it is multi-valent, and binds, and thus clusters or dimerizes, more than two switch domains.

As is discussed herein, instances of an RCAR can include a member, e.g., an inhibitory extracellular domain member, comprising an intracellular signaling domain, e.g., a costimulatory signaling domain. While not wishing to be bound by theory, it is believed that the presence of such a domain promotes persistence of the member in a cell without significant activation in the absence of dimerization switch mediated association of members of the RCAR. Instances of such members are described in the section following immediately hereafter.

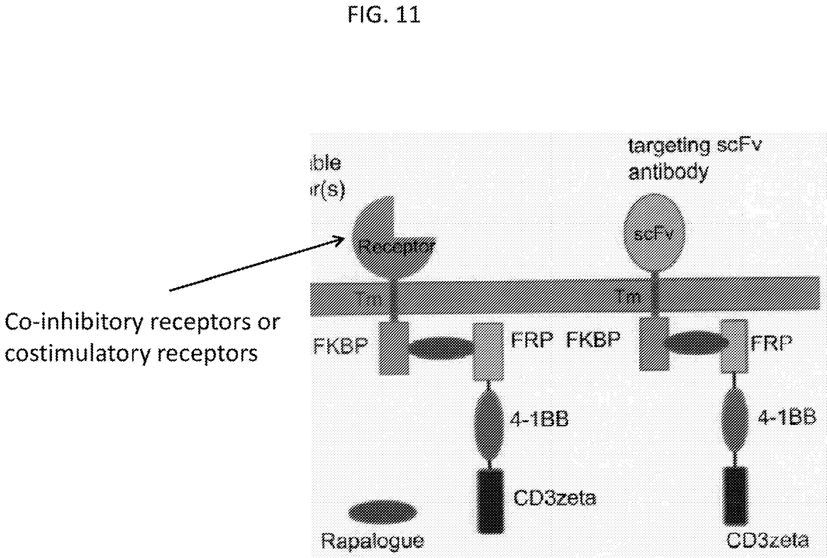

In an instance, the RCAR comprises:
a) an inhibitory extracellular domain member comprising,
   an inhibitory extracellular domain,
   a transmembrane region,
   an intracellular signaling domain, e.g., a costimulatory signaling domain, e.g., selected from Table 2, e.g., a 4-1BB domain, and
   a switch domain;
b) an intracellular signaling member comprising,
   an intracellular signaling domain, e.g., a primary intracellular signaling domain, e.g., selected from Table 1, e.g., a CD3zeta domain, and
   a switch domain; and optionally,
c) an antigen binding member comprising,
   an antigen binding domain,
   a membrane anchoring domain or a transmembrane domain, and
   optionally, a costimulatory signaling domain, e.g.,
      selected from Table 2, e.g., a 4-1BB domain
   See, e.g., Figure 11.

In an instance, the order of elements on the inhibitory extracellular domain member is as follows, with beginning with the amino terminus:
inhibitory extracellular domain /transmembrane domain/intracellular signaling domain, e.g., a costimulatory signaling domain, e.g., selected from Table 2, e.g., a 4-1BB domain /switch domain; or
inhibitory extracellular domain /transmembrane domain /switch domain/ intracellular signaling domain, e.g., a costimulatory signaling domain, e.g., selected from Table 2, e.g., a 4-1BB domain.

In an instance, the order of elements on the intracellular signaling member is as follows, beginning with the amino terminus:
switch domain/ intracellular signaling domain, e.g., a primary intracellular signaling domain, e.g., selected from Table 1, e.g., a CD3zeta domain; or
intracellular signaling domain, e.g., a primary intracellular signaling domain, e.g., selected from Table 1, e.g., a CD3zeta domain/ switch domain.

In an instance, the order of elements on the inhibitory extracellular domain member is as follows, beginning with the carboxy terminus:
inhibitory extracellular domain /transmembrane domain/intracellular signaling domain, e.g., a costimulatory signaling domain, e.g., selected from Table 2, e.g., a 4-1BB domain /switch domain; or
inhibitory extracellular domain /transmembrane domain /switch domain/ intracellular signaling domain, e.g., a costimulatory signaling domain, e.g., selected from Table 2, e.g., a 4-1BB domain.

In an instance, the order of elements on the intracellular signaling member is as follows, beginning with the carboxy terminus:
switch domain/ intracellular signaling domain, e.g., a primary intracellular signaling domain, e.g., selected from Table 1, e.g., a CD3zeta domain; or
intracellular signaling domain, e.g., a primary intracellular signaling domain, e.g., selected from Table 1, e.g., a CD3zeta domain/ switch domain.

In an instance, the first and second switch domains form a FKBP-FRB based switch.

In an instance, the one of the first and second dimerization switches comprises:
a switch domain comprising rapamycin or a rapamycin analog binding sequence having at least 80, 85, 90, 95, 98, or 99 % identity with FKBP, and the other comprises a switch domain comprising a rapamycin or rapamycin analog binding sequence binding sequence having at least 80, 85, 90, 95, 98, or 99 % identity with FRB.

In an instance, the FKBP-FRB based switch comprises a switch domain comprising a FRB binding fragment or analog of FKBP and a switch domain comprising an FKBP binding fragment or analog of FRB, and the FKBP binding fragment or analog of FRB comprises one or more mutations which enhances the formation of a complex between an FKBP switch domain, an FRB switch domain, and the dimerization molecule, or a mutation described in the section herein entitled **MODIFIED FKBP/FRB-BASED DIMERIZATION SWITCHES.** E.g., the FKBP binding fragment or analog of FRB comprises: an E2032 mutation, e.g., an E2032I mutation or E2032L mutation; a T2098 mutation, e.g., a T2098L mutation; or an E2032 and a T2098 mutation, e.g., an E2032I and a T2098L or an E2032L and a T2098L mutation.

In an instance, wherein the switch is an FKBP-FRB based switch, the dimerization molecule is an mTOR inhibitor, e.g., an allosteric mTOR inhibitor, e.g., rapamycin or a rapalog, e.g., RAD001.

In an instance, any of the dosing regimes or formulations of an allosteric mTOR inhibitor, e.g., RAD001, described in the section here for a low, immune enhancing, dose of an allosteric mTOR inhibitor, e.g., RAD001, can be administered to dimerize an FKBP-FRB based switch.

In an instance, the switch is an FKBP-FRB based switch and the dimerization molecule is RAD001.

In an embodiment, 0.1 to 20, 0.5 to 10, 2.5 to 7.5, 3 to 6, or about 5, mgs of RAD001 per week, e.g., delivered once per week, is administered.

In an instance, 0.3 to 60, 1.5 to 30, 7.5 to 22.5, 9 to 18, or about 15 mgs of RAD001 in a sustained release formuation, per week, e.g., delivered once per week,is administered.

In an instance, 0.005 to 1.5, 0.01 to 1.5, 0.1 to 1.5, 0.2 to 1.5, 0.3 to 1.5,0.4 to 1.5, 0.5 to 1.5, 0.6 to 1.5, 0.7 to 1.5, 0.8 to 1.5,1.0 to 1.5, 0.3 to 0.6, or about 0.5 mgs of RAD001 per day, e.g., delivered once once per day, is administered.

In an instance, 0.015 to 4.5, 0.03 to 4.5, 0.3 to 4.5, 0.6 to 4.5, 0.9 to 4.5, 1.2 to 4.5, 1.5 to 4.5, 1.8 to 4.5, 2.1 to 4.5, 2.4 to 4.5,3.0 to 4.5, 0.9 to 1.8, or about 1.5 mgs of RAD001 in a sustained release formulation, per day, e.g., delivered once once per day, is administered.

In an instance, 0.1 to 30, 0.2 to 30, 2 to 30, 4 to 30, 6 to 30, 8 to 30, 10 to 30, 1.2 to 30, 14 to 30, 16 to 30, 20 to 30, 6 to 12, or about 10 mgs of RAD001 in a sustained release formulation, per week, e.g., delivered once once per week, is administered.

In an instance, the dimerization switch comprises a GyrB-GyrB based switch, e.g., an GyrB-GyrB based switch described herein, e.g., an GyrB-GyrB based switch as described herein, e.g., in the Dimerization Switch Module.

In an instance, the dimerization switch comprises a GAI-GID1 based switch, e.g., an GAI-GID1 based switch described herein, e.g., an GAI-GID1 based switch as described herein, e.g., in the Dimerization Switch Module.

In an instance, the dimerization switch comprises a Halotag/SNAP-tag based switch, e.g., a Halotag/SNAP-tag based switch described herein, e.g., a Halotag/SNAP-tag based switch as described herein, e.g., in the Dimerization Switch Module.

In an instance, the RCAR comprises:
a) an intracellular signaling member comprising, beginning with the amino terminus:
   a CD3zeta domain, and
   a first switch domain; and
b) an inhibitory extracellular domain member comprising, beginning with the amino terminus:
   an inhibitory extracellular domain,
   a transmembrane domain,
   a 4-1BB domain, and
   a second switch domain,
   wherein the first and second switch domains form a FKBP-FRB based switch.

In an instance, the RCAR is associated with, e.g., is provided in the same cell with:
an inhibitor of an inhibitory molecule, e.g., an inhibitor of a inhibitory molecule of Table 3.

In an instance, the RCAR is associated with, e.g., is provided in the same cell with, an shRNA that targets a inhibitory molecule, e.g. a coinhibitory molecule from Table 3.

In an instance the RCAR comprises a shRNA that targets PD1.

In an instance, the antigen binding domain binds to a target antigen on a cancer cell but does not activate the RCARX cell, e.g., a RCART cell, until a dimerization molecule is administered.

In an instance, the antigen binding domain binds to a target antigen on a target cell, e.g., a cancer cell, but does not promote an immune effector response, e.g., a T cell activation, until the dimerization molecule, e.g., a heterodimerization molecule or homodimerization molecule, is administered.

In an instance:
the antigen binding member comprises
an antigen binding domain;
a transmembrane domain; and
an intracellular signaling domain, e.g., a costimulatory signaling domain, e.g., a costimulatory signaling domain from Tasble 2, e.g., a 4-1BB domain..
See, e.g., Fig. 11.

In an instance:
the antigen binding member does not comprise a switch domain that forms a dimerization switch with the switch on the inhibitory counter ligand binding member or the switch on the intracellular signaling member.

In an instance the inhibitory counter ligand binding domain is selected from Table 4.

As discussed in the above instance, the antigen binding member comprises an intracellular signaling domain, further embodiments of which are discussed immediately below.

In an instance, the intracellular signaling domain of antigen binding member is a primary intracellular signaling domain, selected, e.g., from Table 1.

In an instance, the primary intracellular signaling domain of antigen binding member comprises a CD3zeta domain.

In an instance, the intracellular signaling domain of antigen binding member is a costimulatory signaling domain, e.g., selected from the list in Table 2.

In an instance, the costimulatory signaling domain comprises a 4-1BB domain.

In an instance, the antigen binding member comprises a second intracellular signaling domain.

In an instance, the second intracellular signaling domain of antigen binding member is a primary intracellular signaling domain, e.g., selected from the list in Table 1.

In an instance, the second intracellular signaling domain of antigen binding member is a costimulatory signaling domain, e.g., selected from the list in Table 2.

In an instance, the first and second intracellular signaling domains of antigen binding member comprise:
a 4-1BB domain and a CD3zeta domain; or
a CD28 domain and a 4-1BB domain.

In an instance, the antigen binding member comprises a third intracellular signaling domain.

In an instance, the third intracellular signaling domain of antigen binding member is a primary intracellular signaling domain, e.g., selected from the list in Table 1.

In an instance, the third intracellular signaling domain of antigen binding member is a costimulatory signaling domain, e.g., selected from the list in Table 2.

In an instance, one of the first, second and third intracellular signaling domain of antigen binding member is a primary intracellular signaling domain, e.g., selected from the list in Table 1, and the other two are costimulatory signaling domains, e.g., selected from the list in Table 2.

In an instance, two of the first, second and third intracellular signaling domains of antigen binding member are primary intracellular signaling domains, e.g., selected from the list in Table 1, and the other is a costimulatory signaling domain, e.g., selected from the list in Table 2.

In an instance, each of the first, second and third intracellular signaling domains of antigen binding member is a primary intracellular signaling domain, e.g., selected from the list in Table 1.

In an instance, each of the first, second and third intracellular signaling domains of antigen binding member is a costimulatory signaling domain, e.g., selected from the list in Table 2.

In an instance, the first, second, and third intracellular signaling domains of antigen binding member comprise: a CD28 domain; a 4-1BB domain, and a CD3zeta domain.

In an instance, antigen binding member comprises a fourth intracellular signaling domain.

In an instance, the fourth intracellular signaling domain of antigen binding member is a primary intracellular signaling domain, e.g., selected from the list in Table 1.

In an instance, the fourth intracellular signaling domain of antigen binding member is a costimulatory signaling domain, e.g., selected from the list in Table 2.

In an instance, one of the first, second ,third and fourth intracellular signaling domain of antigen binding member is a primary intracellular signaling domain, e.g., selected from the list in Table 1 and the other three are costimulatory signaling domains, e.g., selected from the list in Table 2.

In an instance, two of the first, second, third, and fourth intracellular signaling domains of antigen binding member are primary intracellular signaling domains, e.g., selected from the list in Table 1, and the other two are costimulatory signaling domain, e.g., selected from the list in Table 2.

In an instance, three of the first, second, third, and fourth intracellular signaling domains of antigen binding members are selected from the list in Table 1, and the other is a costimulatory signaling domain, e.g., selected from the list in Table 2.

In an instance, each of the first, second, third, and fourth intracellular signaling domains of antigen binding member is a primary intracellular signaling domain, e.g., selected from the list in Table 1.

In an instance, each of the first, second, third, and fourth intracellular signaling domains of antigen binding member is a costimulatory signaling domain, e.g., selected from the list in Table 2.

In an instance, the two or more costimulatory domains can be the same costimulatory signaling domain, e.g., selected from the list in Table 2, or different costimulatory signaling domains, e.g., selected from the list in Table 2.

In an instance, the order of switch domain and the intracellular signaling domain (isd) or domains of antigen binding member is as follows, beginning with amino :
switch/isd;
switch/isd1/isd2;
isd1/switch/isd2;
isd1/isd2/switch;
switch/isd1/isd2/isd3;
isd1/isd2/isd3/switch;
isd1/switch/isd2/isd3; and
isd 1/ isd2/ switch/ isd3.

In an instance, the order of switch domain and the intracellular signaling domain (isd) or domains of antigen binding member is as follows, beginning with carboxy terminus:
switch/isd;
switch/isd1/isd2;
isd1/switch/isd2;
isd1/isd2/switch;
switch/isd1/isd2/isd3 ;
isd1/isd2/isd3/switch;
isd1/switch/isd2/isd3; and
isd1/isd2/switch/isd3.

In an instance:
the antigen binding member comprises
an antigen binding domain;
a switch domain; and
a transmembrane domain.

In an instance:
the intracellular binding member switch domain forms a heterodimerization switch with one or both of:
the inhibitory extracellular domain member switch, and
the antigen binding domain switch.

As discussed above, the switched antigen binding member comprises an intracellular signaling domain, further instances of which are discussed immediately below.

In an instance,the intracellular signaling domain of switched antigen binding member is a primary intracellular signaling domain, selected, e.g., from the list in Table 1.

In an instance, the primary intracellular signaling domain of switched antigen binding member comprises a CD3zeta domain.

In an instance, the intracellular signaling domain of switched antigen binding member is a costimulatory signaling domain, e.g., selected from the list in Table 2.

In an instance, the costimulatory signaling domain comprises a 4-1BB domain.

In an instance, the switched antigen binding member comprises a second intracellular signaling domain.

In an instance, the second intracellular signaling domain of switched antigen binding member is a primary intracellular signaling domain, e.g., selected from the list in Table 1.

In an instance, the second intracellular signaling domain of switched antigen binding member is a costimulatory signaling domain, e.g., selected from the list in Table 2.

In an instance, the first and second intracellular signaling domains of switched antigen binding member comprise:
a 4-1BB domain and a CD3zeta domain; or
a CD28 domain and a 4-1BB domain.

In an instance, the antigen binding member comprises a third intracellular signaling domain.

In an instance, the third intracellular signaling domain of switched antigen binding member is a primary intracellular signaling domain, e.g., selected from the list in Table 1.

In an instance, the third intracellular signaling domain of switched antigen binding member is a costimulatory signaling domain, e.g., selected from the list in Table 2.

In an instance, one of the first, second and third intracellular signaling domain of switched antigen binding member is a primary intracellular signaling domain, e.g., selected from the list in Table 1, and the other two are costimulatory signaling domains, e.g., selected from the list in Table 2.

In an instance, two of the first, second and third intracellular signaling domains of switched antigen binding member are primary intracellular signaling domains, e.g., selected from the list in Table 1, and the other is a costimulatory signaling domain, e.g., selected from the list in Table 2.

In an instance, each of the first, second and third intracellular signaling domains of switched antigen binding member is a primary intracellular signaling domain, e.g., selected from the list in Table 1.

In an instance, each of the first, second and third intracellular signaling domains of switched antigen binding member is a costimulatory signaling domain, e.g., selected from the list in Table 2.

In an instance, the first, second, and third intracellular signaling domains of switched antigen binding member comprise: a CD28 domain; a 4-1BB domain, and a CD3zeta domain.

In an instance, switched antigen binding member comprises a fourth intracellular signaling domain.

In an instance, the fourth intracellular signaling domain of switched antigen binding member is a primary intracellular signaling domain, e.g., selected from the list in Table 1.

In an instance, the fourth intracellular signaling domain of switched antigen binding member is a costimulatory signaling domain, e.g., selected from the list in Table 2.

In an instance, one of the first, second ,third and fourth intracellular signaling domain of switched antigen binding member is a primary intracellular signaling domain, e.g., selected from the list in Table 1 and the other three are costimulatory signaling domains, e.g., selected from the list in Table 2.

In an instance, two of the first, second, third, and fourth intracellular signaling domains of switched antigen binding member are primary intracellular signaling domains, e.g., selected from the list in Table 1, and the other two are costimulatory signaling domain, e.g., selected from the list in Table 2.

In an instance, three of the first, second, third, and fourth intracellular signaling domains of switched antigen binding members are selected from the list in Table 1, and the other is a costimulatory signaling domain, e.g., selected from the list in Table 2.

In an instance, each of the first, second, third, and fourth intracellular signaling domains of switched antigen binding member is a primary intracellular signaling domain, e.g., selected from the list in Table 1.

In an instance, each of the first, second, third, and fourth intracellular signaling domains of switched antigen binding member is a costimulatory signaling domain, e.g., selected from the list in Table 2.

In an instance, the two or more costimulatory domains can be the same costimulatory signaling domain, e.g., selected from the list in Table 2, or different costimulatory signaling domains, e.g., selected from the list in Table 2.

In an instance, the order of switch domain and the intracellular signaling domain (isd) or domains of switched antigen binding member is as follows, beginning with amino terminus:
switch/isd;
switch/isd1/isd2;
isd1/switch/isd2;
isd1/isd2/switch;
switch/isd1/isd2/isd3 ;
isd1/isd2/isd3/switch;
isd1/switch/isd2/isd3; and
isd1/isd2/switch/isd3.

In an instance, the order of switch domain and the intracellular signaling domain (isd) or domains of switched antigen binding member is as follows, the beginning with carboxy terminus:
switch/isd;
switch/isd1/isd2;
isd1/switch/isd2;
isd1/isd2/switch;
switch/isd1/isd2/isd3 ;
isd1/isd2/isd3/switch;
isd1/switch/isd2/isd3; and
isd1/isd2/switch/isd3.

In a switched antigen binding domain embodiment the RCAR is associated with, e.g., is provided in the same cell with:
an inhibitor of an inhibitory molecule, e.g., an inhibitor of a inhibitory molecule of Table 3.

In a switched antigen binding domain instance the RCAR is associated with, e.g., is provided in the same cell with an shRNA that targets a inhibitory molecule, e.g. a coinhibitory molecule from Table 3.

In a switched antigen binding domain instance the RCAR further comprises a shRNA that targets PD1.

In a switched antigen binding domain instance the antigen binding domain binds to a target antigen on a cancer cell but does not activate the RCARX cell, e.g., a RCART cell, until a dimerization molecule is administered.

In a switched antigen binding domain instance the antigen binding domain binds to a target antigen on a target cell, e.g., a cancer cell, but does not promote an immune effector response, e.g., a T cell activation, until the dimerization molecule, e.g., a heterodimerization molecule or homodimerization molecule, is administered.

RCARs disclosed herein can include, e.g., in place of an scFv-based antigen binding domain, an extracelluar domain of an a costimulatory ECD domain. While not wising to be bound by theory, it is believed that engagement of the ECD with its counter ligand activates the immune response via the RCAR. This is discussed immediately below.

In a fourth aspect, the disclosure features, a RCAR, e.g., an isolated, RCAR comprising:
a) a costimulatory ECD member comprising
   a costimulatory ECD domain;
   a transmembrane region, and
   a switch domain;
b) an intracellular signaling member comprising
   an intracellular signaling domain, e.g., a primary intracellular signaling domain, and
   a switch domain; and optionally,
c) an antigen binding member comprising
   an antigen binding domain;
   a transmembrane domain; and
   a switch domain.
   See, e.g., Fig. 11.

In an instance:
the intracellular binding member switch domain forms a heterodimerization switch with one or both of:
the costimulatory ECD member switch, and
the antigen binding member switch.

In an instance, the costimulatory ECD domain is selected from Table 5.

In an instance the intracellular signaling domain is a primary intracellular signaling domain, selected, e.g., from Table 1.

In an instance, the primary intracellular signaling domain comprises a CD3zeta domain.

In an instance, the intracellular signaling domain is a costimulatory signaling domain, e.g., selected from the list in Table 2.

In an instance, the costimulatory signaling domain comprises a 4-1BB domain.

In an instance, the RCAR comprises a second intracellular signaling domain.

In an instance, the second intracellular signaling domain is a primary intracellular signaling domain, e.g., selected from the list in Table 1.

In an instance, the second intracellular signaling domain is a costimulatory signaling domain, e.g., selected from the list in Table 2.

In an instance, the first and second intracellular signaling domains comprise:
a 4-1BB domain and a CD3zeta domain; or
a CD28 domain and a 4-1BB domain.

In an instance, the RCAR comprises a third intracellular signaling domain.

In an instance, the third intracellular signaling domain is a primary intracellular signaling domain, e.g., selected from the list in Table 1.

In an instance, the third intracellular signaling domain is a costimulatory signaling domain, e.g., selected from the list in Table 2.

In an instance, one of the first, second and third intracellular signaling domain is a primary intracellular signaling domain, e.g., selected from the list in Table 1, and the other two are costimulatory signaling domains, e.g., selected from the list in Table 2.

In an instance, two of the first, second and third intracellular signaling domains are primary intracellular signaling domains, e.g., selected from the list in Table 1, and the other is a costimulatory signaling domain, e.g., selected from the list in Table 2.

In an instance, each of the first, second and third intracellular signaling domains is a primary intracellular signaling domain, e.g., selected from the list in Table 1.

In an instance, each of the first, second, and third intracellular signaling domains is a costimulatory signaling domain, e.g., selected from the list in Table 2.

In an instance, the first, second, and third intracellular signaling domains comprise: a CD28 domain; a 4-1BB domain, and a CD3zeta domain.

In an instance, the RCAR comprises a fourth intracellular signaling domain.

In an instance, the fourth intracellular signaling domain is a primary intracellular signaling domain, e.g., selected from the list in Table 1.

In an instance the fourth intracellular signaling domain is a costimulatory signaling domain, e.g., selected from the list in Table 2.

In an instance, one of the first, second, third and fourth intracellular signaling domain is a primary intracellular signaling domain, e.g., selected from the list in Table 1 and the other three are costimulatory signaling domains, e.g., selected from the list in Table 2.

In an instance, two of the first, second, third, and fourth intracellular signaling domains are primary intracellular signaling domains, e.g., selected from the list in Table 1, and the other two are costimulatory signaling domain, e.g., selected from the list in Table 2.

In an instance, three of the first, second, third, and fourth intracellular signaling domains are primary intracellular signaling domains, e.g., selected from the list in Table 1, and the other is a costimulatory signaling domain, e.g., selected from the list in Table 2.

In an instance, each of the first, second, third, and fourth intracellular signaling domains is a primary intracellular signaling domain, e.g., selected from the list in Table 1.

In an instance, each of the first, second, third, and fourth intracellular signaling domains is a costimulatory signaling domain, e.g., selected from the list in Table 2.

In an instance, the two or more costimulatory domains can be the same costimulatory signaling domain, e.g., selected from the list in Table 2, or different costimulatory signaling domains, e.g., selected from the list in Table 2.

In an instance, the order of switch domain and the intracellular signaling domain (isd) or domains is as follows, beginning with the amino terminus:
switch/isd;
switch/isd1/isd2;
isd1/switch/isd2;
isd1/isd2/switch;
switch/isd1/isd2/isd3 ;
isd1/isd2/isd3/switch;
isd1/switch/isd2/isd3; and
isd1/isd2/switch/isd3.

In an instance, the order of switch domain and the intracellular signaling domain (isd) or domains is as follows, beginning with the carboxy terminus:
switch/isd;
switch/isd1/isd2;
isd1/switch/isd2;
isd1/isd2/switch;
switch/isd1/isd2/isd3;
isd1/isd2/isd3/switch;
isd1/switch/isd2/isd3; and
isd 1/ isd2/ switch/ isd3.

In an instance, the switch domains are components of a heterodimerization switch.

In an instance, the switch domains are components of a homodimerization switch.

In an instance, the dimerization switch is intracellular.

In an instance, the dimerization switch is extracellular.

In an instance, the transmembrane domain disposed on the antigen binding member and the dimerization switch, e.g., a heterodimerization switch or homodimerization switch, is intracellular.

In an instance, where the transmembrane domain disposed on the intracellular signaling member and the dimerization switch, e.g., heterodimerization or homodimerization switch, is extracellular.

In an instance, the dimerization switch comprises a FKBP-FRB based switch.

In an instance, the dimerization switch comprises:
a switch domain comprising a rapamycin analog binding sequence having at least 80, 85, 90, 95, 98, or 99 % identity with FKBP, and a switch domain comprising a rapamycin analog binding sequence binding sequence having at least 80, 85, 90,95, 98, or 99 % identity with FRB.

In an instance, the FKBP-FRB based switch comprises a switch domain comprising a FRB binding fragment or analog of FKBP and a switch domain comprising an FKBP binding fragment or analog of FRB, and the FKBP binding fragment or analog of FRB comprises one or more mutations which enhances the formation of a complex between an FKBP switch domain, an FRB switch domain, and the dimerization molecule, or a mutation described in the section herein entitled **MODIFIED FKBP/FRB-BASEDDIMERIZATION SWITCHES.** E.g., the FKBP binding fragment or analog of FRB comprises: an E2032 mutation, e.g., an E2032I mutation or E2032L mutation; a T2098 mutation, e.g., a T2098L mutation; or an E2032 and a T2098 mutation, e.g., an E2032I and a T2098L or an E2032L and a T2098L mutation.

In an instance, the dimerization switch comprises:
a switch domain comprising a rapamycin analog binding sequence that differs by no more than 10, 9, 8, 7, 6, 5, 4, 3, 2, or 1 amino acid residues from the corresponding sequence of FKBP, and a switch domain comprising a rapamycin analog binding sequence that differs by no more than 10, 9, 8, 7, 6, 5, 4, 3, 2, or 1 amino acid residues from the corresponding sequence of FRB.

In an instance, wherein the switch is an FKBP-FRB based switch, the dimerization molecule is an mTOR inhibitor, e.g., an allosteric mTOR inhibitor, e.g., rapamycin or a rapalog, e.g., RAD001.

In an instance, any of the dosing regimes or formulations of an allosteric mTOR inhibitor, e.g., RAD001, described in the section here for a low, immune enhancing, dose of an allosteric mTOR inhibitor, e.g., RAD001, can be administered to dimerize an FKBP-FRB based switch.

In an instance, the switch is an FKBP-FRB based switch and the dimerization molecule is RAD001.

In an instance, 0.1 to 20, 0.5 to 10, 2.5 to 7.5, 3 to 6, or about 5, mgs of RAD001 per week, e.g., delivered once per week, is administered.

In an instance, 0.3 to 60, 1.5 to 30, 7.5 to 22.5, 9 to 18, or about 15 mgs of RAD001 in a sustained release formuation, per week, e.g., delivered once per week,is administered.

In an instance, 0.005 to 1.5, 0.01 to 1.5, 0.1 to 1.5, 0.2 to 1.5, 0.3 to 1.5,0.4 to 1.5, 0.5 to 1.5, 0.6 to 1.5, 0.7 to 1.5, 0.8 to 1.5, 1.0 to 1.5, 0.3 to 0.6, or about 0.5 mgs of RAD001 per day, e.g., delivered once once per day, is administered.

In an instance, 0.015 to 4.5, 0.03 to 4.5, 0.3 to 4.5, 0.6 to 4.5, 0.9 to 4.5, 1.2 to 4.5, 1.5 to 4.5, 1.8 to 4.5, 2.1 to 4.5, 2.4 to 4.5, 3.0to 4.5, 0.9 to 1.8, or about 1.5 mgs of RAD001 in a sustained release formulation, per day, e.g., delivered once once per day, is administered.

In an instance, 0.1 to 30, 0.2 to 30, 2 to 30, 4 to 30, 6 to 30, 8 to 30, 10 to 30, 1.2 to 30, 14 to 30, 16 to 30, 20 to 30, 6 to 12, or about 10 mgs of RAD001 in a sustained release formulation, per week, e.g., delivered once once per week, is administered.

In an instance, the dimerization switch comprises:
a switch domain comprising a rapamycin, or rapamycin analog, binding sequence from FKBP, and a switch domain comprising a rapamycin, or rapamycin analog, binding sequence from FRB, e.g., a sequence comprising a lysine at residue 2098.

In an instance, the dimerization switch comprises:
a switch domain comprising a rapamycin analog binding sequence from FKBP, and a switch domain comprising a rapamycin analog binding sequence from FRB, e.g., a sequence comprising a lysine at residue 2098.

In an instance, the dimerization switch comprises:
a switch domain comprising a AP21967 binding sequence from FKBP, and a switch domain comprising a AP21967 binding sequence from FRB, e.g., a sequence comprising a lysine at residue 2098.

In an instance:
the first switch domain comprises,
   a rapamycin, or rapamycin analog, binding sequence from FKBP;
   a rapamycin analog binding sequence from FKBP; or
      an AP21967 binding sequence from FKBP; and,
the second switch domain comprises,
   a rapamycin, or rapamycin analog, binding sequence from FRB;
   a rapamycin analog binding sequence from FRB; or
   an AP21967 binding sequence from FRB, e.g., a sequence comprising a lysine at residue 2098.

In an instance:
the first switch domain comprises,
   a rapamycin, or rapamycin analog, binding sequence from FRB;
   a rapamycin analog binding sequence from FRB; or
   an AP21967 binding sequence from FRB, e.g., a sequence comprising a lysine at residue 2098; and,
the second switch domain comprises,
   a rapamycin, or rapamycin analog, binding sequence from FKBP;
   a rapamycin analog binding sequence from FKBP; or
      an AP21967 binding sequence from FKBP.

In an instance:
the first switch domain comprises an AP21967 binding sequence from FKBP; and,
the second switch domain comprises an AP21967 binding sequence fromFRB, e.g., a sequence comprising a lysine at residue 2098.

In an instance, the first switch domain comprises an AP21967 binding sequence from FRB, e.g., a sequence comprising a lysine at residue 2098; and,
the second switch domain comprises an AP21967 binding sequence from FKBP.

In an instance, the dimerization molecule is a rapamycin analogue, e.g., AP21967.

In an instance, the dimerization switch comprises a GyrB-GyrB based switch.

In an instance, the dimerization switch comprises:
a switch domain comprising a coumermycin binding sequence having at least 80, 85, 90, 95, 98, or 99 % identity with the 24 K Da amino terminal sub-domain of GyrB.

In an instance, the dimerization switch comprises:
a switch domain comprising a coumermycin binding sequence that differs by no more than 10, 9, 8, 7, 6, 5, 4, 3, 2, or 1 amino acid residues from the corresponding sequence of 24 K Da amino terminal sub-domain of GyrB.

In an instance, the dimerization switch comprises:
a switch domain comprising a coumermycin binding sequence from the 24 K Da amino terminal sub-domain of GyrB.

In an instance, the dimerization switch comprises:
the 24 K Da amino terminal sub-domain of GyrB.

In an instance, the dimerization molecule is a coumermycin.

In an instance, the dimerization switch comprises a GAI-GID1 based switch.

In an instance, the dimerization switch comprises:
a GID1 switch domain comprising a gibberellin, or gibberellin analog, e.g., GA₃, binding sequence having at least 80, 85, 90, 95, 98, or 99 % identity with GID1, and a switch domain comprising a GAI switch domain having at least 80, 85, 90, 95, 98, or 99 % identity with GAI.

In an instance, the dimerization switch comprises:
a GID1 switch domain comprising a gibberellin, or gibberellin analog, e.g., GA₃, binding sequence that differs by no more than 10, 9, 8, 7, 6, 5, 4, 3, 2, or 1 amino acid residues from the corresponding sequence of FKBP, and a GAI switch domain that differs by no more than 10, 9, 8, 7, 6, 5, 4, 3, 2, or 1 amino acid residues from the corresponding sequence of FRB.

In an instance:
the first switch domain comprises a GID1 switch domain; and,
the second switch domain comprises a GAI switch domain.

In an instance:
the first switch domain comprises a GAI switch domain; and,
the second switch domain comprises a GID1 switch domain.

In an instance, the dimerization molecule is GA₃-AM.

In an instance, the dimerization molecule is GA₃.

In an instance, the dimerization molecule is a small molecule, e.g., is other than a polypeptide.

In an instance, the dimerization molecule is a polypeptide, e.g., a polypeptide, e.g., an antibody molecule, or a non-antibody scaffold, e.g., a fribronectin or adnectin, having specific affinity for one or both of the first and second switch domains.

In an instance, the dimerization molecule, e.g. a polypeptide, is an antibody molecule.

In an instance, the dimerization switch comprises a Halotag/SNAP-tag based switch.

In an instance, the dimerization switch comprises:
a Halotag switch domain comprising having at least 80, 85, 90, 95, 98, or 99 % identity with SEQ ID NO: 14, and a SNAP-tag switch domain having at least 80, 85, 90, 95,98, or 99 % identity with SEQ ID NO: 15.

In an instance the dimerization switch comprises:
a Halotag switch domain comprising that differs by no more than 10, 9, 8, 7, 6, 5, 4, 3, 2, or 1 amino acid residues from SEQ ID NO: 14, and a SNAP-tag switch domain that differs by no more than 10, 9, 8, 7, 6, 5, 4, 3, 2, or 1 amino acid residues from SEQ ID NO: 15.

In an instance:
the first switch domain comprises a Halotag switch domain; and,
the second switch domain comprises a SNAP-tag switch domain.

In an instance:
the first switch domain comprises a SNAP-tag switch domain; and,
the second switch domain comprises a Halotag switch domain.

In an instance the dimerization molecule comprises structure 5.

In an instance the dimerization molecule comprises three or more domains, e.g., protein tags that bind a switch domain, e.g., a polypeptide, e.g., an antibody molecule or non-antibody scaffold, having affinity for the domain.

In an instance, the dimerization molecule is a non-covalent dimerization molecule.

In an instance, the dimerization molecule is covalent dimerization molecule.

In an instance, the dimerization switch, e.g., a homodimerization switch, e.g., an extracellular homodimerization switch, comprises switch domains that comprise tag molecules, e.g., a c-myc peptide tag, flag peptide tag, HA peptide tag or V5 peptide tag, and the dimerization switch comprises polypeptides with affinity for the switch domains, e.g., antibody molecules and non-antibody scaffold.

In an instance, the RCAR further comprises a second order dimerization switch.

In an instance, the dimerization molecule has a valency of greater than two, e.g., it is multi-valent, and binds, and thus clusters or dimerizes, more than two switch domains.

In an instance, the antigen binding member comprises
an antigen binding domain,
a transmembrane domain,
a switch domain, and
a costimulatory signaling domain, e.g., a costimulatory signaling domain from Table 2, e.g., a 4-1BB domain.

In an instance, the RCAR comprises:
a) a costimulatory ECD member comprising
   a costimulatory ECD domain;
   a transmembrane region,
   an intracellular signaling domain, e.g., a costimulatory signaling domain, e.g., selected from Table 2, e.g., a 4-1BB domain, and
   a switch domain;
b) an intracellular signaling member comprising
   an intracellular signaling domain, e.g., a primary intracellular signaling domain, e.g., selected from Table 1, e.g., a CD3zeta domain, and
   a switch domain; and optionally,
c) an antigen binding member comprising
   an antigen binding domain;
   a transmembrane domain;
   a switch domain and,
   optionally, an intracellular signaling domain, e.g., a costimulatory signaling domain, e.g., selected from Table 2, e.g., a 4-1BB domain.

In an instance, the order of elements on the costimulatory ECD member is as follows, beginning with the amino terminus:
a costimulatory ECD domain /transmembrane domain/intracellular signaling domain, e.g., a costimulatory signaling domain, e.g., selected from Table 2, e.g., a 4-1BB domain /switch domain; or
a costimulatory ECD domain /transmembrane domain /switch domain/intracellular signaling domain, e.g., a costimulatory signaling domain, e.g., selected from Table 2, e.g., a 4-1BB domain.

In an instance, the order of elements on the intracellular signaling member is as follows, beginning with the amino terminus :
switch domain/ intracellular signaling domain, e.g., a primary intracellular signaling domain, e.g., selected from Table 1, e.g., a CD3zeta domain; or
intracellular signaling domain, e.g., a primary intracellular signaling domain, e.g., selected from Table 1, e.g., a CD3zeta domain/ switch domain.

In an instance, the order of elements on the a costimulatory ECD member is as follows, beginning with the carboxy terminus:
a costimulatory ECD domain /transmembrane domain/intracellular signaling domain, e.g., a costimulatory signaling domain, e.g., selected from Table 2, e.g., a 4-1BB domain /switch domain; or
a costimulatory ECD domain /transmembrane domain /switch domain/ intracellular signaling domain, e.g., a costimulatory signaling domain, e.g., selected from Table 2, e.g., a 4-1BB domain.

In an instance, the order of elements on the intracellular signaling member is as follows, beginning with the carboxy terminus:
switch domain/ intracellular signaling domain, e.g., a primary intracellular signaling domain, e.g., selected from Table 1, e.g., a CD3zeta domain; or
intracellular signaling domain, e.g., a primary intracellular signaling domain, e.g., selected from Table 1, e.g., a CD3zeta domain/ switch domain.

In an instance, the first and second switch domains form a FKBP-FRB based switch.

In an instance, the one of the first and second dimerization switches comprises: a switch domain comprising rapamycin or a rapamycin analog binding sequence having at least 80, 85, 90, 95, 98, or 99 % identity with FKBP, and the other comprises a switch domain comprising a rapamycin or rapamycin analog binding sequence binding sequence having at least 80, 85, 90, 95, 98, or 99 % identity with FRB.

In an instance, wherein the switch is an FKBP-FRB based switch, the dimerization molecule is an mTOR inhibitor, e.g., an allosteric mTOR inhibitor, e.g., rapamycin or a rapalog, e.g., RAD001.

In an instance, any of the dosing regimes or formulations of an allosteric mTOR inhibitor, e.g., RAD001, described in the section here for a low, immune enhancing, dose of an allosteric mTOR inhibitor, e.g., RAD001, can be administered to dimerize an FKBP-FRB based switch.

In an instance, the switch is an FKBP-FRB based switch and the dimerization molecule is RAD001.

In an embodiment, 0.1 to 20, 0.5 to 10, 2.5 to 7.5, 3 to 6, or about 5, mgs of RAD001 per week, e.g., delivered once per week, is administered.

In an instance, 0.3 to 60, 1.5 to 30, 7.5 to 22.5, 9 to 18, or about 15 mgs of RAD001 in a sustained release formuation, per week, e.g., delivered once per week,is administered.

In an instance, 0.005 to 1.5, 0.01 to 1.5, 0.1 to 1.5, 0.2 to 1.5, 0.3 to 1.5,0.4 to 1.5, 0.5 to 1.5, 0.6 to 1.5, 0.7 to 1.5, 0.8 to 1.5, 1.0 to 1.5, 0.3 to 0.6, or about 0.5 mgs of RAD001 per day, e.g., delivered once once per day, is administered.

In an instance, 0.015 to 4.5, 0.03 to 4.5, 0.3 to 4.5, 0.6 to 4.5, 0.9 to 4.5, 1.2 to 4.5, 1.5 to 4.5, 1.8 to 4.5, 2.1 to 4.5, 2.4 to 4.5, 3.0to 4.5, 0.9 to 1.8, or about 1.5 mgs of RAD001 in a sustained release formulation, per day, e.g., delivered once once per day, is administered.

In an instance, 0.1 to 30, 0.2 to 30, 2 to 30, 4 to 30, 6 to 30, 8 to 30, 10 to 30, 1.2 to 30, 14 to 30, 16 to 30, 20 to 30, 6 to 12, or about 10 mgs of RAD001 in a sustained release formulation, per week, e.g., delivered once once per week, is administered.

In an instance, the dimerization switch comprises a GyrB-GyrB based switch, e.g., an GyrB-GyrB based switch described herein, e.g., an GyrB-GyrB based switch as described herein, e.g., in the Dimerization Switch Module.

In an instance, the dimerization switch comprises a GAI-GID1 based switch, e.g., an GAI-GID1 based switch described herein, e.g., an GAI-GID1 based switch as described herein, e.g., in the Dimerization Switch Module.

In an instance, the dimerization switch comprises a Halotag/SNAP-tag based switch, e.g., a Halotag/SNAP-tag based switch described herein, e.g., a Halotag/SNAP-tag based switch as described herein, e.g., in the Dimerization Switch Module.

In an instance, the RCAR comprises:
a) an intracellular signaling member comprising, beginning with the amino terminus:
   a CD3zeta domain, and
   a first switch domain; and
b) a costimulatory ECD domain member comprising, beginning with the amino terminus:
   a costimulatory ECD domain,
   a transmembrane domain,
   a 4-1BB domain, and
   a second switch domain,
   wherein the first and second switch domains form a FKBP-FRB based switch.

In an instance, the RCAR is associated with, e.g., is provided in the same cell with:
an inhibitor of an inhibitory molecule, e.g., an inhibitor of a inhibitory molecule of Table 3.

In an instance, the RCAR is associated with, e.g., is provided in the same cell withan shRNA that targets a inhibitory molecule, e.g. a coinhibitory molecule from Table 3.

In an instance, the RCAR further comprises: a shRNA that targets PD1.

In an instance, the antigen binding domain binds to a target antigen on a cancer cell but does not activate the RCARX cell, e.g., a RCART cell, until a dimerization molecule is administered.

In an instance, the antigen binding domain binds to a target antigen on a target cell, e.g., a cancer cell, but does not promote an immune effector response, e.g., a T cell activation, until the dimerization molecule, e.g., a heterodimerization molecule or homodimerization molecule, is administered.

The disclosure also provides RCARs having a configuration that allows switching of proliferation. For example, upon antigen encounter, the RCAR exhibits constitute primary signal, e.g., target cell killing, and allows regulation of a second signal, e.g., proliferation, survival, and cytokine secretion.

Accordingly, in another aspect, the disclosure features, a regulatable chimeric antigen receptor (RCAR), e.g., an isolated RCAR, wherein the RCAR comprises:
a) an intracellular signaling member comprising:
   optionally, a transmembrane domain or membrane tethering domain;
   a co-stimulatory signaling domain, selected e.g., from Table 2, and
   a switch domain; and
b) an antigen binding member comprising:
   an antigen binding domain,
   a transmembrane domain, and
   a primary intracellular signaling domain, e.g., selected fromTable 1, e.g., a CD3zeta domain,
   wherein the antigen binding member does not comprise a switch domain, or does not comprise a switch domain that dimerizes with a switch domain on the intracellular signaling member.

In an instance, the antigen binding member does not comprise a costimulatory signaling domain.

In an instance, the intracellular signaling member comprises a second costimulatory signaling domain, selected, e.g., from Table 2. In an instance, the two or more costimulatory domains can be the same costimulatory signaling domain, e.g., selected from the list in Table 2, or different costimulatory signaling domains, e.g., selected from the list in Table 2. In an instance the intracellular signaling member comprises: a plurality, e.g., 2 or 3, co-stimulatory signaling domains selected from 41BB, CD28, CD27, ICOS, and OX40.

In an instance, the intracellular signaling member comprises the following co-stimulatory signaling domains, from the extracellular to intracellular direction:
41BB-CD27;
CD27-41BB;
41BB-CD28;
CD28-41BB;
OX40-CD28;
CD28-OX40;
CD28-41BB; or
41BB-CD28.

In an instance, the intracellular signaling member comprises the following co-stimulatory signaling domains: CD28-41BB.

In an instance, intracellular signaling member comprises the following co-stimulatory signaling domains: CD28-OX40.

In an instance, in addition to one or a plurality of co-stimulatory signaling domains, the intracellular signaling member comprises a primary intracellular signaling domain, e.g., selected from Table 1, e.g., a CD3zeta domain.

In an instance, the intracellular signaling domain comprises a CD28 co-stimulatory signaling domain, a 4-1BB co-stimulatory signaling domain, and a CD3zeta domain.

In an instance, the intracellular signaling domain comprises a CD28 co-stimulatory signaling domain, a OX40 co-stimulatory signaling domain, and a CD3zeta domain.

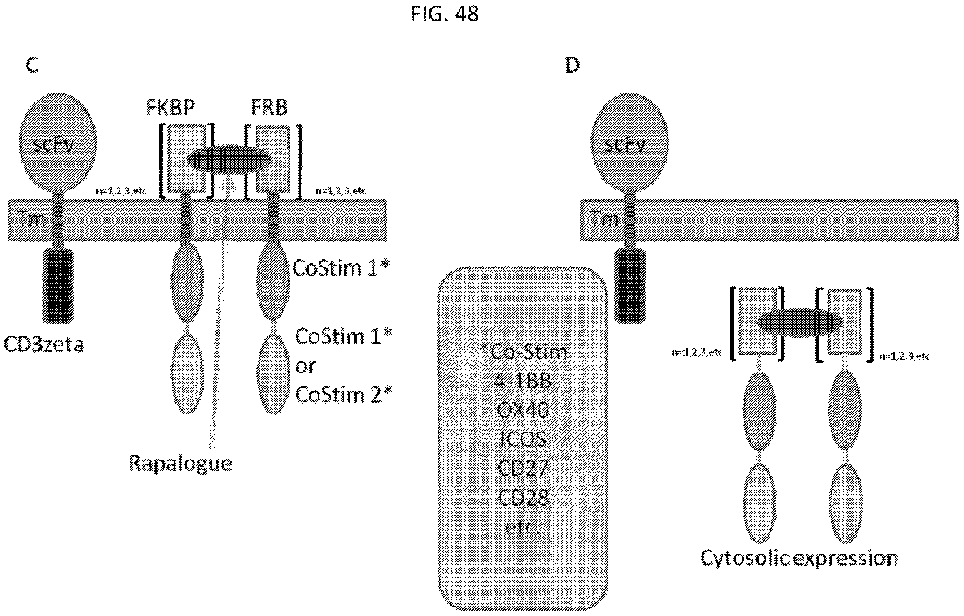

In an instance, the intracellular signaling member does not comprise a transmembrane domain or membrane tethering domain. In such instances, the switch domain is intracellular. In such instances, the intracellular signaling member comprises two costimulatory signaling domains, where the two costimulatory domains are selected from 4-1BB, OX40, CD27, CD28, and ICOS. In an instance, the order of elements on the intracellular signaling member is as follows, from the extracellular to intracellular direction:
a first co-stimulatory signaling domain/ a second costimulatory signaling domain and a switch domain disposed between any of the signaling elements, or, from the extracellular to intracellular direction, after all other signaling elements. See, e.g., Fig. 48D.

In an instance, the intracellular signaling member comprises a transmembrane domain. In such instances the switch domain can be intracellular or extracellular. In such instances, the intracellular signaling member comprises two costimulatory signaling domains, where the two costimulatory domains are selected from 4-1BB, OX40, CD27, CD28, and ICOS.

In an instance where the switch domain is extracellular, the order of elements on the intracellular signaling member is as follows, from the extracellular to intracellular direction:
a switch domain/ a transmembrane domain/ a first co-stimulatory signaling domain/ a second costimulatory signaling domain. See, e.g., Fig. 48C.

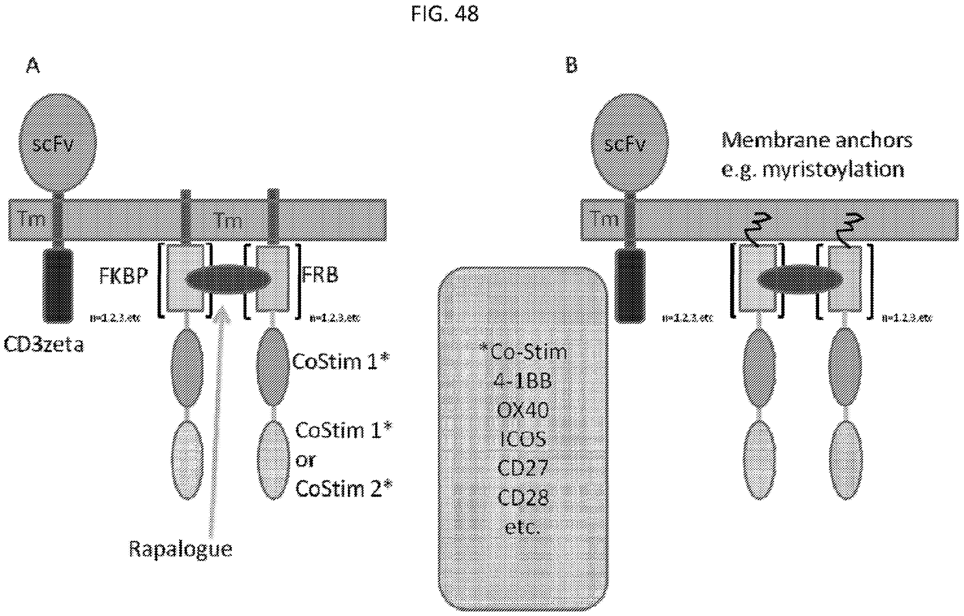

In an instance where the switch domain is intracellular, the order of elements on the intracellular signaling member is as follows, ,rom the extracellular to intracellular direction:
transmembrane domain/ a first co-stimulatory signaling domain/ a second costimulatory signaling domain and a switch domain disposed intracellularly between any of the signaling elements, or, from extracellular to intracellular, after all other signaling elements. See, e.g., Fig. 48A.

In an instance, the intracellular signaling member comprises a membrane tethering domain. In one such instance, the switch domain is intracellular. In such instances, the intracellular signaling member comprises two costimulatory signaling domains, where the two costimulatory domains are selected from 4-1BB, OX40, CD27, CD28, and ICOS. In an instance, the order of elements on the intracellular signaling member is as follows, from the extracellular to intracellular direction:
a membrane tethering domain/a first co-stimulatory signaling domain/ a second costimulatory signaling domain and a switch domain disposed extracellularly, between any of the signaling elements, or, from extracellular to intracellular, after all other signaling elements. See, e.g., Fig. 48B.

In an instance, the switch domain is: extracellular; disposed between the transmembrane domain or membrane tethering domain and a co-stimulatory signaling domain, e.g., the costimulatory signaling domain closest to the membrane; between a first and second costimulatory signaling domain; between a costimulatory signaling domain and a primary intracellular signaling domain; or, from extracellular to intracellular, after all intracellular signaling domains.

In an instance, the order of elements on the intracellular signaling member, from extracellular to intracellular, is as follows:
transmembrane domain or membrane tethering domain/a first co-stimulatory signaling domain/optionally a second costimulatory signaling domain/and optionally a primary intracellular signaling domain, and a switch domain disposed extracellularly, between any of the elements, or, from extracellular to intracellular, after all other elements.

In an instance, the order of elements on the antigen binding member, from extracellular to intracellular, is as follows:
antigen binding domain/transmembrane domain/primary intracellular signaling domain, e.g., selected from Table 1, e.g., a CD3zeta domain.

In an instance, the intracellular signaling member comprises a switch domain from a homodimerization switch.

In an instance, the intracellular signaling member comprises a first switch domain of a heterodimerization switch and the RCAR comprises a second intracellular signaling member which comprises a second switch domain of the heterodimerization switch. In instances, the second intracellular signaling member comprises the same intracellular signaling domains as the intracellular signaling member.

In an instance, the antigen binding member comprises a plurality of, e.g., 2, 3, 4, or 5, antigen binding domains, e.g., scFvs, wherein each antigen binding domain binds to a target antigen. In an instance, two or more of the antigen binding domains can bind to different antigens. In an instance, two or more of the antigen binding domains can bind to the same antigen, e.g., the same or different epitopes on the same antigen. In instances, a linker or hinge region is optionally disposed between two or each of the antigen binding domains.

In an instance the dimerization switch is intracellular.

In an instance the dimerization switch is extracellular.

In an instance, the dimerization switch comprises a FKBP-FRB based switch.

In an instance, the dimerization switch comprises:
a switch domain comprising a rapamycin analog binding sequence having at least 80, 85, 90, 95, 98, or 99 % identity with FKBP, and a switch domain comprising a rapamycin analog binding sequence binding sequence having at least 80, 85, 90, 95, 98, or 99 % identity with FRB.

In an instance, the dimerization switch comprises:
a switch domain comprising a rapamycin analog binding sequence that differs by no more than 10, 9, 8, 7, 6, 5, 4, 3, 2, or 1 amino acid residues from the corresponding sequence of FKBP, and a switch domain comprising a rapamycin analog binding sequence that differs by no more than 10, 9, 8, 7, 6, 5, 4, 3, 2, or 1 amino acid residues from the corresponding sequence of FRB.

In an instance, the dimerization switch comprises a FRB binding fragment or analog of FKBP and an FKBP binding fragment or analog of FRB, and the FKBP binding fragment or analog of FRB comprises one or more mutations which increase the affinity of binding with rapamycin or a rapalog, e.g., RAD001, or a mutation described in the section herein entitled **MODIFIED FKBP/FRB-BASED DIMERIZATION SWITCHES.** E.g., the FKBP binding fragment or analog of FRB comprises: an E2032 mutation, e.g., an E2032I mutation or E2032L mutation; a T2098 mutation, e.g., a T2098L mutation; or an E2032 and a T2098 mutation, e.g., an E2032I and a T2098L or an E2032L and a T2098L mutation.

In an instance, the dimerization switch is a multi switch comprising a plurality of, e.g., 2, 3, 4, 5, 6, 7, 8, 9, or 10, switch domains, independently, on the intracellular signaling member. In instances where the intracellular signaling member comprises a plurality of first switch domains of a heterodimerization switch, e.g., FKBP-based switch domains, the RCAR further comprises a second intracellular signaling member comprising a plurality of second switch domains of a heterodimerization switch, e.g., FRB-based switch domains. In instances where the intracellular signaling member comprises a first and a second switch domain, e.g., a FKBP-based switch domain and a FRB-based switch domain, the RCAR further comprises a second intracellular signaling member comprising a first and a second switch domain, e.g., a FKBP-based switch domain and a FRB-based switch domain.

In an instance, wherein the switch is an FKBP-FRB based switch, the dimerization molecule is an mTOR inhibitor, e.g., an allosteric mTOR inhibitor, e.g., rapamycin or a rapalog, e.g., RAD001.

In an instance, any of the dosing regimes or formulations of an allosteric mTOR inhibitor, e.g., RAD001, described in the section here for a low, immune enhancing, dose of an allosteric mTOR inhibitor, e.g., RAD001, can be administered to dimerize an FKBP-FRB based switch.

In an instance, the switch is an FKBP-FRB based switch and the dimerization molecule is RAD001.

In an instance, 0.1 to 20, 0.5 to 10, 2.5 to 7.5, 3 to 6, or about 5, mgs of RAD001 per week, e.g., delivered once per week, is administered.

In an instance, 0.3 to 60, 1.5 to 30, 7.5 to 22.5, 9 to 18, or about 15 mgs of RAD001 in a sustained release formuation, per week, e.g., delivered once per week,is administered.

In an instance, 0.005 to 1.5, 0.01 to 1.5, 0.1 to 1.5, 0.2 to 1.5, 0.3 to 1.5,0.4 to 1.5, 0.5 to 1.5, 0.6 to 1.5, 0.7 to 1.5, 0.8 to 1.5, 1.0 to 1.5, 0.3 to 0.6, or about 0.5 mgs of RAD001 per day, e.g., delivered once once per day, is administered.

In an instance, 0.015 to 4.5, 0.03 to 4.5, 0.3 to 4.5, 0.6 to 4.5, 0.9 to 4.5, 1.2 to 4.5, 1.5 to 4.5, 1.8 to 4.5, 2.1 to 4.5, 2.4 to 4.5, 3.0to 4.5, 0.9 to 1.8, or about 1.5 mgs of RAD001 in a sustained release formulation, per day, e.g., delivered once once per day, is administered.

In an instance, 0.1 to 30, 0.2 to 30, 2 to 30, 4 to 30, 6 to 30, 8 to 30, 10 to 30, 1.2 to 30, 14 to 30, 16 to 30, 20 to 30, 6 to 12, or about 10 mgs of RAD001 in a sustained release formulation, per week, e.g., delivered once once per week, is administered.

In an instance, the dimerization switch comprises:
a switch domain comprising a rapamycin, or rapamycin analog, binding sequence from FKBP, and a switch domain comprising a rapamycin, or rapamycin analog, binding sequence from FRB, e.g., a sequence comprising a lysine at residue 2098.

In an instance, the dimerization switch comprises:
a switch domain comprising a rapamycin analog binding sequence from FKBP, and a switch domain comprising a rapamycin analog binding sequence from FRB, e.g., a sequence comprising a lysine at residue 2098.

In an instance, the dimerization switch comprises:
a switch domain comprising an AP21967 binding sequence from FKBP, and a switch domain comprising an AP21967 binding sequence from FRB, e.g., a sequence comprising a lysine at residue 2098.

In an instance:
the first switch domain comprises,
   a rapamycin, or rapamycin analog, binding sequence from FKBP;
   a rapamycin analog binding sequence from FKBP; or
      an AP21967 binding sequence from FKBP; and,
the second switch domain comprises,
   a rapamycin, or rapamycin analog, binding sequence from FRB;
   a rapamycin analog binding sequence from FRB; or
   an AP21967 binding sequence from FRB, e.g., a sequence comprising a lysine at residue 2098.

In an instance:
the first switch domain comprises,
   a rapamycin, or rapamycin analog, binding sequence from FRB;
   a rapamycin analog binding sequence from FRB; or
      an AP21967 binding sequence from FRB, e.g., a sequence comprising a lysine at residue 2098; and,
the second switch domain comprises,
   a rapamycin, or rapamycin analog, binding sequence from FKBP;
   a rapamycin analog binding sequence from FKBP; or
      an AP21967 binding sequence from FKBP.

In an instance:
the first switch domain comprises an AP21967 binding sequence from FKBP; and,
the second switch domain comprises an AP21967 binding sequence from FRB, e.g., a sequence comprising a lysine at residue 2098.

In an instance, the first switch domain comprises an AP21967 binding sequence from FRB, e.g., a sequence comprising a lysine at residue 2098; and,
the second switch domain comprises an AP21967 binding sequence from FKBP.

In an instance, the dimerization molecule is a rapamycin analogue, e.g., AP21967.

In an instance, the dimerization switch comprises a GyrB-GyrB based switch.

In an instance, the dimerization switch comprises:
a switch domain comprising a coumermycin binding sequence having at least 80, 85, 90, 95, 98, or 99 % identity with the 24 K Da amino terminal sub-domain of GyrB.

In an instance the dimerization switch comprises:
a switch domain comprising a coumermycin binding sequence that differs by no more than 10, 9, 8, 7, 6, 5, 4, 3, 2, or 1 amino acid residues from the corresponding sequence of 24 K Da amino terminal sub-domain of GyrB.

In an instance, the dimerization switch comprises:
a switch domain comprising a coumermycin binding sequence from the 24 K Da amino terminal sub-domain of GyrB.

In an instance, the dimerization switch comprises:
the 24 K Da amino terminal sub-domain of GyrB.

In an instance, the dimerization molecule is a coumermycin.

In an instance, the dimerization switch comprises a GAI-GID1 based switch.

In an instance, the dimerization switch comprises:
a GID1 switch domain comprising a gibberellin, or gibberellin analog, e.g., GA₃, binding sequence having at least 80, 85, 90, 95, 98, or 99 % identity with GID1, and a switch domain comprising a GAI switch domain having at least 80, 85, 90, 95, 98, or 99 % identity with GAI.

In an instance, the dimerization switch comprises:
a GID1 switch domain comprising a gibberellin, or gibberellin analog, e.g., GA₃, binding sequence that differs by no more than 10, 9, 8, 7, 6, 5, 4, 3, 2, or 1 amino acid residues from the corresponding sequence of G1D1, and a GAI switch domain that differs by no more than 10, 9, 8, 7, 6, 5, 4, 3, 2, or 1 amino acid residues from the corresponding sequence of GAI.

In an instance:
the first switch domain comprises a GID1 switch domain; and,
the second switch domain comprises a GAI switch domain.

In an instance:
the first switch domain comprises a GAI switch domain; and,
the second switch domain comprises a GID1 switch domain.

In an instance, the dimerization molecule is GA₃-AM.

In an instance, the dimerization molecule is GA₃.

In an instance, the dimerization molecule is a small molecule, e.g., is other than a polypeptide.

In an instance, the dimerization molecule is a polypeptide, e.g., a polypeptide, e.g., an antibody molecule, or a non-antibody scaffold, e.g., a fribronectin or adnectin, having specific affinity for one or both of the first and second switch domains.

In an instance, the dimerization molecule, e.g. a polypeptide, is an antibody molecule.

In an instance, the dimerization switch comprises a Halotag/SNAP-tag based switch.

In an instance, the dimerization switch comprises:
a Halotag switch domain comprising having at least 80, 85, 90, 95, 98, or 99 % identity with SEQ ID NO: 14, and a SNAP-tag switch domain having at least 80, 85, 90, 95,98, or 99 % identity with SEQ ID NO: 15.

In an instance, the dimerization switch comprises:
a Halotag switch domain comprising that differs by no more than 10, 9, 8, 7, 6, 5, 4, 3, 2, or 1 amino acid residues from SEQ ID NO: 14, and a SNAP-tag switch domain that differs by no more than 10, 9, 8, 7, 6, 5, 4, 3, 2, or 1 amino acid residues from SEQ ID NO: 15.

In an instance:
the first switch domain comprises a Halotag switch domain; and,
the second switch domain comprises a SNAP-tag switch domain.

In an instance:
the first switch domain comprises a SNAP-tag switch domain; and,
the second switch domain comprises a Halotag switch domain.

In an instance, the dimerization molecule comprises structure 5.

In an instance, the dimerization molecule comprises three or more domains, e.g., protein tags that bind a switch domain, e.g., a polypeptide, e.g., an antibody molecule or non-antibody scaffold, having affinity for the domain.

In an instance, the dimerization molecule is a non-covalent dimerization molecule.

In an instance, the dimerization molecule is covalent dimerization molecule.

In an instance, the dimerization switch, e.g., a homodimerization switch, e.g., an extracellular homodimerization switch, comprises switch domains that comprise tag molecules, e.g., a c-myc peptide tag, flag peptide tag, HA peptide tag or V5 peptide tag, and the dimerization switch comprises polypeptides with affinity for the switch domains, e.g., antibody molecules and non-antibody scaffold.

In an instance, the RCAR further comprises a second order dimerization switch.

In an instance, the dimerization molecule has a valency of greater than two, e.g., it is multi-valent, and binds, and thus clusters or dimerizes, more than two switch domains.

In an instance, the RCAR is associated with, e.g., is provided in the same cell with:
an inhibitor of an inhibitory molecule, e.g., an inhibitor of an inhibitory molecule of Table 3.

In an instance, the RCAR is associated with, e.g., is provided in the same cell with, a nucleic acid inhibitor, e.g., an siRNA, an shRNA, or an antisense molecule, that targets a inhibitory molecule, e.g. a coinhibitory molecule from Table 3.

In an instance, the shRNA targets PD1.

In an instance, dimerization increases the level of proliferation or persistence of the RCARX, e.g., RCART, cell.

In an instance, the RCAR further comprises:
an inhibitory counter ligand binding member comprising,
an inhibitory counter ligand binding domain, selected e.g., from Table 4, and
a transmembrane domain or membrane anchor.

In one embodiment, in a RCAR/NKR-CAR cell, said NKR-CAR comprises an extra-cellular antigen binding domain; a transmembrane domain (e.g., a NKR transmembrane domain) and a cytoplasmic domain (e.g., an NKR cytoplasmic domain). In embodiments, the NKR-CAR comprises a KIR-CAR; a NCR-CAR; a SLAMF-CAR; a FcR-CAR; or a Ly49-CAR. In embodiments, the NKR-CAR comprises an inhibitory NKR-CAR (inhNKR-CAR). In embodiments, the inhNKR-CAR is an inhKIR-CAR, a inhSLAMF-CAR, or an inhLy49-CAR.

In one embodiment, said NKR-CAR comprises a KIR-CAR, e.g., an actKIR-CAR or inhKIR-CAR, a NCR-CAR, e.g., an actNCR-CAR, a SLAMF-CAR, e.g., an inhSLAMF-CAR, a FcR-CAR, e.g., CD16-CAR, e.g., an actCD16-CAR, or CD64-CAR, e.g., an actCD64-CAR, or a Ly49-CAR, e.g., an actLy49-CAR or inhLy49-CAR. In one embodiment, the NKR-CAR comprises a transmembrane domain and an extra-cellular antigen binding domain, and further comprising a hinge domain disposed between said transmembrane domain and said extra-cellular antigen binding domain. In one embodiment, the NKR-CAR is an activating NKR-CAR, and the extra-cellular antigen binding domain is an antigen binding domain described herein.

In an embodiment, the KIR-CAR comprises an extra-cellular antigen binding domain and a transmembrane domain, e.g., a KIR transmembrane domain, or cytoplasmic domain, e.g., an ITIM-containing cytoplasmic domain, or a KIR-cytoplasmic domain. In one embodiment, the KIR-CAR comprises an extra-cellular antigen binding domain, a transmembrane domain, and an ITIM-containing cytoplasmic domain, or a KIR-cytoplasmic domain. In one embodiment, said transmembrane domain can interact with, e.g., bind, the transmembrane domain of DAP12. In one embodiment, said transmembrane domain comprises a positively charged moiety, e.g., an amino acid residue comprising a positively charged moiety, e.g., side chain. In one embodiment, said transmembrane domain comprises a KIR-transmembrane domain.

In one embodiment, said KIR-CAR is an activating KIR-CAR. In one embodiment, said KIR-CAR comprises a KIR-transmembrane domain. In one embodiment, said KIR-CAR is an inhibitory KIR-CAR. In one embodiment, said KIR-CAR comprises a KIR-cytoplasmic domain. In one embodiment, said KIR-CAR comprises an extra-cellular antigen binding domain and a transmembrane domain, e.g., a transmembrane domain comprising a positively charged moiety, e.g., an amino acid residue comprising a positively charged moiety, e.g., side chain, or a KIR-transmembrane domain.

In one embodiment, a KIR-CAR described herein comprises an antigen binding domain comprising an scFv. In one embodiment, said antigen binding domain comprises a single VH domain, e.g., a camelid, shark, or lamprey single VH domain, or a single VH domain derived from a human or mouse sequence, or a non-antibody scaffold, e.g., a fibronectin, e.g., a fibronectin type III antibody-like molecule. In one embodiment, said antigen binding domain comprises a nanobody. In one embodiment, said antigen binding domain comprises a camelid VHH domain.

In one embodiment, the KIR-CAR is an activating KIR-CAR, and the extra-cellular antigen binding domain is an antigen binding domain described herein.

In one embodiment, a KIR-CAR described herein comprises an extracellular hinge domain. In one embodiment, the extracellular hinge domain is other than a KIR hinge domain, e.g., other than a KIR2DS2 hinge domain. In one embodiment, the extracellular hinge domain is derived from a natural molecule. In one embodiment, the extracellular hinge domain is derived from a natural molecule other than a KIR. In one embodiment, the extracellular hinge domain comprises a non-naturally occurring polypeptide sequence. In one embodiment, the extracellular hinge domain comprises the extracellular hinge from human CD8-alpha. In one embodiment, the extracellular hinge domain comprises a synthetic extracellular hinge. In one embodiment, the extracellular hinge domain is less than 50, 20, or 10 amino acids in length. In one embodiment, the extracellular hinge domain has fewer amino acids than a KIR2DS2 hinge domain.

In one embodiment, the KIR-CAR described herein is an actKIR-CAR. In one embodiment, said actKIR-CAR comprises a transmembrane domain comprising a positively charged moiety, e.g., an amino acid residue comprising a positively charged moiety, e.g., a positively charged side chain or an actKIR transmembrane domain. In one embodiment, said actKIR-CAR can interact with and promote signaling from an ITAM-containing polypeptide or adaptor molecule. In one embodiment, said actKIR-CAR can interact with and promote signaling from a DAP12 polypeptide. In one embodiment, said actKIR-CAR comprises a KIR D domain. In one embodiment, said actKIR-CAR comprises a KIR D1 domain. In one embodiment, said actKIR-CAR comprises a KIR D2 domain. In one embodiment, said actKIR-CAR said act KIR-CAR does not comprise a KIR D domain. In one embodiment, said actKIR-CAR comprises a KIR2DS2 transmembrane domain. In one embodiment, said actKIR-CAR further comprises a KIR2DS2 cytoplasmic domain. In one embodiment, said actKIR-CAR does not comprise a KIR D domain.

In one embodiment, the antigen binding domain of a KIR-CAR described herein binds an antigen present on a target cell, e.g., a cancer cell. In one embodiment, said antigen binding domain binds an antigen that is more highly expressed on a target cell, e.g., a cancer cell, than a non-target cell, e.g., a non-cancerous cell, e.g., a non cancerous cell of the same type as the target cell. In one embodiment, said antigen binding domain is binds an antigen described herein, e.g., a tumor antigen described herein. In one embodiment, the tumor antigen is expressed on a solid tumor, e.g., a solid tumor described herein, e.g., mesothelioma (e.g., malignant pleural mesothelioma), lung cancer (e.g., non-small cell lung cancer, small cell lung cancer, squamous cell lung cancer, or large cell lung cancer), pancreatic cancer (e.g., pancreatic ductal adenocarcinoma), ovarian cancer, colorectal cancer and bladder cancer or any combination thereof.

In one embodiment, the KIR-CAR described herein is an inhKIR-CAR. In one embodiment, the inhKIR-CAR comprises an inhKIR transmembrane domain. In one embodiment, the inhKIR-CAR inhKIR-CAR comprises an ITIM-containing cytoplasmic domain, e.g., an inhKIR cytoplasmic domain, e.g., a KIR2DL or KIR3DL cytoplasmic domain. In one embodiment, the inhKIR-CAR comprises a transmembrane other than a KIR transmembrane, e.g., a transmembrane domain from PD-1, CTLA4 or ITIM-containing receptors from ILT (CD85), Siglec, LMIR (CD300) and/ or SLAM gene families of receptors. In one embodiment, the inhKIR-CAR comprises a cytoplasmic domain from an inhibitory receptor other than a KIR, e.g., from PD-1, CTLA4 or ITIM-containing receptors from ILT (CD85), Siglec, LMIR (CD300) and/ or SLAM gene families of receptors. In one embodiment, the inhKIR-CAR comprises a transmembrane and cytoplasmic domain from an inhibitory receptor other than a KIR, e.g., transmembrane and cytoplasmic domain, independently, from e.g., PD-1, CTLA4 or ITIM-containing receptors from ILT (CD85), Siglec, LMIR (CD300) and/ or SLAM gene families of receptors. In one embodiment, said cytoplasmic domain comprises an ITIM. In one embodiment, the inhKIR-CAR comprises a KIR D domain. In one embodiment, the inhKIR-CAR comprises a KIR D0 domain. In one embodiment, the inhKIR-CAR comprises a KIR D1 domain. In one embodiment, the inhKIR-CAR comprises a KIR D2 domain. In one embodiment, the inhKIR-CAR does not comprise a KIR D domain.

In one embodiment, the antigen binding domain of the inhKIR-CARs described herein binds an antigen not present on a target cell, e.g., a cancer cell. In one embodiment, said antigen binding domain binds an antigen that is more highly expressed on a non-target cell, e.g., a non-cancer cell, than a target cell, e.g., cancerous cell, e.g., a cancerous cell of the same type as the target cell. In one embodiment, said antigen binding domain binds desmoglein1/3 (DSG1/3). In an embodiment, an inhCAR, e.g., an inhTCAR or inhNKR-CAR, e.g., an inhKIR-CAR, and an actCAR, e.g., an actTCAR or actNKR-CAR, e.g., an actKIR-CAR, are provided in which the inhCAR comprises an antigen binding domain that targets desmoglein1/3 (DSG1/3) and the actCAR comprises an antigen binding domain that targets an antigen other than DSG1/3, e.g., EGFR. In an embodiment, this pair is used to treat an EGFR expressing cancer, e.g., an adenocarcinoma of the lung or colon. In an embodiment the cancer cells express less DSG1/3 than non-cancer cells. In an embodiment this combination can minimize CAR-mediated attack of skin cells or squamous cells of the GI track (i.e. oral mucosa). In one embodiment, said antigen binding domain binds an ephrin receptor or a claudin.

In an embodiment, a NCR-CAR, e.g., an activating NCR-CAR, comprises an extra-cellular antigen binding domain, a transmembrane domain, e.g., a transmembrane domain comprising a positively charged moiety, e.g., an amino acid residue comprising a positively charged moiety, e.g., a positively charged side chain or an NCR transmembrane domain, and a cytoplasmic domain, e.g., a NCR cytoplasmic domain.

In one embodiment, said NCR-CAR comprises an a transmembrane domain comprising a positively charged moiety, e.g., an amino acid residue comprising a positively charged moiety, e.g., a positively charged side chain, e.g., NCR transmembrane domain, e.g., a NKp30, NKp44, or NKp46 cytoplasmic domain. In one embodiment, said NCR-CAR comprises a cytoplasmic domain which can interact with an adaptor molecule or intracellular signaling molecule comprising, e.g., a DAP12, FcRγ or CD3 ζ cytoplasmic domain. In one embodiment, said NCR-CAR, e.g., a NKp30-CAR, comprises a transmembrane domain which can interact with an adaptor molecule or intracellular signaling molecule, e.g., DAP12. In one embodiment, said NCR-CAR comprises a NKp46-CAR. In one embodiment, said NKp46-CAR, comprises a transmembrane domain comprising a positively charged moiety, e.g., an amino acid residue comprising a positively charged moiety, e.g., a positively charged side chain or, e.g., an NCR transmembrane domain, which can interact with an adaptor molecule or intracellular signaling molecule, e.g., one having a FcRy or CD3 ζ cytoplasmic domain. In one embodiment, said NCR-CAR described herein further comprises a hinge domain disposed between said transmembrane domain and said an extra-cellular antigen binding domain.

In one embodiment, the NCR-CAR is an activating NCR-CAR, and the extra-cellular antigen binding domain is an antigen binding domain described herein.

In an embodiment, the SLAMF-CAR, e.g., an inhibitory SLAMF-CAR, comprises an extra-cellular antigen binding domain, a transmembrane domain, e.g., a transmembrane domain comprising a positively charged moiety, e.g., an amino acid residue comprising a positively charged moiety, e.g., a positively charged side chain, e.g., a SLAMF transmembrane domain, and a SLAMF cytoplasmic domain. In one embodiment, said SLAMF-CAR comprises a SLAMF, CD48, CD229, 2B4, CD84, NTB-A, CRACC, BLAME, or CD2F-10 cytoplasmic domain. In one embodiment, said SLAMF-CAR further comprises a hinge domain, disposed between said transmembrane domain and said an extra-cellular antigen binding domain.

In an embodiment, the FcR-CAR, e.g., CD16-CAR, e.g., comprises an activating CD16-CAR or a CD64-CAR, e.g., an activating CD64-CAR, comprising an extra-cellular antigen binding domain, a transmembrane domain, and a CD 16 or CD64 cytoplasmic domain. In one embodiment, said FcR-CAR is a CD16-CAR. In one embodiment, said FcR-CAR is a CD64-CAR. In one embodiment, said FcR-CAR can interact with an adaptor molecule or intracellular signaling molecule, e.g., a FcRγ or CD3 ζ domain, e.g., via a transmembrane domain, e.g., a transmembrane domain comprising a positively charged moiety, e.g., an amino acid residue comprising a positively charged moiety, e.g., a positively charged side chain or e.g., a CD16 or CD64 transmembrane domain. In one embodiment, said FcR-CAR further comprises a hinge domain, disposed between said transmembrane domain and said an extra-cellular antigen binding domain.

In an embodiment, the Ly49-CAR comprises an extra-cellular antigen binding domain, and a transmembrane domain, e.g., a Ly49-transmembrane domain, or a cytoplasmic domain, e.g., an ITIM-containing cytoplasmic domain, e.g., a Ly49-cytoplasmic domain. In one embodiment, the Ly49-CAR comprises a transmembrane domain and a Ly49-cytoplasmic domain. In one embodiment, said Ly49-CAR is an activating Ly49-CAR, e.g., Ly49D or Ly49H. In one embodiment, said Ly49-CAR comprises a positively charged transmembrane domain, e.g., a positively charged Ly49 transmembrane domain. In one embodiment, said Ly49-CAR can interact with an ITAM-containing cytoplasmic domain, e.g., DAP 12. In one embodiment, said Ly49-CAR comprises a Ly49-transmembrane domain. In one embodiment, said KIR-CAR is an inhibitory Ly49-CAR, e.g., Ly49A or Ly49C. In one embodiment, said Ly49-CAR comprises an ITIM-containing cytoplasmic domain, e.g., a Ly49-cytoplasmic domain. In one embodiment, said Ly49-CAR comprises a Ly49-transmembrane domain or a Ly49-cytoplasmic domain selected, independently from Ly49A-Ly49W. In one embodiment, said Ly49-CAR further comprises a hinge domain, disposed between said transmembrane domain and said an extra-cellular antigen binding domain.

In an embodiment, the RCAR/NKR-CAR cell comprises a nucleic acid encoding a RCAR and a NKR-CAR. In embodiment, a single nucleic acid molecule comprises sequence encoding a RCAR and sequence encoding a NKR-CAR. In embodiments, the nucleic acid comprises a first nucleic acid molecule comprising a sequence encoding a RCAR and a second nucleic acid molecule comprising sequence encoding a NKR-CAR.

In an instance, e.g., in a RCAR/NKR-CAR cell or for use in generating a RCAR/NKR-CAR cell, an isolated nucleic acid encoding a RCAR comprises a sequence encoding the antigen binding member and the intracellular signaling member are present in a single nucleic acid molecule. In an instance, sequence encoding the antigen binding member is operatively linked to a first control region and sequence encoding the intracellular signaling member is operatively linked to a second control region. In an instance, sequence encoding the antigen binding member is transcribed as a first RNA and sequence encoding intracellular signaling member is translated as a second RNA. In an instance, sequence encoding the antigen binding member is present on a first nucleic acid molecule and sequence encoding intracellular signaling member is present on a second nucleic acid molecule. In an instance, sequence encoding the antigen binding member and the intracellular signaling member are present in a single nucleic acid molecule. In an instance, the nucleic acid encodes a RCAR as described in any of Tables 6, 7, 8, 9, 10, or 11.

In instances, e.g., in a RCAR/NKR-CAR cell or for use in generating a RCAR/NKR-CAR cell, the nucleic acid comprises a DNA, or RNA, sequence, e.g., a mRNA, comprising a sequence that encodes a NKR-CAR described herein. In one instance, the nucleic acid further comprising a sequence that encodes an adaptor molecule or intracellular signaling domain that interacts with said NKR-CAR. In one instance, the nucleic acid encodes an activating or inhibiting NKR-CAR, and the encoded extra-cellular antigen binding domain is an antigen binding domain described herein.

In embodiments, the cell comprises a vector system comprising a nucleic acid encoding a RCAR and a NKR-CAR. In embodiments, a single vector comprises a sequence encoding a RCAR and a sequence encoding a NKR-CAR. In embodiments, the vector system comprises a first vector comprising a sequence encoding a RCAR and a second vector comprising a sequence encoding a NKR-CAR.

In an embodiment, all of the elements of a RCAR are encoded on a single vector.

In an embodiment, an element of a RCAR is encoded on a first vector and another element of the RCAR is encoded on a second vector, of the vector system.

In an embodiment, the vector system comprises a DNA, a RNA, a plasmid, a lentivirus vector, adenoviral vector, or a retrovirus vector.

In an embodiment, the vector system comprises a bi-cistronic or tri-cistronic lentivirus vector.

In an embodiment, the vector system comprises a bi-cistronic or tri-cistronic promoter.

In one embodiment, the nucleic acid sequence(s) encoding a RCAR and the nucleic acid sequence(s) encoding a NKR-CAR are disposed on the same nucleic acid molecule, e.g., the same vector, e.g., the same viral vector, e.g., a lenti-viral vector. In one embodiment, the nucleic acid sequence(s) encoding a RCAR is disposed on a first nucleic acid molecule, e.g., a first vector, e.g., a viral vector, e.g., a lenti-viral vector, and the nucleic acid sequence(s) encoding a NKR-CAR is disposed on a second nucleic acid molecule, e.g., a second vector, e.g., a viral vector, e.g., a lenti-viral vector.

In embodiments, e.g., in/on a RCAR/NKR-CAR cell, the antigen binding domain of the RCAR and the antigen binding domain of the NKR-CAR, e.g., the inhNKR-CAR, target different antigens. In embodiments, the antigen binding domain of the RCAR binds to a tumor antigen. In embodiments, the antigen binding domain of the inhNKR-CAR binds to a target antigen that is expressed on normal cells, e.g., non-tumor or non-cancerous cells, but is not highly expressed on tumor or cancerous cells. In embodiments, when the antigen binding domains of the RCAR and the inhNKR-CAR both bind to their target antigen, the cell does not activate.

In embodiments, the antigen binding domain of the RCAR or NKR-CAR does not comprise a variable light domain and a variable heavy domain, and the other (e.g., RCAR or NKR-CAR) is not an scFv.

In an aspect, disclosed herein is a nucleic acid comprising:
(i) a sequence encoding a RCAR and
(ii) a sequence encoding a NKR-CAR,
wherein the RCAR comprises:
A) an intracellular signaling member comprising:
   an intracellular signaling domain, e.g., a primary intracellular signaling domain, and
   a first switch domain;
B) an antigen binding member comprising:
   an antigen binding domain,
   a second switch domain; and optionally,
C) a transmembrane domain, and
wherein the NKR-CAR comprises:
a) an antigen binding domain,
b) a transmembrane domain, e.g., an NKR transmembrane domain, and
c) a cytoplasmic domain, e.g., an NKR cytoplasmic domain.

In instances, the sequence encoding the RCAR comprises:
i) a sequence encoding (A) and (B) is disposed on a single nucleic acid molecule; or
ii) a sequence encoding (A) is disposed on a first nucleic acid molecule, and a sequence encoding (B) is disposed on a second nucleic acid molecule.

In instances, a sequence encoding (A) and (B) are present on a single nucleic acid molecule, and are transcribed as a single transcription product, and are configured as follows:
a promoter, is operably linked to (A), (B), and (D), wherein (D) encodes a cleavable peptide, and element (D) is disposed between (A) and (B).

In other instances, a sequence encoding (A) and a sequence encoding (B) are present on a single nucleic acid molecule, are transcribed as a single transcription product, and are configured as follows:
a promoter is operably linked to (A), (B), and (D), wherein element (D) encodes an IRES, and element (D) is disposed between (A) and (B)

In instances, the nucleic acid further comprises (iii) a sequence that encodes an intracellular signaling domain, e.g., adaptor molecule, that interacts with said NKR-CAR.

In instances, the intracellular signaling domain, e.g., adaptor molecule, produces an inhibiting signal.

In instances, the (ii) sequence encoding a NKR-CAR and (iii) a sequence encoding an intracellular signaling domain, e.g., adaptor molecule, are disposed on the same nucleic acid molecule, e.g., the same vector, e.g., the same viral vector, e.g., a lenti-viral vector.

In some instances, one of (ii) sequence encoding a NKR-CAR and (iii) a sequence encoding an intracellular signaling domain, e.g., adaptor molecule, is disposed on a first nucleic acid molecule, e.g., a first vector, e.g., a viral vector, e.g., a lenti-viral vector, and the other is disposed on a second nucleic acid molecule, e.g., a second vector, e.g., a viral vector, e.g., a lenti-viral vector.

In instances, the nucleic acid comprises a DNA or RNA, e.g., mRNA, sequence.

In another aspect, also disclosed herein is a vector system comprising a nucleic acid described herein. In embodiments, vector system comprises a DNA, a RNA, a plasmid, a lentivirus, an adenoviral vector, or a retrovirus vector.

In an aspect, disclosed herein is a method of treating a subject with a disease associated with a tumor antigen, e.g., a method of providing an anti-tumor immunity in a subject, comprising administering to the subject an effective amount of a RCAR/NKR-CAR cell described herein.

In instances, the disease comprises a proliferative disease, a precancerous condition, and a noncancer related indication associated with the expression of a tumor antigen. In one instance, the disease associated with expression of a tumor antigen is cancer, e.g., a cancer described herein. In one instance, the cancer is a solid tumor, e.g., a solid tumor described herein, e.g., mesothelioma (e.g., malignant pleural mesothelioma), lung cancer (e.g., non-small cell lung cancer, small cell lung cancer, squamous cell lung cancer, or large cell lung cancer), pancreatic cancer (e.g., pancreatic ductal adenocarcinoma), ovarian cancer, colorectal cancer and bladder cancer or any combination thereof. In one instance, the disease is pancreatic cancer, e.g., metastatic pancreatic ductal adenocarcinoma (PDA), e.g., in a subject who has progressed on at least one prior standard therapy. In one instance, the disease is mesothelioma (e.g., malignant pleural mesothelioma), e.g., in a subject who has progressed on at least one prior standard therapy. In one instance, the disease is ovarian cancer, e.g., serous epithelial ovarian cancer, e.g., in a subject who has progressed after at least one prior regimen of standard therapy.

In an instance, the cancer is selected from glioblastoma multiforme (GBM), anaplastic astrocytoma, giant cell glioblastoma, gliosarcoma, anaplastic oligodendroglioma, anaplastic ependymoma, choroid plexus carcinoma, anaplastic ganglioglioma, pineoblastoma, medulloepithelioma, ependymoblastoma, medulloblastoma, supratentorial primitive neuroectodermal tumor, and atypical teratoid/rhabdoid tumor, non-small cell lung carcinomas, lung, breast, prostate, ovarian, colorectal and bladder carcinoma.

In some instances, the cancer is B-cell acute lymphoid leukemia ("BALL"), T-cell acute lymphoid leukemia ("TALL"), acute lymphoid leukemia (ALL), acute myelogenous leukemia (AML); one or more chronic leukemias including but not limited to chronic myelogenous leukemia (CML), chronic lymphocytic leukemia (CLL); additional hematologic cancers or hematologic conditions including, but not limited to B cell prolymphocytic leukemia, blastic plasmacytoid dendritic cell neoplasm, Burkitt's lymphoma, diffuse large B cell lymphoma, follicular lymphoma, hairy cell leukemia, small cell- or a large cell-follicular lymphoma, malignant lymphoproliferative conditions, MALT lymphoma, mantle cell lymphoma, Marginal zone lymphoma, multiple myeloma, myelodysplasia and myelodysplastic syndrome, non-Hodgkin's lymphoma, plasmablastic lymphoma, plasmacytoid dendritic cell neoplasm, Waldenstrom macroglobulinemia.

In instances, the RCAR/NKR-CAR cell is an autologous T cell. For example, the RCAR/NKR-CAR cell is an allogeneic T cell.

In instances, the RCAR/NKR-CAR cell is selected from: an autologous NK cell; and an allogeneic NK cell.

In instances, the subject is a human.

In instances, the method comprises treating the subject for cancer.

In instances, the method comprises administering a dimerization molecule to the subject. For example, the RCAR/NKR-CAR comprises an FKBP-FRB based dimerization switch, and the method comprises administering a dimerization molecule comprising an mTOR inhibitor, e.g., RAD001.

In another aspect, also featured herein is a method of providing a RCAR/NKR-CAR cell described herein comprising:
providing an immune effector cell to a recipient entity; and
receiving from said entity, a RCAR/NKR-CAR cell derived from said immune effector cell, or a daughter cell thereof, wherein the RCAR/NKR-CAR comprises:
   a) a regulatable CAR (RCAR) and a NKR-CAR;
   b) a nucleic acid encoding a RCAR and a NKR-CAR;
   c) a first nucleic acid encoding a RCAR and a second nucleic acid encoding a NKR-CAR; or
   d) a vector system comprising a nucleic acid encoding a RCAR and a NKR-CAR or comprising a first nucleic acid encoding a RCAR and a second nucleic acid encoding a NKR-CAR.

In instances, the entity inserted into said immune effector cell or a daughter cell thereof, one or the following: a) a nucleic acid encoding a RCAR and a NKR-CAR; or b) a first nucleic acid encoding a RCAR and a second nucleic acid encoding a NKR-CAR.

In instances, the method further comprises administering said RCAR and said NKR-CAR to a subject.

In an aspect, also featured herein is a method of providing an RCAR/NKR-CAR cell comprising:
receiving from an entity an immune effector cell from a human; inserting into said immune effector cell or a daughter cell thereof one of the following: a) a nucleic acid encoding a RCAR and a NKR-CAR, or b) a first nucleic acid encoding a RCAR and a second nucleic acid encoding a NKR-CAR, to form a RCAR/NKR-CAR cell; and, optionally, providing said RCAR/NKR-CAR cell to said entity.

In an instance, the entity is a laboratory, hospital, or a hospital provider.

Unless otherwise indicated, when members or elements of a CAR, e.g., RCAR, RNKR-CAR, or NKR-CAR are described herein, the order can be as provided, but other orders are included as well. In other words, in an embodiment, the order is as set out in the text, but in other embodiments, the order can be different.

Based on the disclosure herein, the invention as defined in the attached claims is provided.

### BRIEF DESCRIPTION OF DRAWINGS

The drawings are first briefly described.

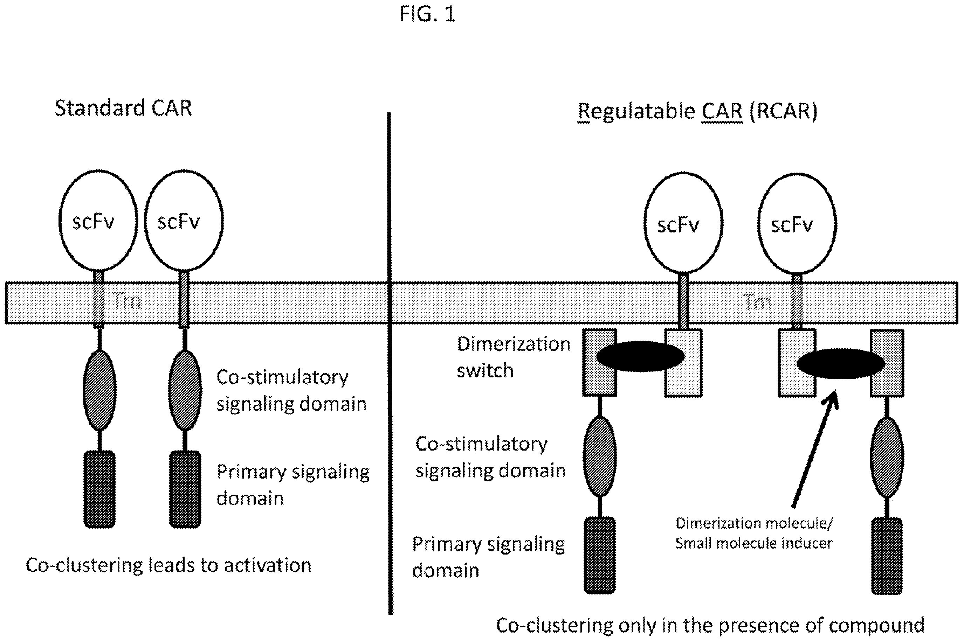

FIG. 1 depicts the structures of a standard CAR compared to a regulatable CAR (RCAR).
FIG. 2 depicts RCARs having a FKBP/FRB heterodimerization induced by a rapamycin analogue. The antigen binding domain may be an scFv, e.g., that targets EGFRvIII. Switch A and switch B show the FKBP and FRB switch domains in different orientations with respect to the antigen binding member and the intracellular signaling member.
FIG. 3 depicts a RCAR having a GyrB dimerization switch induced by Coumermycin. The antigen binding domain may be an scFv, e.g., that targets EGFRvIII. The switch domains are GyrB subunits.
FIG. 4 depicts RCARs having a GAI/GID1 dimerization switch induced by Gibberellin. The antigen binding domain may be an scFv, e.g., that targets EGFRvIII. Switch A and switch B show the GAI and G1D1 switch domains in different orientations with respect to the antigen binding member and the intracellular signaling member.
FIG. 5 depicts an RCAR having an extracellular switch induced by small molecule drug. The extracellular dimerization switch can be an FKBP/FRB switch, a GyrB/GyrB switch, or a GAI/G1D1 switch.
FIG. 6 depicts RCARs having extracellular switches induced by antibody molecule, non-antibody scaffold, or polypeptide. In the RCAR system on the left, the two heterodimerization switch domains are brought together by a dual specific antibody. In the RCAR system on the right, two intracellular signaling members can be brought together by a mono specific antibody to initiate CAR signaling.

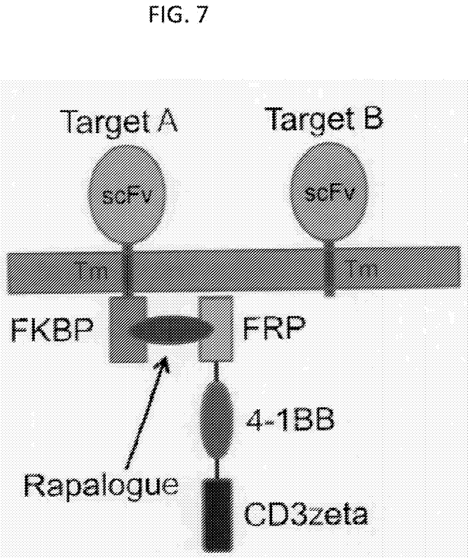

FIG. 7 depicts a dual RCAR system, where two different targets (Target A and Target B) can be targeted.
FIG. 8 provides immune effector cells that can be engineered to express RCARs.
FIG. 9 depicts an RCAR comprising an auxiliary antigen binding member, wherein the auxiliary antigen binding member targets a second antigen (e.g., target B) that is different from the antigen targeted by the antigen binding member comprising a switch domain (e.g., target A). The auxiliary antigen binding member does not comprise a switch domain, and does not dimerize with the intracellular signaling member of the RCAR.
FIG. 10 depicts RCARs that redirect an inhibitory pathway.
FIG. 11 depicts RCARs having context dependent elements, where the switchable receptor can be a redirected switchable co-inhibitory receptor, e.g., any one listed in Table 4, or co-stimulatory receptor, e.g., any one listed in Table 5. Optimal pairs of co-inhibitory receptors or co-stimulatory receptors and targets can be dependent on cancer types, cancer stages, donor types, etc.
FIG. 12A and 12B depict activation as seen with RCAR cells expressing RCAR constructs as shown in FIG. 2. A standard EGFRvIII CART was used for positive control. RCARs were incubated with no heterodimerization molecule (no HD) or with 500nM heterodimerization molecule (HD), where the heterodimerization molecule was AP21967 (FIG. 12A). A dose-response assay was performed using varying concentrations of the heterodimerization molecule A/C heterodimerizer (FIG. 12B). The RCARs tested contained scFv domain that targeted EGFRvIII (left bar in each set) or IgG1 for control (right bar in each set).
FIG. 13 depicts RCARs having a halo/snap tag dimerization switch. The antigen binding domain may be an scFv, e.g., that targets EGFRvIII. Switch A and switch B show the Halo-tag and Snap-tag switch domains in different orientations with respect to the antigen binding member and the intracellular signaling member.
FIG. 14A and 14B depict activation by soluble antibodies and activation by soluble antibodies + 2^{nd} antibodies (second order dimerization switches).
FIG. 15 depicts an RCAR having an extracellular switch and a multi-valent dimerization molecule.
FIG. 16A, 16B, 16C, 16D, and 16E depict vectors for expressing a RCAR.
FIG. 17A and 17B depicts activation of a PD1 CAR and a PD1 RCAR. FIG. 17A shows the results from a NFAT inducible promoter driven luciferase activity of a PD1 CAR as compared to the control treatment by IgG1-Fc. FIG. 17B shows the results from NFAT inducible promoter driven luciferase activity of a PD1 RCAR which include PD1-ECD - TM-FRB and FKBP-4 1BB-CD3 zeta as compared to the control treatment by IgG1-Fc.
FIG. 18 demonstrates a dose response with AP21967 treatment. The bar on the left for each tested dosage is the PD1 RCAR, while the bar the right for each tested dosage is PD1 CAR control.
FIG. 19 the activity of an RCAR having an FKBP-FRB based extracellular switch, e.g., as shown in FIG. 5.
FIG. 20A and 20B shows the activity of an RCAR having a GyrB-GyrB based intracellular switch, e.g., as shown in FIG. 3.
FIG. 21A and 21B shows the activity of an RCAR having a GAI-GID1 based intracellular switch, e.g., as shown in FIG. 4.
FIG. 22 depicts the arrangement of RCAR elements on a single nucleic acid vector. Constructs 143774 and 143775 utilize different IRES (e.g., IRES EV71 or IRES EMCV) between the antigen binding member (e.g., scFv linked to FKBP) and the intracellular binding member (e.g., FRB linked to 41BB/CD3zeta). Constructs 143776 and 143777 utilize two different promoters (e.g., CMV min or EF1 min) between the antigen binding member (e.g., scFv linked to FKBP) and the intracellular binding member (e.g., FRB linked to 41BB/CD3zeta).
FIG. 23 shows RCAR activity from single vector encoded RCARs, where the arrangement of the single vectors are shown in FIG. 22.
FIG. 24A and 24B show activation of a CD19 RCAR by different heterodimerization molecule, RAD001 (FIG. 24A) or rapamycin (FIG. 24B) at the indicated nM concentrations. The RCAR is encoded by the construct 143775, as shown in FIG. 22.
FIG. 25A and 25B show activation of a CD19 RCAR by different heterodimerization molecule, RAD001 (FIG. 24A) or rapamycin (FIG. 24B) at the indicated nM concentrations. The RCAR is encoded by the construct 143775, as shown in FIG. 22.
FIG. 26 depicts a half RCAR structure having a costimulatory signaling domain on the antigen binding member. The costimulatory signaling domain can be any of those listed, or from Table 2.
FIG. 27 is a graphic representation FRAP binding with RAD001. The dotted area represents the pocket that binds to RAD001. Residues that are in proximity of RAD001 or mediate interaction with RAD001 are circled and the amino acid position on FRAP.
FIG. 28 shows the amino acid distribution of the NKK library used to generate libraries of FRB mutants. The different amino acids are listed on the x-axis, and the percent represented in the library is shown on the y-axis.
FIG. 29A and 29B show the protein expression results from each of the different mutant FRB libraries. The 11 different mutant FRB libraries are listed on the x-axis. In FIG. 29A, the y-axis shows the percent of wells expressing the mutant FRB. In Fig. 29B, the y-axis shows the average protein concentration determined for each library.
FIG. 30A, 30B, 30C, 30D, and 30E show the binding curves for the EC50 competition binding assay for FRB mutants: E2032L (FIG. 30A), E2032I (FIG. 30B), T2098L (FIG. 30C), E2032L, T2098L (FIG. 30D), and E2032I, T2098L (FIG. 30E).
FIG. 31A, 31B, and 31C show the binding curves for the EC50 direct binding assay for fRB mutants: E2032L (FIG. 31A), E2032I (FIG. 31B), and T2098L (FIG. 31C).
FIG. 32 depicts human PD1 knockdown at 24, 48 and 72 hours, by the human PD1 shRNA sequences indicated on the x-axis. Knockdown is represented by percentage of PD1 transcript remaining (y-axis). Human PD1 shRNA sequences are provided in Table 19.
FIG. 33 depicts mouse PD1 knockdown at 24 and 48 hours, by the mouse PD1 shRNA sequences indicated on the x-axis. Knockdown is represented by percentage of PD1 transcript remaining (y-axis). Mouse PD1 shRNA sequences are provided in Table 18.
FIG. 34A and 34B are schematic representations of various RCAR constructs with extracellular switches evaluated in FIG. 35. Half RCARs with the extracellular switch are shown in FIG. 34A, with the FKBP and FRB switch domains in two different orientations. Full RCAR full switches with the extracellular switch are shown in FIG. 34B, with the FKBP and FRB switch domains in two different orientations.
FIG. 35A, 35B, 35C, and 35D show the activation of the RCAR half switch constructs of FIG. 34A and 34B with varying concentrations of RAD001. FIG. 35A and 35B show results of activation of the RCAR half switch with the switch domains in both orientations. FIG. 35C and 35D show results of activation of the RCAR full switch with the switch domains in both orientations. NFAT activation is represented by luminescence detected by Luciferase One Glo (y-axis) and the different RAD001 concentrations are listed on the x-axis.
FIG. 36A and 36B show the half RCAR constructs with an intracellular switch. FIG. 36A shows a schematic representation of the half RCAR constructs. FIG. 36B shows the activation of the half RCARs with different co stimulatory signaling domains in the presence or absence of RAD001.
FIG. 37A and 37B show the activation of two half RCAR constructs with varying concentrations of RAD001. Activation of half RCAR with a CD28 costimulatory signaling domain is shown in FIG. 37A. Activation of half RCAR with a 41BB costimulatory signaling domain is shown in FIG. 37B. NFAT activation is represented by luminescence detected by Luciferase One Glo (y-axis) and the different RAD001 concentrations are listed on the x-axis.
FIG. 38 is a panel of images that shows the expression of half RCARs on transduced primary T cells. Numbers indicate the percentage of half RCAR-positive T cells (%) and the mean fluorescence intensity of the CAR-positive population (10³ GeoMean).
FIG. 39 shows the cytotoxicity of half RCAR expressing, transduced T cells. RCARTs were co-cultured with Nalm6 cells expressing luciferase in the presence of different concentration of RAD001.
FIG. 40 shows the proliferation of half RCAR expressing, transduced T cells. RCARTs were co-cultured with Nalm6 cells in the presence of different concentration of RAD001. The number of RCAR-positive CD3-positive T cells was assessed after 4 days of co-culture.
FIG. 41 shows the secretion of IFNγ by CAR expressing, transduced T cells. CARTs were co-cultured with Nalm6 cells in the presence of different concentration of RAD001. The concentration of IFNγ in the cell culture supernatant was determined after 20h of cocultivation.
FIG. 42 shows the activation of RCAR with a covalent halo/snap tag switch. NFAT activation is represented by luminescence detected by Luciferase One Glo (y-axis) and the different NVP-HAL421 concentrations are listed on the x-axis.
FIG. 43 shows the activation of RCAR with a covalent halo/snap tag switch, in the presence of the indicated NVP-HAL421 concentrations. NFAT activation is represented by luminescence detected by Luciferase One Glo (y-axis) and the different NVP-HAL421 concentrations are listed on the x-axis.

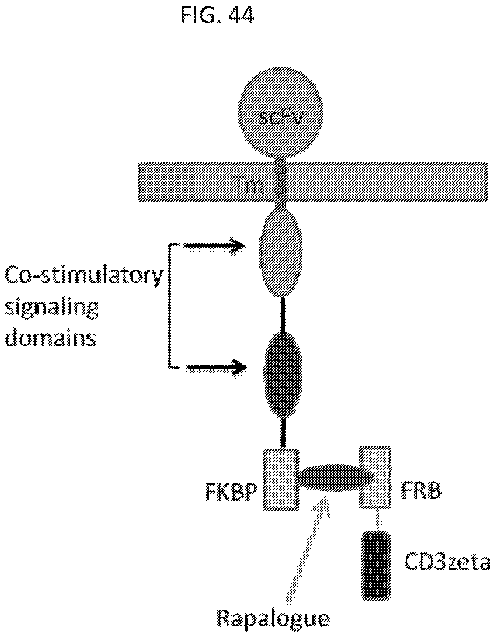

FIG. 44 shows a double half RCAR construct, where two costimulatory signaling domains are present on the antigen binding member.
FIG. 45 shows a universal CAR construct comprising an extracellular dimerization switch, where the antigen binding member comprises a switch domain, but does not comprise a transmembrane domain or membrane anchor.
FIG. 46 shows the structure of an RCAR comprising a an antigen binding member comprising an antigen binding domain (scFv), a transmembrane domain (Tm), and a first switch domain, and an intracellular signaling member comprising a transmembrane domain (Tm), a second switch domain, a co-stimulatory signaling domain (41BB), and a primary intracellular signaling domain (CD3zeta).
FIG. 47A and 47B shows two structures of RCAR comprising a multi switch. In FIG. 47A, the antigen binding member comprises a plurality of first switch domains, e.g., a FKBP switch domain, while the intracellular signaling member comprises a plurality of second switch domains, e.g., a FRB switch domain. The bracketed switch domains represent 1 or more additional switch domains that comprise the multi switch. In FIG. 47B, the antigen binding member comprises a first switch domain, e.g., a FKBP switch domain, and a second switch domain, e.g., a FRB switch domain, and the intracellular binding member comprises a second switch domain, e.g., a FRB switch domain, and a first switch domain, e.g., a FKBP switch domain.
FIG. 48A, 48B, 48C, and 48D shows four configurations of RCAR constructs that can regulate proliferation capacity. The RCAR comprises an antigen binding member comprising an antigen binding domain (e.g., scFv), a transmembrane domain, and a primary intracellular signaling domain (e.g., CD3zeta). The two intracellular signaling members both comprise at least one switch domain, e.g., FKBP and FRB switch domains, and two costimulatory domains, e.g., 2 costimulatory signaling domain 1, or costimulatory signaling domain 1 and costimulatory signaling domain 2, where the costimulatory domains 1 and 2 is selected from 4-1BB, OX40, CD27, CD28, and ICOS. The bracketed switch domains represent 1 or more additional switch domains that comprise the multi switch. In FIG. 48A, the two intracellular signaling members comprise a transmembrane domain and one or more intracellular switch domains. In FIG. 48B, the two intracellular signaling members comprise a membrane anchor, e.g., myristoylation, and one or more intracellular switch domains. In FIG. 48C, the two intracellular signaling members comprise one or more extracellular switch domains and a transmembrane domain. In FIG. 48D, the two intracellular signaling members comprise one or more intracellular switch domains, and does not comprise a transmembrane domain or a membrane anchor.
FIG. 49 is a graphic representation showing NFAT activation of 9E10scFv-containing intracellular members with myc-tag multimer dimerization molecules. Single myc tag (mono-myc), dimer myc tag (di-myc), trime myc tag (tri-myc), tetramer myc tag (tetra-myc), and control (IgG1 Fc) was administered at 100 nm and 1 µM.
FIG. 50, comprising FIGs. 50A and 50B, is a series of schematics showing the structure of naturally occurring inhibitory and activating KIRs (FIG. 50A) and a scFv-based activating KIR-CAR (FIG. 50B).
FIG. 51 is a schematic representation of the lentiviral vector used to deliver an activating KIR-based CAR in combination with the DAP12 signaling molecule.
FIG. 52 is a panel of images demonstrating that a mesothelin-specific actKIR-CARs can be efficiently expressed on the surface of primary human T cells. Human T cells were stimulated with anti-CD3/anti-CD28 microbeads and transduced with the indicated CAR or mock transduced and expanded ex vivo. The expression was detected using a biotinylated goat-anti-mouse F(ab)2-specific polyclonal IgG (Jackson Immunologics) followed by staining with streptavidin-PE.

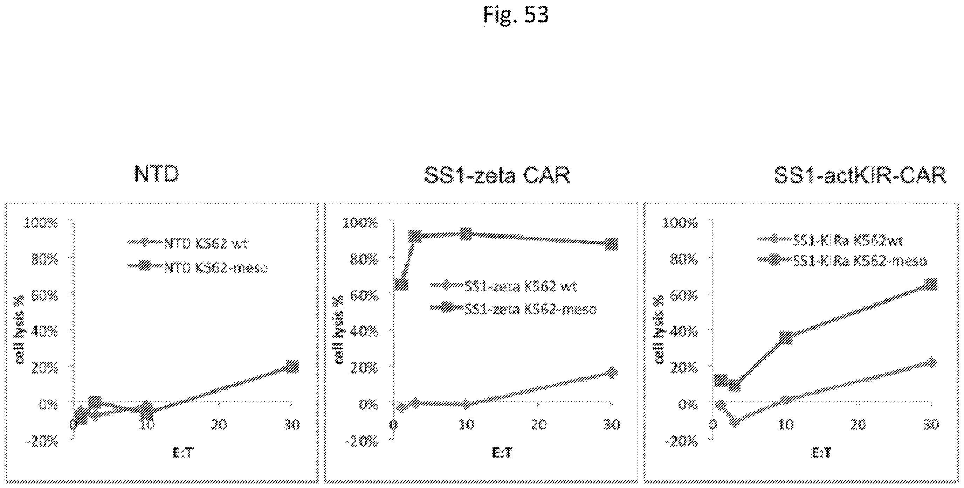

FIG. 53 is a panel of graphs demonstrating that T cells expressing the SS1 actKIR-CAR exhibited cytotoxic activity towards target K562 cells engineered to express the mesothelin ligand (KT-meso). Human T cells were stimulated with anti-CD3/anti-CD28 microbeads, transduced with the indicated CAR or mock transduced and expanded ex vivo. 10⁵ CFSE-labeled K562 cells expressing mesothelin (KT-meso) or wild-type control K562 were incubated with varying ratios of CAR-expressing T cells for 16 hours at 37 °C, 5% CO₂. The K562 target cells were then enumerated by flow cytometry using countbright beads and a viability stain (7AAD). The percentage of K562 cells lysed (percent lysis) was calculated by subtracting the number of viable target cells remaining after incubation with effector T cells from the number of viable K562 remaining after overnight culture without effector T cells, and then dividing by the number of viable K562 remaining after overnight culture without effector T cells.

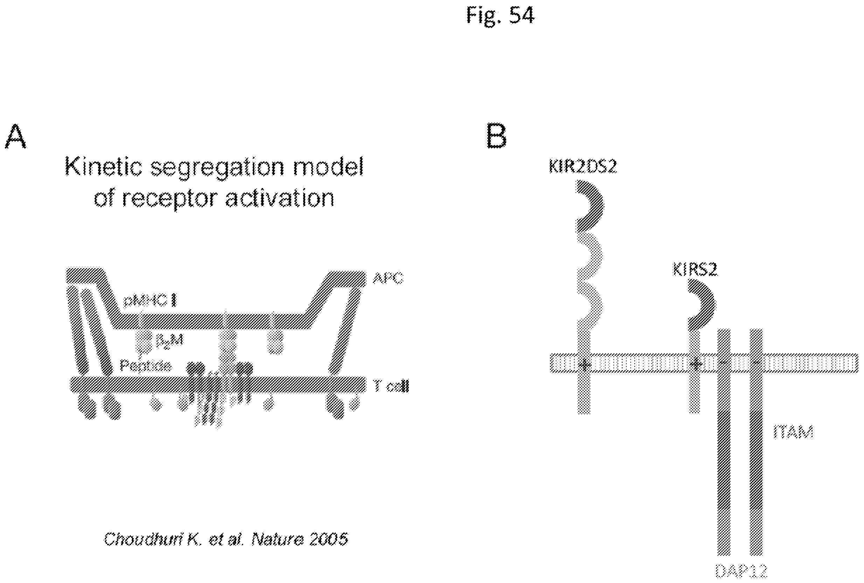

FIG. 54, comprising FIG. 54A and 54B, is a series of schematics showing an activating KIR CAR in which the KIR2DS2 hinge was removed (KIR2S CAR). Based upon the kinetic segregation model of TCR activation diagrammed in FIG. 54A, it is believed that the mesothelin-specific SS1 KIR CAR has a hinge that is too long to permit appropriate segregation. Therefore making the mesothelin-specific KIR CAR hinge shorter is believed to improve the function. For example, FIGs. 54A-B depict optimization of a KIR-CAR (e.g., mesothelin specific KIR-CAR) by varying the length of the receptor ectodomain. FIG. 54B is a schematic showing that the SS1 scFv was fused to the KIR transmembrane domain without the two Ig-like domains from KIR2DS2 as the hinge.

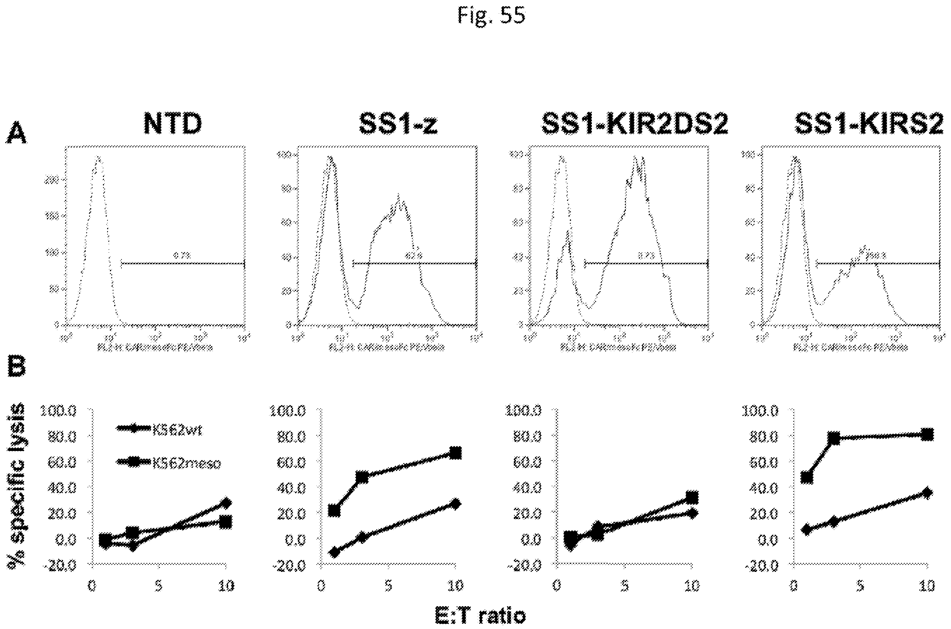

FIG. 55, comprising FIGs. 55A and 55B, is a series of images demonstrating that SS1 scFv based KIRS2 CAR exhibits enhanced cytolytic activity towards mesothelin-expressing target cells compared with the CAR formed by fusion of the SS1 scFv onto full length wildtype KIR2DS2. Primary human T cells were stimulated with CD3/28 microbeads followed by lentiviral transduction with either the SS1-KIR2DS2 activating KIR-CAR, SS1-KIRS2 activating KIR CAR, the SS1-zeta CAR. Mock non-transduced T cells (NTD) were used as a control. The T cells were expanded until the end of log-phase growth. The surface expression of the SS1-specific CARs was determined by flow cytometry using a biotinylated goat anti-mouse F(ab)2 specific polyclonal antibody followed by streptavidin-PE detection as shown in FIG. 55A. Shown in FIG. 55B, K562 target cells with or without mesothelin and stained with CFSE were mixed with the effector T cells characterized in FIG. 55A as indicated using varying effector T cell to target ratios ranging from 10:1 to 1:1. Target K562 cell lysis was assessed using flow cytometry to determine the % of viable CFSE+ cells as described for FIG. 53. Data shown is the calculated % target cell lysis compared against target cells without effector cells.

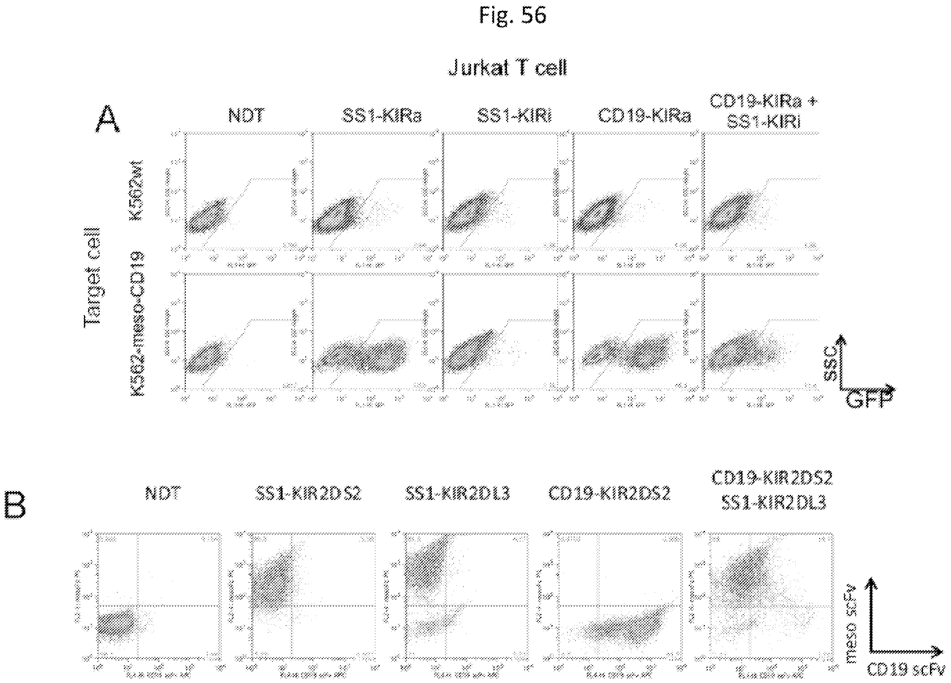

FIG. 56, comprising FIGs. 56A and 56B, is a series of images showing co-expression of the CD19 actKIR-CAR and the SS1 inhKIR-CAR. Jurkat NFAT-GFP reporter cells were transduced with the indicated KIR CAR or non-transduced (NDT) and mixed 1:1 with target cells with or without the CD19 and mesothelin antigens as indicated. Results shows GFP expression at 24 hours following mixing of Jurkat and Target cells (FIG. 56A). FIG. 56B shows surface expression of the mesothelin and CD19 idiotypes as determined by staining with a mesothelin-Fc fusion protein and a monoclonal antibody specific for the FMC63 anti-CD19 scFv idiotype.

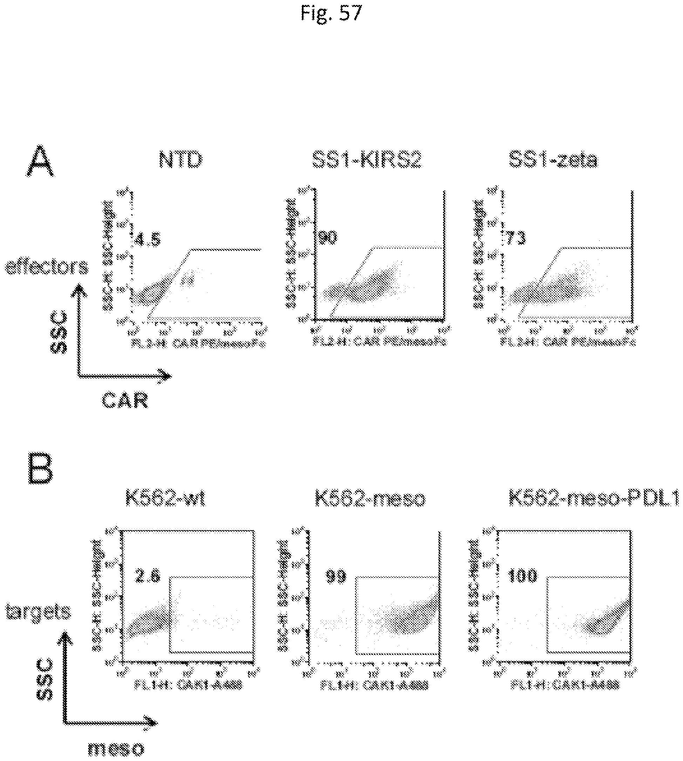

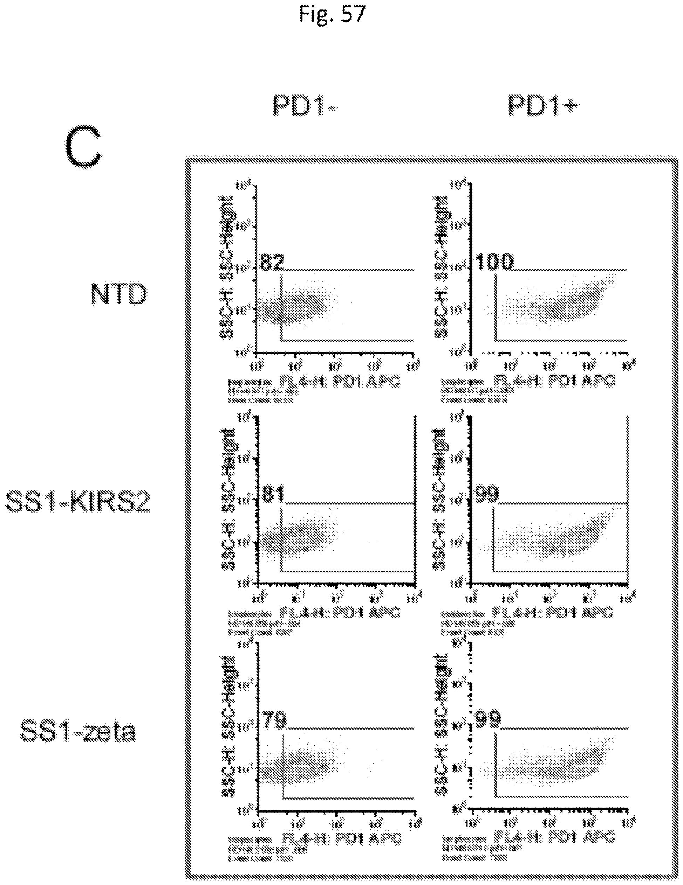

FIG. 57, comprising FIGs. 57A through 57C, is a series of images demonstrating co-expression of wild-type PD-1 with both an activating KIR-based CAR or TCR-zeta based CAR targeting mesothelin. Primary human T cells were stimulated with CD3/28 microbeads followed by lentiviral transduction with either the SS1-KIRS2 activating KIR CAR or the SS1-zeta CAR. Mock non-transduced cells (NTD) were used as a negative control. The T cells were expanded over 9 days, and surface CAR expression was determined by staining with mesothelin-Fc followed by a goat-anti-human Fc specific antibody conjugated to PE (FIG. 57A). K562 cell lines (wildtype [wt], mesothelin expressing [meso] or mesothelin and PD-L1 co-expressing [meso-PDLl]) were stained using the CAK1 anti-mesothelin specific monoclonal antibody to confirm mesothelin expression on the targets (FIG. 57B). The primary human T cells transduced as shown in FIG. 57A were electroporated with 10 ug of in vitro transcribed RNA encoding wild-type PD1 using a BTX ECM830 electroporator (PD1+) or mock transfected (PD1-). The surface expression of PD-1 was expressed using an anti-PDl monoclonal conjugated to APC (FIG. 57C).

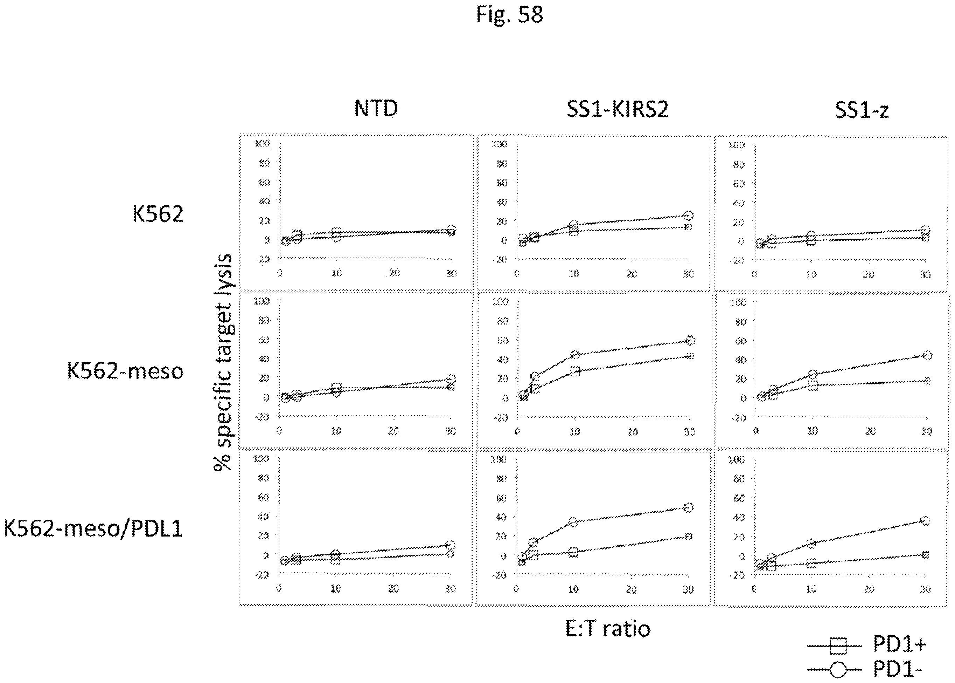

FIG. 58 is a panel of graphs demonstrating that the combination of co-expressing wild-type PD-1 with both an activating KIR-based CAR and TCR-zeta based CAR targeting mesothelin led to PD-1 ligand 1 (PDL-1) dependent inhibition of the mesothelin-specific activating KIR-CAR cytotoxicity. Primary human T cells were stimulated with CD3/28 microbeads followed by lentiviral transduction with either the SS1-KIRS2 activating KIR CAR, the SS1-zeta CAR or mock transduced (NTD). The T cells were expanded over 9 days followed by electroporation of 5x10⁶ T cells with 10 ug of in vitro transcribed RNA encoding wild-type PD1 using a BTX ECM830 electroporator (PD1+) or mock transfected (PD1-). The surface expression of the SS1-specific CAR and PD-1 was determined as shown in FIG. 57. K562 target cells with either no mesothelin or expressing mesothelin with or without PDL-1 were mixed with the different T cells conditions as indicated using varying effector T cell to target ratios of 30:1 to 1:1 as shown. Target K562 cell lysis was assessed using a calcein AM dye method to quantify the remaining viable cells following 4 hours of incubation. Data shown is calculated % target cell lysis compared against target cells without effector cells.

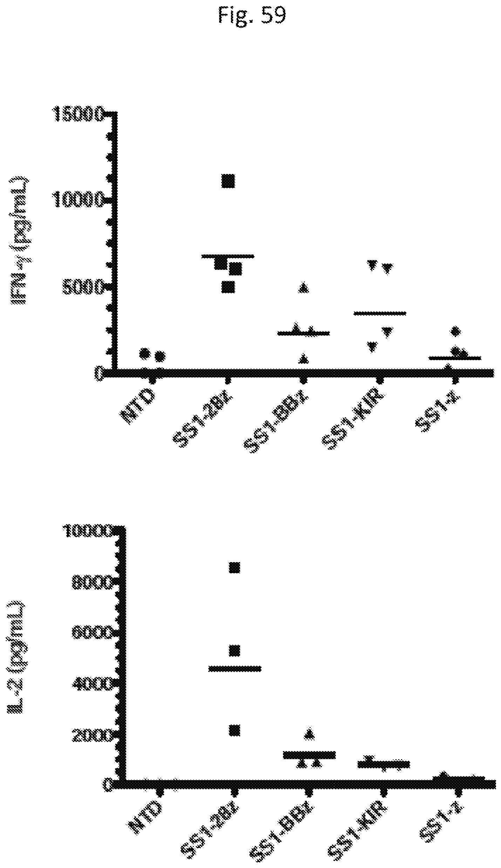

FIG. 59 is a set of graphs demonstrating the interferon-gamma (IFN-γ) and interleukin-2 production by T cells from a donor expressing a mesothelin-specific activating KIR-based CAR (SS1-KIRS2) or TCR-zeta based CAR with or without a costimulatory domain (SS1-z, SS1-28z or SS1-BBz). Primary human T cells were simulated, followed by lentiviral transduction with the indicated activating KIR CAR or TCR-zeta based CAR. Following expansion, the transduced T cells were mixed with K562 (Kwt) or K562-mesothelin cells (Kmeso) at a ratio of 2:1. Cytokine concentrations were determined in supernatants following 24 hours of stimulation by ELISA for the indicated cytokines in multiple independent donors. Repeated measure ANOVA demonstrated a significant CAR effect on IFN-γ (p=0.002) and IL-2 (p=0.0156). SS1-KIRS2/DAP12 (SS1KIR) vs. mock for IFN-γ (posthoc paired t-test, p = 0.0162). SS1-KIR vs. SS1-28z for IL-2 (post-hoc paired t-test, p=0.0408)
FIG. 60 is a heat map of cytokine concentrations in supernatants as assessed by a multiplex luminex-based immunoassay (Cytokine Human 10-Plex Panel, Life Technologies). A heatmap of relative concentration after normalization across donor and conditions to the lowest concentration for each cytokine was generated using the heatmap package implement in R statistical software.

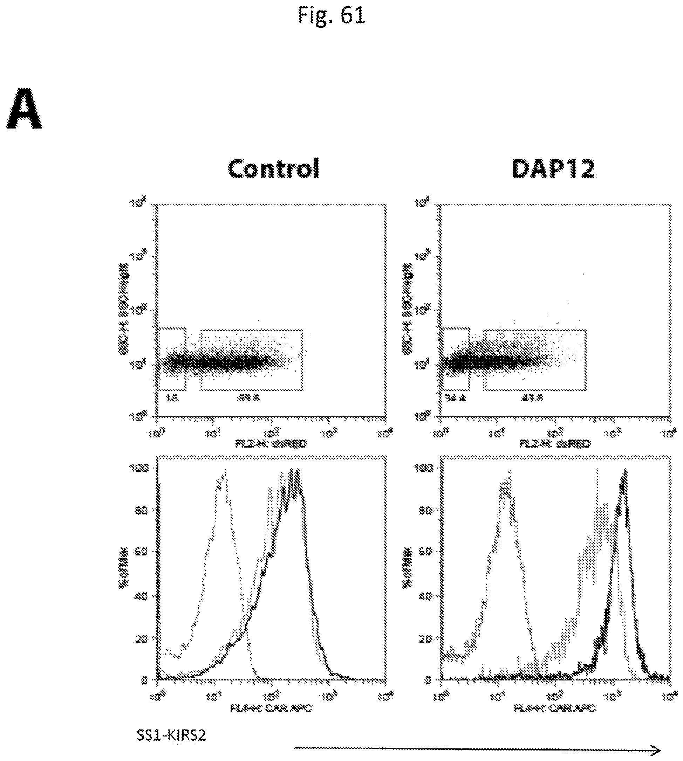

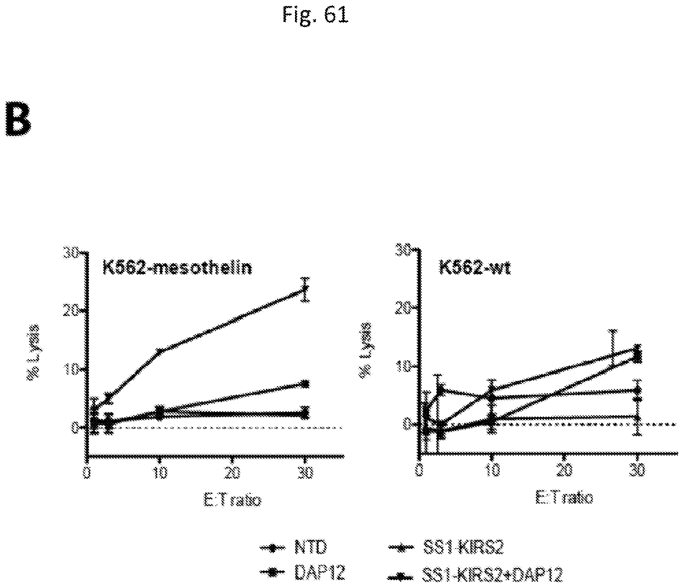

FIG. 61, comprising FIGs. 61A-61B, depicts construction of a mesothelin-specific KIR-based chimeric antigen receptor (KIR-CAR) engineered T cell with robust cytotoxic activity. Primary human T cells were stimulated with CD3/28 microbeads followed by transduction with a lentiviral vector expressing either GFP and dsRed (Control) or DAP12 and dsRed (DAP12). The cells were expanded ex vivo until the end of log phase growth. 5x10⁶ T cells from each transduced population were electroporated with 10 ug of in vitro transcribed RNA encoding SS1-KIRS2 using a BTX ECM830 electroporator. The expression of both dsRed and SS1-KIRS2 was assessed by flow cytometry with the SS1-KIRS2 detected using a biotinylated goat anti-mouse F(ab)2 specific polyclonal antibody followed by streptavidin-PE. The upper panel of FIG. 61A shows the gating strategy for identification of T cells expressing dsRed, which were then analyzed for SS1-KIRS2 expression as shown in the lower portion of the panel. FIG. 61B shows the ability of the cells characterized in FIG. 61A to mediate cytotoxicity against wild-type K562 cells (K562-wt) or K562 cells that express mesothelin (K562-mesothelin) as assessed using a 4-hr ⁵¹Cr-release assay.

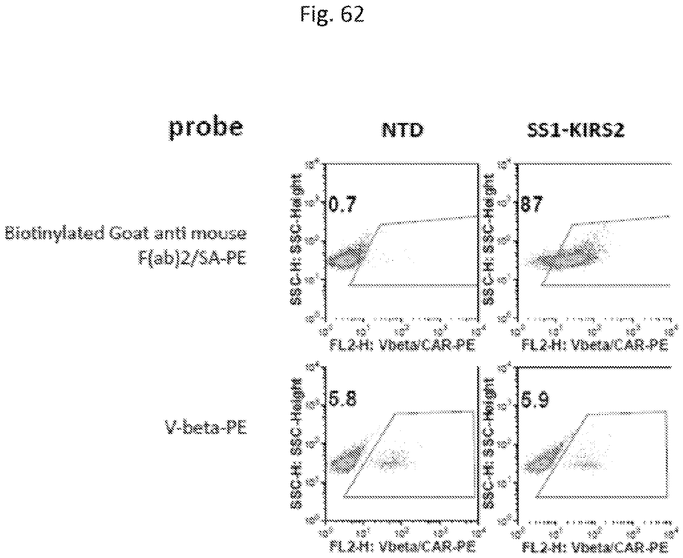

FIG. 62 is a panel of images that shows that the expression of an endogenous TCR is unaffected by SS1-KIRS2 and DAP12 expression. 5x10⁶ primary human T cells were electroporated with 10 ug of in vitro transcribed RNA encoding SS1-KIRS2 or mock transfected using a BTX ECM830 electroporator. After overnight incubation, the transfected T cells were stained for the expression of SS1-KIRS2 using a biotinylated goat anti-mouse F(ab)2 specific polyclonal antibody followed by streptavidin-PE. The expression of Vβ13.1 was assessed using a PE-conjugated monoclonal antibody specific to this Vβ chain of the TCR.

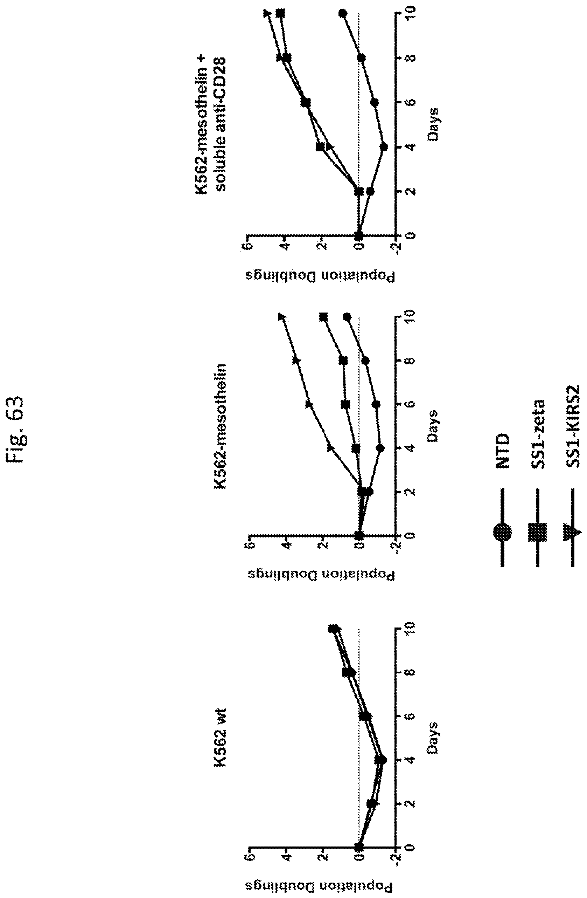

FIG. 63 is a set of graphs that illustrates the ability of a mesothelin-specific KIR-based CAR (SS1-KIRS2) to stimulate T cell proliferation that is antigen-dependent but independent of additional CD28 costimulation. Primary human T cells were stimulated with CD3/28 microbeads followed by lentiviral transduction of SS1-KIRS2 and DAP12 or the mesothelin-specific TCR-zeta CAR (SS1-zeta). Mock non-transduced cells (NTD) were used as a negative control. K562 target cells with either no mesothelin (K562 wt) or expressing mesothelin (K562-mesothelin) were mixed with the different T cells conditions as indicated at a 2:1 ratio of effector T cells to target cells. T cells stimulated with K562-mesothelin were further divided into a condition with or without a monoclonal anti-CD28 agonist antibody (clone 9.3) at 1 ug/mL. The number of viable T cells were enumerated by flow cytometry using bead-based counting at the indicated time points to calculate the number of population doublings following antigen stimulation.

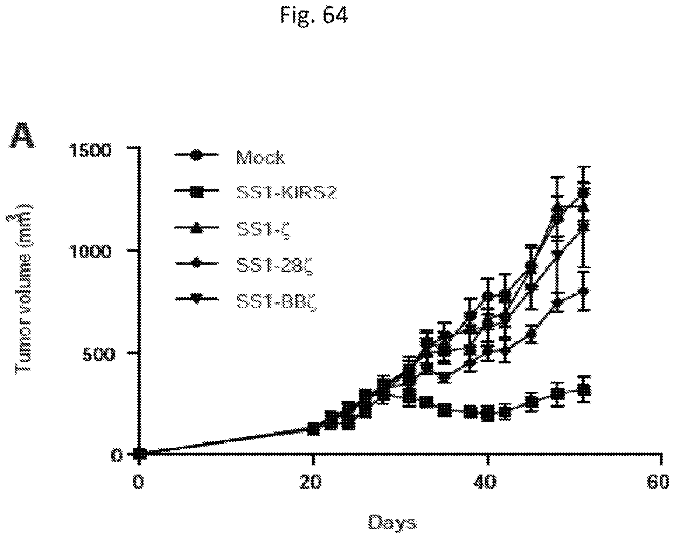

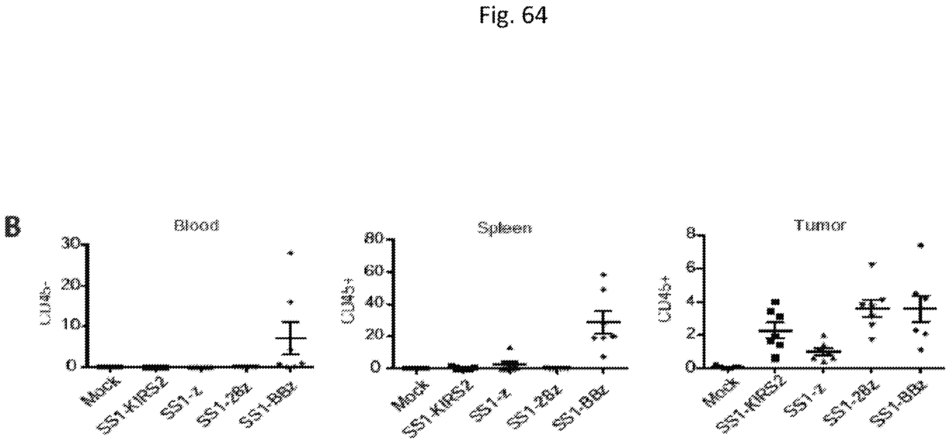

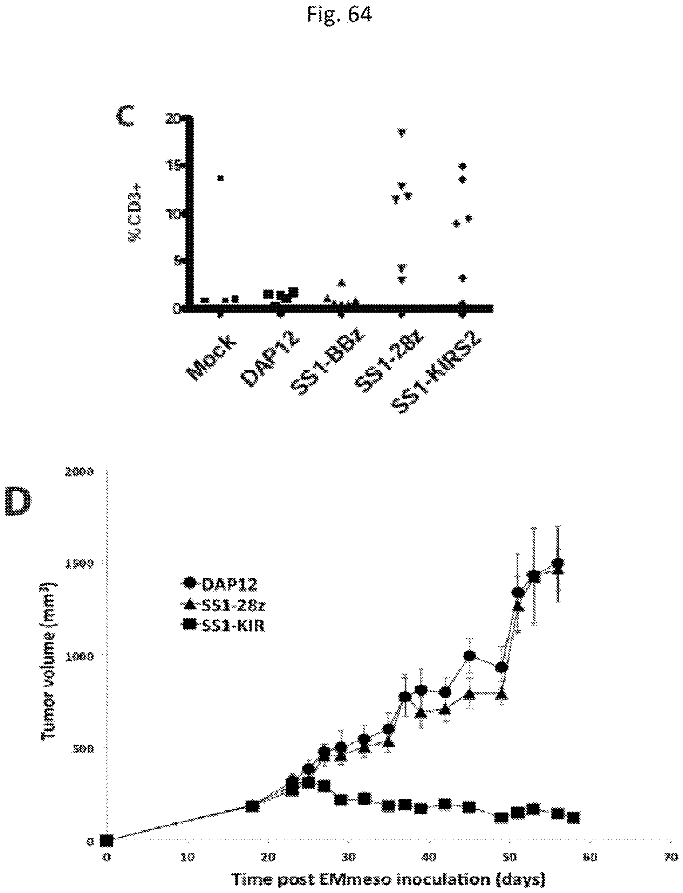

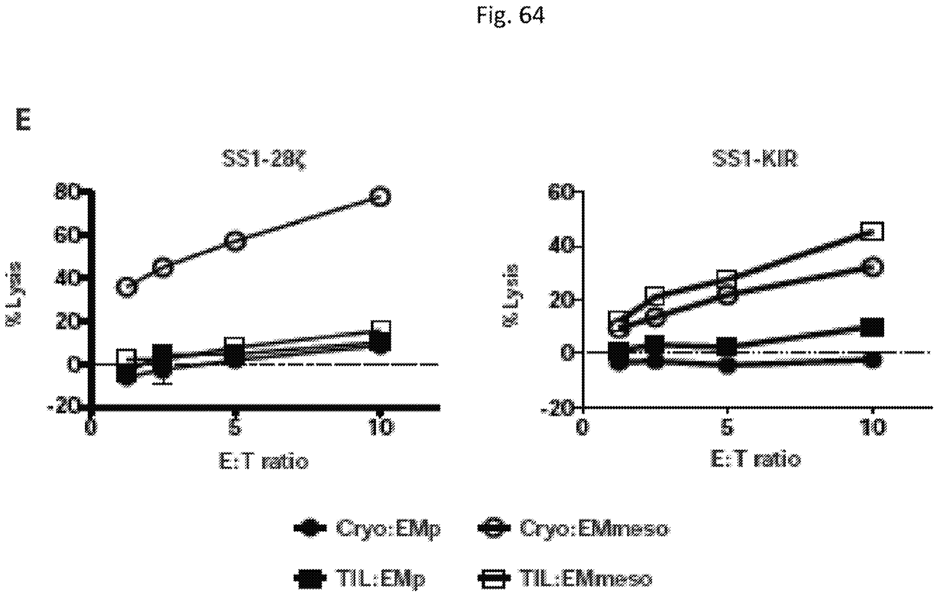

FIG. 64, comprising FIGs. 64A-64E, are a set of images that demonstrates that mesothelin-specific KIR-CAR modified T cells show enhanced anti-tumor activity *in vivo* compared with second generation TCR- ζ based CARs bearing CD28 or CD137 (4-1BB) costimulatory domains. FIG. 64A shows an experiment in which NOD-SCID-γ_{c}^{-/-} (NSG) mice were subcutaneously implanted with 2x10⁶ mesothelioma-derived cells expressing mesothelin (EM-meso cells). 20 days following tumor implantation, each animal was injected intravenously with 5x10⁶ T cells that were stimulated with anti-CD3/anti-CD28 stimulator beads followed lentiviral transduction with a series of CD3ζ-based CAR with or without a costimulatory domain (SS1-ζ, SS1-BBζ and 331-28ζ) or the mesothelin-specific KIR-based CARs, SS1-KIRS2 with DAP12. Mock transduced T cells (NTD) were used as a control. Tumor volume was measured by caliper at the indicated times (n = 7 mice per group). FIG. 64B shows that the *in vivo* activity of the KIR-CAR is independent of T cell engraftment in blood, spleen or tumor. The frequency of human CD45+ T cells was assessed at the end of the experiment by flow cytometry, and data are expressed as a percentage of total viable cells in the blood, spleen and tumor digest. FIG. 64C shows comparable frequencies of CD3+ TILs were observed in 331-28ζ and SS1-KIRS2/DAP12 CAR T cell treated groups. The same model as that shown in FIG. 64A was used. The frequency of CD3+ human lymphocytes in tumors at day 30 (10 days following CAR T infusion) was assessed by flow cytometry. FIG. 64D shows that DAP12-modified T cells require the mesothelin-specific KIR-based CAR for tumor eradication. The same model as that shown in FIG. 64A was used. 4 million T cells expressing DAP12 and dsRed (DAP12), SS1-28z or SS1-KIRS2 and DAP12 (SS1-KIRS2) were injected intravenously on day 20, and tumor volume was assessed over time via caliper measurement. The arrow indicates the time of TIL isolation used for functional and phenotypic analysis. FIG. 64E shows antigen-specific cytotoxic activity of TILs isolated from the mice described in FIG. 64D. Antigen-specific cytotoxicity was assessed by coculturing with firefly luciferase expressing EM-meso cells or EMp cells (parental EM cells lacking mesothelin expression) at indicated E:T ratios for 18 hours.

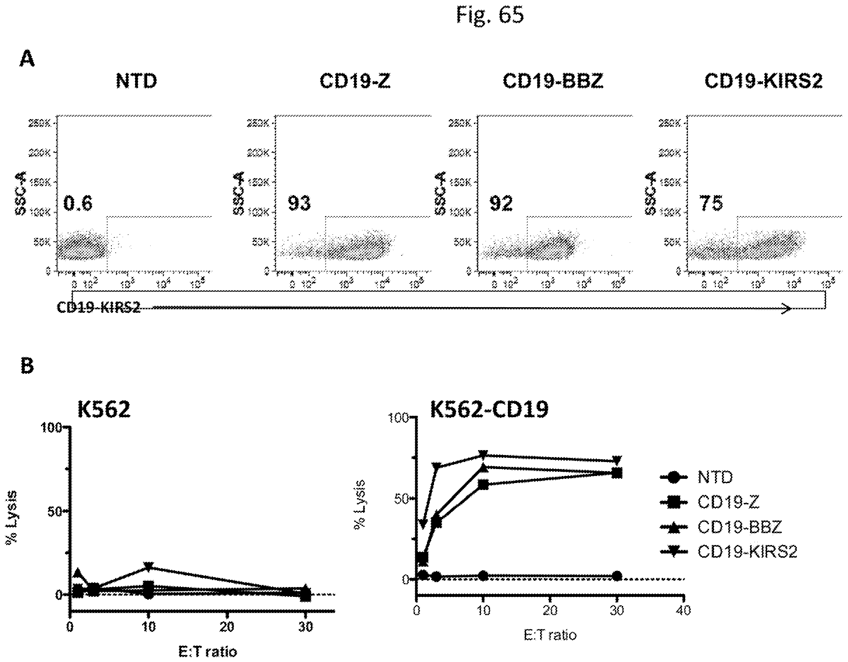

FIG. 65 comprising FIGs. 65A-65B, are a set of images that demonstrates a KIR-based CAR with CD19 specificity can trigger antigen-specific target cell cytotoxicity. Following anti-CD3/anti-CD28 bead activation, T cells were transduced with a bicistronic lentiviral vector expressing DAP12 along with either a CD19-specific KIR-based CAR in which the FMC63-derived scFv is fused to full length KIR2DS2 (CD19-KIR2DS2) or a KIR-based CAR generated by fusing the FMC63 scFv to the transmembrane and cytoplasmic domain of KIR2DS2 via a short linker [Gly]₄-Ser linker(CD19-KIRS2). The transduced T cells were cultured until the end of the log phase growth, and the expression of the CD19-specific KIR-based CAR was assessed by flow cytometry using a biotinylated goat-anti-mouse F(ab)₂ polyclonal antibody followed by SA-PE. ⁵¹Cr-labeled K562 target cells with (K562- CD19) or without (K562-wt) CD19 expression were mixed at varying ratios with T cells to target cells (E:T ratio). Cytotoxicity was determined by measuring the fraction of ⁵¹Cr released into the supernatant at 4 hours. Control T cells that were either mock transduced (NTD) or transduced with a CD3ζ-based CAR specific to CD19 (CD19-z) were also included as negative and positive controls, respectively.

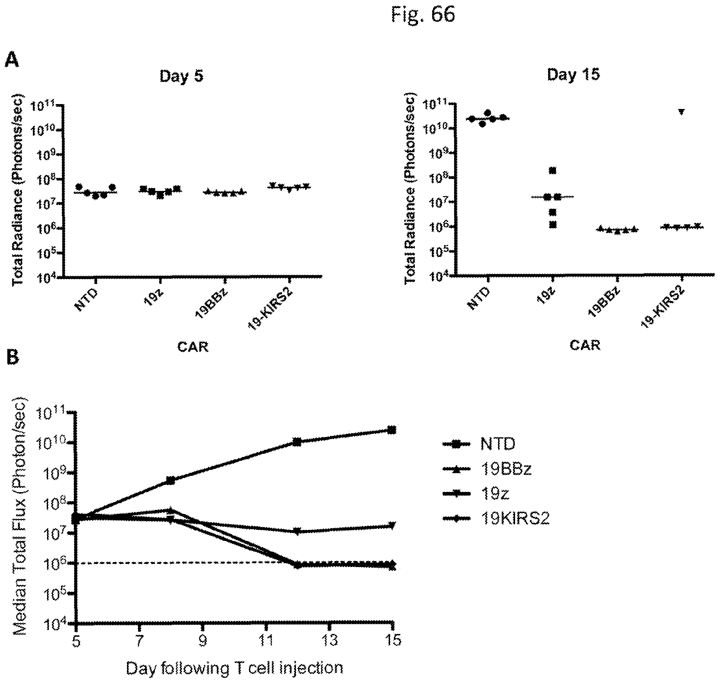

FIG. 66 comprising FIGs. 66A-66B shows CD19-KIRS2 *in vivo* activity. NOD-SCID-γ_{c}^{-/-}(NSG) mice were engrafted intravenously by tail vein injection on day 0 of 1 million Nalm-6 CBG tumor cells, a leukemic cell line expressing CD19. T cells were stimulated with anti-CD3/anti-CD28 stimulator beads followed by lentiviral transduction on day 1 with a series of CD19-specfic CD3ζ-based CAR with or without a costimulatory domain (CD19z, 19BBz) or the CD19-specific KIR-based CARs, CD19-KIRS2 with DAP12 (19KIRS2). Mock non-transduced T cells (NTD) were used as a control. The T cells were expanded until the end of log-phase growth ex vivo and injected intravenously on day 5 post leukemic cell line injection with 2 million CAR T cells per mouse. Tumor burden was assessed via bioluminescent imaging. 5 animals were analyzed for each T cell condition. FIG. 66A shows the individual bioluminescent photon flux for individual animals on day 5 (baseline prior to T cell injection) and at day 15 following leukemic cell engraftment. FIG. 66B shows the median total flux for each treatment group over time.

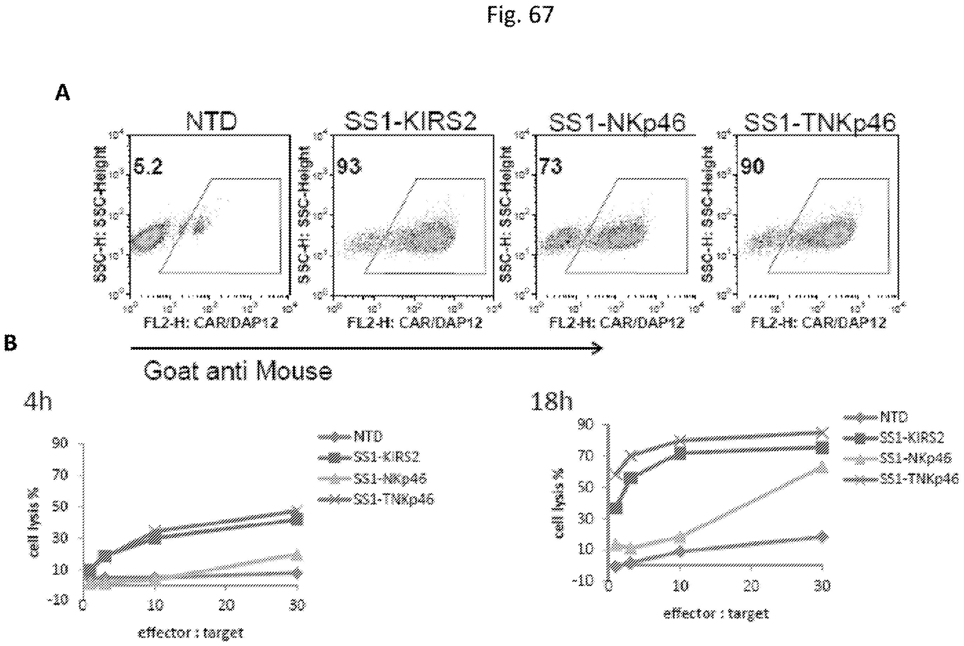

FIG. 67, comprising FIG. 67A and FIG. 67B, are a set of graphs that demonstrates an NKp46-based NCR CAR with mesothelin specificity triggers antigen specific cytotoxicity. Following anti-CD3/anti-CD28 bead activation, T cells were transduced with a bi-cistronic lentiviral vector expressing either DAP12 and SS1-KIRS2 (control), or FcεRγ and a mesothelin specific NKp46-based CAR (SS1-NKp46) or FcεRγ and a mesothelin-specific NKp46 CAR in which the natural NKp46 extracellular domain was truncated (SS1-TNKp46). The expression of the mesothelin-specific CARs was assessed by flow cytometry using a biotinylated goat-anti-mouse F(ab)2 polyclonal antibody followed by SA-PE as shown in FIG. 67A. The T cells were mixed with ⁵¹Cr-labeled K562 target cells expressing mesothelin at varying ratios of effector T cells to target K562 cells (E:T ratio). Cytotoxicity was determined by measuring the fraction of ⁵¹Cr released into the supernatant at 4 hours compared with spontaneous release as shown in FIG. 67B.
FIG. 68 shows a schematic representation of the receptors used in Experiments shown in FIGs. 70-72.
FIG. 69 is a set of images thatdemonstrates the generation and characterization of a K562-meso cell line that express the KIR2DL3 ligand HLA-Cw. K562 cells (K562) or K562 cells expressing mesothelin (K562-meso) were transduced with the HLA-Cw3 allele followed by fluorescence activated cell sorting to obtain K562 cells expressing HLA-Cw with (K562-meso-HLACw) or without (K562-HLACw) expression of mesothelin. HLA-Cw3 expression was assessed by flow cytometry using an APC-conjugated monoclonal antibody that recognizes HLA-A, B and C alleles (clone W6/32).
FIG. 70 is a set of images that demonstrates co-expression of SS1-KIRS2 and KIR2DL3 in primary human T cells. Primary human T cells were stimulated with CD3/28 microbeads followed by lentiviral transduction with SS1-KIRS2 and DAP12 (SS1-KIRS2) or mock transduced (NTD) in combination with wild-type KIR2DL3. The T cells were expanded until the end of log-phase growth. The surface expression of the mesothelin-specific CAR and KIR2DL3 was determined by staining with mesothelin-Fc followed by PE-conjugated goat-anti-human Fc and a monoclonal antibody to the KIR2DL3 ectodomain.
FIG. 71 is a set of graphs that demonstrates that KIR2DL3 coexpressed with a KIR CAR can suppress antigen specific cytotoxicity in the presence of HLA-Cw on the target cells. T cell that were generated and characterized as described in FIG. 70 were mixed with 51-Cr labeled target K562 cells that were generated and characterized as described in FIG. 71. Cytotoxicity was determined by measuring the fraction of ⁵¹Cr released into the supernatant at 4 hours compared target cells without effector cells.
FIG. 72 shows a schematic representation of the receptors used in Experiments shown in FIG. 73.

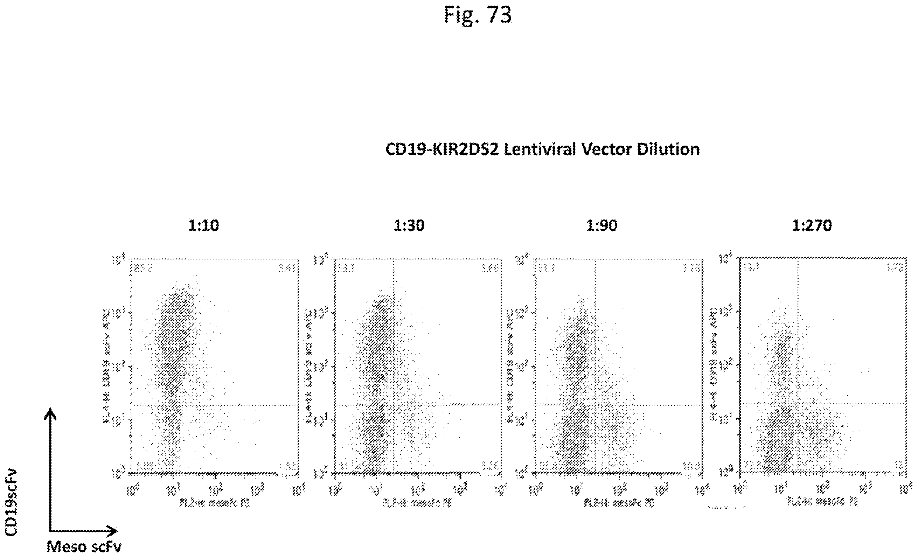

FIG. 73 is a set of images that demonstrates the inability to co-express two scFv-based chimeric receptors on the surface of the T cell while retaining each receptors' respective binding specificity. Jurkat T cells were transduced using a lentiviral vector encoding SS1-KIR2DL3. These cells were subsequently transduced with a second lentiviral vector encoding CD19-KIR2DS2 at varying dilutions of the vector. The expression of the SS1-specific scFv was assessed using mesothelin-Fc followed by PE-conjugated goat-anti-human Fc. The CD19-specific scFv expression was assessed using a PE-conjugated monoclonal antibody specific to the FMC63 idiotype.

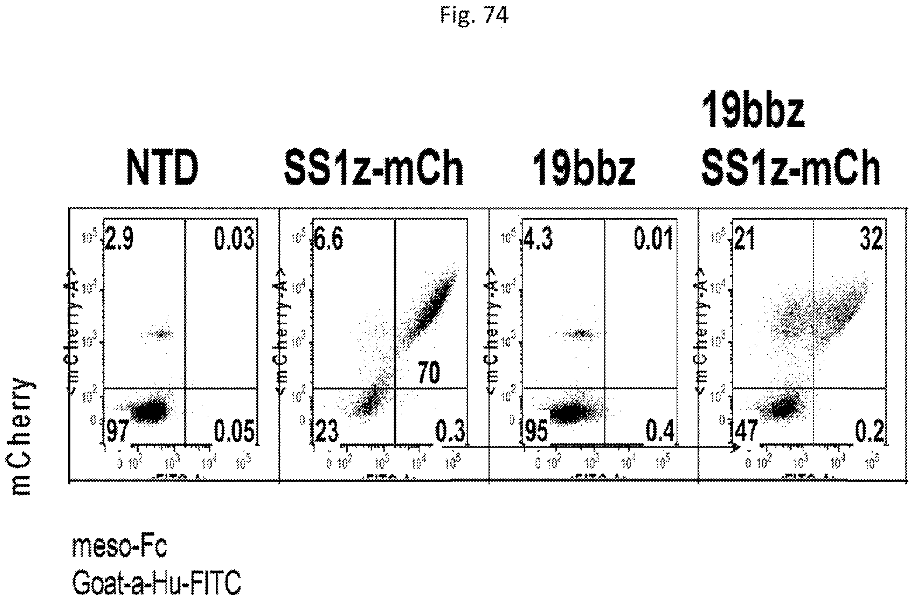

FIG. 74 is a panel of images demonstrating that expression of a CD19-specific CAR also reduced the expression of mesothelin-binding sites on the surface of cells co-expressing an SS1-zeta-mCherry fusion CAR. Primary human T cells were stimulated with CD3/28 microbeads followed by lentiviral transduction with an SS1 scFv zeta CAR bearing a C-terminal mCherry fusion (SS1z-mCh) or the FMC63-derived CD19 specific 41BB-zeta CAR (19bbz) alone or combination. Mock-transduced cells were used as a control. The T cells were expanded until the end of log-phase growth, and dsRed as well as surface CAR expression was determined by flow cytometry after staining with mesothelin-Fc followed by a goat-anti-human Fc specific polyclonal antibody conjugated to FITC.

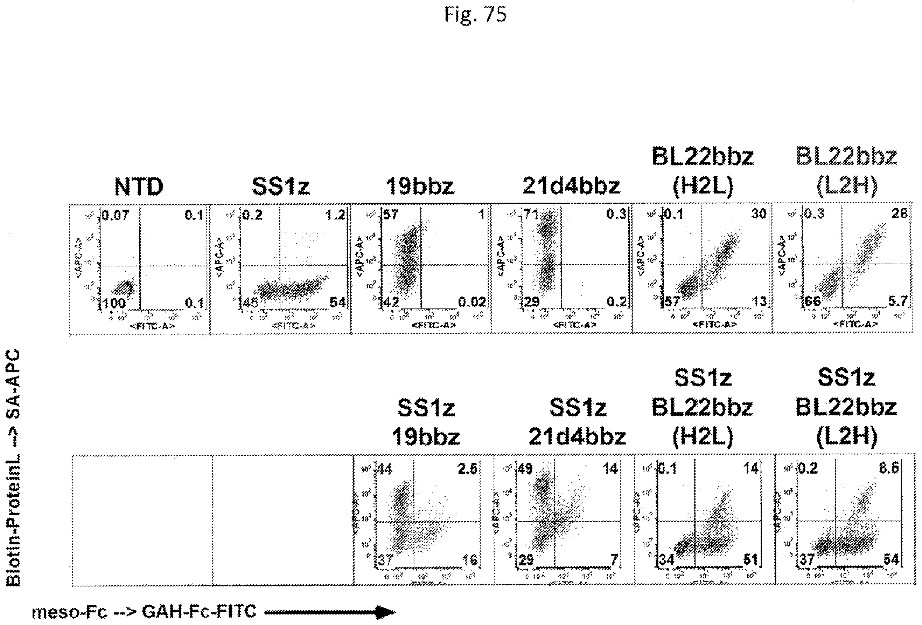

FIG. 75 is a panel of images demonstrating that mutually exclusive expression of binding sites for the SS1 scFv is not unique to the FMC63 scFv. Primary human T cells were stimulated with CD3/28 microbeads followed by lentiviral transduction with either an SS1 scFv zeta CAR or various CD19 specific 41BB-zeta CARs (19BBz [FMC63 scFv, 214d scFv or the BL22 scFv CARs with alternate VH and VL orientations [H2L and L2H]). NTD represents mock-transduced cells used as a staining control. In addition, a separate set of T cells were co-transduced with the SS1 scFv zeta CAR and the different CD19 specific CARs as above. The T cells were expanded until the end of log phase growth, and surface CAR expression was determined by staining with biotinylated protein L (recognizes kappa light chain) followed by streptavidin APC simultaneously with mesothelin-Fc followed by a goat-anti-human Fc specific polyclonal antibody conjugated to PE. The cotransduced cells showed that the mutually exclusive expression observed with FMC63-based CAR is also observed with other scFv-CARs.
FIG. 76, comprising FIGs. 76A-76B, depict the putative mechanism for loss of scFv binding when two scFv molecules are co-expressed on the cell surface (FIG. 76A) and the putative avoidance of this interaction when a camelid single VHH domain-based CAR is expressed on a T cell surface in combination with a scFv-based CAR.

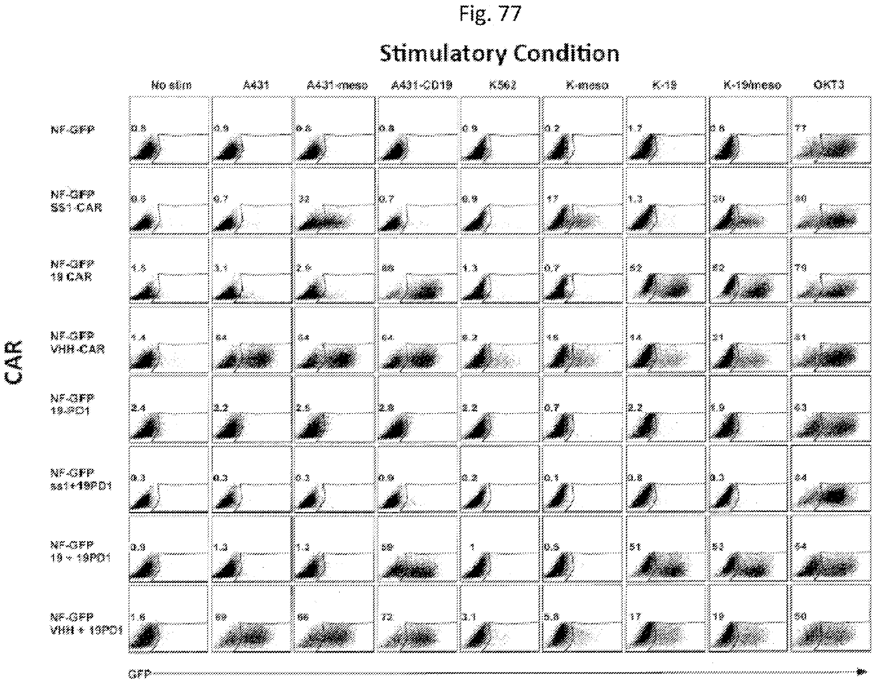

FIG. 77 is a panel of images that demonstrates a camelid single VHH domain-based CAR can be expressed on a T cell surface in combination with a scFv-based CAR without appreciable receptor interaction. Jurkat T cells expressing GFP under an NFAT-dependent promoter (NF-GFP) were transduced with either a mesothelin-specific activating CAR (SS1-CAR), CD19-specific activating (19-CAR) or a CAR generated using a camelid VHH domain specific to EGFR (VHH-CAR). Following transduction with the activating CAR, the cells were then transduced with an additional inhibitory CAR recognizing CD19 (19-PD1) to generate cells co-expressing both the activating and inhibitory CAR (SS1+19PD1, 19+19PD1 or VHH+19PD1). The transduced Jurkat T cells were co-cultured for 24 hours with different cell lines that are either 1) devoid of all target antigens (K562), 2) express mesothelin (K-meso), CD19 (K-19) or EGFR (A431) only, 3) express a combination of EGFR and mesothelin (A431-mesothelin) or CD19 (A431-CD19) or 4) express a combination of CD19 and mesothelin (K-19/meso). Additional conditions that include either no stimulator cells (no stim) or K562 with 1 ug/mL of OKT3 (OKT3) were also included as negative and positive controls for NFAT activation, respectively. GFP expression, as a marker of NFAT activation, was assessed by flow cytometry.

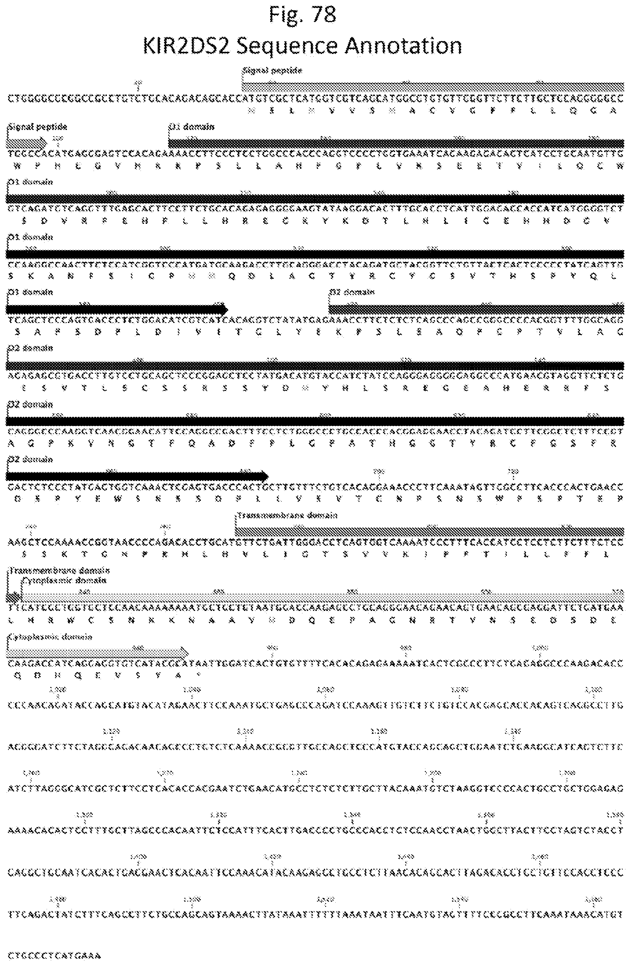

FIG. 78 shows a KIR2DS2 Sequence Annotation (SEQ ID NO: 219 for the nucleic acid sequence shown; SEQ ID NO: 220 for the amino acid sequence shown).

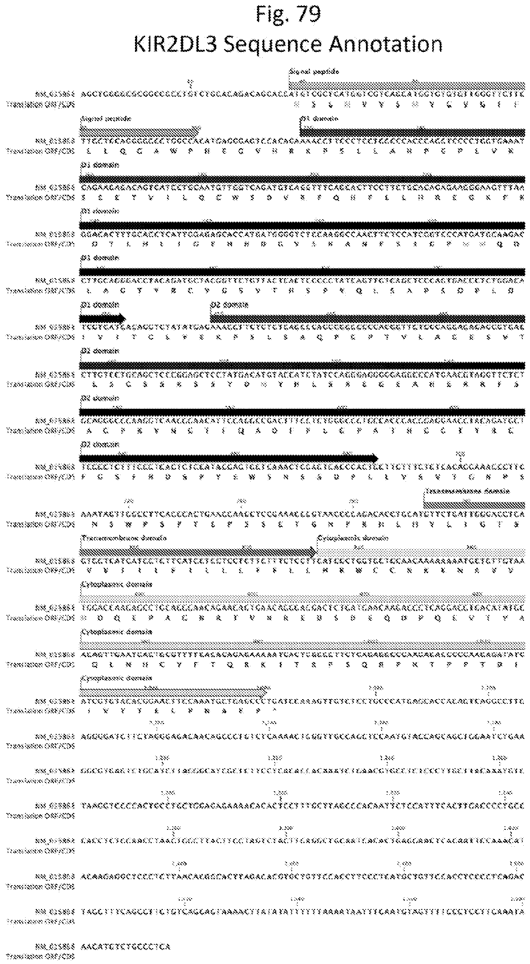

FIG. 79 shows a KIR2DL3 Sequence Annotation (SEQ ID NO: 221 for the nucleic acid sequence shown; SEQ ID NO: 222 for the amino acid sequence shown).

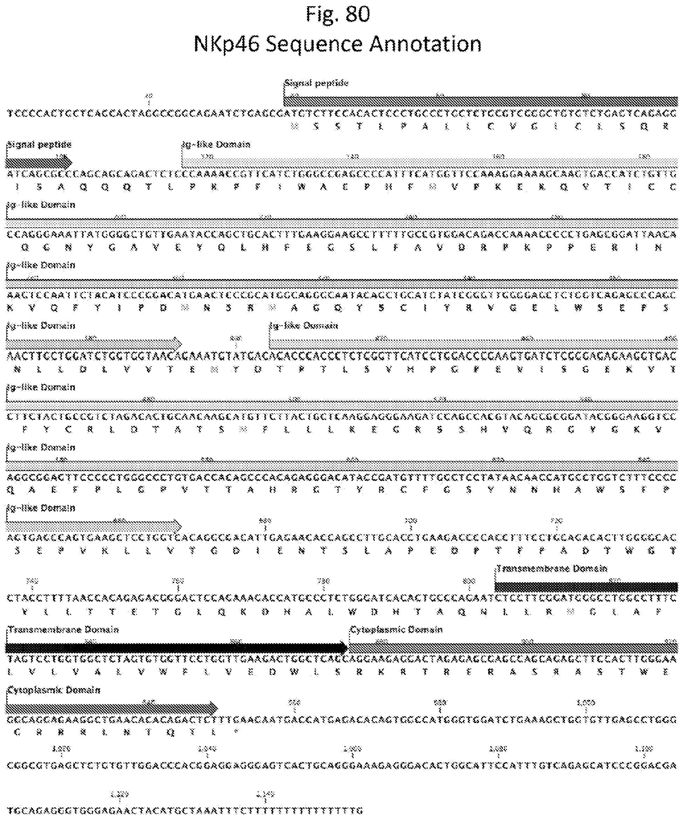

FIG. 80 shows a NKp46 Sequence Annotation (SEQ ID NO: 223 for the nucleic acid sequence shown; SEQ ID NO: 224 for the amino acid sequence shown).

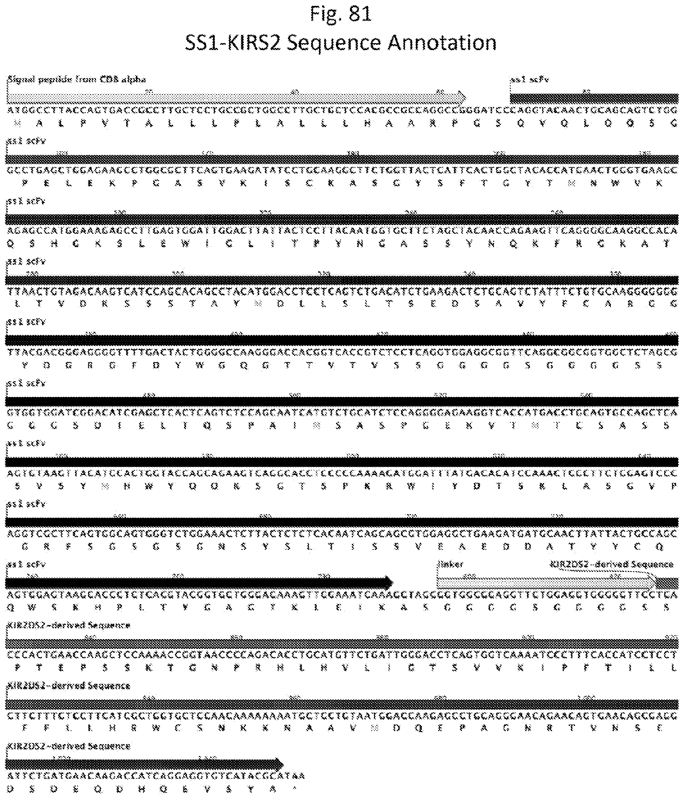

FIG. 81 shows a SS1-KIRS2 Sequence Annotation (SEQ ID NO: 225 for the nucleic acid sequence shown; SEQ ID NO: 226 for the amino acid sequence shown).

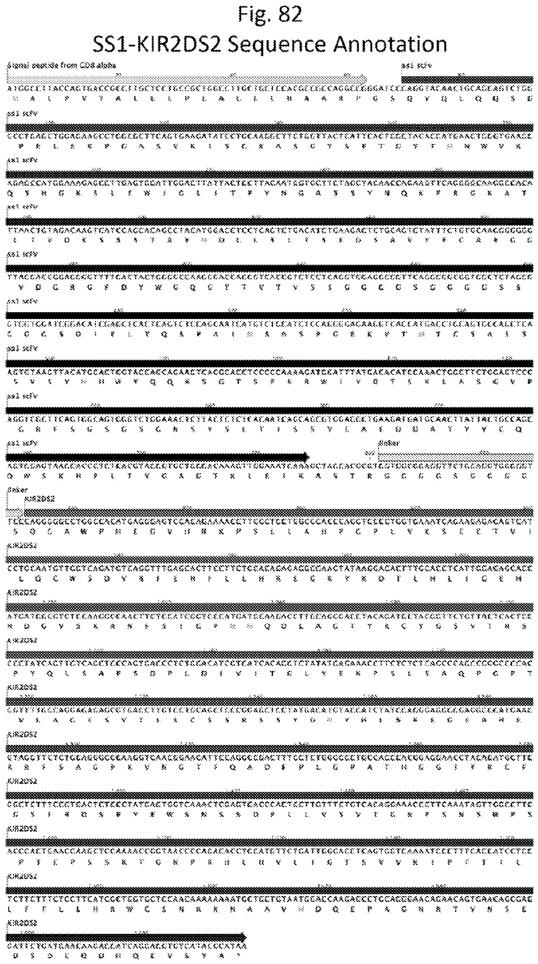

FIG. 82 shows a SS1-KIR2DS2 Sequence Annotation (SEQ ID NO: 227 for the nucleic acid sequence shown; SEQ ID NO: 228 for the amino acid sequence shown).

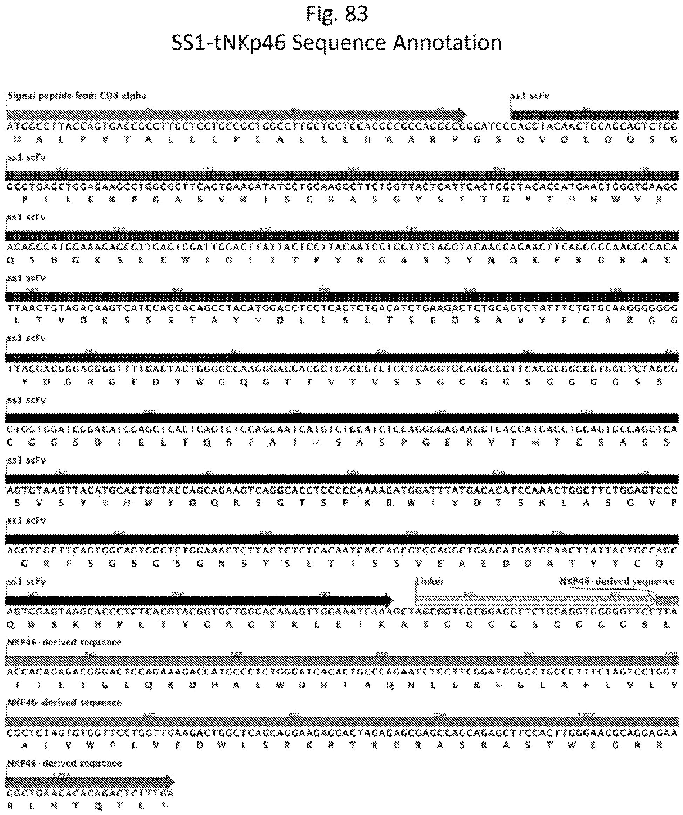

FIG. 83 shows a SS1-tNKp46 Sequence Annotation (SEQ ID NO: 229 for the nucleic acid sequence shown; SEQ ID NO: 230 for the amino acid sequence shown).

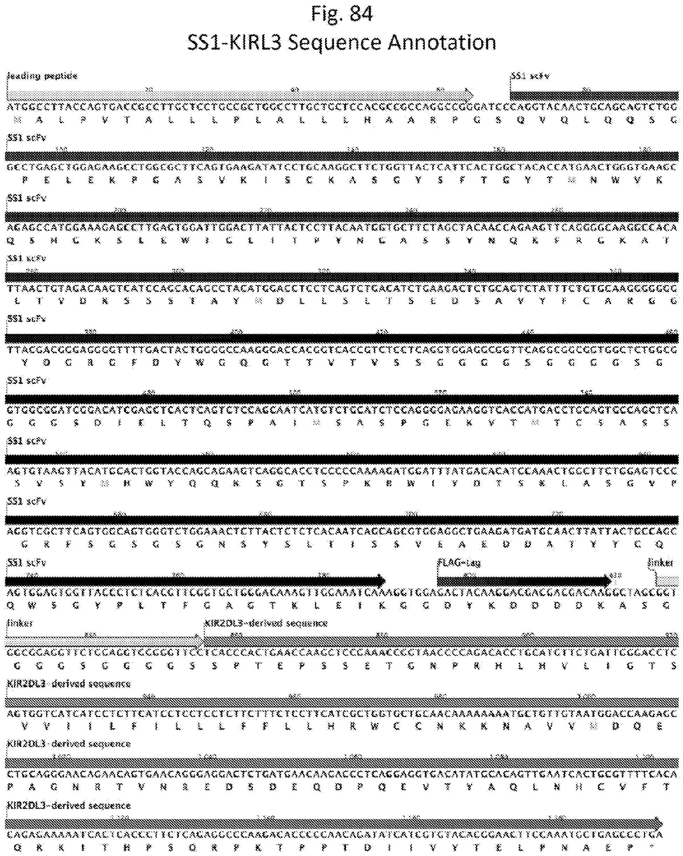

FIG. 84 shows a SS1-KIRL3 Sequence Annotation (SEQ ID NO: 231 for the nucleic acid sequence shown; SEQ ID NO: 232 for the amino acid sequence shown).
FIG. 85 is a pair of graphs that shows that mesothelin-specific CD3ζ and KIR-based CARs have similar antigen specific in vitro cytotoxicity toward mesothelioma-derived cells expressing mesothelin (EM-meso cells). Primary human T cells were stimulated with anti-CD3/CD28 stimulator beads and transduced with a lentiviral vector expressing the SS1-KIRS2 mesothelin-specific CAR. Following expansion, T cells were mixed with ⁵¹Cr-labeled K562 cells expressing EM-meso at the indicated effector to target (E:T) ratio. % lysis was determined.
FIG. 86 is a pair of graphs that shows TILs from 28ζ CART treated mice lost IFNγ secretion with stimulation with mesothelioma-derived cells expressing mesothelin (EM-meso cells). NOD-SCID -γc -/- (NSG) mice were injected subcutaneously with 2x10⁶ EM-meso cells. 5x10⁶ primary human T cells transduced with the indicated CAR were injected IV on day 16. 18 days post CAR T cells infusion, TILs were isolated with CD45 magnetic beads and mixed with EM-meso at the indicated effector to target (E:T) ratio. Cytokine concentrations were determined in supernatants by ELISA.
FIG. 87 shows that SS1-KIRS2/DAP12 T cells mediate robust anti-tumor activity in vivo. NOD-SCID-γc -/- (NSG) mice were injected subcutaneously with 2x10⁶ mesothelioma-derived cells expressing mesothelin (EM-meso cells). 5x10⁶ primary human T cells transduced with the indicated CAR were injected IV on day 20. Tumor volume was measured by caliper at the indicated times.
FIGs. 88A, 88B, 88C, 88D, 88E, and 88F are schematics that show exemplary configurations of a RNKR-CAR.
FIG. 89 is a graph showing the extent of target cell killing by RCAR-expressing T cells (Mix 2) compared to standard CAR-expressing T cells (huCAR19) and T cells expressing no CARs (UTD) at various concentrations of RAD001.

### DETAILED DESCRIPTION

### DEFINITIONS

Unless defined otherwise, all technical and scientific terms used herein have the same meaning as commonly understood by one of ordinary skill in the art to which the invention pertains.

"A" and "an" as the term is used herein, refers to one or to more than one (i.e., to at least one) of the grammatical object of the article. By way of example, "an element" means one element or more than one element.

"About" as the term is used herein, when referring to a measurable value such as an amount, a temporal duration, and the like, is meant to encompass variations of ±20% or in some embodiments ±10%, or in some embodiments ±5%, or in some embodiments ±1%, or in some embodiments ±0.1% from the specified value, as such variations are appropriate to perform the disclosed methods.

"Autologous" as the term is used herein refers to any material derived from the same individual to whom it is later to be re-introduced.

"Allogeneic" as the term is used herein refers to any material derived from a different animal of the same species as the individual to whom the material is introduced. Two or more individuals are said to be allogeneic to one another when the genes at one or more loci are not identical. In some aspects, allogeneic material from individuals of the same species may be sufficiently unlike genetically to interact antigenically.

An "antigen binding domain" as the term is used herein, refers to a molecule that has affinity for a target antigen, typically an antigen on a target cell, e.g., a cancer cell. An exemplary antigen binding domain comprises a polypeptide, e.g., an antibody molecule (which includes an antibody, and antigen binding fragments thereof, e.g., a immunoglobulin, single domain antibody (sdAb, e.g., a nanobody, and an scFv), or a non-antibody scaffold, e.g., a fibronectin, and the like. In embodiments, the antigen binding domain is a single polypeptide. In embodiments, the antigen binding domain comprises, one, two, or more, polypeptides. In embodiments the antigen binding domain comprises a fragment of an antibody, that is sufficient to confer recognition and specific binding to the target antigen. The term "antibody fragment" refers to at least one portion of an antibody, that retains the ability to specifically interact with (e.g., by binding, steric hinderance, stabilizing/destabilizing, spatial distribution) an epitope of an antigen. Examples of an antibody fragment include, but are not limited to, an Fab, Fab', F(ab')₂, or Fv fragment, an scFv antibody fragment, an disulfide-linked Fv (sdFv), a Fd fragment consisting of the VH and CH1 domains, a linear antibody, single domain antibody such as an sdAb, e.g., a nanobody, (either VL or VH), a camelid VHH domain, multi-specific antibodies formed from antibody fragments such as a bivalent fragment comprising two Fab fragments linked by a disulfide brudge at the hinge region, and an isolated CDR or other epitope binding fragments of an antibody. An antigen binding fragment can also be incorporated into single domain antibodies, maxibodies, minibodies, nanobodies, intrabodies, diabodies, triabodies, tetrabodies, v-NAR and bis-scFv (see, e.g., Hollinger and Hudson, Nature Biotechnology 23:1126-1136, 2005). Antigen binding fragments can also be grafted into scaffolds based on polypeptides such as a fibronectin type III (Fn3)(see U.S. Patent No.: 6,703,199, which describes fibronectin polypeptide minibodies).

In an embodiment, the antigen binding domain is a "scFv," which can comprise a fusion protein comprising a VL chain and a VH chain of an antibody, where the VH and VL are linked via a short flexible polypeptide linker. The scFv is capable of being expressed as a single chain polypeptide and retains the specificity of the intact antibody from which it is derived. Moreover, the VL and VH variable chains can be linked in either order, e.g., with respect to the N-terminal and C-terminal ends of the polypeptide, the scFv may comprise VL-linker-VH or may comprise VH-linker-VL. In embodiments, the antigen binding domain comprises a non antibody scaffold, e.g., a fibronectin, ankyrin, domain antibody, e.g., a nanobody, lipocalin, small modular immuno-pharmaceutical, maxybody, Protein A, or affilin. The non antibody scaffold has the ability to bind to target antigen on a cell. In embodiments, the antigen binding domain is a polypeptide or fragment thereof of a naturally occurring protein expressed on a cell. In an embodiment, the antigen binding domain binds a growth factor or hormone receptor. While not wishing to be bound by theory, the antigen binding domain serves to provide specificity for target cells, and in embodiments, optimize and immune effector function by coupling antigen binding to generation of a signal by an intracellular signaling domain on an intracellular signaling member.

"Antigen binding member," as that term is used herein, comprises a binding domain element (e.g., an antigen binding domain, inhibitory extracellular domain, or costimulatory extracellular domain), and, optionally, a transmembrane domain or a membrane anchor. An antigen binding member can also comprise a switch domain. In embodiments, the switch domain on the antigen binding member can form a dimerization switch with a switch domain on an intracellular signaling member. The dimerization switch formed by these two switch domains can couple antigen binding to intracellular signal generation, and thereby optimize an immune effector function of the cell. In embodiments, the antigen binding member comprises an antigen binding domain which is other than the native extracellular domain of a molecule from which an intracellular signaling domain on the intracellular signaling member is derived. In embodiments, the antigen binding member comprises an antigen binding domain which binds an antigen which is not the ligand of the native extracellular domain of a molecule from which an intracellular signaling domain on the intracellular signaling member is derived.

"Anti-cancer effect", as that term is used herein, refers to a biological effect which can be manifested by various means, including but not limited to, e.g., a decrease in tumor volume, a decrease in the number of cancer cells, a decrease in the number of metastases, an increase in life expectancy, decrease in cancer cell proliferation, decrease in cancer cell survival, or amelioration of various physiological symptoms associated with the cancerous condition. An "anti-cancer effect" can also be manifested by the ability of the peptides, polynucleotides, cells and antibodies in prevention of the occurrence of cancer in the first place. The term "anti-tumor effect" refers to a biological effect which can be manifested by various means, including but not limited to, e.g., a decrease in tumor volume, a decrease in the number of cancer cells, a decrease in tumor cell proliferation, or a decrease in tumor cell survival.

"Auxiliary antigen binding member," as that term is used herein, refers to a molecule comprising an antigen binding domain that binds an antigen other than the antigen bound by another antigen binding domain of the RCAR, NKR-CAR, or RNKR-CAR, e.g., other than the antigen binding domain of the antigen binding member. In embodiments it comprises a transmembrane domain or membrane anchoring domain.

"Binding domain element," as that term is used herein, refers to an antigen binding domain, an inhibitory extracellular domain, or a costimulatory extracellular domain.

"Cancer" as the term is used herein, refers to a disease characterized by the uncontrolled growth of aberrant cells. Cancer cells can spread locally or through the bloodstream and lymphatic system to other parts of the body. Examples of various cancers include but are not limited to, breast cancer, prostate cancer, ovarian cancer, cervical cancer, skin cancer, pancreatic cancer, colorectal cancer, renal cancer, liver cancer, brain cancer, lymphoma, leukemia, lung cancer and the like.

The term "Chimeric Antigen Receptor" or alternatively a "CAR" as used herein, refers to a chimeric polypeptide that shares structural or functional properties with a cell immune-function receptor or adaptor molecule, e.g., a T cell or NK cell. Upon binding to cognate antigen, a CAR can activate or inactivate the cytotoxic cell in which it is disposed, or modulate the cell's antitumor activity or otherwise modulate the cells immune response.

CARs include, e.g., TCARs, RCARs, NKR-CARs, RNKR-CARs, which are defined below. In some embodiments, in RCARs, for example, the set of polypeptides include a dimerization switch that, upon the presence of a dimerization molecule, can couple the polypeptides to one another, e.g., can couple an antigen binding domain to an intracellular signaling domain. In embodiments, in NKR-CARs, the set of polypeptides comprises one or more element (e.g., domain) from a natural killer cell receptor (NKR). In embodiments, in RNKR-CARs, the set of polypeptides comprises one or more element (e.g., domain) from a NKR, and the set of polypeptides includes a dimerization switch that, upon the presence of a dimerization molecule, can couple the polypeptides to one another, e.g,. can couple an antigen binding domain to an intracellular signaling domain. In some embodiments, in TCARs (also referred to as standard CARs), for example, an element of the CAR is derived from a T cell, e.g., a T cell receptor. For example, the stimulatory molecule is the zeta chain associated with the T cell receptor complex.

"Costimulatory signaling domain," as that term is used herein, refers to a molecule, e.g., an endogenous molecule, of the RCAR/NKR-CARX, or RNKR-CARX cell that, upon binding to its cognate counter ligand on a target cell, enhance, e.g., increases, an immune effector response.

"Costimulatory extracellular domain" (also referred to as costimulatory ECD or extracellular ligand binding domains of costimulatory molecules) as the terms are used herein, refers to an extracellular domain of a costimulatory molecule, e.g. a costimulatory molecule described herein.

"Derived from" as that term is used herein, indicates a relationship between a first and a second molecule. It generally refers to structural similarity between the first molecule and a second molecule and does not connotate or include a process or source limitation on a first molecule that is derived from a second molecule. For example, in the case of an intracellular signaling domain that is derived from a CD3zeta molecule, the intracellular signaling domain retains sufficient CD3zeta structure such that is has the required function, namely, the ability to generate a signal under the appropriate conditions. It does not connotate or include a limitation to a particular process of producing the intracellular signaling domain, e.g., it does not mean that, to provide the intracellular signaling domain, one must start with a CD3zeta sequence and delete unwanted sequence, or impose mutations, to arrive at the intracellular signaling domain.

"Dimerization molecule," as that term is used herein, refers to a molecule that promotes the association of a first switch domain with a second switch domain. In embodiments, e.g., where the dimerization switch is disposed intracellularly, the dimerization molecule can cross the plasma membrane. In embodiments, e.g., where the dimerization switch is disposed extracellularly, the dimerization molecule need not cross the plasma membrane. In embodiments, the dimerization molecule does not naturally occur in the subject, or does not occur in concentrations that would result in significant dimerization. In embodiments, the dimerization molecule is a small molecule, e.g., rapamycin or a rapalogue. In embodiments, the dimerization molecule is a polypeptide. In embodiments, the dimerization molecule is an antibody molecule, e.g., antibody or antigen-binding fragment thereof. In embodiments, the first and second switch domains of a homodimerization switch or heterodimerization switch associate together in the presence of small molecule dimerization molecule e.g., rapamycin or a rapalogue. In embodiments, the first and second switch domains of a homodimerization switch or heterodimerization switch associate together in the presence of polypeptide dimerization molecule. In embodiments, the first and second switch domains of a homodimerization switch or heterodimerization switch associate together in the presence of a multimeric peptide dimerization molecule. In embodiments, the first and second switch domains of a homodimerization switch or heterodimerization switch associate together in the presence of an antibody molecule dimerization molecule. In embodiments, the antibody molecule comprises a monospecific antibody molecule. In embodiments, the antibody molecule is a dual specific antibody molecule.

Generally, a dimerization molecule will promote the association of at least two switch molecules (and thereby the association of intracellular domains linked to the switch domains). In embodiments the dimerization molecule has a valency of greater than two, e.g., it is multi-valent, and binds, and thus clusters or dimerizes, more than two switch domains. E.g., a dimerization molecule can comprise a plurality, e.g., at least 2, 3, 4, 5, 6, 7, 8, 9 or 10, binding domains, each of which can bind a switch domain.

"dsRNA," as that term is used herein, refers to a nucleic acid molecule, having at least a region of duplexed structure, that is capable of mediating sequence specific inhibition of the expression of a target gene. dsRNAs comprise short interfering RNA (siRNA) and short hairpin RNA (shRNA). In embodiments, shRNA is similar in structure to an siRNA but includes a moiety, typically one or more RNA monomers, that connect a duplex region of sense and an antisense sequence. In an embodiment the shRNA, after intracellular processing (e.g., by Dicer), results in a 19-23 nucleotide duplex siRNA with 2 nucleotide 3' overhangs.

"Endogenous" as that term is used herein, refers to any material, e.g., a polypeptide, from or produced inside an organism, cell, tissue or system.

"Exogenous" as that term is used herein, refers to any material, e.g., a polypeptide, or dimerization molecule, introduced from or produced outside an organism, cell, tissue or system.

"Immune effector cell," as that term is used herein, refers to a cell that is involved in an immune response, e.g., in the promotion of an immune effector response. Examples of immune effector cells include T cells, e.g., alpha/beta T cells and gamma/delta T cells, B cells, natural killer (NK) cells, natural killer T (NKT) cells, mast cells, and myeloic-derived phagocytes. "Immune effector function or immune effector response," as that term is used herein, refers to function or response, e.g., of an immune effector cell, that enhances or promotes an immune attack of a target cell. E.g., an immune effector function or response refers a property of a T or NK cell that promotes killing or the inhibition of growth or proliferation, of a target cell. In the case of a T cell, primary stimulation and costimulation are examples of immune effector function or response. An immune effector function or response can be promoted by the action of a RCAR or RNKR-CAR, and can, e.g., result in a RCAR/NKR-CARX or RNKR-CARX cell that is more effective at proliferation, cytokine production, cytotoxicity or upregulation of cell surface markers such asCD25, CD69, CD 107a.

An "inhibitory extracellular domain," as that term is used herein, refers to polypeptide comprising an extracellular domain of an inhibitory molecule. Normally, binding to its conterligand has an inhibitory effect on the generation of an immune effector response. When linked, e.g., fused, or coupled by a dimerization switch, to an intracellular signaling domain, it redirects an interaction that normally inhibits the generation of an immune effector response into one that promotes an immune effector response.

"Inhibitory binding member," as that term is used herein, refers to a polypeptide that comprises an inhibitory extracellular domain, a transmembrane domain, and a switch domain.

"Inhibitory molecule," as that term is used herein, refers to a molecule, e.g., an endogenous molecule, of RCAR/NKR-CARX or RNKR-CARX cell that, upon binding to its cognate counter ligand on a target cell, minimizes, e.g., suppresses or inhibits, an immune effector response. Examples of inhibitory molecules include PD1, PD-L1, CTLA4, TIM3, LAG3, VISTA, BTLA, TIGIT, LAIR1, and TGFR beta.

"Intracellular signaling domain," as the term is used herein, refers to an intracellular portion of a molecule. The intracellular signaling domain generates a signal that promotes an immune effector function of the RCAR/NKR-CARX or RNKR-CARX cell. Examples of immune effector function include cytolytic activity and helper activity, including the secretion of cytokines.

In an embodiment, the intracellular signaling domain can comprise a primary intracellular signaling domain. Exemplary primary intracellular signaling domains include those derived from the molecules responsible for primary stimulation, or antigen dependent simulation. In an embodiment, the intracellular signaling domain can comprise a costimulatory intracellular domain. Exemplary costimulatory intracellular signaling domains include those derived from molecules responsible for costimulatory signals, or antigen independent stimulation. For example, in the case of a RCART or RNKR-CART, a primary intracellular signaling domain can comprise cytoplasmic sequences of the T cell receptor, and a costimulatory intracellular signaling domain can comprise cytoplasmic sequence from co-receptor or costimulatory molecule.

A primary cytoplasmic signaling sequence (also referred to as a "primary signaling domain") that acts in a stimulatory manner may contain a signaling motif which is known as immunoreceptor tyrosine-based activation motif or ITAM. Examples of an ITAM containing cytoplasmic signaling sequence that is of particular use in the invention includes, but is not limited to, those derived fromCD3 zeta, common FcR gamma (FCER1G), Fc gamma RIIa,, FcR beta (Fc Epsilon R1b), CD3 gamma, CD3 delta , CD3 epsilon, CD79a, CD79b, DAP10, and DAP12. In a specific RNKR-CAR or RCAR of the disclosure, the intracellular signaling domain in any one or more RNKR-CARs or RCARs of the disclosure comprises an intracellular signaling sequence, e.g., a primary signaling sequence of CD3-zeta.

A costimulatory intracellular signaling domain can be derived from the intracellular portion of a costimulatory molecule. A costimulatory molecule can be represented in the following protein families: TNF receptor proteins, Immunoglobulin-like proteins, cytokine receptors, integrins, signaling lymphocytic activation molecules (SLAM proteins), and activating NK cell receptors. Examples of such molecules include CD27, CD28, 4-1BB (CD137), OX40, GITR, CD30, CD40, ICOS, BAFFR, HVEM, ICAM-1, lymphocyte function-associated antigen-1 (LFA-1), CD2, CDS, CD7, CD287, LIGHT, NKG2C, SLAMF7, NKp80, CD160, B7-H3, and a ligand that specifically binds with CD83, and the like. Further examples of such costimulatory molecules include CDS, ICAM-1, GITR, BAFFR, HVEM (LIGHTR), SLAMF7, NKp80 (KLRF1), CD160, CD19, CD4, CD8alpha, CD8beta, IL2R beta, IL2R gamma, IL7R alpha, ITGA4, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, TNFR2, TRANCE/RANKL, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), CD69, SLAMF6 (NTB-A, Ly108), SLAM (SLAMF1, CD150, IPO-3), BLAME (SLAMF8), SELPLG (CD162), LTBR, LAT, GADS, SLP-76, and PAG/Cbp.

The intracellular signaling domain can comprise the entire intracellular portion, or the entire native intracellular signaling domain, of the molecule from which it is derived, or a functional fragment or derivative thereof.

"Intracellular signaling member," as that term is used herein, refers to a polypeptide comprising an intracellular signaling domain and a switch domain. In embodiments it comprises a primary intracellular signal domain, and, optionally, a costimulatory signaling domain. In embodiments with more than one intracellular signaling domain, such domains may be linked to each other in a random or specified order. Optionally, a short oligo- or polypeptide linker, for example, between 2 and 10 amino acids in length, may be disposed between intracellular signaling domains. A glycine-serine doublet provides a particularly suitable linker. In an embodiment, the intracellular signaling member comprises the signaling domain of CD3-zeta and the signaling domain of CD28. In an embodiment, the intracellular signaling member comprises the signaling domain of CD3-zeta and the signaling domain of 4-1BB. In an embodiment, the intracellular signaling domain of 4-1BB is a signaling domain from SEQ ID NO: 138. In an embodiment, the signaling domain of CD3-zeta is a signaling domain from SEQ ID NO: 139.

"Isolated" as that term is used herein refers to a nucleic acid or polypeptide means separated from at least one contaminating compound. With regard to a nucleic acid or polypeptide that exists in nature, it means free of a compound with which it occurs in nature, wherein in embodiments, the contaminating compound is a polynucleotide or polypeptide. With regard to a nucleic acid or polypeptide that is made synthetically, it means free of a sude reactant or compound used in its preparation, e.g., a solvent or starting reactant. For example, a nucleic acid or a polypeptide naturally present in a living animal is not "isolated," but the same nucleic acid or polypeptide partially or completely separated from the coexisting materials of its natural state is "isolated." An isolated nucleic acid or protein can exist in substantially purified form, or can exist in a non-native environment such as, for example, a host cell.

"Low, immune enhancing, dose" when used herein in conjuction with an mTOR inhibitor, e.g., an allosteric mTOR inhibitor, e.g., RAD001 or rapamycin, or a catalytic mTOR inhibitor, refers to a dose of mTOR inhibitor that partially, but not fully, inhibits mTOR activity, e.g., as measured by the inhibition of P70 S6 kinase activity. Methods for evaluating mTOR activity, e.g., by inhibition of P70 S6 kinase, are discussed herein. The dose is insufficient to result in complete immune suppression but is sufficient to enhance the immune response. In an embodiment, the low, immune enhancing, dose of mTOR inhibitor results in a decrease in the number of PD-1 positive T cells and/or an increase in the number of PD-1 negative T cells, or an increase in the ratio of PD-1 negative T cells/PD-1 positive T cells. In an embodiment, the low, immune enhancing, dose of mTOR inhibitor results in an increase in the number of naive T cells. In an embodiment, the low, immune enhancing, dose of mTOR inhibitor results in one or more of the following:
an increase in the expression of one or more of the following markers: CD62L^{high}, CD127^{high}, CD27⁺, and BCL2, e.g., on memory T cells, e.g., memory T cell precursors;
a decrease in the expression of KLRG1, e.g., on memory T cells, e.g., memory T cell precursors; and
an increase in the number of memory T cell precursors, e.g., cells with any one or combination of the following characteristics: increased CD62L^{high}, increased CD127^{high}, increased CD27⁺, decreased KLRG1, and increased BCL2;
   wherein any of the changes described above occurs, e.g., at least transiently, e.g., as compared to a non-treated subject.

"Membrane anchor," "membrane anchoring domain", or "membrane tethering domain" as that term is used herein, refers to a moiety or polypeptide sufficient to anchor an extracellular domain to the plasma membrane. Examples of non-polypeptide moieties include glycophosphatidylinositol (GPI anchor) or a myristoyl group (myristoylation).

"Nucleic acid-based inhibitor," as that term is used herein, refers to a nucleic acid molecule that can inhibit expression of a target gene, e.g., an inhibitory molecule. It comprises double stranded RNA (dsRNA), including short hairpin RNA (shRNA) and short interfering RNA (siRNA), antisense RNA, and microRNA (miRNA). In an embodiment, the nucleic-acid based inhibitor binds to the target mRNA and inhibits the production of protein therefrom, e.g., by cleavage of the target mRNA.

"Regulatable chimeric antigen receptor (RCAR)," as that term is used herein, refers to a set of polypeptides, typically two in the simplest embodiments, which when in a RCARX or RCAR/NKR-CARX cell, provides the RCARX or RCAR/NKR-CARX cell with specificity for a target cell, typically a cancer cell, and with regulatable intracellular signal generation which can optimize an immune effector property of the RCARX or RCAR/NKR-CARX cell, e.g., cytolytic activity, cytokine secretion, cell survival, or proliferation. An RCARX or RCAR/NKR-CARX cell relies at least in part, on an antigen binding domain to provide specificity to a target cell that comprises the antigen bound by the antigen binding domain. In an embodiment, an RCAR includes a dimerization switch that, upon the presence of a dimerization molecule, can couple an intracellular signaling domain to a extracellular recognition element. An extracellular recognition element can be an antigen binding domain, an inhibitory counter ligand binding domain, or costimulatory ECD domain. In an embodiment, an RCAR includes a dimerization switch that, upon the presence of a dimerization molecule, can couple an intracellular signaling domain to a extracellular recognition element, which is not expressed by the RCARX or RCAR/NKR-CARX cell but provided exogenously.

"Regulatable natural killer cell receptor chimeric antigen receptor (RNKR-CAR),"as that term is used herein, refers to a set of polypeptides, typically two in the simplest embodiments, which when in a RNKR-CARX cell, provides the RNKR-CARX cell with specificity for a target cell, typically a cancer cell, and with regulatable intracellular signal generation which can optimize an immune effector property of the RNKR-CARX cell, e.g., cytolytic activity, cytokine secretion, cell survival, or proliferation,. An RNKR-CARX cell relies at least in part, on an antigen binding domain to provide specificity to a target cell that comprises the antigen bound by the antigen binding domain. In an embodiment, an RNKR-CAR includes a dimerization switch that, upon the presence of a dimerization molecule, can couple an intracellular signaling domain to a extracellular recognition element. An extracellular recognition element can be an antigen binding domain, an inhibitory counter ligand binding domain, or costimulatory ECD domain. In an embodiment, an RNKR-CAR includes a dimerization switch that, upon the presence of a dimerization molecule, can couple an intracellular signaling domain to an antigen binding domain. In an embodiment, a RNKR-CAR comprises an NKR cytoplasmic domain or an NKR transmembrane domain; and in an embodiment the NKR cytoplasmic domain is other than a FcR gamma (FCER1G), CD27, NKG2C, SLAMF7, NKP80 (KLRF1), CD160 (BY55), DNAM1 (CD226), SLAMF4 (CD244, 2B4), CD84, CD96 (Tactile), CEACAM1, CRTAM, Ly9 (CD229), PSGL1, CD100 (SEMA4D), SLAMF6 (NTB-A, Ly108), SLAM (SLAMF1, CD150, IPO-3), BLAME (SLAMF8), NKp44, NKp30, and/or NKp46 cytoplasmic domain. In an embodiment an RNKR-CAR comprises an NKR cytoplasmic domain and a primary signaling domain from an NK cell adaptor molecule, e.g., DAP12. In an embodiment an RNKR-CAR comprises an NKR transmembrane domain and a primary signaling domain from an NK cell adaptor molecule, e.g., DAP12. In an embodiment an RNKR-CAR comprises an NKR cytoplasmic domain (other than a FcR gamma (FCER1G), CD27, NKG2C, SLAMF7, NKP80 (KLRF1), CD160 (BY55), DNAM1 (CD226), SLAMF4 (CD244, 2B4), CD84, CD96 (Tactile), CEACAM1, CRTAM, Ly9 (CD229), PSGL1, CD100 (SEMA4D), SLAMF6 (NTB-A, Ly108), SLAM (SLAMF1, CD150, IPO-3), BLAME (SLAMF8), NKp44, NKp30, and/or NKp46 cytoplasmic domain) and a primary signaling domain from a T cell molecule, e.g., CD3zeta.

"RCARX cell," as that term is used herein, refers to a cell comprising RCAR. Any cell that is engineered to express a RCAR can be used as a RCARX cell. In an embodiment the RCARX cell is a T cell, and is referred to as a RCART cell. In an embodiment the RCARX cell is an NK cell, and is referred to as a RCARN cell.

"RNKR-CARX cell" (also referred to as "RNKR-CAR cell"), as that term is used herein, refers to a cell comprising RNKR-CAR. Any cell that is engineered to express a RNKR-CAR can be used as a RNKR-CARX cell. In an embodiment the RNKR-CARX cell is a T cell, and is referred to as a RNKR-CART cell. In an embodiment the RNKR-CARX cell is an NK cell, and is referred to as a RNKR-CARN cell.

"RCAR/NKR-CARX cell," as that term is used herein, refers to a cell comprising a RCAR and a NKR-CAR. Any cell that is engineered to express a RCAR and/or a NKR-CAR can be used as a RCAR/NKR-CARX cell. In an embodiment, the RCAR/NKR-CARX cell is a T cell, and is referred to as a RCAR/NKR-CART cell. In an embodiment, the RCAR/NKR-CARX cell is a NK cell, and is referred to as a RCAR/NKR-CARN cell.

In an embodiment the RCARX, RNKR-CARX, or RCAR/NKR-CARX cell is autologous to the patient. In an embodiment the RCARX, RNKR-CARX, or RCAR/NKR-CARX is allogeneic to the patient. In an embodiment, a patient receives more than one kind of RCARX, RNKR-CARX, or RCAR/NKR-CARX cell, e.g., the patient receives a RCART cell and a RCARN cell, or a RNKR-CART and a RNKR-CARN cell, or a RCAR/NKR-CART and a RCAR/NKR-CARN cell.

"Specifically binds," as that term is used herein, refers to an antibody, or a ligand, which recognizes and binds with a binding partner (e.g., tumor antigen) protein present in a sample, but which antibody or ligand does not substantially recognize or bind other molecules in the sample.

"Switch domain," as that term is used herein, refers to an entity, typically a polypeptide-based entity, that, in the presence of a dimerization molecule, associates with another switch domain. The association results in a functional coupling of a first entity linked to, e.g., fused to, a first switch domain, and a second entity linked to, e.g., fused to, a second switch domain. A first and second switch domain are collectively referred to as a dimerization switch. In embodiments, the first and second switch domains are the same as one another, e.g., they are polypeptides having the same primary amino acid sequence, and are referred to collectively as a homodimerization switch. In embodiments, the first and second switch domains are different from one another, e.g., they are polypeptides having different primary amino acid sequence, and are referred to collectively as a heterodimerization switch. In an embodiment, the switch is intracellular. In embodiments, the switch is extracellular. In embodiments, the switch domain is a polypeptide-based entity, e.g., FKBP-FRB, and the dimerization molecule is small molecule, e.g., a rapalogue. In instances, the switch domain is a polypeptide-based entity, e.g., an scFv that binds a myc peptide, and the dimerization molecule is a polypeptide, a fragment thereof, or a multimer of a polypeptide, e.g., a myc ligand or multimers of a myc ligand that bind to one or more myc scFvs. In instances, the switch domain is a polypeptide-based entity, e.g., myc receptor, and the dimerization molecule is an antibody or fragments thereof, e.g., myc antibody.

"Transmembrane domain," as that term is used herein, refers to a polypeptide that spans the plasma membrane. In an embodiment, it links an extracellular sequence, e.g., a switch domain, an extracellular recognition element, e.g., an antigen binding domain, an inhibitory counter ligand binding domain, or costimulatory ECD domain, to an intracellular sequence, e.g., to a switch domain or an intracellular signaling domain. A transmembrane domain of particular use in this invention may include at least the transmembrane region(s) of e.g., the alpha, beta or zeta chain of the T-cell receptor, CD28, CD3 epsilon, CD45, CD4, CD5, CD8 (e.g., CD8 alpha, CD8 beta), CD9, CD16, CD22, CD33, CD37, CD64, CD80, CD86, CD134, CD137, CD154. In some embodiments, a transmembrane domain includes at least the transmembrane region(s) of a NKR. In some embodiments, a transmembrane domain may include at least the transmembrane region(s) of, e.g., KIRDS2, OX40, CD2, CD27, LFA-1 (CD11a, CD18), ICOS (CD278), 4-1BB (CD137), GITR, CD40, BAFFR, HVEM (LIGHTR), SLAMF7, NKp80 (KLRF1), CD160, CD19, IL2R beta, IL2R gamma, IL7R α, ITGA1, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, TNFR2, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), SLAMF6 (NTB-A, Ly108), SLAM (SLAMF1, CD150, IPO-3), BLAME (SLAMF8), SELPLG (CD162), LTBR, PAG/Cbp.

"Treat", "treatment" and "treating", as those terms are interchangeably used herein, refer to the reduction or amelioration of the progression, severity and/or duration of a proliferative disorder, or the amelioration of one or more symptoms (preferably, one or more discernible symptoms) of a proliferative disorder resulting from the administration of one or more therapies (e.g., one or more therapeutic agents such as a CAR of the invention). In specific embodiments, the terms "treat", "treatment" and "treating" refer to the amelioration of at least one measurable physical parameter of a proliferative disorder, such as growth of a tumor, not necessarily discernible by the patient. In other embodiments the terms "treat", "treatment" and "treating" -refer to the inhibition of the progression of a proliferative disorder, either physically by, e.g., stabilization of a discernible symptom, physiologically by, e.g., stabilization of a physical parameter, or both. In other embodiments the terms "treat", "treatment" and "treating" refer to the reduction or stabilization of tumor size or cancerous cell count.

"Tumor antigen" or "cancer-associated antigen", as those terms are interchangeably used herein, refers to a molecule (typically a protein, carbohydrate or lipid) that is expressed on the surface of a cancer cell, either entirely or as a fragment (e.g., MHC/peptide), and which is useful for the preferential targeting of a pharmacological agent to the cancer cell. In some embodiments, a tumor antigen is a marker expressed by both normal cells and cancer cells, e.g., a lineage marker, e.g., CD19 on B cells. In some embodiments, a tumor antigen is a cell surface molecule that is overexpressed in a cancer cell in comparison to a normal cell, for instance, 1-fold over expression, 2-fold overexpression, 3-fold overexpression or more in comparison to a normal cell. In some enbodiments, a tumor antigen is a cell surface molecule that is inappropriately synthesized in the cancer cell, for instance, a molecule that contains deletions, additions or mutations in comparison to the molecule expressed on a normal cell. In some embodiments, a tumor antigen will be expressed exclusively on the cell surface of a cancer cell, entirely or as a fragment (e.g., MHC/peptide), and not synthesized or expressed on the surface of a normal cell. In some embodiments, the RNKR-CARs, NKR-CARs, or RCARs of the present disclosure includes RNKR-CARs, NKR-CARs, or RCARs comprising an antigen binding domain (e.g., antibody or antibody fragment) that binds to a MHC presented peptide. Normally, peptides derived from endogenous proteins fill the pockets of Major histocompatibility complex (MHC) class I molecules, and are recognized by T cell receptors (TCRs) on CD8 +T lymphocytes. The MHC class I complexes are constitutively expressed by all nucleated cells. In cancer, virus-specific and/or tumor-specific peptide/MHC complexes represent a unique class of cell surface targets for immunotherapy. TCR-like antibodies targeting peptides derived from viral or tumor antigens in the context of human leukocyte antigen (HLA)-A1 or HLA-A2 have been described (see, e.g., Sastry et al., J Virol. 2011 85(5):1935-1942; Sergeeva et al., Blood, 2011 117(16):4262-4272; Verma et al., J Immunol 2010 184(4):2156-2165; Willemsen et al., Gene Ther 2001 8(21) :1601-1608 ; Dao et al., Sci Transl Med 2013 5(176) :176ra33 ; Tassev et al., Cancer Gene Ther 2012 19(2):84-100). For example, TCR-like antibody can be identified from screening a library, such as a human scFv phage displayed library.

"Unswitched auxiliary antigen binding member," as the term is used herein, refers to a polypeptide that comprises: an antigen binding domain which binds an antigen other than the antigen bound by another antigen binding domain of the RNKR-CAR, NKR-CAR, or RCAR; a transmembrane domain; and an intracellular signaling domain, e.g., a primary intracellular signaling domain. Typically, it does not comprise a switch domain that can form a dimerization switch with a switch domain on another component of the RNKR-CAR, NKR-CAR, or RCAR.

"Unit dosage form" as the term is used herein refers to a dosage for suitable one administration. By way of example a unit dosage form can be a tablet, a capsule, or an amount of therapeutic disposed in a delivery device, e.g., a syringe or intravenous drip bag. In an embodiment a unit dosage form is administered in a single administration. In an embodiment more than one unit dosage form, e.g., two tablets, can be administered simultaneously.

"Xenogeneic" as the term is used herein refers to a graft derived from an animal of a different species.

"Adaptor" molecule, as that term is used herein, refers to a polypeptide with a sequence that permits interaction with two or more molecules, and in embodiments, promotes activation or inactivation of a cytotoxic cell. E.g., in the case of DAP12, this comprises interactions with an activating KIR via charge interactions within the transmembrane domain and interactions with signaling molecules like ZAP70 or Syk via a phosphorylated ITAM sequence within the cytoplasmic domain.

The term "antigen" or "Ag" as used herein is defined as a molecule that provokes an immune response. This immune response may involve either antibody production, or the activation of specific immunologically-competent cells, or both. The skilled artisan will understand that any macromolecule, including virtually all proteins or peptides, can serve as an antigen. Furthermore, antigens can be derived from recombinant or genomic DNA. A skilled artisan will understand that any DNA, which comprises a nucleotide sequences or a partial nucleotide sequence encoding a protein that elicits an immune response therefore encodes an "antigen" as that term is used herein. Furthermore, one skilled in the art will understand that an antigen need not be encoded solely by a full length nucleotide sequence of a gene. It is readily apparent that the present disclosure includes, but is not limited to, the use of partial nucleotide sequences of more than one gene and that these nucleotide sequences are arranged in various combinations to encode polypeptides that elicit the desired immune response. Moreover, a skilled artisan will understand that an antigen need not be encoded by a "gene" at all. It is readily apparent that an antigen can be generated synthesized or can be derived from a biological sample. Such a biological sample can include, but is not limited to a tissue sample, a tumor sample, a cell or a biological fluid.

The term "auto-antigen" means, in accordance with the present disclosure, any self-antigen which is recognized by the immune system as if it were foreign. Auto-antigens comprise, but are not limited to, cellular proteins, phosphoproteins, cellular surface proteins, cellular lipids, nucleic acids, glycoproteins, including cell surface receptors.

The term "autoimmune disease" as used herein is defined as a disorder that results from an autoimmune response. An autoimmune disease is the result of an inappropriate and excessive response to a self-antigen. Examples of autoimmune diseases include but are not limited to, Addision's disease, alopecia areata, ankylosing spondylitis, autoimmune hepatitis, autoimmune parotitis, Crohn's disease, diabetes (Type I), dystrophic epidermolysis bullosa, epididymitis, glomerulonephritis, Graves' disease, Guillain-Barr syndrome, Hashimoto's disease, hemolytic anemia, systemic lupus erythematosus, multiple sclerosis, myasthenia gravis, pemphigus vulgaris, psoriasis, rheumatic fever, rheumatoid arthritis, sarcoidosis, scleroderma, Sjogren's syndrome, spondyloarthropathies, thyroiditis, vasculitis, vitiligo, myxedema, pernicious anemia, ulcerative colitis, among others.

As used herein, the term "autologous" is meant to refer to any material derived from the same individual to which it is later to be re-introduced into the individual.

"Allogeneic" refers to a graft derived from a different animal of the same species.

As used herein, the term "conservative sequence modifications" is intended to refer to amino acid modifications that do not significantly affect or alter the binding characteristics of the antibody containing the amino acid sequence. Such conservative modifications include amino acid substitutions, additions and deletions. Modifications can be introduced into an antibody of the disclosure by standard techniques known in the art, such as site-directed mutagenesis and PCR-mediated mutagenesis. Conservative amino acid substitutions are ones in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine, tryptophan), nonpolar side chains (e.g., alanine, valine, leucine, isoleucine, proline, phenylalanine, methionine), beta-branched side chains (e.g., threonine, valine, isoleucine) and aromatic side chains (e.g., tyrosine, phenylalanine, tryptophan, histidine). Thus, for example, one or more amino acid residues within the CDR regions of an antibody of the disclosure can be replaced with other amino acid residues from the same side chain family and the altered antibody can be tested for the ability to bind FRβ using the functional assays described herein.

A "constitutive" promoter is a nucleotide sequence which, when operably linked with a polynucleotide which encodes or specifies a gene product, causes the gene product to be produced in a cell under most or all physiological conditions of the cell.

"Cytoplasmic" and "intracellular", as applied to adaptor molecules and signaling domains are used interchangeably herein. For example, a cytoplasmic or intracellular domain comprises a domain from a cytoplasmic protein (e.g., a protein normally found in the cytosol of a cell), or comprises a domain from a protein, typically a transmembrane protein (e.g., a protein, peptide or polypeptide that comprises a peptide or polypeptide that spans a cell membrane), where the domain is located in the cytoplasm of a cell. In an embodiment, a transmembrane protein includes a membrane embedded receptor. In an embodiment, a cytoplasmic or intracellular domain comprises a cytoplasmic or intracellular domain from a receptor described herein, e.g., a T cell receptor or a NKR. In other examples, a cytoplasmic or intracellular domain comprises a a domain from a T cell or NK cell cytoplasmic protein. In some examples, a cytoplasmic or intracellular domain comprises a domain from an adaptor molecule described herein, e.g., from a T cell or NK cell. In other examples, a cytoplasmic or intracellular domain comprises a cytoplasmic domain from an inhibitory molecule described herein. In an embodiment, a cytoplasmic or intracellular domain comprises a NKR cytoplasmic domain, which is a domain of a NKR that is naturally found in the cytoplasm of a cell. NKR cytoplasmic domains are described in greater detail below. Cytoplasmic or intracellular domains can include signaling domains, e.g., intracellular signaling domains, which can include primary signaling domains and costimulatory domains. Primary and costimulatory domains are described in greater detail herein.

"Encoding" refers to the inherent property of specific sequences of nucleotides in a polynucleotide, such as a gene, a cDNA, or an mRNA, to serve as templates for synthesis of other polymers and macromolecules in biological processes having either a defined sequence of nucleotides (*i.e.,* rRNA, tRNA and mRNA) or a defined sequence of amino acids and the biological properties resulting therefrom. Thus, a gene encodes a protein if transcription and translation of mRNA corresponding to that gene produces the protein in a cell or other biological system. Both the coding strand, the nucleotide sequence of which is identical to the mRNA sequence and is usually provided in sequence listings, and the noncoding strand, used as the template for transcription of a gene or cDNA, can be referred to as encoding the protein or other product of that gene or cDNA.

Unless otherwise specified, a "nucleotide sequence encoding an amino acid sequence" includes all nucleotide sequences that are degenerate versions of each other and that encode the same amino acid sequence. Nucleotide sequences that encode proteins and RNA may include introns.

"Effective amount" or "therapeutically effective amount" are used interchangeably herein, and refer to an amount of a compound, formulation, material, or composition, as described herein effective to achieve a particular biological result. Such results may include, but are not limited to, the inhibition of virus infection as determined by any means suitable in the art.

The term "expression" as used herein is defined as the transcription and/or translation of a particular nucleotide sequence driven by its promoter.

"Expression vector" refers to a vector comprising a recombinant polynucleotide comprising expression control sequences operatively linked to a nucleotide sequence to be expressed. An expression vector comprises sufficient cis-acting elements for expression; other elements for expression can be supplied by the host cell or in an in vitro expression system. Expression vectors include all those known in the art, such as cosmids, plasmids (*e.g.,* naked or contained in liposomes) and viruses (*e.g.,* lentiviruses, retroviruses, adenoviruses, and adeno-associated viruses) that incorporate the recombinant polynucleotide.

FcR-CAR, as that term is used herein, refers to a CAR which shares functional and structural properties with a FcR. FcR-CARs are described herein greater detail below.

"Homologous" as used herein, refers to the subunit sequence identity between two polymeric molecules, *e.g.,* between two nucleic acid molecules, such as, two DNA molecules or two RNA molecules, or between two polypeptide molecules. When a subunit position in both of the two molecules is occupied by the same monomeric subunit; *e.g.,* if a position in each of two DNA molecules is occupied by adenine, then they are homologous at that position. The homology between two sequences is a direct function of the number of matching or homologous positions; *e.g.,* if half (*e.g.,* five positions in a polymer ten subunits in length) of the positions in two sequences are homologous, the two sequences are 50% homologous; if 90% of the positions (*e.g*., 9 of 10), are matched or homologous, the two sequences are 90% homologous.

"Humanized" forms of non-human (e.g., murine) antibodies are chimeric immunoglobulins, immunoglobulin chains or fragments thereof (such as Fv, Fab, Fab', F(ab')2 or other antigen-binding subsequences of antibodies) which contain minimal sequence derived from non-human immunoglobulin. For the most part, humanized antibodies are human immunoglobulins (recipient antibody) in which residues from a complementary-determining region (CDR) of the recipient are replaced by residues from a CDR of a non-human species (donor antibody) such as mouse, rat or rabbit having the desired specificity, affinity, and capacity. In some instances, Fv framework region (FR) residues of the human immunoglobulin are replaced by corresponding non-human residues. Furthermore, humanized antibodies can comprise residues which are found neither in the recipient antibody nor in the imported CDR or framework sequences. These modifications are made to further refine and optimize antibody performance. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin sequence. The humanized antibody optimally also will comprise at least a portion of an immunoglobulin constant region (Fc), typically that of a human immunoglobulin. For further details, see Jones et al., Nature, 321: 522-525, 1986; Reichmann et al., Nature, 332: 323-329, 1988; Presta, Curr. Op. Struct. Biol., 2: 593-596, 1992.

In the context of the present invention, the following abbreviations for the commonly occurring nucleic acid bases are used. "A" refers to adenosine, "C" refers to cytosine, "G" refers to guanosine, "T" refers to thymidine, and "U" refers to uridine.

Unless otherwise specified, a "nucleotide sequence encoding an amino acid sequence" includes all nucleotide sequences that are degenerate versions of each other and that encode the same amino acid sequence. The phrase nucleotide sequence that encodes a protein or an RNA may also include introns to the extent that the nucleotide sequence encoding the protein may in some version contain an intron(s).

KIR-CAR, as that term is used herein, refers to a CAR which shares functional and structural properties with a KIR. KIR-CARs are described herein greater detail below.

A "lentivirus" as used herein refers to a genus of the Retroviridae family. Lentiviruses are unique among the retroviruses in being able to infect non-dividing cells; they can deliver a significant amount of genetic information into the DNA of the host cell, so they are one of the most efficient methods of a gene delivery vector. HIV, SIV, and FIV are all examples of lentiviruses. Vectors derived from lentiviruses offer the means to achieve significant levels of gene transfer in vivo.

Ly49-CAR, as that term is used herein, refers to a CAR which shares functional and structural properties with a Ly49. Ly49-CARs are described herein greater detail below.

NCR-CAR, as that term is used herein, refers to a CAR which shares functional and structural properties with a NCR. NCR-CARs are described herein greater detail below.

NK cell immune-function receptor (or NKR), as that term is used herein, refers to an endogenous naturally occurring transmembrane protein expressed in NK cells, which can engage with a ligand on an antigen presenting cell and modulate an NK cell immune-function response, e.g., it can modulate the cytolytic activity or cytokine secretion of the NK cell. The NKR can contribute to activation (an activating NKR, or actNKR), or inhibition (an inhibitory NKR, or inhNKR). Typically, an NKR comprises an extracellular ligand-binding domain (ECD), a transmembrane domain (TM) and an intracellular cytoplasmic domain (ICD). NKRs include the Killer Immunoglobulin-like Receptor (KIR) family of receptors such as KIR2DS2, the NK cell receptor (NCR) receptor family of receptors such as NKp46 (NCR1), the signaling lymphocyte activation receptor (SLAM) family (SLAMF) of receptors such as 2B4, and the Fc-binding receptors such as the IgG-binding receptor, CD16 (FcγRIII). Examples of NK cell immune-function responses modulated by NKRs comprise target cell killing (often referred to as cytotoxicity or cytolysis), cytokine secretion and/or proliferation. Typically, an NKR suitable for use in the methods and compositions described herein is a human NKR, (or hNKR). In an embodiment, the Ly49 receptor family in Mus musculus, which emerged by convergent evolution to provide the same function as a KIR in murine NK and T cells, is also included.

NKR-CAR, as that term is used herein, refers to a CAR which shares functional and structural properties with a NKR or adaptor molecule from a NK cell. NKR-CARs are described herein greater detail below.

The term "operably linked" refers to functional linkage between a regulatory sequence and a heterologous nucleic acid sequence resulting in expression of the latter. For example, a first nucleic acid sequence is operably linked with a second nucleic acid sequence when the first nucleic acid sequence is placed in a functional relationship with the second nucleic acid sequence. For instance, a promoter is operably linked to a coding sequence if the promoter affects the transcription or expression of the coding sequence. Generally, operably linked DNA sequences are contiguous and, where necessary to join two protein coding regions, in the same reading frame.

"Parenteral" administration of an immunogenic composition includes, e.g., subcutaneous (s.c.), intravenous (i.v.), intramuscular (i.m.), or intrasternal injection, or infusion techniques.

The term "polynucleotide" as used herein is defined as a chain of nucleotides. Furthermore, nucleic acids are polymers of nucleotides. Thus, nucleic acids and polynucleotides as used herein are interchangeable. One skilled in the art has the general knowledge that nucleic acids are polynucleotides, which can be hydrolyzed into the monomeric "nucleotides." The monomeric nucleotides can be hydrolyzed into nucleosides. As used herein polynucleotides include, but are not limited to, all nucleic acid sequences which are obtained by any means available in the art, including, without limitation, recombinant means, i.e., the cloning of nucleic acid sequences from a recombinant library or a cell genome, using ordinary cloning technology and PCR™, and the like, and by synthetic means.

As used herein, the terms "peptide," "polypeptide," and "protein" are used interchangeably, and refer to a compound comprised of amino acid residues covalently linked by peptide bonds. A protein or peptide must contain at least two amino acids, and no limitation is placed on the maximum number of amino acids that can comprise a protein's or peptide's sequence. Polypeptides include any peptide or protein comprising two or more amino acids joined to each other by peptide bonds. As used herein, the term refers to both short chains, which also commonly are referred to in the art as peptides, oligopeptides and oligomers, for example, and to longer chains, which generally are referred to in the art as proteins, of which there are many types. "Polypeptides" include, for example, biologically active fragments, substantially homologous polypeptides, oligopeptides, homodimers, heterodimers, variants of polypeptides, modified polypeptides, derivatives, analogs, fusion proteins, among others. The polypeptides include natural peptides, recombinant peptides, synthetic peptides, or a combination thereof.

The term "promoter" as used herein is defined as a DNA sequence recognized by the synthetic machinery of the cell, or introduced synthetic machinery, required to initiate the specific transcription of a polynucleotide sequence.

As used herein, the term "promoter/regulatory sequence" means a nucleic acid sequence which is required for expression of a gene product operably linked to the promoter/regulatory sequence. In some instances, this sequence may be the core promoter sequence and in other instances, this sequence may also include an enhancer sequence and other regulatory elements which are required for expression of the gene product. The promoter/regulatory sequence may, for example, be one which expresses the gene product in a tissue specific manner.

An "inducible" promoter is a nucleotide sequence which, when operably linked with a polynucleotide which encodes or specifies a gene product, causes the gene product to be produced in a cell substantially only when an inducer which corresponds to the promoter is present in the cell.

TCAR (also referred to as a standard CAR), as that term is used herein, refers to a CAR which shares functional and structural properties with a cell immune-function receptor or adaptor molecule from a T cell. In instances, a TCAR comprises an antigen domain, an intracellular signaling domain, and optionally one or more costimulatory domains.

A "tissue-specific" promoter is a nucleotide sequence which, when operably linked with a polynucleotide encodes or specified by a gene, causes the gene product to be produced in a cell substantially only if the cell is a cell of the tissue type.

A "signal transduction pathway" refers to the biochemical relationship between a variety of signal transduction molecules that play a role in the transmission of a signal from one portion of a cell to another portion of a cell. The phrase "cell surface receptor" includes molecules and complexes of molecules capable of receiving a signal and transmitting signal across the membrane of a cell.

The term "subject" is intended to include living organisms in which an immune response can be elicited (e.g., mammals).

As used herein, a "substantially purified" cell is a cell that is essentially free of other cell types. A substantially purified cell also refers to a cell which has been separated from other cell types with which it is normally associated in its naturally occurring state. In some instances, a population of substantially purified cells refers to a homogenous population of cells. In other instances, this term refers simply to cell that have been separated from the cells with which they are naturally associated in their natural state. In some embodiments, the cells are cultured *in vitro.* In other embodiments, the cells are not cultured *in vitro.*

As used herein, a 5' cap (also termed an RNA cap, an RNA 7-methylguanosine cap or an RNA m7G cap) is a modified guanine nucleotide that has been added to the "front" or 5' end of a eukaryotic messenger RNA shortly after the start of transcription. The 5' cap consists of a terminal group which is linked to the first transcribed nucleotide. Its presence is critical for recognition by the ribosome and protection from RNases. Cap addition is coupled to transcription, and occurs co-transcriptionally, such that each influences the other. Shortly after the start of transcription, the 5' end of the mRNA being synthesized is bound by a cap-synthesizing complex associated with RNA polymerase. This enzymatic complex catalyzes the chemical reactions that are required for mRNA capping. Synthesis proceeds as a multi-step biochemical reaction. The capping moiety can be modified to modulate functionality of mRNA such as its stability or efficiency of translation.

As used herein, "in vitro transcribed RNA" refers to RNA, preferably mRNA, that has been synthesized in vitro. Generally, the in vitro transcribed RNA is generated from an in vitro transcription vector. The in vitro transcription vector comprises a template that is used to generate the in vitro transcribed RNA.

As used herein, a "poly(A)" is a series of adenosines attached by polyadenylation to the mRNA. In the preferred embodiment of a construct for transient expression, the polyA is between 50 and 5000, preferably greater than 64, more preferably greater than 100, most preferably greater than 300 or 400. poly(A) sequences can be modified chemically or enzymatically to modulate mRNA functionality such as localization, stability or efficiency of translation.

As used herein, "polyadenylation" refers to the covalent linkage of a polyadenylyl moiety, or its modified variant, to a messenger RNA molecule. In eukaryotic organisms, most messenger RNA (mRNA) molecules are polyadenylated at the 3' end. The 3' poly(A) tail is a long sequence of adenine nucleotides (often several hundred) added to the pre-mRNA through the action of an enzyme, polyadenylate polymerase. In higher eukaryotes, the poly(A) tail is added onto transcripts that contain a specific sequence, the polyadenylation signal. The poly(A) tail and the protein bound to it aid in protecting mRNA from degradation by exonucleases. Polyadenylation is also important for transcription termination, export of the mRNA from the nucleus, and translation. Polyadenylation occurs in the nucleus immediately after transcription of DNA into RNA, but additionally can also occur later in the cytoplasm. After transcription has been terminated, the mRNA chain is cleaved through the action of an endonuclease complex associated with RNA polymerase. The cleavage site is usually characterized by the presence of the base sequence AAUAAA near the cleavage site. After the mRNA has been cleaved, adenosine residues are added to the free 3' end at the cleavage site.

As used herein, "transient" refers to expression of a non-integrated transgene for a period of hours, days or weeks, wherein the period of time of expression is less than the period of time for expression of the gene if integrated into the genome or contained within a stable plasmid replicon in the host cell.

The term "therapeutic" as used herein means a treatment and/or prophylaxis. A therapeutic effect is obtained by suppression, remission, or eradication of a disease state.

The term "transfected" or "transformed" or "transduced" as used herein refers to a process by which exogenous nucleic acid is transferred or introduced into the host cell. A "transfected" or "transformed" or "transduced" cell is one which has been transfected, transformed or transduced with exogenous nucleic acid. The cell includes the primary subject cell and its progeny.

The phrase "under transcriptional control" or "operatively linked" as used herein means that the promoter is in the correct location and orientation in relation to a polynucleotide to control the initiation of transcription by RNA polymerase and expression of the polynucleotide.

A "vector" is a composition of matter which comprises an isolated nucleic acid and which can be used to deliver the isolated nucleic acid to the interior of a cell. Numerous vectors are known in the art including, but not limited to, linear polynucleotides, polynucleotides associated with ionic or amphiphilic compounds, plasmids, and viruses. Thus, the term "vector" includes an autonomously replicating plasmid or a virus. The term should also be construed to include non-plasmid and non-viral compounds which facilitate transfer of nucleic acid into cells, such as, for example, polylysine compounds, liposomes, and the like. Examples of viral vectors include, but are not limited to, adenoviral vectors, adeno-associated virus vectors, retroviral vectors, lentiviral vectors, and the like.

By the term "stimulation," is meant a primary response induced by binding of a stimulatory molecule with its cognate ligand thereby mediating a signal transduction event, such as, but not limited to, signal transduction via the appropriate receptor, e.g., T or NK receptor.

Ranges: throughout this disclosure, various aspects of the invention can be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 2.7, 3, 4, 5, 5.3, and 6. This applies regardless of the breadth of the range.

### REGULATABLE NATURAL KILLER CELL CHIMERIC ANTIGEN RECEPTORS (RNKR-CARS)

In some aspects, a CAR described herein comprises a regulatable NKR-CAR (RNKR-CAR). In some embodiments, a RNKR-CAR is a CAR that comprises an element (e.g., domain) from a NK cell receptor (e.g., a NK receptor described herein, e.g., selected from Table 24) and that comprises a switch domain that allows it to be regulated (e.g., by a dimerization molecule described herein).

In embodiments, the RNKR-CAR is regulated (e.g., turned on) by the addition of a dimerization molecule described herein. For example, the RNKR-CAR does not signal to downstream effectors unless a dimerization molecule is added. In some embodiments, an advantage of an RNKR-CAR is the ability to temporally regulate (e.g., turn on) the activity of a NKR-CAR. Another advantage of an RNKR-CAR is that it does not require costimulation (e.g., it does not require the presence of a costimulatory domain) to cause proliferation of a cell expressing the RNKR-CAR. In embodiments, the RNKR-CAR is useful for regulating the specificity of a cytotoxic cell, e.g,. T cell, in order to control off-target activity of cytotoxic cell engineered to express the RNKR-CAR.

In an embodiment, the RNKR-CAR comprises an antigen binding member that confers specificity for a target cell, e.g., a cancer cell, which comprises the antigen bound by the antigen binding domain. In instances, the antigen binding member of a RNKR-CAR comprises a binding domain element (e.g., an antigen binding domain, an inhibitory extracellular domain, or a costimulatory extracellular domain), a transmembrane domain, a first switch domain, and optionally a NKR cytoplasmic domain. The antigen binding member can comprise domains in any orientation from N- to C- terminus or extracellular to intracellular direction. Exemplary antigen binding members and their components, e.g., antigen binding domains, extracellular domains of inhibitory molecules, or extracellular domains of costimulatory molecules, are described herein. Exemplary antigen binding domains, inhibitory molecules, costimulatory molecules, transmembrane domains, switch domains, and NKR cytoplasmic domains are described herein, e.g., in the **ANTIGEN BINDING DOMAIN, INHIBITORY MOLECULES, COSTIMULATORY MOLECULE LIGAND BINDING DOMAINS, TRANSMEMBRANE DOMAIN, DIMERIZATION SWITCHES, AND NK CELL IMMUNE-FUNCTION RECEPTORS (NKRS) ANDNK CELLS** (e.g., **KIR-CARS, KIR-CARS, NCRS, SLAM RECEPTORS, FC-BINDING RECEPTORS, LY49 AND RELATED KILLER CELL LECTIN-LIKE RECEPTORS)** sections.

In an embodiment, the RNKR-CAR comprises an intracellular signalling member that generates an intracellular signal in a regulatable manner, e.g., by addition of a molecule, e.g., a dimerization molecule described herein. The intracellular signal can optimize an immune effector property of the RNKR-CARX cell, e.g., activating signals, (e.g., cytolytic activity, cytokine secretion, cell survival, and/or proliferation). In other embodiments, e.g., in the case of an inhibitory RNKR-CARX, the intracellular signal leads to abrogation of activating signals, thereby leading to inhibition of NK cytolytic and cytokine producing activity.

Upon the presence of a dimerization molecule, the intracellular signalling member is coupled to (e.g., binds to, otherwise interacts with, or comes in close proximity to) the antigen binding member. For example, the dimerization molecule couples the first switch to the second switch. In an instance, the intracellular signalling member is coupled by the presence of a dimerization molecule to an extracellular recognition element, e.g., an antigen binding domain of the antigen binding member.

In embodiments, the intracellular binding member comprises a second switch domain, a NKR cytoplasmic domain or an intracellular signaling domain, and optionally a transmembrane domain or membrane tether. The intracellular signaling member can comprise domains in any orientation from N- to C- terminus. Exemplary intracellular binding members and their components are described herein. Exemplary switch domains, NKR cytoplasmic domains, intracellular signaling domains, and transmembrane domains/membrane tethers are described in herein, e.g., at the **INTRACELLULAR SIGNALING DOMAIN, TRANSMEMBRANE DOMAIN, DIMERIZATION SWITCHES, AND NK CELL IMMUNE-FUNCTION RECEPTORS (NKRS) AND NK CELLS** (e.g., **KIR-CARS, KIR-CARS, NCRS, SLAM RECEPTORS, FC-BINDING RECEPTORS, LY49 AND RELATED KILLERCELL LECTIN-LIKE RECEPTORS)** sections.

In embodiments, the antigen binding member comprises an extracellular hinge domain disposed between the transmembrane domain and the binding domain element. Exemplary extracellular hinge domains are described herein, e.g., in the **EXTRACELLULAR HINGE DOMAIN** section.

In embodiments, a RNKR-CAR comprises an element, e.g., domain, from any of the NKRs described herein, e.g., in the **NK CELL IMMUNE-FUNCTION RECEPTORS (NKRs) AND NK CELLS** (e.g., **KIR-CARS, KIR-CARS, NCRS, SLAM RECEPTORS, FC-BINDING RECEPTORS, LY49 AND RELATED KILLER CELL LECTIN-LIKE RECEPTORS)** section. Accordingly, a RNKR-CAR comprises any type of NKR-CAR (e.g., any NKR-CAR described herein) that is regulatable with a switch. For example, the RNKR-CAR comprises a regulatable killer immunoglobulin receptor-CAR (RKIR-CAR); a regulatable NCR-CAR (RNCR-CAR); a regulatable Fc receptor-CAR (RFcR-CAR); a regulatable Ly49 receptor-CAR (RLy49-CAR); or a regulatable SLAMF receptor-CAR (RSLAMF-CAR).

Also provided herein are nucleic acids encoding a RNKR-CAR described herein. Also provided are vector systems comprising nucleic acids encoding a RNKR-CAR described herein. Also provided are cells comprising a RNKR-CAR described herein, or a nucleic acid encoding a RNKR-CAR described herein, or a vector system comprising a nucleic acid encoding a RNKR-CAR described herein. Nucleic acids and vector systems are described in greater detail herein, e.g., in the **VECTORS** section. Cells suitable for use in accordance with the invention, e.g., to express or contain RNKR-CARs, are described herein, e.g., in the **SOURCES OF CELLS** section.

Also disclosed herein is a method of making a RNKR-CAR expressing cell described herein, method of using a RNKR-CAR expressing cell described herein, method of providing a RNKR-CAR expressing cell described herein, or method of treating a subject with a disease associated with a tumor antigen comprising administering a RNKR-CAR expressing cell described herein.

### COMBINATION OF RCAR AND NKR-CAR

Provided herein is a combination of a RCAR and NKR-CAR in/on a cell, e.g., where both types of CARs are expressed in/on the same cell. These cells are referred to as RCAR/NKR-CAR cells, or RCAR/NKR-CARX cells. In some cases, the NKR-CAR comprises an inhibitory NKR-CAR (inhNKR-CAR) and the RCAR comprises an activating RCAR. In some examples, the inhNKR-CAR dampens the activating (e.g., cytolytic activity, cytokine secretion, cell survival, and/or proliferation) signal of the RCAR, e.g., when the RCAR is activated by addition of a dimerization molecule. In some cases, this can prevent overactivation of cell killing properties (e.g., cytolytic activity, cytokine secretion) that may in turn cause killing of excessive numbers of non-target (e.g., non-cancer) cells. In this way, the presence of the inhNKR-CAR in the same cell as a RCAR may serve as a safety mechanism.

In some cases, the antigen binding domain of the RCAR is different from the antigen binding domain of the NKR-CAR (e.g., inhNKR-CAR). For example, the antigen binding domain of the RCAR targets a different antigen than the the antigen binding domain of the NKR-CAR (e.g., inhNR-CAR). In embodiments, the antigen binding domain comprises an antigen binding domain described herein, e.g., in the **ANTIGEN BINDING DOMAIN** section. For example, the antigen binding domain binds to a tumor antigen. In embodiments, the antigen binding domain of the inhNKR-CAR binds to a target antigen that is expressed on normal cells, e.g., non-tumor or non-cancerous cells, but is not highly expressed on tumor or cancerous cells.

In some cases, by targeting a non-cancer or normal cell, the inhNKR-CAR reduces the activation of cell killing properties by the RCAR when the RCAR/NKR-CARX cell is in proximity to the non-cancer or normal cell. For example, the inhNKR-CAR reduces the activation of cell killing properties of the RCAR when the inhNKR-CAR is bound to its target antigen on a non-cancer/normal cell. In other examples, a RCAR/NKR-CARX cell (that expresses both a RCAR and an inhNKR-CAR) exhibits a lower activation of cell killing properties compared to a cell expressing a RCAR without an inhNKR-CAR. In embodiments, when the antigen binding domains of the RCAR and the inhNKR-CAR both bind to their target antigens, the RCAR/inhNKR-CARX cell does not activate.

In embodiments, the antigen binding domain of the RCAR or inhNKR-CAR comprises a variable light domain and a variable heavy domain, and the other (e.g., RCAR or inhNKR-CAR) is not a scFv.

In some embodiments, the RCAR/NKR-CAR cell comprises a RCAR and a NKR-CAR. In embodiments, the RCAR/NKR-CAR cell comprises a nucleic acid encoding a RCAR and a NKR-CAR. In embodiments, a single nucleic acid molecule comprises a sequence encoding a RCAR and a sequence a NKR-CAR. In other embodiments, the nucleic acid comprises a first nucleic acid molecule comprising a sequence encoding a RCAR anda second nucleic acid molecule comprising a sequence encoding a NKR-CAR.

In embodiments, the RCAR/NKR-CAR cell comprises a vector/vector system comprising a nucleic acid encoding a RCAR and a NKR-CAR. In some examples, a single vector comprises a sequence encoding a RCAR and a sequence encoding a NKR-CAR. In other examples, the vector system comprises a first vector comprising a sequence encoding a RCAR and a second vector comprising a sequence encoding a NKR-CAR.

Nucleic acids and vectors/vector systems encoding an RCAR and/or NKR-CAR are described herein.

Methods of using or providing a RCAR/NKR-CAR cell are described herein.

In certain instances, the RCAR comprises a RCAR described herein, e.g., comprising an intracellular signalling member, an antigen binding member, and a transmembrane domain. In instances, the NKR-CAR comprises a NKR-CAR described herein, e.g., comprising an antigen binding domain, a transmembrane domain, and a cytoplasmic domain (e.g., NKR cytoplasmic domain). In some embodiments, the NKR-CAR comprises an inhibitory NKR-CAR, such as inhKIR-CAR, inhSLAMF-CAR, or inhL49-CAR.

### DIMERIZATION SWITCHES

In embodiments, a RCAR or RNKR-CAR described herein comprises a dimerization switch. Dimerization switches can be non-covalent or covalent, depending on the form of interaction between the switch domians.

### NON-COVALENT DIMERIZATION SWITCHES

In a non-covalent dimerization switch, the dimerization molecule promotes a non-covalent interaction between the switch domains. Examples of non-covalent dimerization switches include the FKBP/FRAP-Based Dimerization Switches, GyrB-GyrB Based Dimerization Switches and Gibberelin-Based Dimerization Switches, described herein.

### FKBP/FRB-BASED DIMERIZATION SWITCHES.

FKBP12 (FKBP, or FK506 binding protein) is an abundant cytoplasmic protein that serves as the initial intracellular target for the natural product immunosuppressive drug, rapamycin. Rapamycin binds to FKBP and to the large PI3K homolog FRAP (RAFT, mTOR), thereby acting to dimerize these molecules.

In embodiments, an FKBP/FRAP based switch, also referred to herein as an FKBP/FRB, based switch can use a heterodimerization molecule, e.g., rapamycin or a rapamycin analog. FRB is a 93 amino acid portion of FRAP, that is sufficient for binding the FKBP-rapamycin complex (Chen, J., Zheng, X. F., Brown, E. J. & Schreiber, S. L. (1995) Identification of an 11-kDa FKBP12-rapamycin-binding domain within the 289-kDa FKBP12-rapamycin-associated protein and characterization of a critical serine residue. Proc Natl Acad Sci U S A 92: 4947-51).

The sequences of FKBP is as follows:
FKBP

In embodiments, an FKBP switch domain can comprise a FRB binding fragment of FKBP, e.g., the underlined portion of SEQ ID NO 1, which is:

The sequence of FRB is as follows:

In an embodiment, one switch domain comprises amino acid residues disclosed in SEQ ID NO: 1, or an FRB binding fragment or analog thereof, e.g., SEQ ID NO:141, and one switch domain comprises amino acid residues disclosed in SEQ ID NO: 2 or an FKPB binding fragment or analog thereof. In an embodiment, the FRB binding fragment of FKBP comprises 30, 35, 40, 45, 50, 55, 60, 70, 75, 80, 85 or 90 amino acids of the sequence of FKBP, SEQ ID NO:1, or SEQ ID NO: 141. In an embodiment, the FRB binding fragment of FKBP is at least 5, 10, 15, 20, 25, 30, 35, 40 amino acids shorter than the sequence of FKBP, SEQ ID NO:1, or SEQ ID NO: 141. In an embodiment, the FKBP binding fragment of FRB comprises 30, 35, 40, 45, 50, 55, 60, 70, 75, 80, 85 or 90 amino acids of the sequence of FRB, SEQ ID NO:2. In an embodiment, the FKBP binding fragment of FRB is at least 5, 10, 15, 20, 25, 30, 35, 40 amino acids shorter than the sequence of FRB, SEQ ID NO:2. In an embodiment, the FKBP binding fragment or analog of FRB comprises one or more mutations which enhances the formation of a complex between an FKBP switch domain, an FRB switch domain, and the dimerization molecule, e.g., rapamycin, or a rapalog, e.g., RAD001, or a mutation described in the section herein entitled **MODIFIED FKBP/FRB-BASED DIMERIZATION SWITCHES.** E.g., the FKBP binding fragment or analog of FRB comprises: an E2032 mutation, e.g., an E2032I mutation or E2032L mutation; a T2098 mutation, e.g., a T2098L mutation; or an E2032 and a T2098 mutation, e.g., an E2032I and a T2098L or an E2032L and a T2098L mutation.

In an embodiment, one switch domain comprises amino acid residues disclosed in SEQ ID NO: 1 (or SEQ ID NO:141) and one switch domain comprises amino acid residues disclosed in SEQ ID NO: 2.

In embodiments, a switch domain, or a rapamycin, or rapalog, e.g., RAD001, binding sequence of thereof, will have at least 60, 70, 75, 80, 85, 90, 95, 96, 97, 98, or 99% identity with the FKBP sequence of SEQ ID NO: 1 (or SEQ ID NO: 141). In embodiments, a switch domain, or a rapamycin, or rapalog, e.g., RAD001, binding sequence thereof, will differ by no more than 35, 30, 25, 20, 15, 10, 5, 4, 3, 2, or 1 amino acid residues from the corresponding the sequence of SEQ ID NO: 1 (or SEQ ID NO: 141).

In an embodiment, one switch domain binds FRB (or FRB and rapamycin, or a rapamycin analog) and has at least 60, 70, 75, 80, 85, 90, 95, 96, 97, 98, or 99% identity with, or differs by no more than 35, 30, 25, 20, 15, 10, 5, 4, 3, 2, or 1 amino acid residues from, the FKBP sequence of SEQ ID NO: 1.

In embodiments, a switch domain, or a rapamycin, or rapalog, e.g., RAD001, binding sequence of thereof, will have at least 60, 70, 75, 80, 85, 90, 95, 96, 97, 98, or 99% identity with the FRB sequence of SEQ ID NO: 2. In embodiments, a switch domain, or a rapamycin, or rapalog, e.g., RAD001, binding sequence thereof, will differ by no more than 35, 30, 25, 20, 15, 10, 5, 4, 3, 2, or 1 amino acid residues from the corresponding the sequence of SEQ ID NO: 2.

In an embodiment the other switch domain binds FKBP (or FKBP and rapamycin, or a rapamycin analog) and has at least 60, 70, 75, 80, 85, 90, 95, 96, 97, 98, or 99% identity with, or differs by no more than 35, 30, 25, 20, 15, 10, 5, 4, 3, 2, or 1 amino acid residues from, the FRB sequence of SEQ ID NO: 2. See, e.g., Fig. 2.

In embodiments a switch domain, or a rapamycin, or rapalog, e.g., RAD001, binding sequence of thereof, will have at least 60, 70, 75, 80, 85, 90, 95, 96, 97, 98, or 99% identity with a non-human, e.g., mammalian, e.g., rodent, e.g., mouse, rat or hamster, FKBP sequence. In embodiments, a switch domain, or a rapamycin, or rapalog, e.g., RAD001, binding sequence thereof, will differ by no more than 35, 30, 25, 20, 15, 10, 5, 4, 3, 2, or 1 amino acid residues from a non-human, e.g., mammalian, e.g., rodent, e.g., mouse, rat or hamster FKBP.

In an embodiment, the one switch domain binds FRB (or FRB and rapamycin, or a rapamycin analog) and has at least 60, 70, 75, 80, 85, 90, 95, 96, 97, 98, or 99% identity with, or differs by no more than 35, 30, 25, 20, 15, 10, 5, 4, 3, 2, or 1 amino acid residues from, a non-human, e.g., mammalian, e.g., rodent, e.g., mouse, rat or hamster, FKBP.

In embodiments, a switch domain, or a rapamycin, or rapalog, e.g., RAD001, binding sequence of thereof, will have at least 60, 70, 75, 80, 85, 90, 95, 96, 97, 98, or 99% identity with a non-human, e.g., mammalian, e.g., rodent, e.g., mouse, rat or hamster, FRB sequence. In embodiments, a switch domain, or a rapamycin, or rapalog, e.g., RAD001, binding sequence thereof, will differ by no more than 35, 30, 25, 20, 15, 10, 5, 4, 3, 2, or 1 amino acid residues from a non-human, e.g., mammalian, e.g., rodent, e.g., mouse, rat or hamster, FRB sequence.

In an embodiment the other switch domain binds FKBP (or FKBP and rapamycin, or a rapamycin analog) and has at least 60, 70, 75, 80, 85, 90, 95, 96, 97, 98, or 99% identity with, or differs by no more than 35, 30, 25, 20, 15, 10, 5, 4, 3, 2, or 1 amino acid residues from a non-human, e.g., mammalian, e.g., rodent, e.g., mouse, rat or hamster, FRB.

"FKBP/FRAP, e.g., an FKBP/FRB, based switch" as that term is used herein, refers to a dimerization switch comprising : a first switch domain, which binds rapamycin, or a rapamycin analog, and has at least 70, 75, 80, 85, 90, 95, 96, 97, 98, or 99% identity with, or differs by no more than 30, 25, 20, 15, 10, 5, 4, 3, 2, or 1 amino acid residues from, the FKBP sequence of SEQ ID NO:1 or SEQ ID NO: 141; and a second switch domain, which binds rapamycin, or a rapamycin analog, and has at least 70, 75, 80, 85, 90, 95, 96, 97, 98, or 99% identity with, or differs by no more than 30, 25, 20, 15, 10, 5, 4, 3, 2, or 1 amino acid residues from, the FRB sequence of SEQ ID NO: 2. See, e.g., Fig. 2.

In embodiments, an FKBP/FRB, based switch can use a heterodimerization molecule, e.g., a rapamycin analog, that lacks rapamycin's undesirable properties, e.g., it lacks or has less immunosuppressive activity.

### MODIFIED FKBP/FRB-BASED DIMERIZATION SWITCHES

Also provided herein are improved FKBP/FRB dimerization switches, in which the FRB-based switch domain comprises one or more mutations that optimize performance, e.g., that alter, e.g., enhance the formation of a complex between an FKBP switch domain, an FRB switch domain, and the dimerization molecule, e.g., rapamycin, or a rapalog, e.g., RAD001. In an embodiment, the FRB-based switch domain comprising one or more mutations, also referred to herein as a "mutant FRB",comprises increased affinity for a dimerization molecule, e.g., rapamycin or a rapalog, e.g., in comparison to the affinity of a wild-type FRB-based switch domain for the dimerization molecule.

Without wishing to be bound by theory, it is believed that mutations described herein can allow the use of lower concentrations of the dimerization molecule to assemble the RCAR or RNKR-CAR. Some dimerization molecules that dimerize FKBP/FRB dimerization switches exhibit immunosuppressive effects, and therefore prevent or mitigate the beneficial effects of RCAR or RNKR-CAR therapy. Thus, the ability to use lower concentrations of the dimerization molecule to assemble RCARs or RNKR-CARs can increase the therapeutic window for RCAR- or RNKR-CAR-expressing cell activity, e.g., increase the range of dosages of dimerization molecule that can be used without inducing immunosuppression, and therefore results in the increase of therapeutic benefit of the RCAR- or RNKR-CAR-expressing cell. Alternatively or in addition, without wishing to be bound by theory, it is believed that mutations described herein can result in preferential binding of the dimerization molecule to the mutant FRB instead of binding and inhibiting endogenous FRAP/mTOR. Preventing the inhibition of endogenous FRAP/mTORdecreases or inhibits adverse effects associated with endogenous FRAP/mTOR inhibition, e.g., toxicity or immunosuppression.

A mutant FRB can be identified using the screening method described herein. First, regions or amino acid residues in a wild-type FRB that are present in the dimerization molecule-binding pocket of the natively folded wild-type FRB, or contribute to the interaction, e.g., directly or indirectly, with the dimerization molecule, can be determined from structural data, e.g., x-ray crystallographic structures, or computer modeling, e.g., homology or comparative modeling of homologous proteins bound to the dimerization molecule or derivatives thereof. A candidate mutant FRB can be generated by mutating a target region or target residue e.g., by PCR site-directed mutagenesis. In an embodiment, alibrary of candidate FRB mutants comprising one or more point mutations can be generated using a saturation mutagenesis approach, where a target residue is mutated to all other possible amino acids by randomizing the codon that encodes the target residue. Randomization of each codon corresponding to a target residue can be achieved by using a codon library that represents all 20 amino acids, e.g., a NNK library, where N can be adenine (A), cytosine (C), guanine (G), or thymine (T), and K can be guanine (G) or thymine (T). Table 13 shows the codon distribution of an exemplary NNK library and the corresponding amino acids. Each codon in the NNK library is incorporated at the target residue position, thereby producing a library of candidate FRB mutants for each target residue position where the target residue position has been mutated to every other possible amino acid. The library of candidate FRB mutants can then be screened to identify FRB mutants described herein.

**Table 13. NNK Library**

| DNA base N defined to be A/C/G/T and K defined to be G/T. | |
|---|---|
| **NNK** | **Amino Acid** |
| AAG | Lysine, Lys, K |
| AAT | Asparagine, Asn, N |
| ACG | Theronine, Thr, T |
| ACT | Theronine, Thr, T |
| AGG | Arginine, Arg, R |
| AGT | Serine, Ser, S |
| ATG | Methionine, Met, M |
| ATT | Isoleucine, Ile, I |
| CAG | Glutamine, Gln, Q |
| CAT | Histidine, His, H |
| CCG | Proline, Pro, P |
| CCT | Proline, Pro, P |
| CGG | Arginine, Arg, R |
| CGT | Arginine, Arg, R |
| CTG | Leucine, Leu, L |
| CTT | Leucine, Leu, L |
| GAG | Glutamic acid, Glu, E |
| GAT | Aspartic acid, Asp, D |
| GCG | Alanine, Ala, A |
| GCT | Alanine, Ala, A |
| GGG | Glycine, Gly, G |
| GGT | Glycine, Gly, G |
| GTG | Valine, Val, V |
| GTT | Valine, Val, V |
| TAG | Stop |
| TAT | Tyrosine, Tyr, Y |
| TCG | Serine, Ser, S |
| TCT | Serine, Ser, S |
| TGG | Tryptophan, Trp, W |
| TGT | Cysteine, Cys, C |
| TTG | Leucine, Leu, L |
| TTT | Phenylalanine, Phe, F |

Various screening assays can be used to evaluate each candidate mutant FRB to identify mutant FRB which enhances the formation of a complex between an FKBP switch domain, an FRB switch domain, and the dimerization molecule, e.g., rapamycin, or a rapalog, e.g., RAD001. In a direct binding assay, unlabeled candidate mutant FRB is incubated in solution with tagged wild-type FKBP in the presence of the dimerization molecule, e.g., under conditions suitable for binding of FRB to the dimerization molecule and dimerization of FRB and FKBP. Tagged FKBP can be removed from the reaction by affinity purification; candidate mutant FRB that is able to bind the dimerization molecule and dimerize with the tagged FKBP will also be removed. The amount of free candidate mutant FRB that does not dimerize with the tagged wild-type FKBP can be calculated by determining protein concentration of the reaction. EC50 values for direct binding affinity can then be calculated using methods known in the art.

Alternatively or in addition to the direct binding assay described above, a competition binding assay can also be performed to identify a mutant FRB. In this assay, an untagged candidate mutant FRB is incubated in solution with: 1) wild-type FKBP linked to a first tag, e.g., biotinylated wild-type FKBP; 2) wild-type FRB linked to a second tag, e.g., FLAG-tagged wild-type FRB; and 3) the dimerization molecule; under conditions suitable for binding of FRB to the dimerization molecule and dimerization of FRB and FKBP. The tagged wild-type FKBP and tagged wild-type FRB can be removed from the reaction by affinity purification. The amount of free candidate mutant FRB that does not dimerize with the tagged wild-type FKBP in the presence of wild-type FRB can be calculated by determining protein concentration of the reaction.EC50 values for competition binding affinity can then be calculated using methods known in the art.

In an embodiment, a mutant FRB comprises one or more, e.g., 2, 3, 4, 5, 6, 7, 8, 9, 10 or more, mutations in the amino acid sequence of a wild-type FRB, e.g., a FRB comprising SEQ ID NO: 2. The mutant FRB comprises increased affinity for a dimerization molecule, e.g., as compared to the affinity of wild-type FRB for the dimerization molecule. The amino acid position numbering of a wild-type or mutant FRB referred to herein can be determined from SEQ ID NO: 2, where the first amino acid of SEQ ID NO: 2 is position 2021 and the last amino acid of SEQ ID NO: 2 is position 2113.

In an embodiment, a mutant FRB comprises one or more mutations at the amino acid(s) selected from a leucine at position 2031 (L2031), a glutamic acid at position 2032 (E2032), a serine at position 2035 (S2035), an arginine at position 2036 (R2036), a phenylalanine at position 2039(F2039), a glycine at position 2040 (G2040), a threonine at position 2098 (T2098), a tryptophan at position 2101(W2101), an aspartic acid at position 2102(D2102), a tyrosine at position 2105(Y2105), and a phenylalanine at position 2108 (F2108), where L2031, E2032, S2035, R2036, F2039, G2040, T2098, W2101, D2102, Y2105, and/or F2108 is mutated to any other naturally-occurring amino acid. In an embodiment, a mutant FRB comprises an amino acid sequence selected from SEQ ID NOs: 170-180, where X can be any naturally occurring amino acid. Amino acid sequences of exemplary mutant FRB switch domains having increased affinity for RAD001 are provided in Table 14 below. A screen as described herein can be performed to identify a mutant FRB.

**Table 14. Exemplary mutant FRBs.**

| **FRB mutant** | **Amino Acid Sequence** | **SEQ ID NO:** |
|---|---|---|
| L2031 mutant | | 170 |
| E2032 mutant | | 171 |
| S2035 mutant | | 172 |
| R2036 mutant | | 173 |
| F2039 mutant | | 174 |
| G2040 mutant | | 175 |
| T2098 mutant | | 176 |
| W2101 mutant | | 177 |
| D2102 mutant | | 178 |
| Y2105 mutant | | 179 |
| F2108 mutant | | 180 |

A screen was performed to evaluate candidate mutant FRBs, as further described in Example 17.

In an embodiment, a mutant FRB e.g., comprises one or more mutations at the amino acid(s) selected from L2031, E2032, S2035, R2036, F2039, G2040, T2098, W2101, D2102, Y2105, and F2108, where the wild-type amino acid is mutated to any other naturally-occurring amino acid. In an embodiment, a mutant FRB comprises a mutation at E2032, where E2032 is mutated to phenylalanine (E2032F), methionine (E2032M), arginine (E2032R), valine (E2032V), tyrosine (E2032Y),isoleucine (E2032I), e.g., SEQ ID NO: 181, or leucine (E2032L), e.g., SEQ ID NO: 182. In an embodiment, a mutant FRB comprises a mutation at T2098, where T2098 is mutated to phenylalanine (T2098F) or leucine (T2098L), e.g., SEQ ID NO: 183. In an embodiment, a mutant FRB comprises a mutation at E2032 and at T2098, where E2032 is mutated to any amino acid, and where T2098 is mutated to any amino acid, e.g., SEQ ID NO: 184. In an embodiment, a mutant FRB comprises an E2032I and a T2098L mutation, e.g., SEQ ID NO: 185. In an embodiment, a mutant FRB comprises an E2032L and a T2098L mutation, e.g., SEQ ID NO: 186. Amino acid sequences of exemplary mutant FRB switch domains are provided in Table 15 below.

**Table 15. Exemplary mutant FRBs.**

| **FRB mutant** | **Amino Acid Sequence** | **SEQ ID NO:** |
|---|---|---|
| E2032I mutant | | 181 |
| E2032L mutant | | 182 |
| T2098L mutant | | 183 |
| E2032X, T2098X mutant | | 184 |
| E2032I, T2098L mutant | | 185 |
| E2032L, T2098L mutant | | 186 |

The mutant FRB allows the use of dosages of RAD001 lower than the dosage currently used in clinical settings, or lower than a dosage that induces immunosuppression in a subject, to stimulate dimerization of a FKBP-FRB based switch. In an embodiment, a dose of RAD001 that stimulates dimerization of a modified FKBP-FRB based switch, e.g., comprising a mutant FRB described herein, is lower than the dosage currently used to treat cancer, e.g., a dose of RAD001 comprises less than 10 mg per day, e.g., less than 10 mg, 9 mg, 8 mg, 7 mg, 6 mg, 5 mg, 4 mg, 3 mg, 2 mg, 1 mg per day. In an embodiment, a dose of RAD001 that stimulates dimerization of a modified FKBP-FRB based switch, e.g., comprising a mutant FRB described herein, comprises less than 1 mg per day, e.g., 0.5 mg per day. In an embodiment, a dose of RAD001 that stimulates dimerization of a modified FKBP-FRB based switch, e.g., comprising a mutant FRB described herein, comprises less than 10 mg per week, e.g., 5 mg per week. Additional dosages of dimerization molecules suitable for use with the modified FKBP-FRB based switches are described herein in the section entited "Pharmaceutial Compositions and Treatments".

### AP21967 AND AP21967-BINDING FRB

In an embodiment, the dimerization molecule is a rapamycin analog, e.g., AP21967, that does not bind wild-type endogenous FRAP, e.g., FRB, but that does bind a modified FRB. While not wishing to be bound by theory it is believed that the lack of binding to endogenous FRB reduces immunosuppressive activity. An exemplary modified FRB contains a single amino acid change (T2098L). Incorporation of this mutation into the FRB component of a dimerization switch allows AP21967 to be used as a dimerization molecule.

In an embodiment, one switch domain comprises sequence from FKBP that binds a rapamycin analog, e.g., AP21967, and the other switch domain comprises sequence from FRB that binds a rapamycin analog, e.g., AP21967, binding.

In an embodiment, one switch domain comprises amino acid residues disclosed in SEQ ID NO: 1 and one switch domain comprises amino acid residues disclosed in SEQ ID NO: 2.

In embodiments the switch domain, or a rapamycin analog, e.g., AP21967, binding sequence of thereof, will have at least 70, 75, 80, 85, 90, 95, 96, 97, 98, or 99% identity with the FKBP sequence of SEQ ID NO: 1. In embodiments, the switch domain, or a rapamycin analog, e.g., AP21967, binding sequence thereof, will differ by no more than 30, 25, 20, 15, 10, 5, 4, 3, 2, or 1 amino acid residues from the corresponding the sequence of SEQ ID NO: 1

In embodiments the switch domain, or a rapamycin analog, e.g., AP21967, binding sequence of thereof, will have at least 70, 75, 80, 85, 90, 95, 96, 97, 98, or 99% identity with the FRB sequence of SEQ ID NO: 142. In embodiments, the switch domain, or a rapamycin analog, e.g., AP21967, binding sequence thereof, will differ by no more than 30, 25, 20, 15, 10, 5, 4, 3, 2, or 1 amino acid residues from the corresponding FRB sequence of SEQ ID NO: 142.

### Structure 1: AP21967

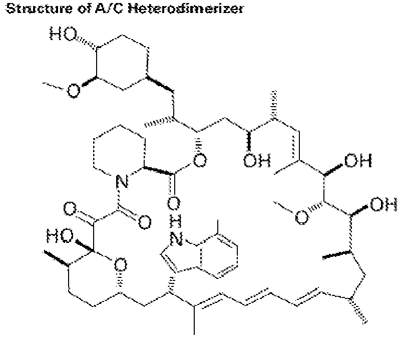

Similar switches have been used to control the localization and activity of signaling domains as described above (see, e.g., Graef, I. A., Holsinger, L. J., Diver, S., Schreiber, S. L. & Crabtree, G. R. (1997) Proximity and orientation underlie signaling by the non-receptor tyrosine kinase ZAP70. Embo J 16: 5618-28).

Candidate sequences for use as switch domain comprising a rapamycin analog, e.g., AP21967, binding sequence from FKBP, or a rapamycin analog, e.g., AP21967, binding sequence from FRB can be evaluated by incorporating the candidate into a system such as that described herein.

### DIMERIZATION MOLECULES FOR FKPB/FRB BASED SWITCHES

Rapamycin and rapamycin analogs (sometimes referred to as rapalogs), can be used as dimerization molecules in FKBP-FRB based dimerization switches. In an embodiment the dimerization molecule can be selected from rapamycin (sirolimus), RAD001 (everolimus), zotarolimus, temsirolimus, AP-23573 (ridaforolimus), biolimus and AP21967.

Rapamycin is a known macrolide antibiotic produced by *Streptomyces hygroscopicus* having the structure shown in Formula A.

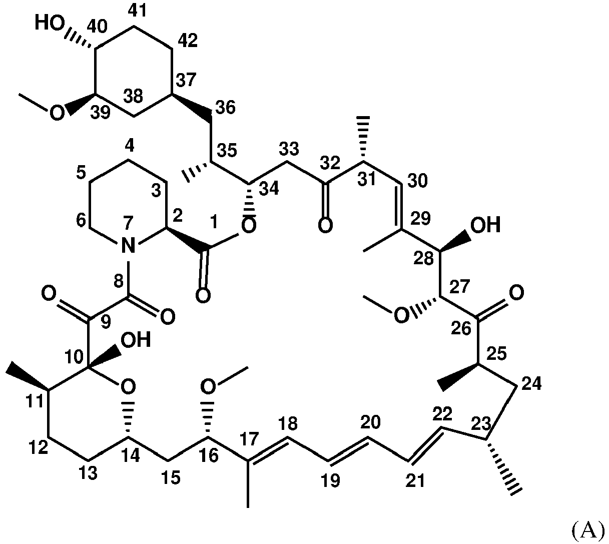

See, e.g., McAlpine, J.B., et al., J. Antibiotics (1991) 44:688; Schreiber, S.L., et al., J. Am. Chem. Soc. (1991) 113:7433; U.S. Patent No. 3,929,992. There are various numbering schemes proposed for rapamycin. To avoid confusion, when specific rapamycin analogs are named herein, the names are given with reference to rapamycin using the numbering scheme of formula A.

Numerous rapamycin analogs can be used as a heterodimerization molecule in a FKBP/FRAP-based dimerization switch. For example, O-substituted analogues in which the hydroxyl group on the cyclohexyl ring of rapamycin is replaced by OR₁ in which R₁ is hydroxyalkyl, hydroxyalkoxyalkyl, acylaminoalkyl, or aminoalkyl; e.g. RAD001, also known as, everolimus as described in US 5,665,772 and WO94/09010. Other suitable rapamycin analogs include those substituted at the 26- or 28-position. The rapamycin analog may be an epimer of an analog mentioned above, particularly an epimer of an analog substituted in position 40, 28 or 26, and may optionally be further hydrogenated, e.g. as described in US 6,015,815, WO95/14023 and WO99/15530, e.g. ABT578 also known as zotarolimus or a rapamycin analog described in US 7,091,213, WO98/02441 and WO01/14387, e.g. AP23573 also known as ridaforolimus.

Examples of rapamycin analogs suitable for use in the present invention from US 5,665,772 include, but are not limited to, 40-O-benzyl-rapamycin, 40-O-(4'-hydroxymethyl)benzyl-rapamycin, 40-O-[4'-(1,2-dihydroxyethyl)]benzyl-rapamycin, 40-O-allyl-rapamycin, 40-O-[3'-(2,2-dimethyl-1,3-dioxolan-4(S)-yl)-prop-2'-en-1'-yl]-rapamycin, (2'E,4'S)-40-O-(4',5'-dihydroxypent-2'-en-1'-yl)-rapamycin, 40-O-(2-hydroxy)ethoxycarbonylmethyl-rapamycin, 40-O-(2-hydroxy)ethyl-rapamycin, 40-O-(3-hydroxy)propyl-rapamycin, 40-O-(6-hydroxy)hexyl-rapamycin, 40-O-[2-(2-hydroxy)ethoxy]ethyl-rapamycin, 40-O-[(3S)-2,2-dimethyldioxolan-3-yl]methyl-rapamycin, 40-O-[(2S)-2,3-dihydroxyprop-1-yl]-rapamycin,40-O-(2-acetoxy)ethyl-rapamycin,40-O-(2-nicotinoyloxy)ethyl-rapamycin, 40-O-[2-(N-morpholino)acetoxylethyl-rapamycin, 40-O-(2-N-imidazolylacetoxy)ethyl-rapamycin, 40-O-[2-(N-methyl-N'-piperazinyl)acetoxy]ethyl-rapamycin, 39-O-desmethyl-39,40-O,O-ethylene-rapamycin, (26R)-26-dihydro-40-O-(2-hydroxy)ethyl-rapamycin, 40-O-(2-aminoethyl)-rapamycin, 40-O-(2-acetaminoethyl)-rapamycin, 40-O-(2-nicotinamidoethyl)-rapamycin, 40-O-(2-(N-methyl-imidazo-2'-ylcarbethoxamido)ethyl)-rapamycin, 40-O-(2-ethoxycarbonylaminoethyl)-rapamycin, 40-O-(2-tolylsulfonamidoethyl)-rapamycin and 40-O-[2-(4',5'-dicarboethoxy-1',2',3'-triazol-1'-yl)-ethyl]-rapamycin.

Other examples of rapamycin analogs where the hydroxyl group on the cyclohexyl ring of rapamycin and/or the hydroxy group at the 28 position is replaced with an hydroxyester group are known, for example, rapamycin analogs found in US RE44,768, e.g. temsirolimus.

Other rapamycin analogs include those wherein the methoxy group at the 16 position is replaced with another substituent, preferably (optionally hydroxy-substituted) alkynyloxy, benzyl, orthomethoxybenzyl or chlorobenzyl and/or wherein the mexthoxy group at the 39 position is deleted together with the 39 carbon so that the cyclohexyl ring of rapamycin becomes a cyclopentyl ring lacking the 39 position methyoxy group; e.g. as described in WO95/16691 and WO96/41807. The analogs can be further modified such that the hydroxy at the 40-position of rapamycin is alkylated and/or the 32-carbonyl is reduced.

Rapamycin analogs from WO95/16691 include, but are not limited to, 16-demthoxy-16-(pent-2-ynyl)oxy-rapamycin, 16-demthoxy-16-(but-2-ynyl)oxy-rapamycin, 16-demthoxy-16-(propargyl)oxy-rapamycin, 16-demethoxy-16-(4-hydroxy-but-2-ynyl)oxy-rapamycin, 16-demthoxy-16-benzyloxy-40-O-(2-hydroxyethyl)-rapamycin, 16-demthoxy-16-benzyloxy-rapamycin, 16-demethoxy-16-*ortho*-methoxybenzyl-raparnycin, 16-demethoxy-40-O-(2-methoxyethyl)-16-pent-2-ynyl)oxy-rapamycin, 39-demethoxy-40-desoxy-39-formyl-42-nor-rapamycin, 39-demethoxy-40-desoxy-39-hydroxymethyl-42-nor-rapamycin, 39-demethoxy-40-desoxy-39-carboxy-42-nor-rapamycin, 39-demethoxy-40-desoxy-39-(4-methyl-piperazin-1-yl)carbonyl-42-nor-rapamycin, 39-demethoxy-40-desoxy-39-(morpholin-4-yl)carbonyl-42-nor-rapamycin, 39-demethoxy-40-desoxy-39-[N-methyl, N-(2-pyridin-2-yl-ethyl)]carbamoyl-42-nor-rapamycin and 39-demethoxy-40-desoxy-39-(p-toluenesulfonylhydrazonomethyl)-42-nor-rapamycin.

Rapamycin analogs from WO96/41807 include, but are not limited to, 32-deoxo-rapamycin, 16-O-pent-2-ynyl-32-deoxo-rapamycin, 16-O-pent-2-ynyl-32-deoxo-40-O-(2-hydroxy-ethyl)-rapamycin, 16-O-pent-2-ynyl-32-(S)-dihydro-40-O-(2-hydro xyethyl)-rapamycin, 32(S)-dihydro-40-O-(2-methoxy)ethyl-rapamycin and 32(S)-dihydro-40-O-(2-hydroxyethyl)-rapamycin.

Another suitable rapamycin analog is biolimus as described in US2005/0101624.

RAD001, otherwise known as everolimus (Afinitor®), has the chemical name (41R,9S,12S,15R,16E,18R,19R,21R,23S,24E,26E,28E,30S,32S,35R)-1,18-dihydroxy-12-{(1R)-2-[(1S,3R,4R)-4-(2-hydroxyethoxy)-3-methoxycyclohexyl]-1-methylethyl}-19,30-dimethoxy-15,17,21,23,29,35-hexamethyl-11,36-dioxa-4-aza-tricyclo[30.3.1.04,9]hexatriaconta-16,24,26,28-tetraene-2,3,10,14,20-pentaone (also known as 40-O-(2-hydroxy)ethyl-rapamycin) and the following chemical structure:

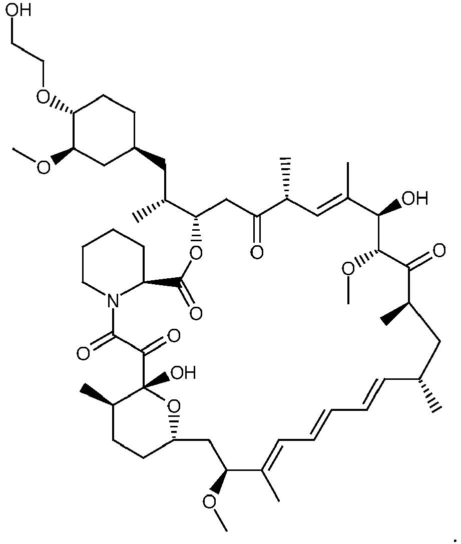

### GYRB-GYRB BASED DIMERIZATION SWITCHES

Coumermycin, a product of *Streptomyces,* binds the amino-terminal 24K subdomain of the B subunit of bacterial DNA gyrase, GyrB. Coumermycin binds two GyrB subunits, see, e.g., Rarrar et al., (1996) Activation of the Raf-1 kinase cascade by coumermycin induced dimerization, Nature 383: 178; Gilbert et al. (1994) The 24 kDa N-terminal sub-domain of the DNA gyrase B protein binds coumarin drugs, Molecular Microbiology 12: 365. Thus, coumermcyn can be used as a dimerization molecule in a homodimerization switch comprising switch domains that comprise a coumermycin binding sequence of GyrB.

In an embodiment the switch domain comprises a coumermycin binding sequence from the 24 K Da amino terminal sub-domain of GyrB.

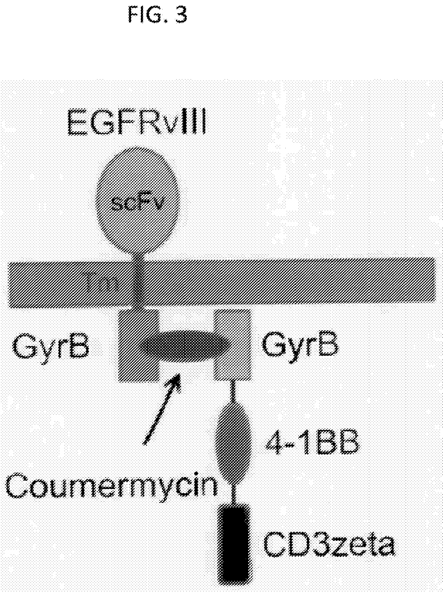

In embodiments the switch domain, or a coumermycin binding sequence of the switch domain thereof, will have at least 70, 75, 80, 85, 90, 95, 96, 97, 98, or 99% identity with the GyrB sequence of Rarrar et al., (1996). In embodiments, the switch domain, or a coumermycin binding sequence thereof, will differ by no more than 30, 25, 20, 15, 10, 5, 4, 3, 2, or 1 amino acid residues from the corresponding sequence of Rarrar et al., (1996). See, e.g., Fig. 3.

Candidate sequences for use as switch domain comprising coumermycin binding sequence from the 24 K Da amino terminal sub-domain of GyrB, can be evaluated by incorporating the candidate into a system such as that described in Rarrar et al., (1996).

### Structure 2: coumermycin

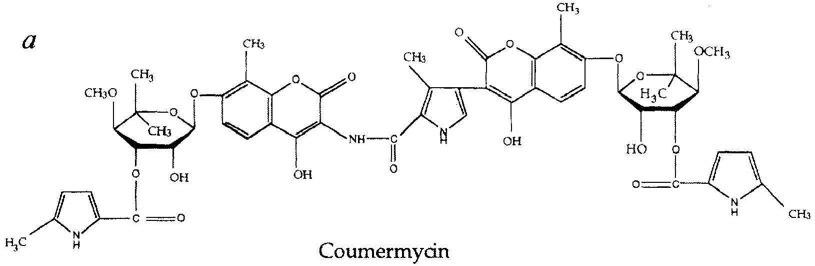

### GIBBERELLIN-BASED DIMERIZATION SWITCHES.

Gibberellins are plant hormones that regulate plant growth and development. Gibberellin binds to its receptor, gibberellin insensitive dwarf 1 (GID1) and induces a conformational change in GID1. The new conformation allows GID1 to bind another protein, gibberellin insentivive (GAI). Gibberellin, or a giberellin analog, e.g., GA₃-AM/GA₃, can be used to dimerize a switch domain comprising GA₃ binding sequence from GID1 (a GIDI switch domain) and a switch domain comprising sequence from GAI sufficient to bind GA₃-bound GID1. GA₃-AM can cross the plasma membrane of target cells. Once inside the cells, GA₃-AM is cleaved by an esterase to form GA₃. See Miyamoto et al. (2010) Rapid and orthogonal logic gating with a gibberellins-induced dimerization system, Nat. Chem. Biol. 8:465.

In an embodiment one switch domain (a GAI switch domain) comprises, a sequence of GAI sufficient to bind to a gibberellin analog, e.g., GA_{3;} and once bound to the analog, e.g., GA_{3,} bind to GID1; and one switch domain (a GID1 switch domain) comprises sequence of GID1 sufficient to bind to a GAI switch domain bound to a gibberellin analog, e.g., GA₃.

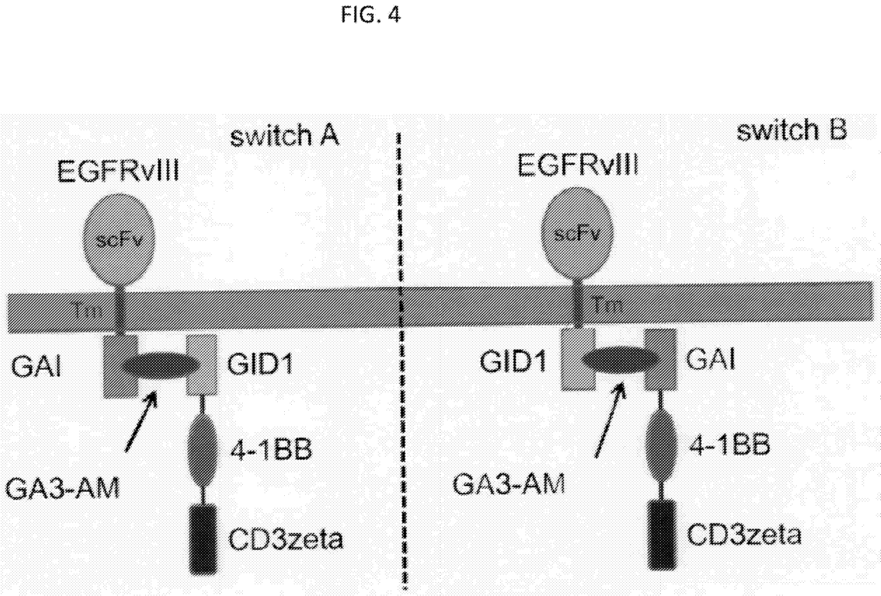

In embodiments, a GAI switch domain, or a sequence of GAI is sufficient to bind to a gibberellin analog, e.g., GA_{3;} and once bound to the analog, e.g., GA_{3,} bind to GID1, thereof, will have at least 70, 75, 80, 85, 90, 95, 96, 97, 98, or 99% identity with a GAI sequence of Miyamoto et al. (2010); or will differ by no more than 30, 25, 20, 15, 10, 5, 4, 3, 2, or 1 amino acid residues from the corresponding a sequence of Miyamoto et al. (2010). See, e.g., Fig. 4.

In embodiments, a GID1 switch domain, or a sequence of GID1 sufficient to bind to a GAI switch domain, thereof, will have at least 70, 75, 80, 85, 90, 95, 96, 97, 98, or 99% identity with the GID1 sequence of Miyamoto et al. (2010); or will differ by no more than 30, 25, 20, 15, 10, 5, 4, 3, 2, or 1 amino acid residues from the corresponding of Miyamoto et al. (2010).

Candidate sequences for use as a GAI or GID1 switch domain, can be evaluated by incorporating the candidate into a system such as that described in a sequence of Miyamoto et al. (2010).

### Structure 3: GA₃-AM and GA₃

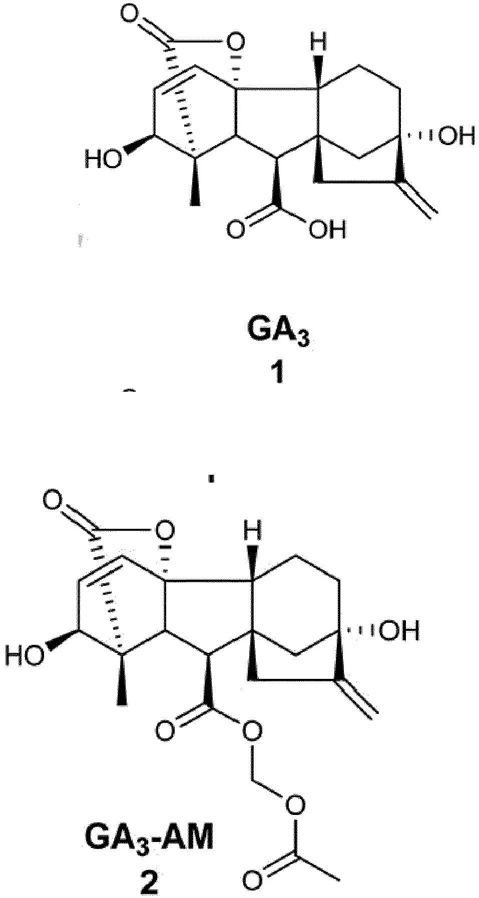

### TAG/BINDER SWITCHES

In instances a dimerization switch, e.g., a homodimerization switch, e.g., an extracellular homodimerization switch, comprises switch domains that comprise tag molecules, e.g., a c-myc peptide tag, flag peptide tag, HA peptide tag or V5 peptide tag. Suitable dimerization switches include polypeptides with affinity for the switch domains, e.g., antibody molecules and non-antibody scaffold. See, e.g., Fig. 6.

### COVALENT DIMERIZATION SWITCHES

In a covalent dimerization switch, the dimerization molecule promotes a covalent interaction between the switch domains. In an embodiment, a dimerization switch comprises first and second switch domains, which, upon contact with a dimerization molecule, are covalently linked to one another. In instances, a covalent dimerization switch is a homodimerization switch, wherein the dimerization molecule covalently couples a first and second switch domain having the same structure. In instances of a covalent homodimerization switch, the linking molecule comprises a first and second moiety, each of which can bind a switch domain, thereby covalently linking the switch domains. The first and second moiety can have the same structure or different structures. In embodiments, a covalent dimerization switch is a heterodimerization switch, wherein the dimerization molecule covalently couples first and second switch domains having structures that differ from one another. In embodiments of a covalent heterodimerization switch, the linking molecule can have a first moiety that covalently binds the first switch domain, but not the second switch domain, and a second moiety that covalently binds the second switch domain, but not the first switch domain. In embodiments the dimerization molecule comprises an additional moiety that alters its solubility or cell permeability. E.g., in the case of an intracellular covalent heterodimerization switch, the dimerization molecule can comprise a moiety that optimizes the cell permeability of the dimerization molecule.

A Halotag/SNAP-tag switch is an example of a covalent heterodimerization switch. In an embodiment, the dimerization molecule comprises a first moiety, e.g., an O6-benzylguanine moiety, that reacts covalently with a SNAP-tag domain, a second moiety, e.g., a chloroalkane moiety, that reacts with a Halotag domain, and a moiety that renders the dimerization molecule cell permeable.

Covalent dimerization switches are described in Erhart et al., 2013 Chem Biol 20(4): 549-557. HaXS species described therein are useful as dimerization molecules in a Halotag/SNAP-tag switch. In embodiments, a covalent dimerization molecule minimizes potential kinetic limitations related to off rates and need for accumulation of non-covalent dimerization molecules in the cell as prerequisites to activation of the required signal cascades, e.g., for T-cell mediated killing.

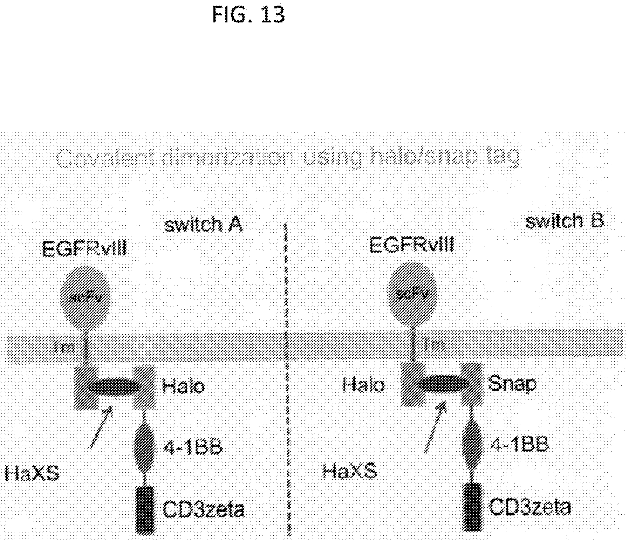

In an embodiment, a Halotag/SNAP-tag dimerization comprises a first switch domain comprising a Halo-Tag moiety, e.g., SEQ ID NO: 14, or a functional derivative or fragment thereof, and a second switch domain comprising a SNAP-Tag, e.g., SEQ ID NO: 15, or a functional derivative or fragment thereof. In embodiments the dimerization molecule comprises functional groups for linking a Halo-Tag with a SNAP-Tag along with a cell penetrating core. Structure 5 depicts a dimerization molecule suitable for use in this system. See, e.g., Fig. 13.
A Halo-tag Domain (SEQ ID NO: 14)
   Gseigtgfpfdphyvevlgermhyvdvgprdgtpvlflhgnptssyvwrniiphvapthrciapdligmgksdkpdlgyff ddhvrfmdafiealgleevvlvihdwgsalgfhwakrnpervkgiafmefirpiptwdewpefaretfqafrttdvgrkliid qnvfiegtlpmgvvrpltevemdhyrepflnpvdreplwrfpnelpiagepanivalveeymdwlhqspvpkllfwgtpg vlippaeaarlakslpnckavdigpglnllqednpdligseiarwlstleisg
A SNAP-tag domain (SEQ ID NO: 15)
   Mdkdcemkrttldsplgklelsgceqglhriiflgkgtsaadavevpapaavlggpeplmqatawlnayfhqpeaieefpvp alhhpvfqqesftrqvlwkllkvvkfgevisyshlaalagnpaataavktalsgnpvpilipchrvvqgdldvggyegglavk ewllaheghrlgkpglg

### Structure 5; HaXS

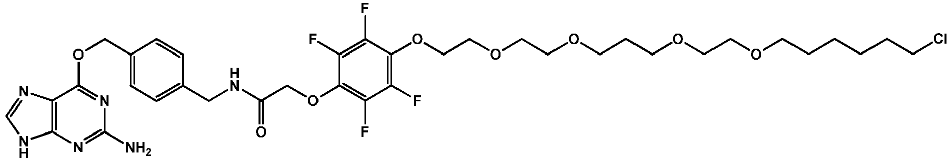

In an embodiment, one switch domain comprises amino acid residues disclosed in SEQ ID NO: 14 and one switch domain comprises amino acid residues disclosed in SEQ ID NO: 15.

In embodiments the first switch domain, will have at least 70, 75, 80, 85, 90, 95, 96, 97, 98, or 99% identity with the sequence of SEQ ID NO: 14. In embodiments, the first switch domain, will differ by no more than 30, 25, 20, 15, 10, 5, 4, 3, 2, or 1 amino acid residues from the corresponding the sequence of SEQ ID NO: 14.

In embodiments the second switch domain, will have at least 70, 75, 80, 85, 90, 95, 96, 97, 98, or 99% identity with the sequence of SEQ ID NO: 15. In embodiments, the second switch domain, will differ by no more than 30, 25, 20, 15, 10, 5, 4, 3, 2, or 1 amino acid residues from the corresponding the sequence of SEQ ID NO: 15.

Candidate sequences for use as a switch domain, can be evaluated by incorporating the candidate into a system such as those described herein.

### MULTI PLE SWITCH DOMAINS

In an embodiment, a dimerization switch described herein comprises multiple switch domains, and is sometimes referred to herein as a multi switch. A multi switch comprises a plurality of, e.g., 2, 3, 4, 5, 6, 7, 8, 9, or 10, switch domains, independently, on a first member, e.g., an antigen binding member, and a second member, e.g., an intracellular signaling member. Optionally, a linker, spacer, or hinge region, e.g., as described herein, is disposed between two switch domains on the member, e.g., the antigen binding member or the intracellular signaling member.

In an embodiment, the first member comprises a plurality of first switch domains, e.g., FKBP-based switch domains, and the second member can comprise a plurality of second switch domains, e.g., FRB-based switch domains. E.g., the antigen binding member comprises a plurality of first switch domains, e.g., FKBP-based switch domains, and the intracellular signaling member comprises a plurality of second switch domains, e.g., FRB-based switch domains. See, e.g., Fig. 47A.

In an embodiment, the first member comprises a first and a second switch domain, e.g., a FKBP-based switch domain and a FRB-based switch domain, and the second member comprises a first and a second switch domain, e.g., a FKBP-based switch domain and aFRB-based switch domain. E.g., the antigen binding member comprises a first and a second switch domain, e.g., a FKBP-based switch domain and a FRB-based switch domain, and the intracellular signaling member comprises a first and a second switch domain, e.g., a FKBP-based switch domain and a FRB-based switch domain. See, e.g., Fig. 47B.

In an embodiment, a dimerization switch, e.g., an FKBP/FRB based dimerization switch, comprises an asymmetrical distribution of switch domains on a first and second member wherein the number of switch domains on the first member is not equal to the number of switch domains on the second member. In an embodiment one member comprises at least X switch domains, wherein X is a plurality, e.g., 2, 3, 4, 5, 6, 7, 8, 9 or 10, and the other member has fewer switch domains, e.g., 1, 2, 3, 4, or 5 fewer switch domains than the first mentioned member. In an embodiment, a member comprises two switch domains for an FKBP-FRB based dimerization switch and the other member comprises less than two switch domains for an FKBP/FRB based dimerization switch. See, e.g., Fig. 47A.

In an embodiment, the dimerization switch, e.g., an FKBP-FRB based dimerization switch, comprises a symmetrical distribution of switch domains, wherein the number of switch domains on one member is equal to the number of switch domains on the other member. See, e.g., Fig. 47A and 47B.

In an embodiment, the first member and second member comprises a plurality of homodimerization switch domains, e.g., Gibberellin-based switch domains. E.g., the antigen binding member comprises a plurality of homodimerization switch domains, e.g., GyrB-based switch domains, and the intracellular signaling member comprises a plurality of homodimerization switch domains, e.g., GyrB-based switch domains.

### SECOND ORDER DIMERIZATION SWITCHES

In an embodiment an RCAR or RNKR-CAR comprises a first order dimerization switch which comprises a first and second switch domain. The dimerization molecule of the first order dimerization switch promotes association of the first and second switch domain. This dimerization switch can be referred to as a first order dimerization switch. In embodiments, a second order dimerization switch is also present. In the second order dimerization switch, the first order dimerization molecule serves as a second order switch domains. The second order dimerization molecule promotes the association of two or more second order switch domains (each of which comprises a first order dimerization molecule). The dimerization or clustering induces by the second order switch further increases the level of clustering of intracellular domains--in such embodiments the second order dimerization molecule results in more clustering than would be seen if only a first order switch was used. The first order dimerization molecule promotes association (or clustering) of the first order switch domains, e.g., homodimerization switch domains (and of intracellular signaling domains attached thereto). Such first order switch domains can comprise a tag molecule such as c-myc peptide tag, flag peptide tag, HA peptide tag or V5 peptide tag. In such instances the first order dimerization molecule can comprise an antibody, or other binder, directed to the switch domain. At the second order, the second order dimerization molecule promotes the association or clustering of the first order dimerization molecules. In other words, a second order switch comprises switch domains with comprise the first order dimerization molecule and a dimerization molecule e.g., an antibody against the first order dimerization molecule, that causes association of the second order switch domains. The first and second order does not imply any sequence to the addition of the first and second order dimerization molecules. In embodiments the first order dimerization molecule is administered first, or is contacted with its switch domains first, prior to the administration, or contacting the first order dimerization molecules with the second order dimerization molecule. In embodiments the second order dimerization molecule is administered first, or is contacted with its switch domains first, prior to the administration, or contacting the first order dimerization molecules with its switch domains.

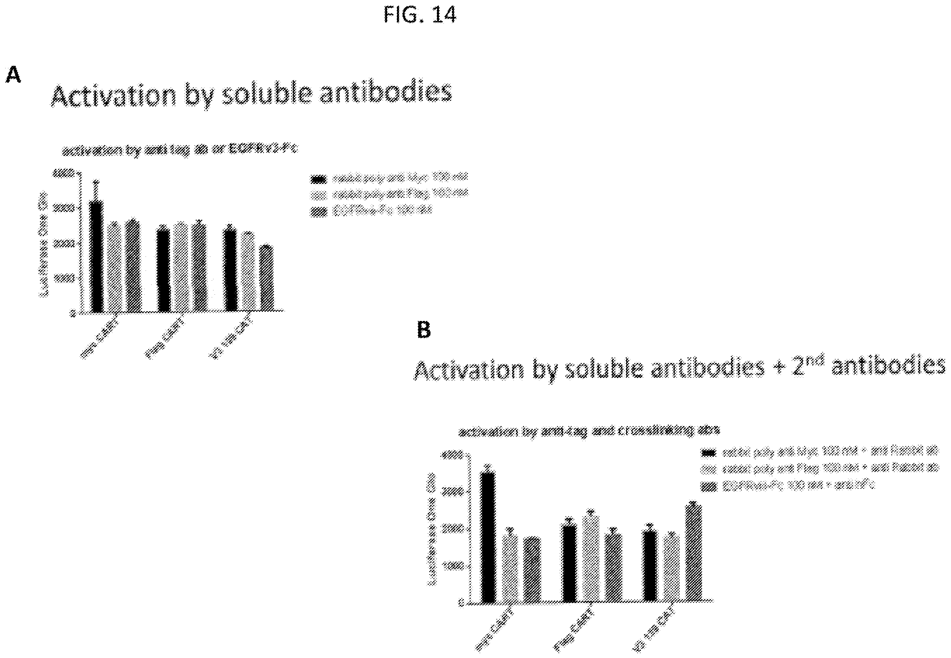

See, e.g., Fig. 14.

Third and higher order switch domains can also be used.

### DIMERIZATION MOLECULE

While not wishing to be bound by theory, it is believed that in some embodiments, referred to herein as a bi-domain binding dimerization molecule, the dimerization molecule comprises a first domain binding moiety that binds, or interacts, with a first switch domain, and a second domain binding moiety that binds, or interacts, with a second switch domain. While not wishing to be bound by theory, in some embodiments, referred to herein as a conformation-dependent dimerization molecule, the dimerization molecule binds or interacts with one of the switch domains, and alters the conformation of that switch domain such that it binds the other switch domain. Again, while not wishing to be bound by theory, it is believed that some dimerization molecules could operate by a combination or those, or other, mechanisms. Association between the switch domains is promoted by the dimerization molecule. In the presence of dimerization molecule interaction or association between switch domains allows for signal transduction between a polypeptide associated with, e.g., fused to, a first switch domain, and a polypeptide associated with, e.g., fused to, a second switch domain. In the presence of non-limiting levels of dimerization molecule signal transduction is increased by 1.1, 1.2, 1.3, 1.4, 1.5, 1.6, 1.7, 1.8, 1.9, 2, 5, 10, 50, 100 fold, e.g., as measured in a system described herein.

In embodiments, the dimerization molecule is a small molecule, e.g., AP21967.

In embodiments the dimerization molecule is a small molecule, e.g., is other than a polypeptide.

In embodiments, the dimerization molecule is a polypeptide, e.g., a polypeptide, e.g., an antibody molecule, or a non-antibody scaffold, e.g., a fibronectin or adnectin, having specific affinity for one or both of the first and second switch domains. In embodiments, the dimerization molecule is a multimeric polypeptide, e.g., a polypeptide comprising at least one, two, three, four, five, or more protein domains linked together by a linker, e.g., a GS linker. In embodiments, the dimerization molecule is an antibody or fragment thereof. In an embodiment, the heterodimerization molecule is an antibody, e.g., a monospecific antibody, or fragment thereof or a dual specific antibody, or fragment thereof.

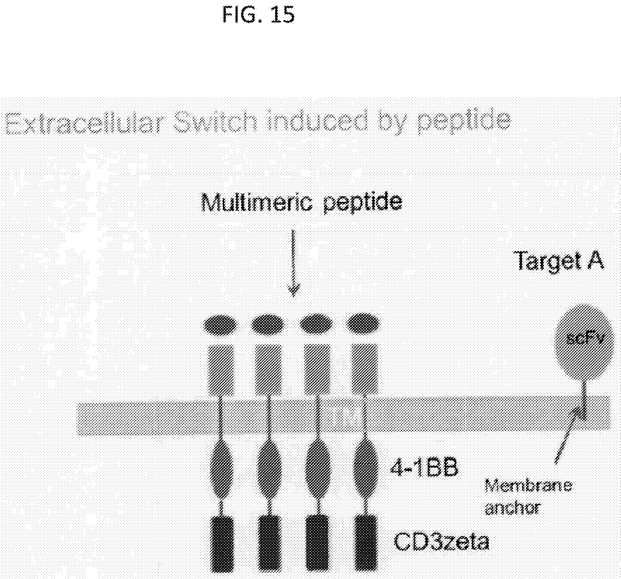

In an embodiment, the dimerization switch is a heterodimerization switch i.e., has first and second switch domains that are different from one another and the dimerization molecule is a heterodimerization molecule. In an embodiment, the heterodimerization molecule is a small molecule that binds to one or both of first and second switch domains. In an embodiment, the heterodimerization molecule is a polypeptide, or fragment thereof having specific affinity for one or both of the first and second switch domains. In an embodiment, the heterodimerization molecule is a mutimeric polypeptide, or fragment thereof having specific affinity for the first and second switch domains. In an embodiment, the heterodimerization molecule is a mutimeric polypeptide, or fragment thereof having specific affinity for multiple switch domains, see, e.g., Fig. 15. In an embodiment, the heterodimerization molecule is an antibody, or fragment thereof having specific affinity for one or both of the first and second switch domains.

In an embodiment, the dimerization switch is a homodimerization switch, i.e., has first and second switch domains that are the same as one another and the dimerization molecule is a homodimerization molecule. In an embodiment, the homodimerization n molecule is a small molecule that binds to one or both of first and second switch domains. In an embodiment, the homodimerization molecule is a polypeptide, or fragment thereof having specific affinity for one or both of the first and second switch domains. In an embodiment, the homodimerization molecule is a mutimeric polypeptide, or fragment thereof having specific affinity for the first and second switch domains. In an embodiment, the homodimerization molecule is a multimeric polypeptide, or fragment thereof having specific affinity for multiple switch domains, see5. 17. In an embodiment, the homodimerization molecule is an antibody, or fragment thereof having specific affinity for one or both of the first and second switch domains.
Dimerization molecules can be non-covalent or covalent, depending on the form of interaction between the switch domians.

In an embodiment, the dimerization molecule is poorly permealbe though the plasma membrane. In an embodiment, the dimerization molecule comprises a moiety, e.g., a charged moiety that inhibits entry into cells. E.g., a dimerization molecule, e.g., rapamycin or a rapamycin analog, can be modified so as to inhibit entry into cells. Such dimerization molecules can be used with RCARs or RNKR-CARs having extracellular switches. Their relatively poor entry into cells does not compromise the ability to invoke dimerization (because the switch is extracellular) but can reduce toxicity. GA₃, which is does not readily permeate cells, can be used with external GID1-GAI based switch. In an embodiment, a dimerization molecule that has been modified accumulates in a cell only 50, 40, 20, or 10% as much as the unmodified dimerization molecule.

### MULTI-VALENT DIMERIZATION MOLECULES

Generally, a dimerization molecule promotes the association of at least two switch molecules. In embodiments this association of switch domains promotes the association of intracellular domains linked to the switch domains. In embodiments the dimerization molecule has a valency of greater than two, e.g., it is multi-valent, and binds, and thus clusters or dimerizes, more than two switch domains. E.g., a dimerization molecule can comprise a plurality, e.g., at least 2, 3, 4, 5, 6, 7, 8, 9 or 10, binding domains, each of which can bind a switch domain. In embodiments, the switch domain is an antibody molecule, non-antibody scaffold, ligand, or other polypeptide having affinity for a dimerization molecule. Exemplary multi-valent dimerization molecules comprise molecules that comprise more than two domains, e.g., more than two domains each comprising a c-myc peptide tag, flag peptide tag, HA peptide tag or V5 peptide tag domain. A multi-valent dimerization molecule can be a first order or second order dimerization molecule. See, e.g., Fig. 14.

### DOMAIN ARRANGEMENTS

A RCAR described herein, e.g., for use in a RCAR/NKR-CARX cell, comprises domains arranged in a variety of configurations.

In an instance, both a primary signaling domain and a costimulatory signaling domain are separated from the antigen binding domain by a switch.

Accordingly, in one instance the RCAR arrangement comprises a first and second chimeric construct wherein:
(1) the first chimeric construct, e.g., an antigen binding member, comprises: an antigen binding domain; a transmembrane domain; and a first intracellular switch domain, e.g., FRB (in this instance the first chimeric construct does not comprise an intracellular signaling domain); and
(2) a second chimeric construct, e.., an intracellular signaling domain, (which in this instance does not comprise a transmembrane domain or membrane anchor) comprising: a second intracellular switch domain, e.g., FKBP; and a signaling domain, e.g., a primary or secondary signaling domian.

In an instance, both a primary signaling domain, e.g., a CD3zeta domain, and a costimulatory signaling domain, e.g., a 4-1BB domain, are present on the second chimeric construct. In an instance, the order on the second chimeric construct is: a second switch domain, pa rimary signaling domain, e.g., a CD3zeta domain, and a costimulatory signaling domain, e.g., a 4-1BB domain. In an instance, the order on the second chimeric construct is a second switch domain, a costimulatory signaling domain, e.g., a 4-1BB domain, and a primary signaling domain, e.g., a CD3zeta domain. In an instance, the order on the second chimeric construct is a costimulatory signaling domain, e.g., a 4-1BB domain, a second switch domain, and a primary signaling domain, e.g., a CD3zeta domain. In an instance, the order on the second chimeric construct is a primary signaling domain, e.g., a CD3zeta domain, a second switch domain, and a costimulatory signaling domain, e.g., a 4-1BB domain.

The instances refer to FRB on the first chimeric constructs and FKBP on the second chimeric constructs but the placement can be reversed.

The order of the domains in the instances are given in the N-terminus to C-terminus direction, but especially with regard to intracellular chimeric constructs, the order can be from C-terminus to N-terminus.

In an instance one, but not both, of the primary signaling domain and the costimulatory signaling domain, is separated by a switch from the antigen binding domain.

Accordingly, in another instance, the RCAR arrangement comprises:
(1) a first chimeric construct, e.g., an antigen binding member, comprising: an antigen binding domain; a transmembrane domain; a first intracellular switch domain, e.g., FRB; and an intracellular signaling domain, e.g., a primary signaling domain, e.g., a CD3zeta domain, or a costimulatory signaling domain, e.g., a 4-1BB domain; and
(2) a second chimeric construct, e.g., an intracellular signaling member, (which in this instance does not comprise a transmembrane domain or membrane anchor) comprising: a second intracellular switch domain, e.g., FKBP; and an intracellular signaling domain, e.g., a primary signaling domain, e.g., a CD3zeta domain, or a costimulatory signaling domain, e.g., a 4-1BB domain.

In an instance, the order on the first chimeric construct is an antigen binding domain, a transmembrane domain, a first intracellular switch domain, e.g., FRB, and an intracellular signaling domain, e.g., a primary signaling domain, e.g., a CD3zeta domain, or a costimulatory signaling domain, e.g., a 4-1BB domain.

In an instance, the order on the first chimeric construct is an antigen binding domain, a transmembrane domain, an intracellular signaling domain, e.g., a primary signaling domain, e.g., a CD3zeta domain, or a costimulatory signaling domain, e.g., a 4-1BB domain, and a first intracellular switch domain, e.g., FRB.

In an instance, the first chimeric construct comprises one, but not both of, a primary signaling domain, e.g., a CD3zeta domain, and a costimulatory signaling domain, e.g., a 4-1BB domain.

In an instance, the order on the second chimeric construct is: a second intracellular switch domain, e.g., FKBP, and one, but not both of, a primary signaling domain, e.g., a CD3zeta domain, and a costimulatory signaling domain, e.g., a 4-1BB domain.

In an instance, the order on the second chimeric construct is: one, but not both of, a primary signaling domain, e.g., a CD3zeta domain, and a costimulatory signaling domain, e.g., a 4-1BB domain and a second intracellular switch domain, e.g., FKBP.

In an instance:
(1) the first chimeric construct, e.g., an antigen binding member, comprises: an antigen binding domain, e.g., an scFv; a transmembrane domain; a costimulatory signaling domain, e.g., a 4-1BB domain; and a first switch domain; and
(2) the second chimeric construct, e.g., an intracellular signaling member, comprises a second switch domain; and a primary signaling domain, e.g., a CD3zeta domain (and in instances, no transmembrane domain or membrane anchor).

In an instance:
(1) the first chimeric construct, e.g., an antigen binding member, comprises: an antigen binding domain, e.g., an scFv; a transmembrane domain; a primary signaling domain, e.g., a CD3zeta domain; and a first switch domain; and
(2) the second chimeic construct, e.g., an intracellular signaling member, comprises: a second switch domain; and a costimulatory signaling domain, e.g., a 4-1BB domain (and in instances, no transmembrane domain or membrane anchor).

In one instance the RCAR arrangement comprises a first and
second chimeric construct wherein:
(1) the first chimeric construct, e.g., an antigen binding member, comprises: an antigen binding domain; a transmembrane domain; a first intracellular signaling domain, and a first intracellular switch domain, e.g., FRB; and
(2) a second chimeric construct, e.g., an intracellular signaling member, (which in this instance does not comprise a transmembrane domain or membrane anchor) comprising: a second intracellular switch domain, e.g., FKBP; and a intracellular signaling domain, e.g., a primary or costimulatory signaling domian.

The instances refer to FRB on the first chimeric constructs and FKBP on the second chimeric constructs but the placement can be reversed.

The orders the instances are given in the N-terminus to C-terminus direction, but especially with regard to intracellular chimeric constructs, the order can be from C-terminus to N-terminus.

### RCAR MEMBERS, E.G., ANTIGEN BINDING DOMAINS OR OTHER EXTRACELLULAR BINDING DOMAINS, HAVING A COSTIMULATORY SIGNALING DOMAIN

Persistence and expansion of T-lymphocytes expressing the chimeric antigen receptor on the surface is mediated by inclusion of various intracellular domains fused to the membrane bound receptor. E.g., an element of a RCAR (e.g., an RCAR of a RCAR/NKR-CARX cell) having an extracellular domain that engages a target ligand on a target cell, e.g., a cancer cell, can comprise a co-stimulatory intracellular signaling domain, e.g., a costimulatory signaling domain selected from Table 2.

In instances, placement of a co-stimulatory intracellular signaling domain, e.g., 4-1BB, onto the first switch domain from the CD3 zeta on the second switch domain will positively modulate RCAR activity *in vivo* while limiting the activity of the CAR in the absence of the dimerization switch molecule.

RCAR members having an an extracellular domain that engages a target ligand on a cell, e.g., an antigen binding domain, can comprise a plurality, e.g., 2, or 3, co-stimulatory intracellular signaling domains, e.g., selected from Table 2. In an instance, the RCAR member comprises a plurality of costimulatory signaling domains selected from 41BB, CD28, CD27, ICOS, and OX40. By way of example, the member, e.g., an antigen binding member, comprises, from the extracellular to intracellular direction:
41BB-CD27;
CD27-41BB;
41BB-CD28;
CD28-41BB;
OX40-CD28;
CD28-OX40;
CD28-41BB; or
41BB-CD28.

An antigen binding member can comprises: a plurality, e.g., 2 or 3 costimulatory signaling domains, chosen e.g., from Table2, e.g., selectred from 41BB, CD28, CD27, ICOS, and OX40. The costimulatory signaling domains can be disposed in any order, but exemplary configurations include the following (in the direction of extracellular to intracellular):
41BB-CD27;
CD27-41BB;
41BB-CD28;
CD28-41BB;
OX40-CD28;
CD28-OX40;
CD28-41BB; or
41BB-CD28.

In an instance, the antigen binding member comprises the following costimulatory signaling domains: CD28-41BB.

In an instance, the antigen binding member comprises the following costimulatory signaling domains: CD28-OX40.

In an instance an antigen binding member comprises a) [an antigen binding domain]- [a transmembrane domain]-[a first costimulatory signaling domain]-[a second costimulatory signaling domain] and
wherein the first and second costimulatory signaling domains:
(i) are each independient selected from Table 2;
(ii) are each independently selected from 41BB, CD28, CD27, ICOS, and OX40;
(iii) comprise one of the following pairs of costimulatory signaling domains (from the extracellular to intracellular direction):
   41BB-CD27;
   CD27-41BB;
   41BB-CD28;
   CD28-41BB;
   OX40-CD28;
   CD28-OX40;
   CD28-41BB; or
   41BB-CD28.
(iv) comprise the following pairs of costimulatory signaling domains: CD28-41BB; or
(v) comprise the following pairs of costimulatory signaling domains: CD28-OX40; and
   (b) a [switch domain],
   wherein the switch domain is disposed:
   (i) between the transmembrane domain and the first costimulatory signaling domain;
   (ii) between the first costimulatory signaling domain and the second costimulatory signaling domain; or
   (iii) after the second costimulatory signaling domain,
   and optionally, the switch domain comprises an FKBP binding fragment or analog of FRB, and the FKBP binding fragment or analog of FRB comprises one or more mutations which enhances the formation of a complex between an FKBP switch domain, an FRB switch domain, and the dimerization molecule, or a mutation described in the section herein entitled **MODIFIED FKBP/FRB-BASED DIMERIZATION SWITCHES.** E.g., the FKBP binding fragment or analog of FRB comprises: an E2032 mutation, e.g., an E2032I mutation or E2032L mutation; a T2098 mutation, e.g., a T2098L mutation; or an E2032 and a T2098 mutation, e.g., an E2032I and a T2098L or an E2032L and a T2098L mutation;
   and optionally, the antigen binding member does not comprise a primary intracellular signaling domain.

In an instance, the antigen binding member comprises: a plurality, e.g., 2 or 3 costimulatory signaling domains, chosen e.g., from Table 2, e.g., a combination of costimulatory signaling domains describd herein, and the intracellular signaling member comprises a CD3zeta domain.

Provided below are amino acid sequences of exemplary RCAR members comprising an antigen binding member comprising the following structure: [an antigen binding domain]-[a transmembrane domain]-[a first costimulatory signaling domain]-[a second costimulatory signaling domain]-[switch domain]. For the exemplary RCARs listed below, the antigen binding domain comprises an CD19 scFv (the sequence is underlined), a first costimulatory signaling domain (the sequence is italicized), a second costimulatory signaling domain (the sequence is italicized and in bold), and a switch domain (the sequence is underlined and in bold).

**Table 16. Exemplary antigen binding members**

| **Antigen binding member** | **Amino Acid Sequence** | **SEQ ID NO:** |
|---|---|---|
| CD19 scFv-*OX40-****CD28-*FKBP** | | 187 |
| CD19 scFv-*OX40-****CD28-*FRB E2032I/T2098L** | | 188 |
| CD19 scFv-*CD27-****CD28-*FKBP** | | 189 |
| CD19 scFv-*CD27-****CD28-*FRB E2032I/T2098L** | | 190 |
| | | |
| CD19 scFv-*41 BB-****CD28-*FKBP** | | 191 |
| CD19 scFv-*41 BB-****CD28-*FRB E2032I/T2098L** | | 192 |

Provided below are amino acid sequences for the intracellular signaling member comprising a switch domain (the sequence is bolded and underlined) and a primary signaling domain (the sequence is italicized).

**Table 17. Exemplary intracellular signaling domain**

| **Intracellular signaling member** | **Amino Acid Sequence** | **SEQ ID NO:** |
|---|---|---|
| **FRB E2032I/T2098L** *-CD3zeta* | | 193 |
| **FKBP***-CD3zeta* | | 194 |

The disclosure also provides RCARs having a configuration that allows switching of proliferation. For example, upon antigen encounter, the RCAR exhibits constitutive primary signal, e.g., target cell killing, and allows regulation of a second signal, e.g., proliferation, survival, and cytokine secretion.

Accordingly, in another aspect, the disclosure features, a regulatable chimeric antigen receptor (RCAR), e.g., an isolated RCAR, wherein the RCAR comprises:
a) an intracellular signaling member comprising:
   optionally, a transmembrane domain or membrane tethering domain;
   a co-stimulatory signaling domain, selected e.g., from Table 2, and
   a switch domain; and
b) an antigen binding member comprising:
   an antigen binding domain,
   a transmembrane domain, and
   a primary intracellular signaling domain, e.g., selected from Table 1, e.g., a CD3zeta domain,
   wherein the antigen binding member does not comprise a switch domain, or does not comprise a switch domain that dimerizes with a switch domain on the intracellular signaling member.

In an instance, the antigen binding member does not comprise a costimulatory signaling domain.

In an instance, the intracellular signaling member comprises a second costimulatory signaling domain, selected, e.g., from Table 2. In an instance, the two or more costimulatory domains can be the same costimulatory signaling domain, e.g., selected from the list in Table 2, or different costimulatory signaling domains, e.g., selected from the list in Table 2. In an instance the intracellular signaling member comprises: a plurality, e.g., 2 or 3, co-stimulatory signaling domains selected from 41BB, CD28, CD27, ICOS, and OX40.

In an instance, the intracellular signaling member comprises the following co-stimulatory signaling domains, from the extracellular to intracellular direction:
41BB-CD27;
CD27-41BB;
41BB-CD28;
CD28-41BB;
OX40-CD28;
CD28-OX40;
CD28-41BB; or
41BB-CD28.

In an instance, the intracellular signaling member comprises the following co-stimulatory signaling domains: CD28-41BB.

In an instance, intracellular signaling member comprises the following co-stimulatory signaling domains: CD28-OX40.

In an instance, in addition to one or a plurality of co-stimulatory signaling domains, the intracellular signaling member comprises a primary intracellular signaling domain, e.g., selected from Table 1, e.g., a CD3zeta domain.

In an instance, the intracellular signaling domain comprises a CD28 co-stimulatory signaling domain, a 4-1BB co-stimulatory signaling domain, and a CD3zeta domain.

In an instance, the intracellular signaling domain comprises a CD28 co-stimulatory signaling domain, a OX40 co-stimulatory signaling domain, and a CD3zeta domain.

In an instance, the intracellular signaling member does not comprise a transmembrane domain or membrane tethering domain. In such instances, the switch domain is intracellular. In such instances, the intracellular signaling member comprises two costimulatory signaling domains, where the two costimulatory domains are selected from 4-1BB, OX40, CD27, CD28, and ICOS. In an instance, the order of elements on the intracellular signaling member is as follows, from the extracellular to intracellular direction:
a first co-stimulatory signaling domain/ a second costimulatory signaling domain and a switch domain disposed between any of the signaling elements, or, from the extracellular to intracellular direction, after all other signaling elements. See, e.g., Fig. 48D.

In an instance, the intracellular signaling member comprises a transmembrane domain. In such instances the switch domain can be intracellular or extracellular. In such instances, the intracellular signaling member comprises two costimulatory signaling domains, where the two costimulatory domains are selected from 4-1BB, OX40, CD27, CD28, and ICOS.

In an instance where the switch domain is extracellular, the order of elements on the intracellular signaling member is as follows, from the extracellular to intracellular direction:
a switch domain/ a transmembrane domain/ a first co-stimulatory signaling domain/ a second costimulatory signaling domain. See, e.g., Fig. 48C.

In an instance where the switch domain is intracellular, the order of elements on the intracellular signaling member is as follows, ,rom the extracellular to intracellular direction:
transmembrane domain/ a first co-stimulatory signaling domain/ a second co stimulatory signaling domain and a switch domain disposed intracellularly between any of the signaling elements, or, from extracellular to intracellular, after all other signaling elements. See, e.g., Fig. 48A.

In an instance, the intracellular signaling member comprises a membrane tethering domain. In one such instance, the switch domain is intracellular. In such instances, the intracellular signaling member comprises two costimulatory signaling domains, where the two costimulatory domains are selected from 4-1BB, OX40, CD27, CD28, and ICOS. In an instance, the order of elements on the intracellular signaling member is as follows, from the extracellular to intracellular direction:
a membrane tethering domain/a first co-stimulatory signaling domain/ a second costimulatory signaling domain and a switch domain disposed extracellularly, between any of the signaling elements, or, from extracellular to intracellular, after all other signaling elements. See, e.g., Fig. 48B.

In an instance, the switch domain is: extracellular; disposed between the transmembrane domain or membrane tethering domain and a co-stimulatory signaling domain, e.g., the costimulatory signaling domain closest to the membrane; between a first and second costimulatory signaling domain; between a costimulatory signaling domain and a primary intracellular signaling domain; or, from extracellular to intracellular, after all intracellular signaling domains.

In an instance, the order of elements on the intracellular signaling member, from extracellular to intracellular, is as follows:
transmembrane domain or membrane tethering domain/a first co-stimulatory signaling domain/optionally a second costimulatory signaling domain/and optionally a primary intracellular signaling domain, and a switch domain disposed extracellularly, between any of the elements, or, from extracellular to intracellular, after all other elements.

In an instance, the order of elements on the antigen binding member, from extracellular to intracellular, is as follows:
antigen binding domain/transmembrane domain/primary intracellular signaling domain, e.g., selected from Table 1, e.g., a CD3zeta domain.

### UNIVERSAL RCARS AND RNKR-CARS

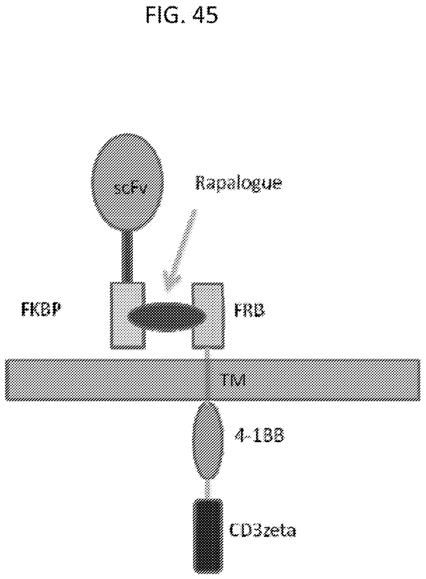

An instance provides RCARs or RNKR-CARs wherein the antigen binding member is not tethered to the surface of the RCARX (e.g., RCART) or RNKR-CARX (e.g., RNKR-CART) cell. Typically, such an RCARX (e.g., RCART) or RNKR-CARX (e.g., RNKR-CART) cell will include an intracellular signaling domain having an external or extracellular first switch domain. The cell can be contacted with an antigen binding member that comprises an antigen binding domain and a second switch domain (and no transmembrane domain or membrane tethering domain). This allows an RCARX (e.g., RCART) or RNKR-CARX (e.g., RNKR-CART) cell having an intracellular signaling member to be conveniently be paired with one or more antigen binding domains, without transforming the cell with sequence that encodes the antigen binding member. An aliquot of RCARX (e.g., RCART) or RNKR-CARX (e.g., RNKR-CART) cells comprising the intracellular signaling member but not an antigen binding member can be provided. As needed, a RCARX (e.g., RCART) or RNKR-CARX (e.g., RNKR-CART) cell, e.g., having selected antigen binding properties, can be provided by adding an antigen binding member. Such a RCAR or RNKR-CAR is sometimes referred to herein as universal RCAR or universal RNKR-CAR, respectively. See, e.g., Fig. 45. E.g., an RCARX (e.g., RCART) or RNKR-CARX (e.g., RNKR-CART) cell having the intracellular binding domain can be contacted, e.g., *ex vivo,* with the antigen binding member of a universal RCAR or universal RNKR-CAR, and optionally a dimerization molecule. The antigen binding member can be selected from a panel of antigen binding members that comprise different antigen binding domains, e.g., that bind to different antigens. In an instance, based on the genotype or phenotype of a subject or subject tumor, e.g., tumor aggressiveness, tumor type, disease stage, prior treatment and the like, an antigen binding domain is selected. In an instance, immune effector cells, e.g., T cells, can be obtained from a subject and universal RCART or RNKR-CART cells made therefrom. A first aliquot of the RCARX or RNKR-CARX cells can be combined with a first antigen binding domain. A second aliquot can be combined with a second antigen binding domain. E.g., a universal RCARX or universal RNKR-CARX with the first antigen binding domain can be used in a first course of treatment and a universal RCARX or universal RNKR-CARX with the second antigen binding domain can be used as a second course of treatment, e.g., administered after the initiation of the first course of treatment. In an instance, more than one antigen binding domain, e.g., 2, 3, or 4, antigen binding domains, are contacted with an RCARX (e.g., RCART) or RNKR-CARX (e.g., RNKR-CART) cell to provide a cell having RCARs or RNKR-CARs with more than one antigen specificity.

In an embodiment, the RCARX or RNKR-CARX is a natural killer cell. These cells can be isolated from the subject. In an embodiment, the cells are stable cell lines of natural killer cells, e.g., a stable allogeneic NK-92 cell line available, from Conkwest. These stable NK-92 cell lines were derived from NK-92 cells that were obtained, transfected and cultured using the methods described by Gong et al (April 1994), Leukemia Macmillan Press, Ltd, 8: 652-658, and disclosed in EP1007630. An NK-92 cell, or a cell from a NK cell line with properties similar to the NK-92 cell line can also be used.

### RNKR-CARS AND NKR-CARs

Disclosed herein are compositions and methods for regulating the specificity and activity of cytotoxic cells, e.g., T cells or NK cells, e.g., with a non-naturally occurring chimeric antigen receptor (CAR), e.g., a RNKR-CAR or a NKR-CAR. In an instance the CAR is an NKR-CAR. Alternatively, the CAR is a RNKR-CAR. A NKR-CAR or RNKR-CAR is a CAR which shares functional and structural properties with a NK cell immune-function receptor (or NKR). For example, a RNKR-CAR or a NKR-CAR comprises an element, e.g., domain, from a NKR. NKRs, RNKR-CARs, and NKR-CARs are described herein, e.g., in the section below. As is discussed below, a variety of NKRs (e.g., a variety of NKR elements/domains) can serve as the basis for a RNKR-CAR or a NKR-CAR.

In one aspect, the present invention provides compositions and methods for regulating the specificity and activity of T cells, or other cytotoxic cells, e.g., NK cells. In an instance, a chimeric antigen receptor (a CAR), e.g., a NK cell receptor CAR (a NKR-CAR) based on an NK cell receptor (a NKR), e.g., a KIR-CAR, a NCR-CAR, a SLAMF-CAR, a FcR-CAR, or a Ly49-CAR is provided. In an instance, a CAR, e.g., a RNKR-CAR, e.g., a RKIR-CAR, a RNCR-CAR, a RSLAMF-CAR, a RFcR-CAR, or a Rly49-CAR is provided. In one instance, the disclosure provides a type of chimeric antigen receptor (CAR) wherein the CAR is termed an NKR, e.g., a "NKR-CAR" or "RNKR-CAR," which is a CAR design comprising a component of a receptor found on natural killer (NK) cells. In one instance, the NK receptor includes but is not limited to a killer cell immunoglobulin-like receptor (KIR). KIRs can function as an activating KIR or an inhibiting KIR.

One advantage of the NKR-CARs (e.g., KIR-CARs) or RNKR-CARs (e.g., RKIR-CARs) is that a NKR-CAR (e.g., a KIR-CAR) or RNKR-CAR (e.g., RKIR-CAR), provides a method for regulating cytotoxic cell, e.g., T cell, specificity to control off-target activity of the engineered T cell. In some instances, the NKR-CARs (e.g, KIR-CARs) or RNKR-CARs (e.g., RKIR-CARs) of the disclosure do not require a costimulation to proliferate.

NKR-CARs or RNKR-CARs can deliver a signal through an adaptor protein, e.g., an ITAM containing adaptor protein. In one instance, the NKR-CARs (e.g., KIR-CARs) or RNKR-CARs (e.g., RKIR-CARs) described herein comprise an activating KIR or regulatable activating KIR which delivers its signal through an interaction with the immunotyrosine-based activation motif (ITAM) containing membrane protein, DAP12 that is mediated by residues within the transmembrane domains of these proteins.

In an embodiment, a NKR-CAR or RNKR-CAR can deliver an inhibitory signal by means of an inhibitory motif. In one embodiment, the KIR-CARs or RKIR-CARs described herein comprise an inhibitory KIR which delivers its signal through an interaction with the immunotyrosine-based inhibitory motifs (ITIMs). KIRs bearing cytoplasmic domains that contain ITIMs abrogate the activating signal leading to inhibition of NK cytolytic and cytokine producing activity. However, the invention should not be limited to inhibitory KIRs. Rather, any inhibitory protein having a cytoplasmic domain that is associated with an inhibitory signal can be used in the construction of the CARs of the invention.

Accordingly, the disclosure provides a composition comprising a NKR-CAR (e.g., a KIR-CAR) or RNKR-CAR (e.g., RKIR-CAR), vectors comprising the same, compositions comprising a NKR-CAR (e.g., a KIR-CAR) or RNKR-CAR (e.g., RKIR-CAR), vectors packaged in viral particles, and recombinant T cells or other cytotoxic cells comprising a NKR-CAR (e.g., a KIR-CAR) or RNKR-CAR (e.g., RKIR-CAR). The disclosure also includes methods of making a genetically modified T cell or other cytotoxic cell, e.g., a NK cell, or cultured NK cell, e.g., a NK92 cell, expressing a RNKR-CAR (e.g., a RKIR-CAR (RKIR-CART)) or NKR-CAR (e.g., KIR-CAR (KIR-CART)), wherein the expressed RNKR-CAR, (e.g., RKIR-CAR) or expressed NKR-CAR (e.g., KIR-CAR), comprises an antigen recognition domain of a specific antibody with an intracellular signaling molecule from a NKR, e.g., a KIR. For example, in some instances, the intracellular signaling molecule includes, but is not limited to, a KIR ITAM, a KIR ITIM, and the like.

Accordingly, the disclosure provides compositions and methods to regulate the specificity and activity of T cells or other cytotoxic cells modified to express a NKR-CAR (e.g., a KIR-CAR) or RNKR-CAR (e.g., RKIR-CAR). The present invention also provides cells comprising a plurality of types of NKR-CARs, e.g., KIR-CARs (e.g. activating NKR-CARs, e.g., actKIR-CARs and inhibiting NKR-CAR, e.g., a inhKIR-CAR), wherein the plurality of types of NKR-CARs, e.g., KIR-CARs, participate in signaling to regulate T cell activation. In instances, also provided are cell comprising a plurality of types of RNKR-CARs (e.g., activating RNKR-CAR and inhibiting RNKR-CARs), types of NKR-CARs (e.g., activating or inhibiting NKR-CARs) and/or types of RCARs, where the plurality of the types of NKR-CARs, RNKR-CARS, and/or RCARs participate in signaling to regulate T cell activation. In this aspect, it is beneficial to effectively control and regulate RCAR/NKR-CARX cytotoxic cells, e.g., RCAR/KIR-CART cells, or to effectively control and regulate RNKR-CARX cells, such that they kill tumor cells while not affecting normal bystander cells. Thus, in one instance, the present disclosure also provides methods of killing cancerous cells while minimizing the depletion of normal non-cancerous cells, thereby improving the specificity of a CAR, e.g., NKR-CAR(e.g., a KIR-CAR), RCAR, and/or RNKR-CAR therapy.

In one instance, the compositions and methods described herein include the physical separation of a plurality of types of CARs expressed on a cell. In instances, binding of a plurality of types of NKR-CARs (e.g., KIR-CARs), RCARs, and/or RNKR-CARs to their target antigen is required for RCAR/NKR-CARX cytotoxic cell or RNKR-CARX cytotoxic cell activation. For example, in such an approach, each CAR from the plurality of types of CARs have different intracellular signaling domains. For example, when a plurality of types of CARs is used to induce RCAR/NKR-CARX or RNKR-CARX cell activation, the first type of CARs can only comprise an intracellular domain from an activating NKR (e.g., KIR) and the second type of CAR can only comprise an intracellular domain from an inhibiting NKR (e.g, KIR). In this manner, conditional activation of T cells is generated by engagement of the activating CAR (e.g., RCAR, actKIR-CAR, or RactKIR-CAR) to an antigen on a malignant cell of interest. An inhibitory KIR-CAR (inhKIR-CAR) or inhibitory RKIR-CAR (RinhKIR-CAR) bearing an antigen binding moiety directed against an antigen that is present on a normal, but not malignant cell provides dampening of the activating effects from the activating CAR when the T cell encounters normal cells.

In one instance, the present disclosure provides a T cell or other cytotoxic cell engineered to express at least two CARs, e.g., wherein the first CAR, e.g., a RCAR, is an activating RCAR, and the second CAR, e.g., a NKR-CAR, is an inhNKR-CAR, e.g., an inhKIR-CAR. In one instance, the disclosure provides an inhNKR-CAR, e.g., an inhKIR-CAR, wherein binding of the inhNKR-CAR, e.g., an inhKIR-CAR, to a normal cell results in inhibition of the cytotoxic cell, e.g., inhibition of RCAR T cell activity. In one instance, binding of the inhNKR-CAR, e.g., an inhKIR-CAR, to an antigen associated with a non-cancerous cell results in the death of the CAR-expressing cytotoxic cell, e.g., a RCAR T cell or KIR-CAR T cell.

In one instance, an inhNKR-CAR of the disclosure can be used in combination with existing CARs in order to regulate the activity of the CARs. Exemplary CARs have been described in PCT/US11/64191.

It has also been discovered that, in cells having a plurality of chimeric membrane embedded receptors comprising an antigen binding domain (CMERs), interactions between the antigen binding domain of the CMERs can be undesirable, e.g., because interaction inhibits the ability of one or more of the antigen binding domains to bind its cognate antigen or might generate novel binding sites with unknown cognate antigen. Accordingly, disclosed herein are cells having a first and a second non-naturally occurring CMER wherein the antigen binding domains minimizes such interactions. Also disclosed herein are nucleic acids encoding a first and a second non-naturally occurring such CMERs, as well as methods of making and using such cells and nucleic acids. In an instance, the antigen binding domain of one of said first said second non-naturally occurring CMER, comprises an scFv, and the other comprises a single VH domain, e.g., a camelid, shark, or lamprey single VH domain, or a single VH domain derived from a human or mouse sequence or a non-antibody scaffold.

### NK CELL IMMUNE-FUNCTION RECEPTORS (NKRS) AND NK CELLS

As discussed herein, NK cell immune-function receptor (or NKR) refers to an endogenous naturally occurring transmembrane protein expressed in NK cells, which can engage with a ligand on an antigen presenting cell and modulate an NK cell immune-function response, e.g., it can modulate the cytolytic activity or cytokine secretion of the NK cell.

NK cells are mononuclear cells that develop in the bone marrow from lymphoid progenitors, and morphological features and biological properties typically include the expression of the cluster determinants (CDs) CD16, CD56, and/or CD57; the absence of the alpha/beta or gamma/delta TCR complex on the cell surface; the ability to bind to and kill target cells that fail to express "self' major histocompatibility complex (MHC)/human leukocyte antigen (HLA) proteins; and the ability to kill tumor cells or other diseased cells that express ligands for activating NK receptors. NK cells are characterized by their ability to bind and kill several types of tumor cell lines without the need for prior immunization or activation. NK cells can also release soluble proteins and cytokines that exert a regulatory effect on the immune system; and can undergo multiple rounds of cell division and produce daughter cells with similar biologic properties as the parent cell. Upon activation by interferons and/or cytokines, NK cells mediate the lysis of tumor cells and of cells infected with intracellular pathogens by mechanisms that require direct, physical contacts between the NK cell and the target cell. Lysis of target cells involves the release of cytotoxic granules from the NK cell onto the surface of the bound target, and effector proteins such as perforin and granzyme B that penetrate the target plasma membrane and induce apoptosis or programmed cell death. Normal, healthy cells are protected from lysis by NK cells. NK cell activity is regulated by a complex mechanism that involves both stimulating and inhibitory signals.

Briefly, the lytic activity of NK cells is regulated by various cell surface receptors that transduce either positive or negative intracellular signals upon interaction with ligands on the target cell. The balance between positive and negative signals transmitted via these receptors determines whether or not a target cell is lysed (killed) by a NK cell. NK cell stimulatory signals can be mediated by Natural Cytotoxicity Receptors (NCR) such as NKp30, NKp44, and NKp46; as well as NKG2C receptors, NKG2D receptors, certain activating killer cell immunoglobulin-like receptors (KIRs), and other activating NK receptors (Lanier, Annual Review of Immunology 2005; 23:225-74). NK cell inhibitory signals can be mediated by receptors like Ly49, CD94/NKG2A, as well as certain inhibitory KIRs, which recognize major histocompatibility complex (MHC) class I molecules (Karre et al., Nature 1986; 319:675-8; Ohlen et al, Science 1989; 246:666-8). These inhibitory receptors bind to polymorphic determinants of MHC class I molecules (including HLA class I) present on other cells and inhibit NK cell-mediated lysis.

### KIR-CARs

Disclosed herein is a chimeric antigen receptor (CAR) molecule, e.g., a RNKR-CAR or a NKR-CAR, comprising an antigen binding moiety and an element (e.g., domain) of a killer cell immunoglobulin-like receptor (KIR-CAR). In one instance, the RKIR-CAR or KIR-CAR of the disclosure is expressed on the surface of a T cell or an NK cell.

### KIR-CARS

KIRs, referred to as killer cell immunoglobulin-like receptors, have been characterized in humans and non-human primates, and are polymorphic type 1 trans-membrane molecules present on certain subsets of lymphocytes, including NK cells and some T cells. KIRs interact with determinants in the alpha 1 and 2 domains of the MHC class I molecules and, as described elsewhere herein, distinct KIRs are either stimulatory or inhibitory for NK cells.

RNKR-CARs and NKCARs described herein include RKIR-CARs and KIR-CARs, respectively, which share functional and structural properties with KIRs. For example, RKIR-CARs and KIR-CARs comprise an element (e.g., domain) from a KIR.

KIRs are a family of cell surface proteins found on NK cells. They regulate the killing function of these cells by interacting with MHC class I molecules, which are expressed on all cell types. This interaction allows them to detect virally infected cells or tumor cells. Most KIRs are inhibitory, meaning that their recognition of MHC suppresses the cytotoxic activity of the NK cell that expresses them. Only a limited number of KIRs have the ability to activate cells.

The KIR gene family have at least 15 gene loci (KIR2DL1, KIR2DL2/L3, KIR2DL4, KIR2DL5A, KIR2DL5B, KIR2DS1, KIR2DS2, KIR2DS3, KIR2DS4, KIR2DS5, KIR3DL1/S1, KIR3DL2, KIR3DL3 and two pseudogenes, KIR2DP1 and KIR3DP1) encoded within a 100-200 Kb region of the Leukocyte Receptor Complex (LRC) located on chromosome 19 (19q13.4). The LRC constitutes a large, 1 Mb, and dense cluster of rapidly evolving immune genes which contains genes encoding other cell surface molecules with distinctive Ig-like extra-cellular domains. In addition, the extended LRC contains genes encoding the transmembrane adaptor molecules DAP10 and DAP12.

KIR genes vary in length from 4 to 16 Kb (full genomic sequence) and can contain four to nine exons. KIR genes are classified as belonging to one of three groups according to their structural features: (1) Type I KIR2D genes, which encode two extra-cellular domain proteins with a D1 and D2 conformation; (2) The structurally divergent Type II KIR2D genes which encode two extra-cellular domain proteins with a D0 and D2 conformation; and finally (3) KIR3D genes encoding proteins with three extra-cellular Ig-like domains (D0, D1 and D2).

Type I KIR2D genes, which include the pseudogene KIR2DP1 as well as KIR2DL1-3 and KIR2DS1-5 genes, possess eight exons as well as a pseudoexon 3 sequence. This pseudoexon is inactivated in Type I KIR2D. In some cases this is due to a nucleotide substitution located on the intron 2-exon 3 splice-site where its nucleotide sequence exhibits a high-degree of identity to KIR3D exon 3 sequences and possesses a characteristic three base pair deletion. In other cases a premature stop codon initiates differential splicing of exon 3. Within the Type I KIR2D group of genes, KIR2DL1 and KIR2DL2 share a common deletion in exon 7 distinguishing them from all other KIR in this exon, which results in a shorter exon 7 sequence. Similarly, within Type I KIR2D, KIR2DL1-3 differ from KIR2DS 1-5 only in the length of their cytoplasmic tail encoding region in exon 9. The KIR2DP1 pseudogene structure differs from that of KIR2DL1-3 in that the former has a shorter exon 4 sequence, due to a single base pair deletion.

Type II KIR2D genes include KIR2DL4 and KIR2DL5. Unlike KIR3D and Type I KIR2D, Type II KIR2D characteristically have deleted the region corresponding to exon 4 in all other KIR. Additionally, Type II KIR2D genes differ from Type I KIR2D genes in that the former possess a translated exon 3, while Type I KIR2D genes have an untranslated pseudoexon 3 sequence in its place. Within the Type II KIR2D genes, KIR2DL4 is further differentiated from KIR2DL5 (as well as from other KIR genes) by the length of its exon 1 sequence. In KIR2DL4, exon 1 was found to be six nucleotides longer and to possess an initiation codon different from those present in the other KIR genes. This initiation codon is in better agreement with the 'Kozak transcription initiation consensus sequence' than the second potential initiation codon in KIR2DL4 that corresponds to the initiation codon present in other KIR genes.

KIR3D genes possess nine exons and include the structurally related KIR3DL1, KIR3DS1, KIR3DL2 and KIR3DL3 genes. KIR3DL2 nucleotide sequences are the longest of all KIR genes and span 16,256 bp in full genomic sequences and 1,368 bp in cDNA. Within the KIR3D group, the four KIR genes differ in the length of the region encoding the cytoplasmic tail in exon 9. The length of the cytoplasmic tail of KIR proteins can vary from 14 amino acid residues long (in some KIR3DS1 alleles) to 108 amino acid residues long (in KIR2DL4 proteins). Additionally, KIR3DS1 differs from KIR3DL1 or KIR3DL2 in that the former has a shorter exon 8 sequence. KIR3DL3 differs from other KIR sequences in that it completely lacks exon 6. The most extreme KIR gene structure difference observed was that of KIR3DP1. This gene fragment completely lacks exons 6 through 9, and occasionally also exon 2. The remaining portions of the gene which are present (exon 1, 3, 4 and 5) share a high level of sequence identity to other KIR3D sequences, in particular to KIR3DL3 sequences.

KIR proteins possess characteristic Ig-like domains on their extracellular regions, which in some KIR proteins are involved in HLA class I ligand binding. They also possess transmembrane and cytoplasmic regions which are functionally relevant as they define the type of signal which is transduced to the NK cell. KIR proteins can have two or three Ig-like domains (hence KIR2D or KIR3D) as well as short or long cytoplasmic tails (represented as KIR2DS or KIR2DL). Two domain KIR proteins are subdivided into two groups depending on the origin of the membrane distal Ig-like domains present. Type I KIR2D proteins (KIR2DL1, KIR2DL2, KIR2DL3, KIR2DS1, KIR2DS2, KIR2DS3, KIR2DS4 and KIR2DS5) possess a membrane-distal Ig-like domain similar in origin to the KIR3D D1 Ig-like domain but lack a D0 domain. This D1 Ig-like domain is encoded mainly by the fourth exon of the corresponding KIR genes. The Type II KIR2D proteins, KIR2DL4 and KIR2DL5, possess a membrane-distal Ig-like domain of similar sequence to the D0 domain present in KIR3D proteins, however, Type II KIR2D lack a D1 domain. Long cytoplasmic tails usually contain two Immune Tyrosine-based Inhibitory Motifs (ITIM) which transduce inhibitory signals to the NK cell. Short cytoplasmic tails possess a positively charged amino acid residue in their transmembrane region which allows them to associate with a DAP12 signaling molecule capable of generating an activation signal

Exceptions to this is KIR2DL4, which contains only one N-terminus ITIM. In addition, KIR2DL4 also possesses a charged residue (arginine) in its transmembrane domain, a feature which allows this receptor to elicit both inhibitory and activating signals. KIR control the response of human NK cells by delivering inhibitory or activating signals upon recognition of MHC class I ligands on the surface of potential target cells.

KIR proteins vary in length from 306 to 456 amino acid residues. Although the differences in protein length are mostly the consequence of the number of Ig-like domains present, cytoplasmic region length diversity is also an influencing factor. The leader peptide of most KIR proteins is 21 amino acid residues long. However, the presence of a different initiation codon generates a correspondingly longer leader peptide in KIR2DL4 proteins.

The D0 Ig-like domain present in Type II KIR2D proteins and KIR3D proteins is approximately 96 amino acid residues in length. The D1 domain of Type I KIR2D and of KIR3D proteins is 102 amino acid residues long, while the D2 domain of all KIR proteins is 98 amino acid residues long. The length of the stem region varies from the 24 amino acid residues present in most KIR proteins, to only seven amino acid residues in the divergent KIR3DL3 protein. The transmembrane region is 20 amino acid residues long for most KIR proteins, but one residue shorter on KIR2DL1 and KIR2DL2 proteins as a result of a three base pair deletion in exon 7. Finally, the cytoplasmic region of KIR proteins exhibits greater length variations, ranging from 23 amino acid residues in some KIR3DS1 alleles to the 96 amino acid residues present in KIR3DL2 proteins.

Amino acid sequences for human KIR polypeptides (Homo sapiens) are available in the NCBI database, see e.g., accession number NP_037421.2 (GI:134268644), NP_703144.2 (GI:46488946), NP_001229796.1 (GI:338968852), NP_001229796.1 (GI:338968852), NP_006728.2 (GI:134268642), NP_065396.1 (GI: 11968154), NP_001018091.1 (GI:66267727), NP_001077008.1 (GI:134133244), NP_036444.1 (GI:6912472), NP_055327.1 (GI:7657277), NP_056952.2 (GI:71143139), NP_036446.3 (GI:116517309), NP_001074239.1 (GI:124107610), NP_002246.5 (GI: 124107606), NP_001074241.1 (GI: 124107604), NP_036445.1 (GI:6912474).

The nomenclature for KIRs is based upon the number of extracellular domains (KIR2D and KIR3D having two and three extracellular Ig-domains, respectively) and whether the cytoplasmic tail is long (KIR2DL or KIR3DL) or short (KIR2DS or KIR3DS). The presence or absence of a given KIR is variable from one NK cell to another within the NK population present in a single individual. Among humans, there is also a relatively high level of polymorphism of KIR genes, with certain KIR genes being present in some, but not all individuals. The expression of KIR alleles on NK cells is stochastically regulated, meaning that, in a given individual, a given lymphocyte may express one, two, or more different KIRs, depending on the genoptype of the individual. The NK cells of a single individual typically express different combinations of KIRs, providing a repertoire of NK cells with different specificities for MHC class I molecules.

Certain KIR gene products cause stimulation of lymphocyte activity when bound to an appropriate ligand. The activating KIRs all have a short cytoplasmic tail with a charged trans-membrane residue that associates with an adapter molecule having an Immunoreceptor Tyrosine-based Activation Motifs (ITAMs) which transduce stimulatory signals to the NK cell. By contrast, inhibitory KIRs have a long cytoplasmic tail containing Immunoreceptor Tyrosine-based Inhibitory Motif (ITIM), which transduce inhibitory signals to the NK cell upon engagement of their MHC class I ligands. The known inhibitory KIRs include members of the KIR2DL and KIR3DL subfamilies. Inhibitory KIRs having two Ig domains (KIR2DL) recognize HLA-C allotypes: KIR2DL2 (formerly designated p58.2) and the closely related, allelic gene product KIR2DL3 both recognize "group 1" HLA-C allotypes (including HLA-Cw1, -3, -7, and -8), whereas KIR2DL1 (p58.1) recognizes "group 2" HLA-C allotypes (such as HLA-Cw2, -4, -5, and -6). The recognition by KIR2DL1 is dictated by the presence of a Lys residue at position 80 of HLA-C alleles. KIR2DL2 and KIR2DL3 recognition is dictated by the presence of an Asn residue at position 80 in HLA-C. Importantly, the great majority of HLA-C alleles have either an Asn or a Lys residue at position 80. Therefore, KIR2DL1, -2, and -3 collectively recognize essentially all HLA-C allotypes found in humans. One KIR with three Ig domains, KIR3DL1 (p70), recognizes an epitope shared by HLA-Bw4 alleles. Finally, KIR3DL2 (p140), a homodimer of molecules with three Ig domains, recognizes HLA-A3 and -A11.

However, the disclosure should not be limited to inhibitory KIRs comprising a cytoplasmic tail containing ITIM. Rather, any inhibitory protein having a cytoplasmic domain that is associated with an inhibitory signal can be used in the construction of the CARs (e.g., RNKR-CARs or NKR-CARs) of the disclosure. Non-limiting examples of an inhibitory protein include but are not limited CTLA-4, PD-1, and the like. These proteins are known to inhibit T cell activation.

Accordingly, the disclosure provides a RKIR-CAR or a KIR-CAR comprising an extracellular domain that comprises a target-specific binding element otherwise referred to as an antigen binding domain fused to a KIR or fragment thereof. In one instance, the KIR is an activating KIR that comprises a short cytoplasmic tail that associates with an adapter molecule having an Immunoreceptor Tyrosine-based Activation Motifs (ITAMs) which transduce stimulatory signals to the NK cell (referred elsewhere herein as actKIR-CAR). In one instance, the KIR is an inhibitory KIR that comprises a long cytoplasmic tail containing Immunoreceptor Tyrosine-based Inhibitory Motif (ITIM), which transduce inhibitory signals (referred elsewhere herein as RinhKIR-CAR or inhKIR-CAR). In some instances, it is desirable to remove the hinge region for the activating KIRs when constructing a RactKIR-CAR or an actKIR-CAR. This is because the disclosure is partly based on the discovery that an activating RKIR-CAR or activation KIR-CAR in which the KIR2DS2 hinge was removed to generate the KIR2S CAR, this KIRS2 CAR exhibited enhanced cytolytic activity compared to a RactKIR-CAR or an actKIR-CAR comprising a full length wildtype KIR2DS2.

The nucleic acid sequences coding for the desired molecules of the invention can be obtained using recombinant methods known in the art, such as, for example by screening libraries from cells expressing the gene, by deriving the gene from a vector known to include the same, or by isolating directly from cells and tissues containing the same, using standard techniques. Alternatively, the gene of interest can be produced synthetically, rather than cloned.

The present disclosure includes retroviral and lentiviral vector constructs expressing a RKIR-CAR or KIR-CAR that can be directly transduced into a cell. The present disclosure also includes an RNA construct that can be directly transfected into a cell. A method for generating mRNA for use in transfection involves in vitro transcription (IVT) of a template with specially designed primers, followed by polyA addition, to produce a construct containing 3' and 5' untranslated sequence ("UTR"), a 5' cap and/or Internal Ribosome Entry Site (IRES), the gene to be expressed, and a polyA tail, typically 50-2000 bases in length. RNA so produced can efficiently transfect different kinds of cells. In one instance, the template includes sequences for the RKIR-CAR or KIR-CAR.

In an embodiment, a RKIR-CAR or KIR-CAR comprises an antigen binding domain and a KIR transmembrane domain. In an embodiment, a RKIR-CAR or KIR-CAR comprises an antigen binding domain and a KIR intracellular domain, e.g., a RinhKIR-CAR or an inhKIR intracellular domain.

KIR D domain, as that term is used herein, refers to a DO, D1, or D2 domain of a KIR.

KIR D domain, as that term is used herein, refers to a polypeptide domain having structural and functional properties of a D domain of a KIR.

KIR D0 domain, as that term is used herein, refers to a D0 domain of a KIR. In an embodiment the KIR D0 domain of a RKIR-CAR or KIR-CAR has at least 70, 80, 85, 90, 95, or 99% homology with a reference sequence, e.g., a naturally occurring KIR D0 domain or a KIR D0 domain described herein. In embodiments the KIR D0 domain of a RKIR-CAR or KIR-CAR differs at no more than 15, 10, 5, 2, or 1% of its residues from a reference sequence, e.g., a naturally occurring KIR D0 domain or a KIR D0 domain described herein. In embodiments the KIR D0 domain of a RKIR-CAR or KIR-CAR differs at no more than 5, 4, 3, 2 or 1 residue from a reference sequence, e.g., a naturally occurring KIR D0 domain or a KIR D0 domain described herein. In embodiments the KIR D0 domain of a RKIR-CAR or KIR-CAR does not differ from, or shares 100% homology with, a reference sequence, e.g., a naturally occurring KIR D0 domain or a KIR D0 domain described herein.

KIR D1 domain, as that term is used herein, refers to a polypeptide domain having structural and functional properties of a D1 domain of a KIR. In an embodiment the KIR D1 domain of a RKIR-CAR or KIR-CAR has at least 70, 80, 85, 90, 95, or 99% homology with a reference sequence, e.g., a naturally occurring KIR D1 domain or a KIR D1 domain described herein. In embodiments the KIR D1 domain of a RKIR-CAR or KIR-CAR differs at no more than 15, 10, 5, 2, or 1% of its residues from a reference sequence, e.g., a naturally occurring KIR D1 domain or a KIR D1 domain described herein. In embodiments the KIR D1 domain of a RKIR-CAR or KIR-CAR differs at no more than 5, 4, 3, 2 or 1 residue from a reference sequence, e.g., a naturally occurring KIR D0 domain or a KIR D1 domain described herein. In embodiments the KIR D1 domain of a RKIR-CAR or KIR-CAR does not differ from, or shares 100% homology with, a reference sequence, e.g., a naturally occurring KIR D1 domain or a KIR D1 domain described herein.

KIR D2 domain, as that term is used herein, refers to a polypeptide domain having structural and functional properties of a D2 domain of a KIR. In an embodiment the KIR D2 domain of a RKIR-CAR or KIR-CAR has at least 70, 80, 85, 90, 95, or 99% homology with a reference sequence, e.g., a naturally occurring KIR D2 domain or a KIR D2 domain described herein. In embodiments the KIR D2 domain of a RKIR-CAR or KIR-CAR differs at no more than 15, 10, 5, 2, or 1% of its residues from a reference sequence, e.g., a naturally occurring KIR D2 domain or a KIR D2 domain described herein. In embodiments the KIR D2 domain of a RKIR-CAR or KIR-CAR differs at no more than 5, 4, 3, 2 or 1 residue from a reference sequence, e.g., a naturally occurring KIR D2 domain or a KIR D2 domain described herein. In embodiments the KIR D2 domain of a RKIR-CAR or KIR-CAR does not differ from, or shares 100% homology with, a reference sequence, e.g., a naturally occurring KIR D2 domain or a KIR D0 domain described herein.

KIR hinge or stem domain, as that term is used herein, refers to a polypeptide domain having structural and functional properties of a hinge or stem domain of a KIR. In an embodiment the KIR hinge or stem domain of a RKIR-CAR or KIR-CAR has at least 70, 80, 85, 90, 95, or 99% homology with a reference sequence, e.g., a naturally occurring KIR hinge or stem domain or a KIR hinge or stem domain described herein. In embodiments the KIR hinge or stem domain of a RKIR-CAR or KIR-CAR differs at no more than 15, 10, 5, 2, or 1% of its residues from a reference sequence, e.g., a naturally occurring KIR hinge or stem domain or a KIR hinge or stem domain described herein. In embodiments the KIR hinge or stem domain of a RKIR-CAR or KIR-CAR differs at no more than 5, 4, 3, 2 or 1 residue from a reference sequence, e.g., a naturally occurring KIR hinge or stem domain or a KIR hinge or stem domain described herein. In embodiments the KIR hinge or stem domain of a RKIR-CAR or KIR-CAR does not differ from, or shares 100% homology with, a reference sequence, e.g., a naturally occurring KIR hinge or stem domain or a KIR hinge or stem domain described herein.

KIR transmembrane domain, as that term is used herein, refers to a polypeptide domain having structural and functional properties of a transmembrane domain of a KIR. In an embodiment the KIR transmembrane domain of a RKIR-CAR or KIR-CAR has at least 70, 80, 85, 90, 95, or 99% homology with a reference sequence, e.g., a naturally occurring KIR transmembrane domain or a KIR transmembrane domain described herein. In embodiments the KIR transmembrane domain of a RKIR-CAR or KIR-CAR differs at no more than 15, 10, 5, 2, or 1% of its residues from a reference sequence, e.g., a naturally occurring KIR transmembrane domain or a KIR transmembrane domain described herein. In embodiments the KIR transmembrane domain of a RKIR-CAR or KIR-CAR differs at no more than 5, 4, 3, 2 or 1 residue from a reference sequence, e.g., a naturally occurring KIR transmembrane domain or a KIR transmembrane domain described herein. In embodiments the KIR transmembrane domain of a RKIR-CAR or KIR-CAR does not differ from, or shares 100% homology with, a reference sequence, e.g., a naturally occurring KIR transmembrane domain or a KIR transmembrane domain described herein.

KIR intracelluar domain, as that term is used herein, refers to a polypeptide domain having structural and functional properties of an intracellular domain of a KIR. KIR intracellular domains comprise inhibitory KIR intracellular domains (referred to herein as inhKIR intracellular domains) and activating KIR intracellular domains (referred to herein as actKIR intracellular domains). In an embodiment the inhKIR intracellular domain comprises an ITIM sequence. In an embodiment the KIR intracellular domain of a RKIR-CAR or KIR-CAR has at least 70, 80, 85, 90, 95, or 99% homology with a reference sequence, e.g., a naturally occurring KIR intracellular domain or a KIR intracellular domain described herein. In embodiments the KIR intracellular domain of a RKIR-CAR or KIR-CAR differs at no more than 15, 10, 5, 2, or 1% of its residues from a reference sequence, e.g., a naturally occurring KIR intracellular domain or a KIR intracellular domain described herein. In embodiments the KIR intracellular domain of a RKIR-CAR or KIR-CAR differs at no more than 5, 4, 3, 2 or 1 residue from a reference sequence, e.g., a naturally occurring KIR intracellular domain or a KIR intracellular domain described herein. In embodiments the KIR intracellular domain of a RKIR-CAR or KIR-CAR does not differ from, or shares 100% homology with, a reference sequence, e.g., a naturally occurring KIR intracellular domain or a KIR intracellular domain described herein.

### NCRs

RNKR-CARs or NKR-CARs described herein include RNCR-CARs or NCR-CARs, respectively, which share functional and structural properties with NCRs. For example, RNCR-CARs and NCR-CARs comprise an element (e.g., domain) from a NCR.

Natural killer (NK) cells are cytotoxic lymphoid cells specialized in destroying tumors and virus-infected cells. Unlike cytotoxic T lymphocytes, NK cells do not express antigen-specific receptors. The recognition of transformed cells occurs via the association of a multitude of cell-surface receptors with surface markers on the target cell. The NK cell surface receptors can be distinguished according to whether they activate or inhibit NK cell-mediated cytotoxicity. Numerous interactions between different receptors appear to lead to the formation of synapses between NK and target cells. The integration of activating and inhibiting signals at the synapse dictates whether or not the NK cells exert their cytolytic function on the target cell. Among the activating receptors, the family of Ig-like molecules is termed natural cytotoxicity receptors (NCRs). These natural cytotoxicity receptors include NKp30, NKp44 and NKp46 molecules. The NCRs are key activating receptors for NK cells in tumor cell recognition. All three NCRs are involved in the clearance of both tumor and virus-infected cells. In the latter, the antiviral activity is initiated by the interaction of NKp44 with hemagglutinin of influenza virus or Sendai virus. NKp46 targets virus-infected cells by binding to influenza virus hemagglutinin or Sendai virus hemagglutinin-neuraminidase. In contrast, it has been shown that NK cell-mediated cytotoxicity is inhibited by binding of NKp30 to the human cytomegaloviral protein pp65 (see, e.g., Arnon, et. al., Nat. Immunol. (2005) 6:515-523).

Amino acid sequences for a human NCR polypeptides (Homo sapiens) are available in the NCBI database, see e.g., accession number NP_004819.2 (GI: 153945782), 014931.1 (GI:47605770), O95944.2 (GI:251757303), 076036.1 (GI:47605775), NP_001138939.1 (GI:224586865), and/or NP_001138938.1 (GI:224586860).

In an embodiment, a RNCR-CAR or NCR-CAR comprises an antigen binding domain and a NCR transmembrane domain. In an embodiment, a RNCR-CAR or NCR-CAR comprises an antigen binding domain and a NCR intracellular domain.

NCR extracellular domain, as that term is used herein, refers to a polypeptide domain having structural and functional properties of a extracellular domain of a NCR. In an embodiment the NCR extracellular domain of a RNCR-CAR or NCR-CAR has at least 70, 80, 85, 90, 95, or 99% homology with a reference sequence, e.g., a naturally occurring NCR extracellular domain or a NCR extracellular domain described herein. In embodiments the NCR extracellular domain of a RNCR-CAR or NCR-CAR differs at no more than 15, 10, 5, 2, or 1% of its residues from a reference sequence, e.g., a naturally occurring NCR extracellular domain or a NCR extracellular domain described herein. In embodiments the NCR extracellular domain of a RNCR-CAR or NCR-CAR differs at no more than 5, 4, 3, 2 or 1 residue from a reference sequence, e.g., a naturally occurring NCR extracellular domain or a NCR extracellular domain described herein. In embodiments the NCR extracellular domain of a RNCR-CAR or NCR-CAR does not differ from, or shares 100% homology with, a reference sequence, e.g., a naturally occurring NCR extracellular domain or a NCR extracellular domain described herein.

NCR hinge or stem domain, as that term is used herein, refers to a polypeptide domain having structural and functional properties of a hinge or stem domain of a NCR. In an embodiment the NCR hinge or stem domain of a RNCR-CAR or NCR-CAR has at least 70, 80, 85, 90, 95, or 99% homology with a reference sequence, e.g., a naturally occurring NCR hinge or stem domain or a NCR hinge or stem domain described herein. In embodiments the NCR hinge or stem domain of a RNCR-CAR or NCR-CAR differs at no more than 15, 10, 5, 2, or 1% of its residues from a reference sequence, e.g., a naturally occurring NCR hinge or stem domain or a NCR hinge or stem domain described herein. In embodiments the NCR hinge or stem domain of a RNCR-CAR or NCR-CAR differs at no more than 5, 4, 3, 2 or 1 residue from a reference sequence, e.g., a naturally occurring NCR hinge or stem domain or a NCR hinge or stem domain described herein. In embodiments the NCR hinge or stem domain of a RNCR-CAR or NCR-CAR does not differ from, or shares 100% homology with, a reference sequence, e.g., a naturally occurring NCR hinge or stem domain or a NCR hinge or stem domain described herein.

NCR transmembrane domain, as that term is used herein, refers to a polypeptide domain having structural and functional properties of a transmembrane domain of a NCR. In an embodiment the NCR transmembrane domain of a RNCR-CAR or NCR-CAR has at least 70, 80, 85, 90, 95, or 99% homology with a reference sequence, e.g., a naturally occurring NCR transmembrane domain or a NCR transmembrane domain described herein. In embodiments the NCR transmembrane domain of a RNCR-CAR or NCR-CAR differs at no more than 15, 10, 5, 2, or 1% of its residues from a reference sequence, e.g., a naturally occurring NCR transmembrane domain or a NCR transmembrane domain described herein. In embodiments the NCR transmembrane domain of a RNCR-CAR or NCR-CAR differs at no more than 5, 4, 3, 2 or 1 residue from a reference sequence, e.g., a naturally occurring NCR transmembrane domain or a NCR transmembrane domain described herein. In embodiments the NCR transmembrane domain of a RNCR-CAR or NCR-CAR does not differ from, or shares 100% homology with, a reference sequence, e.g., a naturally occurring NCR transmembrane domain or a NCR transmembrane domain described herein.

NCR intracelluar domain, as that term is used herein, refers to a polypeptide domain having structural and functional properties of an intracellular domain of a NCR. In an embodiment the NCR intracellular domain of a RNCR-CAR or NCR-CAR has at least 70, 80, 85, 90, 95, or 99% homology with a reference sequence, e.g., a naturally occurring NCR intracellular domain or a NCR intracellular domain described herein. In embodiments the NCR intracellular domain of a RNCR-CAR or NCR-CAR differs at no more than 15, 10, 5, 2, or 1% of its residues from a reference sequence, e.g., a naturally occurring NCR intracellular domain or a NCR intracellular domain described herein. In embodiments the NCR intracellular domain of a RNCR-CAR or NCR-CAR differs at no more than 5, 4, 3, 2 or 1 residue from a reference sequence, e.g., a naturally occurring NCR intracellular domain or a NCR intracellular domain described herein. In embodiments the NCR intracellular domain of a RNCR-CAR or NCR-CAR does not differ from, or shares 100% homology with, a reference sequence, e.g., a naturally occurring NCR intracellular domain or a NCR intracellular domain described herein.

### SLAM Receptors

RNKR-CARs or NKR-CARs described herein include RSLAMF-CARs or SLAMF-CARs, respectively, which share functional and structural properties with SLAMFs. For example, RSLAMF-CARs and SLAMF-CARs comprise an element (e.g., domain) from a SLAMF receptor.

The signaling lymphocyte activation molecule (SLAM) family of immune cell receptors is closely related to the CD2 family of the immunoglobulin (Ig) superfamily of molecules. The SLAM family (SLAMF) currently includes nine members named SLAM, CD48, CD229, 2B4, CD84, NTB-A, CRACC, BLAME, and CD2F-10. In general, SLAM molecules possess two to four extracellular Ig domains, a transmembrane segment, and an intracellular tyrosine-rich region. The molecules are differentially expressed on a variety of immune cell types. Several are self ligands and SLAM has been identified as the human measles virus receptor. Several small SH2- containing adaptor proteins are known to associate with the intracellular domains of SLAM family members and modulate receptor signaling including SH2D1A (also known as SLAM-associated protein [SAP]) and SH2D1B (also known as EAT2). For example, in T and NK cells, activated SLAM family receptors become tyrosine phosphorylated and recruit the adaptor SAP and subsequently the Src kinase Fyn. The ensuing signal transduction cascade influences the outcome of T cell-antigen presenting cell and NK cell-target cell interactions.

Amino acid sequences for human SLAM receptor polypeptides (Homo sapiens) are available in the NCBI database, see e.g., accession number NP_057466.1 (GI: 7706529), NP_067004.3 (GI: 19923572), NP_003028.1 (GI:4506969), NP_001171808.1 (GI: 296434285), NP_001171643.1 (GI:296040491), NP_001769.2 (GI:21361571), NP_254273.2 (GI: 226342990), NP_064510.1 (GI: 9910342) and/or NP_002339.2 (GI: 55925578)

In an embodiment, a RSLAMF-CAR or SLAMF-CAR comprises an antigen binding domain and a SLAMF transmembrane domain. In an embodiment, a RSLAMF-CAR or SLAMF-CAR comprises an antigen binding domain and a SLAMF intracellular domain.

SLAMF extracellular domain, as that term is used herein, refers to a polypeptide domain having structural and functional properties of a extracellular domain of a SLAMF. In an embodiment the SLAMF extracellular domain of a RSLAMF-CAR or SLAMF-CAR has at least 70, 80, 85, 90, 95, or 99% homology with a reference sequence, e.g., a naturally occurring SLAMF extracellular domain or a SLAMF extracellular domain described herein. In embodiments the SLAMF extracellular domain of a RSLAMF-CAR or SLAMF-CAR differs at no more than 15, 10, 5, 2, or 1% of its residues from a reference sequence, e.g., a naturally occurring SLAMF extracellular domain or a SLAMF extracellular domain described herein. In embodiments the SLAMF extracellular domain of a RSLAMF-CAR or SLAMF-CAR differs at no more than 5, 4, 3, 2 or 1 residue from a reference sequence, e.g., a naturally occurring SLAMF extracellular domain or a SLAMF extracellular domain described herein. In embodiments the SLAMF extracellular domain of a RSLAMF-CAR or SLAMF-CAR does not differ from, or shares 100% homology with, a reference sequence, e.g., a naturally occurring SLAMF extracellular domain or a SLAMF extracellular domain described herein.

SLAMF hinge or stem domain, as that term is used herein, refers to a polypeptide domain having structural and functional properties of a hinge or stem domain of a SLAMF. In an embodiment the SLAMF hinge or stem domain of a RSLAMF-CAR or SLAMF-CAR has at least 70, 80, 85, 90, 95, or 99% homology with a reference sequence, e.g., a naturally occurring SLAMF hinge or stem domain or a SLAMF hinge or stem domain described herein. In embodiments the SLAMF hinge or stem domain of a RSLAMF-CAR or SLAMF-CAR differs at no more than 15, 10, 5, 2, or 1% of its residues from a reference sequence, e.g., a naturally occurring SLAMF hinge or stem domain or a SLAMF hinge or stem domain described herein. In embodiments the SLAMF hinge or stem domain of a RSLAMF-CAR or SLAMF-CAR differs at no more than 5, 4, 3, 2 or 1 residue from a reference sequence, e.g., a naturally occurring SLAMF hinge or stem domain or a SLAMF hinge or stem domain described herein. In embodiments the SLAMF hinge or stem domain of a RSLAMF-CAR or SLAMF-CAR does not differ from, or shares 100% homology with, a reference sequence, e.g., a naturally occurring SLAMF hinge or stem domain or a SLAMF hinge or stem domain described herein.

SLAMF transmembrane domain, as that term is used herein, refers to a polypeptide domain having structural and functional properties of a transmembrane domain of a SLAMF. In an embodiment the SLAMF transmembrane domain of a RSLAMF-CAR or SLAMF-CAR has at least 70, 80, 85, 90, 95, or 99% homology with a reference sequence, e.g., a naturally occurring SLAMF transmembrane domain or a SLAMF transmembrane domain described herein. In embodiments the SLAMF transmembrane domain of a RSLAMF-CAR or SLAMF-CAR differs at no more than 15, 10, 5, 2, or 1% of its residues from a reference sequence, e.g., a naturally occurring SLAMF transmembrane domain or a SLAMF transmembrane domain described herein. In embodiments the SLAMF transmembrane domain of a RSLAMF-CAR or SLAMF-CAR differs at no more than 5, 4, 3, 2 or 1 residue from a reference sequence, e.g., a naturally occurring SLAMF transmembrane domain or a SLAMF transmembrane domain described herein. In embodiments the SLAMF transmembrane domain of a RSLAMF-CAR or SLAMF-CAR does not differ from, or shares 100% homology with, a reference sequence, e.g., a naturally occurring SLAMF transmembrane domain or a SLAMF transmembrane domain described herein.

SLAMF intracelluar domain, as that term is used herein, refers to a polypeptide domain having structural and functional properties of an intracellular domain of a SLAMF. In an embodiment the SLAMF intracellular domain of a RSLAMF-CAR or SLAMF-CAR has at least 70, 80, 85, 90, 95, or 99% homology with a reference sequence, e.g., a naturally occurring SLAMF intracellular domain or a SLAMF intracellular domain described herein. In embodiments the SLAMF intracellular domain of a RSLAMF-CAR or SLAMF-CAR differs at no more than 15, 10, 5, 2, or 1% of its residues from a reference sequence, e.g., a naturally occurring SLAMF intracellular domain or a SLAMF intracellular domain described herein. In embodiments the SLAMF intracellular domain of a RSLAMF-CAR or SLAMF-CAR differs at no more than 5, 4, 3, 2 or 1 residue from a reference sequence, e.g., a naturally occurring SLAMF intracellular domain or a SLAMF intracellular domain described herein. In embodiments the SLAMF intracellular domain of a RSLAMF-CAR or SLAMF-CAR does not differ from, or shares 100% homology with, a reference sequence, e.g., a naturally occurring SLAMF intracellular domain or a SLAMF intracellular domain described herein.

### Fc-binding Receptors

RNKR-CARs and NKCARs described herein include CARs based on the Fc receptors, RFcR-CARs or FcR-CARs, respectively, which share functional and structural properties with CD16 and CD64. Exemplary RFcR-CARs include RCD16 -CARs and CD64-CARs. Exemplary FcR-CARs include CD16-CARs and CD64-CARs. For example, RFcR-CARs and FcR-CARs comprise an element (e.g., domain) from a FcR, e.g., CD16 or CD64.

Upon activation, NK cells produce cytokines and chemokines abundantly and at the same time exhibit potent cytolytic activity. Activation of NK cells can occur through the direct binding of NK cell receptors to ligands on the target cell, as seen with direct tumor cell killing, or through the crosslinking of the Fc receptor (CD 16; FcγRIII) by binding to the Fc portion of antibodies bound to an antigen-bearing cell. This CD16 engagement (CD16 crosslinking) initiates NK cell responses via intracellular signals that are generated through one, or both, of the CD16-associated adaptor chains, FcRγ or CD3 ζ. Triggering of CD16 leads to phosphorylation of the γ or ζ chain, which in turn recruits tyrosine kinases, syk and ZAP-70, initiating a cascade of signal transduction leading to rapid and potent effector functions. The most well-known effector function is the release of cytoplasmic granules carrying toxic proteins to kill nearby target cells through the process of antibody-dependent cellular cytotoxicity. CD16 crosslinking also results in the production of cytokines and chemokines that, in turn, activate and orchestrate a series of immune responses.

However, unlike T and B lymphocytes, NK cells are thought to have only a limited capacity for target recognition using germline-encoded activation receptors (Bottino et al., Curr Top Microbiol Immunol. 298:175-182 (2006); Stewart et al., Curr Top Microbiol Immunol. 298:1-21 (2006)). NK cells express the activating Fc receptor CD 16, which recognizes IgG-coated target cells, thereby broadening target recognition (Ravetch & Bolland, Annu Rev Immunol. 19:275-290 (2001); Lanier Nat. Immunol. 9(5):495-502 (2008); Bryceson & Long, Curr Opin Immunol. 20(3):344-352 (2008)). The expression and signal transduction activity of several NK cell activation receptors requires physically associated adaptors, which transduce signals through immunoreceptor tyrosine-based activation motifs (ITAMs). Among these adaptors, FcRγ and CD3 ζ chains can associate with CD16 and natural cytotoxicity receptors (NCRs) as either disulfide-linked homo-dimers or hetero-dimers, and these chains have been thought to be expressed by all mature NK cells.

Amino acid sequence for CD16 (Homo sapiens) is available in the NCBI database, see e.g., accession number NP_000560.5 (GI: 50726979), NP_001231682.1 (GI: 348041254)

In an embodiment, a RFcR-CAR or FcR-CAR comprises an antigen binding domain and a FcR transmembrane domain. In an embodiment, a RFcR-CAR or FcR-CAR comprises an antigen binding domain and a FcR intracellular domain.

CD16 extracellular domain, as that term is used herein, refers to a polypeptide domain having structural and functional properties of a extracellular domain of a CD16. In an embodiment the CD 16 extracellular domain of a RCD 16-CAR or CD16-CAR has at least 70, 80, 85, 90, 95, or 99% homology with a reference sequence, e.g., a naturally occurring CD 16 extracellular domain or a CD 16 extracellular domain described herein. In embodiments the CD 16 extracellular domain of a RCD 16-CAR or CD16-CAR differs at no more than 15, 10, 5, 2, or 1% of its residues from a reference sequence, e.g., a naturally occurring CD 16 extracellular domain or a CD 16 extracellular domain described herein. In embodiments the CD 16 extracellular domain of a RCD 16-CAR or CD16-CAR differs at no more than 5, 4, 3, 2 or 1 residue from a reference sequence, e.g., a naturally occurring CD16 extracellular domain or a CD16 extracellular domain described herein. In embodiments the CD16 extracellular domain of a RCD 16-CAR or CD16-CAR does not differ from, or shares 100% homology with, a reference sequence, e.g., a naturally occurring CD16 extracellular domain or a CD16 extracellular domain described herein.

CD16 hinge or stem domain, as that term is used herein, refers to a polypeptide domain having structural and functional properties of a hinge or stem domain of a CD16. In an embodiment the CD16 hinge or stem domain of a RCD 16-CAR or CD16-CAR has at least 70, 80, 85, 90, 95, or 99% homology with a reference sequence, e.g., a naturally occurring CD16 hinge or stem domain or a CD16 hinge or stem domain described herein. In embodiments the CD16 hinge or stem domain of a RCD 16-CAR or CD16-CAR differs at no more than 15, 10, 5, 2, or 1% of its residues from a reference sequence, e.g., a naturally occurring CD16 hinge or stem domain or a CD16 hinge or stem domain described herein. In embodiments the CD16 hinge or stem domain of a RCD 16-CAR or CD16-CAR differs at no more than 5, 4, 3, 2 or 1 residue from a reference sequence, e.g., a naturally occurring CD 16 hinge or stem domain or a CD 16 hinge or stem domain described herein. In embodiments the CD 16 hinge or stem domain of a RCD 16-CAR or CD 16-CAR does not differ from, or shares 100% homology with, a reference sequence, e.g., a naturally occurring CD 16 hinge or stem domain or a CD 16 hinge or stem domain described herein.

CD 16 transmembrane domain, as that term is used herein, refers to a polypeptide domain having structural and functional properties of a transmembrane domain of a CD 16. In an embodiment the CD 16 transmembrane domain of a RCD 16-CAR or CD 16-CAR has at least 70, 80, 85, 90, 95, or 99% homology with a reference sequence, e.g., a naturally occurring CD 16 transmembrane domain or a CD 16 transmembrane domain described herein. In embodiments the CD 16 transmembrane domain of a RCD 16-CAR or CD 16-CAR differs at no more than 15, 10, 5, 2, or 1% of its residues from a reference sequence, e.g., a naturally occurring CD 16 transmembrane domain or a CD 16 transmembrane domain described herein. In embodiments the CD 16 transmembrane domain of a RCD 16-CAR or CD16-CAR differs at no more than 5, 4, 3, 2 or 1 residue from a reference sequence, e.g., a naturally occurring CD 16 transmembrane domain or a CD 16 transmembrane domain described herein. In embodiments the CD 16 transmembrane domain of a RCD 16-CAR or CD16-CAR does not differ from, or shares 100% homology with, a reference sequence, e.g., a naturally occurring CD 16 transmembrane domain or a CD 16 transmembrane domain described herein.

CD16 intracellular domain, as that term is used herein, refers to a polypeptide domain having structural and functional properties of an intracellular domain of a CD 16. In an embodiment the CD 16 intracellular domain of a RCD 16-CAR or CD16-CAR has at least 70, 80, 85, 90, 95, or 99% homology with a reference sequence, e.g., a naturally occurring CD 16 intracellular domain or a CD 16 intracellular domain described herein. In embodiments the CD 16 intracellular domain of a RCD 16-CAR or CD 16-CAR differs at no more than 15, 10, 5, 2, or 1% of its residues from a reference sequence, e.g., a naturally occurring CD 16 intracellular domain or a CD 16 intracellular domain described herein. In embodiments the CD 16 intracellular domain of a RCD 16-CAR or CD 16-CAR differs at no more than 5, 4, 3, 2 or 1 residue from a reference sequence, e.g., a naturally occurring CD16 intracellular domain or a CD 16 intracellular domain described herein. In embodiments the CD 16 intracellular domain of a RCD 16-CAR or CD 16-CAR does not differ from, or shares 100% homology with, a reference sequence, e.g., a naturally occurring CD16 intracellular domain or a CD 16 intracellular domain described herein.

CD64 extracellular domain, as that term is used herein, refers to a polypeptide domain having structural and functional properties of a extracellular domain of a CD64. In an embodiment the CD64 extracellular domain of a RCD64-CAR or CD64-CAR has at least 70, 80, 85, 90, 95, or 99% homology with a reference sequence, e.g., a naturally occurring CD64 extracellular domain or a CD64 extracellular domain described herein. In embodiments the CD64 extracellular domain of a RCD64-CAR or CD64-CAR differs at no more than 15, 10, 5, 2, or 1% of its residues from a reference sequence, e.g., a naturally occurring CD64 extracellular domain or a CD64 extracellular domain described herein. In embodiments the CD64 extracellular domain of a RCD64-CAR or CD64-CAR differs at no more than 5, 4, 3, 2 or 1 residue from a reference sequence, e.g., a naturally occurring CD64 extracellular domain or a CD64 extracellular domain described herein. In embodiments the CD64 extracellular domain of a RCD64-CAR or CD64-CAR does not differ from, or shares 100% homology with, a reference sequence, e.g., a naturally occurring CD64 extracellular domain or a CD64 extracellular domain described herein.

CD64 hinge or stem domain, as that term is used herein, refers to a polypeptide domain having structural and functional properties of a hinge or stem domain of a CD64. In an embodiment the CD64 hinge or stem domain of a RCD64-CAR or CD64-CAR has at least 70, 80, 85, 90, 95, or 99% homology with a reference sequence, e.g., a naturally occurring CD64 hinge or stem domain or a CD64 hinge or stem domain described herein. In embodiments the CD64 hinge or stem domain of a RCD64-CAR or CD64-CAR differs at no more than 15, 10, 5, 2, or 1% of its residues from a reference sequence, e.g., a naturally occurring CD64 hinge or stem domain or a CD64 hinge or stem domain described herein. In embodiments the CD64 hinge or stem domain of a RCD64-CAR or CD64-CAR differs at no more than 5, 4, 3, 2 or 1 residue from a reference sequence, e.g., a naturally occurring CD64 hinge or stem domain or a CD64 hinge or stem domain described herein. In embodiments the CD64 hinge or stem domain of a RCD64-CAR or CD64-CAR does not differ from, or shares 100% homology with, a reference sequence, e.g., a naturally occurring CD64 hinge or stem domain or a CD64 hinge or stem domain described herein.

CD64 transmembrane domain, as that term is used herein, refers to a polypeptide domain having structural and functional properties of a transmembrane domain of a CD64. In an embodiment the CD64 transmembrane domain of a RCD64-CAR or CD64-CAR has at least 70, 80, 85, 90, 95, or 99% homology with a reference sequence, e.g., a naturally occurring CD64 transmembrane domain or a CD64 transmembrane domain described herein. In embodiments the CD64 transmembrane domain of a RCD64-CAR or CD64-CAR differs at no more than 15, 10, 5, 2, or 1% of its residues from a reference sequence, e.g., a naturally occurring CD64 transmembrane domain or a CD64 transmembrane domain described herein. In embodiments the CD64 transmembrane domain of a RCD64-CAR or CD64-CAR differs at no more than 5, 4, 3, 2 or 1 residue from a reference sequence, e.g., a naturally occurring CD64 transmembrane domain or a CD64 transmembrane domain described herein. In embodiments the CD64 transmembrane domain of a RCD64-CAR or CD64-CAR does not differ from, or shares 100% homology with, a reference sequence, e.g., a naturally occurring CD64 transmembrane domain or a CD64 transmembrane domain described herein.

CD64 intracelluar domain, as that term is used herein, refers to a polypeptide domain having structural and functional properties of an intracellular domain of a CD64. In an embodiment the CD64 intracellular domain of a RCD64-CAR or CD64-CAR has at least 70, 80, 85, 90, 95, or 99% homology with a reference sequence, e.g., a naturally occurring CD64 intracellular domain or a CD64 intracellular domain described herein. In embodiments the CD64 intracellular domain of a RCD64-CAR or CD64-CAR differs at no more than 15, 10, 5, 2, or 1% of its residues from a reference sequence, e.g., a naturally occurring CD64 intracellular domain or a CD64 intracellular domain described herein. In embodiments the CD64 intracellular domain of a RCD64-CAR or CD64-CAR differs at no more than 5, 4, 3, 2 or 1 residue from a reference sequence, e.g., a naturally occurring CD64 intracellular domain or a CD64 intracellular domain described herein. In embodiments the CD64 intracellular domain of a RCD64-CAR or CD64-CAR does not differ from, or shares 100% homology with, a reference sequence, e.g., a naturally occurring CD64 intracellular domain or a CD64 intracellular domain described herein.

### Ly49 and related Killer Cell Lectin-like Receptors

RNKR-CARs and NKCARs described herein include RLy49CARs or Ly49-CARs, respectively, which share functional and structural properties with Ly49. For example, RLy49-CARs and Ly49-CARs comprise an element (e.g., domain) from a Ly49 receptor.

The Ly49 receptors derive from at least 23 identified genes (Ly49A-W) in mice. These receptors share many of the same roles in mouse NK cells and T cells as that played by KIRs in humans despite their different structure (type II integral membrane proteins of the C-type lectin superfamily), and they also contain a considerable degree of genetic variation like human KIRs. The remarkable functional similarity between Ly49 and KIR receptors suggest that these groups of receptors have evolved independently yet convergently to perform the same physiologic functionals in NK cells and T cells.

Like KIRs in humans, different Ly49 receptors recognize different MHC class I alleles and are differentially expressed on subsets of NK cells. The original prototypic Ly49 receptors, Ly49A and Ly49C possess a cytoplasmic domain bearing two immunotyrosine-based inhibitory motifs (ITIM) similar to inhibitory KIRs such as KIR2DL3. These domains have been identified to recruit the phosphatase, SHP-1, and like the inhibitory KIRs, serve to limit the activation of NK cells and T cells. In addition to the inhibitory Ly49 molecules, several family members such as Ly49D and Ly49H have lost the ITIM-containing domains, and have instead acquired the capacity to interact with the signaling adaptor molecule, DAP12 similar to the activating KIRs such as KIR2DS2 in humans.

Amino acid sequence for Ly49 family members are available in the NCBI database, see e.g., accession numbers AAF82184.1 (GI: 9230810), AAF99547.1 (GI: 9801837), NP_034778.2 (GI: 133922593), NP_034779.1 (GI: 6754462), NP_001095090.1 (GI: 197333718), NP_034776.1 (GI: 21327665), AAK11559.1 (GI: 13021834) and/or NP_038822.3 (GI: 9256549).

In an embodiment, a RLy49-CAR or Ly49-CAR comprises an antigen binding domain and a Ly49 transmembrane domain. In an embodiment, a RLy49-CAR or Ly49-CAR comprises an antigen binding domain and a Ly49 intracellular domain.

LY49 extracellular domain, as that term is used herein, refers to a polypeptide domain having structural and functional properties of a extracellular domain of a LY49. In an embodiment the LY49 extracellular domain of a RLy49-CAR or LY49-CAR has at least 70, 80, 85, 90, 95, or 99% homology with a reference sequence, e.g., a naturally occurring LY49 extracellular domain or a LY49 extracellular domain described herein. In embodiments the LY49 extracellular domain of a RLy49-CAR or LY49-CAR differs at no more than 15, 10, 5, 2, or 1% of its residues from a reference sequence, e.g., a naturally occurring LY49 extracellular domain or a LY49 extracellular domain described herein. In embodiments the LY49 extracellular domain of a RLy49-CAR or LY49-CAR differs at no more than 5, 4, 3, 2 or 1 residue from a reference sequence, e.g., a naturally occurring LY49 extracellular domain or a LY49 extracellular domain described herein. In embodiments the LY49 extracellular domain of a RLy49-CAR or LY49-CAR does not differ from, or shares 100% homology with, a reference sequence, e.g., a naturally occurring LY49 extracellular domain or a LY49 extracellular domain described herein.

LY49 hinge or stem domain, as that term is used herein, refers to a polypeptide domain having structural and functional properties of a hinge or stem domain of a LY49. In an embodiment the LY49 hinge or stem domain of a RLy49-CAR or LY49-CAR has at least 70, 80, 85, 90, 95, or 99% homology with a reference sequence, e.g., a naturally occurring LY49 hinge or stem domain or a LY49 hinge or stem domain described herein. In embodiments the LY49 hinge or stem domain of a RLy49-CAR or LY49-CAR differs at no more than 15, 10, 5, 2, or 1% of its residues from a reference sequence, e.g., a naturally occurring LY49 hinge or stem domain or a LY49 hinge or stem domain described herein. In embodiments the LY49 hinge or stem domain of a RLy49-CAR or LY49-CAR differs at no more than 5, 4, 3, 2 or 1 residue from a reference sequence, e.g., a naturally occurring LY49 hinge or stem domain or a LY49 hinge or stem domain described herein. In embodiments the LY49 hinge or stem domain of a RLy49-CAR or LY49-CAR does not differ from, or shares 100% homology with, a reference sequence, e.g., a naturally occurring LY49 hinge or stem domain or a LY49 hinge or stem domain described herein.

LY49 transmembrane domain, as that term is used herein, refers to a polypeptide domain having structural and functional properties of a transmembrane domain of a LY49. In an embodiment the LY49 transmembrane domain of a RLy49-CAR or LY49-CAR has at least 70, 80, 85, 90, 95, or 99% homology with a reference sequence, e.g., a naturally occurring LY49 transmembrane domain or a LY49 transmembrane domain described herein. In embodiments the LY49 transmembrane domain of a RLy49-CAR or LY49-CAR differs at no more than 15, 10, 5, 2, or 1% of its residues from a reference sequence, e.g., a naturally occurring LY49 transmembrane domain or a LY49 transmembrane domain described herein. In embodiments the LY49 transmembrane domain of a RLy49-CAR or LY49-CAR differs at no more than 5, 4, 3, 2 or 1 residue from a reference sequence, e.g., a naturally occurring LY49 transmembrane domain or a LY49 transmembrane domain described herein. In embodiments the LY49 transmembrane domain of a RLy49-CAR or LY49-CAR does not differ from, or shares 100% homology with, a reference sequence, e.g., a naturally occurring LY49 transmembrane domain or a LY49 transmembrane domain described herein.

LY49 intracelluar domain, as that term is used herein, refers to a polypeptide domain having structural and functional properties of an intracellular domain of a LY49. In an embodiment the LY49 intracellular domain of a RLy49-CAR or LY49-CAR has at least 70, 80, 85, 90, 95, or 99% homology with a reference sequence, e.g., a naturally occurring LY49 intracellular domain or a LY49 intracellular domain described herein. In embodiments the LY49 intracellular domain of a RLy49-CAR or LY49-CAR differs at no more than 15, 10, 5, 2, or 1% of its residues from a reference sequence, e.g., a naturally occurring LY49 intracellular domain or a LY49 intracellular domain described herein. In embodiments the LY49 intracellular domain of a RLy49-CAR or LY49-CAR differs at no more than 5, 4, 3, 2 or 1 residue from a reference sequence, e.g., a naturally occurring LY49 intracellular domain or a LY49 intracellular domain described herein. In embodiments the LY49 intracellular domain of a RLy49-CAR or LY49-CAR does not differ from, or shares 100% homology with, a reference sequence, e.g., a naturally occurring LY49 intracellular domain or a LY49 intracellular domain described herein.

In addition to the NKRs described above, other receptors (e.g., immune cell receptors, e.g., NKRs) can be used in accordance with the compositions and methods provided herein (e.g., in NKR-CARs or RNKR-CARs). Other receptors (e.g., immune cell receptors, e.g., NKRs) include but are not limited to: NKG2D, CD160 (TM containing splice variant(s)), DNAM1, CRTAM, CD27, PSGL1, CD96, CD100, NKp80,CEACAM1, and CD244.

Genbank Accession numbers for the amino acid sequences of these receptors are, e.g., human NKG2D (NP_031386.2; GI:169234653), human CD160 (XP_005272987.1; GI:530363840, or XP_005272986.1; GI:530363838), human DNAM1 (NP_001290548.1; GI:746816112, or NP_001290547.1; GI:746816106), human CRTAM (NP_001291711.1; GI:755571554, or NP_062550.2; GI:51593098), human CD27 (NP_001233.1; GI:4507587), human PSGL1 (NP_001193538.1; GI:331284238, or NP_002997.2; GI:331284236), human CD96 (NP_937839.1; GI:38683840, or NP_005807.1; GI:5032141), human CD100 (NP_006369.3; GI:214010218, or NP_001135759.1; GI:214010220), human NKp80 (NP_001278751.1; GI:625180299, or NP_001278752.1; GI:625180293, or NP_057607.1; GI:7705574), human CEACAM1 (NP_001703.2 GI:19923195, or NP_001020083.1 GI:68161541,or NP_001171742.1 GI:296317305, or NP_001171744.1 GI:296317312, or NP_001171745.1 GI:296317314, or NP_001192273.1 GI:329112547), and human CD244 (NP_057466.1; GI:7706529, or NP_001160135.1; GI:262263435, or NP_001160136.1; GI:262263438).

### INHIBITORY RNKR-CARs AND NKR-CARs

The present disclosure provides compositions and methods for limiting the depletion of non-cancerous cells by a type of CAR-expressingcell therapy (e.g., CART or CARN therapy). As disclosed herein, a type of CAR-expressing cell therapy comprises the use of NK receptors including but is not limited to activating and inhibitory receptors of NK cells known as killer cell immunoglobulin-like receptor (KIR). Accordingly the disclosure provides compositions and methods of using a RNKR-CAR, e.g., a RKIR-CAR, including but is not limited to an activating RNKR-CAR (RactNKR-CAR), e.g., an activating RKIR-CAR (RactKIR-CAR) and an inhibitory RNKR-CAR (RinhNKR-CAR), e.g., an inhibitory RKIR-CAR (RinhKIR-CAR). Also provided are compositions and methods of using a NKR-CAR, e.g., a KIR-CAR, including but is not limited to an activating NKR-CAR (actNKR-CAR), e.g., an activating KIR-CAR (actKIR-CAR) and an inhibitory NKR-CAR (inhNKR-CAR), e.g., an inhibitory KIR-CAR (inhKIR-CAR).

In some embodiments, the KIR of an RinhKIR-CAR or inhKIR-CAR is an inhibitory KIR that comprises a long cytoplasmic tail containing Immunoreceptor Tyrosine-based Inhibitory Motif (ITIM), which transduce inhibitory signals (referred elsewhere herein as RinhKIR-CAR or inhKIR-CAR).

In some embodiments, an RinhKIR-CAR or inhKIR-CAR comprises a cytoplasmic domain of an inhibitory molecule other than KIR. These inhibitory molecules can, in some embodiments, decrease the ability of a cell to mount an immune effector response. Cytoplasmic domains of inhibitory molecules may be coupled, e.g., by fusion, to transmembrane domains of KIR. Exemplary inhibitory molecules are shown in table 3.

In some embodiments, an RinhKIR-CAR or inhKIR-CAR comprises a PD1 cytoplasmic domain. A PD1 cytoplasmic domain, as that term is used herein, refers to a polypeptide domain having structural and functional properties of a cytoplasmic domain of a PD1. In an embodiment the PD1 cytoplasmic domain of a RKIR-CAR or KIR-CAR has at least 70, 80, 85, 90, 95, or 99% homology with a reference sequence, e.g., a naturally occurring PD1 cytoplasmic domain or a PD1 cytoplasmic domain described herein (SEQ ID NO: 164). In embodiments the PD1 cytoplasmic domain of a RKIR-CAR or KIR-CAR differs at no more than 15, 10, 5, 2, or 1% of its residues from a reference sequence, e.g., a naturally occurring PD1 cytoplasmic domain or a PD1 cytoplasmic domain described herein. In embodiments the PD 1 cytoplasmic domain of a RKIR-CAR or KIR-CAR differs at no more than 5, 4, 3, 2 or 1 residue from a reference sequence, e.g., a naturally occurring PD1 cytoplasmic domain or a PD 1 cytoplasmic domain described herein. In embodiments the PD1 cytoplasmic domain of a RKIR-CAR or KIR-CAR does not differ from, or shares 100% homology with, a reference sequence, e.g., a naturally occurring PD1 cytoplasmic domain or a PD1 cytoplasmic domain described herein.

In some embodiments, an RinhKIR-CAR or inhKIR-CAR comprises a CTLA-4 cytoplasmic domain. A CTLA-4 cytoplasmic domain, as that term is used herein, refers to a polypeptide domain having structural and functional properties of a cytoplasmic domain of a CTLA-4. In an embodiment the CTLA-4 cytoplasmic domain of a RKIR-CAR or KIR-CAR has at least 70, 80, 85, 90, 95, or 99% homology with a reference sequence, e.g., a naturally occurring CTLA-4 cytoplasmic domain or a CTLA-4 cytoplasmic domain described herein (SEQ ID NO: 165). In embodiments the CTLA-4 cytoplasmic domain of a RKIR-CAR or KIR-CAR differs at no more than 15, 10, 5, 2, or 1% of its residues from a reference sequence, e.g., a naturally occurring CTLA-4 cytoplasmic domain or a CTLA-4 cytoplasmic domain described herein. In embodiments the CTLA-4 cytoplasmic domain of a RKIR-CAR or KIR-CAR differs at no more than 5, 4, 3, 2 or 1 residue from a reference sequence, e.g., a naturally occurring CTLA-4 cytoplasmic domain or a CTLA-4 cytoplasmic domain described herein. In embodiments the CTLA-4 cytoplasmic domain of a RKIR-CAR or KIR-CAR does not differ from, or shares 100% homology with, a reference sequence, e.g., a naturally occurring CTLA-4 cytoplasmic domain or a CTLA-4 cytoplasmic domain described herein.

In an embodiment, an RinhNKR-CAR or inhNKR-CAR, e.g., an RinhNKR-CAR or inhKIR-CAR, upon engagement with an antigen on a non-target or bystander cell, inactivates the cytotoxic cell comprising the RinhNKR-CAR or inhNKR-CAR. While much of the description below relates to RinhNKR-CAR or inhKIR-CARs, the invention includes the analogous application of other RinhNKR-CAR or inhNKR-CARs.

In one embodiment, T cells expressing the RactKIR-CAR or actKIR-CAR exhibit an antitumor property when bound to its target, whereas T cells expressing an RinhKIR-CAR or inhKIR-CAR results in inhibition of cell activity when the RinhKIR-CAR or inhKIR-CAR is bound to its target.

Regardless of the type of RKIR-CAR or KIR-CAR, RKIR-CARs and KIR-CARs are engineered to comprise an extracellular domain having an antigen binding domain fused to a cytoplasmic domain. In one embodiment, RKIR-CARs or KIR-CARs, when expressed in a T cell, are able to redirect antigen recognition based upon the antigen specificity. An exemplary antigen is CD19 because this antigen is expressed on B cell lymphoma. However, CD19 is also expressed on normal B cells, and thus CARs comprising an anti-CD 19 domain may result in depletion of normal B cells. Depletion of normal B cells can make a treated subject susceptible to infection, as B cells normally aid T cells in the control of infection. The present disclosure provides for compositions and methods to limit the depletion of normal tissue during RKIR-CAR-expressing or KIR-CAR-expressing cell therapy. In one instance, the present disclosure provides methods to treat cancer and other disorders using RKIR-CAR-expressing or KIR-CAR-expressing cell therapy while limiting the depletion of healthy bystander cells.

In one instance, the disclosure comprises controlling or regulating RKIR-CAR-expressing or KIR-CAR-expressing cell activity. In one instance, the disclosure comprises compositions and methods related to genetically modifying immune cells, e.g., T or NK cells, to express a plurality of types of RKIR-CARs or KIR-CARs, where RKIR-CAR-expressing or KIR-CAR-expressing cell activation is dependent on the binding of a plurality of types of RKIR-CARs or KIR-CARs to their target receptor. Dependence on the binding of a plurality of types of RKIR-CARs or KIR-CARs improves the specificity of the lytic activity of the RKIR-CAR-expressing or KIR-CAR-expressing cell, thereby reducing the potential for depleting normal healthy tissue.

In another instance, the disclosure comprises compositions and methods related to genetically modifying immune cells, e.g,. T or NK cells, with an inhibitory RKIR-CAR or KIR-CAR. In one instance, the inhibitory RKIR-CAR or KIR-CAR comprises an extracellular antigen binding domain that recognizes an antigen associated with a normal, non-cancerous, cell and an inhibitory cytoplasmic domain.

In one embodiment, the invention provides a dual RKIR-CAR or KIR-CAR where an immune cell is genetically modified to express an RinhKIR-CAR or inhKIR-CAR and an RactKIR-CAR and actKIR-CAR. In one embodiment, binding of the RinhKIR-CAR or inhKIR-CAR to a normal, non-cancerous cell results in the inhibition of the dual RKIR-CAR-expresing or KIR-CAR-expressing cell. For example, in one embodiment, binding of the RinhKIR-CAR or inhKIR-CAR to a normal, non-cancerous cell results in the death of the dual RKIR-CAR-expresing or KIR-CAR-expressing cell. In another embodiment, binding of the RinhKIR-CAR or inhKIR-CAR to a normal, non-cancerous cell results in inhibiting the signal transduction of the RactKIR-CAR or actKIR-CAR. In yet another embodiment, binding of the RinhKIR-CAR or inhKIR-CAR to a normal, non-cancerous cell results in the induction of a signal transduction signal that prevents the RactKIR-CAR-expressing or actKIR-CAR-expressing cell from exhibiting its anti-tumor activity. Accordingly, the dual RKIR-CAR or KIR-CAR comprising at least one RinhKIR-CAR or inhKIR-CAR and at least one RactKIR-CAR or actKIR-CAR of the disclosure provides a mechanism to regulate the activity of the dual RKIR-CAR-expressing or KIR-CAR-expressing cell.

In one instance, the present disclosure provides methods for treating cancer and other disorders using RKIR-CAR-expressing or KIR-CAR-expressing cell therapies while minimizing the depletion of normal healthy tissue. The cancer may be a hematological malignancy, a solid tumor, a primary or a metastasizing tumor. Other diseases treatable using the compositions and methods of the disclosure include viral, bacterial and parasitic infections as well as autoimmune diseases.

### EXTRACELLULAR HINGE DOMAIN

Extracellular hinge domain, as that term is used herein, refers to a polypeptide sequence of a RCAR, RNKR-CAR or NKR-CAR disposed between the transmembrane domain and antigen binding domain. In an embodiment the extracellular hinge domain allows sufficient distance from the outer surface of the cell and the antigen binding domain as well as flexibility to minimize steric hinderance between the cell and the antigen binding domain. In an embodiment the extracellular hinge domain is sufficiently short or flexible that it does not interfere with engagement of the cell that includes the RCAR, RNKR-CAR or NKR-CAR with an antigen bearing cell, e.g., a target cell. In an embodiment the extracellular hinge domain is from 2 to 20, 5 to 15, 7 to 12, or 8 to 10 amino acids in length. In an embodiment the hinge domain includes at least 50, 20, or 10 residues. In embodiments the hinge is 10 to 300, 10 to 250, or 10 to 200 residues in length. In an embodiment the distance from which the hinge extends from the cell is sufficiently short that the hinge does not hinder engagement with the surface of a target cell. In an embodiment the hinge extends less than 20, 15, or 10 nanometers from the surface of the cytotoxic cell. Thus, suitability for a hinge can be influenced by both linear length, the number of amino acid residues and flexibility of the hinge. An IgG4 hinge can be as long as 200 amino acids in length, but the distance it extends from the surface of the cytotoxic cell is smaller due to Ig-domain folding. A CD8alpha hinge, which is ∼43 amino acids is rather linear at ∼ 8 nm in length. In contrast, the IgG4 C2 & C3 hinge) is ∼200 amino acids in length, but has a distance from the cytotoxic cell surface comparable to that of the CD8 alpha hinge. While not wishing to be bound by theory, the similarity in extension is influenced by flexibility.

In some instances, the extracellular hinge domain is, e.g., a hinge from a human protein, a fragment thereof, or a short oligo- or polypeptide linker.

In some embodiments, the hinge is an artificial sequence. In one embodiment, the hinge is a short oligopeptide linker comprising a glycine-serine doublet.

In some embodiments, the hinge is a naturally occurring sequence. In some embodiments, the hinge can be a human Ig (immunoglobulin) hinge, or fragment thereof. In one embodiment, for example, the hinge comprises (e.g., consists of) the amino acid sequence of the IgG4 hinge (SEQ ID NO: 166). In one embodiment, for example, the hinge comprises (e.g., consists of) the amino acid sequence of the IgD hinge (SEQ ID NO: 167). In some embodiments, the hinge can be a human CD8 hinge, or fragment thereof. In one embodiment, for example, the hinge comprises (e.g., consists of) the amino acid sequence of the CD8 hinge (SEQ ID NO: 168).

| Table 24. Exemplary NKR domains, e.g., transmembrane, hinge or stem, or intracellular (e.g., cytoplasmic) domains (identified by the NKR from which the domain is derived) | |
|---|---|
| Killer immunoglobulin receptors (KIRs): | KIR2DL1 |
| | KIR2DL2/L3 |
| | KIR2DL4 |
| | KIR2DL5A |
| | KIR2DL5B |
| | KIR2DS1 |
| | KIR2DS2 |
| | KIR2DS3 |
| | KIR2DS4 |
| | KIR2DS5 |
| | KIR3DL1/S 1 |
| | KIR3DL2 |
| | KIR3DL3 |
| | KIR2DP1 |
| | KIR2DP1 |
| NCRs: | NKp30 |
| | NKp44 |
| | NKp46 |
| SLAM Receptors: | SLAM |
| | CD48 |
| | CD229 |
| | 2B4 |
| | CD84 |
| | NTB-A |
| | CRACC |
| | BLAME |
| | CD2F-10 |
| | SLAMF6 |
| | SLAMF7 |
| Fc-binding Receptors: | CD16, FcgRIII |
| | CD64 |
| Ly49 Receptors: | Ly49 |
| Lectin-related NK cell receptor | Ly49A |
| | Ly49C |
| Other NK receptors: | NKG2D |
| | CD160 (TM containing splice v aria nt(s)) |
| | DNAM1 |
| | CRTAM |
| | CD27 |
| | PSGL1 |
| | CD96 |
| | CD100 |
| | NKp80 |
| | CEACAM1 |
| | CD244 |

### ANTIGEN BINDING DOMAIN

The CARs described herein, e.g., the RCARs, NKR-CARs, and RNKR-CARs described herein, include an antigen binding domain in the extracellular region of the antigen binding member. An "antigen binding domain" as the term is used herein, refers to a molecule that has affinity for a target antigen, typically an antigen on a target cell, e.g., a cancer cell. An exemplary antigen binding domain comprises a polypeptide, e.g., an antibody molecule (which includes an antibody, and antigen binding fragments thereof, e.g., a immunoglobulin, single domain antibody (sdAb), and an scFv), or a non-antibody scaffold, e.g., a fibronectin, and the like. In embodiments, the antigen binding domain is a single polypeptide. In embodiments, the antigen binding domain comprises, one, two, or more, polypeptides.

The choice of an antigen binding domain can depend upon the type and number of ligands or receptors that define the surface of a target cell. For example, the antigen binding domain may be chosen to recognize a ligand or receptor that acts as a cell surface marker on target cells associated with a particular disease state. Examples of cell surface markers that may act as ligands or receptors include a cell surface marker associated with a particular disease state, e.g., cell surface makers for viral diseases, bacterial diseases parasitic infections, autoimmune diseases and disorders associated with unwanted cell proliferation, e.g., a cancer, e.g., a cancer described herein.

In the context of the present disclosure, "tumor antigen" or "proliferative disorder antigen" or "antigen associated with a proliferative disorder" refers to antigens that are common to specific proliferative disorders. In certain aspects, the proliferative disorder antigens of the present disclosure are derived from, cancers including but not limited to primary or metastatic melanoma, thymoma, lymphoma, sarcoma, lung cancer (e.g., NSCLC or SCLC), liver cancer, non-Hodgkin's lymphoma, Hodgkin's lymphoma, leukemias, multiple myeloma, glioblastoma, neuroblastoma, uterine cancer, cervical cancer, renal cancer, thyroid cancer, bladder cancer, kidney cancer, mesothelioma, and adenocarcinomas such as breast cancer, prostate cancer, ovarian cancer, pancreatic cancer, colon cancer and the like. In some embodiments, the cancer is B-cell acute lymphoid leukemia ("BALL"), T-cell acute lymphoid leukemia ("TALL"), acute lymphoid leukemia (ALL), acute myelogenous leukemia (AML); one or more chronic leukemias including but not limited to chronic myelogenous leukemia (CML), chronic lymphocytic leukemia (CLL); additional hematologic cancers or hematologic conditions including, but not limited to B cell prolymphocytic leukemia, blastic plasmacytoid dendritic cell neoplasm, Burkitt's lymphoma, diffuse large B cell lymphoma, follicular lymphoma, hairy cell leukemia, small cell- or a large cell-follicular lymphoma, malignant lymphoproliferative conditions, MALT lymphoma, mantle cell lymphoma, Marginal zone lymphoma, multiple myeloma, myelodysplasia and myelodysplastic syndrome, non-Hodgkin's lymphoma, plasmablastic lymphoma, plasmacytoid dendritic cell neoplasm, Waldenstrom macroglobulinemia.

In one embodiment, the tumor antigen comprises one or more antigenic cancer epitopes immunologically recognized by tumor infiltrating lymphocytes (TIL) derived from a cancer tumor of a mammal.

Tumor antigens are proteins that are produced by tumor cells that elicit an immune response, particularly T-cell mediated immune responses. The selection of the antigen binding domain of the disclosure will depend on the particular type of cancer to be treated. Tumor antigens are well known in the art and include, for example, a glioma-associated antigen, carcinoembryonic antigen (CEA), EGFRvIII, IL-11Ra, IL-13Ra, EGFR, FAP, B7H3, Kit, CA-IX, CS-1, MUC1, BCMA, bcr-abl, HER2, β-human chorionic gonadotropin, alphafetoprotein (AFP), ALK, CD19, CD123, cyclin B1, lectin-reactive AFP, Fos-related antigen 1, ADRB3, thyroglobulin, EphA2, RAGE-1, RU1, RU2, SSX2, AKAP-4, LCK, OY-TES1, PAX5, SART3, CLL-1, fucosyl GM1, GloboH, MN-CA IX, EPCAM, EVT6-AML, TGS5, human telomerase reverse transcriptase, plysialic acid, PLAC1, RU1, RU2 (AS), intestinal carboxyl esterase, lewisY, sLe, LY6K, mut hsp70-2, M-CSF, MYCN, RhoC, TRP-2, CYP1B1, BORIS, prostase, prostate-specific antigen (PSA), PAX3, PAP, NY-ESO-1, LAGE-1a, LMP2, NCAM, p53, p53 mutant, Ras mutant, gp100, prostein, OR51E2, PANX3, PSMA, PSCA, Her2/neu, hTERT, HMWMAA, HAVCR1, VEGFR2, PDGFR-beta, survivin and telomerase, legumain, HPV E6,E7, sperm protein 17, SSEA-4, tyrosinase, TARP, WT1, prostate-carcinoma tumor antigen-1 (PCTA-1), ML-IAP, MAGE, MAGE-A1,MAD-CT-1, MAD-CT-2, MelanA/MART1, XAGE1, ELF2M, ERG (TMPRSS2 ETS fusion gene), NA17, neutrophil elastase, sarcoma translocation breakpoints, NY-BR-1, ephrinB2, CD20, CD22, CD24, CD30, CD33, CD38, CD44v6, CD97, CD171, CD179a, androgen receptor, FAP, insulin growth factor (IGF)-I, IGF-II, IGF-I receptor, GD2, o-acetyl-GD2, GD3, GM3, GPRC5D, GPR20, CXORF61, folate receptor (FRa), folate receptor beta, ROR1, Flt3, TAG72, TN Ag, Tie 2, TEM1, TEM7R, CLDN6, TSHR, UPK2, and mesothelin. In a preferred embodiment, the tumor antigen is selected from the group consisting of folate receptor (FRa), mesothelin, EGFRvIII, IL-13Ra, CD123, CD19, CD33, BCMA, GD2, CLL-1, CA-IX, MUC1, HER2, and any combination thereof.

In one embodiment, the tumor antigen comprises one or more antigenic cancer epitopes associated with a malignant tumor. Malignant tumors express a number of proteins that can serve as target antigens for an immune attack. These molecules include but are not limited to tissue-specific antigens such as MART-1, tyrosinase and GP 100 in melanoma and prostatic acid phosphatase (PAP) and prostate-specific antigen (PSA) in prostate cancer. Other target antigens include transformation-related molecules such as the oncogene HER-2/Neu/ErbB-2. Yet another group of target antigens are onco-fetal antigens such as carcinoembryonic antigen (CEA). In B-cell lymphoma the tumor-specific idiotype immunoglobulin constitutes a truly tumor-specific immunoglobulin antigen that is unique to the individual tumor. B-cell differentiation antigens such as CD19, CD20 and CD37 are other candidates for target antigens in B-cell lymphoma.

Non-limiting examples of tumor antigens include the following: Differentiation antigens such as MART-1/MelanA (MART-I), gp100 (Pmel 17), tyrosinase, TRP-1, TRP-2 and tumor-specific multilineage antigens such as MAGE-1, MAGE-3, BAGE, GAGE-1, GAGE-2, p15; overexpressed embryonic antigens such as CEA; overexpressed oncogenes and mutated tumor-suppressor genes such as p53, Ras, HER-2/neu; unique tumor antigens resulting from chromosomal translocations; such as BCR-ABL, E2A-PRL, H4-RET, IGH-IGK, MYL-RAR; and viral antigens, such as the Epstein Barr virus antigens EBVA and the human papillomavirus (HPV) antigens E6 and E7. Other large, protein-based antigens include TSP-180, MAGE-4, MAGE-5, MAGE-6, RAGE, NY-ESO, p185erbB2, p180erbB-3, c-met, nm-23H1, PSA, TAG-72, CA 19-9, CA 72-4, CAM 17.1, NuMa, K-ras, beta-Catenin, CDK4, Mum-1, p 15, p 16, 43-9F, 5T4, 791Tgp72, alpha-fetoprotein, beta-HCG, BCA225, BTAA, CA 125, CA 15-3\CA 27.29\BCAA, CA 195, CA 242, CA-50, CAM43, CD68\P1, CO-029, FGF-5, G250, Ga733\EpCAM, HTgp-175, M344, MA-50, MG7-Ag, MOV18, NB/70K, NY-CO-1, RCAS1, SDCCAG16, TA-90\Mac-2 binding protein\cyclophilin C-associated protein, TAAL6, TAG72, TLP, and TPS.

Depending on the desired antigen to be targeted, the RCARs, NKR-CARs, or RNKR-CARs described herein can be engineered to include the appropriate antigen bind domain that is specific to the desired antigen target.

A RCAR, NKR-CAR, or RNKR-CAR as described herein, comprises an antigen binding domain (e.g., antibody or antibody fragment) that binds to a MHC presented-peptide. Normally, peptides derived from endogenous proteins fill the pocket of Major histocompatibility complex (MHC) class I molecules, and are recognized by T cell receptors (TCRs) on CD8 + T lymphocytes. The MHC class I complexes are constitutively expressed by all nucleated cells. In cancer, virus-specific and/or tumor-specific peptide/MHC complexes represent a unique class of cell surface targets for immunotherapy. TCR-like antibodies targeting peptides derived from viral or tumor antigens in the context of human leukocyte antigen (HLA)-A1 or HLA-A2 have been described (see, e.g., Sastry et al., J Virol. 2011 85(5):1935-1942; Sergeeva et al., Bood, 2011 117(16):4262-4272; Verma et al., J Immunol 2010 184(4):2156-2165; Willemsen etal., Gene Ther 2001 8(21) :1601-1608 ; Dao et al., Sci Transl Med 2013 5(176) :176ra33 ; Tassev etal., Cancer Gene Ther 2012 19(2):84-100). For example, TCR-like antibody can be identified from screening a library, such as a human scFv phage displayed library. Accordingly, the present disclosure provides a CAR, e.g., a RCAR, NKR-CAR, or RNKR-CAR described herein, that comprises an antigen binding domain that binds to a MHC presented peptide of a molecule selected from any tumor antigen described above that is expressed intracellularly, e.g., p53, BCR-Abl, Ras, K-ras, NY-ESO-1, and c-met.

Also provided herein are RCARs, NKR-CARs, or RNKR-CARs wherein the antigen binding member comprises a plurality of antigen binding domains. Without wishing to be bound by theory, it is believed that an antigen binding member comprising two or more antigen binding domains can result in additive or synergistic enhancement of activation and effector functions when the two or more corresponding antigens are encountered. Without wishing to be bound by theory, it is also believed that an antigen binding member comprising two or more antigen binding domains can increase the specificity of the effector cells for cancer cells versus normal cell, to offset antigen escape or to allow for targeting the cancer cell and the cancer microenvironment.

In this embodiment, the antigen binding member can comprise a plurality of, e.g., 2, 3, 4, or 5, antigen binding domains, e.g., scFvs, wherein each antigen binding domain binds to a target antigen. In an embodiment, two or more of the antigen binding domains can bind to different antigens. In an embodiment, two or more of the antigen binding domains can bind to the same antigen, e.g., the same or different epitopes on the same antigen. In an embodiment, the plurality of antigen binding domains are linked to each other, e.g., the C-terminus of a first antigen binding domain is linked to the N-terminus of a second antigen binding domain. In an embodiment, the C-terminus of a first antigen binding domain is linked to the N-terminus of a second antigen binding domain by a covalent bond, e.g., a peptide bond. The order of the antigen binding domains can be optimized for increased binding of the target antigens simulaneously, e.g., by the relative size of the corresponding target antigens. For example, for the larger of the target antigens, the corresponding antigen binding domain is disposed closer to the transmembrane domain of the antigen binding member; and for the smaller of the target antigens, the corresponding antigen binding domain is disposed farther from the transmembrane domain of the antigen binding member, e.g., more extracellularly. (See, e.g., Grada et al., 2013, *Mol Ther,* 2:e105).

In some embodiments, a linker or hinge region is disposed between each of the antigen binding domains, e.g., a linker or hinge region is disposed between the C-terminus of a first antigen binding domain and the N-terminus of a second antigen binding domain. By way of example, an antigen binding member comprising two antigen binding domains (e.g., ABD₁ and ABD₂) can be arranged in the following configuration: [ABD₁]-[linker/hinge]-[ABD₂]. Additional antigen binding domains can be added in a similar manner, optionally with linker or hinge regions disposed between the C-terminus of an antigen binding domain and the N-terminus of the next antigen binding domain. Linkers or hinge regions suitable for use in linking a plurality of antigen binding members are flexible, non-cleavable, and allow near-free motion of each antigen binding domain independent from the other antigen binding domains to encourage binding with multiple target antigens simultaneously. Any flexible linker or hinge region known in the art can be used. Examples of linkers include peptide linkers comprising glycine and serine residues, e.g., (GGGS)n, where n is a positive integer equal to or greater than 1, e.g., n= 1, 2, 3, 4, 5, 6, 7, 8, 9, or 10 (SEQ ID NO: 150). Examples of hinge regions include SEQ ID NO: 136.

### Antigen binding domains derived from an Antibody Molecule

The antigen binding domain can be derived from an antibody molecule, e.g., one or more of monoclonal antibodies, polyclonal antibodies, recombinant antibodies, human antibodies, humanized antibodies, single-domain antibodies e.g., a heavy chain variable domain (VH), a light chain variable domain (VL) and a variable domain (VHH) from, e.g., human or camelid origin. In some instances, it is beneficial for the antigen binding domain to be derived from the same species in which the RCAR, NKR-CAR, or RNKR-CAR will ultimately be used in, e.g., for use in humans, it may be beneficial for the antigen binding domain of the CAR, e.g., the RCAR, NKR-CAR, or RNKR-CAR, e.g., described herein, to comprise a human or a humanized antigen binding domain. Antibodies can be obtained using known techniques known in the art.

The term "antibody," as used herein, refers to an immunoglobulin molecule which specifically binds with a target antigen. An antibody can be intact immunoglobulin derived from natural sources or from recombinant sources and can be an immunoreactive portion of intact immunoglobulin. Antibodies are typically tetramers of immunoglobulin molecules. The antibody molecule described herein may exist in a variety of forms where the antigen binding portion of the antibody is expressed as part of a contiguous polypeptide chain including, for example, a single domain antibody fragment (sdAb), a single chain antibody (scFv) and a humanized or human antibody, e.g., as described herein.

The term "antibody fragment" refers to a portion of an intact antibody and refers to the antigenic determining variable regions of an intact antibody. Examples of antibody fragments include, but are not limited to, a single chain domain antibody (sdAb), Fab, Fab', F(ab')2, and Fv fragments, linear antibodies, scFv antibodies, a linear antibody, single domain antibody such as an sdAb (either VL or VH), a camelid VHH domain, and multispecific antibodies formed from antibody fragments.

An "antibody heavy chain," as used herein, refers to the larger of the two types of polypeptide chains present in all antibody molecules in their naturally occurring conformations.

An "antibody light chain," as used herein, refers to the smaller of the two types of polypeptide chains present in all antibody molecules in their naturally occurring conformations. κ and λ light chains refer to the two major antibody light chain isotypes.

By the term "synthetic antibody" as used herein, is meant an antibody molecule which is generated using recombinant DNA technology, such as, for example, an antibody molecule expressed by a bacteriophage as described herein. The term should also be construed to mean an antibody molecule which has been generated by the synthesis of a DNA molecule encoding the antibody molecule and which DNA molecule expresses an antibody protein, or an amino acid sequence specifying the antibody, wherein the DNA or amino acid sequence has been obtained using synthetic DNA or amino acid sequence technology which is available and well known in the art.

In embodiments, the antigen binding domain comprises a fragment of an antibody that is sufficient to confer recognition and specific binding to the target antigen. Examples of an antibody fragment include, but are not limited to, an Fab, Fab', F(ab')₂, or Fv fragment, an scFv antibody fragment, a linear antibody, single domain antibody such as an sdAb (either VL or VH), a camelid VHH domain, and multi-specific antibodies formed from antibody fragments.

In an embodiment, the antigen binding domain is a "scFv," which can comprise a fusion protein comprising a VL chain and a VH chain of an antibody, where the VH and VL are, e.g., linked via a short flexible polypeptide linker, e.g., a linker described herein. The scFv is capable of being expressed as a single chain polypeptide and retains the specificity of the intact antibody from which it is derived. Moreover, the VL and VH variable chains can be linked in either order, e.g., with respect to the N-terminal and C-terminal ends of the polypeptide, the scFv may comprise VL-linker-VH or may comprise VH-linker-VL. An scFv that can be prepared according to method known in the art (see, for example, Bird et al., (1988) Science 242:423-426 and Huston et al., (1988) Proc. Natl. Acad. Sci. USA 85:5879-5883).

As described above and elsewhere, scFv molecules can be produced by linking VH and VL chians together using flexible polypeptide linkers. In some embodiments, the scFv molecules comprise flexible polypeptide linker with an optimized length and/or amino acid composition. The flexible polypeptide linker length can greatly affect how the variable regions of a scFv fold and interact. In fact, if a short polypeptide linker is employed (e.g., between 5-10 amino acids, intrachain folding is prevented. For examples of linker orientation and size see, e.g., Hollinger et al. 1993 Proc Natl Acad. Sci. U.S.A. 90:6444-6448, U.S. Patent Application Publication Nos. 2005/0100543, 2005/0175606, 2007/0014794, and PCT publication Nos. WO2006/020258 and WO2007/024715. In one embodiment, the peptide linker of the scFv consists of amino acids such as glycine and/or serine residues used alone or in combination, to link variable heavy and variable light chain regions together. In one embodiment, the flexible polypeptide linker is a Gly/Ser linker and, e.g., comprises the amino acid sequence (Gly-Gly-Gly-Ser)n, where n is a positive integer equal to or greater than 1. For example, n=1, n=2, n=3. n=4, n=5 and n=6, n=7, n=8, n=9 and n=10. In one embodiment, the flexible polypeptide linkers include, but are not limited to, (Gly4 Ser)4 or (Gly4 Ser)3. In another embodiment, the linkers include multiple repeats of (Gly2Ser), (GlySer) or (Gly3Ser).

In some embodiments, the antigen binding domain is a single domain antigen binding (SDAB) molecules. A SDAB molecule includes molecules whose complementary determining regions are part of a single domain polypeptide. Examples include, but are not limited to, heavy chain variable domains, binding molecules naturally devoid of light chains, single domains derived from conventional 4-chain antibodies, engineered domains and single domain scaffolds other than those derived from antibodies (e.g., described in more detail below). SDAB molecules may be any of the art, or any future single domain molecules. SDAB molecules may be derived from any species including, but not limited to mouse, human, camel, llama, fish, shark, goat, rabbit, and bovine. This term also includes naturally occurring single domain antibody molecules from species other than *Camelidae* and sharks.

In one aspect, an SDAB molecule can be derived from a variable region of the immunoglobulin found in fish, such as, for example, that which is derived from the immunoglobulin isotype known as Novel Antigen Receptor (NAR) found in the serum of shark. Methods of producing single domain molecules derived from a variable region of NAR ("IgNARs") are described in WO 03/014161 and Streltsov (2005) Protein Sci. 14:2901-2909.

According to another aspect, an SDAB molecule is a naturally occurring single domain antigen binding molecule known as a heavy chain devoid of light chains. Such single domain molecules are disclosed in WO 9404678 and Hamers-Casterman, C. et al. (1993) Nature 363:446-448, for example. For clarity reasons, this variable domain derived from a heavy chain molecule naturally devoid of light chain is known herein as a VHH or nanobody to distinguish it from the conventional VH of four chain immunoglobulins. Such a VHH molecule can be derived from *Camelidae* species, for example in camel, llama, dromedary, alpaca and guanaco. Other species besides *Camelidae* may produce heavy chain molecules naturally devoid of light chain; such VHHs are within the scope of the invention.

Antibody proteins obtained from members of the camel and dromedary (Camelus bactrianus and Calelus dromaderius) family including new world members such as llama species (Lama paccos, Lama glama and Lama vicugna) have been characterized with respect to size, structural complexity and antigenicity for human subjects. Certain IgG antibodies from this family of mammals as found in nature lack light chains, and are thus structurally distinct from the typical four chain quaternary structure having two heavy and two light chains, for antibodies from other animals. See PCT/EP93/02214 (WO 94/04678 published 3 March 1994).

A region of the camelid antibody which is the small single variable domain identified as VHH can be obtained by genetic engineering to yield a small protein having high affinity for a target, resulting in a low molecular weight antibody-derived protein known as a "camelid nanobody". See U.S. patent number 5,759,808 issued June 2, 1998; see also Stijlemans et al., (2004) J Biol Chem 279:1256-1261; Dumoulin et al., (2003) Nature 424:783-788; Pleschberger et al., (2003) Bioconjugate Chem 14:440-448; Cortez-Retamozo et al., (2002) Int J Cancer 89:456-62; and Lauwereys et al., (1998) EMBO J 17:3512-3520. Engineered libraries of camelid antibodies and antibody fragments are commercially available, for example, from Ablynx, Ghent, Belgium . (e.g., US20060115470; Domantis (US20070065440, US20090148434). As with other antibodies of non-human origin, an amino acid sequence of a camelid antibody can be altered recombinantly to obtain a sequence that more closely resembles a human sequence, *i.e.,* the nanobody can be "humanized". Thus the natural low antigenicity of camelid antibodies to humans can be further reduced.

The camelid nanobody has a molecular weight approximately one-tenth that of a human IgG molecule, and the protein has a physical diameter of only a few nanometers. One consequence of the small size is the ability of camelid nanobodies to bind to antigenic sites that are functionally invisible to larger antibody proteins, *i.e.,* camelid nanobodies are useful as reagents detect antigens that are otherwise cryptic using classical immunological techniques, and as possible therapeutic agents. Thus yet another consequence of small size is that a camelid nanobody can inhibit as a result of binding to a specific site in a groove or narrow cleft of a target protein, and hence can serve in a capacity that more closely resembles the function of a classical low molecular weight drug than that of a classical antibody.

The low molecular weight and compact size further result in camelid nanobodies being extremely thermostable, stable to extreme pH and to proteolytic digestion, and poorly antigenic. Another consequence is that camelid nanobodies readily move from the circulatory system into tissues, and even cross the blood-brain barrier and can treat disorders that affect nervous tissue. Nanobodies can further facilitated drug transport across the blood brain barrier. See U.S. patent application 20040161738 published August 19, 2004. These features combined with the low antigenicity to humans indicate great therapeutic potential. Further, these molecules can be fully expressed in prokaryotic cells such as E. coli and are expressed as fusion proteins with bacteriophage and are functional.

An antigen binding domain can comprise a a camelid antibody or nanobody, or an antigen binding fragment thereof. Such antibodies can have high affinity for its cognate antigen. In certain embodiments herein, the camelid antibody or nanobody is naturally produced in the camelid animal, *i.e.,* is produced by the camelid following immunization with antigen or a peptide fragment thereof. Alternatively, the camelid nanobody is engineered, *i.e.,* produced by selection for example from a library of phage displaying appropriately mutagenized camelid nanobody proteins using panning procedures with the target antigen. Engineered nanobodies can further be customized by genetic engineering to have a half life in a recipient subject of from 45 minutes to two weeks. In a specific embodiment, the camelid antibody or nanobody is obtained by grafting the CDRs sequences of the heavy or light chain of the human antibodies of the disclosure into nanobody or single domain antibody framework sequences, as described for example in PCT/EP93/02214.

An antigen binding domain can comprise a single domain antibody, e.g., which relies only on a heavy chain variable region for binding, e.g., a nanobody. Nanobodies suitable for use herein can be made by the methods described in US2010/0028341, WO2009/030285, and WO2010/007376.

In certain embodiments, the SDAB molecule is a single chain fusion polypeptide comprising one or more single domain molecules (e.g., nanobodies), devoid of a complementary variable domain or an immunoglobulin constant, *e.g.,* Fc, region, that binds to one or more target antigens.

The SDAB molecules can be recombinant, CDR-grafted, humanized, camelized, de-immunized and/or in vitro generated (e.g., selected by phage display).

In one embodiment, the antigen biding domain portion comprises a human antibody or a fragment thereof.

In some embodiments, a non-human antibody is humanized, where specific sequences or regions of the antibody are modified to increase similarity to an antibody naturally produced in a human. In an embodiment, the antigen binding domain is humanized.

Non human antibodies can be humanized using a variety of techniques known in the art, e.g., CDR-grafting (see, e.g., European Patent No. EP 239,400; International Publication No. WO 91/09967; and U.S. Pat. Nos. 5,225,539, 5,530,101, and 5,585,089), veneering or resurfacing (see, e.g., European Patent Nos. EP 592,106 and EP 519,596; Padlan, 1991, Molecular Immunology, 28(4/5):489-498; Studnicka et al., 1994, Protein Engineering, 7(6):805-814; and Roguska et al., 1994, PNAS, 91:969-973), chain shuffling (see, e.g., U.S. Pat. No. 5,565,332), and techniques disclosed in, e.g., U.S. Patent Application Publication No. US2005/0042664, U.S. Patent Application Publication No. US2005/0048617, U.S. Pat. No. 6,407,213, U.S. Pat. No. 5,766,886, International Publication No. WO 9317105, Tan et al., 2002, J. Immunol., 169:1119-25; Caldas et al., 2000, Protein Eng., 13(5):353-60; Morea et al., 2000, Methods, 20:267-79; Baca et al., 1997, J. Biol. Chem., 272:10678-84; Roguska et al., 1996, Protein Eng., 9(10):895-904; Couto et al., 1995, Cancer Res., 55 :5973s-5977; Couto et al., 1995, Cancer Res., 55(8):1717-22; Sandhu 1994 Gene, 150(2):409-10; and Pedersen et al., 1994, J. Mol. Biol., 235(3):959-73. Often, framework residues in the framework regions will be substituted with the corresponding residue from the CDR donor antibody to alter, for example improve, antigen binding. These framework substitutions are identified by methods well-known in the art, e.g., by modeling of the interactions of the CDR and framework residues to identify framework residues important for antigen binding and sequence comparison to identify unusual framework residues at particular positions. (See, e.g., Queen et al., U.S. Pat. No. 5,585,089; and Riechmann et al., 1988, Nature, 332:323). In preferred embodiments, the humanized antibody molecule comprises a sequence described herein, e.g., a variable light chain and/or a variable heavy chain described herein,.

A humanized antibody has one or more amino acid residues introduced into it from a source which is nonhuman. These nonhuman amino acid residues are often referred to as "import" residues, which are typically taken from an "import" variable domain. Thus, humanized antibodies comprise one or more CDRs from nonhuman immunoglobulin molecules and framework regions from human. Humanization of antibodies is well-known in the art and can essentially be performed following the method of Winter and co-workers (Jones et al., Nature, 321:522-525 (1986); Riechmann et al., Nature, 332:323-327 (1988); Verhoeyen et al., Science, 239:1534-1536 (1988)), by substituting rodent CDRs or CDR sequences for the corresponding sequences of a human antibody, i.e., CDR-grafting (EP 239,400; PCT Publication No. WO 91/09967; and U.S. Pat. Nos. 4,816,567; 6,331,415; 5,225,539; 5,530,101; 5,585,089; 6,548,640).

In such humanized chimeric antibodies, substantially less than an intact human variable domain has been substituted by the corresponding sequence from a nonhuman species. In practice, humanized antibodies are typically human antibodies in which some CDR residues and possibly some framework (FR) residues are substituted by residues from analogous sites in rodent antibodies. Humanization of antibodies can also be achieved by veneering or resurfacing (EP 592,106; EP 519,596; Padlan, 1991, Molecular Immunology, 28(4/5):489-498; Studnicka et al., Protein Engineering, 7(6):805-814 (1994); and Roguska et al., PNAS, 91:969-973 (1994)) or chain shuffling (U.S. Pat. No. 5,565,332).

In some instances, the antibody of the disclosure is further prepared using an antibody having one or more of the VH and/or VL sequences disclosed herein can be used as starting material to engineer a modified antibody, which modified antibody may have altered properties as compared to the starting antibody. In various instances, the antibody is engineered by modifying one or more amino acids within one or both variable regions (i.e., VH and/or VL), for example within one or more CDR regions and/or within one or more framework regions.

In another aspect, the antigen binding domain is a T cell receptor ("TCR"), or a fragment thereof, for example, a single chain TCR (scTCR). Methods to make such TCRs are known in the art. See, e.g., Willemsen RA et al, Gene Therapy 7: 1369-1377 (2000); Zhang T et al, Cancer Gene Ther 11: 487-496 (2004); Aggen et al, Gene Ther. 19(4):365-74 (2012). For example, scTCR can be engineered that contains the Vα and Vβ genes from a T cell clone linked by a linker (e.g., a flexible peptide). This approach is very useful to cancer associated target that itself is intracellular, however, a fragment of such antigen (peptide) is presented on the surface of the cancer cells by MHC. The TCR sequences may be naturally occurring, or a non-naturally occurring synthetic sequences.

### Non-Antibody Scaffolds

In instances, the antigen binding domain comprises a non antibody scaffold, e.g., a fibronectin, ankyrin, domain antibody, lipocalin, small modular immuno-pharmaceutical, maxybody, Protein A, or affilin. The non antibody scaffold has the ability to bind to target antigen on a cell. In instances, the antigen binding domain is a polypeptide or fragment thereof of a naturally occurring protein expressed on a cell. In some instances, the antigen binding domain comprises a non-antibody scaffold. A wide variety of non-antibody scaffolds can be employed so long as the resulting polypeptide includes at least one binding region which specifically binds to the target antigen on a target cell.

Non-antibody scaffolds include: fibronectin (Novartis, MA), ankyrin (Molecular Partners AG, Zurich, Switzerland), domain antibodies (Domantis, Ltd., Cambridge, MA, and Ablynx nv, Zwijnaarde, Belgium), lipocalin (Pieris Proteolab AG, Freising, Germany), small modular immuno-pharmaceuticals (Trubion Pharmaceuticals Inc., Seattle, WA), maxybodies (Avidia, Inc., Mountain View, CA), Protein A (Affibody AG, Sweden), and affilin (gamma-crystallin or ubiquitin) (Scil Proteins GmbH, Halle, Germany).

Fibronectin scaffolds can be based on fibronectin type III domain (e.g., the tenth module of the fibronectin type III (¹⁰ Fn3 domain)). The fibronectin type III domain has 7 or 8 beta strands which are distributed between two beta sheets, which themselves pack against each other to form the core of the protein, and further containing loops (analogous to CDRs) which connect the beta strands to each other and are solvent exposed. There are at least three such loops at each edge of the beta sheet sandwich, where the edge is the boundary of the protein perpendicular to the direction of the beta strands (see US 6,818,418). Because of this structure, this non-antibody scaffold mimics antigen binding properties that are similar in nature and affinity to those of antibodies. These scaffolds can be used in a loop randomization and shuffling strategy in vitro that is similar to the process of affinity maturation of antibodies in vivo.

The ankyrin technology is based on using proteins with ankyrin derived repeat modules as scaffolds for bearing variable regions which can be used for binding to different targets. The ankyrin repeat module is a 33 amino acid polypeptide consisting of two antiparallel α-helices and a β-turn. Binding of the variable regions is mostly optimized by using ribosome display.

Avimers are derived from natural A-domain containing protein such as HER3. These domains are used by nature for protein-protein interactions and in human over 250 proteins are structurally based on A-domains. Avimers consist of a number of different "A-domain" monomers (2-10) linked via amino acid linkers. Avimers can be created that can bind to the target antigen using the methodology described in, for example, U.S. Patent Application Publication Nos. 20040175756; 20050053973; 20050048512; and 20060008844. Affibody affinity ligands are small, simple proteins composed of a three-helix bundle based on the scaffold of one of the IgG-binding domains of Protein A. Protein A is a surface protein from the bacterium Staphylococcus aureus. This scaffold domain consists of 58 amino acids, 13 of which are randomized to generate affibody libraries with a large number of ligand variants (See e.g., US 5,831,012). Affibody molecules mimic antibodies, they have a molecular weight of 6 kDa, compared to the molecular weight of antibodies, which is 150 kDa. In spite of its small size, the binding site of affibody molecules is similar to that of an antibody.

Protein epitope mimetics (PEM) are medium-sized, cyclic, peptide-like molecules (MW 1-2kDa) mimicking beta-hairpin secondary structures of proteins, the major secondary structure involved in protein-protein interactions. Antigen binding domains, e.g., those comprising scFv, single domain antibodies, or camelid antibodies, can be directed to any target receptor/ligand described herein, e.g., the the PD1 receptors, PD1-L1 or PD1-L2.

### MISMATCHED ANTIGEN BINDING DOMAINS

In some cases, a cell described herein comprises a CAR (e.g., RCAR, NKR-CAR, or RNKR-CAR) containing a first antigen binding domain and a second CAR (e.g., RCAR, NKR-CAR, or RNKR-CAR) containing a second antigen binding domain. For example, a cell described herein comprises a RCAR and an inhNKR-CAR, where the RCAR and inhNKR-CAR comprise different antigen binding domains. It has been discovered, that cells having a plurality of chimeric membrane embedded receptors each comprising an antigen binding domain (CMERs) that interactions between the antigen binding domain of the CMER can be undesirable, e.g., because it inhibits the ability of one or more of the antigen binding domains to bind its cognate antigen. Accordingly, disclosed herein are a first and a second non-naturally occurring CMER, comprising antigen binding domains that minimize such interactions when expressed in the same cell wherein said first CMER is an RCAR, RNKR-CAR, or NKR-CAR. In an instance a plurality of CMERs comprises two CARs, e.g., two of RCARs, RNKR-CARs, or NKR-CARs. In an instance a plurality of CMERs comprises a a RCAR and a NKR-CAR, e.g., a RCAR and an inhNKR-CAR.

In some instances, the disclosure comprises a first and second CMER, wherein the antigen binding domain of one of said first CMER said second CMER does not comprise a variable light domain and a variable heavy domain, wherein one of said first and second CMER is a RCAR, RNKR-CAR, or NKR-CAR. In some instances, the antigen binding domain of one of said first CMER said second CMER is an scFv, and the other is not an scFv, wherein one of said first and second CMER is a RCAR, RNKR-CAR, or NKR-CAR. In some instances, the antigen binding domain of one of said first CMER said second CMER comprises a single VH domain, e.g., a camelid, shark, or lamprey single VH domain, or a single VH domain derived from a human or mouse sequence or a non-antibody scaffold, wherein one of said first and second CMER is a RCAR, RNKR-CAR, or NKR-CAR. In some instances, the antigen binding domain of one of said first CMER said second CMER comprises a nanobody, wherein one of said first and second CMER is a RCAR, RNKR-CAR, or NKR-CAR. In some instances, the antigen binding domain of one of said first CMER said second CMER comprises a camelid VHH domain, wherein one of said first and second CMER is a RCAR, RNKR-CAR, or NKR-CAR.

In some instances, the antigen binding domain of one of said first CMER said second CMER comprises an scFv, and the other comprises a single VH domain, e.g., a camelid, shark, or lamprey single VH domain, or a single VH domain derived from a human or mouse sequence, wherein one of said first and second CMER is a RCAR, RNKR-CAR, or NKR-CAR. In some instances, the antigen binding domain of one of said first CMER said second CMER comprises an scFv, and the other comprises a nanobody, wherein one of said first and second CMER is a RCAR, RNKR-CAR, or NKR-CAR. In some instances, the antigen binding domain of one of said first CMER said second CMER comprises an scFv, and the other comprises a camelid VHH domain, wherein one of said first and second CMER is a RCAR, RNKR-CAR, or NKR-CAR.

In some instances, when present on the surface of a cell, binding of the antigen binding domain of said first CMER to its cognate antigen is not substantially reduced by the presence of said second CMER, wherein one of said first and second CMER is a RCAR, RNKR-CAR, or NKR-CAR. In some instances, binding of the antigen binding domain of said first CMER to its cognate antigen in the presence of said second CMER is 85%, 90%, 95%, 96%, 97%, 98% or 99% of binding of the antigen binding domain of said first CMER to its cognate antigen in the absence of said second CMER, wherein one of said first and second CMER is a RCAR, RNKR-CAR, or NKR-CAR.

In some instances, when present on the surface of a cell, the antigen binding domains of said first CMER said second CMER, associate with one another less than if both were scFv antigen binding domains, wherein one of said first and second CMER is a RCAR, RNKR-CAR, or NKR-CAR. In some instances, the antigen binding domains of said first CMER said second CMER, associate with one another 85%, 90%, 95%, 96%, 97%, 98% or 99% less than if both were scFv antigen binding domains, wherein one of said first and second CMER is a RCAR, RNKR-CAR, or NKR-CAR.

### INTRACELLULAR SIGNALING DOMAIN

In embodiments, an intracellular signaling domain produces an intracellular signal when an extracellular domain, e.g., an antigen binding domain, to which it is fused, or coupled by a dimerization switch, binds a counter ligand. Intracellular signaling domains can include primary intracellular signaling domains and costimulatory signaling domains. In an embodiment, a RNKR-CAR, RCAR, or NKR-CAR molecule can be constructed for expression in an immune cell, e.g., a T or NK cell, such that the RNKR-CAR, RCAR, or NKR-CAR molecule comprises a domain, e.g., a primary intracellular signaling domains, costimulatory signaling domain, inhibitory domains, etc., that is derived from a polypeptide that is typically associated with the immune cell. For example, a RNKR-CAR, RCAR, or NKR-CAR for expression in a T cell can comprise a 41BB domain and an CD3 zeta domain. In this instance, both the 41BB and CD3 zeta domains are derived from polypeptides associated with the T cell. In another embodiment, a RNKR-CAR, RCAR, or NKR-CAR molecule can be constructed for expression in an immune cell e.g., a T or NK cell, such that the RNKR-CAR, RCAR, or NKR-CAR molecule comprises a domain that is derived from a polypeptide that is not typically associated with the immune cell. For example, a RNKR-CAR, RCAR, or NKR-CAR for expression in a T cell can comprise a KIR domain derived from a NK cell. Alternatively, a RNKR-CAR, RCAR, or NKR-CAR for expression in a NK cell can comprise a 41BB domain and a CD3 zeta domain derived from a T cell (See e.g. WO2013/033626).

### PRIMARY INTRACELLULAR SIGNALING DOMAIN

In an embodiments a primary intracellular signaling domain produces an intracellular signal when an extracellular domain, e.g., an antigen binding domain, to which it is fused, or coupled by a dimerization switch, binds cognate antigen. It is derived from a primary stimulatory molecule, e.g., it comprises intracellular sequence of a primary stimulatory molecule. It comprises sufficient primary stimulatory molecule sequence to produce an intracellular signal, e.g., when an antigen binding domain to which it is fused, or coupled by a dimerization switch, binds cognate antigen.

A primary stimulatory molecule, is a molecule, that upon binding cognate ligand, mediates an immune effector response, e.g., in the cell in which it is expressed. Typically, it generates an intracellular signal that is dependent on binding to a cognate ligand that comprises antigen. The TCR/CD3 complex contains exemplary primary stimulatory molecules; the complex generates an intracellular signal upon binding to cognate ligand, e.g., an MHC molecule loaded with a peptide. Typically, e.g., in the case of the TCR/CD3 primary stimulatory molecule, the generation of an intracellular signal by a primary intracellular signaling domain is dependent on binding of the primary stimulatory molecule to its ligand, e.g., antigen.

Primary stimulation can mediate altered expression of certain molecules, such as downregulation of TGF-β, and/or reorganization of cytoskeletal structures, and the like. Stimulation, can, e.g., in the presence of costimulation, result in an optimization, e.g., an increase, in an immune effector function of the RNKR-CARX, RCAR/NKR-CARX, or RCARX cell, e.g., RNKR-CART, RCAR/NKR-CART, or RCART cell or RNKR-CARN, RCAR/NKR-CARN, or RCARN. Stimulation, e.g., in the context of a RNKR-CART, RCAR/NKR-CART, or RCART cell, can mediate a T cell response, e.g., proliferation, cytokine secretion, killing, activation, differentiation, and the like.

In an embodiment, the primary intracellular signaling domain comprises a signaling motif, e.g., an immunoreceptor tyrosine-based activation motif or ITAMs. A primary intracellular signaling domain can comprise ITAM containing cytoplasmic signaling sequences from TCR zeta (CD3 zeta), FcR gamma, FcR beta, CD3 gamma, CD3 delta, CD3 epsilon, CD5, CD22, CD79a, and CD79b.

Exemplary primary intracellular signaling domains are provided in Table 1.

| Table 1 Primary Intracellular Signaling Domains. In embodiments the domain comprises an ITAM. |
|---|
| FcR gamma (FCER1G) |
| FcR beta (FCER1B) |
| CD3 gamma |
| CD3 delta |
| CD3 epsilon |
| CD3 zeta (TCR zeta) |
| CD79a |
| CD79b |
| DAP10 |
| DAP12 |
| CD32 (Fc gamma RIIa) |

A primary intracellular signaling domain comprises a functional fragment, or analog, of a primary stimulatory molecule (e.g., CD3 zeta - GenBank accno. BAG36664.1). It can comprise the entire intracellular region or a fragment of the intracellular region which is sufficient for generation of an intracellular signal when an antigen binding domain to which it is fused, or coupled by a dimerization switch, binds cognate antigen. In embodiments the primary intracellular signaling domain has at least 70, 75. 80. 85, 90, 95, 98, or 99 % sequence identity with a naturally occurring primary stimulatory molecule, e.g., a human CD3 zeta (GenBank Acc No. AAY57330.1), or other mammalian, e.g., a nonhuman species, e.g., rodent, monkey, ape or murine intracellular primary stimulatory molecule. In embodiments the primary intracellular signaling domain has at least 70, 75. 80. 85, 90, 95, 98, or 99 % sequence identity with SEQ ID NO: 139. In embodiments, the primary stimulatory molecule may comprise 1, 2, 3, 4, 5, 6, 7, 8, 9, or 10 mutations, e.g., in SEQ ID NO: 139.

In embodiments the primary intracellular signaling domain, has at least 70, 75, 80, 85, 90, 95, 96, 97, 98, or 99% identity with, or differs by no more than 30, 25, 20, 15, 10, 5, 4, 3, 2, or 1 amino acid residues from the corresponding residues of a naturally occurring human primary stimulatory molecule, e.g., a naturally occurring human primary stimulatory molecule disclosed herein, e.g., SEQ ID NO: 139.

### COSTIMULATORY SIGNALING DOMAIN

In an embodiment, a costimulatory signaling domain produces an intracellular signal when an extracellular domain, e.g., an antigen binding domain to which it is fused, or coupled by a dimerization switch, binds cognate ligand. It is derived from a costimulatory molecule. It comprises sufficient costimulatory molecule sequence to produce an intracellular signal, e.g., when an extracellular domain, e.g., an antigen binding domain, to which it is fused, or coupled by a dimerization switch, binds cognate ligand.

Costimulatory molecules are cell surface molecules, other than antigen receptors or their counter ligands that promote an immune effector response. In some cases they are required for an efficient or enhanced immune response. Typically, a costimulatory molecule generates an intracellular signal that is dependent on binding to a cognate ligand that is, in instances, other than an antigen, e.g., the antigen recognized by an antigen binding domain of a RNKR-CARX, RCAR/NKR-CARX, or RCARX cell, e.g., RNKR-CART, RCAR/NKR-CART, or RCART cell or RNKR-CARN, RCAR/NKR-CARN, or RCARN. Typically, signaling from a primary stimulatory molecule and a costimulatory molecule contribute to an immune effector response, and in some cases both are required for efficient or enhanced generation of an immune effector response.

A costimulatory signaling domain comprises a functional fragment, or analog, of a costimulatory molecule (e.g., 4-1BB). It can comprise the entire intracellular region or a fragment of the intracellular region which is sufficient for generation of an intracellular signal, e.g., when an antigen binding domain to which it is fused, or coupled by a dimerization switch, binds cognate antigen. In embodiments, the costimulatory signaling domain has at least 70, 75, 80, 85, 90, 95,98, or 99 % sequence identity with a naturally occurring costimulatory molecule, e.g., a human, or other mammalian, e.g., a nonhuman species, e.g., rodent, monkey, ape or murine intracellular costimulatory molecule. In embodiments the costimulatory signaling domain has at least 70, 75, 80, 85, 90, 95, 98, or 99 % sequence identity with SEQ ID NO: 138.

Exemplary costimulatory signaling domains (intracellular signaling domains) are provided in Table 2.

| Table 2: Costimulatory Signaling Domains for RCARX, RNKR-CARX, or RCAR/NKR-CARX (identified by the Costimulatory Molecules from which they are derived) |
|---|
| CD27 |
| CD28, |
| 4-1BB (CD137) |
| OX40 |
| CD30 |
| CD40 |
| ICOS (CD278) |
| ICAM-1 |
| LFA-1 (CD1 1 a/CD18) |
| CD2 |
| CD7 |
| LIGHT |
| NKG2C |
| B7-H3 |
| a ligand that specifically binds with CD83 |
| CDS |
| GITR |
| BAFFR |
| HVEM (LIGHTR) |
| SLAMf7 |
| NKP80 (KLRF1) |
| CD160 (BY55) |
| CD19 |
| CD4 |
| CD8 alpha |
| CD8 beta |
| IL2R beta |
| IL2R gamma |
| IL7R alpha |
| ITGA4 |
| VLA1 |
| CD49a |
| ITGA4 |
| IA4 |
| CD49D |
| ITGA6 |
| VLA-6 |
| C49f |
| ITGAD |
| CD11d |
| ITGAE |
| CD103 |
| ITGAL |
| CD11a |
| LFA-1 |
| ITGAM |
| CD11b |
| ITGAX |
| CD11c |
| ITGB1 |
| CD29 |
| ITGB2 |
| CD18 |
| ITGB7 |
| TNFR2 |
| TRANCE/RANKL |
| DNAM1 (CD226) |
| SLAMF4 (C244, 2B4) |
| CD84 |
| CD96 (Tactile) |
| CEACAM1 |
| CRTAM |
| Ly9 (CD229) |
| PSGL1 |
| CD100 (SEMA4D) |
| CD69 |
| SLAMF6 (NTB-A, Ly108) |
| SLAM (SLAMF1, CD150, IPO-3) |
| BLAME (SLAMF8) |
| SELPLG (CD162) |
| LTBR |
| LAT |
| GADS |
| PAG/Cbp |
| SLP-76 |
| NKp44 |
| NKp30 |
| NKp46 |

In embodiments the costimulatory signaling domain, has at least 70, 75, 80, 85, 90, 95, 96, 97, 98, or 99% identity with, or differs by no more than 30, 25, 20, 15, 10, 5, 4, 3, 2, or 1 amino acid residues from the corresponding residues of a naturally occurring human primary stimulatory molecule, e.g., a naturally occurring human costimulatory molecule disclosed herein.

### Intracellular Signaling domains or adaptor molecules, e.g., DAP12

Some RNKR-CARs or NKR-CARs interact with other molecules, e.g., molecules comprising an intracellular signaling domain, e.g., an ITAM. In an embodiment a intracellular signaling domain is DAP12.

DAP12 is so named because of its structural features, and presumed function. Certain cell surface receptors lack intrinsic functionality, which hypothetically may interact with another protein partner, suggested to be a 12 kD protein. The mechanism of the signaling may involve an ITAM signal.

The DAP12 was identified from sequence databases based upon a hypothesized relationship to CD3 (see Olcese, et al. (1997) J. Immunol. 158:5083-5086), the presence of an ITAM sequence (see Thomas (1995) J. Exp. Med. 181:1953-1956), certain size predictions (see Olcese; and Takase, et al. (1997) J. Immunol. 159:741-747, and other features. In particular, the transmembrane domain was hypothesized to contain a charged residue, which would allow a salt bridge with the corresponding transmembrane segments of its presumed receptor partners, KIR CD94 protein, and possibly other similar proteins. See Daeron, et al. (1995) Immunity 3:635-646.

In fact, many of the known KIR, MIR, ILT, and CD94/NKG2 receptor molecules may actually function with an accessory protein which is part of the functional receptor complex. See Olcese, et al. (1997) J. Immunol. 158:5083-5086; and Takase, et al. (1997) J. Immunol. 159:741-747.

A DAP 12 domain, as that term is used herein, refers to a polypeptide domain having structural and functional properties of a cytoplasmic domain of a DAP 12, and will typically include an ITAM domain. In an embodiment a DAP 12 domain of a RKIR-CAR or KIR-CAR has at least 70, 80, 85, 90, 95, or 99% homology with a reference sequence, e.g., a naturally occurring DAP 12 or a DAP 12 described herein. In embodiments the DAP 12 domain of a RKIR-CAR or KIR-CAR differs at no more than 15, 10, 5, 2, or 1% of its residues from a reference sequence, e.g., a naturally occurring DAP 12 or a DAP 12 described herein. In embodiments the DAP 12 domain of a RKIR-CAR or KIR-CAR differs at no more than 5, 4, 3, 2 or 1 residue from a reference sequence, e.g., a naturally occurring DAP 12 or a DAP 12 described herein. In embodiments the DAP 12 domain of a RKIR-CAR or KIR-CAR does not differ from, or shares 100% homology with, a reference sequence, e.g., a naturally occurring DAP 12 or a DAP 12 described herein.

The DAP10 was identified partly by its homology to the DAP12, and other features. In particular, in contrast to the DAP12, which exhibits an ITAM activation motif, the DAP10 exhibits an ITIM inhibitory motif. The MDL-1 was identified by its functional association with DAP12.

The functional interaction between, e.g., DAP12 or DAP10, and its accessory receptor may allow use of the structural combination in receptors which normally are not found in a truncated receptor form. Thus, the mechanism of signaling through such accessory proteins as the DAP12 and DAP10 allow for interesting engineering of other KIR-like receptor complexes, e.g., with the KIR, MIR, ILT, and CD94 NKG2 type receptors. Truncated forms of intact receptors may be constructed which interact with a DAP12 or DAP10 to form a functional signaling complex.

The primate nucleotide sequence of DAP12 corresponds to the DAP12 sequence in SEQ ID NO: 158; the amino acid sequence corresponds to the DAP12 sequence in SEQ ID NO: 159. The signal sequence appears to run from met(-26) to gln(-1) or ala1; the mature protein should run from about ala1 (or gln2), the extracellular domain from about ala1 to pro14; the extracellular domain contains two cysteines at 7 and 9, which likely allow disulfide linkages to additional homotypic or heterotypic accessory proteins; the transmembrane region runs from about gly15 or val16 to about gly39; and an ITAM motif from tyr65 to leu79 (YxxL-6/8x-YxxL). The LVA03A EST was identified and used to extract other overlapping sequences. See also Genbank Human ESTs that are part of human DAP12; some, but not all, inclusive Genbank Accession # AA481924; H39980; W60940; N41026; R49793; W60864; W92376; H12338; T52100; AA480109; H12392; W74783; and T55959.

### AUXILIARY ANTIGEN BINDING MEMBER

An auxiliary antigen binding member can be included in a RCAR or RNKR-CAR. In embodiments, its inclusion can increase the safety and efficacy of the RNKR-CARX or RCARX cell, e.g., by increasing specificity by the binding to an additional, e.g., second target cell antigen. In embodiments, binding of both the antigen binding member, and the auxiliary antigen binding member can give greater specificity than seen with either alone. In embodiments the RCAR or RNKR-CAR will include two, three, four, five, six, seven, eight, nine, or ten, auxiliary antigen binding members, all of which bind different antigens.

In an embodiment the auxiliary antigen binding domain does not comprise a switch domain that can form a dimerization switch with a switch domain on the antigen binding member or the intracellular signaling member. In embodiments the auxiliary antigen binding domain does not comprise an intracellular signaling domain. In an embodiment, the antigen binding domain is directed against a mesothelin receptor and the auxiliary antigen binding domain is directed against a folate receptor. In an embodiment, the antigen binding domain is directed against a folate receptor and the auxiliary antigen binding domain is directed against a mesothelin receptor.

### INHIBITORY MOLECULES: INHIBITION

In one instance, the subject can be administered an agent which enhances the activity or fitness of a CAR-expressing cell, e.g., a RNKR-CAR-expressing cell, RCAR-expressing cell, NKR-expressing cell, or RCAR/NKR-CAR-expressing cell. For example, in one instance, the agent can be an agent which inhibits a molecule that modulates or regulates, e.g., inhibits, T cell function. In some instances, the molecule that modulates or regulates T cell function is an inhibitory molecule. Inhibitory molecules, e.g., PD1, can, in some instances, decrease the ability of a RNKR-CARX, RCAR/NKR-CARX, or RCARX cell to mount an immune effector response. Examples of inhibitory molecules are provided in Table 3. Inhibition of an inhibitory molecule that modulates or regulates, e.g., inhibits, T cell function, e.g., by inhibition at the DNA, RNA or protein level, can optimize performance of a RNKR-CARX, RCAR/NKR-CARX, or RCARX cell. In instances an agent, e.g., an inhibitory nucleic acid, e.g., a dsRNA, e.g., an siRNA or shRNA, or e.g., an inhibitory protein or system, e.g., a clustered regularly interspaced short palindromic repeats (CRISPR), a transcription-activator like effector nuclease (TALEN), or a zinc finger endonuclease (ZFN), e.g., as described herein, can be used to inhibit expression of a molecule that modulates or regulates, e.g., inhibits, cell function in the RNKR-CARX, RCAR/NKR-CARX, or RCARX cell. In an instance, the inhibitor is an shRNA. In an instance, the inhibitory molecule is inhibited within a RNKR-CARX, RCAR/NKR-CARX, or RCARX cell. In these instances, a dsRNA molecule that inhibits expression of the inhibitory molecule is linked to the nucleic acid that encodes a component, e.g., all of the components, of the RNKR-CAR, NKR-CAR, or RCAR. Exemplary inhibitory molecules, useful e.g., as shRNA targets, are provided in Table 3.

| Table 3: Inhibitory molecules |
|---|
| CD160 |
| 2B4 |
| PD1 |
| TIM3 |
| LAG3 |
| TIGIT |
| CTLA-4 |
| BTLA |
| LAIR1 |
| PD-L1 |
| VISTA |
| CEACAM (e.g., CEACAM-1, CEACAM-3 and/orCEACAM-5) |
| TGFR beta |

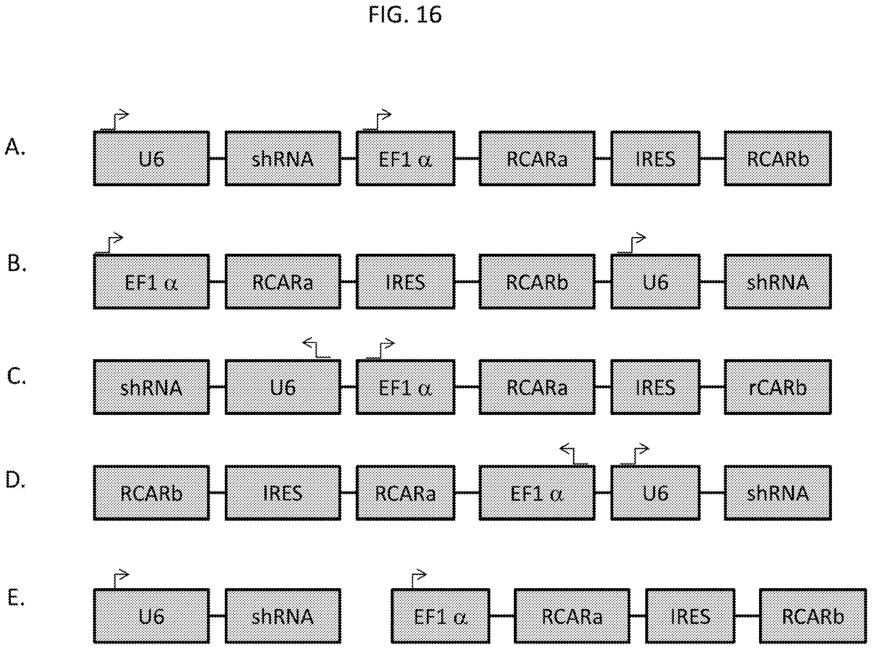

In an instance, a nucleic acid molecule that encodes a dsRNA molecule that inhibits expression of the molecule that modulates or regulates, e.g., inhibits, T-cell function is operably linked to a promoter, e.g., a H1- or a U6-derived promoter such that the dsRNA molecule that inhibits expression of the molecule that modulates or regulates, e.g., inhibits, T-cell function is expressed, e.g., is expressed within a RCAR-expressing, RNKR-CAR-expressing, or NKR-CAR-expressing cell. See e.g., Tiscornia G., "Development of Lentiviral Vectors Expressing siRNA", Chapter 3, in Gene Transfer: Delivery and Expression of DNA and RNA (eds. Friedmann and Rossi). Cold Spring Harbor Laboratory Press, Cold Spring Harbor, NY, USA, 2007; Brummelkamp TR, et al. (2002) Science 296: 550-553; Miyagishi M, et al. (2002) Nat. Biotechnol. 19: 497-500. In an instance, the nucleic acid molecule that encodes a dsRNA molecule that inhibits expression of the molecule that modulates or regulates, e.g., inhibits, T-cell function is present on the same vector, e.g., a lentiviral vector, that comprises a nucleic acid molecule that encodes a component, e.g., all of the components, of the RNKR-CAR, NKR-CAR, or RCAR. In such an instance, the nucleic acid molecule that encodes a dsRNA molecule that inhibits expression of the molecule that modulates or regulates, e.g., inhibits, T-cell function is located on the vector, e.g., the lentiviral vector, 5'- or 3'- to the nucleic acid that encodes a component, e.g., all of the components, of the RNKR-CAR, NKR-CAR, or RCAR. The nucleic acid molecule that encodes a dsRNA molecule that inhibits expression of the molecule that modulates or regulates, e.g., inhibits, T-cell function can be transcribed in the same or different direction as the nucleic acid that encodes a component, e.g., all of the components, of the RNKR-CAR, NKR-CAR, or RCAR. In an instance the nucleic acid molecule that encodes a dsRNA molecule that inhibits expression of the molecule that modulates or regulates, e.g., inhibits, T-cell function is present on a vector other than the vector that comprises a nucleic acid molecule that encodes a component, e.g., all of the components, of the RNKR-CAR, NKR-CAR, or RCAR. In an instance, the nucleic acid molecule that encodes a dsRNA molecule that inhibits expression of the molecule that modulates or regulates, e.g., inhibits, T-cell function it transiently expressed within a RNKR-CAR-expressing, NKR-expressing, and/or RCAR-expressing cell. In an instance, the nucleic acid molecule that encodes a dsRNA molecule that inhibits expression of the molecule that modulates or regulates, e.g., inhibits, T-cell function is stably integrated into the genome of a RNKR-CAR-expressing, NKR-expressing, and/or RCAR-expressing cell. FIG. 16 depicts examples of vectors for expressing a component, e.g., all of the components, of the RNKR-CAR, NKR-CAR, or RCAR with a dsRNA molecule that inhibits expression of the molecule that modulates or regulates, e.g., inhibits, T-cell function.

Examples of dsRNA molecules useful for inhibiting expression of a molecule that modulates or regulates, e.g., inhibits, T-cell function, wherein the molecule that modulates or regulates, e.g., inhibits, T-cell function is PD-1 are provided in Example 10 and Tables 18 and 19.

### REDIRECTED SWITCHABLE INHIBITORY RECEPTORS:

### INHIBITORY EXTRACELLULAR DOMAINS

Extracellular domains of inhibitory receptors can be coupled, e.g., by dimerization switches to intracellular signaling domains that promote an immune effector response. Thus, engagement with a counterligand of the coinhibitory molecule is redirected into an optimization of immune effector response. Extracellular domains of inhibitory receptors can be used in actRNKR-CARs or RCARs.

In one instance, the extracellular domain (ECD) of an inhibitory molecule, e.g., an inhibitory molecule described herein such as, e.g., Programmed Death 1 (PD1), can be fused to a transmembrane domain and intracellular signaling domain described herein, e.g., an intracellular signaling domain comprising a costimulatory signaling domain such as, e.g., 41BB OX40, Cd28, CD27, and/or a primary signaling domain, e.g., of CD3 zeta. In an instance, an actRNKR-CAR described herein comprises an ECD of an inhibitory molecule and an element (e.g., domain) of an activating NKR, e.g., a TM domain and/or a NKR cytoplasmic domain from an activating NKR. In one instance, the inhibitory molecule actRNKR-CAR or RCAR, e.g., PD1 actRNKR-CAR or PD1 RCAR, can be used alone. In one instance, the inhibitory molecule CAR, e.g., inhibitory molecule actRNKR-CAR (e.g., PD1 actRNKR-CAR) or inhibitory molecule RCAR (e.g., PD1 RCAR), can be used in combination with another CAR, e.g., CD19CAR (e.g., a CD19RCAR or a regulatable CD19CAR). In one instance, the PD1 RCAR or PD1 actRNKR-CAR (or PD1 CAR) improves the persistence of the T cell. Examples of inhibitory molecules include PD1, PD-L1, CTLA4, TIM3, CEACAM (e.g., CEACAM-1, CEACAM-3 and/orCEACAM-5), LAG3, VISTA, BTLA, TIGIT, LAIR1, CD160, 2B4 and TGFR beta. In one instance, the inhibitory molecule actRNKR-CAR or inhibitory molecule RCAR comprises a first polypeptide, e.g., of an inhibitory molecule such as PD1, LAG3, CTLA4, CD160, BTLA, LAIR1, TIM3, CEACAM (e.g., CEACAM-1, CEACAM-3 and/or CEACAM-5), 2B4 and TIGIT, or a fragment of any of these (e.g., at least a portion of an extracellular domain of any of these), and a second polypeptide which is an intracellular signaling member described herein (e.g., comprising a costimulatory domain (e.g., 41BB, CD27 or CD28, e.g., as described herein) and/or a primary signaling domain (e.g., a CD3 zeta signaling domain described herein).

In one instance, the PD1 RNKR-CAR or PD1 RCAR improves the persistence of the RNKR-CAR or RCAR-expressing cell. In one instance, the PD1 RNKR-CAR or PD1 RCAR comprises the extracellular domain of PD1 indicated as underlined in SEQ ID NO: 147. In one instance, the PD1 RNKR-CAR or PD1 RCAR comprises, the amino acid sequence of SEQ ID NO:147.
Malpvtalllplalllhaarppgwfldspdrpwnpptfspallvvtegdnatftcsfsntsesfvlnwyrmspsnqtdklaafpedrsqpgqdcrfrvtqlpngrdfhmsvvrarrndsgtylcgaislapkaqikeslraelrvterraevptahpspsprpagqfqtlvtttpaprpptpaptiasqplslrpeacrpaaggavhtrgldfacdiyiwaplagtcgvlllslvitlyckrgrkkllyifkqpfmrpvqttqeedgcscrfpeeeeggcelrvkfsrsadapaykqgqnqlynelnlgrreeydvldkrrgrdpemggkprrknpqeglynelqkdkmaeayseigmkgerrrgkghdglyqglstatkdtydalhmqalppr (SEQ ID NO: 147).

In one instance, the PD1 RNKR-CAR or PD1 RCAR comprises the amino acid sequence provided below. pgwfldspdrpwnpptfspallvvtegdnatftcsfsntsesfvlnwyrmspsnqtdklaafpedrsqpgqdcrfrvtqlpngrdfh msvvrarrndsgtylcgaislapkaqikeslraelrvterraevptahpspsprpagqfqtlvttpaprpptpaptiasqplslrpeacr paaggavhtrgldfacdiyiwaplagtcgvlllslvitlyckrgrkkllyifkqpfmrpvqttqeedgcscrfpeeeeggcelrvkfsrs adapaykqgqnqlynelnlgrreeydvldkrrgrdpemggkprrknpqeglynelqkdkmaeayseigmkgerrrgkghdgly qglstatkdtydalhmqalppr (SEQ ID NO: 148)

In one instance, the PD1 RNKR-CAR or PD1 RCAR, e.g., the PD1 RNKR-CAR or PD1 RCAR described herein, is encoded by a nucleic acid sequence shown below, or at least the comprises the nucleic acid sequence encoding the extracellular domain of PD 1 (shown in underline below).

Exemplary inhibitory extracellular domains are provided in Table 4.

| Table 4: Extracellular counter ligand binding domains from coinhibitory molecules (identified by the Coinibitory Molecules from which they are derived) |
|---|
| B7-H1 |
| B7-1 |
| CD160 |
| P1H |
| 2B4 |
| PD1 |
| TIM3 |
| CEACAM (e.g., CEACAM-1, CEACAM-3, and/or CEACAM-5) |
| LAG3 |
| TIGIT |
| CTLA-4 |
| BTLA |
| LAIR1 |
| TGF-beta receptor |

In instances, the inhibitory extracellular domain, has at least 70, 75, 80, 85, 90, 95, 96, 97, 98, or 99% identity with, or differs by no more than 30, 25,20, 15,10, 5, 4, 3, 2, or 1 amino acid residues from the corresponding residues of a naturally occurring human inhibitory molecule, e.g., a naturally occurring human primary stimulatory molecule disclosed herein.

### COSTIMULATORY MOLECULE LIGAND BINDING DOMAINS

Extracellular ligand binding domains of costimulatory molecules, referred to as a Costimulatory ECD domain, can be coupled, e.g., by dimerization switches, to intracellular signaling domains that promote an immune effector response. Thus, engagement with a counter ligand of the costimulatory molecule results in optimization of immune effector response. Costimulatory ECD domains can be used in actRNKR-CARs or RCARs. In an instance, an actRNKR-CAR described herein comprises an a costimulatory ECD domain, e.g., and further compries an element (e.g., domain) of an activating NKR, e.g., a TM domain and/or a NKR cytoplasmic domain from an activating NKR.

Exemplary Costimulatory ECD domains are provided in the Table 5.

| Table 5: Costimulatory ECD domains from costimulatory molecules (identified by the Costimulatory Molecules from which they are derived) |
|---|
| ICOS |
| CD28 |
| CD27 |
| HVEM |
| LIGHT |
| CD40L |
| 4-1BB |
| OX40 |
| DR3 |
| GITR |
| CD30 |
| TIM1 |
| SLAM |
| CD2 |
| CD226 |

In instances, the Costimulatory ECD domain, has at least 70, 75,80, 85,90, 95,96, 97, 98, or 99% identity with, or differs by no more than 30, 25,20, 15,10, 5, 4, 3, 2, or 1 amino acid residues from the corresponding residues of a naturally occurring human inhibitory molecule, e.g., a naturally occurring human costimulatory molecule disclosed herein.

### TRANSMEMBRANE DOMAIN

In instances, a RNKR-CAR, NKR-CAR, or RCAR comprises a transmembrane domain that is fused to an extracellular sequence, e.g., an extracellular recognition element, which can comprise an antigen binding domain, an inhibitory counter ligand binding domain, or costimulatory ECD domain. In embodiments, a RNKR-CAR, NKR-CAR, or RCAR comprises a transmembrane domain that is fused to an intracellular sequence, e.g. primary intracellular signaling domain, costimulatory signaling domain, or dimerization switch. In an embodiment, the transmembrane domain is one that naturally is associated with one of the domains in the RNKR-CAR, NKR-CAR, or RCAR. In an embodiment, the transmembrane domain is one that is not naturally associated with one of the domains in the RNKR-CAR, NKR-CAR, or RCAR.

In embodiments, the transmembrane domain is one which minimizes interactions with other elements, e.g., other transmembrane domains. In some instances, the transmembrane domain minimizes binding of such domains to the transmembrane domains of the same or different surface membrane proteins, e.g., to minimize interactions with other members of the receptor complex. Suitable examples can be derived by selection or modification of amino acid substitution of a known transmembrane domain. In an embodiment, the transmembrane domain is capable of promoting homodimerization with another RNKR-CAR, NKR-CAR, or RCAR on the cell surface.

The transmembrane domain may comprise a naturally occurring, or a non-naturally occurring synthetic sequence. Where naturally occurring, the transmembrane domain may be derived from any membrane-bound or transmembrane protein. In an embodiment, the transmembrane region is capable of signaling, via a dimerization switch, to the intracellular domain(s) whenever the RNKR-CAR, NKR-CAR, or RCAR has bound to a target.

Transmembrane regions suitable for use in molecules described herein may be derived from any one or more of e.g., the alpha, beta or zeta chain of the T-cell receptor, CD28, CD3 epsilon, CD45, CD4, CD5, CD8, CD9, CD16, CD22, CD33, CD37, CD64, CD80, CD86, CD134, CD137, CD154. In some embodiments, a transmembrane domain may include at least the transmembrane region(s) of, e.g., KIRDS2, OX40, CD2, CD27, LFA-1 (CD11a, CD18), ICOS (CD278), 4-1BB (CD137), GITR, CD40, BAFFR, HVEM (LIGHTR), SLAMF7, NKp80 (KLRF1), CD160, CD19, IL2R beta, IL2R gamma, IL7Rα, ITGA1, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, TNFR2, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), SLAMF6 (NTB-A, Ly108), SLAM (SLAMF1, CD150, IPO-3), BLAME (SLAMF8), SELPLG (CD162), LTBR, PAG/Cbp. In an embodiment the transmembrane domain is derived from CD8. In an embodiment the transmembrane domain is derived from CD28. In one aspect, the transmembrane domain is a transmembrane domain from the sequence provided as SEQ ID NO: 137.

In an embodiment, the transmembrane domain comprises a NKR transmembrane domain. For example, the NKR transmembrane comprises a transmembrane selected from Table 24. In embodiments, the NKR transmembrane comprises a KIR, NCR, SLAMF receptor, FcR, Ly49 receptor transmembrane domain. In embodiments, the KIR transmembrane domain comprises an actKIR transmembrane domain, KIR2DS2 transmembrane domain. In embodiments, the NCR transmembrane domain comprises a NKp30, NKp44, or NKp46 transmembrane domain. In embodiments, the FcR transmembrane domain comprises a CD16 or CD64 transmembrane domain. In embodiments, the Ly49 transmembrane domain comprises a transmembrane domain selected independently from Ly49A-Ly49W.

In an embodiment, the transmembrane domain comprises a positively charged moiety, e.g., an amino acid residue comprising a positively charged moiety, e.g., a positively charged side chain.

In embodiments, the transmembrane domain can interact with, e.g., bind, an adapter or intracellular signaling molecule, e.g., an intracellular signaling molecule described herein, e.g,. in Table 1 or 2, e.g., a DAP12, FcRy or CD3 ζ cytoplasmic domain. In embodiments, the transmembrane domain interacts with an ITAM-containing cytoplasmic domain. For example, the transmembrane domain can interact with, e.g., bind the transmembrane domain of and adapter or intracellular signaling molecule described herein, e.g., DAP12.

In an embodiment, a sequence, e.g., a hinge or spacer sequence, can be disposed between a transmembrane domain and another sequence or domain to which it is fused. In embodiments, a variety of human hinges (aka "spacers") can be employed as well, e.g., including but not limited to the human Ig (immunoglobulin) hinge. Optionally, a short oligo- or polypeptide linker, between 2 and 10 amino acids in length may form the linkage between the transmembrane domain and another domain, e.g., a switch or intracellular signaling domain, of a RNKR-CAR, NKR-CAR, or RCAR. A glycine-serine doublet provides a particularly suitable linker. In one aspect, the hinge or spacer is the amino acid sequence provided as SEQ ID NO: 136. In one aspect, the hinge or spacer comprises a KIR2DS2 hinge.

In an embodiment, the transmembrane domain may be a non-naturally occurring sequence, in which case can comprise predominantly hydrophobic residues such as leucine and valine. In an embodiment, a triplet of phenylalanine, tryptophan and valine will be found at each end of a transmembrane domain.

### ICARs

RNKR-CARs or RCARs disclosed herein can include an inhibitory CAR (iCAR) member. For example, a cell comprises an antigen binding member and intracellular signaling member of a RCAR, and an iCAR. In an embodiment a cell comprises an antigen binding member and an intracellular signaling member of a RNKR-CAR, and an iCAR. In an embodiment a cell comprises an RCAR, an NKR-CAR and an iCAR. An iCAR member comprises: an antigen binding domain (or other extracelluar domain) that recognizes an antigen on a non-target, e.g., a noncancer, cell; a transmembrane domain; and, a domain from an inhibitory molecule, e.g., an intracellular domain from an inhibitory molecule, e.g., from PD-1, CTLA4, or from a protein listed in Table 12. In an embodiment, the iCAR member comprises a second inhibitory intracellular signaling domain, e.g., from PD-1, CTLA4, or from a protein listed in Table 12.

Upon engagement of the antigen binding domain (or other extracelluar domain) of the iCAR member with its target antigen (or counter-ligand), the iCAR contributes to inhibiting, e.g., reversibly inhibiting, or minimizing, activation of the cell comprising the iCAR. As such, inclusion of an iCAR member in a RNKR-CAR or RCAR, e.g., and RNKR-CARX or RCARX, cell, can limit damage to non-target, e.g., bystander, cells. While not wishing to be bound by theory, it is believed that an iCAR member, upon engagement with its antigen (or counter-ligand), limits one or more of cytokine secretion, cytotoxicity, and proliferation. In embodiments the effect is temporary, and upon subsequent engagement with a target cell the RNKR-CAR or RCAR, e.g., RNKR-CARX or RCARX, cell is activated and attacks the target cell.

A target antigen for an iCAR member can be an antigen that has an expression profile on target cells and non-target cells such that an acceptably high level of attack on target cells and an acceptably low level of attack on non-target cells is achieved. Not only choice of antigen, but iCAR affinity for its antigen (or counter-ligand), CAR (e.g., RNKR-CAR or RCAR) affinity for its antigen, level of expression of the iCAR, or levels of expression of the CAR (e.g., RNKR-CAR or RCAR) can be used to optimize the ratio of on-target/off-target response.

In an embodiment, the antigen is absent, or down-regulated on tumor cells. In an embodiment the antigen comprises an HLA molecule. In an embodiment the antigen comprises a cell suface tumor suppressor antigen. In an embodiment the antigen comprises PCML (or another antigen that is down-regulated in lymphomas, breast or prostate cancer), HYAL2, DCC, or SMAR1.

In an embodiment, the antigen comprises a protein, carbohydrate, lipid, or a post-translational modification of a cell surface moiety, e.g., a mucin-type O-glycan (a core 3 O-glycan).

In an embodiment, the antigen comprises a moiety that is down-regulated by tumor cells undergoing an epithelial to mesenchymal transition.

In an embodiment, the antigen comprises E-cadherin.

In an embodiment a domain from an inhibitory molecule, e.g., an intracellular signaling domain from PD-1 or CTLA4, produces an intracellular signal when an extracellular domain, e.g., an antigen binding domain, to which it is fused binds cognate antigen (or counter ligand). The inhibitory intracellular signaling domain is derived from an inhibitory molecule, e.g., it comprises intracellular sequence of an inhibitory molecule. It comprises sufficient inhibitory molecule sequence to produce an intracellular signal, e.g., when an antigen binding domain to which it is fused binds cognate antigen.

In an embodiment, the primary intracellular signaling domain comprises a signaling motif, e.g., an immunoreceptor tyrosine-based activation motif or ITIM.

A domain from an inhibitory molecule, comprises a functional fragment, or analog, of an inhibitory molecule intracellular domain. It can comprise the entire intracellular region or a fragment of the intracellular region which is sufficient for generation of an intracellular signal when an antigen binding domain to which it is fused, binds cognate antigen. In embodiments the inhibitory intracellular signaling domain has at least 70, 75, 80, 85, 90, 95, 98, or 99 % sequence identity with, or differs by no more than 30, 25, 20, 15, 10, 5, 4, 3, 2, or 1 amino acid residues from, the corresponding residues oa naturally occurring inhibitory molecule, e.g., a a molecule from Table 12.

Exemplary inhibitory molecules which can provide intracellular signaling domains are provided in Table 12.

| Table 12: Inhibitory molecules |
|---|
| B7-H1 |
| B7-1 |
| CD160 |
| P1H |
| 2B4 |
| PD1 |
| TIM3 |
| CEACAM (e.g., CEACAM-1, CEACAM-3, and/orCEACAM-5) |
| LAG3 |
| TIGIT |
| CTLA-4 |
| BTLA |
| LAIR1 |
| TGF-beta receptor |

Thus, in one, aspect, disclosed herein is, an RNKR-CAR or RCAR comprising an iCAR member. The iCAR member comprises:
an antigen binding domain (or other extracelluar domain) that recognizes an antigen on a non-target, e.g., a noncancer cell;
a transmembrane domain; and
a domain from an inhibitory molecule,, e.g., fromPD-1, CTLA4, or from a protein listed in Table 4.

In an embodiment, the iCAR member comprises a second inhibitory intracellular signaling domain, e.g., from PD-1, CTLA4, or from a protein listed in Table 12.

### VECTORS

The present disclosure also provides vectors which comprise a RCAR encoding sequence, RNKR-CAR encoding sequence, or NKR-CAR encoding sequence. Vectors derived from viruses, e.g., lentivirus, are suitable tools to achieve long-term gene transfer since they allow long-term, stable integration of a transgene and its propagation in daughter cells. Lentiviral vectors have the added advantage over vectors derived from retroviruses e.g., murine leukemia viruses, in that they can transduce non-proliferating cells, such as hepatocytes. They also have the added advantage of low immunogenicity.

In an embodiment, the expression of nucleic acids encoding RCARs, RNKR-CARs, or NKR-CARs is achieved by a nucleic acid encoding the RCAR, RNKR-CAR, or NKR-CAR polypeptide or portions or components thereof operably linked to a promoter, which is incorporated into an expression vector. The vectors can be suitable for replication and integration into eukaryotes. Typical vectors contain transcription and translation terminators, initiation sequences, and promoters useful for regulation of the expression of the desired nucleic acid sequence.

The nucleic acid can be cloned into a number of types of vectors. For example, the nucleic acid can be cloned into a vector including, but not limited to a plasmid, a phagemid, a phage derivative, an animal virus, and a cosmid. Vectors of particular interest include expression vectors, replication vectors, probe generation vectors, and sequencing vectors.

In an embodiment, the vector is a viral vector. Viral vector technology is known in the art and is described, for example, in Sambrook et al., 2012, Molecular Cloning: A Laboratory Manual, volumes 1-4, Cold Spring Harbor Press, NY), and in other virology and molecular biology manuals. In an embodiment, viruses, which are useful as vectors are retroviruses, adenoviruses, adeno- associated viruses, herpes viruses, and lentiviruses. In an embodiment the vector is a lentivirus vector. In general, a suitable vector contains an origin of replication functional in at least one organism, a promoter sequence, convenient restriction endonuclease sites, and one or more selectable markers, (e.g., WO 01/96584; WO 01/29058; and U.S. Pat. No. 6,326,193).

In an embodiment, a vector which expresses two or more genes, each gene is expressed separately under the control of a different promoter region, e.g., by using bi or tri-cistronic promoters. Expression of two or more genes from the same vector can be achieved by using either a multiple promoter plasmid e.g., bi or tri-cistronic promoters. Examples of multiple promoter containing lentivirus vectors are known in the literature. For example the vector pLENTI-bi-cistronic drives the expression of two genes using the PKG promoter and the mini CMV promoter in opposite directions (Applied Biological Material Inc., Richmond, BC, Canada). Similar the tri-cistronic vector pLENTI-tri-cistronic drives expression of three genes. In this configuration one gene can be induced by the mini-CMV promoter while the second and third gene can be induced by the PGK promoter separating the two genes with a T2A peptide cleavage site.

In another embodiment, bi- or tri-cistronic vectors may also be constructed making use of internal ribosomal entry sites (IRES) such as for example the element from the encephalomyocarditis virus (EMCV) for translation of two or more open reading frames (ORFs). Such vectors are designed to drive transcription of the bi- or tri-cistronic message under control of a strong human promoter regulatory region e.g. CMV or EF1alpha. IRESs are relatively short DNA sequences that can initiate RNA translation in a 5' cap-independent fashion. Whereas the first cistron is translated in a cap-dependent manner driven by a strong mammalian promoter, the subsequent ones utilize intercistronic regions of viral origin such as the internal ribosomal entry site of poliovirus or the cap-independent translation enhancer of encephalomyocarditis virus for enhanced translation. (N Chinnasamy et al. (2009), Production of Multicistronic HIV-1 Based Lentiviral Vectors; Methods Mol Biol 515: 1-14).

Additional promoter elements, e.g., enhancers, regulate the frequency of transcriptional initiation. Typically, these are located in the region 30-110 bp upstream of the start site, although a number of promoters have been shown to contain functional elements downstream of the start site as well. The spacing between promoter elements frequently is flexible, so that promoter function is preserved when elements are inverted or moved relative to one another. In the thymidine kinase (tk) promoter, the spacing between promoter elements can be increased to 50 bp apart before activity begins to decline. Depending on the promoter, it appears that individual elements can function either cooperatively or independently to activate transcription.

Another example of a promoter is the immediate early cytomegalovirus (CMV) promoter sequence. This promoter sequence is a strong constitutive promoter sequence capable of driving high levels of expression of any polynucleotide sequence operatively linked thereto. However, other constitutive promoter sequences may also be used, including, but not limited to the simian virus 40 (SV40) early promoter, mouse mammary tumor virus (MMTV), human immunodeficiency virus (HIV) long terminal repeat (LTR) promoter, MoMuLV promoter, an avian leukemia virus promoter, an Epstein-Barr virus immediate early promoter, a Rous sarcoma virus promoter, as well as human gene promoters such as, but not limited to, the actin promoter, the myosin promoter, the elongation factor-1α promoter (EF1α), the hemoglobin promoter, and the creatine kinase promoter. Further, embodiments are not limited to the use of constitutive promoters. Embodiments comprise inducible promoters. The use of an inducible promoter provides a molecular switch capable of turning on expression of the polynucleotide sequence which it is operatively linked when such expression is desired, or turning off the expression when expression is not desired. Examples of inducible promoters include, but are not limited to a metallothionine promoter, a glucocorticoid promoter, a progesterone promoter, and a tetracycline promoter.

Sequence encoding various elements of an RCAR or RNKR-CAR can be disposed on the same nucleic acid molecule, e.g., the same plasmid or vector, e.g., viral vector, e.g., lentiviral vector. E.g., both (i) sequence encoding an antigen binding member and (ii) sequence encoding an intracellular signaling member, can be present on the same nucleic acid, e.g., vector. Production of the corresponding proteins can be achieved, e.g., by the use of separate promoters, or by the use of a bicistronic transcription product (which can result in the production of two proteins by cleavage of a single translation product, the production of two proteins by ribosomal-skip during the translation from one transcription product, or by the translation of two separate protein products).

Accordingly, in an embodiment, (i) sequence encoding an antigen binding member and (ii) sequence encoding an intracellular signaling member, are present on a single nucleic acid molecule, are transcribed as a single transcription product, and are configured as follows:
a promoter, e.g., a promoter described herein, e.g., an EF1alpha promoter, is operably linked to (i), (ii), and to (iii) sequence encoding peptide, e.g., a cleavable peptide, e.g., a P2A or F2A sequence. Element (iii) is disposed between (i) and (ii). In an embodiment, (i), (ii), and (iii) are transcribed as a single RNA. In an embodiment, the order, on the nucleic acid, is (i)-(iii)-(ii). In an embodiment, the order, on the nucleic acid, is (ii)-(iii)-(i).

In an embodiment element (iii) comprises: a P2A or F2A sequence, or effective fragment thereof.

Amino acid and nucleic acid sequences for P2A and F2A are provided below:
P2A:
   Ggcagcggcgccaccaacttcagcctgctgaagcaggccggcgacgtggaggaaaaccctggcccc SEQ ID NO:143 GSGATNFSLLKQAGDVEENPGP SEQ ID NO:144
F2A:
   Gtgaagcagaccctgaacttcgacctgctgaaactggccggcgacgtggagagcaatcccggccct SEQ ID NO:145 VKQTLNFDLLKLAGDVESNPGP SEQ ID NO:146

In an embodiment (i) and (ii) form an RCAR or RNKR-CAR having an intracellular switch.

In an embodiment (i) and (ii) form an RCAR or RNKR-CAR having an extracellular switch.

In an embodiment (ii) comprises sequence that encode a 4-1BB domain and a CD3zeta domain.

In an embodiment (ii) comprises sequence that encode a DAP12 domain or a CD3zeta domain.

In an embodiment (ii) comprises sequence that encode a NKR cytoplasmic domain.

In an embodiment (ii) comprises sequence that encode a NKR cytoplasmic domain and a DAP12 or CD3zeta domain.

In an embodiment (i) comprises sequence that encode a costimulatory signaling domain, e.g., a 4-1BB domain.

In an embodiment (i) comprises sequence that encode a NKR cytoplasmic domain.

In an embodiment, (i) sequence encoding an antigen binding member and (ii) sequence encoding an intracellular signaling member, are present on a single nucleic acid molecule, are transcribed as a single transcription product, and are configured as follows:
a promoter, e.g., a promoter described herein, e.g., an EF1alpha promoter, is operably linked to (i), (ii), and to (iii) sequence encoding an IRES, e.g., an EMCV or EV71 IRES. In an embodiment (iii) is disposed between (i) and (ii). In an embodiment, (i), (ii), and (iii) are transcribed as a single RNA. In an embodiment, the order, on the nucleic acid, is (i)-(iii)-(ii). In an embodiment, the order, on the nucleic acid, is (ii)-(iii)-(i).

In an embodiment (i) and (ii) form an RCAR or RNKR-CAR having an intracellular switch.

In an embodiment (i) and (ii) form an RCAR or RNKR-CAR having an extracellular switch.

In an embodiment (ii) comprises sequence that encode a 4-1BB domain and a CD3zeta domain.

In an embodiment (ii) comprises sequence that encode a DAP12 domain or a CD3zeta domain.

In an embodiment (ii) comprises sequence that encode a NKR cytoplasmic domain.

In an embodiment (ii) comprises sequence that encode a NKR cytoplasmic domain and a DAP12 or CD3zeta domain.

In an embodiment (i) comprises sequence that encode a costimulatory signaling domain, e.g., a 4-1BB domain.

In an embodiment (i) comprises sequence that encode a NKR cytoplasmic domain.

In another embodiment, (i) sequence encoding an antigen binding member and (ii) sequence encoding an intracellular signaling member, are transcribed as separate transcription products, are present on a single nucleic acid molecule, and are configured as follows:
a promoter, e.g., a promoter described herein, e.g., an EF1alpha promoter, is operably linked to (i), and a second promoter, e.g., a promoter described herein, can be operable linked to (ii). In an embodiment (i) and (ii) are transcribed as separate mRNAs. In an embodiment, the order, on the nucleic acid, is first promoter-(i)-second promoter-(ii). In an embodiment, the order, on the nucleic acid, is first promoter-(ii)-second promoter-(i). In an embodiment the first promoter is a promoter described herein, e.g., an EF1alpha promoter. In an embodiment, the second promoter is a promoter described herein, e.g., a CMV or EF1 alpha promoter. In an embodiment the second promoter is a minimal promoter.

In an embodiment (i) and (ii) form an RCAR or RNKR-CAR having an intracellular switch.

In an embodiment (i) and (ii) form an RCAR or RNKR-CAR having an extracellular switch.

In an embodiment (ii) comprises sequence that encode a 4-1BB domain and a CD3zeta domain.

In an embodiment (ii) comprises sequence that encode a DAP12 domain or a CD3zeta domain.

In an embodiment (ii) comprises sequence that encode a NKR cytoplasmic domain.

In an embodiment (ii) comprises sequence that encode a NKR cytoplasmic domain and a DAP12 or CD3zeta domain.

In an embodiment (i) comprises sequence that encode a costimulatory signaling domain, e.g., a 4-1BB domain.

In an embodiment (i) comprises sequence that encode a NKR cytoplasmic domain.

Sequence encoding (i) an inhibitory extracellular domain member and (ii) sequence encoding an intracellular signaling member, can be present on the same nucleic acid, e.g., vector.

Accordingly, in an embodiment, (i) sequence encoding inhibitory extracellular domain member and (ii) sequence encoding an intracellular signaling member, are present on a single nucleic acid molecule, are transcribed as a single transcription product, and are configured as follows:
a promoter, e.g., a promoter described herein, e.g., an EF1alpha promoter, is operably linked to (i), (ii), and to (iii) sequence encoding peptide, e.g., a cleavable peptide, e.g., a P2A or F2A sequence. Element (iii) is disposed between (i) and (ii). In an embodiment, (i), (ii), and (iii) are transcribed as a single RNA. In an embodiment, the order, on the nucleic acid, is (i)-(iii)-(ii). In an embodiment, the order, on the nucleic acid, is (ii)-(iii)-(i).

In an embodiment element (iii) comprises: a P2A or P3A sequence, or effective fragment thereof.

In an instance, (i) sequence encoding an inhibitory extracellular member and (ii) sequence encoding an intracellular signaling member, are present on a single nucleic acid molecule, are transcribed as a single transcription product, and are configured as follows:
a promoter, e.g., a promoter described herein, e.g., an EFlalpha promoter, is operably linked to (i), (ii), and to (iii) sequence encoding an IRES, e.g., an EMCV or EV71 IRES. In an instance (iii) is disposed between (i) and (ii). In an instance, (i), (ii), and (iii) are transcribed as a single RNA. In an instance, the order, on the nucleic acid, is (i)-(iii)-(ii). In an instance, the order, on the nucleic acid, is (ii)-(iii)-(i).

In an instance (i) and (ii) form an RCAR or RNKR-CAR having an intracellular switch.

In an instance (i) and (ii) form an RCAR or RNKR-CAR having an extracellular switch.

In an instance (ii) comprises sequence that encode a 4-1BB domain and a CD3zeta domain.

In an instance (ii) comprises sequence that encode a DAP12 domain or a CD3zeta domain.

In an instance (ii) comprises sequence that encode a NKR cytoplasmic domain.

In an instance (ii) comprises sequence that encode a NKR cytoplasmic domain and a DAP12 or CD3zeta domain.

In an instance (i) comprises sequence that encode a costimulatory signaling domain, e.g., a 4-1BB domain.

In an instance (i) comprises sequence that encode a NKR cytoplasmic domain.

In another instance, (i) sequence encoding an inhibitory extracellular member and (ii) sequence encoding an intracellular signaling member, are transcribed as separate transcription products, are present on a single nucleic acid molecule, and are configured as follows:
a promoter, e.g., a promoter described herein, e.g., an EFlalpha promoter, is operably linked to (i), and a second promoter, e.g., a promoter described herein, can be operable linked to (ii). In an instance (i) and (ii) are transcribed as separate mRNAs. In an instance, the order, on the nucleic acid, is first promoter-(i)-second promoter-(ii). In an instance, the order, on the nucleic acid, is first promoter-(ii)-second promoter-(i).

In an instance the first promoter is a promoter described herein, e.g., an EFlalpha promoter. In an instance, the second promoter is a promoter described herein, e.g., a CMV or EF1 promoter. In an instance the second promoter is a minimal promoter.

In an instance (i) and (ii) form an RCAR or RNKR-CAR having an intracellular switch.

In an instance (i) and (ii) form an RCAR or RNKR-CAR having an extracellular switch.

In an instance (ii) comprises sequence that encode a 4-1BB domain and a CD3zeta domain.

In an instance (ii) comprises sequence that encode a DAP12 domain or a CD3zeta domain.

In an instance (ii) comprises sequence that encode a NKR cytoplasmic domain.

In an instance (ii) comprises sequence that encode a NKR cytoplasmic domain and a DAP12 or CD3zeta domain.

In an instance (i) comprises sequence that encode a costimulatory signaling domain, e.g., a 4-1BB domain.

In an instance (i) comprises sequence that encode a NKR cytoplasmic domain.

Sequence encoding (i) a costimulatory ECD member and (ii) sequence encoding an intracellular signaling member, can be present on the same nucleic acid, e.g., vector.

Accordingly, in an instance, (ia) sequence encoding costimulatory ECD member and (iai) sequence encoding an intracellular signaling member, are present on a single nucleic acid molecule, are transcribed as a single transcription product, and are configured as follows:
a promoter, e.g., a promoter described herein, e.g., an EFlalpha promoter, is operably linked to (i), (ii), and to (iii) sequence encoding peptide, e.g., a cleavable peptide, e.g., a P2A or F2A sequence. Element (iii) is disposed between (i) and (ii). In an instance, (i), (ii), and (iii) are transcribed as a single RNA. In an instance, the order, on the nucleic acid, is (i)-(iii)-(ii). In an instance, the order, on the nucleic acid, is (ii)-(iii)-(i).

In an instance element (iii) comprises: a P2A or P3A sequence, or effective fragment thereof.

In an instance, (ib) sequence encoding a costimulatory ECD member and (iib) sequence encoding an intracellular signaling member, are present on a single nucleic acid molecule, are transcribed as a single transcription product, and are configured as follows:
a promoter, e.g., a promoter described herein, e.g., an EFlalpha promoter, is operably linked to (ib), (iib), and to (iii) sequence encoding an IRES, e.g., an EMCV or EV71 IRES. In an instance (iii) is disposed between (ib) and (iib). In an instance, (ib), (iib), and (iii) are transcribed as a single RNA. In an instance, the order, on the nucleic acid, is (ib)-(iii)-(iib). In an instance, the order, on the nucleic acid, is (iib)-(iii)-(ib).

In an instance (ib) and (iib) form an RCAR or RNKR-CAR having an intracellular switch.

In an instance (ib) and (iib) form an RCAR or RNKR-CAR having an extracellular switch.

In an instance (iib) comprises sequence that encode a 4-1BB domain and a CD3zeta domain.

In an instance (iib) comprises sequence that encode a DAP12 domain or a CD3zeta domain.

In an instance (iib) comprises sequence that encode a NKR cytoplasmic domain.

In an instance (iib) comprises sequence that encode a NKR cytoplasmic domain and a DAP12 or CD3zeta domain.

In an instance (ib) comprises sequence that encode a costimulatory signaling domain, e.g., a 4-1BB domain.

In an instance (ib) comprises sequence that encode a NKR cytoplasmic domain.

In another instance, (ib) sequence encoding an a costimulatory ECD member and (iib) sequence encoding an intracellular signaling member, are transcribed as separate transcription products, are present on a single nucleic acid molecule, and are configured as follows:
a promoter, e.g., a promoter described herein, e.g., an EFlalpha promoter, is operably linked to (ib), and a second promoter, e.g., a promoter described herein, can be operable linked to (iib). In an instance (ib) and (iib) are transcribed as separate mRNAs. In an instance, the order, on the nucleic acid, is first promoter-(ib)-second promoter-(iib). In an instance, the order, on the nucleic acid, is first promoter-(iib)-second promoter-(ib). In an instance the first promoter is a promoter described herein, e.g., an EFlalpha promoter. In an instance, the second promoter is a promoter described herein, e.g., a CMV or EF1 promoter. In an instance the second promoter is a minimal promoter.

In an instance (ib) and (iib) form an RCAR or RNKR-CAR having an intracellular switch.

In an instance (ib) and (iib) form an RCAR or RNKR-CAR having an extracellular switch.

In an instance (iib) comprises sequence that encode a 4-1BB domain and a CD3zeta domain.

In an instance (iib) comprises sequence that encode a DAP12 domain or a CD3zeta domain.

In an instance (iib) comprises sequence that encode a NKR cytoplasmic domain.

In an instance (iib) comprises sequence that encode a NKR cytoplasmic domain and a DAP12 or CD3zeta domain.

In an instance (ib) comprises sequence that encode a costimulatory signaling domain, e.g., a 4-1BB domain.

In an instance (ib) comprises sequence that encode a NKR cytoplasmic domain.

In some embodiments, a nucleic acid encoding an RNKR-CAR comprises:
a sequence encoding (a) an antigen binding member and (b) an intracellular signaling member are disposed on a single nucleic acid molecule.

In other embodiment, a nucleic acid encoding a RNKR-CAR comprises a sequence encoding (a) an antigen binding member that is disposed on a first nucleic acid molecule, and a sequence encoding (b) an intracellular signaling member that is disposed on a second nucleic acid molecule.

In some embodiments, a nucleic acid encoding a RNKR-CAR further comprises a sequence encoding (c) an adaptor molecule or intracellular signaling molecule, e.g., DAP12 or Fcgamma R. For example, a sequence encoding (a), (b) and (c), is provided on a single nucleic acid molecule. In another example, a sequence encoding two of (a), (b), and (c), is provided on a first nucleic acid molecule and sequence encoding the other is provided on a second nucleic acid molecule. In another example, a sequence encoding (a) is provided on a first nucleic acid molecule, sequence encoding (b) is provided on a second nucleic acid molecule, and sequence encoding (c) is provided on a third nucleic acid molecule.

In some embodiments, a nucleic acid encoding a RNKR-CAR also comprises a sequence encoding a second CAR, e.g., a CAR described herein, e.g., a standard CAR, RCAR, RNKR-CAR, or NKR-CAR.

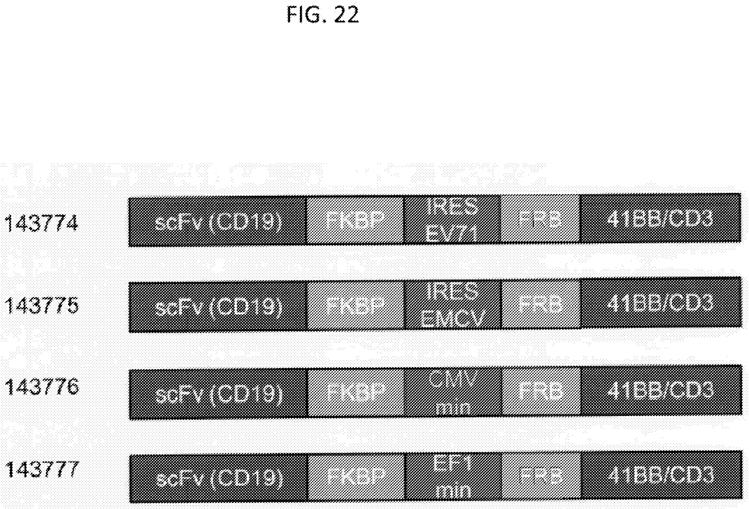

Embodiments of single molecule constructs include those depicted in Fig. 22.

In some aspects, a nucleic acid described herein, e.g., for introducing into a cell to produce a RCAR/NKR-CAR cell, comprises (i) a sequence encoding a RCAR and (ii) a sequence encoding a NKR-CAR.

Exemplary constructs comprising a nucleic acid encoding a RCAR are described herein. In some embodiments, the nucleic acid further comprises a sequence that encodes an intracellular signaling domain, e.g., adaptor molecule, that interacts with the NKR-CAR. For example, the sequence encoding the intracellular signaling domain is disposed on the same nucleic acid molecule, e.g., same vector, as the sequence encoding the NKR-CAR. In other examples, the sequence encoding the intracellular signaling domain is disposed on a separate nucleic acid molecule, e.g., separate vector, from the sequence encoding the NKR-CAR.

In some embodiments, the sequence(s) encoding the RCAR and the sequence encoding the NKR-CAR are disposed on the same nucleic acid molecule, e.g., same vector. For example, the sequence(s) encoding the RCAR and the sequence encoding the NKR-CAR are separated by a sequence encoding a cleavable peptide sequence described herein or by a sequence encoding an IRES described herein.

In embodiments, a promoter described herein, e.g., an EF1alpha promoter, is operably linked to a sequence encoding the RCAR or the sequence encoding the NKR-CAR.

In an embodiment a promoter that is capable of expressing RCAR, NKR-CAR or RNKR-CAR transgene is a mammalian T cell is the EF1alpha promoter (EF1α). The native EF1α promoter drives expression of the alpha subunit of the elongation factor-1 complex, which is responsible for the enzymatic delivery of aminoacyl tRNAs to the ribosome. The EF1α promoter has been used in mammalian expression plasmids and has been shown to be effective in driving RCAR, NKR-CAR, or RNKR-CAR expression from transgenes cloned into a lentiviral vector. See, e.g., Milone et al., Mol. Ther. 17(8): 1453-1464 (2009). In an embodiment, the EF1α promoter comprises the sequence provided as SEQ ID NO: 140.

In order to assess the expression of a RCAR, NKR-CAR, or RNKR-CAR polypeptide or portions thereof, the vector to be introduced into a cell can also contain either a selectable marker gene or a reporter gene or both to facilitate identification and selection of expressing cells from the population of cells sought to be transfected or infected through viral vectors. In embodiments, the selectable marker may be carried on a separate piece of DNA and used in a co- transfection procedure. Useful selectable markers include, for example, antibiotic-resistance genes, such as neo and the like.

Reporter genes are used for identifying potentially transfected cells and for evaluating the functionality of regulatory sequences. In general, a reporter gene is a gene that is not present in or expressed by the recipient organism or tissue and that encodes a polypeptide whose expression is manifested by some easily detectable property, e.g., enzymatic activity. Expression of the reporter gene is assayed at a suitable time after the DNA has been introduced into the recipient cells. Suitable reporter genes may include genes encoding luciferase, beta-galactosidase, chloramphenicol acetyl transferase, secreted alkaline phosphatase, or the green fluorescent protein gene (e.g., Ui-Tei et al., 2000 FEBS Letters 479: 79-82). Suitable expression systems are known and may be prepared using known techniques or obtained commercially. In general, the construct with the minimal 5' flanking region showing the highest level of expression of reporter gene is identified as the promoter. Such promoter regions may be linked to a reporter gene and used to evaluate agents for the ability to modulate promoter- driven transcription.

Methods of introducing into and expressing genes in a cell are known in the art. In the context of an expression vector, the vector can be readily introduced into a host cell, e.g., mammalian, bacterial, yeast, or insect cell by any method in the art. For example, the expression vector can be transferred into a host cell by physical, chemical, or biological means.

Physical methods for introducing a polynucleotide into a host cell include calcium phosphate precipitation, lipofection, particle bombardment, microinjection, electroporation, and the like. Methods for producing cells comprising vectors and/or exogenous nucleic acids are well-known in the art. See, for example, Sambrook et al., 2012, Molecular Cloning: A Laboratory Manual, volumes 1 -4, Cold Spring Harbor Press, NY).

Biological methods for introducing a polynucleotide into a host cell include the use of DNA and RNA vectors as described above. Viral vectors, and especially retroviral vectors, have become the most widely used method for inserting genes into mammalian, e.g., human cells. Other viral vectors can be derived from lentivirus, poxviruses, herpes simplex virus I, adenoviruses and adeno-associated viruses, and the like. See, for example, U.S. Pat. Nos. 5,350,674 and 5,585,362.

Chemical means for introducing a polynucleotide into a host cell include colloidal dispersion systems, such as macromolecule complexes, nanocapsules, microspheres, beads, and lipid-based systems including oil-in-water emulsions, micelles, mixed micelles, and liposomes. An exemplary colloidal system for use as a delivery vehicle in vitro and in vivo is a liposome (e.g., an artificial membrane vesicle). Other methods of state-of-the-art targeted delivery of nucleic acids are available, such as delivery of polynucleotides with targeted nanoparticles.

Disclosed herein are methods for producing an in vitro transcribed RNA NKR-CARs, RCARs, or RNKR-CARs. The present disclosure also includes an NKR-CAR encoding, RCAR encoding, or RNKR-CAR encoding RNA construct that can be directly transfected into a cell. A method for generating mRNA for use in transfection can involve in vitro transcription (IVT) of a template with specially designed primers, followed by polyA addition, to produce a construct containing 3' and 5' untranslated sequence ("UTR"), a 5' cap and/or Internal Ribosome Entry Site (IRES), the nucleic acid to be expressed, and a polyA tail, typically 50-2000 bases in. RNA so produced can efficiently transfect different kinds of cells. In one aspect, the template includes sequences for the NKR-CAR, RCAR, or RNKR-CAR.

In one aspect the NKR-CAR, RCAR, or RNKR-CAR is encoded by a messenger RNA (mRNA). In one aspect the mRNA encoding the NKR-CAR, RCAR, or RNKR-CAR is introduced into a T cell for production of a NKR-CAR, RCAR, or RNKR-CAR cell.

In one embodiment, the in vitro transcribed RNA NKR-CAR, RCAR, or RNKR-CAR can be introduced to a cell as a form of transient transfection. The RNA is produced by in vitro transcription using a polymerase chain reaction (PCR)-generated template. DNA of interest from any source can be directly converted by PCR into a template for in vitro mRNA synthesis using appropriate primers and RNA polymerase. The source of the DNA can be, for example, genomic DNA, plasmid DNA, phage DNA, cDNA, synthetic DNA sequence or any other appropriate source of DNA. In one instance, the desired temple for in vitro transcription is a NKR-CAR, RCAR, or RNKR-CAR of the present disclosure. For example, the template for the RNA NKR-CAR, RCAR, or RNKR-CAR comprises an extracellular region comprising a single chain variable domain of an anti-tumor antibody; a hinge region, a transmembrane domain (e.g., a transmembrane domain of a receptor, e.g., T cell receptor or NKR). In one instance, the desired templates for in vitro transcription comprises RNKR-CAR, RCAR, or NKR-CAR on a separate template from an intracellular signaling domain or adaptor molecule domain. For example, the desired templates for in vitro transcription comprises KIR-CAR and DAP12 or CD3zeta on separate templates. In one instance, the desired templates for in vitro transcription comprises RNKR-CAR, RCAR, or NKR-CAR on a separate template from an intracellular signaling domain or adaptor molecule domain. For example, the desired temple for in vitro transcription comprises KIR-CAR and DAP12 or CD3zeta on the same template. In an instance, the template for DAP12 or CD3zeta comprises a transmembrane domain and an intracellular region.

In one embodiment, the DNA to be used for PCR contains an open reading frame. The DNA can be from a naturally occurring DNA sequence from the genome of an organism. In one embodiment, the nucleic acid can include some or all of the 5' and/or 3' untranslated regions (UTRs). The nucleic acid can include exons and introns. In one embodiment, the DNA to be used for PCR is a human nucleic acid sequence. In another embodiment, the DNA to be used for PCR is a human nucleic acid sequence including the 5' and 3' UTRs. The DNA can alternatively be an artificial DNA sequence that is not normally expressed in a naturally occurring organism. An exemplary artificial DNA sequence is one that contains portions of genes that are ligated together to form an open reading frame that encodes a fusion protein. The portions of DNA that are ligated together can be from a single organism or from more than one organism.

PCR is used to generate a template for in vitro transcription of mRNA which is used for transfection. Methods for performing PCR are well known in the art. Primers for use in PCR are designed to have regions that are substantially complementary to regions of the DNA to be used as a template for the PCR. "Substantially complementary," as used herein, refers to sequences of nucleotides where a majority or all of the bases in the primer sequence are complementary, or one or more bases are non-complementary, or mismatched. Substantially complementary sequences are able to anneal or hybridize with the intended DNA target under annealing conditions used for PCR. The primers can be designed to be substantially complementary to any portion of the DNA template. For example, the primers can be designed to amplify the portion of a nucleic acid that is normally transcribed in cells (the open reading frame), including 5' and 3' UTRs. The primers can also be designed to amplify a portion of a nucleic acid that encodes a particular domain of interest. In one embodiment, the primers are designed to amplify the coding region of a human cDNA, including all or portions of the 5' and 3' UTRs. Primers useful for PCR can be generated by synthetic methods that are well known in the art. "Forward primers" are primers that contain a region of nucleotides that are substantially complementary to nucleotides on the DNA template that are upstream of the DNA sequence that is to be amplified. "Upstream" is used herein to refer to a location 5, to the DNA sequence to be amplified relative to the coding strand. "Reverse primers" are primers that contain a region of nucleotides that are substantially complementary to a double-stranded DNA template that are downstream of the DNA sequence that is to be amplified. "Downstream" is used herein to refer to a location 3' to the DNA sequence to be amplified relative to the coding strand.

Any DNA polymerase useful for PCR can be used in the methods disclosed herein. The reagents and polymerase are commercially available from a number of sources.

Chemical structures with the ability to promote stability and/or translation efficiency may also be used. The RNA preferably has 5' and 3' UTRs. In one embodiment, the 5' UTR is between one and 3000 nucleotides in length. The length of 5' and 3' UTR sequences to be added to the coding region can be altered by different methods, including, but not limited to, designing primers for PCR that anneal to different regions of the UTRs. Using this approach, one of ordinary skill in the art can modify the 5' and 3' UTR lengths required to achieve optimal translation efficiency following transfection of the transcribed RNA.

The 5' and 3' UTRs can be the naturally occurring, endogenous 5' and 3' UTRs for the nucleic acid of interest. Alternatively, UTR sequences that are not endogenous to the nucleic acid of interest can be added by incorporating the UTR sequences into the forward and reverse primers or by any other modifications of the template. The use of UTR sequences that are not endogenous to the nucleic acid of interest can be useful for modifying the stability and/or translation efficiency of the RNA. For example, it is known that AU-rich elements in 3' UTR sequences can decrease the stability of mRNA. Therefore, 3' UTRs can be selected or designed to increase the stability of the transcribed RNA based on properties of UTRs that are well known in the art.

In one embodiment, the 5' UTR can contain the Kozak sequence of the endogenous nucleic acid. Alternatively, when a 5' UTR that is not endogenous to the nucleic acid of interest is being added by PCR as described above, a consensus Kozak sequence can be redesigned by adding the 5' UTR sequence. Kozak sequences can increase the efficiency of translation of some RNA transcripts, but does not appear to be required for all RNAs to enable efficient translation. The requirement for Kozak sequences for many mRNAs is known in the art. In other embodiments the 5' UTR can be 5'UTR of an RNA virus whose RNA genome is stable in cells. In other embodiments various nucleotide analogues can be used in the 3' or 5' UTR to impede exonuclease degradation of the mRNA.

To enable synthesis of RNA from a DNA template without the need for gene cloning, a promoter of transcription should be attached to the DNA template upstream of the sequence to be transcribed. When a sequence that functions as a promoter for an RNA polymerase is added to the 5' end of the forward primer, the RNA polymerase promoter becomes incorporated into the PCR product upstream of the open reading frame that is to be transcribed. In one preferred embodiment, the promoter is a T7 polymerase promoter, as described elsewhere herein. Other useful promoters include, but are not limited to, T3 and SP6 RNA polymerase promoters. Consensus nucleotide sequences for T7, T3 and SP6 promoters are known in the art.

In a preferred embodiment, the mRNA has both a cap on the 5' end and a 3' poly(A) tail which determine ribosome binding, initiation of translation and stability mRNA in the cell. On a circular DNA template, for instance, plasmid DNA, RNA polymerase produces a long concatameric product which is not suitable for expression in eukaryotic cells. The transcription of plasmid DNA linearized at the end of the 3' UTR results in normal sized mRNA which is not effective in eukaryotic transfection even if it is polyadenylated after transcription.

On a linear DNA template, phage T7 RNA polymerase can extend the 3' end of the transcript beyond the last base of the template (Schenborn and Mierendorf, Nuc Acids Res., 13:6223-36 (1985); Nacheva and Berzal-Herranz, Eur. J. Biochem., 270:1485-65 (2003).

The conventional method of integration of polyA/T stretches into a DNA template is molecular cloning. However polyA/T sequence integrated into plasmid DNA can cause plasmid instability, which is why plasmid DNA templates obtained from bacterial cells are often highly contaminated with deletions and other aberrations. This makes cloning procedures not only laborious and time consuming but often not reliable. That is why a method which allows construction of DNA templates with polyA/T 3' stretch without cloning highly desirable.

The polyA/T segment of the transcriptional DNA template can be produced during PCR by using a reverse primer containing a polyT tail, such as 100T tail (size can be 50-5000 T (SEQ ID NO: 169), or after PCR by any other method, including, but not limited to, DNA ligation or in vitro recombination. Poly(A) tails also provide stability to RNAs and reduce their degradation. Generally, the length of a poly(A) tail positively correlates with the stability of the transcribed RNA. In one embodiment, the poly(A) tail is between 100 and 5000 adenosines.

Poly(A) tails of RNAs can be further extended following in vitro transcription with the use of a poly(A) polymerase, such as E. coli polyA polymerase (E-PAP). In one embodiment, increasing the length of a poly(A) tail from 100 nucleotides to between 300 and 400 nucleotides results in about a two-fold increase in the translation efficiency of the RNA. Additionally, the attachment of different chemical groups to the 3' end can increase mRNA stability. Such attachment can contain modified/artificial nucleotides, aptamers and other compounds. For example, ATP analogs can be incorporated into the poly(A) tail using poly(A) polymerase. ATP analogs can further increase the stability of the RNA.

5' caps on also provide stability to RNA molecules. In a preferred embodiment, RNAs produced by the methods disclosed herein include a 5' cap. The 5' cap is provided using techniques known in the art and described herein (Cougot, et al., Trends in Biochem. Sci., 29:436-444 (2001); Stepinski, et al., RNA, 7:1468-95 (2001); Elango, et al., Biochim. Biophys. Res. Commun., 330:958-966 (2005)).

The RNAs produced by the methods disclosed herein can also contain an internal ribosome entry site (IRES) sequence. The IRES sequence may be any viral, chromosomal or artificially designed sequence which initiates cap-independent ribosome binding to mRNA and facilitates the initiation of translation. Any solutes suitable for cell electroporation, which can contain factors facilitating cellular permeability and viability such as sugars, peptides, lipids, proteins, antioxidants, and surfactants can be included.

In some embodiments, the mRNA can be introduced directly to the cell or patient in a non-viral delivery system and injected directly into the patient. In the case where a non-viral delivery system is utilized, an exemplary delivery vehicle is a liposome. The use of lipid formulations is contemplated for the introduction of the nucleic acids into a host cell (in vitro, ex vivo or in vivo). In an embodiment, the nucleic acid may be associated with a lipid. The nucleic acid associated with a lipid may be encapsulated in the aqueous interior of a liposome, interspersed within the lipid bilayer of a liposome, attached to a liposome via a linking molecule that is associated with both the liposome and the oligonucleotide, entrapped in a liposome, complexed with a liposome, dispersed in a solution containing a lipid, mixed with a lipid, combined with a lipid, contained as a suspension in a lipid, contained or complexed with a micelle, or otherwise associated with a lipid. Lipid, lipid/DNA or lipid/expression vector associated compositions are not limited to any particular structure in solution. For example, they may be present in a bilayer structure, as micelles, or with a "collapsed" structure. They may also simply be interspersed in a solution, possibly forming aggregates that are not uniform in size or shape. Lipids are fatty substances which may be naturally occurring or synthetic lipids. For example, lipids include the fatty droplets that naturally occur in the cytoplasm as well as the class of compounds which contain long-chain aliphatic hydrocarbons and their derivatives, such as fatty acids, alcohols, amines, amino alcohols, and aldehydes.

Lipids suitable for use can be obtained from commercial sources. For example, dimyristyl phosphatidylcholine ("DMPC") can be obtained from Sigma, St. Louis, MO; dicetyl phosphate ("DCP") can be obtained from K & K Laboratories (Plainview, NY); cholesterol ("Choi") can be obtained from Calbiochem-Behring; dimyristyl phosphatidylglycerol ("DMPG") and other lipids may be obtained from Avanti Polar Lipids, Inc. (Birmingham, AL.). Stock solutions of lipids in chloroform or chloroform/methanol can be stored at about -200C. Chloroform is used as the only solvent since it is more readily evaporated than methanol. "Liposome" is a generic term encompassing a variety of single and multilamellar lipid vehicles formed by the generation of enclosed lipid bilayers or aggregates. Liposomes can be characterized as having vesicular structures with a phospholipid bilayer membrane and an inner aqueous medium. Multilamellar liposomes have multiple lipid layers separated by aqueous medium. They form spontaneously when phospholipids are suspended in an excess of aqueous solution. The lipid components undergo self-rearrangement before the formation of closed structures and entrap water and dissolved solutes between the lipid bilayers (Ghosh et al., 1991 Glycobiology 5: 505-10). However, compositions that have different structures in solution than the normal vesicular structure are also encompassed. For example, the lipids may assume a micellar structure or merely exist as nonuniform aggregates of lipid molecules. Also contemplated are lipofectamine-nucleic acid complexes.

RCAR, RNKR-CAR, or NKR-CAR components can be encoded on one or more nucleic acid molecules. Exemplary nucleic acid molecules include viral vectors, e.g., lentiviral vectors, retroviral vectors, adenoviral vectors, and the like. In embodiments, the components can be provided on a single nucleic acid molecule, e.g., viral vector, e.g., lentiviral vector, retroviral vectors, adenoviral vectors, and the like, or can be disposed on more than one nucleic acid molecule, e.g., viral vector, e.g., lentiviral vector, retroviral vectors, adenoviral vectors, and the like.

Tables 6-11 below provide exemplary configurations of dimerization switches on RCARs or RNKR-CARs.

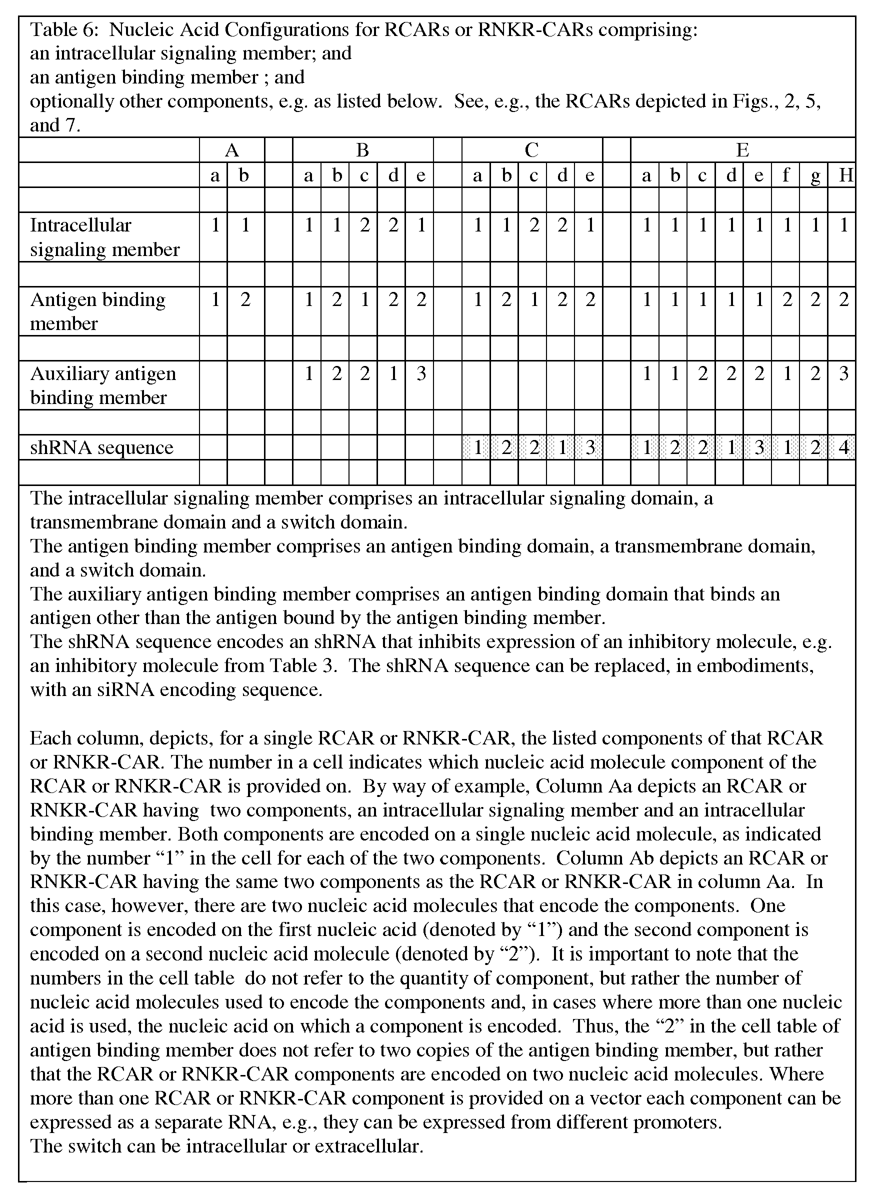

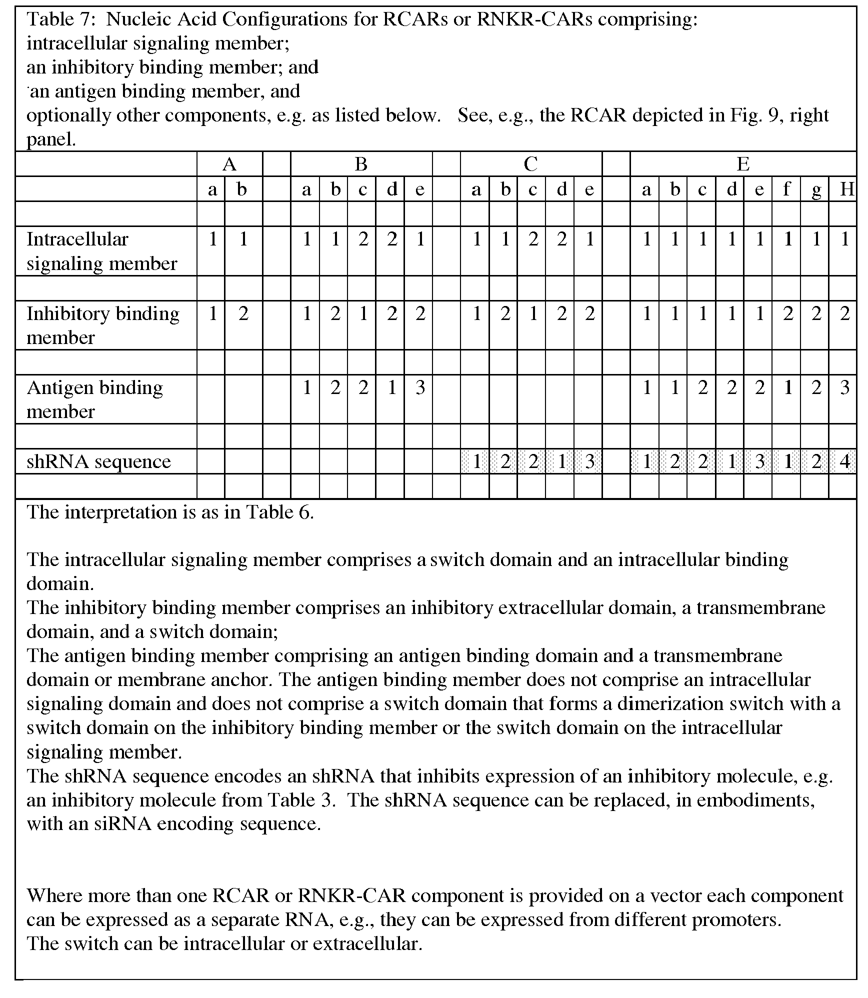

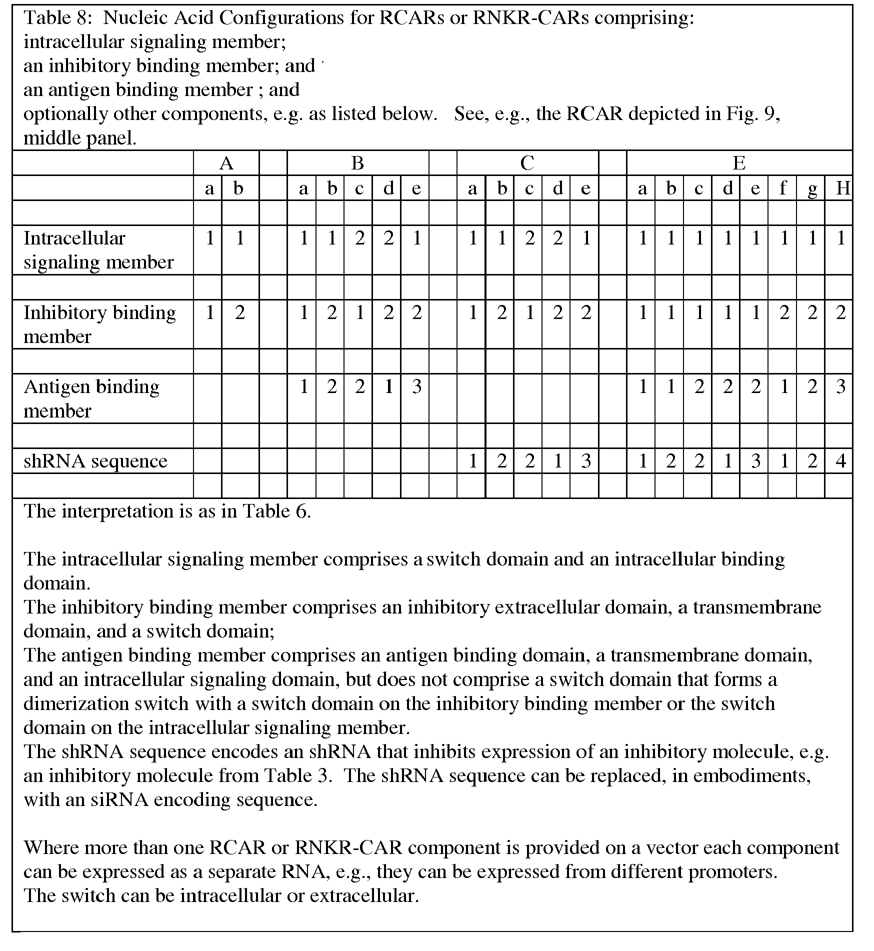

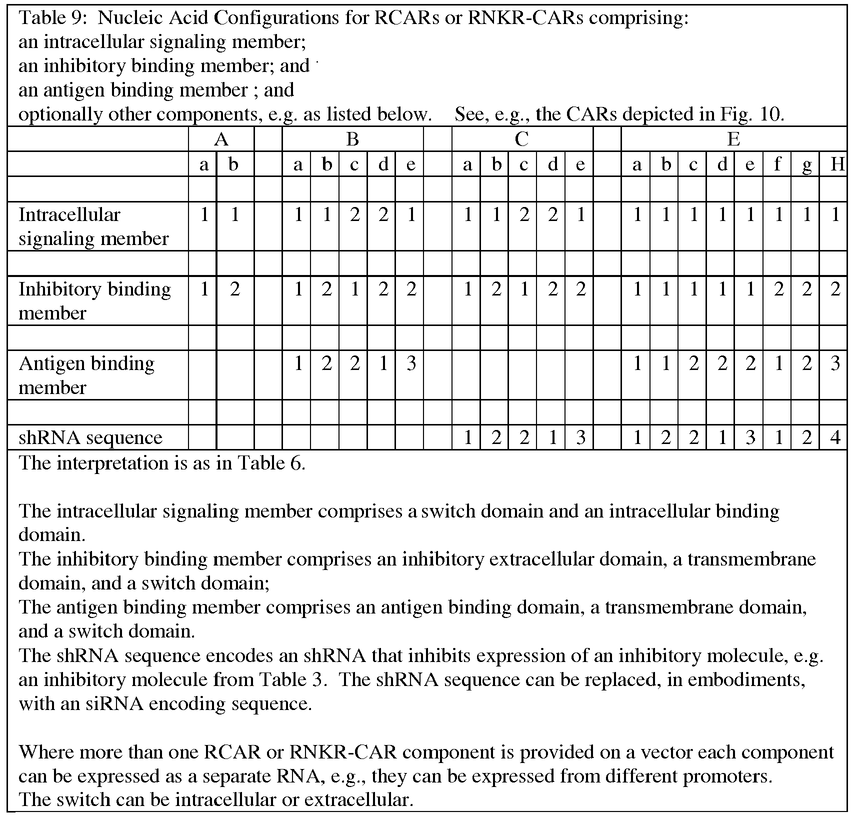

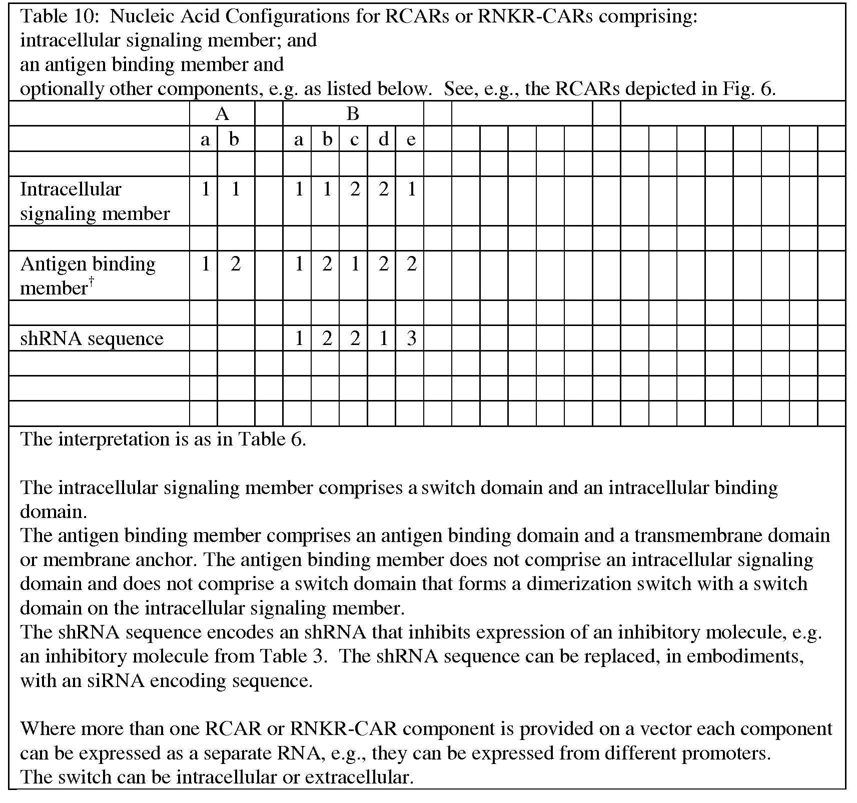

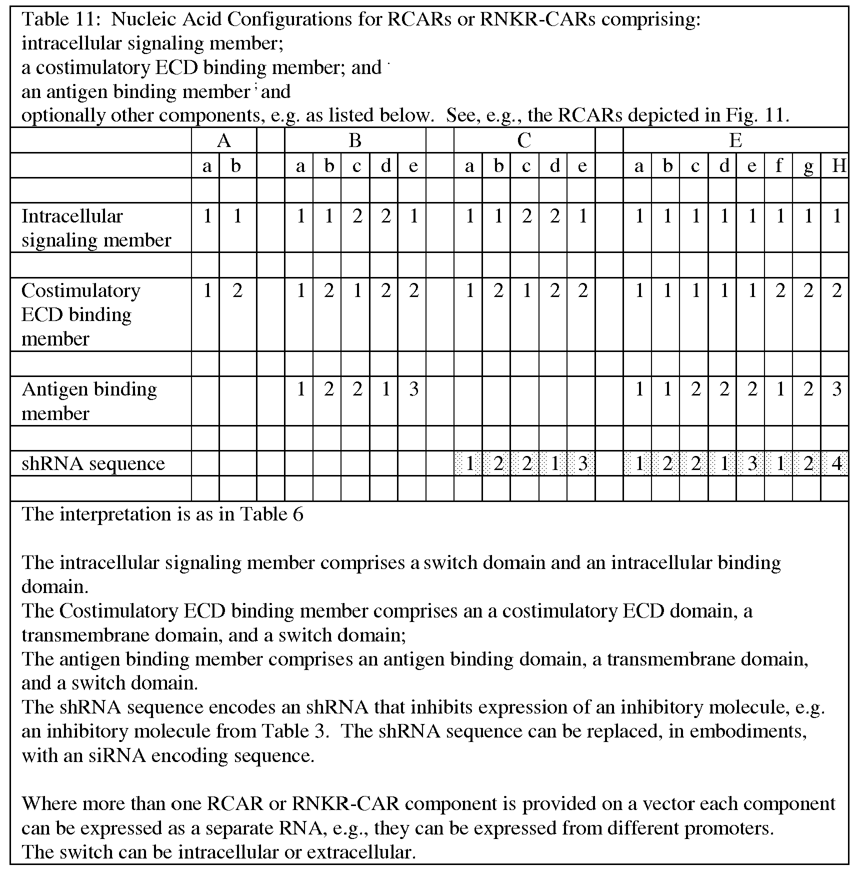

### NUCLEIC ACID BASED INHIBITORS

### DOUBLE STRANDED RNA (DSRNA)

A nucleic acid based inhibitor useful for decreasing the expression of target gene, e.g., an inhibitory molecule gene, comprises dsRNA, such as shRNA. While not wishing to be bound by theory it is believed that the dsRNA acts by an RNAi mechanism. RNAi refers to the process of sequence-specific post-transcriptional gene silencing in animals mediated by short interfering RNAs (siRNAs). dsRNA, as used herein includes siRNA and shRNA.

The dsRNA can be chemically synthesized, expressed from a vector or enzymatically synthesized. dsRNAs can be unmodified or, e.g., in the case of dsRNAs administered as RNA, can be chemically modified. Ezymatically synthesized dsRNAs can be chemically to improve various properties of native dsRNA molecules, such as through increased resistance to nuclease degradation *in vivo* and/or through improved cellular uptake.

The dsRNAs targeting nucleic acid can be composed of two separate RNA molecules referred to herein as siRNA, or of one RNA molecule, which is folded to form a hairpin structure, referred to herein as shRNA. In embodiments, a suitable dsRNA for inhibiting expression of a target gene can be identified by screening an siRNA library, such as an adenoviral or lentiviral siRNA library. A dsRNA, e.g., a shRNA, can be provided to a cell as RNA, or in the form of a DNA that is transcribed to provide the dsRNA, e.g., shRNA. A dsRNA, e.g., a shRNA, gene can be expressed from a vector, e.g., viral vector, such as a lentiviral or adenoviral vector. A dsRNA, e.g., an shRNA, can be expressed by a polymerase III promoters, e.g. a U6 or HI promoter or by a polymerase II promoter. shRNA can be expressed in the cell from a DNA construct encoding a sequence of single stranded RNA and its complement, separated by a stuffer, or linker, fragment, allowing the RNA molecule to fold back on itself, creating a dsRNA molecule with a hairpin loop. While not wishing to be bound by theory, it is believed that shRNA expressed from a DNA sequence encoding the shRNA is processed by Dicer to siRNA, which continues along the RNAi pathway via RISC to silence the target gene.

In an embodiment the inhibitor is a dsRNA .e.g., an shRNA, that comprises a duplexed region that is about 15 to about 30 base pairs in length (*e.g.,* about 16, 17, 18, 19, 20, 21, 22, 23, 24, 25, 26, 27, 28, or 29 base pairs in length. In an embodiment the inhibitor is an shRNA, comprising a duplexed region that is about 15 to about 30 base pairs in length (*e.g.,* about 16, 17, 18, 19, 20, 21, 22, 23, 24, 25, 26, 27, 28, or 29 base pairs in length). In an embodiment, the dsRNA, includes overhanging ends of about 1 to about 3 (*e.g.,* about 1, 2, or 3) nucleotides. By "overhang" is meant that 3'-end of one strand of the dsRNA extends beyond the 5'-end of the other strand, or vice versa. The dsRNA can have an overhang on one or both ends of the dsRNA molecule. In some embodiments, the single-stranded overhang is located at the 3'-terminal end of the antisense strand, or, alternatively, at the 3'-terminal end of the sense strand. In some embodiments, the overhang is a TT or UU dinucleotide overhang, *e.g.,* a TT or UU dinucleotide overhang. For example, in an embodiment, the dsRNA includes a 21-nucleotide antisense strand, a 19 base pair duplex region, and a 3'-terminal dinucleotide. In yet another embodiment, a dsRNA includes a duplex nucleic acid where both ends are blunt, or alternatively, where one of the ends is blunt.

In an embodiment the shRNA, after intracellular processing (e.g., by Dicer), results in a 19-23 nucleotide duplex siRNA with 2 nucleotide 3' overhangs.

In an embodiment, the dsRNA, e.g., a shRNA, includes a first and a second sequence, each sequence is about 18 to about 28 nucleotides in length, *e.g.,* about 19 to about 23 nucleotides in length, wherein the first sequence of the dsRNA includes a nucleotide sequence having sufficient complementarity to the target RNA for the dsRNA to direct cleavage of the target via RNA interference, and the second sequence of the dsRNA includes a nucleotide sequence that is complementary to the first strand.

In an embodiment, an dsRNA includes a first and a second sequence that from a duplexed region, wherein each sequence of the duplexed region is about 18 to about 28 nucleotides in length, *e.g.,* about 19 to about 23 nucleotides in length. The first sequence of the hsRNA includes a nucleotide sequence having sufficient complementarity to the target RNA for the hsRNA to direct cleavage of the target via RNA interference, and the second strand of the hsRNA includes a nucleotide sequence that is complementary to the first strand.

In an embodiment, the dsRNA (e.g., the sequences or strands of the duplexed region of an shRNA) includes an antisense sequence having a nucleotide sequence that is complementary to a nucleotide sequence of the target gene or a portion thereof, and a sense sequence having a nucleotide sequence substantially similar to the nucleotide sequence of the target gene or a portion thereof. In an embodiment, the antisense sequence and the sense sequence, independently, include about 15 to about 30 (*e.g.,* about 15, 16, 17, 18, 19, 20, 21, 22, 23, 24, 25, 26, 27, 28, 29, or 30) nucleotides, where the antisense sequence includes about 15 to about 30 (*e.g.,* about 15, 16, 17, 18, 19, 20, 21, 22, 23, 24, 25, 26, 27, 28, 29, or 30) nucleotides that are complementary to nucleotides of the sense sequence.

In an embodiment, a dsRNA is provided as an RNA (and not as a DNA which is transcribed to provide the dsRNA) and includes one or more chemical modifications. Nonlimiting examples of such chemical modifications include without limitation phosphorothioate internucleotide linkages, 2'-deoxyribonucleotides, 2'-O-methyl ribonucleotides, 2'-deoxy-2'-fluoro ribonucleotides, "universal base" nucleotides, "acyclic" nucleotides, 5-C-methyl nucleotides, and terminal glyceryl and/or inverted deoxy abasic residue incorporation. Such chemical modifications have been shown to preserve RNAi activity in cells while at the same time, dramatically increasing the serum stability of these compounds. Furthermore, one or more phosphorothioate substitutions are well-tolerated and have been shown to confer substantial increases in serum stability for modified dsRNA constructs. The dsRNA can include modified nucleotides as a percentage of the total number of nucleotides present in the molecule. As such, the dsRNA can generally include about 5% to about 100% modified nucleotides (*e.g*., about 5%, 10%, 15%, 20%, 25%, 30%, 35%, 40%, 45%, 50%, 55%, 60%, 65%, 70%, 75%, 80%, 85%, 90%, 95% or 100% modified nucleotides).

### ANTISENSE

Suitable nucleic acid based inhibitors include antisense nucleic acids. While not being bound by theory it is believed that antisense inhibition is typically based upon hydrogen bonding-based hybridization of oligonucleotide strands or segments such that at least one strand or segment is cleaved, degraded, or otherwise rendered inoperable.

An antisense agent can have a chemical modification described above as being suitable for dsRNA.

Antisense agents can include, for example, from about 8 to about 80 nucleobases (*i.e.,* from about 8 to about 80 nucleotides), *e.g.,* about 8 to about 50 nucleobases, or about 12 to about 30 nucleobases. Antisense compounds include ribozymes, external guide sequence (EGS) oligonucleotides (oligozymes), and other short catalytic RNAs or catalytic oligonucleotides which hybridize to the target nucleic acid and modulate its expression. Anti-sense compounds can include a stretch of at least eight consecutive nucleobases that are complementary to a sequence in the target gene. An oligonucleotide need not be 100% complementary to its target nucleic acid sequence to be specifically hybridizable. An oligonucleotide is specifically hybridizable when binding of the oligonucleotide to the target interferes with the normal function of the target molecule to cause a loss of utility, and there is a sufficient degree of complementarity to avoid non-specific binding of the oligonucleotide to non-target sequences under conditions in which specific binding is desired, *i.e.,* under physiological conditions in the case of *in vivo* assays or therapeutic treatment or, in the case of *in vitro* assays, under conditions in which the assays are conducted.

While not being bound by theory it is believed that the functions of mRNA to be interfered with include all key functions such as, for example, translocation of the RNA to the site of protein translation, translation of protein from the RNA, splicing of the RNA to yield one or more mRNA species, and catalytic activity which may be engaged in by the RNA. Binding of specific protein(s) to the RNA may also be interfered with by antisense oligonucleotide hybridization to the RNA.

### SEQUENCE IDENTITY

Percent identity in the context of two or more nucleic acids or polypeptide sequences, refers to two or more sequences that are the same. Two sequences are "substantially identical" if two sequences have a specified percentage of amino acid residues or nucleotides that are the same (i.e., 60% identity, optionally 70%, 71%. 72%. 73%, 74%, 75%, 76%, 77%, 78%, 79%, 80%,81%, 82%, 83%, 84%, 85%, 86%, 87%, 88%, 89%, 90%, 91%, 92%, 93%, 94%, 95%, 96%, 97%, 98%, 99% identity over a specified region, or, when not specified, over the entire sequence, e.g., of the shorter of the compared sequences), when compared and aligned for maximum correspondence over a comparison window, or designated region as measured using one of the following sequence comparison algorithms or by manual alignment and visual inspection. Optionally, the identity exists over a region that is at least about 50 nucleotides (or 10 amino acids) in length, or more preferably over a region that is 100 to 500 or 1000 or more nucleotides (or 20, 50, 200 or more amino acids) in length.

For sequence comparison, typically one sequence acts as a reference sequence, to which test sequences are compared. When using a sequence comparison algorithm, test and reference sequences are entered into a computer, subsequence coordinates are designated, if necessary, and sequence algorithm program parameters are designated. Default program parameters can be used, or alternative parameters can be designated. The sequence comparison algorithm then calculates the percent sequence identities for the test sequences relative to the reference sequence, based on the program parameters. Methods of alignment of sequences for comparison are known in the art. Optimal alignment of sequences for comparison can be conducted, e.g., by the local homology algorithm of Smith and Waterman, (1970) Adv. Appl. Math. 2:482c, by the homology alignment algorithm of Needleman and Wunsch, (1970) J. Mol. Biol. 48:443, by the search for similarity method of Pearson and Lipman, (1988) Proc. Nat'l. Acad. Sci. USA 85:2444, by computerized implementations of these algorithms (GAP, BESTFIT, FASTA, and TFASTA in the Wisconsin Genetics Software Package, Genetics Computer Group, 575 Science Dr., Madison, WI), or by manual alignment and visual inspection (see, e.g., Brent et al., (2003) Current Protocols in Molecular Biology).

Two examples of algorithms that are suitable for determining percent sequence identity and sequence similarity are the BLAST and BLAST 2.0 algorithms, which are described in Altschul et al., (1977) Nuc. Acids Res. 25:3389-3402; and Altschul et al., (1990) J. Mol. Biol. 215:403-410, respectively. Software for performing BLAST analyses is publicly available through the National Center for Biotechnology Information.

The percent identity between two amino acid sequences can also be determined using the algorithm of E. Meyers and W. Miller, (1988) Comput. Appl. Biosci. 4:11-17) which has been incorporated into the ALIGN program (version 2.0), using a PAM120 weight residue table, a gap length penalty of 12 and a gap penalty of 4. In addition, the percent identity between two amino acid sequences can be determined using the Needleman and Wunsch (1970) J. Mol. Biol. 48:444-453) algorithm which has been incorporated into the GAP program in the GCG software package (available at www.gcg.com), using either a Blossom 62 matrix or a PAM250 matrix, and a gap weight of 16, 14, 12, 10, 8, 6, or 4 and a length weight of 1, 2, 3, 4, 5, or 6.

In an embodiment, the present invention contemplates modifications of the antigen binding domain (e.g., svFv) amino acid sequence that generate functionally equivalent molecules. For example, the VH or VL of an scFv of RCAR, RNKR-CAR, or NKR-CAR can be modified to retain at least about 70%, 71%. 72%. 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% identity of the starting VH or VL sequences of the scFv.

In certain embodiments the polypeptide sequences encoded by the nucleic acid sequences are modified by replacing one or more amino acid residues with another amino acid residue from the same side chain family, i.e., a conservative substitutions. Families of amino acid residues having similar side chains have been defined in the art, including basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine).

Alternative methods useful for decreasing the expression of target gene, e.g., an inhibitory molecule gene as described herein, includes a clustered regularly interspaced short palindromic repeats (CRISPR), a transcription-activator like effector nuclease (TALEN), or a zinc finger endonuclease (ZFN), e.g., as described herein.

### SOURCES OF CELLS

In embodiments, prior to expansion and genetic modification or other modification, a source of cells, e.g., T cells or natural killer (NK) cells, can be obtained from a subject. The term "subject" is intended to include living organisms in which an immune response can be elicited (e.g., mammals). Examples of subjects include humans, monkeys, chimpanzees, dogs, cats, mice, rats, and transgenic species thereof. T cells can be obtained from a number of sources, including peripheral blood mononuclear cells, bone marrow, lymph node tissue, cord blood, thymus tissue, tissue from a site of infection, ascites, pleural effusion, spleen tissue, and tumors.

### T CELLS

In an embodiment, the cells are T cells. T cell lines available in the art, may be used. In embodiments, T cells can be obtained from a unit of blood collected from a subject using any number of techniques known to the skilled artisan, such as Ficoll™ separation. In one aspect, cells from the circulating blood of an individual are obtained by apheresis. The apheresis product typically contains lymphocytes, including T cells, monocytes, granulocytes, B cells, other nucleated white blood cells, red blood cells, and platelets. In one aspect, the cells collected by apheresis may be washed to remove the plasma fraction and, optionally, to place the cells in an appropriate buffer or media for subsequent processing steps. In one embodiment, the cells are washed with phosphate buffered saline (PBS). In an alternative embodiment, the wash solution lacks calcium and may lack magnesium or may lack many if not all divalent cations.

Initial activation steps in the absence of calcium can lead to magnified activation. As those of ordinary skill in the art would readily appreciate a washing step may be accomplished by methods known to those in the art, such as by using a semi-automated "flow-through" centrifuge (for example, the Cobe 2991 cell processor, the Baxter CytoMate, or the Haemonetics Cell Saver 5) according to the manufacturer's instructions. After washing, the cells may be resuspended in a variety of biocompatible buffers, such as, for example, Ca-free, Mg-free PBS, PlasmaLyte A, or other saline solution with or without buffer. Alternatively, the undesirable components of the apheresis sample may be removed and the cells directly resuspended in culture media.

In one aspect, T cells are isolated from peripheral blood lymphocytes by lysing the red blood cells and depleting the monocytes, for example, by centrifugation through a PERCOLL™ gradient or by counterflow centrifugal elutriation.

The methods described herein can include, e.g., selection of a specific subpopulation of immune effector cells, e.g., T cells, that are a T regulatory cell-depleted population, CD25+ depleted cells, using, e.g., a negative selection technique, e.g., described herein. Preferably, the population of T regulatory depleted cells contains less than 30%, 25%, 20%, 15%, 10%, 5%, 4%, 3%, 2%, 1% of CD25+ cells.

In one embodiment, T regulatory cells, e.g., CD25+ T cells, are removed from the population using an anti-C25 antibody, or fragment thereof, or a CD25-binding ligand, IL-2. In one embodiment, the anti-CD25 antibody, or fragment thereof, or CD25-binding ligand is conjugated to a substrate, e.g., a bead, or is otherwise coated on a substrate, e.g., a bead. In one embodiment, the anti-CD25 antibody, or fragment thereof, is conjugated to a substrate as described herein.

In one embodiment, the T regulatory cells, e.g., CD25+ T cells, are removed from the population using CD25 depletion reagent from Militenyi™. In one embodiment, the ratio of cells to CD25 depletion reagent is 1e7 cells to 20 uL, or 1e7 cells to 15 uL, or 1e7 cells to 10 uL, or 1e7 cells to 5 uL, or 1e7 cells to 2.5 uL, or 1e7 cells to 1.25 uL.

In one embodiment, the population of immune effector cells to be depleted includes about 6 x 10⁹ CD25+ T cells. In other aspects, the population of immune effector cells to be depleted include about 1 x 10⁹ to 1x 10¹⁰ CD25+ T cell, and any integer value in between. In one embodiment, the resulting population T regulatory depleted cells has 2 x 10⁹ T regulatory cells, e.g., CD25+ cells, or less (e.g., 1 x 10⁹, 5 x 10⁸, 1 x 10⁸, 5 x 10⁷, 1 x 10⁷, or less CD25+ cells).

In one embodiment, the T regulatory cells, e.g., CD25+ cells, are removed from the population using the CliniMAC system with a depletion tubing set, such as, e.g., tubing 162-01. In one embodiment, the CliniMAC system is run on a depletion setting such as, e.g., DEPLETION2.1.

The methods described herein can include more than one selection step, e.g., more than one depletion step. Enrichment of a T cell population by negative selection can be accomplished, e.g., with a combination of antibodies directed to surface markers unique to the negatively selected cells. One method is cell sorting and/or selection via negative magnetic immunoadherence or flow cytometry that uses a cocktail of monoclonal antibodies directed to cell surface markers present on the cells negatively selected. For example, to enrich for CD4+ cells by negative selection, a monoclonal antibody cocktail can include antibodies to CD14, CD20, CD11b, CD16, HLA-DR, and CD8.

The methods described herein can further include removing cells from the population which express a tumor antigen, e.g., a tumor antigen that does not comprise CD25, e.g., CD19, CD30, CD38, CD123, CD20, CD14 or CD11b, to thereby provide a population of T regulatory depleted, e.g., CD25+ depleted, and tumor antigen depleted cells that are suitable for expression of a CAR, e.g., a CAR described herein. In one embodiment, tumor antigen expressing cells are removed simultaneously with the T regulatory, e.g., CD25+ cells. For example, an anti-C25 antibody, or fragment thereof, and an anti-tumor antigen antibody, or fragment thereof, can be attached to the same substrate, e.g., bead, which can be used to remove the cells or an anti-CD25 antibody, or fragment thereof, or the anti-tumor antigen antibody, or fragment thereof, can be attached to separate beads, a mixture of which can be used to remove the cells. In other embodiments, the removal of T regulatory cells, e.g., CD25+ cells, and the removal of the tumor antigen expressing cells is sequential, and can occur, e.g., in either order.

Also provided are methods that include removing cells from the population which express a check point inhibitor, e.g., a check point inhibitor described herein, e.g., one or more of PD1+ cells, LAG3+ cells, and TIM3+ cells, to thereby provide a population of T regulatory depleted, e.g., CD25+ depleted cells, and check point inhibitor depleted cells, e.g., PD1+, LAG3+ and/or TIM3+ depleted cells. Exemplary check point inhibitors include B7-H1, B&-1, CD160, P1H, 2B4, PD1, TIM3, CEACAM (e.g., CEACAM-1, CEACAM-3 and/or CEACAM-5), LAG3, TIGIT, CTLA-4, BTLA and LAIR1. In one embodiment, check point inhibitor expressing cells are removed simultaneously with the T regulatory, e.g., CD25+ cells. For example, an anti-C25 antibody, or fragment thereof, and an anti-check point inhibitor antibody, or fragment thereof, can be attached to the same bead which can be used to remove the cells, or an anti-CD25 antibody, or fragment thereof, and the anti-check point inhibitor antibody, or fragment there, can be attached to separate beads, a mixture of which can be used to remove the cells. In other embodiments, the removal of T regulatory cells, e.g., CD25+ cells, and the removal of the check point inhibitor expressing cells is sequential, and can occur, e.g., in either order.

Methods described herein can include a positive selection step. For example, T cells can isolated by incubation with anti-CD3/anti-CD28 (e.g., 3x28)-conjugated beads, such as DYNABEADS® M-450 CD3/CD28 T, for a time period sufficient for positive selection of the desired T cells. In one embodiment, the time period is about 30 minutes. In a further embodiment, the time period ranges from 30 minutes to 36 hours or longer and all integer values there between. In a further embodiment, the time period is at least 1, 2, 3, 4, 5, or 6 hours. In yet another embodiment, the time period is 10 to 24 hours, e.g., 24 hours. Longer incubation times may be used to isolate T cells in any situation where there are few T cells as compared to other cell types, such in isolating tumor infiltrating lymphocytes (TIL) from tumor tissue or from immunocompromised individuals. Further, use of longer incubation times can increase the efficiency of capture of CD8+ T cells. Thus, by simply shortening or lengthening the time T cells are allowed to bind to the CD3/CD28 beads and/or by increasing or decreasing the ratio of beads to T cells (as described further herein), subpopulations of T cells can be preferentially selected for or against at culture initiation or at other time points during the process. Additionally, by increasing or decreasing the ratio of anti-CD3 and/or anti-CD28 antibodies on the beads or other surface, subpopulations of T cells can be preferentially selected for or against at culture initiation or at other desired time points.

In one embodiment, a T cell population can be selected that expresses one or more of IFN-^{γ}, TNFα, IL-17A, IL-2, IL-3, IL-4, GM-CSF, IL-10, IL-13, granzyme B, and perforin, or other appropriate molecules, e.g., other cytokines. Methods for screening for cell expression can be determined, e.g., by the methods described in PCT Publication No.: WO 2013/126712.

For isolation of a desired population of cells by positive or negative selection, the concentration of cells and surface (e.g., particles such as beads) can be varied. In certain aspects, it may be desirable to significantly decrease the volume in which beads and cells are mixed together (e.g., increase the concentration of cells), to ensure maximum contact of cells and beads. For example, in one aspect, a concentration of 10 billion cells/ml, 9 billion/ml, 8 billion/ml, 7 billion/ml, 6 billion/ml, or 5 billion/ml is used. In one aspect, a concentration of 1 billion cells/ml is used. In yet one aspect, a concentration of cells from 75, 80, 85, 90, 95, or 100 million cells/ml is used. In further aspects, concentrations of 125 or 150 million cells/ml can be used.

Using high concentrations can result in increased cell yield, cell activation, and cell expansion. Further, use of high cell concentrations allows more efficient capture of cells that may weakly express target antigens of interest, such as CD28-negative T cells, or from samples where there are many tumor cells present (e.g., leukemic blood, tumor tissue, etc.). Such populations of cells may have therapeutic value and would be desirable to obtain. For example, using high concentration of cells allows more efficient selection of CD8+ T cells that normally have weaker CD28 expression.

In a related aspect, it may be desirable to use lower concentrations of cells. By significantly diluting the mixture of T cells and surface (e.g., particles such as beads), interactions between the particles and cells is minimized. This selects for cells that express high amounts of desired antigens to be bound to the particles. For example, CD4+ T cells express higher levels of CD28 and are more efficiently captured than CD8+ T cells in dilute concentrations. In one aspect, the concentration of cells used is 5 x 10⁶/ml. In other aspects, the concentration used can be from about 1 x 10⁵/ml to 1 x 10⁶/ml, and any integer value in between.

In other aspects, the cells may be incubated on a rotator for varying lengths of time at varying speeds at either 2-10°C or at room temperature.

T cells for stimulation can also be frozen after a washing step. Wishing not to be bound by theory, the freeze and subsequent thaw step provides a more uniform product by removing granulocytes and to some extent monocytes in the cell population. After the washing step that removes plasma and platelets, the cells may be suspended in a freezing solution. While many freezing solutions and parameters are known in the art and will be useful in this context, one method involves using PBS containing 20% DMSO and 8% human serum albumin, or culture media containing 10% Dextran 40 and 5% Dextrose, 20% Human Serum Albumin and 7.5% DMSO, or 31.25% Plasmalyte-A, 31.25% Dextrose 5%, 0.45% NaCl, 10% Dextran 40 and 5% Dextrose, 20% Human Serum Albumin, and 7.5% DMSO or other suitable cell freezing media containing for example, Hespan and PlasmaLyte A, the cells then are frozen to -80°C at a rate of 1° per minute and stored in the vapor phase of a liquid nitrogen storage tank. Other methods of controlled freezing may be used as well as uncontrolled freezing immediately at -20° C or in liquid nitrogen.

In certain aspects, cryopreserved cells are thawed and washed as described herein and allowed to rest for one hour at room temperature prior to activation using the methods of the present invention.

Also contemplated in the context of the invention is the collection of blood samples or apheresis product from a subject at a time period prior to when the expanded cells as described herein might be needed. As such, the source of the cells to be expanded can be collected at any time point necessary, and desired cells, such as T cells, isolated and frozen for later use in immune effector cell therapy for any number of diseases or conditions that would benefit from immune effector cell therapy, such as those described herein. In one aspect a blood sample or an apheresis is taken from a generally healthy subject. In certain aspects, a blood sample or an apheresis is taken from a generally healthy subject who is at risk of developing a disease, but who has not yet developed a disease, and the cells of interest are isolated and frozen for later use. In certain aspects, the T cells may be expanded, frozen, and used at a later time. In certain aspects, samples are collected from a patient shortly after diagnosis of a particular disease as described herein but prior to any treatments. In a further aspect, the cells are isolated from a blood sample or an apheresis from a subject prior to any number of relevant treatment modalities, including but not limited to treatment with agents such as natalizumab, efalizumab, antiviral agents, chemotherapy, radiation, immunosuppressive agents, such as cyclosporin, azathioprine, methotrexate, mycophenolate, and FK506, antibodies, or other immunoablative agents such as CAMPATH, anti-CD3 antibodies, cytoxan, fludarabine, cyclosporin, FK506, rapamycin, mycophenolic acid, steroids, FR901228, and irradiation.

In a further aspect of the present invention, T cells are obtained from a patient directly following treatment that leaves the subject with functional T cells. In this regard, it has been observed that following certain cancer treatments, in particular treatments with drugs that damage the immune system, shortly after treatment during the period when patients would normally be recovering from the treatment, the quality of T cells obtained may be optimal or improved for their ability to expand ex vivo. Likewise, following ex vivo manipulation using the methods described herein, these cells may be in a preferred state for enhanced engraftment and in vivo expansion. Thus, it is contemplated within the context of the present invention to collect blood cells, including T cells, dendritic cells, or other cells of the hematopoietic lineage, during this recovery phase. Further, in certain aspects, mobilization (for example, mobilization with GM-CSF) and conditioning regimens can be used to create a condition in a subject wherein repopulation, recirculation, regeneration, and/or expansion of particular cell types is favored, especially during a defined window of time following therapy. Illustrative cell types include T cells, B cells, dendritic cells, and other cells of the immune system.

In one instance, the immune effector cells expressing a CAR molecule, e.g., a CAR molecule described herein, e.g., a RCAR, RNKR-CAR, or NKR-CAR described herein, are obtained from a subject that has received a low, immune enhancing dose of an mTOR inhibitor. In an instance, the population of immune effector cells, e.g., T cells, to be engineered to express a CAR, e.g., a RCAR, RNKR-CAR, or NKR-CAR, are harvested after a sufficient time, or after sufficient dosing of the low, immune enhancing, dose of an mTOR inhibitor, such that the level of PD1 negative immune effector cells, e.g., T cells, or the ratio of PD1 negative immune effector cells, e.g., T cells/ PD1 positive immune effector cells, e.g., T cells, in the subject or harvested from the subject has been, at least transiently, increased.

In other instances, population of immune effector cells, e.g., T cells, which have, or will be engineered to express a CAR, e.g., a RCAR, RNKR-CAR, or NKR-CAR, can be treated ex vivo by contact with an amount of an mTOR inhibitor that increases the number of PD1 negative immune effector cells, e.g., T cells or increases the ratio of PD1 negative immune effector cells, e.g., T cells/ PD1 positive immune effector cells, e.g., T cells.

In one embodiment, a T cell population is diaglycerol kinase (DGK)-deficient. DGK-deficient cells include cells that do not express DGK RNA or protein, or have reduced or inhibited DGK activity. DGK-deficient cells can be generated by genetic approaches, e.g., administering RNA-interfering agents, e.g., siRNA, shRNA, miRNA, to reduce or prevent DGK expression. Alternatively, DGK-deficient cells can be generated by treatment with DGK inhibitors described herein.

In one embodiment, a T cell population is Ikaros-deficient. Ikaros-deficient cells include cells that do not express Ikaros RNA or protein, or have reduced or inhibited Ikaros activity, Ikaros-deficient cells can be generated by genetic approaches, e.g., administering RNA-interfering agents, e.g., siRNA, shRNA, miRNA, to reduce or prevent Ikaros expression. Alternatively, Ikaros-deficient cells can be generated by treatment with Ikaros inhibitors, e.g., lenalidomide.

In embodiments, a T cell population is DGK-deficient and Ikaros-deficient, e.g., does not express DGK and Ikaros, or has reduced or inhibited DGK and Ikaros activity. Such DGK and Ikaros-deficient cells can be generated by any of the methods described herein.

### NK CELLS

In an embodiment, the cells are natural killer cells. These cells can be isolated from patients. In an embodiment, the cells are stable cell lines of natural killer cells, e.g., a stable allogeneic NK-92 cell line available, from Conkwest. These stable NK-92 cell lines were derived from NK-92 cells that were obtained, transfected and cultured using the methods described by *Gong etal* (April 1994), Leukemia Macmillan Press, Ltd, 8: 652-658, and disclosed in EP1007630. An NK cell line with properties similar to the NK-92 cell line can also be used. In an embodiment, NK cells from the circulating blood of an individual are obtained by apheresis. In an embodiment, NK cells are engineered to express a RNKR-CAR or a RCAR in combination with a NKR-CAR (e.g., inhNKR-CAR), and these engineered RNKR-CARN or RCAR/NKR-CARN cells can be used to treat a patient other than a patient from whom the NK cells were isolated. Hence, these RNKR-CARN or RCAR/NKR-CARN cells are "universal" cells in that can be administered to multiple patients without adverse effects. That is to say that NK cells can be isolated from one patient and engineered to express RNKR-CAR or RCAR + NKR-CAR, thereby producing RNKR-CARN or RCAR/NKR-CARN cells, respectively, and these RNKR-CARN or RCAR/NKR-CARN cells can then be administered to the same or different patient. NK cells, e.g., NK-92 cells, do not express killer inhibitory receptors, and therefore cannot be inactivated by evading cancer cells. Methods for isolation and use of NK cells (e.g., NK-92 cell lines or similar NK cell lines derived from peripheral blood mononuclear cells from a patient with non-Hodgkins lymphoma) have been described (See Zhang et al (2013) Retargeting NK-92 for anti-melanoma activity by a TCR-like single domain antibody; Immunol Cell Biol. 91: 615-624; Tonn et al. (2013) Treatment of patients with advanced cancer with the natural killer cell-line NK-92, Cytotherapy, 15: 1563-1570.

The NK-92 cell line was found to exhibit the CD56^{high}, CD2, CD7, CD1 la, CD28, CD45, and CD54 surface markers It furthermore does not display the CD1, CD3, CD4, CD5, CD8, CD10, CD14, CD 16, CD19, CD20, CD23, and CD34 markers Growth of NK-92 cells in culture is dependent upon the presence of recombinant interieukin 2 (rIL-2), with a dose as low as 10 IU/mL being sufficient to maintain proliferation. NK cell lines with similar properties can also be used.

NK-92 cells are readily maintained in culture medium, such as enriched alpha minimum essential medium (MEM, Sigma Chemical Co. St Louis, MO) supplemented with fetal calf serum (for example, at 12 5%, Sigma Chemical Co., St Louis, MO), and horse serum (for example, at 12.5%, (Sigma Chemical Co., St Louis, MO) Initially, 10M hydrocortisone is required, but in subsequent passages it is found that hydrocortisone may be omitted. In addition, IL-2, such as recombinant human IL-2 (500 U/mL, Chiron, Emeryville, CA), is required for long-term growth. When suspension cultures are maintained in this fashion with semiweekly changes of medium, the cells exhibit a doubling time of about 24 h.

NK-92 cells *in vitro* demonstrate lytic activity against a broad range of malignant target cells. These include cell lines derived from circulating target cells such as acute and chronic lymphoblastic and myelogenous leukemia, lymphoma, myeloma, melanoma, as well as cells from solid tumors such as prostate cancer, neuroblastoma, and breast cancer cell lines.

### OTHER IMMUNE EFFECTOR CELLS

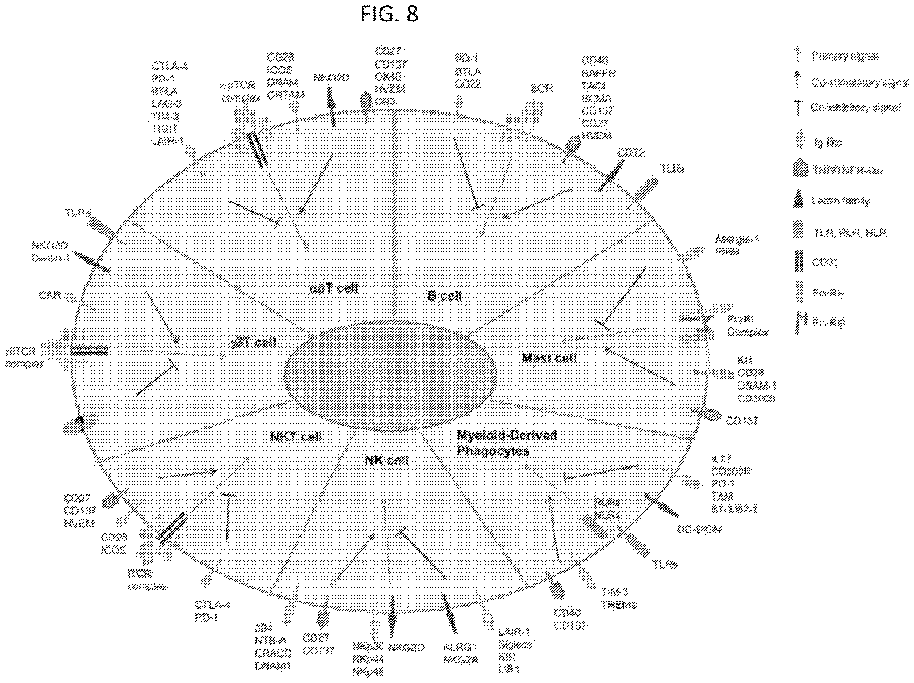

In another instance, any number of immune effector cells may be isolated and engineered to express a RNKR-CAR or a RCAR in combination with a NKR-CAR (e.g., inhNKR-CAR), e.g., B cell, mast cells. Myeloid derived phagocytes, NKT cells, or γδT cells. Exemplary immune effector cells are listed in Fig. 8.

### ALLOGENEIC CAR

In embodiments described herein, the immune effector cell can be an allogeneic immune effector cell, e.g., T cell or NK cell. For example, the cell can be an allogeneic T cell, e.g., an allogeneic T cell lacking expression of a functional T cell receptor (TCR) and/or human leukocyte antigen (HLA), e.g., HLA class I and/or HLA class II.

A T cell lacking a functional TCR can be, e.g., engineered such that it does not express any functional TCR on its surface, engineered such that it does not express one or more subunits that comprise a functional TCR or engineered such that it produces very little functional TCR on its surface. Alternatively, the T cell can express a substantially impaired TCR, e.g., by expression of mutated or truncated forms of one or more of the subunits of the TCR. The term "substantially impaired TCR" means that this TCR will not elicit an adverse immune reaction in a host.

A T cell described herein can be, e.g., engineered such that it does not express a functional HLA on its surface. For example, a T cell described herein, can be engineered such that cell surface expression HLA, e.g., HLA class 1 and/or HLA class II, is downregulated.

In some embodiments, the T cell can lack a functional TCR and a functional HLA, e.g., HLA class I and/or HLA class II.

Modified T cells that lack expression of a functional TCR and/or HLA can be obtained by any suitable means, including a knock out or knock down of one or more subunit of TCR or HLA. For example, the T cell can include a knock down of TCR and/or HLA using siRNA, shRNA, clustered regularly interspaced short palindromic repeats (CRISPR) transcription-activator like effector nuclease (TALEN), or zinc finger endonuclease (ZFN).In some embodiments, the allogenic cell can be a cell which does not expresses or expresses at low levels an inhibitory molecule, e.g. by any mehod described herein. For example, the cell can be a cell that does not express or expresses at low levels an inhibitory molecule, e.g., that can decrease the ability of a CAR-expressing cell to mount an immune effector response. Examples of inhibitory molecules include PD1, PD-L1, CTLA4, TIM3, LAG3, VISTA, BTLA, TIGIT, LAIR1, CD160, 2B4 and TGFR beta. Inhibition of an inhibitory molecule, e.g., by inhibition at the DNA, RNA or protein level, can optimize a CAR-expressing cell performance. In embodiments, an inhibitory nucleic acid, e.g., an inhibitory nucleic acid, e.g., a dsRNA, e.g., an siRNA or shRNA, a clustered regularly interspaced short palindromic repeats (CRISPR), a transcription-activator like effector nuclease (TALEN), or a zinc finger endonuclease (ZFN), e.g., as described herein, can be used.

### siRNA and shRNA to inhibit TCR or HLA

In some embodiments, TCR expression and/or HLA expression can be inhibited using siRNA or shRNA that targets a nucleic acid encoding a TCR and/or HLA in a T cell.

Expression of siRNA and shRNAs in T cells can be achieved using any conventional expression system, e.g., such as a lentiviral expression system.

Exemplary shRNAs that downregulate expression of components of the TCR are described, e.g., in US Publication No.: 2012/0321667. Exemplary siRNA and shRNA that downregulate expression of HLA class I and/or HLA class II genes are described, e.g., in U.S. publication No.: US 2007/0036773.

### CRISPR to inhibit TCR or HLA

"CRISPR" or "CRISPR to TCR and/or HLA" or "CRISPR to inhibit TCR and/or HLA" as used herein refers to a set of clustered regularly interspaced short palindromic repeats, or a system comprising such a set of repeats. "Cas", as used herein, refers to a CRISPR-associated protein. A "CRISPR/Cas" system refers to a system derived from CRISPR and Cas which can be used to silence or mutate a TCR and/or HLA gene.

Naturally-occurring CRISPR/Cas systems are found in approximately 40% of sequenced eubacteria genomes and 90% of sequenced archaea. Grissa et al. (2007) BMC Bioinformatics 8: 172. This system is a type of prokaryotic immune system that confers resistance to foreign genetic elements such as plasmids and phages and provides a form of acquired immunity. Barrangou et al. (2007) Science 315: 1709-1712; Marragini et al. ( 2008) Science 322: 1843-1845.

The CRISPR/Cas system has been modified for use in gene editing (silencing, enhancing or changing specific genes) in eukaryotes such as mice or primates. Wiedenheft et al. (2012) Nature 482: 331-8. This is accomplished by introducing into the eukaryotic cell a plasmid containing a specifically designed CRISPR and one or more appropriate Cas.

The CRISPR sequence, sometimes called a CRISPR locus, comprises alternating repeats and spacers. In a naturally-occurring CRISPR, the spacers usually comprise sequences foreign to the bacterium such as a plasmid or phage sequence; in the TCR and/or HLA CRISPR/Cas system, the spacers are derived from the TCR or HLA gene sequence.

RNA from the CRISPR locus is constitutively expressed and processed by Cas proteins into small RNAs. These comprise a spacer flanked by a repeat sequence. The RNAs guide other Cas proteins to silence exogenous genetic elements at the RNA or DNA level. Horvath et al. (2010) Science 327: 167-170; Makarova et al. (2006) Biology Direct 1: 7. The spacers thus serve as templates for RNA molecules, analogously to siRNAs. Pennisi (2013) Science 341: 833-836.

As these naturally occur in many different types of bacteria, the exact arrangements of the CRISPR and structure, function and number of Cas genes and their product differ somewhat from species to species. Haft et al. (2005) PLoS Comput. Biol. 1: e60; Kunin et al. (2007) Genome Biol. 8: R61; Mojica et al. (2005) J. Mol. Evol. 60: 174-182; Bolotin et al. (2005) Microbiol. 151: 2551-2561; Pourcel et al. (2005) Microbiol. 151: 653-663; and Stern et al. (2010) Trends. Genet. 28: 335-340. For example, the Cse (Cas subtype, E. coli) proteins (e.g., CasA) form a functional complex, Cascade, that processes CRISPR RNA transcripts into spacer-repeat units that Cascade retains. Brouns et al. (2008) Science 321: 960-964. In other prokaryotes, Cas6 processes the CRISPR transcript. The CRISPR-based phage inactivation in E. coli requires Cascade and Cas3, but not Cas1 or Cas2. The Cmr (Cas RAMP module) proteins in Pyrococcus furiosus and other prokaryotes form a functional complex with small CRISPR RNAs that recognizes and cleaves complementary target RNAs. A simpler CRISPR system relies on the protein Cas9, which is a nuclease with two active cutting sites, one for each strand of the double helix. Combining Cas9 and modified CRISPR locus RNA can be used in a system for gene editing. Pennisi (2013) Science 341: 833-836.

The CRISPR/Cas system can thus be used to edit a TCR and/or HLA gene (adding or deleting a basepair), or introducing a premature stop which thus decreases expression of a TCR and/or HLA. The CRISPR/Cas system can alternatively be used like RNA interference, turning off TCR and/or HLA gene in a reversible fashion. In a mammalian cell, for example, the RNA can guide the Cas protein to a TCR and/or HLA promoter, sterically blocking RNA polymerases.

Artificial CRISPR/Cas systems can be generated which inhibit TCR and/or HLA, using technology known in the art, e.g., that described in U.S. Publication No.20140068797.

### TALEN to inhibit TCR and/or HLA

"TALEN" or "TALEN to HLA and/or TCR" or "TALEN to inhibit HLA and/or TCR" refers to a transcription activator-like effector nuclease, an artificial nuclease which can be used to edit the HLA and/or TCR gene.

TALENs are produced artificially by fusing a TAL effector DNA binding domain to a DNA cleavage domain. Transcription activator-like effects (TALEs) can be engineered to bind any desired DNA sequence, including a portion of the HLA or TCR gene. By combining an engineered TALE with a DNA cleavage domain, a restriction enzyme can be produced which is specific to any desired DNA sequence, including a HLA or TCR sequence. These can then be introduced into a cell, wherein they can be used for genome editing. Boch (2011) Nature Biotech. 29: 135-6; and Boch et al. (2009) Science 326: 1509-12; Moscou et al. (2009) Science 326: 3501.

TALEs are proteins secreted by Xanthomonas bacteria. The DNA binding domain contains a repeated, highly conserved 33-34 amino acid sequence, with the exception of the 12th and 13th amino acids. These two positions are highly variable, showing a strong correlation with specific nucleotide recognition. They can thus be engineered to bind to a desired DNA sequence.

To produce a TALEN, a TALE protein is fused to a nuclease (N), which is a wild-type or mutated FokI endonuclease. Several mutations to FokI have been made for its use in TALENs; these, for example, improve cleavage specificity or activity. Cermak et al. (2011) Nucl. Acids Res. 39: e82; Miller et al. (2011) Nature Biotech. 29: 143-8; Hockemeyer et al. (2011) Nature Biotech. 29: 731-734; Wood et al. (2011) Science 333: 307; Doyon et al. (2010) Nature Methods 8: 74-79; Szczepek et al. (2007) Nature Biotech. 25: 786-793; and Guo et al. (2010) J. Mol. Biol. 200: 96.

The FokI domain functions as a dimer, requiring two constructs with unique DNA binding domains for sites in the target genome with proper orientation and spacing. Both the number of amino acid residues between the TALE DNA binding domain and the FokI cleavage domain and the number of bases between the two individual TALEN binding sites appear to be important parameters for achieving high levels of activity. Miller et al. (2011) Nature Biotech. 29: 143-8.

A HLA or TCR TALEN can be used inside a cell to produce a double-stranded break (DSB). A mutation can be introduced at the break site if the repair mechanisms improperly repair the break via non-homologous end joining. For example, improper repair may introduce a frame shift mutation. Alternatively, foreign DNA can be introduced into the cell along with the TALEN; depending on the sequences of the foreign DNA and chromosomal sequence, this process can be used to correct a defect in the HLA or TCR gene or introduce such a defect into a wt HLA or TCR gene, thus decreasing expression of HLA or TCR.

TALENs specific to sequences in HLA or TCR can be constructed using any method known in the art, including various schemes using modular components. Zhang et al. (2011) Nature Biotech. 29: 149-53; Geibler et al. (2011) PLoS ONE 6: e19509.

### Zinc finger nuclease to inhibit HLA and/or TCR

"ZFN" or "Zinc Finger Nuclease" or "ZFN to HLA and/or TCR" or "ZFN to inhibit HLA and/or TCR" refer to a zinc finger nuclease, an artificial nuclease which can be used to edit the HLA and/or TCR gene.

Like a TALEN, a ZFN comprises a FokI nuclease domain (or derivative thereof) fused to a DNA-binding domain. In the case of a ZFN, the DNA-binding domain comprises one or more zinc fingers. Carroll et al. (2011) Genetics Society of America 188: 773-782; and Kim et al. (1996) Proc. Natl. Acad. Sci. USA 93: 1156-1160.

A zinc finger is a small protein structural motif stabilized by one or more zinc ions. A zinc finger can comprise, for example, Cys2His2, and can recognize an approximately 3-bp sequence. Various zinc fingers of known specificity can be combined to produce multi-finger polypeptides which recognize about 6, 9, 12, 15 or 18-bp sequences. Various selection and modular assembly techniques are available to generate zinc fingers (and combinations thereof) recognizing specific sequences, including phage display, yeast one-hybrid systems, bacterial one-hybrid and two-hybrid systems, and mammalian cells.

Like a TALEN, a ZFN must dimerize to cleave DNA. Thus, a pair of ZFNs are required to target non-palindromic DNA sites. The two individual ZFNs must bind opposite strands of the DNA with their nucleases properly spaced apart. Bitinaite et al. (1998) Proc. Natl. Acad. Sci. USA 95: 10570-5.

Also like a TALEN, a ZFN can create a double-stranded break in the DNA, which can create a frame-shift mutation if improperly repaired, leading to a decrease in the expression and amount of HLA and/or TCR in a cell. ZFNs can also be used with homologous recombination to mutate in the HLA or TCR gene.

ZFNs specific to sequences in HLA AND/OR TCR can be constructed using any method known in the art. Cathomen et al. (2008) Mol. Ther. 16: 1200-7; and Guo et al. (2010) J. Mol. Biol. 400: 96.

### ACTIVATION AND EXPANSION OF T CELLS

Immune effector cells such as T cells may be activated and expanded generally using methods as described, for example, in U.S. Patents 6,352,694; 6,534,055; 6,905,680; 6,692,964; 5,858,358; 6,887,466; 6,905,681; 7,144,575; 7,067,318; 7,172,869; 7,232,566; 7,175,843; 5,883,223; 6,905,874; 6,797,514; 6,867,041; and U.S. Patent Application Publication No. 20060121005.

Generally, a population of immune effector cells may be expanded by contact with a surface having attached thereto an agent that stimulates a CD3/TCR complex associated signal and a ligand that stimulates a costimulatory molecule on the surface of the T cells. In particular, T cell populations may be stimulated as described herein, such as by contact with an anti-CD3 antibody, or antigen-binding fragment thereof, or an anti-CD2 antibody immobilized on a surface, or by contact with a protein kinase C activator (e.g., bryostatin) in conjunction with a calcium ionophore. For co-stimulation of an accessory molecule on the surface of the T cells, a ligand that binds the accessory molecule is used. For example, a population of T cells can be contacted with an anti-CD3 antibody and an anti-CD28 antibody, under conditions appropriate for stimulating proliferation of the T cells. To stimulate proliferation of either CD4+ T cells or CD8+ T cells, an anti-CD3 antibody and an anti-CD28 antibody. Examples of an anti-CD28 antibody include 9.3, B-T3, XR-CD28 (Diaclone, Besançon, France) can be used as can other methods commonly known in the art (Berg et al., Transplant Proc. 30(8):3975-3977, 1998; Haanen et al., J. Exp. Med. 190(9):13191328, 1999; Garland et al., J. Immunol Meth. 227(1-2):53-63, 1999).

In certain aspects, the primary stimulatory signal and the costimulatory signal for the T cell may be provided by different protocols. For example, the agents providing each signal may be in solution or coupled to a surface. When coupled to a surface, the agents may be coupled to the same surface (i.e., in "cis" formation) or to separate surfaces (i.e., in "trans" formation). Alternatively, one agent may be coupled to a surface and the other agent in solution. In one aspect, the agent providing the costimulatory signal is bound to a cell surface and the agent providing the primary activation signal is in solution or coupled to a surface. In certain aspects, both agents can be in solution. In one aspect, the agents may be in soluble form, and then cross-linked to a surface, such as a cell expressing Fc receptors or an antibody or other binding agent which will bind to the agents. In this regard, see for example, U.S. Patent Application Publication Nos. 20040101519 and 20060034810 for artificial antigen presenting cells (aAPCs) that are contemplated for use in activating and expanding T cells in the present invention.

In one aspect, the two agents are immobilized on beads, either on the same bead, i.e., "cis," or to separate beads, i.e., "trans." By way of example, the agent providing the primary activation signal is an anti-CD3 antibody or an antigen-binding fragment thereof and the agent providing the costimulatory signal is an anti-CD28 antibody or antigen-binding fragment thereof; and both agents are co-immobilized to the same bead in equivalent molecular amounts. In one aspect, a 1:1 ratio of each antibody bound to the beads for CD4+ T cell expansion and T cell growth is used. In certain aspects of the present invention, a ratio of anti CD3:CD28 antibodies bound to the beads is used such that an increase in T cell expansion is observed as compared to the expansion observed using a ratio of 1:1. In one particular aspect an increase of from about 1 to about 3 fold is observed as compared to the expansion observed using a ratio of 1:1. In one aspect, the ratio of CD3:CD28 antibody bound to the beads ranges from 100:1 to 1:100 and all integer values there between. In one aspect, more anti-CD28 antibody is bound to the particles than anti-CD3 antibody, i.e., the ratio of CD3:CD28 is less than one. In certain aspects, the ratio of anti CD28 antibody to anti CD3 antibody bound to the beads is greater than 2:1. In one particular aspect, a 1:100 CD3:CD28 ratio of antibody bound to beads is used. In one aspect, a 1:75 CD3:CD28 ratio of antibody bound to beads is used. In a further aspect, a 1:50 CD3:CD28 ratio of antibody bound to beads is used. In one aspect, a 1:30 CD3:CD28 ratio of antibody bound to beads is used. In one preferred aspect, a 1:10 CD3:CD28 ratio of antibody bound to beads is used. In one aspect, a 1:3 CD3:CD28 ratio of antibody bound to the beads is used. In yet one aspect, a 3:1 CD3:CD28 ratio of antibody bound to the beads is used.

Ratios of particles to cells from 1:500 to 500:1 and any integer values in between may be used to stimulate T cells or other target cells. As those of ordinary skill in the art can readily appreciate, the ratio of particles to cells may depend on particle size relative to the target cell. For example, small sized beads could only bind a few cells, while larger beads could bind many. In certain aspects the ratio of cells to particles ranges from 1:100 to 100:1 and any integer values in-between and in further aspects the ratio comprises 1:9 to 9:1 and any integer values in between, can also be used to stimulate T cells. The ratio of anti-CD3- and anti-CD28-coupled particles to T cells that result in T cell stimulation can vary as noted above, however certain preferred values include 1:100, 1:50, 1:40, 1:30, 1:20, 1:10, 1:9, 1:8, 1:7, 1:6, 1:5, 1:4, 1:3, 1:2, 1:1, 2:1, 3:1, 4:1, 5:1, 6:1, 7:1, 8:1, 9:1, 10:1, and 15:1 with one preferred ratio being at least 1:1 particles per T cell. In one aspect, a ratio of particles to cells of 1:1 or less is used. In one particular aspect, a preferred particle: cell ratio is 1:5. In further aspects, the ratio of particles to cells can be varied depending on the day of stimulation. For example, in one aspect, the ratio of particles to cells is from 1:1 to 10:1 on the first day and additional particles are added to the cells every day or every other day thereafter for up to 10 days, at final ratios of from 1:1 to 1:10 (based on cell counts on the day of addition). In one particular aspect, the ratio of particles to cells is 1:1 on the first day of stimulation and adjusted to 1:5 on the third and fifth days of stimulation. In one aspect, particles are added on a daily or every other day basis to a final ratio of 1:1 on the first day, and 1:5 on the third and fifth days of stimulation. In one aspect, the ratio of particles to cells is 2:1 on the first day of stimulation and adjusted to 1:10 on the third and fifth days of stimulation. In one aspect, particles are added on a daily or every other day basis to a final ratio of 1:1 on the first day, and 1:10 on the third and fifth days of stimulation. One of skill in the art will appreciate that a variety of other ratios may be suitable for use in the present invention. In particular, ratios will vary depending on particle size and on cell size and type. In one aspect, the most typical ratios for use are in the neighborhood of 1:1, 2:1 and 3:1 on the first day.

In further aspects, the cells, such as T cells, are combined with agent-coated beads, the beads and the cells are subsequently separated, and then the cells are cultured. In an alternative aspect, prior to culture, the agent-coated beads and cells are not separated but are cultured together. In a further aspect, the beads and cells are first concentrated by application of a force, such as a magnetic force, resulting in increased ligation of cell surface markers, thereby inducing cell stimulation.

By way of example, cell surface proteins may be ligated by allowing paramagnetic beads to which anti-CD3 and anti-CD28 are attached (3x28 beads) to contact the T cells. In one aspect the cells (for example, 10⁴ to 10⁹ T cells) and beads (for example, DYNABEADS® M-450 CD3/CD28 T paramagnetic beads at a ratio of 1:1) are combined in a buffer, for example PBS (without divalent cations such as, calcium and magnesium). Again, those of ordinary skill in the art can readily appreciate any cell concentration may be used. For example, the target cell may be very rare in the sample and comprise only 0.01% of the sample or the entire sample (i.e., 100%) may comprise the target cell of interest. Accordingly, any cell number is within the context of the present invention. In certain aspects, it may be desirable to significantly decrease the volume in which particles and cells are mixed together (i.e., increase the concentration of cells), to ensure maximum contact of cells and particles. For example, in one aspect, a concentration of about 2 billion cells/ml is used. In one aspect, greater than 100 million cells/ml is used. In a further aspect, a concentration of cells of 10, 15, 20, 25, 30, 35, 40, 45, or 50 million cells/ml is used. In yet one aspect, a concentration of cells from 75, 80, 85, 90, 95, or 100 million cells/ml is used. In further aspects, concentrations of 125 or 150 million cells/ml can be used. Using high concentrations can result in increased cell yield, cell activation, and cell expansion. Further, use of high cell concentrations allows more efficient capture of cells that may weakly express target antigens of interest, such as CD28-negative T cells. Such populations of cells may have therapeutic value and would be desirable to obtain in certain aspects. For example, using high concentration of cells allows more efficient selection of CD8+T cells that normally have weaker CD28 expression.

In one instance, cells transduced with a nucleic acid encoding a CAR, e.g., a CAR described herein (e.g., RCAR, RNKR-CAR, or NKR-CAR), are expanded, e.g., by a method described herein. In one instance, the cells (e.g., expressing a RCAR and a NKR-CAR, or expressing a RNKR-CAR) are expanded in culture for a period of several hours (e.g., about 2, 3, 4, 5, 6, 7, 8, 9, 10, 15, 18, 21 hours) to about 14 days (e.g., 1, 2, 3, 4, 5, 6, 7, 8, 9, 10, 11, 12, 13 or 14 days). In one instance, the cells are expanded for a period of 4 to 9 days. In one instance, the cells are expanded for a period of 8 days or less, e.g., 7, 6 or 5 days. In one instance, the cells are expanded in culture for 5 days, and the resulting cells are more potent than the same cells expanded in culture for 9 days under the same culture conditions. Potency can be defined, e.g., by various T cell functions, e.g. proliferation, target cell killing, cytokine production, activation, migration, or combinations thereof. In one instance, the cells expanded for 5 days show at least a one, two, three or four fold increase in cells doublings upon antigen stimulation as compared to the same cells expanded in culture for 9 days under the same culture conditions. In one instance, the cells are expanded in culture for 5 days, and the resulting cells exhibit higher proinflammatory cytokine production, e.g., IFN-γ and/or GM-CSF levels, as compared to the same cells expanded in culture for 9 days under the same culture conditions. In one instance, the cells expanded for 5 days show at least a one, two, three, four, five, ten fold or more increase in pg/ml of proinflammatory cytokine production, e.g., IFN-γ and/or GM-CSF levels, as compared to the same cells expanded in culture for 9 days under the same culture conditions.

Several cycles of stimulation may also be desired such that culture time of T cells can be 60 days or more. Conditions appropriate for T cell culture include an appropriate media (e.g., Minimal Essential Media or RPMI Media 1640 or, X-vivo 15, (Lonza)) that may contain factors necessary for proliferation and viability, including serum (e.g., fetal bovine or human serum), interleukin-2 (IL-2), insulin, IFN-γ, IL-4, IL-7, GM-CSF, IL-10, IL-12, IL-15, TGFβ, and TNF-α or any other additives for the growth of cells known to the skilled artisan. Other additives for the growth of cells include, but are not limited to, surfactant, plasmanate, and reducing agents such as N-acetyl-cysteine and 2-mercaptoethanol. Media can include RPMI 1640, AIM-V, DMEM, MEM, α-MEM, F-12, X-Vivo 15, and X-Vivo 20, Optimizer, with added amino acids, sodium pyruvate, and vitamins, either serum-free or supplemented with an appropriate amount of serum (or plasma) or a defined set of hormones, and/or an amount of cytokine(s) sufficient for the growth and expansion of T cells. Antibiotics, e.g., penicillin and streptomycin, are included only in experimental cultures, not in cultures of cells that are to be infused into a subject. The target cells are maintained under conditions necessary to support growth, for example, an appropriate temperature (e.g., 37° C) and atmosphere (e.g., air plus 5% CO₂).

In one embodiment, the cells are expanded in an appropriate media (e.g., media described herein) that includes one or more interleukin that result in at least a 200-fold (e.g., 200-fold, 250-fold, 300-fold, 350-fold) increase in cells over a 14 day expansion period, e.g., as measured by a method described herein such as flow cytometry. In one embodiment, the cells are expanded in the presence IL-15 and/or IL-7 (e.g., IL-15 and IL-7).

T cells that have been exposed to varied stimulation times may exhibit different characteristics. For example, typical blood or apheresed peripheral blood mononuclear cell products have a helper T cell population (TH, CD4+) that is greater than the cytotoxic or suppressor T cell population (TC, CD8+). Ex vivo expansion of T cells by stimulating CD3 and CD28 receptors produces a population of T cells that prior to about days 8-9 consists predominately of TH cells, while after about days 8-9, the population of T cells comprises an increasingly greater population of TC cells. Accordingly, depending on the purpose of treatment, infusing a subject with a T cell population comprising predominately of TH cells may be advantageous. Similarly, if an antigen-specific subset of TC cells has been isolated it may be beneficial to expand this subset to a greater degree.

Further, in addition to CD4 and CD8 markers, other phenotypic markers vary significantly, but in large part, reproducibly during the course of the cell expansion process. Thus, such reproducibility enables the ability to tailor an activated T cell product for specific purposes.

### EVALUATION OF EFFICACY

Candidate RCARs can be generated using the components and methods described herein. Such candidate RCARs can be tested for efficacy in vivo by administering candidate RCARs into mouse models of cancer and monitoring and assessing anti-cancer or anti-tumor effect and overall survival of the mice.

By way of example, the efficacy of an RCAR having an antigen binding domain that comprises an anti-human CD19 antibody can be assayed in a mouse model of cancer, e.g., a CD19/ALL mouse model. Primary human acute lymphoblastic leukemia (ALL) cells are implanted, e.g., intravenously, in immune compromised mice, e.g., NOD.Cg-Prkdc^{scid} Il2rg^{tm1Wjl}/SzJ (NSG or NOD scid gamma) mice. After a period of time sufficient for establishment of ALL, e.g., 2-3 weeks, candidate RCAR-expressing cells can be administered. Following treatment with the candidate RCAR-expressing cells, the mice are analyzed, e.g., weekly, for disease progression, tumor burden, infiltration and/or persistence of RCAR-expressing cells, using various methods known in the art. For example, the percentage of human ALL cells, e.g., human CD19+ cells in the blood, to indicate disease burden. Overall survival, e.g. morbidity, of the mice after treatment can also be assessed.

Various assays can be used to evaluate the activity of the RCAR molecule, such as but not limited to, the ability to expand T cells following antigen stimulation, sustain T cell expansion in the absence of re-stimulation, and anti-cancer activities in appropriate animal models. Assays to evaluate the effects of the RCAR, e.g., an EGFRvIII RCAR, are described in further detail below.

Western blot analysis of RCAR expression in primary T cells can be used to detect their presence using published methods for CARs. See, e.g., Milone et al., Molecular Therapy 17(8): 1453-1464 (2009). Very briefly, T cells (1:1 mixture of CD4⁺ and CD8⁺ T cells) expressing the RCARs are expanded in vitro for more than 10 days followed by lysis and SDS-PAGE under reducing conditions. RCARs containing the full length TCR-ζ cytoplasmic domain and the endogenous TCR-ζ chain are detected by western blotting using an antibody to the TCR-ζ chain. The same T cell subsets are used for SDS-PAGE analysis under non-reducing conditions to permit evaluation of covalent dimer formation.

In vitro expansion of RCAR⁺ T cells (i.e., RCART cells) following antigen stimulation can be measured by flow cytometry. For example, a mixture of CD4⁺ and CD8⁺ T cells are stimulated with αCD3/αCD28 aAPCs followed by transduction with lentiviral vectors expressing GFP under the control of the promoters to be analyzed. Exemplary promoters include the CMV IE gene, EF-1α, ubiquitin C, or phosphoglycerokinase (PGK) promoters. GFP fluorescence is evaluated on day 6 of culture in the CD4⁺ and/or CD8⁺ T cell subsets by flow cytometry. See, e.g., Milone et al., Molecular Therapy 17(8): 1453-1464 (2009). Alternatively, a mixture of CD4⁺ and CD8⁺ T cells are stimulated with αCD3/αCD28 coated magnetic beads on day 0, and transduced with RCAR on day 1 using a bicistronic lentiviral vector expressing RCAR along with eGFP using a 2A ribosomal skipping sequence. Cultures are re-stimulated with RCAR constructs in the presence of antiCD3 and anti-CD28 antibody (K562-BBL-3/28) following washing. Exogenous IL-2 is added to the cultures every other day at 100 IU/ml. GFP⁺ T cells are enumerated by flow cytometry using bead-based counting. See, e.g., Milone et al., Molecular Therapy 17(8): 1453-1464 (2009).

Sustained RCAR⁺ T cell expansion in the absence of re-stimulation can also be measured. See, e.g., Milone et al., Molecular Therapy 17(8): 1453-1464 (2009). Briefly, mean T cell volume (fl) is measured on day 8 of culture using a Coulter Multisizer III particle counter following stimulation with αCD3/αCD28 coated magnetic beads on day 0, and transduction with the indicated RCAR on day 1.

Assessment of cell proliferation and cytokine production has been previously described, e.g., at Milone et al., Molecular Therapy 17(8): 1453-1464 (2009). Briefly, assessment of RCAR-mediated proliferation is performed in microtiter plates by mixing washed T cells with target cells, such as U87MG, BHK or CHO cells expressing a tumor antigen, e.g., EGFRvIII or EGFR wildtype (wt) or CD32 and CD137 (KT32-BBL) for a final T-cell:target cell ratio of 1:1. Anti-CD3 (clone OKT3) and anti-CD28 (clone 9.3) monoclonal antibodies are added to cultures with KT32-BBL cells to serve as a positive control for stimulating T-cell proliferation since these signals support long-term CD8⁺ T cell expansion ex vivo. T cells are enumerated in cultures using CountBright™ fluorescent beads (Invitrogen, Carlsbad, CA) and flow cytometry as described by the manufacturer. RCAR⁺ T cells are identified by GFP expression using T cells that are engineered with eGFP-2A linked RCAR-expressing lentiviral vectors. For RCAR+ T cells not expressing GFP, the RCAR+ T cells are detected with biotinylated recombinant protein, e.g., EGFRvIII and a secondary avidin-PE conjugate. CD4+ and CD8⁺ expression on T cells are also simultaneously detected with specific monoclonal antibodies (BD Biosciences). Cytokine measurements are performed on supernatants collected 24 hours following re-stimulation using the human TH1/TH2 cytokine cytometric bead array kit (BD Biosciences, San Diego, CA) according the manufacturer's instructions. Fluorescence is assessed using a FACScalibur flow cytometer, and data is analyzed according to the manufacturer's instructions.

Cytotoxicity can be assessed by a standard ⁵¹Cr-release assay. See, e.g., Milone et al., Molecular Therapy 17(8): 1453-1464 (2009). Briefly, target cells (e.g., U87MG, BHK or CHO cells expressing RCAR, e.g., EGFRvIII or EGFR wildtype (wt) are loaded with ⁵¹Cr (as NaCrO₄, New England Nuclear, Boston, MA) at 37°C for 2 hours with frequent agitation, washed twice in complete RPMI and plated into microtiter plates. Effector T cells are mixed with target cells in the wells in complete RPMI at varying ratios of effector cell:target cell (E:T). Additional wells containing media only (spontaneous release, SR) or a 1% solution of triton-X 100 detergent (total release, TR) are also prepared. After 4 hours of incubation at 37°C, supernatant from each well is harvested. Released ⁵¹Cr is then measured using a gamma particle counter (Packard Instrument Co., Waltham, MA). Each condition is performed in at least triplicate, and the percentage of lysis is calculated using the formula: % Lysis = (ER- SR) / (TR - SR), where ER represents the average ⁵¹Cr released for each experimental condition. Alternative cytotoxicity assays may also be used, such as flow based cytotoxicity assays. Other assays, including those described in the Example section herein as well as those that are known in the art can also be used to evaluate the RCAR constructs.

Analogous methods can be used to testRNKR-CARs, e.g., to evaluate efficacy of RNKR-CARs. Analogous methods can also be used to test the combination of RCAR and inhNKR-CAR, e.g., in a cell, e.g., to evaluate the efficacy and/or safety of the combination of CARs.

### THERAPEUTIC APPLICATION

Methods for inhibiting the proliferation or reducing acancer in a cancer antigen-expressing cell population, e.g., an EGFRvIII-expressing cell population, are disclosed herein. In certain instances, the immune effector cell engineered to express a RCAR and a NKR-CAR (e.g., RCAR/NKR-CARX cells, e.g., RCAR/NKR-CART cells, RCAR/NKR-CARN cells) or to express a RNKR-CAR(e.g., RNKR-CARX cells) reduces the quantity, number, amount or percentage of cells and/or cancer cells by at least 25%, at least 30%, at least 40%, at least 50%, at least 65%, at least 75%, at least 85%, at least 95%, or at least 99% in a subject with a cancer associated with antigen-expressing cells relative to a negative control. In an instance, the subject is a human.

Methods disclosed herein includes a type of cellular therapy where immune effector cells are genetically modified to express RCAR and NKR-CAR, or RNKR-CAR. The resulting CARX cells (e.g., RCAR/NKR-CARX cells, or RNKR-CARX cells) are infused into a recipient in need thereof. The infused RCAR/NKR-CARX cell, or RNKR-CARX cell is able to kill or inhibit tumor cells in the recipient. Unlike antibody therapies, RCAR/NKR-CARX cells or RNKR-CARX cells are able to replicate in vivo resulting in long-term persistence that can lead to sustained tumor control. In various instances, the RCAR/NKR-CARX cells or RNKR-CARX cells, administered to the patient, or their progeny, persist in the patient for at least four months, five months, six months, seven months, eight months, nine months, ten months, eleven months, twelve months, thirteen months, fourteen month, fifteen months, sixteen months, seventeen months, eighteen months, nineteen months, twenty months, twenty-one months, twenty-two months, twenty-three months, two years, three years, four years, or five years after administration of the RCAR/NKR-CARX cells or RNKR-CARX cells, to the patient.

Without wishing to be bound by any particular theory, the anti-cancer immune response elicited by the RCAR/NKR-CARX cells or RNKR-CARX cells may be an active or a passive immune response, or alternatively may be due to a direct vs. indirect immune response. In an embodiment, the RCAR/NKR-CARX cells or RNKR-CARX cells exhibit specific proinflammatory cytokine secretion and potent cytolytic activity in response to human cancer cells expressing the target antigen, resist soluble RCAR or RNKR-CAR inhibition, mediate bystander killing and mediate regression of an established human tumor. In an embodiment, the RCAR/NKR-CARX cells or RNKR-CARX cells may be a type of vaccine for ex vivo immunization and/or in vivo therapy in a mammal. In an embodiment, the mammal is a human.

In embodiments, with respect to ex vivo immunization, at least one of the following occurs in vitro prior to administering the cell into a mammal: i) expansion of the cells, ii) introducing a nucleic acid encoding a RCAR, NKR-CAR, RCAR/NKR-CAR, or RNKR-CAR to the cells or iii) cryopreservation of the RCARX, RCAR/NKR-CARX, or RNKR-CARX cells. Ex vivo procedures are known in the art and are discussed more fully below. Briefly, cells are isolated from a mammal (e.g., a human) and genetically modified (i.e., transduced or transfected in vitro) with a vector expressing a RCAR, NKR-CAR, RCAR/NKR-CAR, or RNKR-CAR disclosed herein. The resulting RCAR/NKR-CARX cells or RNKR-CARX cells can be administered to a mammalian recipient to provide a therapeutic benefit. The mammalian recipient may be a human and the RCAR/NKR-CARX cells or RNKR-CARX cells can be autologous with respect to the recipient. Alternatively, the cells can be allogeneic, syngeneic or xenogeneic with respect to the recipient.

A procedure for ex vivo expansion of hematopoietic stem and progenitor cells is described in U.S. Pat. No. 5,199,942, can be applied to the cells described herein. Other suitable methods are known in the art therefore the methods disclosed herein are not limited to any particular method of ex vivo expansion of the cells. Briefly, ex vivo culture and expansion of T cells comprises: (1) collecting CD34+hematopoietic stem and progenitor cells from a mammal from peripheral blood harvest or bone marrow explants; and (2) expanding such cells ex vivo. In addition to the cellular growth factors described in U.S. Pat. No. 5,199,942, other factors such as flt3-L, IL-1, IL-3 and c-kit ligand, can be used for culturing and expansion of the cells.

In addition to using a cell-based vaccine in terms of ex vivo immunization, also provided are compositions and methods for in vivo immunization to elicit an immune response directed against an antigen in a patient.

Generally, the cells activated and expanded as described herein may be utilized in the treatment and prevention of diseases that arise in individuals who are immunocompromised. In particular, the RCAR/NKR-CARX cells or RNKR-CARX cells are used in the treatment of diseases, disorders and conditions associated with expression of a tumor antigen. In certain instances, the RCAR/NKR-CARX cells or RNKR-CARX cells are used in the treatment of patients at risk for developing diseases, disorders and conditions associated with expression of tumor antigen. Thus, the present disclosure provides methods for the treatment or prevention of diseases, disorders and conditions associated with expression of tumor antigen comprising administering to a subject in need thereof, a therapeutically effective amount of RCAR/NKR-CAR modified cells or RNKR-CAR modified cells.

The RCAR/NKR-CARX cells or RNKR-CARX cells may be administered either alone, or as a pharmaceutical composition in combination with diluents and/or with other components such as IL-2 or other cytokines or cell populations.

In one aspect, the present invention relates to treatment of a subject *in vivo* using a CAR (e.g., RCAR, NKR-CAR, or RNKR-CAR) such that growth of cancerous tumors is inhibited. A CAR (e.g., RCAR, NKR-CAR, or RNKR-CAR) may be used alone to inhibit the growth of cancerous tumors. Alternatively, CAR (e.g., RCAR, NKR-CAR, or RNKR-CAR) may be used in conjunction with other CARs (e.g., RCAR, NKR-CAR, or RNKR-CAR), immunogenic agents, standard cancer treatments, or other antibodies.

In another aspect, a method of treating a subject, *e.g.,* reducing or ameliorating, a hyperproliferative condition or disorder (*e.g.,* a cancer), *e.g.,* solid tumor, a soft tissue tumor, or a metastatic lesion, in a subject is disclosed. As used herein, the term "cancer" is meant to include all types of cancerous growths or oncogenic processes, metastatic tissues or malignantly transformed cells, tissues, or organs, irrespective of histopathologic type or stage of invasiveness. Cancers that may be treated include tumors that are not vascularized, or not yet substantially vascularized, as well as vascularized tumors. The cancers may comprise non-solid tumors (such as hematological tumors, for example, leukemias and lymphomas) or may comprise solid tumors. Types of cancers to be treated with the CARs (e.g., RCARs, NKR-CARs, or RNKR-CARs) of the disclosure include, but are not limited to, carcinoma, blastoma, and sarcoma, and certain leukemia or lymphoid malignancies, benign and malignant tumors, and malignancies *e.g.,* sarcomas, carcinomas, and melanomas. Adult tumors/cancers and pediatric tumors/cancers are also included. In one embodiment, the cancer to be treated is a solid tumor, e.g., a solid tumor described herein. In another embodiment, the cancer to be treated is a hematologic cancer.

Hematologic cancers are cancers of the blood or bone marrow. Examples of hematological (or hematogenous) cancers include leukemias, including acute leukemias (such as acute lymphocytic leukemia, acute myelocytic leukemia, acute myelogenous leukemia and myeloblastic, promyelocytic, myelomonocytic, monocytic and erythroleukemia), chronic leukemias (such as chronic myelocytic (granulocytic) leukemia, chronic myelogenous leukemia, and chronic lymphocytic leukemia), polycythemia vera, lymphoma, Hodgkin's disease, non-Hodgkin's lymphoma (indolent and high grade forms), multiple myeloma, Waldenstrom's macroglobulinemia, heavy chain disease, myelodysplastic syndrome, hairy cell leukemia and myelodysplasia.

Solid tumors are abnormal masses of tissue that usually do not contain cysts or liquid areas. Solid tumors can be benign or malignant. Different types of solid tumors are named for the type of cells that form them (such as sarcomas, carcinomas, and lymphomas). Examples of solid tumors include but are not limited to sarcomas and carcinomas, include fibrosarcoma, myxosarcoma, liposarcoma, chondrosarcoma, osteosarcoma, and other sarcomas, synovioma, mesothelioma, Ewing's tumor, leiomyosarcoma, rhabdomyosarcoma, colon carcinoma, lymphoid malignancy, pancreatic cancer, breast cancer, lung cancers, ovarian cancer, prostate cancer, hepatocellular carcinoma, squamous cell carcinoma, basal cell carcinoma, adenocarcinoma, sweat gland carcinoma, medullary thyroid carcinoma, papillary thyroid carcinoma, pheochromocytomas sebaceous gland carcinoma, papillary carcinoma, papillary adenocarcinomas, medullary carcinoma, bronchogenic carcinoma, renal cell carcinoma, hepatoma, bile duct carcinoma, choriocarcinoma, Wilms' tumor, cervical cancer, testicular tumor, seminoma, bladder carcinoma, melanoma (e.g., an advanced stage melanoma), and CNS tumors (such as a glioma (such as brainstem glioma and mixed gliomas), glioblastoma (also known as glioblastoma multiforme) astrocytoma, CNS lymphoma, germinoma, medulloblastoma, Schwannoma craniopharyogioma, ependymoma, pinealoma, hemangioblastoma, acoustic neuroma, oligodendroglioma, menangioma, neuroblastoma, retinoblastoma and brain metastases).

In embodiments, solid tumors include malignancies, *e.g.,* sarcomas, adenocarcinomas, and carcinomas, of the various organ systems, such as those affecting liver, lung, breast, lymphoid, gastrointestinal (*e.g.,* colon), genitourinary tract (*e.g.,* renal, urothelial cells), prostate and pharynx. Adenocarcinomas include malignancies such as most colon cancers, rectal cancer, renal-cell carcinoma, liver cancer, non-small cell carcinoma of the lung, cancer of the small intestine and cancer of the esophagus.

In embodiments, cancers that can be treated include bone cancer, pancreatic cancer, skin cancer, cancer of the head or neck, cutaneous or intraocular malignant melanoma, uterine cancer, ovarian cancer, rectal cancer, cancer of the anal region, stomach cancer, testicular cancer, uterine cancer, carcinoma of the fallopian tubes, carcinoma of the endometrium, carcinoma of the cervix, carcinoma of the vagina, carcinoma of the vulva, Hodgkin's Disease, non-Hodgkin's lymphoma, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, chronic or acute leukemias including acute myeloid leukemia, chronic myeloid leukemia, acute lymphoblastic leukemia, chronic lymphocytic leukemia, solid tumors of childhood, lymphocytic lymphoma, cancer of the bladder, cancer of the kidney or ureter, carcinoma of the renal pelvis, neoplasm of the central nervous system (CNS), primary CNS lymphoma, tumor angiogenesis, spinal axis tumor, brain stem glioma, pituitary adenoma, Kaposi's sarcoma, epidermoid cancer, squamous cell cancer, T-cell lymphoma, environmentally induced cancers including those induced by asbestos, and combinations of said cancers. Treatment of metastatic cancers, *e.g.,* metastatic cancers that express PD-L1 (Iwai et al. (2005) Int. Immunol. 17:133-144) can be effected using the CAR molecules described herein. In instances, metastatic lesions of the aforementioned cancers can be treated or prevented using the methods and compositions of the disclosure.

Exemplary cancers whose growth can be inhibited include cancers typically responsive to immunotherapy. Non-limiting examples of cancers for treatment include melanoma (e.g., metastatic malignant melanoma), renal cancer (e.g. clear cell carcinoma), prostate cancer (e.g. hormone refractory prostate adenocarcinoma), breast cancer, colon cancer and lung cancer (e.g. non-small cell lung cancer). Additionally, refractory or recurrent malignancies can be treated using the molecules described herein.In one embodiment, the subject can be administered an agent which reduces or ameliorates a side effect associated with the administration of a CAR-expressing cell (e.g., RNKR-CARX cell or RCAR/NKR-CARX cell).

Side effects associated with the administration of a CAR-expressing cell include, but are not limited to CRS, and hemophagocytic lymphohistiocytosis (HLH), also termed Macrophage Activation Syndrome (MAS). Symptoms of CRS include high fevers, nausea, transient hypotension, hypoxia, and the like. CRS may include clinical constitutional signs and symptoms such as fever, fatigue, anorexia, myalgias, arthalgias, nausea, vomiting, and headache. CRS may include clinical skin signs and symptoms such as rash. CRS may include clinical gastrointestinal signs and symptoms such as nausea, vomiting and diarrhea. CRS may include clinical respiratory signs and symptoms such as tachypnea and hypoxemia. CRS may include clinical cardiovascular signs and symptoms such as tachycardia, widened pulse pressure, hypotension, increased cardiac output (early) and potentially diminished cardiac output (late). CRS may include clinical coagulation signs and symptoms such as elevated d-dimer, hypofibrinogenemia with or without bleeding. CRS may include clinical renal signs and symptoms such as azotemia. CRS may include clinical hepatic signs and symptoms such as transaminitis and hyperbilirubinemia. CRS may include clinical neurologic signs and symptoms such as headache, mental status changes, confusion, delirium, word finding difficulty or frank aphasia, hallucinations, tremor, dymetria, altered gait, and seizures. Accordingly, the methods described herein can comprise administering a CAR-expressing cell described herein to a subject and further administering one or more agents to manage elevated levels of a soluble factor resulting from treatment with a CAR-expressing cell. In one embodiment, the soluble factor elevated in the subject is one or more of IFN-γ, TNFα, IL-2 and IL-6. In an embodiment, the factor elevated in the subject is one or more of IL-1, GM-CSF, IL-10, IL-8, IL-5 and fraktalkine. Therefore, an agent administered to treat this side effect can be an agent that neutralizes one or more of these soluble factors. In one embodiment, the agent that neutralizes one or more of these soluble forms is an antibody or antigen binding fragment thereof. Examples of such agents include, but are not limited to a steroid (e.g., corticosteroid), an inhibitor of TNFα, and an inhibitor of IL-6. An example of a TNFα inhibitor is an anti-TNFa antibody molecule such as, infliximab, adalimumab, certolizumab pegol, and golimumab. Another example of a TNFα inhibitor is a fusion protein such as entanercept. Small molecule inhibitor of TNFα include, but are not limited to, xanthine derivatives (e.g. pentoxifylline) and bupropion. An example of an IL-6 inhibitor is an anti-IL-6 antibody molecule such as tocilizumab (toc), sarilumab, elsilimomab, CNTO 328, ALD518/BMS-945429, CNTO 136, CPSI-2364, CDP6038, VX30, ARGX-109, FE301, and FM101. In one embodiment, the anti-IL-6 antibody molecule is tocilizumab. An example of an IL-1R based inhibitor is anakinra.

In one embodiment, the antigen binding moiety portion of the RCAR/NKR-CARX (e.g., RCAR/NKR-CART cells of the invention is designed to treat a particular cancer. In one embodiment, the RCAR/NKR-CARX cells of the invention are modified to express a first activating RCAR targeting a first antigen and a second inhNKR-CAR targeting a second antigen, where the first antigen is expressed on a particular tumor or cancer and the second antigen is not expressed on a particular tumor or cancer. In this manner, conditional activation of immune effector cells, e.g., T cells, is generated by engagement of the RCAR (e.g., bearing an antigen binding domain targeting an antigen on the malignant cell of interest) and the inhNKR-CAR (e.g., bearing an antigen binding domain directed against an antigen that is present on normal, but not malignant tissue) provides inhibition of the activating signal from the RCAR when the RCAR/NKR-CARX cell encounters normal cells. Examples of antigens that serve as useful targets for inhibitory CARs (e.g., inhNKR-CARs) include the ephrin receptors (Pasquale, 2010, Nat Rev Cancer 10(3):165-80) and claudins (Singh et al., 2010, J Oncol, 2010:541957), which are expressed by epithelial cells from normal tissues, but often selectively lost by cancers (e.g. EPHA7).

### PHARMACEUTICAL COMPOSITIONS AND TREATMENTS

Pharmaceutical compositions may comprise a RCAR/NKR-CARX cells e.g., RCAR/NKR-CART cells or RCAR/NKR-CARN cells, or RNKR-CARX cells (e.g., RNKR-CART or RNKR-CARN cells) in combination with one or more pharmaceutically or physiologically acceptable carriers, diluents or excipients. Such compositions may comprise buffers such as neutral buffered saline, phosphate buffered saline and the like; carbohydrates such as glucose, mannose, sucrose or dextrans, mannitol; proteins; polypeptides or amino acids such as glycine; antioxidants; chelating agents such as EDTA or glutathione; adjuvants (e.g., aluminum hydroxide); and preservatives. In an embodiment, the pharmaceutical compositions are formulated for intravenous administration.

Pharmaceutical compositions may be administered in a manner appropriate to the disease to be treated (or prevented). The quantity and frequency of administration will be determined by such factors as the condition of the patient, and the type and severity of the patient's disease, although appropriate dosages may be determined by clinical trials.

When "an immunologically effective amount," "an anti-cancer effective amount," "a cancer-inhibiting effective amount," or "therapeutic amount" is indicated, the precise amount of the compositions to be administered can be determined by a physician with consideration of individual differences in age, weight, disease state, e.g., tumor size, extent of infection or metastasis, and condition of the patient (subject). In instances, a pharmaceutical composition comprising the RCAR/NKR-CARX cells (e.g., RCAR/NKR-CART cells or RCAR/NKR-CARN cells) or RNKR-CARX cells (e.g., RNKR-CART or RNKR-CARN cells) described herein may be administered at a dosage of 10⁴ to 10⁹ cells/kg body weight, in some instances 10⁵ to 10⁶ cells/kg body weight, including all integer values within those ranges. T cell compositions may also be administered multiple times at these dosages.

In certain instances RCAR/NKR-CARX cells (e.g., RCAR/NKR-CART cells or RCAR/NKR-CARN cells) or RNKR-CARX cells (e.g., RNKR-CART or RNKR-CARN cells) are activated and expanded to therapeutic levels, and are administered to a patient by using infusion techniques that are commonly known in immunotherapy (see, e.g., Rosenberg et al., New Eng. J. of Med. 319:1676, 1988). The optimal dosage and treatment regime for a particular patient can be determined by one skilled in the art of medicine by monitoring the patient for signs of disease and adjusting the treatment accordingly.

In certain instances, it may be desired to draw blood (or have an apheresis performed), activate and genetically modify the T cells therefrom according to the present disclosure, and reinfuse the patient with these activated and expanded genetically modified T cells. This process can be carried out multiple times every few weeks. In certain instances, T cells can be activated from blood draws of from 10cc to 400cc. In certain instances, T cells are activated from blood draws of 20cc, 30cc, 40cc, 50cc, 60cc, 70cc, 80cc, 90cc, or 100cc. Not to be bound by theory, using this multiple blood draw/multiple reinfusion protocol may serve to select out certain populations of T cells.

The administration of the subject compositions may be carried out in any convenient manner, including by aerosol inhalation, injection, ingestion, transfusion, implantation or transplantation. The compositions described herein may be administered to a patient subcutaneously, intradermally, intratumorally, intranodally, intramedullary, intramuscularly, by intravenous (i.v.) injection, or intraperitoneally. In one embodiment, the T cell compositions of the present invention are administered to a patient by intradermal or subcutaneous injection. In another embodiment, the T cell compositions of the present invention are preferably administered by i.v. injection. The compositions of T cells may be injected directly into a tumor, lymph node, or site of infection.

In one instance, the CAR (e.g., RNKR-CAR, RCAR, and/or NKR-CAR) is introduced into immune effector cells (e.g., T cells, NK cells), e.g., using in vitro transcription, and the subject (e.g., human) receives an initial administration of CAR immune effector cells (e.g., T cells, NK cells) of the disclosure, and one or more subsequent administrations of the CAR immune effector cells (e.g., T cells, NK cells) of the disclosure, wherein the one or more subsequent administrations are administered less than 15 days, e.g., 14, 13, 12, 11, 10, 9, 8, 7, 6, 5, 4, 3, or 2 days after the previous administration. In one embodiment, more than one administration of the CAR immune effector cells (e.g., T cells, NK cells) of the invention are administered to the subject (e.g., human) per week, e.g., 2, 3, or 4 administrations of the CAR immune effector cells (e.g., T cells, NK cells) of the invention are administered per week. In one embodiment, the subject (e.g., human subject) receives more than one administration of the CAR immune effector cells (e.g., T cells, NK cells) per week (e.g., 2, 3 or 4 administrations per week) (also referred to herein as a cycle), followed by a week of no CAR immune effector cells (e.g., T cells, NK cells) administrations, and then one or more additional administration of the CAR immune effector cells (e.g., T cells, NK cells) (e.g., more than one administration of the CAR immune effector cells (e.g., T cells, NK cells) per week) is administered to the subject. In another embodiment, the subject (e.g., human subject) receives more than one cycle of CAR immune effector cells (e.g., T cells, NK cells), and the time between each cycle is less than 10, 9, 8, 7, 6, 5, 4, or 3 days. In one embodiment, the CAR immune effector cells (e.g., T cells, NK cells) are administered every other day for 3 administrations per week. In one embodiment, the CAR immune effector cells (e.g., T cells, NK cells) of the invention are administered for at least two, three, four, five, six, seven, eight or more weeks.

In embodiments, the RCAR/NKR-CARX cells or RNKR-CARX cells with RCARs or RNKR-CARs comprising one or more switch domains, generate an intracellular signal that promotes an immune effector response in the presence of a dimerization molecule, e.g., a small molecule heterodimerization molecule, e.g., RAD001 or AP21967.

The administration of the dimerization molecule may be carried out in any convenient manner, including by aerosol inhalation, injection, ingestion, transfusion, or implantation. In an embodiment the dimerization molecule is administered orally. The dimerization molecule may be administered to a patient transarterially, subcutaneously, intradermally, intratumorally, intranodally, intramedullary, intramuscularly, by intravenous (i.v.) injection, or intraperitoneally. In an embodiment, the dimerization molecule is administered orally, e.g., in tablet form. In an embodiment, the dimerization molecule is administered by intradermal or subcutaneous injection. In an embodiment, an embodiment the dimerization molecule is administered by i.v. injection.

In an instance, the dimerization molecule is administered after the RCAR/NKR-CARX cells or RNKR-CARX cells have been infused into the patient. In one instance, the dimerization molecule is administered one day after the RCAR/NKR-CARX cells or RNKR-CARX cells have been infused into the patient. In one embodiment, the dimerization molecule is administered 2, 3, 4, 5, 6, 7, 8, 9, 10,11, 12,13, 14,15, 16,17, 18, 19, 20,21,22, 23, 24, 25, 26, 27, 28,29, or 30 days after the RCAR/NKR-CARX cells or RNKR-CARX cells have been infused into the patient. In an instance the dimerization molecule is administered after administration of the RCAR/NKR-CARX cells or RNKR-CARX cells, e.g., on or after 1, 2, 3, 4, 5, 6 ,7, 8, 9, 10, 11, 12,16, 17,18, 19, 20, 21, 22, or 23 hours, or on or after 1, 2, 3, 4, 5, 6, 7 or 8 days, after administration of the RCAR/NKR-CARX cells or RNKR-CARX cells. In one instance, the dimerization molecule is administered more than once to the after the RCAR/NKR-CARX cells or RNKR-CARX cells have been infused into the patient, e.g., based on a dosing schedule tailored for the patient, e.g., administration of the dimerization molecule on a bi-weekly, weekly, monthly, 6-monthly, yearly basis. In an instance, dosing of the dimerization molecule will be daily, every other day, twice a week, or weekly, but in instances will not exceed 5 mg, 10 mg, 15 mg, 20 mg, 30 mg, 40 mg, or 50 mg, weekly. In an instance, the dimerization molecule is dosed continuously, e.g. by use of a pump, e.g., a wearable pump. In an instance continuous administration lasts for at least 4 hours, 12 hours, 24 hours, 2 days, 3 days, 4 days or 5 days. In an instance, a FKBP-FRB heterodimerization molecule, e.g., rapamycin, or a rapalog, e.g., AP21967 or RAD001, is administered at a dose of no greater than about 0.5 mg in a 24 hr period.

In an instance a dimerization molecule is administered at the same time, e.g., on the same day, as the administration of the RCAR/NKR-CARX cells or RNKR-CARX cells.

In an instance, the patient is monitored after the dimerization molecule has been administered for a decrease in cancer. If the cancer reappears, the dimerization molecule can be readministered at that time. In an instance, a subject will undergo additional or subsequent, e.g., second, third or fourth, RCAR/NKR-CARX or RNKR-CARX cell infusions, e.g., at weekly or monthly intervals, or as determined to be needed. In an instance, a subsequent administration is accompanied with, or followed by, administration of the dimerization molecule. In an instance subsequent administration of RCAR/NKR-CARX cells or RNKR-CARX cells, or dimerization molecule continues, e.g., until tumor burden is cleared, no additional benefit is perceived, or a preselected criterion is met. In an instance, a method disclosed herein comprises administration of cellular therapy wherein T cells are genetically modified to express a chimeric antigen receptor (CAR), e.g., a RCAR, NKR-CAR, and/or RNKR-CAR. The CAR T cell is infused to a recipient in need thereof. The infused cell is able to kill tumor cells in the recipient but only in the presence of the dimerization molecule. In addition, in the presence of the dimerization molecule, RCAR/NKR-CART cells or RNKR-CART cells will expand and replicate in vivo upon engagement of their target antigen which will lead to sustained tumor control. Cytokine release during tumor cell killing may also be measured in the serum.

This expansion and cytokine production can be measured in the patient by routine blood draws and subsequent analysis of CAR expression and serumcytokine levels. This method will also inform one skilled in the art to modify dosing strategy of the dimerization molecule to maintain the functional RCAR/NKR-CART or RNKR-CART cell population. It is envisioned that dosing of the dimerization molecule will continue as long as tumor burden is being reduced.

In further instances, the RCAR/NKRX cells or RNKR-CARX cells may be used in a treatment regimen in combination with chemotherapy, radiation, immunosuppressive agents, such as cyclosporin, azathioprine, methotrexate, mycophenolate, and FK506, antibodies, or other immunoablative agents such as CAMPATH, anti-CD3 antibodies or other antibody therapies, cytoxin, fludarabine, cyclosporin, FK506, rapamycin, mycophenolic acid, steroids, FR901228, cytokines, and irradiation. Drugs that inhibit either the calcium dependent phosphatase calcineurin (cyclosporine and FK506) or inhibit the p70S6 kinase that is important for growth factor induced signalling (rapamycin). (Liu et al., Cell 66:807-815, 1991; Henderson et al., Immun. 73:316-321, 1991; Bierer et al., Curr. Opin. Immun. 5:763-773, 1993) can also be used.

In a further embodiment, the cell compositions are administered to a patient in conjunction with (e.g., before, simultaneously or following) bone marrow transplantation, T cell ablative therapy using either chemotherapy agents such as, fludarabine, external-beam radiation therapy (XRT), cyclophosphamide, or antibodies such as OKT3 or CAMPATH. In an embodiment, the cell compositions are administered following B-cell ablative therapy such as agents that react with CD20, e.g., Rituxan. For example, in an embodiment, subjects may undergo standard treatment with high dose chemotherapy followed by peripheral blood stem cell transplantation. In certain embodiments, following the transplant, subjects receive an infusion of the expanded immune cells described herein. In an instance where RCAR/NKRX cells or RNKR-CARX cells are administered post-transplant, the immune effector cells, e.g., T cells, used to make the RCAR/NKRX cells or RNKR-CARX cells, are obtained from the subject after transplant. In an instance, the immune effector cells, e.g., T cells, used to make the RCAR/NKRX cells or RNKR-CARX cells, are of donor origin, e.g., they are derived from donor cells implanted in the subject..

In an additional instance, expanded cells are administered before or following surgery. In an instance, RCAR/NKRX cells or RNKR-CARX cells are administered to the subject after surgery that debulks the tumor.

In a particular exemplary instance, subjects may undergo leukapheresis, wherein leukocytes are collected, enriched, or depleted ex vivo to select and/or isolate the cells of interest, e.g., T cells. These T cell isolates may be expanded by methods known in the art and treated such that one or more RCAR, NKR-CAR, or RNKR-CAR constructs of the disclosed herein may be introduced, thereby creating RCAR/NKR-CART or RNKR-CART cell. Subjects in need thereof may subsequently undergo standard treatment with high dose chemotherapy followed by peripheral blood stem cell transplantation. In certain instances, following or concurrent with the transplant, subjects receive an infusion of the expanded RCAR/NKR-CART or RNKR-CART cells disclosed herein. In an additional instance, expanded cells are administered before or following surgery.

### ADJUNCTIVE TREATMENT WITH A LOW, IMMUNE ENHANCING, DOSE OF AN MTOR INHIBITOR.

As used herein, the term "mTOR inhibitor" refers to a compound or ligand, or a pharmaceutically acceptable salt thereof, which inhibits the mTOR kinase in a cell. In an instance an mTOR inhibitor is an allosteric inhibitor. In an instance an mTOR inhibitor is a catalytic inhibitor.

The administration of a low, immune enhancing dose of an mTOR inhibitor can be combined with the administration of RCAR/NKR-CARX or RNKR-CARX cells described herein, e.g., immune effector cells (e.g., T cells or NK cells) engineered to express a Regulatable Chimeric Antigen Receptor (RCAR) and a NKR-CAR, or to express a RNKR-CAR.

Administration of a low, immune enhancing, dose of an mTOR inhibitor can optimize the performance of immune effector cells in the subject. Depending on the timing and dosage of the mTOR inhibitor, the performance of harvested T cells, non-harvested T cells, or both can be optimized.

While not wishing to be bound by theory, it is believed that treatment with a low, immune enhancing, dose (e.g., a dose that is insufficient to completely suppress the immune system but sufficient to improve immune function) is accompanied by a decrease in PD-1 positive T cells or an increase in PD-1 negative cells, at least transiently, as compared to a non-treated subject. PD-1 positive T cells, but not PD-1 negative T cells, can be exhausted by engagement with cells which express a PD-1 ligand, e.g., PD-L1 or PD-L2. In addition or alternatively, again while not wishing to be bound by theory, it is believed that a low, immune enhancing, dose of an mTOR inhibitor can increase naive T cell numbers, e.g., at least transiently, e.g., as compared to a non-treated subject. Alternatively or additionally, again while not wishing to be bound by theory, it is believed that treatment with an mTOR inhibitor after a sufficient amount of time or sufficient dosing results in one or more of the following:
an increase in the expression of one or more of the following markers: CD62L^{high}, CD127^{high}, CD27⁺, and BCL2, e.g., on memory T cells, e.g., memory T cell precursors;
a decrease in the expression of KLRG1, e.g., on memory T cells, e.g., memory T cell precursors; and
an increase in the number of memory T cell precursors, e.g., cells with any one or combination of the following characteristics: increased CD62L^{high}, increased CD127^{high}, increased CD27⁺, decreased KLRG1, and increased BCL2;
and wherein any of the changes described above occurs, e.g., at least transiently, e.g., as compared to a non-treated subject.

Memory T cell precursors are memory T cells that are early in the differentiation program. For example, memory T cells have one or more of the following characteristics: increased CD62L^{high}, increased CD127^{high}, increased CD27⁺, decreased KLRG1, and/or increased BCL2.

In one instance, the cells expressing a CAR molecule, e.g., a CAR molecule described herein, are administered in combination with a low, immune enhancing dose of an mTOR inhibitor, e.g., an allosteric mTOR inhibitor, including rapalogs such as RAD001.

In an instance, administration of a low, immune enhancing, dose of an mTOR inhibitor, e.g., an allosteric inhibitor, e.g., RAD001, or a catalytic inhibitor, is initiated or completed prior to administration (e.g., prior to harvest or after harvest of the immune effector cells, e.g., T cells engineered to express a RCAR and NKR-CAR, or T cells engineered to express a RNKR-CAR, but prior to admistration of the RCAR/NKR-CARX or RNKR-CARX cells) of a RCAR/NKR-CARX or RNKR-CARX cell described herein, e.g., an immune effector cells, e.g., T cells, engineered to express a RCAR and NKR-CAR or to express RNKR-CAR, or is initiated or completed after administration of a RCAR/NKR-CARX or RNKR-CARX cell described herein.

In an instance, a dose of an mTOR inhibitor is associated with, or provides, mTOR inhibition of at least 5 but no more than 90%, at least 10 but no more than 90%, at least 15, but no more than 90%, at least 20 but no more than 90%, at least 30 but no more than 90%, at least 40 but no more than 90%, at least 50 but no more than 90%, at least 60 but no more than 90%, at least 70 but no more than 90%, at least 5 but no more than 80%, at least 10 but no more than 80%, at least 15, but no more than 80%, at least 20 but no more than 80%, at least 30 but no more than 80%, at least 40 but no more than 80%, at least 50 but no more than 80%, at least 60 but no more than 80%, at least 5 but no more than 70%, at least 10 but no more than 70%, at least 15, but no more than 70%, at least 20 but no more than 70%, at least 30 but no more than 70%, at least 40 but no more than 70%, at least 50 but no more than 70%, at least 5 but no more than 60%, at least 10 but no more than 60%, at least 15, but no more than 60%, at least 20 but no more than 60%, at least 30 but no more than 60%, at least 40 but no more than 60%, at least 5 but no more than 50%, at least 10 but no more than 50%, at least 15, but no more than 50%, at least 20 but no more than 50%, at least 30 but no more than 50%, at least 40 but no more than 50%, at least 5 but no more than 40%, at least 10 but no more than 40%, at least 15, but no more than 40%, at least 20 but no more than 40%, at least 30 but no more than 40%, at least 35 but no more than 40%, at least 5 but no more than 30%, at least 10 but no more than 30%, at least 15, but no more than 30%, at least 20 but no more than 30%, or at least 25 but no more than 30%.

In an instance, a dose of an mTOR inhibitor is associated with, or provides, mTOR inhibition of at least 1, 2, 3, 4 or 5 but no more than 20%, at least 1, 2, 3, 4 or 5 but no more than 30%, at least 1, 2, 3, 4 or 5, butno more than 35, at least 1, 2, 3, 4 or 5 butno more than 40%, or at least 1, 2, 3, 4 or 5 but no more than 45%.

In an instance, a dose of an mTOR inhibitor is associated with, or provides, mTOR inhibition of at least 1, 2, 3, 4 or 5 but no more than 90%.

As is discussed herein, the extent of mTOR inhibition can be expressed as the extent of P70 S6 kinase inhibition, e.g., the extent of mTOR inhibition can be determined by the level of decrease in P70 S6 kinase activity, e.g., by the decrease in phosphorylation of a P70 S6 kinase substrate. The level of mTOR inhibition can be evaluated by a method described herein, e.g. by the Boulay assay, or measurement of phosphorylated S6 levels by western blot.

mTOR phosphorylates the kinase P70 S6, thereby activating P70 S6 kinase and allowing it to phosphorylate its substrate. The extent of mTOR inhibition can be expressed as the extent of P70 S6 kinase inhibition, e.g., the extent of mTOR inhibition can be determined by the level of decrease in P70 S6 kinase activity, e.g., by the decrease in phosphorylation of a P70 S6 kinase substrate. One can determine the level of mTOR inhibition, by measuring P70 S6 kinase activity (the ability of P70 S6 kinase to phsophorylate a substrate), in the absence of inhibitor, e.g., prior to administration of inhibitor, and in the presences of inhibitor, or after the administration of inhibitor. The level of inhibition of P70 S6 kinase gives the level of mTOR inhibition. Thus, if P70 S6 kinase is inhibited by 40%, mTOR activity, as measured by P70 S6 kinase activity, is inhibited by 40%. The extent or level of inhibition referred to herein is the average level of inhibition over the dosage interval. By way of example, if the inhibitor is given once per week, the level of inhibition is given by the average level of inhibition over that interval, namely a week.

Boulay et al., Cancer Res, 2004, 64:252-61, teaches an assay that can be used to assess the level of mTOR inhibition (referred to herein as the Boulay assay). In an instance, the assay relies on the measurement of P70 S6 kinase activity from biological samples before and after administration of an mTOR inhibitor, e.g., RAD001. Samples can be taken at preselected times after treatment with an mTOR ihibitor, e.g., 24, 48, and 72 hours after treatment. Biological samples, e.g., from skin or peripheral blood mononuclear cells (PBMCs) can be used. Total protein extracts are prepared from the samples. P70 S6 kinase is isolated from the protein extracts by immunoprecipitation using an antibody that specifically recognizes the P70 S6 kinase. Activity of the isolated P70 S6 kinase can be measured in an in vitro kinase assay. The isolated kinase can be incubated with 40S ribosomal subunit substrates (which is an endogenous substrate of P70 S6 kinase) and gamma-³²P under conditions that allow phosphorylation of the substrate. Then the reaction mixture can be resolved on an SDS-PAGE gel, and ³²P signal analyzed using a PhosphorImager. A ³²P signal corresponding to the size of the 40S ribosomal subunit indicates phosphorylated substrate and the activity of P70 S6 kinase. Increases and decreases in kinase activity can be calculated by quantifying the area and intensity of the ³²P signal of the phosphorylated substrate (e.g., using ImageQuant, Molecular Dynamics), assigning arbitrary unit values to the quantified signal, and comparing the values from after administration with values from before administration or with a reference value. For example, percent inhibition of kinase activity can be calculated with the following formula: 1-(value obtained after administration/value obtained before administration) X 100. As described above, the extent or level of inhibition referred to herein is the average level of inhibition over the dosage interval.

Methods for the evaluation of kinase activity, e.g., P70 S6 kinase activity, are also provided in US 7,727,950.

The level of mTOR inhibition can also be evaluated by a change in the ratio of PD1 negative to PD1 positive T cells. T cells from peripheral blood can be identified as PD1 negative or positive by art-known methods.

### EXAMPLES

The disclosure is further described in detail by reference to the following experimental examples. These examples are provided for purposes of illustration only, and are not intended to be limiting unless otherwise specified. Thus, the disclosure should in no way be construed as being limited to the following examples, but rather, should be construed to encompass any and all variations which become evident as a result of the teaching provided herein.

Without further description, it is believed that one of ordinary skill in the art can, using the preceding description and the following illustrative examples, make and utilize the compounds of the present disclosure and practice the claimed methods. The following working examples specifically point out various aspects of the present disclosure, and are not to be construed as limiting in any way the remainder of the disclosure.

### Example 1: Regulatable CAR using a Rapalogue Switch

This example illustrates an important general concept underlying embodiments of regulatable chimeric antigen receptors (RCARs), that is based on the separation of an antigen binding member ("binding event") from an intracellular signaling member ("signaling event"). In the presence of a dimerization molecule, e.g., a small molecule, the switch domains of the antigen binding member and the intracellular signaling member associate and trigger signal transduction in the now associated RCAR molecule. By way of example, an extracellular antigen binding domain, such as a scFv, is fused to a transmembrane domain and a first switch domain (e.g., a switch domain from FKBP or FRB) of the dimerization switch (e.g., a heterodimerization switch). The intracellular signaling domain comprises a second switch domain (e.g., FRB or FKBP) of the heterodimerization switch and one or more intracellular signaling domains such as 4-1BB and CD3zeta. Dimerization and initiation of the signaling cascade was achieved by the addition of a small molecule heterodimerizer ("heterodimerization molecule" because the switch domains are not the same) which links the extracellular binding domain to the intracellular signaling domain. In this example the small molecule inducing dimerization can be rapamycin or analogs thereof (termed "rapalogue"). The rapamycin or rapalogues function by binding with high affinity to FKBP and to the FRB domain of mTOR, thereby acting as a heterodimerizer to induce complex formation Choi, J., et al(1996) Structure of the FKBP12-rapamycin complex interacting with the binding domain of human FRAP Science 273: 239-42).

The following examples illustrate that the dimerization switch can be on the inside or the outside of the cell.

For illustrative purposes only, the EGFRvIII scFv fragment termed "139" was used as an extracellular antigen binding domain to generate the RCARs. This scFv is derived from a human antibody to EGFRvIII (Morgan et al., 2012 Hum Gene Ther 23(10): 1043-53). To generate a RCAR, a pair of constructs was generated and co-expressed in the target immune effector cell. The various switch domains of the heterodimerization switch can be linked to different domains of the RCAR construct.

"Switch 1" comprises a pair of constructs. The first construct was designed in which the antigen binding domain (139 scFv) was constructed by fusing a leader sequence to the 139 scFv followed by a hinge region, a transmembrane region, a linker and the first intracellular switch domain - FRB (SEQ ID NO: 3). The second construct was designed by fusing the second switch domain - FKBP to a second linker and the signaling domains 4-1BB followed by CD3zeta (SEQ ID NO: 4).

"Switch 2" comprises a pair of constructs. The first construct was designed in which the antigen binding domain was constructed by fusing a leader sequence to the 139 scFv followed by a hinge region, a transmembrane region, a linker, and the first intracellular switch domain - FKBP (SEQ ID NO: 5). The corresponding intracellular signaling construct was designed by fusing the second switch domain - FRB to a second linker and the intracellular signaling domains 4-1BB followed by CD3zeta (SEQ ID NO: 6).

The EGFRvIII CAR sequence for 139 scFv was cloned with the signaling domains for 4-1BB and CD3 zeta. The non-regulatable CAR construct (139scFv-BBZ, SEQ ID NO: 7) is expressed from the pELNS vector for lentivirus production and is used in the subsequent experiments as a control.

### Structure 1: AP21967

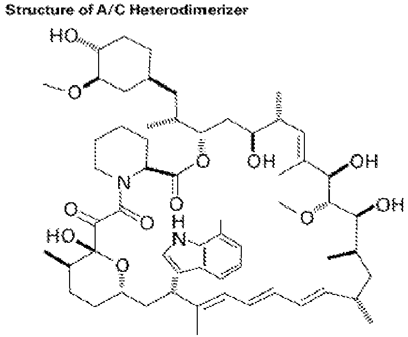

### Materials and Methods

### Surface expression of EGFRvIII CAR constructs and staining by FACS

Jurkat E6 cells were electroporated with the various EGFRvIII CAR constructs or the 139 CAR control vector using Amaxa Cell Line Nucleofector Kit V (Lonza, Colgne AG, Germany) and program X-001. One day after the transfection, 0.5x10⁶ cells were placed into each well of a V-shape 96 well plate (Greiner Bio-One, Germany) in 0.2 ml FACS buffer (DPBS buffer containing 5% FBS) and incubated for 10 minutes at room temperature. Cells were then spun down and resuspended in 0.2 ml of the FACS buffer with 100 nM of EGFRvIII-Fc and incubated at 4°C for 60 minutes. Cells were then washed with FACS buffer two times, and incubated with 0.2 ml of the FACS buffer with 1 µl of PE antihuman IgG Fc (Jackson ImmunoResearch Laboratories, West Grove, PA) for 30 minutes at 4°C in the dark. After washing with 0.2 ml of FACS buffer two times, cells were analyzed on a LSRII (BD Biosciences, San Jose, CA) machine using the FACSDiva software (BD Biosciences, San Jose, CA). Immunofluorescence staining was analyzed as the relative log fluorescence of live cells, and the percentage of the PE positive cells were measured.

### Generation of Jurkat reporter cell line for initial characterization of CAR function

As an alternative to primary T cell transduction and activation, a Jurkat-NFAT reporter cell line can be used to evaluate the functional activity of CAR constructs. The Jurkat T cell line (E6-1) was transfected with a NFAT-luciferase reporter construct and a stable, clonal cell line Jurkat cells with NFAT-LUC reporter (JNL), was selected for further characterization based on strong induction of the NFAT reporter following PMA and ionomycin stimulation.

### Transfection of Jurkat reporter cell line and activation of NFAT by switch 1 or switch 2

Jurkat cells with NFAT-LUC reporter (JNL) were grown to the density of 0.5 x 10⁶/ml in Jurkat cell growth media with puromycin at 0.5 µg/ml. For each transfection 2.5 x 10⁶ cells were spin down at 100g for 10 minutes. Two µg of DNA per construct were used per transfection. Amaxa Nucleofector solution V and supplement I was mixed and 100 µl was added into the tube with DNA construct. The mixture was then added to the cells and transferred to the electroporation cuvette. Electroporation was done under setting X-001 using Amaxa Nucleofector II Device. 0.5 ml of growth media was added immediately after eletroporation and the mixture were transferred into 2 ml growth media in one well of the 6-well plate. After one hour, A/Z compound was applied at the final concentration of 500 nM. The cells were incubated in the 37°C incubator with 5% CO₂ overnight for 18 hrs. Tissue culture plate was coated with 5 µg/ml of EGFRvIII-Fc or IgG1-Fc for 2 hrs, blocked with the blocking buffer (DPBS with 5% serum) for 1 hour. The transfected cells with or without A/Z compound were resuspended and added to the target plate with 100 µl per well and incubated for 18 hrs. Luciferase One Glo reagent 100 µl was added per well. The samples were incubated for 5 min at 37°C and then luminescence is measured using a luminometer.

### Dose response of rapalogue on NFAT activation

The ability of RCAR constructs to demonstrate rapalogue dependent signal activation following target antigen engagement of the antigen binding domain was measured with the Jurkat cells with NFAT-LUC reporter (JNL) reporter cell line. Specifically, JNL were grown to the density of 0.5 x 10⁶/ml in Jurkat cell growth media with puromycin at 0.5 µg/ml. For each transfection 2.5 x 10⁶ cells were spin down at 100g for 10 minutes. Two µg of DNA per construct were used per transfection. Amaxa Nucleofector solution V and supplement I was mixed and 100 µl was added into the tube with DNA construct. The mixture was then added to the cells and transferred to the electroporation cuvette. Electroporation was done under setting X-001 using Amaxa Nucleofector II Device. 0.5 ml of growth media was added immediately after eletroporation and the mixture were transferred into 2 ml growth media in one well of the 6-well plate. After one hour, the rapalogue compound at various concentrations was added to cells. The cells were incubated in the 37°C incubator with 5% CO₂ overnight for 18 hrs. Tissue culture plate was coated with 5 µg/ml of EGFRvIII-Fc or IgG1-Fc for 2 hrs, blocked with the blocking buffer (DPBS with 5% serum) for 1 hour. The transfected cells were added to the target plate with 100 µl per well and incubated further for 18 hrs. Luciferase One Glo reagent 100 µl was added per well. The samples were incubated for 5 min at 37°C and then luminescence is measured using a luminometer.

### Results

### Jurkat reporter assay to test target mediated activity of the rapalogue regulated CAR-EGFRvIII

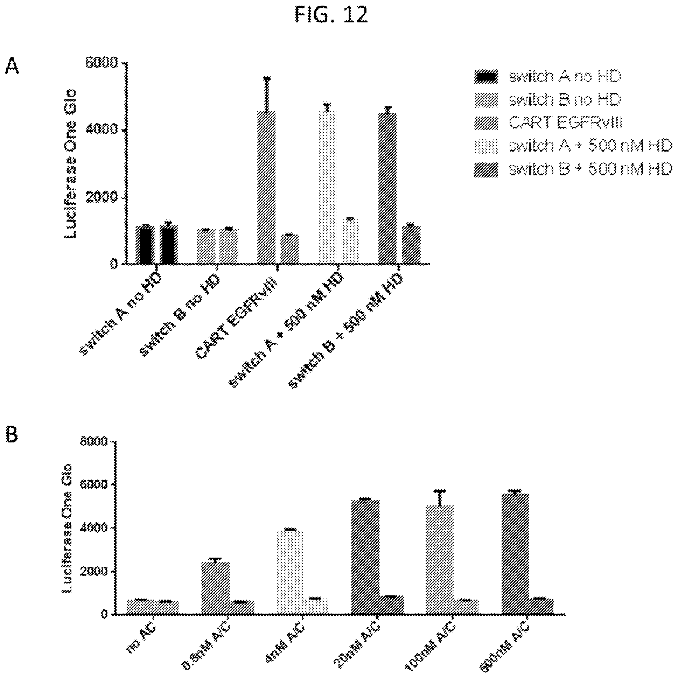

Collectively, these experiments demonstrate that various components of a RCAR can be engineered separately and combined in the presence of a dimerization molecule to activate signaling in the RCAR._Surface expression of the scFv was assessed by flow cytometry and was shown to be about -50% for all the constructs (data not shown). The JNL-RCAR-EGFRvIII cells were then stimulated with or without rapalogue (heterodimerization molecule) demonstrating that the RCAR constructs expressed on the surface of the cells. JNL parental cells and JNL cells expressing a control CAR were included as additional controls. Fig. 12A shows the results from studies designed to investigate the signaling events between the RCAR expressing the antigen binding domain, e.g., scFv, and RCAR expressing the intracellular signaling domains in the presence of a heterodimerization molecule. The data shows significant target regulated activation upon stimulation with the rapalogue equal to the control RCAR. Furthermore, no significant on-target activation was observed in the absence of the rapalogue, indicating a switch mediated by the small molecule regulated heterodimerization of the switch domains (FKBP and FRB). Finally, no activation was observed against a control target (IgG1-Fc only) (open bars). These data demonstrate specificity of the RCAR constructs for EGFRvIII target in the presence of the rapalogue only, and lack of cross-reactivity to control target or in the absence of rapalogue. These data show for the first time that RCAR constructs can be regulated with a heterodimerization switch.

To determine the dose response, rapaloque ("A/C heterodimerizer" AP21967), at various concentrations, was applied to the transfected cells that were cotransfected with EGFRvIII clone 139-CD8alphaTM-FKBP (SEQ ID NO: 5, construct 66) and FRB-4-1BB-CD3zeta (SEQ ID NO: 6, construct 67) and NFAT activation was measured. EGFRvIII 139scFv-BBz (SEQ ID NO: 7) was used as a control.

As shown in Fig. 12B, the fold activation of NFAT remained relatively constant from a concentration of 500 nM to as low as 20 nM. Surprisingly, even at 0.8 nM concentration of rapalogue, there is still 2-3 fold of induction of NFAT mediated by the target. The decrease in fold activation was likely due to a decrease in the extent of dimerized 4-1BB-CD3 zeta, while the expression was quite comparable throughout the various concentrations of the rapalogue treatment. This result indicated that rapalogue is potent in mediating the dimerization of FKRP and FBP in the context of a heterodimerization switch used in RCARs. One would expect to see similar results when the switches are reversed such that the FKBP is on the intracellular signaling domain and the FRB is on the 139 scFv antigen binding domain. As in Fig. 12A, there was no activation observed against a control target IgG1-Fc (second set of bars for each pair of bars).

The concept, methods and compositions described in this example are applicable to RNKR-CARs. The concept, methods and compositions described in this example are applicable to RCAR/NKR-CARX cells and/or RNKR-CARX cells.

### Example 2: Regulatable CAR using a Coumermycin Switch

The following example illustrates the use of coumermycin regulated dimerization (Farrar, M. A. et al, 1996, nature. 383,: 178-181) to activate a RCAR construct. In this example the switch is designed by fusing a leader sequence to the 139 scFv followed by a hinge region, a transmembrane region, a linker and the first intracellular switch domain - GyrB (SEQ ID NO: 8). The second construct was designed by fusing the second switch domain GyrB to a second linker and the intracellular signaling domains 4-1BB followed by CD3zeta (SEQ ID NO: 9). As the switch domain are the same, this dimerization switch is referred to as "homodimerization switch." Signal activation of the T cell will be regulated by addition of the small molecule coumermycin (Structure 2).

### Structure 2; Coumermycin

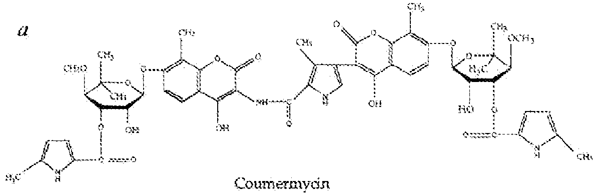

### Materials and Methods

### Synthesis of DNA for regulatable CAR using the coumermycin switch

Coumermycin is commercially available from several vendors. The sequence for the 139 scFv was cloned with the signaling domains for 4-1BB and CD3 zeta. The non-regulatable CAR construct, 139scFv-BBZ, SEQ ID: 7, can be used as a control. For the coumermycin RCAR, the 139 scFv was cloned with a transmembrane domain followed by the GyrB switch domain at the c-terminus (SEQ ID NO:8) and the corresponding activation construct made by fusing GyrB to a linker and the signaling domains 4-1BB followed by CD3zeta (SEQ ID NO:9). Jurkat assays were performed essentially as described in Example 1, with the exception that the final step involves incubating the transfected cells for 18 hrs in the presence of varying concentrations of coumermycin. Luciferase One Glo reagent 100 µl was added per well. The samples were incubated for 5 min at 37°C and then luminescence was measured using a luminometer.

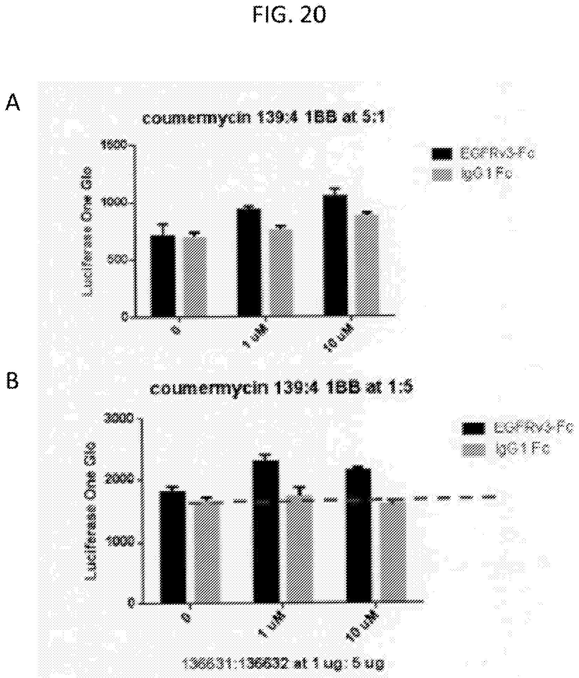

Fig. 20A and 20B depict the effect of addition of coumermycin on signal at two different ratios of antigen binding member:intracellular signaling member (5:1 and 1:5). Addition of coumerycin resulted in a significant increase in signal at both ImM and 10 mM, at both ratios.

The concept, methods and compositions described in this example are applicable to RNKR-CARs. The concept, methods and compositions described in this example are applicable to RCAR/NKR-CARX cells and/or RNKR-CARX cells.

### Example 3: Regulatable CAR using a Gibberellin Switch

The following example illustrates the use of gibberellin mediated heterodimerization to activate a RCAR construct (Takafumi M et al, 2012. Nat Chem Biol.; 8(5):465-470). To generate a RCAR, a pair of constructs was generated and co-expressed in the target cell. The various heterodimerization domains of the switch domains can be linked to different domains of the RCAR construct.

"Switch 1" comprises a pair of constructs. The first construct was designed in by fusing a leader sequence to the 139 scFv followed by a hinge region, a transmembrane region, a linker and the first intracellular switch domain - GAI (SEQ ID NO: 10). The corresponding second construct was designed by fusing the second switch domain -GID1, to a second linker and the signaling domains 4-1BB followed by CD3zeta (SEQ ID NO: 11).

"Switch 2" comprises a pair of constructs. The first construct was designed by fusing a leader sequence to the scFv followed by a hinge region, a transmembrane region, a linker and the first intracellular switch domain - GID1 (SEQ ID NO: 12). The corresponding second construct was designed by fusing the second switch domain -GAI, to a second linker and the intracellular signaling domains 4-1BB followed by CD3zeta (SEQ ID NO: 13).

Induction of heterodimerization switch is achieved by addition of Gibberellic Acid Acetoxymethyl Ester (structure 3, commercially available from Toronto Research Chemicals, Inc)

### Structure 3: Gibberellic Acid Acetoxymethyl Ester

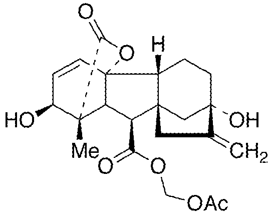

### Materials and Methods

### Synthesis of DNA for regulatable CAR using the coumermycin switch

Gibberellic Acid Acetoxymethyl Ester is commercially available from Toronto Research Chemicals, Inc. The sequence for the 139 scFv will be cloned with the signaling domains for 4-1BB and CD3 zeta. The non-regulatable CAR construct, 139scFv-BBZ, SEQ ID: 7, can be used as a control. For the Gibberellin RCAR,
"Switch 1" comprises a pair of constructs. In the first construct the 139 scFv will be cloned with the GAI-switch domain at the c-terminus (SEQ ID NO:10) and the corresponding second construct designed by fusing the GID1-switch domain to a linker and the intracellular signaling domains 4-1BB followed by CD3zeta (SEQ ID NO:11).

"Switch 2" comprises a pair of constructs. In the first construct, the 139 scFv will be cloned with the GID1-switch domain at the c-terminus (SEQ ID NO:12) and the corresponding second construct designed by fusing the GAI-switch domain to a linker and the intracellular signaling domains 4-1BB followed by CD3zeta (SEQ ID NO:13).

Jurkat assays were performed as described in Example 1, with the exception that the final step involved incubating the transfected cells for 18 hrs in the presence of varying concentrations of gibberellic acid acetoxymethyl ester or giberellic acid. Luciferase One Glo reagent 100 µl was added per well. The samples were incubated for 5 min at 37°C and then luminescence was measured using a luminometer.

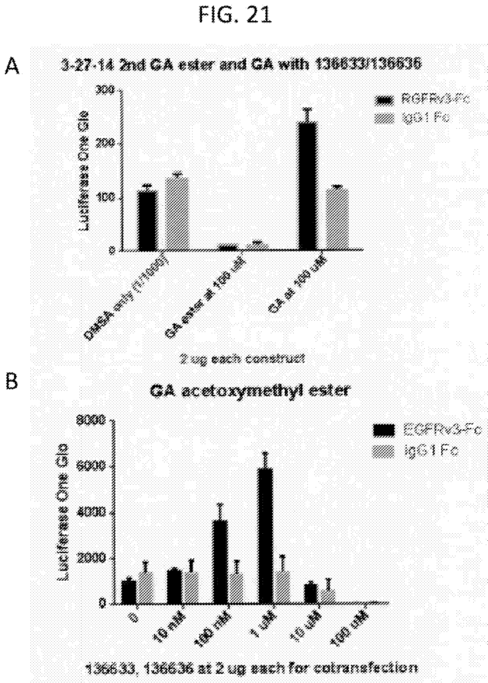

Fig. 21A shows the effect of DMSA, gibberellic acid acetoxymethyl ester, and giberellic acid on NFAT expression.

Fig. 21B shows the response of NFAT expression to increasing dose of gibberellic acid acetoxymethyl ester.

The concept, methods and compositions described in this example are applicable to RNKR-CARs. The concept, methods and compositions described in this example are applicable to RCAR/NKR-CARX cells and/or RNKR-CARX cells.

### Example 4: Internal Covalent Switch

The use of specific covalent cross-linking agents as alternatives for heterodimerization has recently been described (Erhart et al., 2013 Chem Biol 20(4): 549-557), although not in the context of RCARs. In embodiments, these agents, designated HaXS, can overcome potential kinetic limitations related to off rates and need for accumulation of non-covalent molecules in the cell as prerequisites to activation of the required signal cascades for T-cell mediated killing. HaXS contain functional groups for linking a Halo-Tag (see, e.g., SEQ ID NO:14) with a SNAP-Tag (see e.g., SEQ ID NO:15) along with a cell penetrating core. Evaluation of HaXS molecules in the context of regulatable RCARs will be performed using EGFRvIII RCAR (139 scFv) as a model system. See, e.g., Fig. 13.

### Materials and Methods

### Synthesis of HaXS and DNA for regulatable CAR

A representative HaXS (Structure 5) will be chemically synthesized as described by Erhart *et. Al supra.* The sequence for the 139 scFv will be cloned with the intracellular signaling domains for 4-1BB and CD3 zeta. The non-regulatable CAR construct, 139scFv-BBZ, SEQ ID: 7, will be used as a control. For the HaXs RCAR, the various heterodimerization domains of the switch domains can be linked to different domains of the RCAR construct.

"Switch 1" comprises a pair of constructs. In the first construct, the 139 scFV will be cloned with the SNAP-tag at the c-terminus (SEQ ID NO:16) and the corresponding second construct designed by fusing the Halo-Tag to a linker and the intracellular signaling domains 4-1BB followed by CD3zeta (SEQ ID NO:17). "Switch 2" comprises a pair of constructs. In the first construct, the 139 scFV will be cloned with the Halo-tag at the c-terminus (SEQ ID NO:18) and the corresponding second construct designed by fusing the SNAP-Tag to a linker and the intracellular signaling domains 4-1BB followed by CD3zeta (SEQ ID NO:19). Jurkat assays will be performed as described in Example 1, with the exception that the final step will involve incubating the transfected cells for 18 hrs in the presence of varying concentrations of HaXS. Luciferase One Glo reagent 100 µl will be added per well. The samples will be incubated for 5 min at 37°C and then luminescence will be measured using a luminometer.

### Structure 5; HaXS

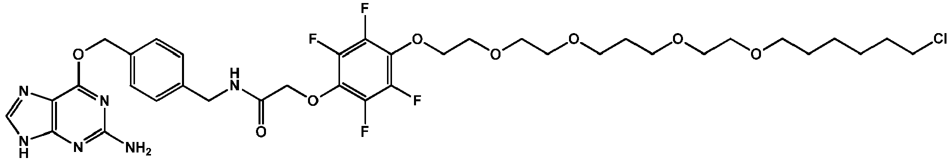

The concept, methods and compositions described in this example are applicable to RNKR-CARs. The concept, methods and compositions described in this example are applicable to RCAR/NKR-CARX cells and/or RNKR-CARX cells.

### Example 5: Peptide Based Dimerization Switch - Myc Tag

In embodiments, T cell-mediated cell death requires the linking of an external binding event to the signaling activation cascade in the intracellular space, see, e.g., Fig. 6. Co-localization and clustering of these intracellular signaling domains via an externally displayed switch domain (e.g., c-myc peptide tag) and a soluble ligand with the target cell bound receptor/scFv (e.g., anti-myc antibody) is allows for induction of the desired signaling response. The extracellular switch domain can be any tag molecule such as c-myc peptide tag, flag peptide tag, HA peptide tag or V5 peptide tag, that interacts with the appropriate dimerization molecule. In these cases the dimerization molecule acts as a crosslinking reagent, and can include antibodies against the peptide tag molecules comprised by the switch domain. The extracellular domain can also be scFvs against cancer antigens, such as scFv (e.g., 139) against EGFRvIII antigen. In this case the crosslinking reagent can be antigen itself such as EGFRvIII-Fc molecule. When a switch domain acting as a single crosslinking reagent, such as an anti-tag antibody, is insufficient to form clusters and activate a signal, a further crosslinking reagent can be used. This can be antibodies against anti-tag antibodies, or antibodies against Fc to further crosslink EGFRvIII-Fc. Specifically, in this example, the switch is an external homodimerization switch and the first and second switch domains are myc tag peptides that are linked to the intracellular transmembrane and intracellular signaling domains. The antigen binding domain, e.g., 139 scFv, does not have a switch domain and serves only to bind to the target cancer cell. In this example, signaling is initiated by the addition of a crosslinking rabbit polyclonal myc antibody (i.e., a homodimerization molecule) that binds to the each of the external first and second myc peptide switch domains causing them to cluster. This clustering of the external homodimerization switch causes the intracellular signaling domains to cluster and activate signaling - see Fig. 14A. Further enhancement of the intracellular signaling event is achieved by increasing the external co-clustering with the addition of further cross-linking antibodies. In this example, further clustering is achieved by the addition of an anti-rabbit antibody against the rabbit polyclonal myc antibody as shown in Fig. 14B.

### Methods and materials

RCAR with flag or c-myc peptide tag displayed on the cell surface will be designed by fusing the corresponding sequences to a linker and the intracellular signaling domains 4-1BB followed by CD3zeta (SEQ ID NO: 20 and 21, respectively). The non-regulatable CAR construct, 139scFv-BBZ, SEQ ID: 7, will be used as a control. Jurkat cells with NFAT-LUC reporter (JNL) were grown to the density of 0.5 x 10⁶/ml in Jurkat cell growth media with puromycin at 0.5 µg/ml. For each transfection 2.5 x 10⁶ cells were spun down at 100g for 10 minutes. Two µg of DNA per construct was used per transfection. Amaxa Nucleofector solution V and supplement I were mixed and 100 µl was added into the tube with DNA construct. The mixture was then added to the cells and transferred to the electroporation cuvette. Electroporation was done under setting X-001 using Amaxa Nucleofector II Device. 0.5 ml of growth media was added immediately after eletroporation and the mixture were transferred into 2 ml growth media in one well of the 6-well plate. The cells were incubated in the 37°C incubator with 5% CO₂ overnight. Rabbit polyclonal antibody against myc tag was added to the cells which were transfected with RCAR construct with myc tag as the extracellular domain. The concentration of the polyclonal antibody was at 100 nM. One and half hour later, 100 nM of anti rabbit antibody was added in order to enhance cluster formation. The cell mixture was incubated at 37°C for 18 hrs. Luciferase One Glo reagent 100 µl was added per well. The samples were incubated for 5 min at 37°C and then luminescence was measured using a luminometer.

Similarly, a construct with Flag peptide tag as the extracellular domain was used to transfect reporter cells. Rabbit polyclonal antibody against Flag peptide tag was used at 100 nM; and to facilitate further clustering, an antibody against the rabbit polyclonal antibody was used at 100 nM. In addition, a construct with scFv 139 as the extracellular domain was used to transfect reporter cells, and EGFRvIII-Fc at 100 nM was used, and one and half hour later anti-Fc antibody was used.

As shown in Fig. 14A, RCAR with c-myc peptide tag as the extracellular domain showed higher NFAT activity when incubated with rabbit polyclonal anti-myc antibody as compared to other antibodies. This indicated that the activation was specifically due to crosslinking of c-myc peptide tag on the cell surface forming clusters. The activation was further enhanced by the addition of anti rabbit antibody, as show in Fig. 14A.

RCAR with Flag tag only showed activation when both crosslinking reagents were added, i.e., both the rabbit polyclonal anti-Flag antibody and anti-rabbit antibody. Similarly, RCAR with 139 scFv as the extracellular domain showed activation when EGRFvIII-Fc and anti-Fc antibody were added. See Fig. 14.

The concept, methods and compositions described in this example are applicable to RNKR-CARs. The concept, methods and compositions described in this example are applicable to RCAR/NKR-CARX cells and/or RNKR-CARX cells.

### Example 6: Peptide-based multimerization of activation signaling domains

In embodiments, T cell-mediated cell death requires the linking of an external binding event to the signaling activation cascade in the intracellular space. Co-localization and clustering of these intracellular signaling domains via an external displayed molecule, a switch domain, and a dimerization molecule that comprises a multimerized soluble ligand in combination with a target cell bound receptor/scFv can be used to induce the desired response. See, e.g., Fig. 15. Exemplification of this principle in the context of regulatable CARs can be performed using EGFRvIII RCAR (139sFv) as a model system with an additional scFv (as switch domains) displayed to various multimers of the c-myc peptide tag (as the dimerization molecule).

### Materials and Methods

### Synthesis of c-myc peptide multimers and DNA for regulatable RCAR

Monomers, dimers, trimers and tetramers of c-myc peptide (SEQ ID NO:22-25) will be synthesized via standard solid-phase peptide synthesis and used as multimeric dimerization switches. Each of the c-myc peptide monomers is linked by a GS linker. The sequence for the 139 scFv will be cloned with the intracellular signaling domains for 4-1BB and CD3 zeta. The non-regulatable CAR construct, 139scFv-BBZ, SEQ ID: 7, will be used as a control. For the c-myc regulatable CAR, the 139 scFv will be cloned with the CD8 alpha transmembrane domain (SEQ ID NO: 26) and the corresponding intracellular signaling construct designed by fusing the 9E10 anti-myc scFv to linker and the intracellular signaling domains 4-1BB followed by CD3zeta (9e10 scFv-BBZ, SEQ ID NO: 27).
c-myc monomer peptide (SEQ ID NO: 22)
   EEQKLISEEDL
c-myc dimer peptide (SEQ ID NO: 23)
   EEQKLISEEDLGGGGSGGGGSGGGGSEEQKLISEEDL

### NFAT Activation Assay

Jurkat cells with NFAT-LUC reporter (JNL) were grown to the density of 0.5 x 10⁶/ml in Jurkat cell growth media with puromycin at 0.5 µg/ml. For each transfection 2.5 x 10⁶ cells were spin down at 100g for 10 minutes. Two µg of DNA per construct were used per transfection. Amaxa Nucleofector solution V and supplement I was mixed and 100 µl was added into the tube with DNA construct. The mixture was then added to the cells and transferred to the electroporation cuvette. Electroporation was done under setting X-001 using Amaxa Nucleofector II Device. 0.5 ml of growth media was added immediately after electroporation and the mixture were transferred into 2 ml growth media in one well of the 6-well plate and incubated for two hours. For myc tags in solution, the cells were distributed into 96-wells, monomer, dimer, trimer or tetramer myc tags or IgG1 Fc were applied at 100 nM. The cells were incubated for 18 hr. Alternatively, tissue culture plate was coated with 100 nM of various myc tags or IgG1 Fc for two hours, blocked with the blocking buffer (DPBS with 5% serum) for 1 hour. The transfected cells were added to the target plate with 100 µl per well and incubated for 18 hrs. Luciferase One Glo reagent 100 µl was added per well. The samples were incubated for 5 min at 37°C and then luminescence is measured using Envision plate reader.

### Results

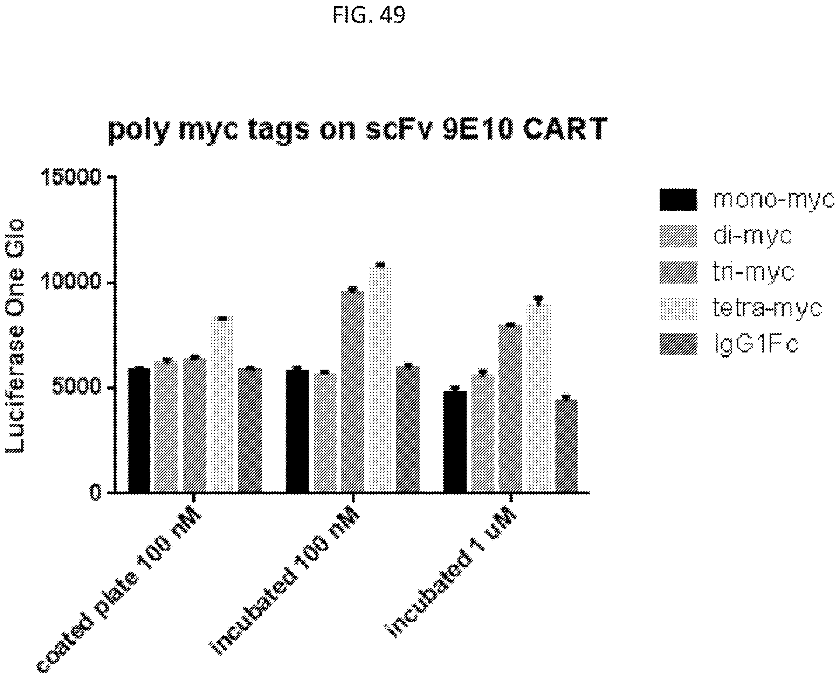

As shown in the Fig. 49, NFAT activation depended on the number of multimers of the myc tags. Higher NFAT activation was observed when higher multimer, e.g., trimer or tetramer, myc tags were used for the transfected cells. The poly myc tag in solution at 100 nM resulted in increased NFAT activation compared to activation from the coated plate with 100 nM myc tag. These results show that multimers of myc tags can be used to switch on extracellular switch RCARs.

The concept, methods and compositions described in this example are applicable to RNKR-CARs. The concept, methods and compositions described in this example are applicable to RCAR/NKR-CARX cells and/or RNKR-CARX cells.

### Example 7: Redirected Inhibitory RCAR

A general principle of the immune system is that T cells sense their microenvironment, and then either are activated or inhibited, depending on the signals that they sense. This finely tuned balance is transmitted by several activating receptors such as CD28 and ICOS and several inactivating receptors e.g. CTLA4, PD-1 and BTLA (Riley et al., 2005, Blood 105:13-21). The ligands for PD-1 are PDL1 and PDL2. PD-1 ligands are often expressed in the tumor microenvironment, and the engagement of PD-1 (programmed cell death 1) on T cells by PDL1 or PDL2, can lead to T cell inactivation. Limitations in treatment options to overcome this T cell inactivation in the tumor microenvironment would therefore be beneficial. Methods disclosed herein redirect the inhibitory signal using adoptive T cell therapy thereby rendering the negative regulatory signal, for example triggered through activation of PD1 receptor, into a positive signal that enhances the T cell activity when engaged. The general concept as outlined here is based on the regulatable switch RCAR as previously described. However in this embodiment, the RCAR comprises a cancer targeting moiety comprising an extracellular domain of an inhibitory receptor such as PD1. Additionally, the RCAR, may or may not display a standard CAR or a scFv tethered to the membrane used as a homing reagent for a particular cancer cell. See, e.g., the RCARs depicted in Fig. 9. In this example the switch is designed by fusing a leader sequence to the extracellular domain of PD1 followed by a hinge region, a transmembrane region, a linker and the first intracellular switch domain - FKBP (SEQ ID NO: 28). The second construct was designed by fusing the second switch domain FRB to a second linker and the internal signaling domains 4-1BB followed by CD3zeta (SEQ ID NO: 6). Activation of the T cell will be regulated by engagement of the PD1 ligands PDL1 or PDL2 in the presence of dimerization molecule, e.g., rapalogue, only. Co-expression of standard CAR or a targeting scFv may also be included in the RCAR to enhance specificity of the chimeric T cell. The concept, compositions, and methods described in this example are also applicable to RNKR-CARs, e.g., to design/generate redirected inhibitory RNKR-CARs. The concept, methods and compositions described in this example are applicable to RCAR/NKR-CARX cells and/or RNKR-CARX cells.

### Materials and Methods

### Synthesis of DNA for redirecting inhibitory RCAR

The sequence for the extracellular domain of human PD1 receptor will be cloned with the FRB-domain at the c-terminus (SEQ ID NO:29) and the corresponding activation construct designed by fusing the FKBP-domain to a linker and the activation domains 4-1BB followed by CD3zeta (SEQ ID NO:4). In another embodiment, the extracellular domain of human PD1 receptor will be cloned with the FRBPdomain at the c-terminus (SEQ ID NO:28) and the corresponding activation construct designed by fusing the FRB-domain to a linker and the activation domains 4-1BB followed by CD3zeta (SEQ ID NO:6). Jurkat assays will be performed as described in Example 1. Stimulation of the redirected inhibitory CAR can be achieved by addition of extracellular ligand PD1L or PD2L or by co-incubation of cells expressing PD1L or PD2L. Luciferase One Glo reagent 100 µl will be added per well. The samples will be incubated for 5 min at 37°C and then luminescence will be measured using a luminometer.

### Activation assay of redirected inhibitory RCAR

Jurkat cells with NFAT-LUC reporter (JNL) were grown to the density of 0.5 x 10⁶/ml in Jurkat cell growth media with puromycin at 0.5 µg/ml. For each transfection 3 x 10⁶ cells were spin down at 100g for 10 minutes. Four µg DNA per construct were used per transfection. Amaxa Nucleofector solution V and supplement I was mixed and 100 µl was added into the tube with DNA construct. The mixture was then added to the cells and transferred to the electroporation cuvette. Electroporation was done under setting X-001 using Amaxa Nucleofector II Device. 0.5 ml of growth media was added immediately after eletroporation and the mixture were transferred into 2 ml growth media in one well of the 6-well plate. After two hours, the rapalogue compound at various concentrations was added to cells. The cells were applied to tissue culture plate wells that were coated by the target. Tissue culture plate was coated with 5 µg/ml of PDL1-Fc or IgG1-Fc or any target for 2 hrs at 37°C, then blocked with the blocking buffer (DPBS with 5% serum) for 30 minutes. The transfected cells were added to the target plate with 100 µl per well and incubated further for 16 hrs. Luciferase One Glo reagent 100 µl was added per well. The samples were incubated for 5 min at 37°C and then luminescence is measured using Envision plate reader.

The PD1 CAR construct comprises PD1-ECD -TM- 41BB- CD3zeta. This construct may improve the persistence of cells transfected with the construct, e.g., CART cells transfected with PD1 CAR.

The PD1 RCAR (switchable PD1 CAR) construct uses the FRB-FKBP heterodimerization switch and the rapalogue hetermodimerization molecule AP21967. Specifically, the PD1 RCAR comprises a PD1-ECD-TM - FRB construct; and FKBP - 41BB- CD3 zeta construct, that were co-transfected into Jurkat cells. These constructs may improve the persistence of cells transfected with the construct, e.g., RCART cells transfected with PD1 RCAR.

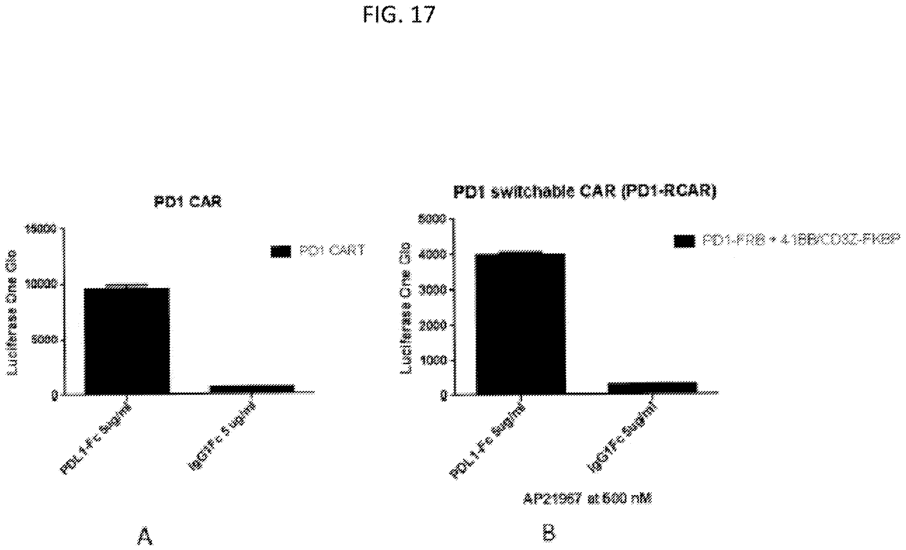

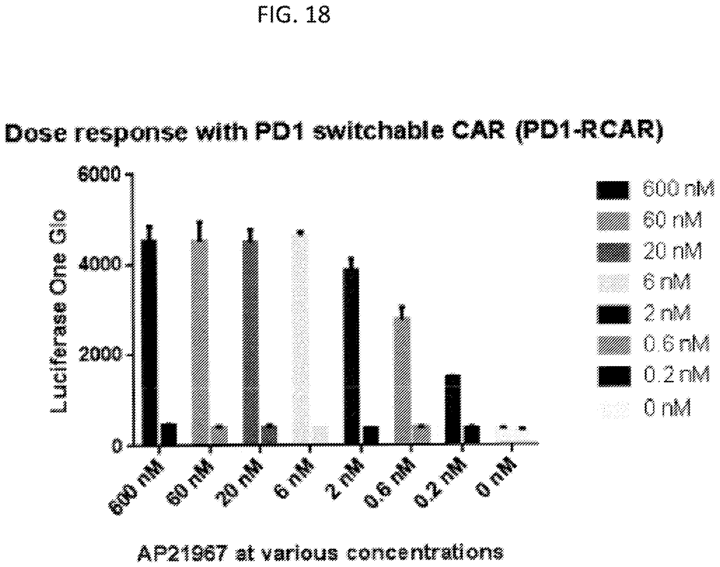

As shown in Fig. 17A: PD1 CAR showed significant PD1 induced activation of NFAT inducible promoter driven luciferase activity, as compared to the control treatment by IgG1-Fc. This suggest that PD1 interaction with PDL-1 is sufficient in causing clustering of PD1 on Jurkat cell surface and triggers the strong activation of the NFAT pathway. This PD1 CAR was used as a control for the PD1 RCAR experiments. In Fig. 17B, when Jurkat reporter cells were co-transfected by the PD1 RCAR which include PD1-ECD -TM-FRB and FKBP-4 1BB-CD3 zeta, and incubated with 500 nM AP21967 heterodimerization molecule, there was significant induction of signaling by PDL-1 target, which indicate that AP21967 heterodimerization molecule induced dimerization of PD1 from one construct with 4 1BB-CD3 zeta of another construct, and this resulted activation of NFAT signaling. To further investigate the effect of AP21967 heterodimerization molecule on the RCAR, varying concentrations of the AP21967 heterodimerization molecule were added to the cells. As shown in Fig. 18, there was a dose response with AP21967 treatment. Each bar on the left is the PD1 RCAR, while each bar on is PD1 CAR control at different concentrations of AP21967. The AP21967 was potent even at sub nM concentration in inducing the dimerization of the heterodimerization switch resulting in activation of NFAT signaling.

### Example 8: Switchable CART activation by co-cluster receptors including PD1

Cancer cells not only abnormally express cancer antigens but can also express PD1 ligands, which provide for escape from immune attack by effector T-cells. PD1, upon ligand binding, forms micro-clusters with TCR and directly inhibits T-cell activation. The formation of clusters by co-stimulators or co-inhibitors is used by nature to enhance or inhibit an immune response similar to a digital event, i.e., it is either "on" or "off". In other words, the immune response is turned on when multiple factors align. This differentiates "real" signals from "noise," which can be harmful. See Fig. 10.

The phenomenon of co-stimulator/co-inhibitor clusters can be used for CART therapy to ensure the targeting/killing specificity. This can be combined with small molecule (rapalogue) as the dimerization switch for RCAR cell activity. In embodiment this will increase the therapeutic window and reduce toxic side effects.

Recognition and binding of cancer cells by T-cells represent the important initial step to establish the killing specificity. Co-targeting of cancer cells by cancer cell specific antigen which is recognized by low affinity scFv and PDL1/2 by PD1 can be achieved through low affinity interactions: PD1 with PDL1 or PDL2 from cancer cells, and low affinity interaction of scFv with cancer antigens on cancer cells. This dual binding event is amplified through the avidity effect, and is further amplified through micro-cluster formation of scFv and PD1. These recognition and binding events will enable the immune synapse formation between target cells and RCART cells, the first step for RCART regulated cancer cell killing. The addition of rapalogue will dimerize/cluster 4-1BB/CD3 zeta, which will further activate RCART cells for cancer cell killing.

The concept, methods and compositions described in this example are applicable to RNKR-CARs. The concept, methods and compositions described in this example are applicable to RCAR/NKR-CARX cells and/or RNKR-CARX cells.

### Experimental procedure

Mouse or human PD1 ECD will be fused with a transmembrane domain followed by an FKBP switch domain. Low affinity scFv against one of the tumor antigens such as mesothelin will be fused with a transmembrane domain followed by a FKBP switch domain. The FRB switch domain will be fused with intracellular signaling domains 4-1BB and CD3 zeta. See, e.g., the RCAR depicted in Fig. 10. Jurkat T-cells with NFAT driven luciferase reporter will be co-transfected with the three constructs: PD1-TM-FKBP, scFv-TM-FKPB and FRB-4-1BB-CD3 zeta. Transfection will be done with Amaxa nucleofactor with 3 X 10⁶ Jurkat cells per transfection with 2 µg DNA per construct. The cells will be incubated at 37°C with 8% CO₂ for one day incubation after co-transfection. The Jurkat cells will then be applied to the wells that are coated with 5 µg/ml of human mesothelin and 5 µg/ml of mouse PD-L1 or PD-L2. After one day of incubation at 37°C, luciferase activity will be measured by One-Glo reagent from Promega.

In addition, the co-transfected cells will be mixed with cancer cells that express both mesothelin and PDL1 or PDL2, as well as normal cells at 1:0.3 ratio of effector CART cells: target cells. After one day of incubation, luciferase activity will be measured.

The target cancer cells that show overexpression of PDL1 or PDL2 will be tested.

### Example 9: Small molecule regulated CAR activation - extracellular switch

T cell-mediated cell death requires the linking of the external binding event to the signaling activation cascade in the intracellular space. In an embodiment, a RCAR comprises and extracellular dimerization switch, e.g., an extracellular heterodimerization switch activated by a small molecule heterodimerization molecule. See, e.g., the RCAR depicted in Fig. 5. The following example illustrates the design of such an "extracellular small molecule" switch.

In this example, the switch is designed by fusing a leader sequence to the 139 scFv followed by linker and the first switch domain - FKBP, a hinge region and a transmembrane region (SEQ ID NO: 31). The second construct is designed by fusing a leader sequence to the FRB switch domain, a transmembrane region and the intracellular signaling domains 4-1BB followed by CD3zeta (SEQ ID NO: 32).

Alternatively a pair of constructs with the following design (139-FRB-TM (SEQ ID NO: 30) FKBP-TM-41BB-CD3zeta (SEQ ID NO: 33) may also be used.

Co-transfection of the T cell of the pair of construct will yield a tuneable extracellular activation switch regulated by addition of the small molecule rapalogue (Structure 1).

The extracellular switch transfections and activations were carried out essentially as described for the intacellular switches in Example 1.

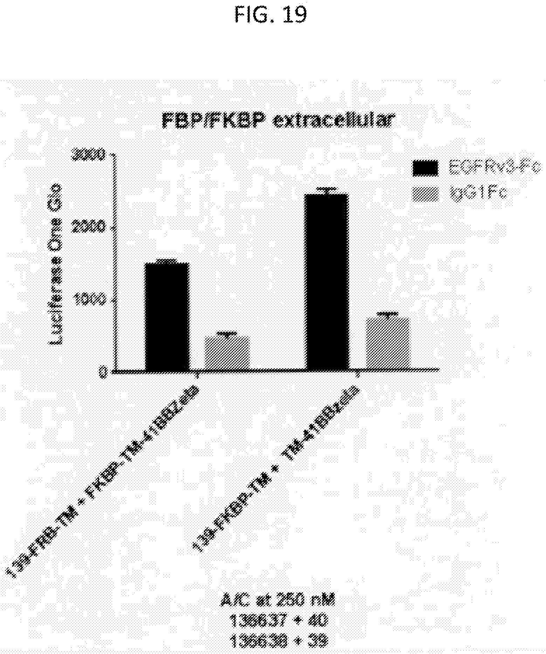

Fig. 19 shows the results from studies designed to investigate the signaling events between an RCAR expressing the antigen binding domain, e.g., scFv, and an RCAR expressing the intracellular signaling domains, wherein the heterodimerization switch domains are external, in the presence of a heterodimerization molecule. The data shows significant target regulated activation upon stimulation with the rapalogue at 250 nM. Furthermore, no significant on-target activation was observed in the absence of the rapalogue, indicating an external switch mediated by the small molecule regulated heterodimerization of the switch domains (FKBP and FRB). Finally, no activation was observed against a control target (IgG1-Fc only). These data demonstrate specificity of an externally-switched RCAR constructs for EGFRvIII target in the presence of the rapalogue only, and lack of cross-reactivity to control target or in the absence of rapalogue.

Additional RCAR constructs comprising extracellular switches and functional analysis thereof are described in Example 18.

The concept, methods and compositions described in this example are applicable to RNKR-CARs. The concept, methods and compositions described in this example are applicable to RCAR/NKR-CARX cells and/or RNKR-CARX cells.

### Example 10: Targeting mouse and human Programmed Cell Death 1 (PDCD1) gene using shRNA

In addition to the RCAR constructs described in Examples 1-9, further regulation can be provided by engineering shRNA into the nucleic acids encoding the RCAR Commonly used promoters such as U6 and H1 may be placed downstream of the RCAR lentivirus constructs. PDCD1, TIM3, or other negative regulators of T cell activity may be attenuated by the expression of suitable shRNA constructs. A generic map showing different configurations of constructs encoding the regulatable CARwith a shRNA for coexpression of RCAR and an shRNA is provided in Fig. 16.

The two switch domain containing members (RCARa and RCARb), e.g., an antigen binding member and an intracellular signaling member, are regulated by a combination of the EF1alpha promoter and a suitable IRES element such as the EMCV. As shown in Fig. 16, the EF1 alpha promoter is located upstream of a first RCAR member, e.g., RCARa, which is followed by an IRES element, and finally the second RCAR member, e.g., RCARb. In embodiments, the shRNA is regulated by the U6 promoter. In embodiments , the shRNA and RCAR encoding elements are present on a single vector. Fig. 16A-16D show the various configurations on a single vector, e.g., where the U6 regulated shRNA is upstream or or downstream of the EF1alpha regulated RCAR encoding elements. In the exemplary constructs depicted in Fig. 16A and 16B, the transcription occurs through the U6 and EF1 alpha promoters in the same direction. In the exemplary constructs depicted in Fig. 16C and 16D, the transcription occurs through the U6 and EF1alpha promoters in different directions. In another embodiment, the shRNA (and corresponding U6 promoter) is on a first vector, and the RCAR (and corresponding EF1alpha promoter) is on a second vector (Fig. 16E). Constitutive attenuation of inhibitory receptors such as PD1 in T cells specifically targeting cancer cells should overcome PD1 pathway mediated immune suppression in the tumor microenvironment.

The concept, methods and compositions described in this example are applicable to RNKR-CARs. The concept, methods and compositions described in this example are applicable to RCAR/NKR-CARX cells and/or RNKR-CARX cells.

### Computational analysis for sequence selection

shRNA design was carried out to identify shRNAs targeting the mouse and human gene Programmed Cell Death 1 (PDCD1) gene. The design used the NM_008798.2 (mouse) and NM_005018.2 transcript from the NCBI RefSeq collection, respectively. The predicted potency and specificity of all possible 19mers was predicted from each sequence. For potency prediction, 19mer sequences that contained a BioPred score > 0.8, and a Dharmacon score > 4 were selected. For specificity, 19mer sequences that lacked repeats longer than 4 nucleotides and that did not contain seed-region matches to multiple (>10) known human miRNAs were selected. In addition, 19mer sequences when searched against the human and mouse transcriptome, respectively (defined as the set of NM_and XM_ records within the human, mouse NCBI Refseq set) using the BLAST algorithm were discarded when either the antisense or the sense strand (including position 1 or 2 counting from the 5'end of the guide strand) had > 15 consecutive nucleotides in common with any other mRNA transcript in NCBI Refseq. Furthermore, sequences containing motifs reported to potentially induce innate immune as well as cytotoxic response were discarded.

In a final step, the acceptable 19mer sequences were sorted according to the predicted potency score and the top twelve sequences were selected in form of their corresponding 21-mer sequences for shRNA synthesis.

Provided in Table 18 below are the names of PDCD1 (PD1) RNAi agents (derived from their position in the mouse PDCD1 gene sequence NM_008798.2), along with the SEQ ID NOs: 34-81 representing the DNA sequence. Both sense (S) and antisense (AS) sequences are presented as 19mer and 21mer sequences are in this table. Also note that the position (PoS, e.g., 176) is derived from the position number in the mouse PDCD1 gene sequence NM_008798.2. SEQ ID NOs are indicated in groups of 12 that correspond with "sense 19" SEQ ID NOs: 34-45; "sense 21" SEQ ID NOs: 46-57; "asense 21" SEQ ID NOs: 58-69; "asense 19" SEQ ID NOs: 70-81.

**Table 18. Mouse PDCD1 (PD1) shRNA sequences**

| **Position on NM_0087 98.2** | **Target region** | **Sense19** | **Sense21** | **Asense21** | **Asense19** |
|---|---|---|---|---|---|
| 176 | CDS | | | | |
| | | 34) | (SEQ ID NO: 46) | (SEQ ID NO: 58) | NO: 70) |
| 260 | CDS | | | | |
| 359 | CDS | | | | |
| 528 | CDS | | | | |
| 581 | CDS | | | | |
| 584 | CDS | | | | |
| 588 | CDS | | | | |
| 609 | CDS | | | | |
| 919 | CDS | | | | |
| 1021 | 3'UTR | | | | |
| 1097 | 3'UTR | | | | |
| 1101 | 3'UTR | | | | |

Provided in Table 19 below are the names of PDCD1 (PD1) RNAi agents (derived from their position in the human PDCD1 gene sequence, along with the SEQ ID NOs. 82-129 representing the DNA sequence. Both sense (S) and antisense (AS) sequences are presented as 19mer and 21mer sequences. SEQ ID NOs are indicated in groups of 12 that correspond with "sense 19" SEQ ID NOs: 82-93; "sense 21" SEQ ID NOs: 94-105; "asense 21" SEQ ID NOs: 106-117; "asense 19" SEQ ID NOs: 118-129.

**Table 19. Human PDCD1 (PD1) shRNA sequences**

| **Position on NM_0050 18.2** | **Target region** | **Sense19** | **Asense19** | **Sense21** | **Asense21** |
|---|---|---|---|---|---|
| 145 | CDS | | | | |
| 271 | CDS | | | | |
| | | | | | |
| 393 | CDS | | | | |
| 1497 | 3'UTR | | | | |
| 1863 | 3'UTR | | | | |
| 1866 | 3'UTR | | | | |
| 1867 | 3'UTR | | | | |
| 1868 | 3'UTR | | | | |
| 1869 | 3'UTR | | | | |
| 1870 | 3'UTR | | | | |
| 2079 | 3'UTR | | | | |
| 2109 | 3'UTR | | | | |

### Validation of shRNA-mediated PD1 knockdown

shRNA sequences targeting human and mouse PD1 were validated by an in vitro knockdown assay. The human 21mer shRNA sequences listed in Table 19 (SEQ ID NOs: 106-117) and the mouse 21mer shRNA sequences listed in Table 18 (SEQ ID NOs: 46-57) were
were synthesized according to the following design scheme.
**5'-G *xxxxxxxxxxxxxxxxxxxxx*** TTCAAGAGA *yyyyyyyyyyyyyyyyyyyyy* **TTTTTT-3'** (SEQ ID NO: 151)

A single 5' G was added to the target sense sequence depicted above, with the target sense sequence designated by ***x*** (provided in Tables 18 or 19), followed by a hairpin loop of the sequence TTCAAGAGA (SEQ ID NO: 152), the corresponding target anti-sense sequence (designated by ***y***) and a 3' poly-T terminator sequence (underlined above). To facilitate cloning each construct was also flanked by a 5' BamHI and a 3' EcoRI site. Finally the synthesized constructs were sub-cloned into pLVX-shRNA2 (Clontech) using recombinant DNA techniques.

The human PD1 knockdown assay was performed as follows. Two million Jurkat JNL cells were nucleofected with 1-2µg shRNA plasmid DNA (depending on stock concentration) using Lonza's SE Cell Line 4D Kit and pulse code CL-116, according to the manufacturer's recommendations. Cells were immediately resuspended and plated in Antibiotic Free Growth Media to a density of 1x10⁶/mL. Nucleofected Jurkat JNL cells were incubated at 37°C, 5% CO₂, 24 hours, 48 hours or 72 hours. At the end of each time point cell lysis was performed using QIAGEN's FastLane Cell Multiplex kit according to the manufacturer's recommendations. Multiplex qPCR was performed on diluted cell lysates using hPDCD1 Taqman probe, Hs01550088_m1, and hGAPDH Taqman probe, 4326317E. Percent transcript remaining was determined using the delta delta Ct method relative to the PBS nucleofection control. Error bars are standard deviation of technical and biological replicates.

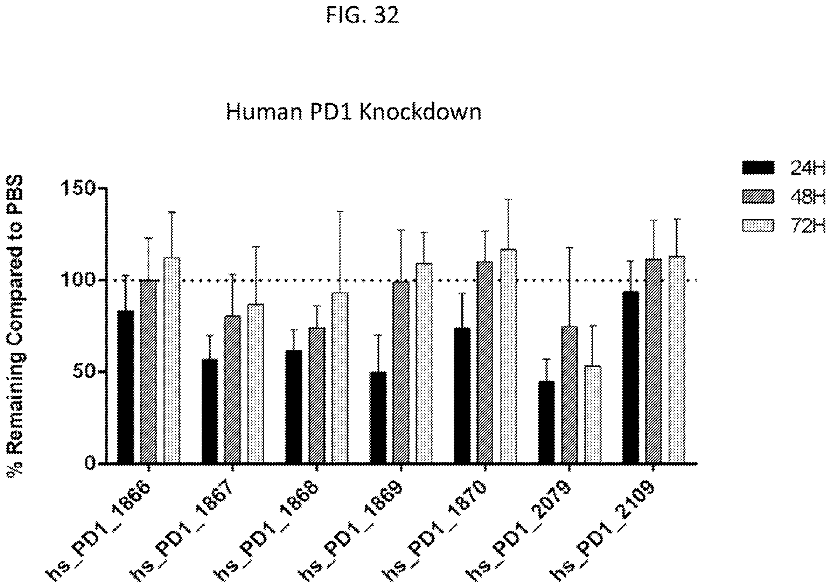

As shown in Figure 32, all tested human PD1 shRNA constructs knocked down PD1 expression, as represented by lower than 100% of PD1 transcript remaining, e.g., at 24 hours after nucleofection. The shRNAs targeting positions 1867, 1868, 1869, 1870, and 2079 demonstrated reduced PD1 expression to less than 75% of normal expression levels, with the shRNA targeting position 2079 having the most robust knockdown at all three tested timepoints, 24, 48, and 72 hours.

The mouse PD1 knockdown assay was performed as follows. Two hundred thousand EL4 cells were nucleofected with 0.5-1µg shRNA plasmid DNA (depending on stock concentration) using Lonza's SF Cell Line 4D Kit and pulse code CM-120-AA, according to the manufacturer's recommendations. Cells were immediately resuspended and plated in Antibiotic Free Growth Media to a density of 2.5x10⁶/mL. Nucleofected EL4 cells were incubated at 37°C, 5% CO₂, 24 hours or 48 hours. At the end of each time point cell lysis was performed using QIAGEN's FastLane Cell Multiplex kit according to the manufacturer's recommendations. Multiplex qPCR was performed on diluted cell lysates using mPDCD1 Taqman probe, Mm01285676_m1, and mβ-actin Taqman probe, 4352341E. Percent transcript remaining was determined using the delta delta Ct method relative to the PBS nucleofection control. Error bars are standard deviation of technical and biological replicates.

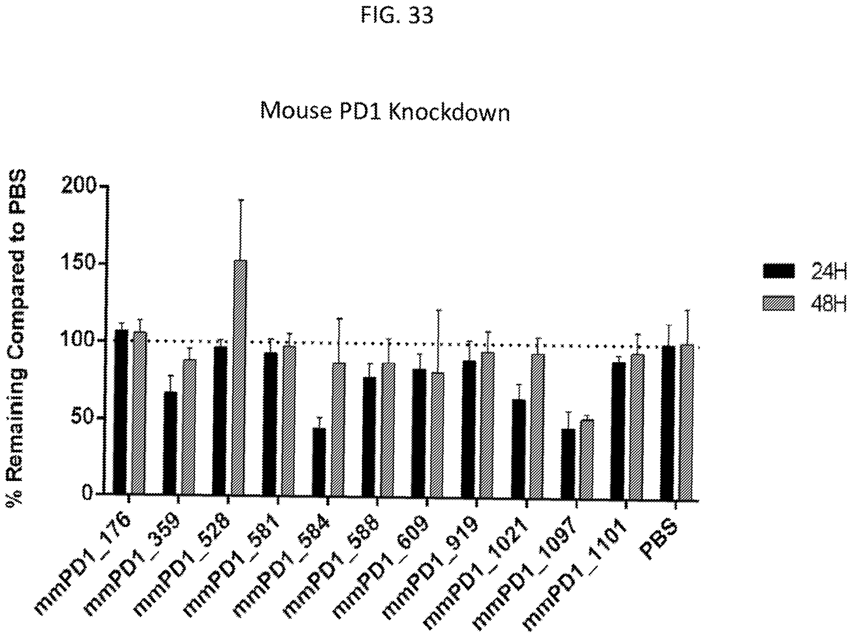

As shown in Figure 33, the different shRNA constructs tested exhibited varying levels of PD1 knockdown. The shRNA constructs targeting positions 584 (mmPD1_584) and 1097 (mmPD1_1097) demonstrated the most robust PD1 knockdown of all of the constructs.

### Example 11: Analysis of Regulatable CAR Constructs in T cells

To evaluate the feasibility of regulating CAR technology, the regulatable CARs will be cloned into a lentiviral CAR expression vector with the CD3zeta chain and the 4-1BB costimulatory molecule in different configurations under the control of an EF1 alpha promoter. Sequence encoding an antigen binding domain, e.g., an scFv described herein, can be inserted beteen the leader and hinge sequences described below.

### RCAR Components - Nucleic Acid Sequences

leader (nucleic acid sequence); (SEQ ID NO: 130)
• hinge (nucleic acid sequence); (SEQ ID NO: 131)
• transmembrane (nucleic acid sequence); (SEQ ID NO: 132)
• 4-1BB Intracellular domain (nucleic acid sequence); (SEQ ID NO: 133)
• CD3 zeta (nucleic acid sequence); (SEQ ID NO: 134)

### RCAR Components - Amino Acid Sequences

- leader (amino acid sequence) (SEQ ID NO: 135)
   MALPVTALLLPLALLLHAARP
- hinge (amino acid sequence) (SEQ ID NO: 136)
   TTTPAPRPPTPAPTIASQPLSLRPEACRPAAGGAVHTRGLDFACD
- transmembrane (amino acid sequence) (SEQ ID NO: 137)
   IYIWAPLAGTCGVLLLSLVITLYC
- 4-1BB Intracellular domain (amino acid sequence) (SEQ ID NO: 138)
   KRGRKKLLYIFKQPFMRPVQTTQEEDGCSCRFPEEEEGGCEL
- CD3 zeta domain (amino acid sequence) (SEQ ID NO: 139)

The optimal construct will be selected based on the quantity and quality of the effector T cell response regulatable RCAR transduced T cells in response to EGFRvIII+ and EGFR wild type targets. Effector T cell responses include, but are not limited to, cellular expansion, proliferation, doubling, cytokine production and target cell killing or cytolytic activity (degranulation).

### Generation of regulatable CAR T cells

The RCAR lentiviral transfer vectors are used to produce the genomic material packaged into the VSVg psuedotyped lentiviral particles. Lentiviral transfer vector DNA is mixed with the three packaging components of VSVg, gag/pol and rev in combination with lipofectamine reagent to transfect them together in to 293T cells. After 24 and 48hr, the media is collected and filtered. The media is concentrated by ultracentrifugation. Alternatively, a single collection is done 30 hr after media change. Virus containing media is alternatively used unconcentrated or concentrated by Lenti-X concentrator (Clontech, Cat# 631232). The resulting viral preparation is stored at -80C. The number of transducing units is determined by titration on SupT1 cells.

For example, redirected EGFRvIII-specific RCART cells are produced by activating fresh T cells by engaging with CD3x28 beads for 24hrs and then adding the appropriate number of transducing units to obtain the desired percentage of transduced T cells. These modified T cells are allowed to expand until they become rested and come down in size (-300 fl) at which point they are cryopreserved for later analysis. The cell numbers and sizes are measured using a Coulter multisizer III, a Nexcelom Cellometer Vision or Millipore Scepter. Before cryopreserving, percentage of cells transduced (expressing the EGFRvIII-specific CAR on the cell surface) and their relative fluorescence intensity of that expression are determined by flow cytometric analysis on an LSRII. From the histogram plots, the relative expression levels of the CARs can be examined by comparing percentage transduced with their relative fluorescent intensity.

### Evaluating cytolytic activity, proliferation capabilities and cytokine secretion of EGFRvIII redirected, regulatable CAR T cells.

To evaluate the functional abilities of EGFRvIII-specific RCAR T cells to kill, proliferate and secrete cytokines, the cells are thawed and allowed to recover overnight or used right away. In addition to the RCAR constructs, the standard second generation EGFRvIII-clone 139-BBz CAR is used for comparative purposes while SS1-BBz (mesothelin-specific) is used as non-targeting expressed CAR for background CAR/T cell effect. For this flow based cytotoxicity assay, the target cells are stained with CSFE to quantitate their presence. The target cells are also stained for EGFRvIII expression to confirm similar target antigens levels. The cytolytic activities of EGFRvIII CAR T cells are measured at a titration of effector:target cell ratios of 10:1, 3:1, 1:1, 0.3:1 and 0:1 where effectors were defined as T cells expressing the anti-EGFRvIII chimeric receptor. Assays were initiated by mixing an appropriate number of T cells with a constant number of targets cells. A dose titration of the dimerization molecule is also added to the cultures, from 0 - 1000 nM concentrations for the rapalogue or anti-Myc antibody. After 4 or 16hrs, total volume of each mixture is removed and each well washed. The T cells are stained for CD3 and all cells stained with live/dead marker 7AAD. After the final wash, the pelleted cells are re-suspended in a specific volume with a predetermined number of counting beads. Cell staining data is collected by LSRII flow cytometry and analyzed with FlowJo software using beads to quantitate results.

For measuring cell proliferation and cytokine production of RCAR-EGFRvIII T cells, cells are thawed and allowed to recover overnight. In addition to the RCAR constructs, the standard second generation EGFRvIII-clone 139-BBz CAR is used for comparative purposes while SS1-BBz (mesothelin-specific) is used as non-targeting expressed CAR for background CAR/T cell effect. The T cells are directed towards U87, a glioblastoma, astrocytoma cell line expressing or not expressing EGFRvIII. In addition, CD3x28 beads are used to evaluate the potential of T cells to respond to the second round of endogenous immunological signals. A dose titration of the dimerization molecule is also added to the cultures, from 0 - 1000 nM concentrations for the rapalogue or anti-Myc antibody. To analyze proliferation, T cells are stained with CSFE. The proliferation is the dilution of the CSFE stain reflecting the separation of the parental markings now into two daughter cells. The assay tests only an effector:target ratios of 1:1 and 1:0 where effectors were defined as total T cells (CD4 and 8) normalized to express the anti-EGFRvIII chimeric receptor at a common percentage. The assay is done in duplicate and 24hrs after mixing of the cells, supernatant is removed for cytokine production. After 5 days, T cells are stained for live/dead with Live/Dead Violet (Invitrogen), then stained for CAR expression and phenotyped as either CD4 or CD8 cells. After the final wash, the pelleted cells are re-suspended in a specific volume with a predetermined number of BD counting beads. Cell staining data is collected by LSRII flow cytometry and analyzed with FlowJo software using beads to quantitate results. Total cell counts are determined by number of cells counted relative to a specific number of beads multiplied by the fraction of beads yet to be counted.

### Evaluation of Regulatable CARTs in vivo

The following experiments were designed to address whether the function of the regulatable CARTs can be modulated in vivo. Experiments are designed to test the in vivo delivery and function of the dimerization molecule in the context of a tumor model. Parameters to be measured include, but are not limited to, CART expansion, activation status of CART cells in the periphery as measured by FACS of peripheral blood samples, and tumor cell killing. Three different in vivo experiments are envisioned to address the utility of the RCAR and the dimerization molecule. These experiments will include assessment of 1) the basic function of the RCAR with the dimerization molecule, 2) a PD-1 dependent tumor model with the redirected switchable inhibitory RCAR, and 3) a PD-1 dependent tumor model with a RCAR and co-expression of a shRNA to PD-1.

The immunodeficient NOD/*scid*/γcnull (NSG) mouse is a suitable xenotransplantation model to engraft human tumor cell lines or primary tumors and human T cells. Following engraftment, the human T cells can be maintained in NSG mice for approximately 2 months, or until fatal xenogeneic GVHD (xGVHD) develops, which depends on the dose of human T cells infused. The administration of the dimerization molecule may be carried out in any convenient manner, based on the pharmacokinetic properties of the molecule and the optimized dosage and treatment regimen previously determined.

The concept, methods and compositions described in this example are applicable to RNKR-CARs. The concept, methods and compositions described in this example are applicable to RCAR/NKR-CARX cells and/or RNKR-CARX cells.

### Example 12: In vivo analysis of the basic RCAR with a dimerization molecule

To evaluate turning on RCART function in vivo with the dimerization molecule, human T cells expressing either switch 1 or switch 2 with a human CD19-specific scFv (FMC63) will be tested in an ALL tumor model using the NALM6-Luciferase (NALM6-Luc) tumor cell line which expresses human CD 19. The FMC63 scFv has been validated extensively as part of a second generation CAR construct (CD19-41BB-zeta) that mediates complete tumor regression in preclinical models (Milone et al., Molecular Therapy 17(8): 1453-1464 (2009)). In brief, lentiviral constructs will be generated with switch 1 or switch 2 using the FMC63 scFv and these constructs will be transduced into primary human T cells. The T cells will be expanded ex vivo for 10-12 days and then cryopreserved by methods previously described. NSG mice will be implanted with NALM6-Luc tumor cell line by intravenous inoculation and tumor burden allowed to establish for 5-8 days. Tumor burden can be measured by standard imaging for luciferase activity. Mice are randomized as to treatment groups which include the following groups: 1) Mock/PBS, 2) Untransduced T cells, 3) CART19 (2^{nd} generation intact CAR), 4) CD19-Switch 1, 5) CD19-Switch 1 plus rapalogue or RAD001 and 6) Rapalogue or RAD001 alone. Treatment groups will receive either PBS (groups 1 and 6) or 5x10⁶ CART cells/ mouse 5-8 days post tumor implantation. At a predetermined time following T cell infusion, e.g. 1-3 days, mice will be dosed with the rapalogue or RAD001. Dosage and schedule will be based on doses of the dimerization molecule which does not inhibit tumor cell growth by itself. Throughout the course of the study, mice will be imaged for tumor burden every other day or as frequently as needed to accurately assess the impact on tumor growth. At various intervals, blood samples will be collected for immunophenotyping analysis and peripheral T cell counts.

Dose-dependent effects of the rapalogue or RAD001 can be further assessed in additional experiments with treated groups receiving different doses of the rapalogue or RAD001 and/or alternative dosing regimens. The time course of tumor regression as well as degree of tumor regression can both be quantitated based on the imaging analysis.

The concept, methods and compositions described in this example are applicable to RNKR-CARs. The concept, methods and compositions described in this example are applicable to RCAR/NKR-CARX cells and/or RNKR-CARX cells.

### Example 13: In vivo analysis of the basic RCAR along while targeting a checkpoint inhibitor with a shRNA

To evaluate the redirected switchable inhibitory RCAR, human T cells expressing the RCAR will be evaluated in a mesothelin xenograft tumor model which expresses PD-L1. T cells will be generated which co-express switch 1 with a human mesothelin-specific scFv (SS1) along with a shRNA to PD-1. The SS1 scFv has been extensively validated as part of a second generation CAR construct that mediates tumor regression in preclinical models (Carpenito et al, PNAS, 106: 3360-3365, 2009). In brief, tumor cells expressing mesothelin and PD-L1 will be injected in to the flanks of NSG mice. These tumor cells may derive from a primary tumor such as M108 or from a tumor cell line such as OvCAR8. For OvCAR8, the cell line may be engineered to express PD-L1 if it does not express endogenous levels of the protein. Once tumors are established and the tumor burden is about 200-300 mm³, mice will be randomized into the following treatment groups: 1) Mock/PBS, 2) Untransduced T cells, 3) SS1-BBz (2^{nd} generation intact CAR), 4) Meso-Switch 1 plus rapalogue, 5) Meso-Switch 1 with PD-1 shRNA and rapalogue, and 6) Rapalogue alone. Treatment groups will receive either PBS (groups 1 and 6) or 5x105 CART cells/mouse. At a predetermined time following T cell infusion, e.g. 1-3 days, mice will be dosed with the rapalogue. Throughout the course of the study, tumor volume will be measured with callipers. At various intervals, blood samples will be collected for immunophenotyping analysis and peripheral T cell counts. Levels of PD-1 expression on the RCART cells will also be measured to assess the level of target knockdown by the shRNA.

Dose-dependent effects of the rapalogue can be further assessed in additional experiments with treated groups receiving different doses of the rapalogue and/or alternative dosing regimens. The time course of tumor regression as well as degree of tumor regression can both be quantitated based on the tumor volume.

The concept, methods and compositions described in this example are applicable to RNKR-CARs. The concept, methods and compositions described in this example are applicable to RCAR/NKR-CARX cells and/or RNKR-CARX cells.

### Example 14: Expression of Single Vector Constructs

Lenti viral vectors were constructed that encode two elements of an RCAR.

Construct 143775 is a single nucleic acid vector which comprises an EF1 alpha promoter operably linked to sequence encoding an CD19 scFV-based antigen binding member, an IRES, and an intracellular signaling member comprising a 4-1BB domain and a CD3zeta domain. It expresses a single transcript from which the two products are separately transcribed.

Construct 143776 is a single nucleic acid vector which comprises an EF1 alpha promoter operably linked to sequence encoding an CD19 scFV-based antigen binding member, and a CMV minimal promoter operably linked to a sequence encoding an intracellular signaling member comprising a 4-1BB domain and a CD3zeta domain.

Construct 143777 is a single nucleic acid vector which comprises an EF1 alpha promoter operably linked to sequence encoding an CD19 scFV-based antigen binding member, and a CMV minimal promoter operably linked to a sequence encoding an intracellular signaling member comprising a 4-1BB domain and a CD3zeta domain.

The constructs and viral particles were made and tested essentially as described in Example 11. Viral particles were introduced into JNL cells and evaluated for activity essentially as described in Example 1 except that the target was CD19 (as opposed to EGFRviii).

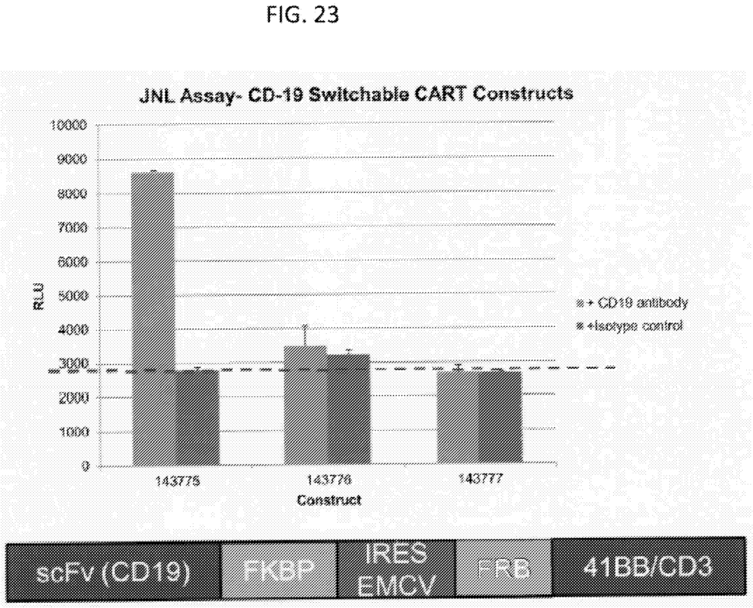

Fig. 23 shows the ability of RCART constructs expressed from the single vector construct to activate the RCART. The dimerization molecule was RAD001. The graph shows that elements of an RCAR encoded by a single vector are expressed and respond to dimerization molecule to activate cells, as measured by expression of a reporter promoter under NFAT control.

The concept, methods and compositions described in this example are applicable to RNKR-CARs. The concept, methods and compositions described in this example are applicable to RCAR/NKR-CARX cells and/or RNKR-CARX cells.

### Example 15: Dimerization Molecule Dose Response

Construct 143775 was introduced into cells and the affect of dimerization molecule concentration on activation evaluated essentially as described in Example 14.

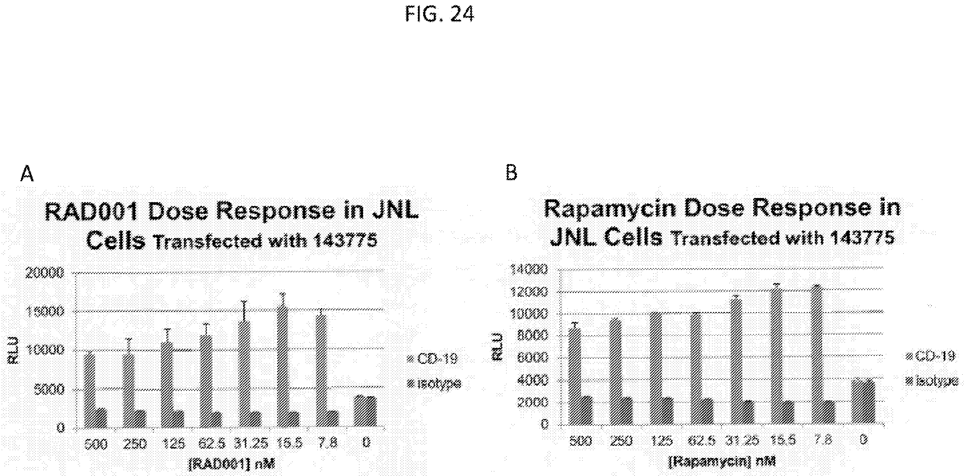

Fig 24A shows the effect of nM doses of RAD001 on activation. Fig 24B shows the effect of nM doses of rapamycin on activation.

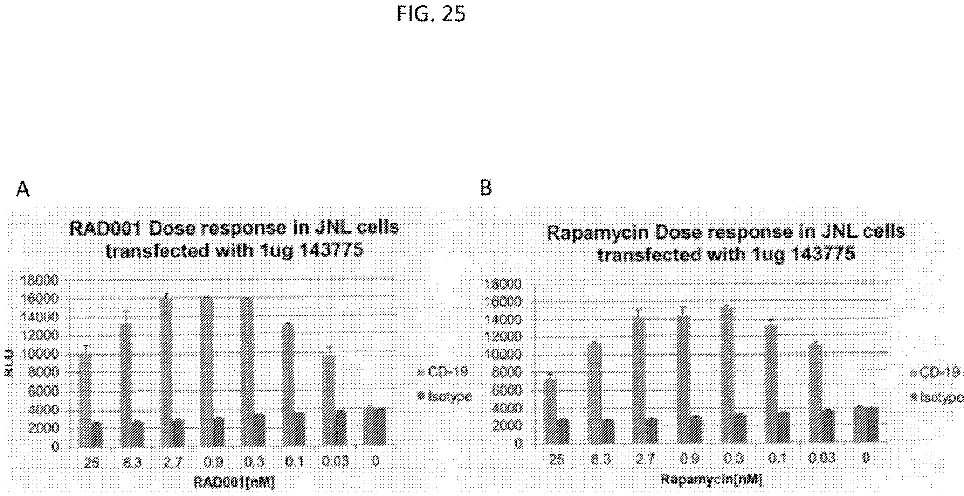

Fig 25A shows the effect of nM to sub-nM doses of RAD001 on activation. Fig 25B shows the effect of nM to sub-nM doses of rapamycin on activation. The data show that both RAD001 and rapamycin are effective at a broad range of concentrations.

The concept, methods and compositions described in this example are applicable to RNKR-CARs. The concept, methods and compositions described in this example are applicable to RCAR/NKR-CARX cells and/or RNKR-CARX cells.

### Example 16: Optimized Members Having an Antigen Binding Domain or Other Extracellular Binding Domain

### Construction

The DNA encoding for the amino acid sequence of the anti-human EGFRvIII 139 scFv will be cloned with a CD8 hinge and transmembrane domain followed by the endodomain for 4-1BB and the first intracellular switch domain FKBP. The DNA corresponding to the intracellular signaling member having the second switch domain FRB and CD3 zeta will also be synthesized. Additionally, DNA will be synthesized whereby the FRB and FKBP domains are exchanged for one another. Additional switches may also be cloned substituting the co-stimulatatory endodomains shown in Table 2 for 4-1BB.

### Generation of Jurkat reporter cell line for initial characterization of CAR function

As an alternative to primary T cell transduction and activation, a Jurkat-NFAT reporter cell line can be used to evaluate the functional activity of CAR constructs. The Jurkat T cell line (E6-1) is transfected with a NFAT-luciferase reporter construct and a stable, clonal cell line (JNL) is selected for further characterization based on strong induction of the NFAT reporter following PMA and ionomycin stimulation.

### Transfection of Jurkat cell with NFAT-LUC reporter and the functional assay using purified proteins

Jurkat cells with NFAT-LUC reporter (JNL) will be grown to the density of 5 X10⁵ cells per mL in Jurkat cell growth media with puromycin at 0.5 ug/ml. For each transfection 2.5 X10⁶ cells will be spin down at 100g for 10 minutes. Two ug of DNA per construct will be used per transfection. Amaxa Nucleofector solution V and supplement I will be mixed and 100 ul will be added into the tube with each DNA construct. The mixture will be then added to the cells and transferred to the electroporation cuvette. Electroporation will be done under setting X-001 using Amaxa Nucleofector II Device. 500uL Growth media shall be added immediately after eletroporation and the mixture subsequently transferred into an additional 2 ml growth media in one well of the 6-well plate. The cells will be incubated in the 37C incubator with 5% CO2 for 24 hours. Tissue culture plate will be coated with 5 ug/ml of EGFRvIII-Fc or 5 ug/ml of IgG1-Fc for 2 hrs and blocked with 5% serum in DPBS for 1 hour. The transfected cells will be added to the target plate with 100 ul per well and incubated further for 18 hrs in the presence of varying concentrations of a suitable rapalogue. Luciferase One Glo reagent 100 ul will be added per well. The samples shall be incubated for 5 min at 37C and then luminescence will be measured using a luminometer.

The degree to which the construct improves persistence can be evaluated by methods described herein.

The concept, methods and compositions described in this example are applicable to RNKR-CARs. The concept, methods and compositions described in this example are applicable to RCAR/NKR-CARX cells and/or RNKR-CARX cells.

### Example 17: Generation and Characterization of Mutant FKBP/FRB Dimerization Switches

In this example, mutation of the residues involved in binding between the switch domains, e.g., FRB or FKBP, with the dimerization molecule was performed to identify mutations that enhance formation of a complex between FKBP, FRB, and the dimerization molecule, e.g., rapamycin or a rapalog, e.g., RAD001. Libraries of candidate mutant FKBP and FRB switch domains were generated and screened as described herein. Mutant FKBP or FRB allows the use of circulating concentrations of the dimerization molecule, e.g., RAD001, which are less than the concentrations used to mediate immunosuppression.

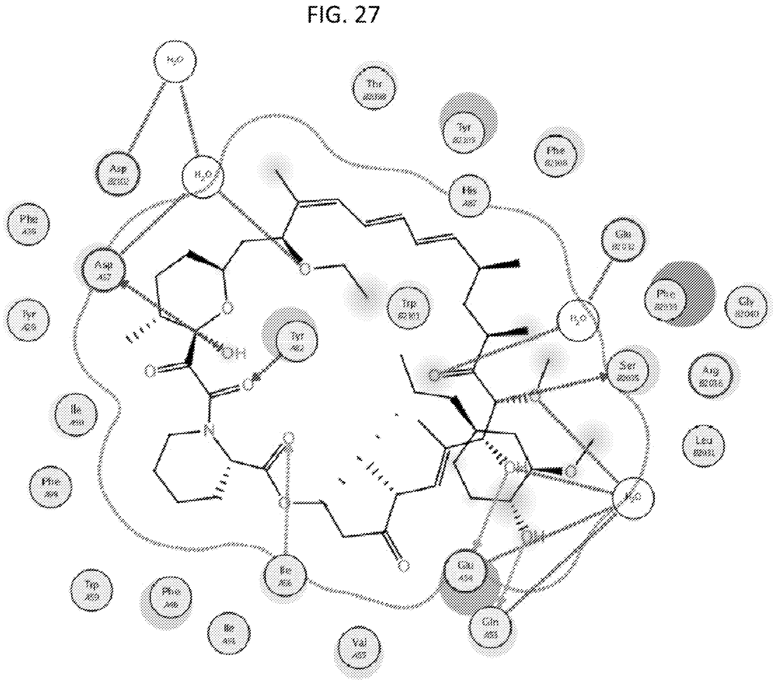

The interface between FKBP, FRB, and rapamycin is clearly defined allowing for inspection of the FRB/rapamycin and FRB/FKBP interface. In the 2.2 Å x-ray structure of the ternary FKBP/FRB/rapamycin complex, FRB residues Leu2031, Glu2032, Ser2035, Arg2036, Phe2039, Gly2040, Thr2098, Trp2101, Tyr2015, and Phe2108 make 38 direct contacts with rapamycin and FRB residues Arg2042 and Asp2102 make water mediated contacts with the compound (Liang et al., 1999, J. Acta Cryst. D55:736-744). Figure 27 shows the rapamycin interaction with FKBP and FRB which were determined in the x-ray structure of the ternary complex, RCSB code 2FAP, generated using the Molecular Operating Environment (MOE) (Clark et al., 2007, J. Chem. Inf. Model. 47(5):1933-1944). The FRB molecule is chain B in the structure.

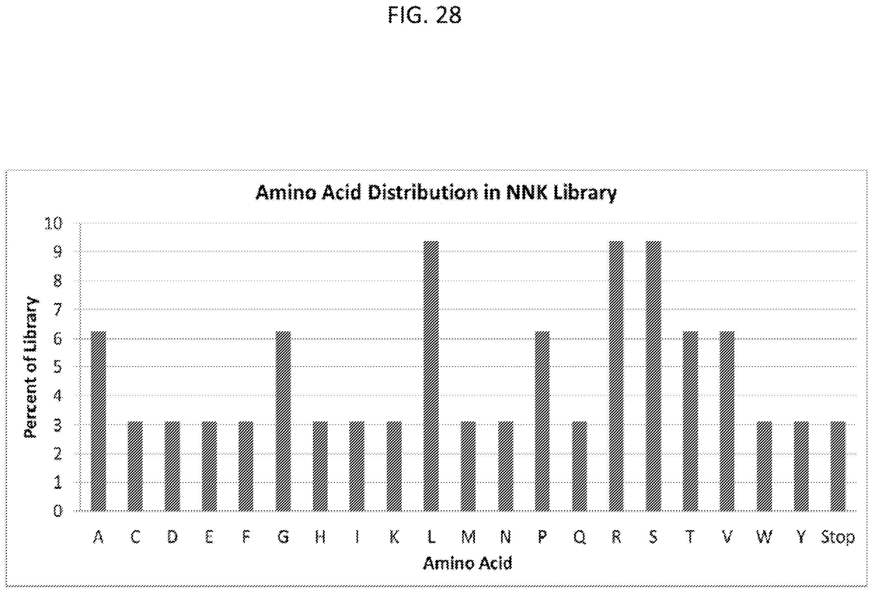

The FRB residues chosen for mutation included: L2031, E2032, S2035, R2036, F2039, G2040, T2098, W2101, D2102, Y2105, and F2108. Each point mutant library was generated by randomizing the codon at the desired position using an NNK library, where N can be adenine (A), cytosine (C), guanine (G), or thymine (T), and K can be guanine (G) or thymine (T). Table 13 shows the codon distribution of an NNK library and the corresponding amino acids. Figure 28 shows the distributions of the amino acids produced from the codons in the NNK library, ranging from a low of 3.1% to a high 9.4%. Each point mutant library was cloned into the pNAT43 vector with a N-terminal histidine tag. SEQ ID NOs: 195-205 give the amino acid composition of each point mutant library, where **X** indicates the position of the NNK library. The DNA for each library was transformed into Acella chemically competent *E. coli,* plated onto 100 mm LB agar plates with 50 µg/mL kanamycin sulfate, and incubated overnight at 37°C. 94 colonies from each library plate were transferred to Costar 2 mL pyramidal bottom 96-well plates with 1 mL of ZYP-5052 auto induction medium containing 75 µg/mL kanamycin sulfate. The plates were incubated for 40 hours at 800 rpm at 30°C in a micro plate incubator.

The candidate FRB clones were isolated as follows. First, the cells were lysed. The cells were pelleted by centrifugation at 2,000 × g at 4°C for 30 minutes. The supernatant was discarded and the cell pellets were stored at -80°C. The 96-well plates containing the cell pellets were removed from storage at -80°C and thawed at room temperature for 1 hour. 0.5 mL of 50 mM HEPES pH 7.5, 150 mM NaCl, 5 mM EDTA, 0.25% (v/v) Triton X-100, 2.5 mg/mL lysozyme were added to each well. The pellets were resuspended by pipetting 180 µL 60 times. The samples were incubated at room temperature for 0.1 to 1 hour. 0.5 mL of 50 mM HEPES pH 7.5, 150 mM NaCl, 20 mM CaCl₂, 20 mM MgCl₂, 0.5 mg/mL DNase I were added to each well. The samples were mixed by pipetting 180 µL 10 times. The plates were incubated for 30 minutes at room temperature. The lysed cells were pelleted by centrifugation at 2,000 × g at 4°C for 30 minutes. The supernatant was discarded from each plate by inversion followed by gentle tapping. The plates were stored overnight at -80°C.

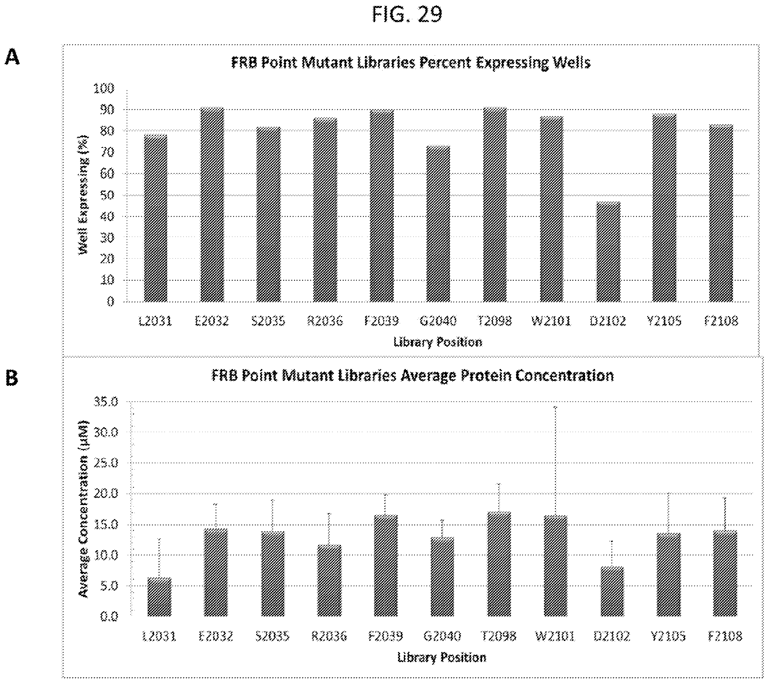

Next, the stored lysates were processed by affinity purification to isolate the mutant FRB as follows. The following morning, the plates were removed from storage at -80°C and thawed at room temperature for 1 hour. 0.7 mL of 50 mM HEPES, 500 mM NaCl, 5 mM TCEP, 5% (v/v) Triton X-100, pH 7.5 were added to each well. The pellets were resuspended by pipetting 180 µL 50 times, followed by a 1 hour incubation at room temperature. The plates were centrifuged for 30 minutes at 2,000 × g at 4°C and the supernatant for each was discarded. 0.5 mL of 50 mM HEPES pH 7.5, 1 mM TCEP, 60% ethanol were added to each well. The pellets were resuspended by pipetting 180 µL 50 times, followed by a 1 hour incubation at room temperature. The plates were centrifuged for 30 minutes at 2,000 × g at 4°C and the supernatant for each was discarded. 0.5 mL of 50 mM HEPES pH 7.5, 500 mM NaCl, 1 mM TCEP, 8 M urea were added to each well. The pellets were resuspended by pipetting 180 µL 50 times and incubated overnight at room temperature. The following morning, the samples were transferred to 20 µm fritted 96-well plates. The samples were filtered through the plates into new 2 mL Costar 96-well plates by centrifugation for 5 minutes at 1,500 × g at 4°C. A 25% slurry of Ni Sepharose 6 Fast Flow resin in 50 mM HEPES pH 7.5, 500 mM NaCl, 1 mM TCEP, 8 M urea was prepared. 100 µL of slurry, 25 µL of resin, were added to each well. The resin was incubated with the samples for 1 hour at room temperature. The resin was then transferred to 20 µm fritted 96-well plates and the column flow-through was removed by vacuum. 500 µL of 50 mM HEPES pH 7.5, 150 mM NaCl, 1 mM TCEP, 4 M urea was added to each well, incubated for 5 minutes, and removed by vacuum. 500 µL of 50 mM HEPES pH 7.5, 150 mM NaCl, 1 mM TCEP, 2 M urea was added to each well, incubated for 5 minutes, and removed by vacuum. 500 µL of 50 mM HEPES pH 7.5, 150 mM NaCl, 1 mM TCEP, 1 M urea was added to each well, incubated for 5 minutes, and removed by vacuum. 500 µL of 50 mM HEPES pH 7.5, 150 mM NaCl, 1 mM TCEP, 25 mM imidazole was added to each well, incubated for 5 minutes, and removed by vacuum. 200 µL of 50 mM HEPES pH 7.5, 150 mM NaCl, 1 mM TCEP, 500 mM imidazole was added to each and incubated for 5 minutes. The bound protein was eluted by centrifugation for 2 minutes at 500 × g at 4°C into a new 300 µL BD Falcon 96-well plate. The protein concentration in mg/mL for each well was measured using the Bradford assay with BSA as the standard. The protein concentrations were converted to µM by using the molecular weight for wild type FRB. The point mutant libraries had expression in a least 50% of the wells except for FRB D2102, which was 47%. Figure 29A shows the expression levels of each library and Figure 29B shows the average concentration for the expressing wells.

The inhibition for each well expressing protein for each library was calculated by using the well known to contain no protein as blank measurements. For each library plate, the average for the blank wells was calculated. Expressing wells with values greater than the average for the blank wells were defined to have 0% inhibition. The percent inhibition for wells with values less than or equal to the average for the blank wells was calculated by subtracting the average for the blank wells from the well value, dividing by -1 multiplied by the average for the blank wells, and multiplying by 100. When the well value was 0, there was 100% inhibition and when the well value was equal to the average of the blank wells, there was 0% inhibition. Wells with inhibition greater than or equal to 75% were chosen for re-array. Table 26 shows the number of wells selected for each library and the number of wells expected to be wild type FRB. 320 out of 1034 wells were chosen, 31.3%. The selected wells were grown, purified, and analyzed as described. The DNA for each of the selected wells was sequenced to identify the individual mutations. The protein concentration for each of the mutants was assessed by the Bradford assay. The activity of each mutant was compared with the ability of wild type FRB to bind to everolimus, e.g., RAD001, in multiple assay formats.

**Table 20. Wells selected for retesting for each point mutant library in the initial screen**

| **Library** | **Wells Selected** | **Expected Wild Type Wells** |
|---|---|---|
| L2031 | 41 | 9 |
| E2032 | 82 | 3 |
| S2035 | 33 | 9 |
| R2036 | 9 | 9 |
| F2039 | 15 | 3 |
| G2040 | 49 | 6 |
| T2098 | 57 | 6 |
| W2101 | 1 | 3 |
| D2102 | 11 | 3 |
| Y2105 | 6 | 3 |
| F2108 | 16 | 3 |

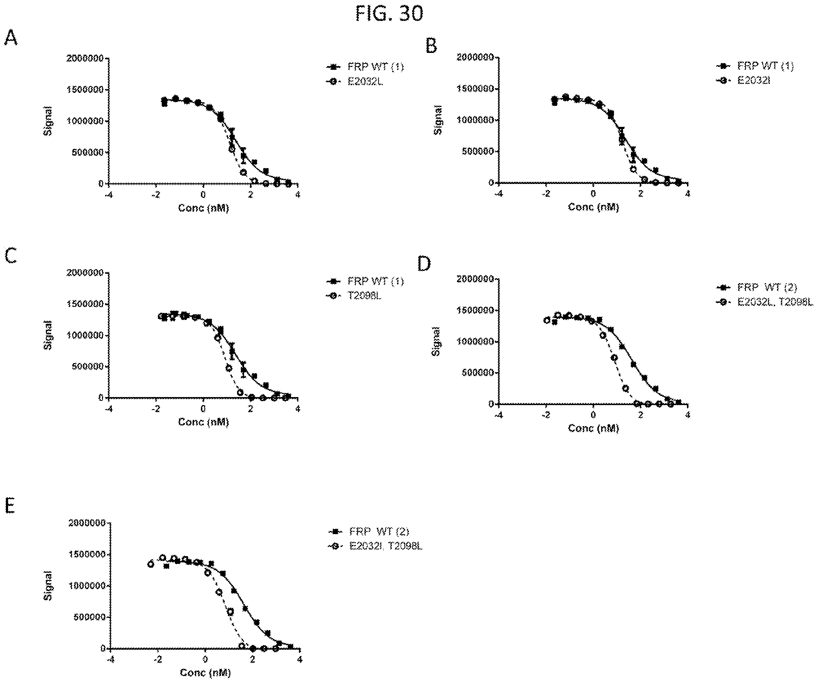

For the competition assay, FRB mutations of interest are ranked compared to wild type FRB. Unlabeled FRB proteins of interest (SEQ ID NOs: 207-211) and unlabeled wild type FRB (SEQ ID NO: 206) were serial diluted 1:3 from a starting final concentration of between 0.9 and 4uM dependent upon expression and added in solution with 30nM (final) wild type Flag-FRB (SEQ ID NO: 212) and 30nM (final) biotinylated wild-type FKBP (SEQ ID NO: 218) in the presence of 60nM (final) everolimus in a 96 well ½ surface flat-bottom plate (PerkinElmer). All dilutions were made in 1x AlphaLISA Immunoassay buffer (PerkinElmer). The plate was incubated for one hour at room temperature with mild shaking. Anti-Flag acceptor beads (PerkinElmer) were then added at 10ug/ml final concentration and incubated for one hour at room temperature with mild shaking. Streptavidin donor beads (PerkinElmer), were then added at a final concentration of 40ug/ml and the plate was protected from light for a 30 minute room temperature incubation with mild shaking. The plate was then read on the PerkinElmer EnVision Multiplate reader equipped with the Alpha Module using excitation of 680nm and a 570nm Emission filter. The EC50s of each FRB sequence from the competition assay are shown in Table 27 in comparison to WT FRB analyzed in the same plate. Single point mutations E2032L (SEQ ID NO: 208) and E2032I (SEQ ID NO: 207) were approximately 2-fold better than wild type (FIG. 30A and 30B; T2098L (SEQ ID NO: 209) was 3-fold improved (FIG. 30C). FRB proteins incorporating mutations at both sites (SEQ ID NOs: 210 and 211) demonstrated 5-fold relative improvement (Figure 30D and 30E).

**Table 21. EC50 Values from Competition Assay**

| Mutation | Ec50 (nM) |
|---|---|
| Wild type FRB (1) | 23.33 |
| E2032L | 12.94 |
| E2032I | 16.61 |
| T2098L | 8.255 |
| Wild type FRB (2) | 42.62 |
| E2032L, T2098L | 8.047 |
| E2032I, T2098L | 7.138 |

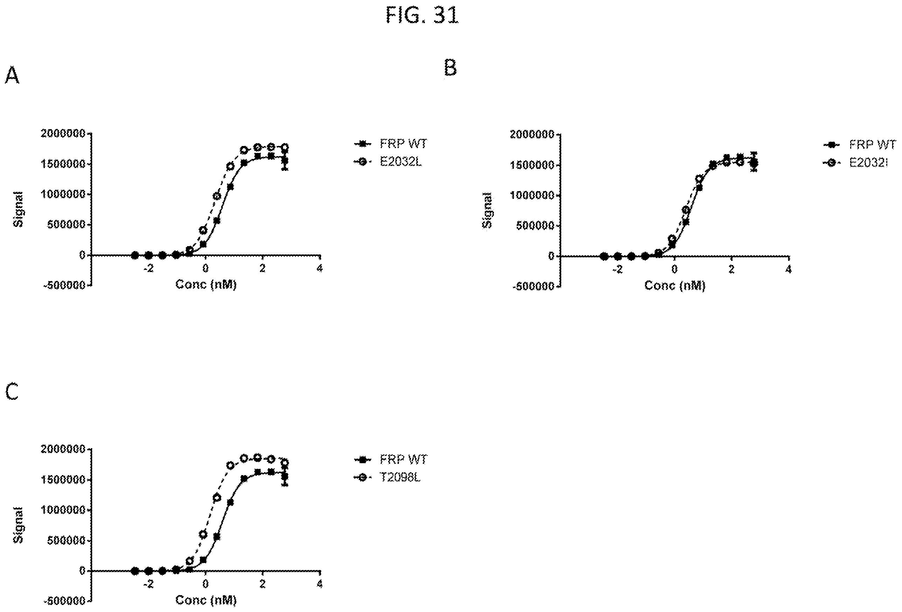

FRB mutations were also ranked in an alternative assay format. Briefly, FRB proteins incorporating single and double mutations (SEQ ID NO: 213-217) were produced as FLAG tagged constructs in *E. coli* as described previously. 30nM (final) of biotinylated FKBP (SEQ ID NO: 218) and each FLAG FRB protein were combined in the presence of everolimus serial diluted 1:3 from a starting final concentration of 600nM into a 96 well ½ surface flat-bottom plate (PerkinElmer) and incubated for one hour at room temperature. All dilutions were made in 1x AlphaLISA Immunoassay buffer (PerkinElmer). Anti-Flag acceptor beads (PerkinElmer) were added at 10ug/ml final concentration and incubated for one hour at room temperature. Streptavidin donor beads (PerkinElmer), were then added at a final concentration of 40ug/ml and the plate was protected from light and incubated for 30 minutes at room temperature. The plate was then read on the PerkinElmer EnVision Multiplate reader equipped with the Alpha Module using excitation of 680nm and a 570nm Emission filter. The EC50s of each FRB sequence from this assay are shown in Table 28. Single point mutations E2032I (SEQ ID NO: 213) and E2032L (SEQ ID NO: 214) were approximately 1.5-2-fold better than wild type (FIG. 31A and 31B); T2098L (SEQ ID NO: 215) was 3-fold improved (FIG. 31C). Flag-tagged FRB proteins which incorporated the double mutations (SEQ ID NO: 216 and 217) demonstrated limited dynamic range in this assay and therefore could not be evaluated.

**Table 22: EC50 Values from Direct Binding Assay**

| Mutation | Ec50 (nM) |
|---|---|
| Wild type FRB | 3.899 |
| E2032L | 2.146 |
| E2032I | 2.461 |
| T2098L | 1.442 |

**Table 23: Sequences of candidate mutant FRB and FRB used in binding assays**

| **Name** | **Amino Acid Sequence** | **SEQ ID NO:** |
|---|---|---|
| L2031 library | | 195 |
| E2032 library | | 196 |
| S2035 library | | 197 |
| R2036 library | | 198 |
| F2039 library | | 199 |
| G2040 library | | 200 |
| T2098 library | | 201 |
| W2101 library | | 202 |
| D2102 library | | 203 |
| Y2105 library | | 204 |
| F2108 library | | 205 |
| His-FRB (wild-type FRB) | | 206 |
| His-FRB E2032I | | 207 |
| His-FRB E2032L | | 208 |
| His-FRB T 2098L | | 209 |
| His-FRB | | 210 |
| E2032I, T2098L | | |
| His-FRB E2032L, T2098L | | 211 |
| His-FLAG-FRB (wild-type FRB) | | 212 |
| His-FLAG-FRB E2032I | | 213 |
| His-FLAG-FRB E2032L | | 214 |
| His-FLAG-FRB T2098L | | 215 |
| His-FLAG-FRB E2032I, T2098L | | 216 |
| His-FLAG-FRB E2032L, T2098L | | 217 |
| His-Avidin-FKBP (wild-type FKBP) | | 218 |

The concept, methods and compositions described in this example are applicable to RNKR-CARs. The concept, methods and compositions described in this example are applicable to RCAR/NKR-CARX cells and/or RNKR-CARX cells.

### Example 18: Activation of RCAR with an extracellular switch

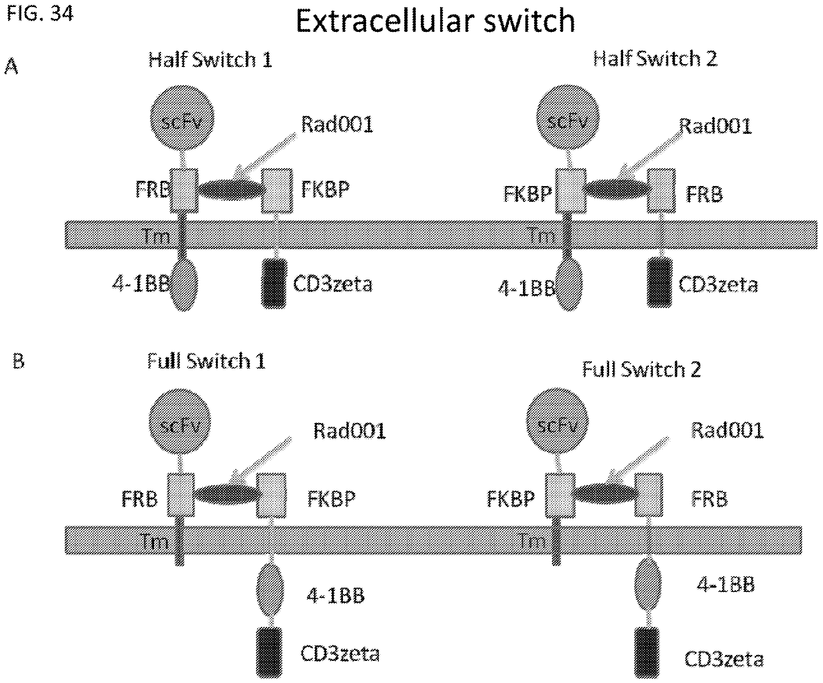

In this example, the activation of RCAR with an extracellular switch was evaluated in the presence of the dimerization molecule RAD001. Four RCARs were tested: two half switch constructs, e.g., with the dimerization switch in both orientations (Fig. 34A), and two full switch constructs, e.g., with the dimerization switch in both orientations (Fig. 34B), where the dimerization switch is an extracellular FKBP/FRB dimerization, as depicted in Figs. 34A and 34B. In the half-switch constructs, the antigen binding member comprises a scFV domain, an extracellular switch domain, a transmembrane domain, an intracellular costimulatory signaling domain 4-1BB, and the intracellular signaling member comprises an extracellular switch domain, a transmembrane domain, and an intracellular primary signaling domain, CD3zeta (Fig. 34A). In the full-switch constructs, the antigen binding member comprises a scFv domain, an extracellular switch domain, and a transmembrane domain, and the intracellular signaling member comprises an extracellular switch domain, a transmembrane domain, an intracellular costimulatory signaling domain, 4-1BB, and an intracellular primary signaling domain, CD3zeta (Fig. 34B).

### Materials and Methods

The RCAR constructs were expressed in a Jurkat reporter cell line. Jurkat cells with NFAT-LUC reporter (JNL) were grown to the density of 0.5 x 10⁶/ml in Jurkat cell growth media with puromycin at 0.5 µg/ml. For each transfection 2.5 x 10⁶ cells were spin down at 100g for 10 minutes. Two µg of DNA per RCAR construct were used per transfection. Amaxa Nucleofector solution V and supplement I was mixed and 100 µl was added into the tube with DNA construct. The mixture was then added to the cells and transferred to the electroporation cuvette. Electroporation was done under setting X-001 using Amaxa Nucleofector II Device. 0.5 ml of growth media was added immediately after eletroporation and the mixture were transferred into 2 ml growth media in one well of the 6-well plate.

After one hour, RAD001 compound was applied at various concentrations: 0 nM, 0.01 nM, 0.03 nM, 0.1 nM, 0.3 nM, 1 nM, 5 nM, and 50 nM. Tissue culture plate was coated with 5 µg/ml of anti anti-CD 19 antibody or isotype control for 2 hrs, blocked with the blocking buffer (DPBS with 5% serum) for 1 hour. The transfected cells with or without Rad001 were resuspended and added to the target plate with 100 µl per well and incubated for 18 hrs. Luciferase One Glo reagent 100 µl was added per well. The samples were incubated for 5 min at 37°C and then luminescence was measured using Envision plate reader.

### Results

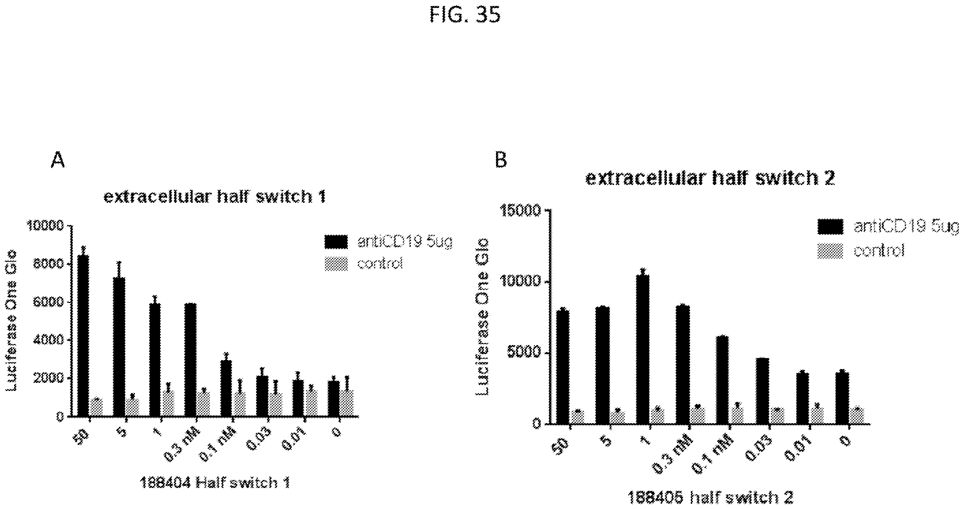

As shown Figs. 35A and 35B, increasing the concentration of RAD001 correlated with increase in NFAT activity for both extracellular half switches, as represented by the luminescence detected. A dose-dependent response was observed for both RCAR half switch constructs. For the half switch 2, NFAT activity peaked at the 1 nM RAD001 dose, while NFAT activity decreased at the higher Rad001 dosages (5 nM and 50 nM). The observed decrease in activation may be due to general immune suppression by the higher dosages of RAD001. For the half switch 1, no inhibition of activity was observed with the higher dosages of RAD001, e.g., 5 nM and 50 nM.

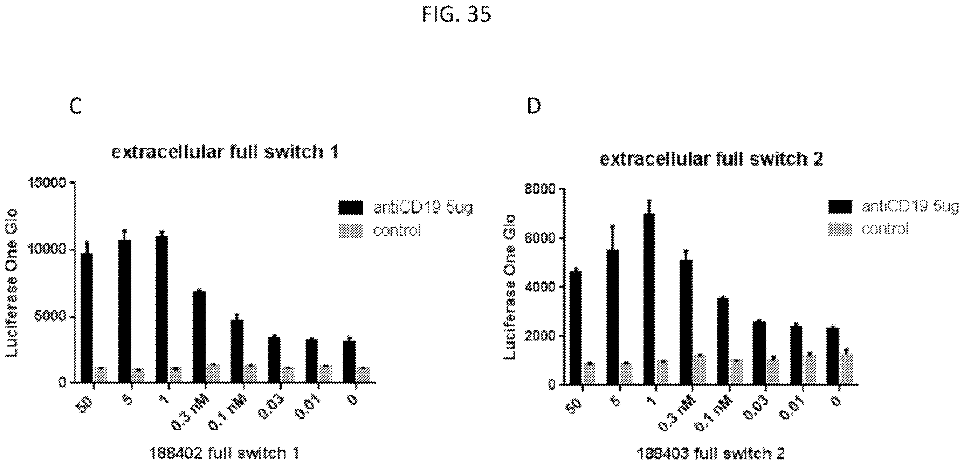

As shown Figs. 35C and 35D, increasing the concentration of RAD001 also correlated with increase in NFAT activity for both extracellular full switches, as represented by the luminescence detected. The pattern of activation for the RCAR full switches was similar to that of the RCAR half switch 2 (Fig. 34B), where NFAT activity peaked at the 1 nM RAD001 dose and decreased at the higher Rad001 dosages, e.g., 5 nM and 50 nM.

These data show that RAD001 triggers dimerization of the extracellular FKBP/FRB switch domains present in the RCAR half switch and full switch constructs and target-dependent NFAT activation.

The concept, methods and compositions described in this example are applicable to RNKR-CARs. The concept, methods and compositions described in this example are applicable to RCAR/NKR-CARX cells and/or RNKR-CARX cells.

### Example 19: Activation of RCAR half switches

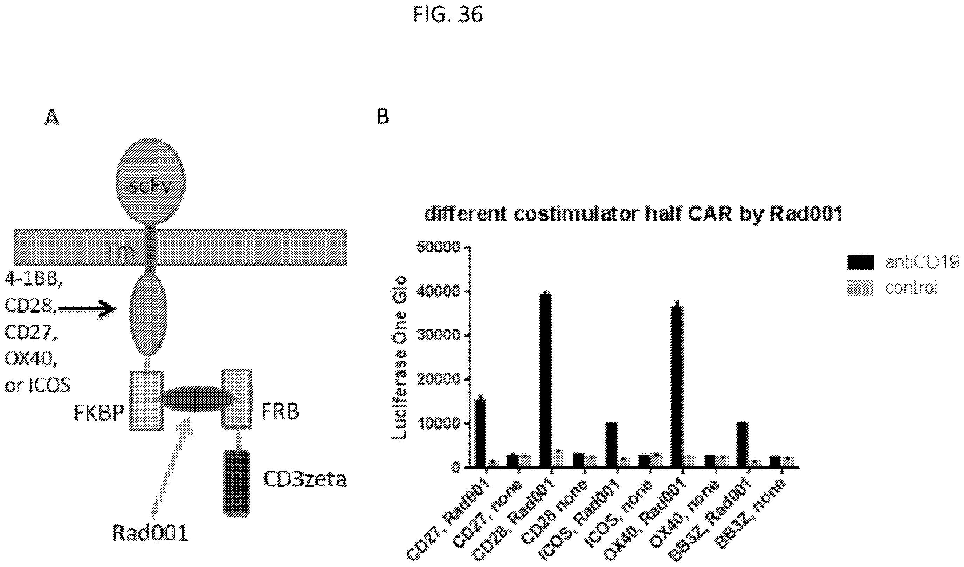

In this example, the activation of a RCAR half switch was evaluated in the presence of the dimerization molecule RAD001. Two RCAR constructs were tested where the dimerization switch is an intracellular FKBP/FRB dimerization switch, and the switch domains are located in both orientations with respect to the antigen binding member and the intracellular member. For example, the antigen binding member comprises a scFV domain, a transmembrane domain, an intracellular switch domain, and an intracellular costimulatory signaling domain; and the intracellular signaling member comprises an extracellular switch domain, a transmembrane domain, and an intracellular primary signaling domain, e.g., CD3zeta (Fig. 36A).

### Materials and Methods

Jurkat cells with NFAT-LUC reporter (JNL) were grown to the density of 0.5 x 10⁶/ml in Jurkat cell growth media with puromycin at 0.5 µg/ml. For each transfection 2.5 x 10⁶ cells were spin down at 100g for 10 minutes. Two µg of DNA per construct were used per transfection. Amaxa Nucleofector solution V and supplement I was mixed and 100 µl was added into the tube with DNA construct. The mixture was then added to the cells and transferred to the electroporation cuvette. Electroporation was done under setting X-001 using Amaxa Nucleofector II Device. 0.5 ml of growth media was added immediately after eletroporation and the mixture were transferred into 2 ml growth media in one well of the 6-well plate.

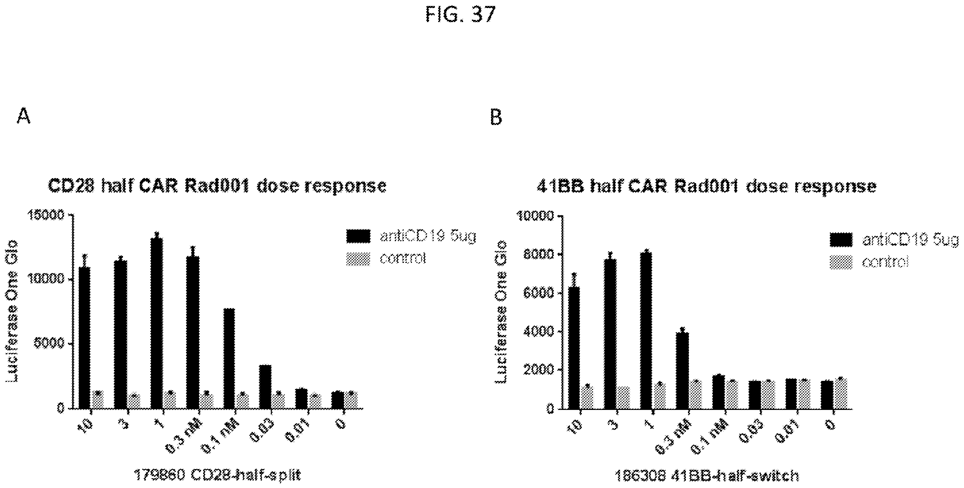

After one hour, RAD001 compound was delivered at 50 nM when testing RCARs with different costimulatory signaling domains (Fig. 36), or various concentrations: 0 nM, 0.01 nM, 0.03 nM, 0.1 nM, 0.3 nM, 1 nM, 3 nM, and 10 nM (Fig. 37). Tissue culture plate was coated with 5 µg/ml of anti anti-CD 19 antibody or isotype control for 2 hrs, blocked with the blocking buffer (DPBS with 5% serum) for 1 hour. The transfected cells with or without RAD001 were resuspended and added to the target plate with 100 µl per well and incubated for 18 hrs. Luciferase One Glo reagent 100 µl was added per well. The samples were incubated for 5 min at 37°C and then luminescence is measured using Envision plate reader.

### Results

Five different RCAR half switch constructs were generated with different costimulatory signaling domains: CD27, CD28, ICOS, OX40, and 4-1BB; and NFAT activation after incubation with Rad001 was evaluated. As shown in Fig. 36B, RCARs containing CD27, CD28, ICOS, OX40 and 41BB as the costimulatory domain, with CD3zeta as the primary signaling domain, were all able to activate the target-dependent NFAT activity after addition of RAD001. The RCAR with CD28-CD3 zeta and OX40-CD3 zeta dimmers were demonstrated to have the most robust activation as compared to the other costimulatory signaling domain-CD3 zeta dimers (e.g., 4-1BB-CD3 zeta, ICOS-CD3zeta, and OX40-CD3zeta).

Two RCAR half switches, containing either CD28 or 4-1BB costimulatory signaling domains, were also assessed for NFAT activity in response to increasing doses of RAD001. As shown in Fig. 37A and 37B, both half switches generally exhibited dose-dependent NFAT activity, where increased RAD001 concentrations correlated with increased luminescence, or NFAT activity. For both half switches, NFAT activity peaked at the 1nM RAD001 concentration, with NFAT activity decreasing at the higher RAD001 concentrations (e.g., 3 nM and 10 nM).

These results show that RAD001 triggers dimerization of RCAR half switches with various different costimulatory signaling domains and target-dependent NFAT activation.

The concept, methods and compositions described in this example are applicable to RCAR/NKR-CARX cells.

### Example 20: Analysis of Half-Switch Constructs

To evaluate the feasibility of the half-switch technology, lentiviruses were produced for all of the half-switch constructs and T cells were transduced. The constructs tested were composed of two genes, coexpressed from one vector using the EMCV IRES. The genes encoded two proteins, which were a) the anti-CD 19 scFv fused to the CD8 hinge and transmembrane domain, a costimulatory signaling domain and the FKBP heterodimerizing domain and b) the FRB heterodimerizing fused to the CD3z cytoplasmic domain. The costimulatory signaling domains tested were 41BB, CD28, CD27, ICOS and OX40. The half-switches were compared to a 41BB full-switch CAR and the non-regulatable 41BB CAR. All CAR-transduced T cells (CARTs) were tested for effector T cell responses, namely target cell killing and target cell-induced proliferation and cytokine production.

### Materials and Methods

### Generation of CAR-transduced T cells (CARTs)

The CAR lentiviral transfer vectors are used to produce the genomic material packaged into the VSVg pseudotyped lentiviral particles. Lentiviral transfer vector DNA is mixed with the three packaging components VSVg env, gag/pol and rev in combination with lipofectamine reagent to transfect Lenti-X 293T cells. Medium is changed after 24h and after another 24h, the media is collected, filtered and stored at -80°C. CARTs are generated by transduction of fresh or frozen naive T cells obtained by negative magnetic selection of healthy donor blood. T cells are activated by incubation with anti-CD3/anti-CD28 beads for 24h, after which 1mL of viral supernatant or concentrated virus (moi=10) is added to the cultures. These modified T cells are allowed to expand for about 10 days. The percentage of cells transduced (expressing the CARs on the cell surface) and the level of CAR expression (relative fluorescence intensity, Geo Mean) are determined by flow cytometric analysis between days 7 and 9. The combination of slowing growth rate and T cell size approaching -300 fL determines the state for T cells to be cryopreserved for later analysis.

### Evaluating cytolytic activity, proliferation and cytokine secretion of CARTs

To evaluate the functionality of CARTs, the T cells are thawed, counted and viability assessed by Cellometer. The number of CAR-positive cells in each culture is normalized using non-transduced T cells. The induction of the regulatable CARTs was tested in titrations with RAD001, starting at 50nM. The target cell line used in all co-culture experiments is Nalm-6, a human pre-B cell acute lymphoblastic leukemia (ALL) cell line expressing CD19 and transduced to express luciferase. The human glioblastoma line U87MG expressing luciferase serves as negative control.

The cytolytic activities of CARTs are measured at an effector:target ratio of 4:1, where effectors were defined as total T cells and targets the respective positive or negative cancer lines. After 20h of co-culture, cultures are lysed and a substrate for luciferase is added (BrightGlo) to quantify surviving target cells. Plates are read out on the luminometer (EnVision) and specific lysis (%) is calculated as lum(sample)/lum(max)^{∗}100.

For measuring cytokine production by CARTs, T cells are cultured with target cells at a ratio of 1:1. In addition, PMA/Ionomycin is used to evaluate the maximal secretion of the CART populations and CAR T cells alone give a read-out of basal activity. The assay is run for 24h, when the media is removed for cytokine analysis using the CBA kit for human cytokine detection; the amounts of IFNγ, IL2 and TNFα were measured.

For measuring the proliferation of CARTs, T cells are cultured with target cells at a ratio of 1:1. The assay is run for 4 days, when cells are stained for CD3, CD4, CD8 and CAR expression. The number of T cells is assessed by flow cytometry using counting beads as reference.

### Results

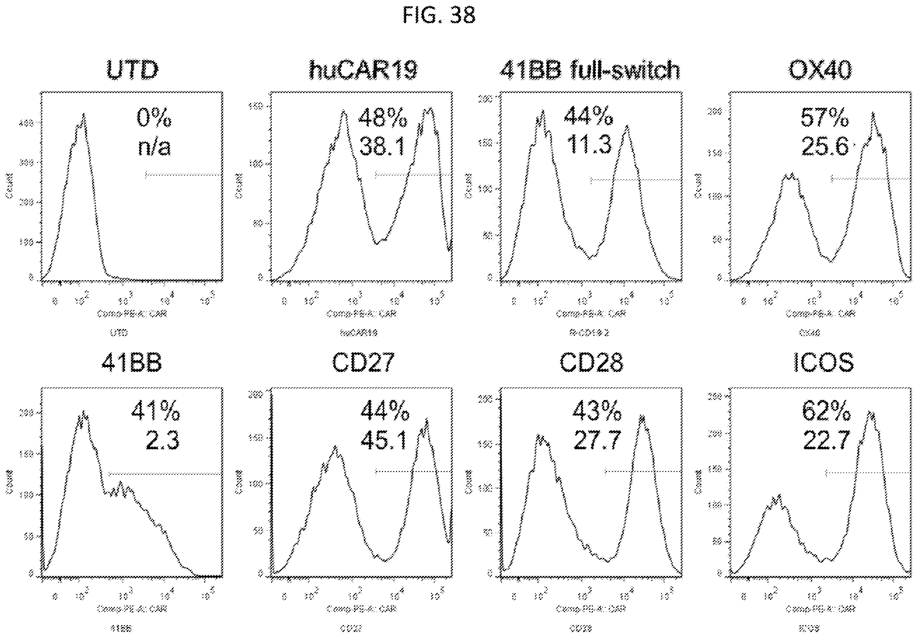

Most CARs used for this experiment showed very similar surface expression; the standard huCART19, the 41BB full-switch as well as the OX40, CD27, CD28 and ICOS half-switches are well expressed and comparable regarding percent CAR+ population and number of CAR molecules per cell (GeoMean). Only the 41BB half-switch showed lower expression on a per-cell basis, while the population of CAR-positive cells is similar to the other CARTs (Fig. 38).

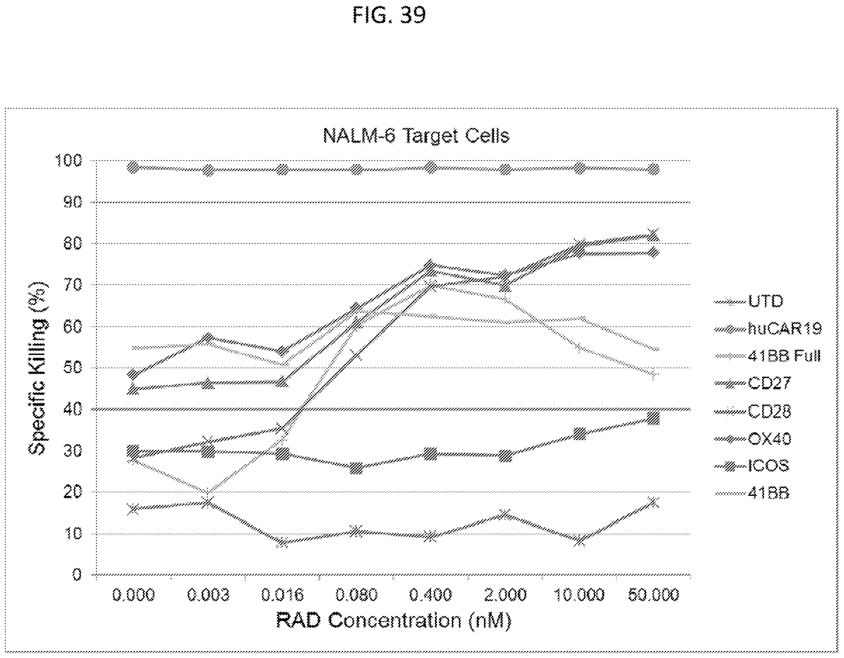

The potential of these CARs to kill CD19-positive target cells (Nalm6-Luc) in the presence of different concentrations of RAD001 was tested in a 20h assay. Almost 100% killing was seen for the non-inducible huCART19, while non-transduced T cells (UTD) showed background killing (Fig. 39). The 41BB full-switch CARTs showed an induction of killing with increasing RAD concentrations, with a maximum at 0.4 nM RAD001, and a decrease in killing at higher concentrations. CD28, CD27 and OX40 half-switch CARTs showed an increase in killing, with a maximum at the highest concentration of RAD001. The maximal killing seen for these half-switches was higher than seen for the full-switch. The 41BB and ICOS half-switch CARTs showed non-inducible killing at moderate and low levels, respectively.

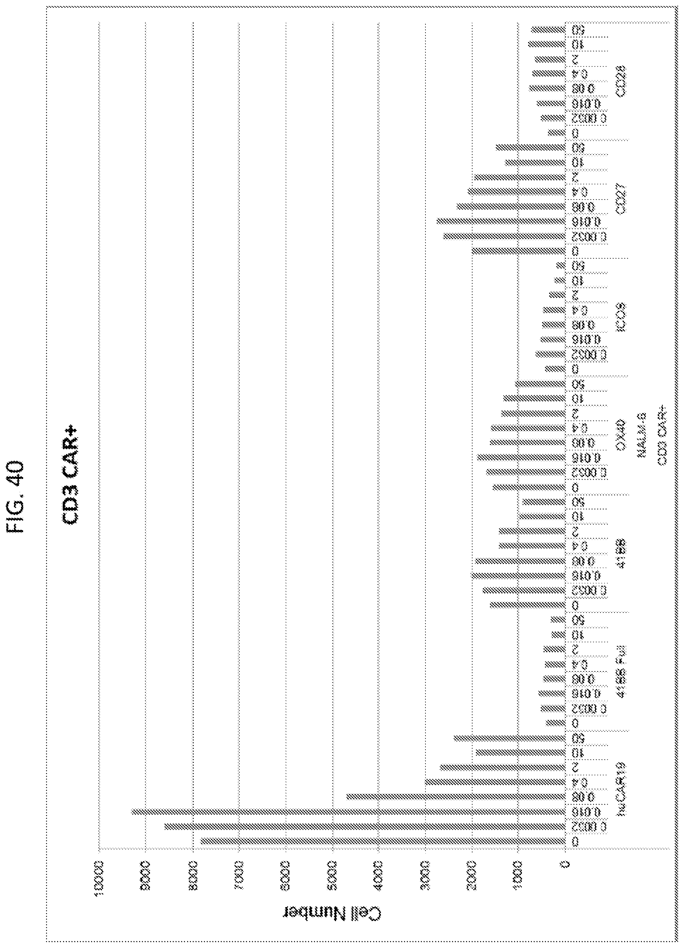

The proliferative capacity of CART cells was tested in a 4 day co-culture assay. Number of CAR-positive CD3-positive T cells was assessed after culturing the differently transduced T cells with Nalm6 (Fig. 40). huCART19 cells expanded dramatically when cultured in the presence of less than 0.016 nM of RAD001, and to a lesser extent at higher concentrations of the compound. Half-switches 41BB, OX40 and CD27 expanded to similar, lower levels, showing a maximum at 0.016 nM RAD001. The ICOS and CD28 half-switches and the 41BB full-switch did not show detectable expansion.

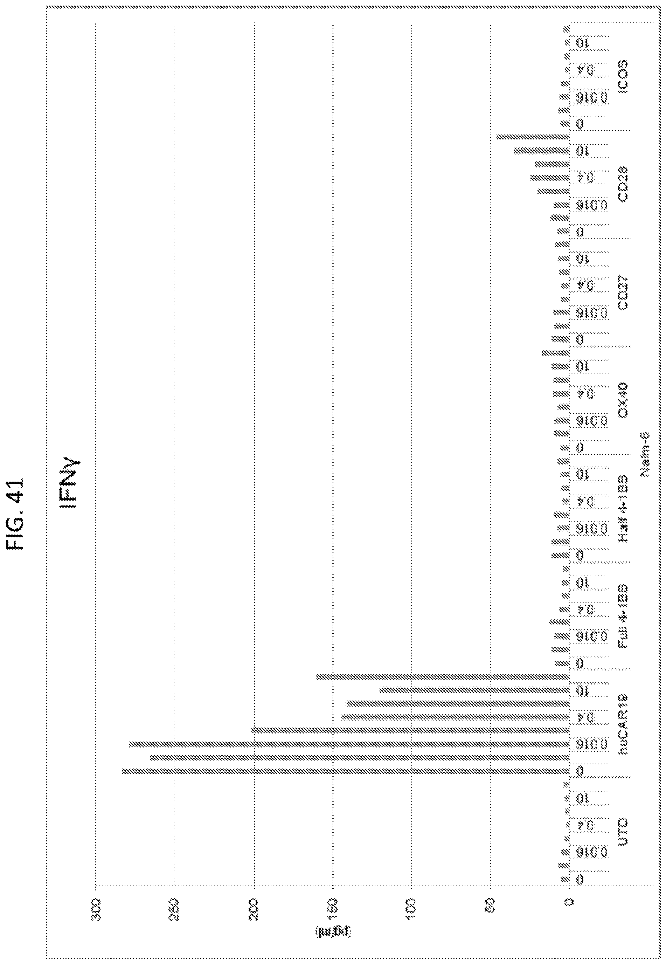

The capabilities of the regulatable CARTs to produce cytokine were tested in a similar assay, where CART cells were cultured with Nalm6 cells at a ratio of 1:1 for 20h. The supernatant was harvested and concentrations of IFNγ were measured. Again, we saw the strongest function by huCART19 and an inhibition at higher RAD001 levels (Fig. 41). Among the switch CARTs, the CD28 half-switch was the only showing a clear increase in secreted IFN. The highest induction was seen for the highest levels of RAD001.

The concept, methods and compositions described in this example are applicable to RCAR/NKR-CARX cells.

### Example 21: Covalent dimerization switch by Halo/snap tag

In this example, the activation of a RCAR with a covalent dimerization switch was evaluated after addition of the dimerization molecule NVP-HAL421. Examples of RCAR constructs with covalent dimerization switches are shown in FIG. 13.

### Materials and Methods

Jurkat cells with NFAT-LUC reporter (JNL) were grown to the density of 0.5 x 10⁶/ml in Jurkat cell growth media with puromycin at 0.5 µg/ml. For each transfection 2.5 x 10⁶ cells were spin down at 100g for 10 minutes. Two µg of DNA per construct were used per transfection. Amaxa Nucleofector solution V and supplement I was mixed and 100 µl was added into the tube with DNA construct. The mixture was then added to the cells and transferred to the electroporation cuvette. Electroporation was done under setting X-001 using Amaxa Nucleofector II Device. 0.5 ml of growth media was added immediately after electroporation and the mixture were transferred into 2 ml growth media in one well of the 6-well plate.

After one hour, NVP-HAL421 was applied at various concentrations, e.g., 0 nM, 50 nM, 500 nM, and 5 µM; or 0 nM, 0.06 nM, 0.2 nM, 0.6 nM, 2 nM, 6 nM, 20 nM, and 60 nM. Tissue culture plate was coated with 5 µg/ml of EGFRVIII-Fc or IgG1 Fc control for 2 hrs, blocked with the blocking buffer (DPBS with 5% serum) for 1 hour. The transfected cells with or without were re-suspended and added to the target plate with 100 µl per well and incubated for 18 hrs. Luciferase One Glo reagent 100 µl was added per well. The samples were incubated for 5 min at 37°C and then luminescence is measured using Envision plate reader.

### Results

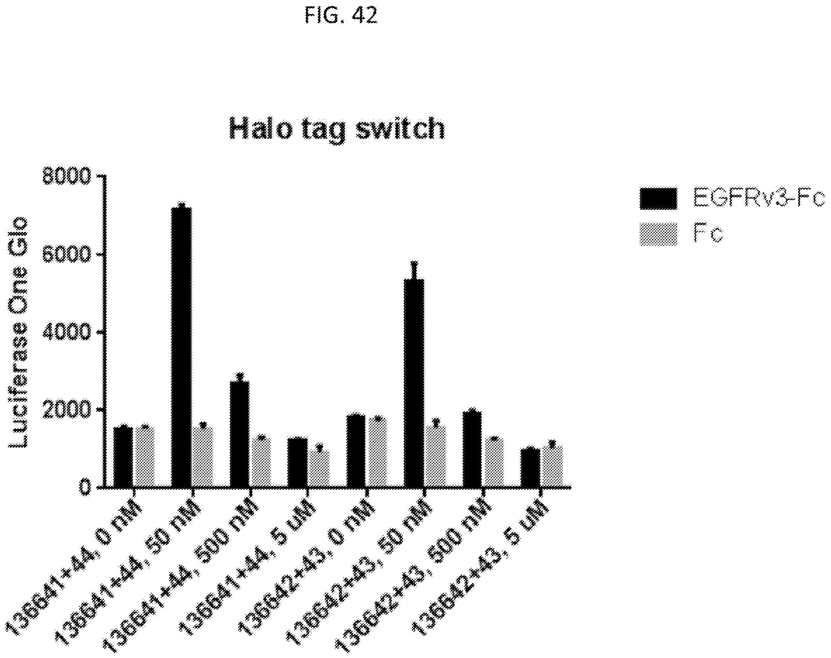

The concept of a RCAR with halo-tag and snap-tag as the switch domains is illustrated in Fig. 13. JNL cells were co-transfected with an RCAR construct comprising halo-tag and snap-tag switch domains. The addition of NVP-HAL421 at 50 nM resulted in covalent linkage between the halo-tag and snap-tag switch domains, which in term lead to NFAT activation, as shown in Fig 42. The level of NFAT activity peaked at 50 nM of NVP-HAL421, while NFAT activation was decreased at the higher concentrations of NVP-HAL421 (500 nM and 5 µM), as compared to the activity at 50 nM. This decrease in target-specific activation could be due to the toxicity of the compound.

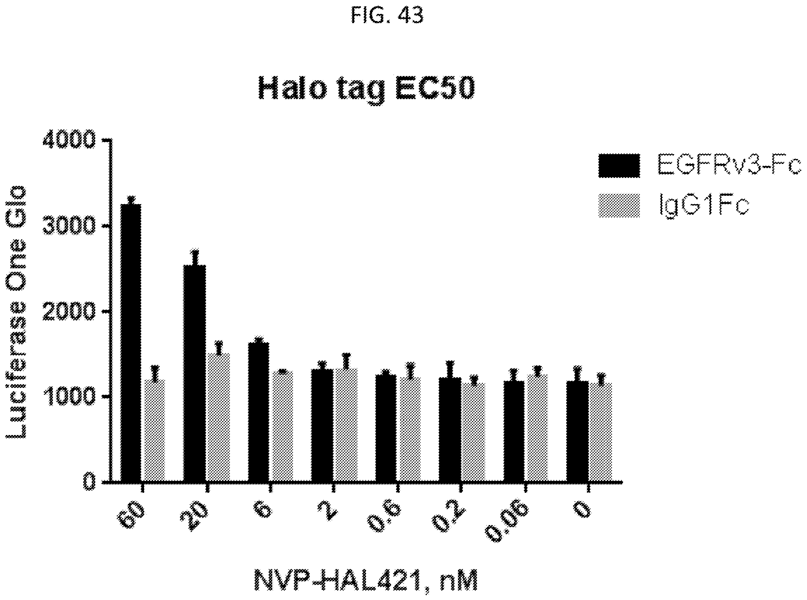

A second assay was performed to evaluate the NFAT activity for varying dosages of NVP-HAL421 between 0nM and 60 nM. As shown in Fig. 43, NFAT activity increased as dosage of dimerization molecule NVP-HAL421 increased, with the highest level of NFAT activity detected at the highest tested dosage, 60 nM.

The concept, methods and compositions described in this example are applicable to RNKR-CARs. The concept, methods and compositions described in this example are applicable to RCAR/NKR-CARX cells and/or RNKR-CARX cells.

### Example 22: Killing assay with RCAR-expressing primary T cells

The ability for RCAR T cells (RCAR T cell generation described above) to target and kill tumor cells was assessed *in vitro.* A luciferase-based assay was used, with tumor cell lines expressing luciferase reporters. Target cells (tumor cells) were plated at 25,000 cells/well. RCAR T cells, as well as standard CAR-expressing T cells and T cells expressing no CARs were added at the effector:target ratio 1:1 in duplicates. Additionally, RAD001 was added in seven 5-fold dilution steps, starting at 50 nM. One duplicate received pure medium, no RAD001. The cells were cultured for 20 hours, after which the luminescence of each well was detected to determine the percentage of target cells killed (BrightGlo, Promega protocol). Specific killing (%) = Luminescence(sample) / Luminescence(no T cells) ^{∗} 100. The luciferase-expressing Nalm6 (human pre-B ALL) cell line was used as the target cell.

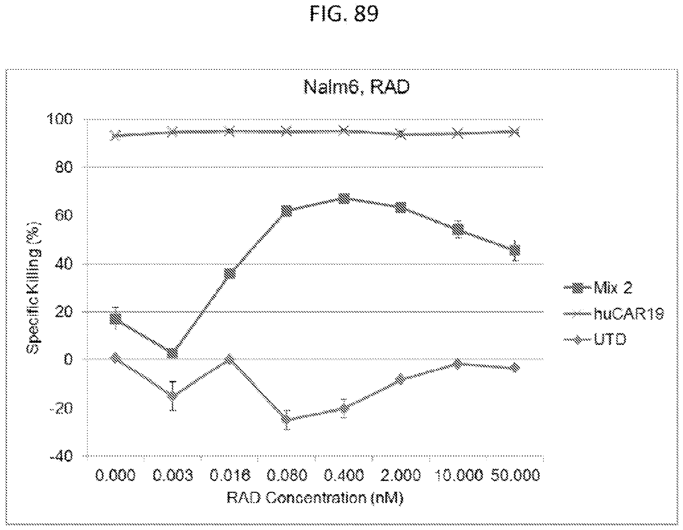

As shown in FIG. 89, target cell killing by T cells expressing the standard CART19 was not influenced by the addition of RAD001. Killing by RCAR-expressing T cells was induced by the addition of RAD001 in a dose dependent manner; showing background levels with no RAD001 and maximal killing at a RAD001 concentration around 1 nM.

The concept, methods and compositions described in this example are applicable to RNKR-CARs. The concept, methods and compositions described in this example are applicable to RCAR/NKR-CARX cells and/or RNKR-CARX cells.

### Example 23: Chimeric NK receptors

The results presented herein demonstrate an alternative approach to constructing CARs for T cells that can be more finely regulated compared with current CAR designs. Experiments were designed to develop a novel, regulated CAR system that comprises at least two or three chimeric fusion proteins. The primary T cell activating signal and inhibitory signals are based upon naturally occurring activating and inhibitory receptors of NK cells known as killer cell immunoglobulin-like receptors (KIRs).

KIRs exist as both activating and inhibitory forms that depend upon the intracellular domain of the receptor. Activating KIRs deliver their signals through an interaction with the immunotyrosine-based activation motif (ITAM) containing membrane protein, DAP12 that is recruited by residues within the transmembrane domains of these proteins. Inhibitory KIRs bear a cytoplasmic domain that contains immunotyrosine-based inhibitory motifs (ITIMs), which abrogate the activating signal leading to inhibition of NK cytolytic and cytokine producing activity. Similar to TCRs, KIRs belong to the immunoglobulin family of protein receptors, and many bind to invariant MHC and MHC-like ligands. Without wishing to be bound by any particular theory, it is believed that these interactions are utilized to naturally distinguish normal cells (usually expressing high density MHC class I) from malignant or virally infected cells (often with low or missing MHC class I).

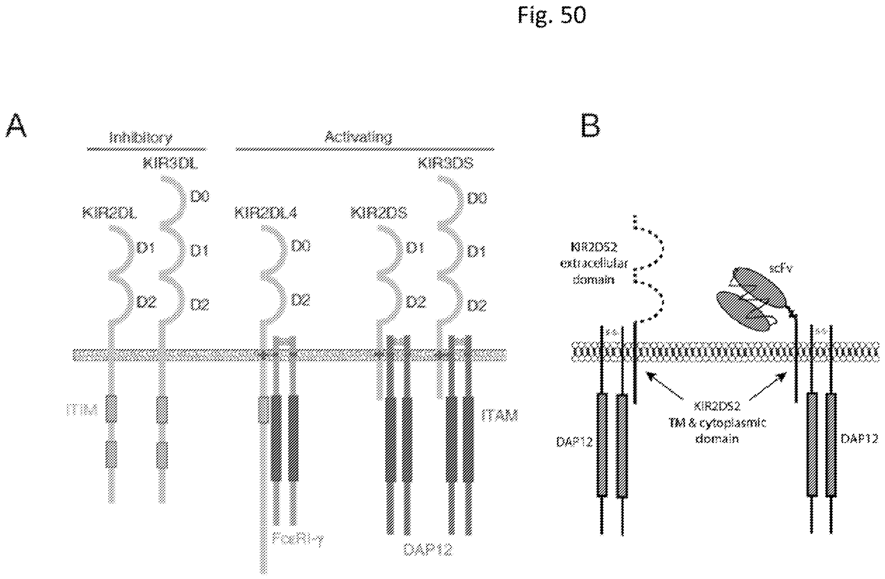

KIR-like chimeric antigen receptors (KIR-CARs) have been constructed which fuse an scfv to a target antigen of interest with activating and inhibitory KIRs as shown in Figure 50. Conditional activation of T cells is generated by engagement of an activating KIR-CAR (actKIR-CAR) or standard TCR-zeta CAR bearing an scfv to an antigen on the malignant cell of interest. An inhibitory CAR (inhCAR) bearing an scfv directed against an antigen that is present on normal, but not malignant tissue would provide dampening of the activating CAR primary signal when the T cell encounters normal cells. Examples of antigens that serve as useful targets for inhibitory CARs include the ephrin receptors (Pasquale, 2010, Nat Rev Cancer 10(3):165-80) and claudins (Singh et al., 2010, J Oncol, 2010:541957), which are expressed by epithelial cells from normal tissues, but often selectively lost by cancers (e.g. EPHA7).

The concept, methods and compositions described in this example are applicable to RNKR-CARs. The concept, methods and compositions described in this example are applicable to RCAR/NKR-CARX cells and/or RNKR-CARX cells.

### Example 24: Activating KIR-CAR Construction and Activity

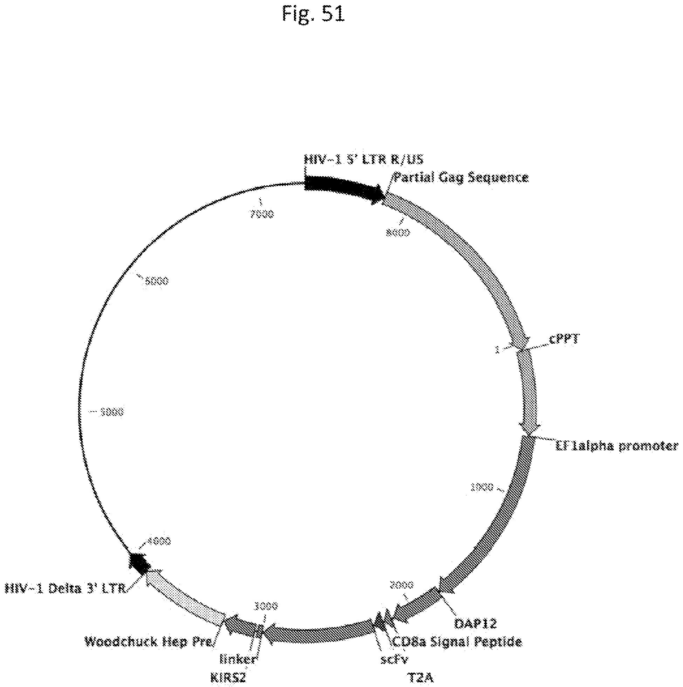

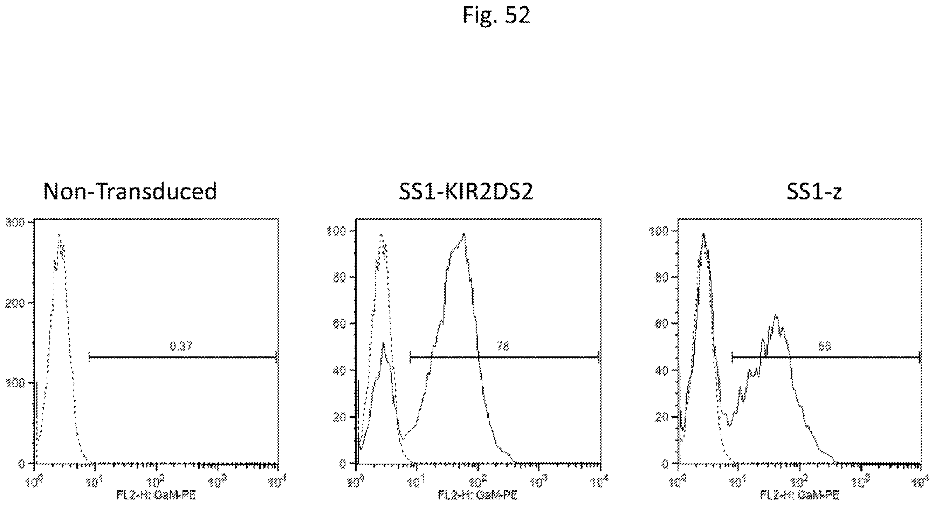

Experiments were designed to construct activating KIR-CARs based upon fusion of the anti-CD 19 or anti-mesothelin scFv (SS-1) that were previously incorporated into CARs based upon the TCR-zeta cytoplasmic domain that are currently in clinical trials. The human KIR2DS2 activating KIR receptor was chosen as the initial base receptor for the actKIR-CAR. In order to deliver activating signals, the actKIR-CARs required coexpression of DAP12, which is not expressed normally in T cells. Therefore, a lentiviral vector that expresses both the actKIR-CAR with human DAP12 using a "bicistronic" gene cassette based upon the 2A ribosomal skip peptide was constructed. A diagram of the lentiviral vector is illustrated in Figure 51. Initial studies demonstrated that the actKIR-CARs were efficiently expressed in primary human T cells and the SS1 actKIR-CAR bound to mesothelin (Figure 52). Similar to the previously developed and published SS1 scFv CD3 zeta (SS1-ζ) CAR (Carpenito et al., 2009, Proc Natl Acad Sci USA 106(9):3360-5), T cells expressing the SS1 actKIR-CAR demonstrated cytotoxic activity towards target K562 cells engineered to express the mesothelin ligand (KT-meso) as shown in Figure 53. Neither receptor exhibits killing of wild-type K562 lacking the mesothelin target.

Since the cytotoxic activity of the SS1 KIR CAR towards mesothelin-positive target cells was lower than the standard TCRzeta-based CAR targeting the same antigen with comparable CAR surface expression, it is believed that the mesothelin CAR may have an extracellular hinge (based upon wild-type KIR2DS2) that is non-optimal for segregation from CD45 due to its length. The kinetic segregation of activating ITAM-based receptors from CD45 is believed to be a key mechanisms for TCR activation, and dependent upon a length scale between the T cell and target cell membranes of -14-15 nm (Choudhuri et al., 2005, Nature 436(7050):578-82). It is estimated that the KIR2DS2 based SS1 KIR-CAR to have a length scale of greater than 20 nm based upon the partial crystal structure of mesothelin demonstrating that the SS1 epitope is likely at an -10 nm distance from the target cell membrane (Ma et al., 2012, J Biol Chem 287(40):33123-31) and CAR that is estimated to be ∼ 10 nm assuming each Ig-like domain is -3.5 nm in the KIR2DS2 hinge in addition to the scFv. Therefore an activating KIR CAR in which the KIR2DS2 hinge was removed (KIRS2 CAR) as shown schematically in Figure 54 was constructed. It was shown that an SS1 scFv based KIRS2 CAR exhibited enhanced cytolytic activity towards mesothelin-expressing target cells compared with the CAR formed by fusion of the SS1 scFv onto full length wildtype KIR2DS2 (Figure 55). This optimized KIRS2 CAR also showed enhanced activity over the SS1 scFv based TCRzeta CAR having a CD8 alpha extracellular hinge.

The concept, methods and compositions described in this example are applicable to RNKR-CARs. The concept, methods and compositions described in this example are applicable to RNKR-CARX cells.

### Example 25: InhKIR-CAR Construction and Activity

An inhibitory KIR-CAR was constructed based upon the fusion of the anti-mesothelin SS1 scFv to the inhibitory KIR2DL3 receptor base. Initial studies demonstrated that the inhKIR-CARs efficiently expressed in primary human T cells. CD 19 actKIR-CAR, SS1 actKIR-CAR and SS1 inhKIR-CAR alone or in combination have been introduced into Jurkat T cells bearing a dsGFP reporter under the control of an NFAT-driven promoter to monitor activation of this critical T cell signaling pathway. While Jurkat T cells expressing CD19 actKIR-CAR or SS1 actKIR-CAR alone are efficiently activated by K562 expressing both CD19 and mesothelin (KT-meso/CD19), Jurkat T cells co-expressing the CD19 actKIR-CAR and the SS1 inhKIR-CAR showed markedly reduced activation by the same KT-meso/CD19 target cells (Figure 56A); however, analysis of the surface expression of the CD19 and mesothelin scFv binding using idiotype specific reagents surprisingly demonstrated that the expression of the different scFv target specificities were mutually exclusive (Figure 56B).

The concept, methods and compositions described in this example are applicable to RNKR-CARs. The concept, methods and compositions described in this example are applicable to RCAR/NKR-CARX cells and/or RNKR-CARX cells.

### Example 26: Sensitivity of activating KIR-CAR designs to natural inhibitory receptor systems

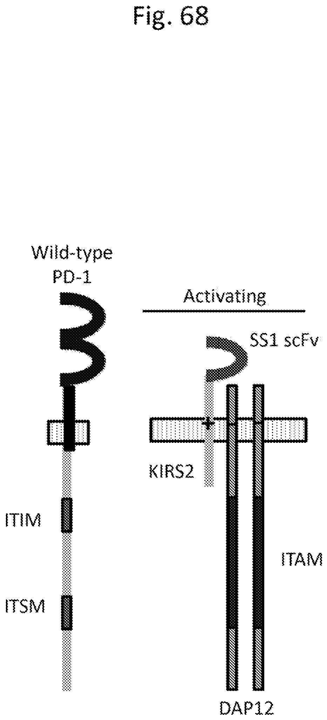

Since co-expression of two scFv CARs is limited, a strategy was pursued to evaluate the sensitivity of the KIR-based activating CARs to inhibitory signals derived from the PD-1 receptor. PD-1 is a natural receptor in T cells that uses an ITIM in the cytoplasmic domain similar to inhibitory KIRs to recruit phosphatases that negatively regulate TCR signaling. A schematic representation is shown in Figure 68. The results presented herein demonstrate that wild-type PD-1 can be over-expressed with both an activating KIR-based CAR and a TCR-zeta based CAR targeting mesothelin (Figures 57A and 57C). The results also show that this combination led to PD-1 ligand 1 (PDL-1) dependent inhibition of the mesothelin-specific activating KIR-CAR cytotoxicity (Figure 58). In the context of normal PD-1 expression by the T cells (i.e. T cells without PD-1 transfection), the KIR-CAR exhibits less inhibition when encountering PD-L1 overexpressing target cells compared with the TCR-zeta based CAR. Without wishing to be bound by any particular theory, it is believed that this may be an advantage of the KIR-CARs when encountering tumors that commonly express inhibitory receptor ligands.

The concept, methods and compositions described in this example are applicable to RNKR-CARs. The concept, methods and compositions described in this example are applicable to RCAR/NKR-CARX cells and/or RNKR-CARX cells.

### Example 27: Co-stimulation dependent activation of KIR CARs

Experiments were designed to evaluate the effects of chimeric co-stimulatory receptors (CCRs) in the KIR-CAR system compared to that described with standard CARs by Kloss et al. (Kloss et al., 2013, Nat Biotechnol 31(1):71-5). Experiments have also been designed to evaluate the costimulatory dependent activation requirements for KIRs by engaging the endogenous CD28 receptor in T cell using the agonist antibody, clone 9.3. As shown in Figure 63, the KIRS2 CAR showed robust proliferation in response to mesothelin-positive targets in the absence of CD28 costimulation. This proliferation is superior to that observed with a TCR-zeta CAR where co-stimulation has been shown to be critical to proliferation. This data suggests that the KIR-based CAR may not have the same costimulation requirements as TCR-zeta CARs for antigen-specific proliferation (Milone et al., 2009, Mol Ther 17(8):1453-64; Carpenito et al., 2009, Proc Natl Acad Sci USA 106(9):3360-5), and this costimulation independence may be a significant advantage of KIR-based CARs to current TCR-zeta-based CARs. Experiments have been designed to evaluate the KIR-based CARs in humanized mice to test the KIR-based CAR against CARs with and without costimulatory domains in an in vivo pre-clinical setting (data and experiments in example 27 are also presented in example 28).

The concept, methods and compositions described in this example are applicable to RNKR-CARs. The concept, methods and compositions described in this example are applicable to RCAR/NKR-CARX cells and/or RNKR-CARX cells.

### Example 28: Killer Immunoglobulin-like Receptor (KIR)-based Chimeric Antigen Receptors (CARs) Trigger Robust Cytotoxic Activity in Solid Tumors

Chimeric antigen receptors (CARs) bearing an antigen-binding domain linked in *cis* to the cytoplasmic domains of CD3-ζ and costimulatory receptors provide a potent method for engineering T cell cytotoxicity towards tumors (Grupp et al., The New England journal of medicine, 368(16):1509-18, 2013; Brentjens et al., Science translational medicine, 5(177):177ra38, 2013; Porter et al., The New England journal of medicine, 365(8):725-33, 2011). An alternative chimeric receptor in which a single chain variable fragment (scFv) targeting mesothelin (SS1) was fused to the transmembrane and cytoplasmic domain of KIR2DS2, a stimulatory killer immunoglobulin-like receptor (KIR) normally expressed by natural killer (NK) cells is described herein. This SS1-KIRS2 KIR-based CAR triggers robust antigen-specific cytotoxic activity and effector function in vitro sich as cytokine secretion and proliferation when introduced into human T cells in combination with adaptor molecule DAP12. T cells modified to express a KIR-CAR and DAP12 exhibit significantly enhanced anti-tumor activity in a resistant tumor xenograft model compared with T cells transduced with a standard CD3ζ-based CAR, suggesting that the KIR-based CAR can overcome inhibitory signals within tumors that limit second and third generation CD3ζ-based CARs. The data presented herein support future clinical evaluation of a KIR-based CAR in cancers including solid tumors.

"First generation" CARs were designed by the incorporation of a cytoplasmic domain containing the immunotyrosine-based activation motif (ITAM) into a single chimeric receptor that uses a single chain variable fragment (scFv) from an antibody for specific antigen targeting (Sadelain et al., Cancer discovery, 3(4):388-98, 2013). A number of different additional signaling domains from co-stimulatory receptors such as CD28, ICOS, 4-1BB and OX-40 were later incorporated in tandem into these receptors to enhance the proliferation, survival and function of T cells (Finney HM et al. J Immunol. 1998;161:2791-2797; Maher J. et al. Nat Biotech 2002; 20:70-75; Finney HM et al. J Immunol. 2004; 18:676-684; Milone et al., 2009, Mol Ther 17(8):1453-64; Carpenito et al., 2009, Proc Natl Acad Sci USA 106(9):3360-5). These "second generation" (one co-stimulatory domain) and "third generation" (2 co-stimulatory domains) CARs demonstrate enhanced function in preclinical animal models of cancer, and several co-stimulation-enhanced CARs are currently in early phase human clinical trials for cancer (reviewed in Barrett DM et al. Ann Rev Med 2014; 65:333-347).

Although single-chain CARs trigger robust antigen-specific cytotoxic activity, natural receptors utilizing the highly conserved ITAM domains are generally structured into multi-chain complexes composed of separate ligand binding and ITAM-containing signaling chains, such as the T cell receptor (TCR)-CD3 complex, the B cell receptor (BCR)-Igα/β complex and the Fc receptor (FcR) complex. The potential benefits of a multi-chain immunoreceptor complex are manifold, including greater diversity of signals available through the multiple interactions between ligand binding and signaling molecules and sustained ITAM signaling that is separable from the internalization of the ligand-binding chain (Sigalov et al., Advances in experimental medicine and biology, 640:ix-xi, 2008). The consequences of combining several receptor components normally found in heterologous receptors into a CAR has not been fully elucidated; however, anergy and antigen-independent signaling have been observed with some designs (Brocker, Blood, 96(5):1999-2001, 2000; Brocker et al., The Journal of experimental medicine. 181(5):1653-9, 1995; Milone et al., Molecular therapy : the journal of the American Society of Gene Therapy, 17(8):1453-64, 2009).

The invention claimed herein describes CARs constructed upon a more "natural" multi-chain immunoreceptor design having greater potency in activating T cells due to the naturally-selected interactions between the subunits within the receptor complex and other receptors within immune cells. The killer immunoglobulin-like receptor (KIR) and DAP12 multichain immunoreceptor complex was chosen as the foundation for the CAR (Thielens et al., Current opinion in immunology, 24(2):239-45, 2012). Although expressed by natural killer cells where they contribute to their natural cytotoxicity, KIR expression has also been observed in both CD4+ and CD8+ T cells (Moretta et al., Immunological reviews, 155:105-17, 1997; Falk et al., Human immunology; 61(12):1219-32, 2000; Remtoula et al., Journal of immunology, 180(5):2767-71, 2008). Activating KIRs, such as KIR2DS2, possess a short cytoplasmic domain with no known endogenous signaling activity. However, KIRs form a non-covalent complex with dimers of DAP12, an ITAM-containing adaptor molecule capable of binding Syk and Zap70 kinases in NK cells (Lanier et al., Nature, 391(6668):703-7, 1998). In addition to stimulating cytotoxicity upon ligand binding, KIRs have also been shown to exhibit costimulatory effects within T cells in the absence of DAP12 suggesting that these molecules might be able to provide both primary triggering activity and costimulation in T cells (Snyder et al., Journal of immunology, 173(6):3725-31, 2004).

A KIR-based CAR was constructed by splicing the mesothelin-specific SS1 scFv onto the transmembrane and short cytoplasmic domain of the activating KIR, KIR2DS2 (SS1-KIRS2) as illustrated schematically in Figure 50 (Hassan et al., Clinical cancer research : an official journal of the American Association for Cancer Research, 8(11):3520-6, 2002). The ITAM-containing adaptor molecule, DAP12 is constitutively expressed in natural killer (NK) cells, but it is only expressed in a subset of human T cells (Moretta *et al*.). Therefore, a bicistronic lentiviral vector encoding both the mesothelin-specific KIR-based CAR (SS1-KIRS2) and the DAP12 molecule separated by the *Thoseaasigna* virus 2A (T2A) sequence was generated in order to achieve co-expression of both molecules (Fig. 51). Transduction of primary human T cells with SS1-KIRS2 and DAP12 bicistroinic lentivirus following anti-CD3 and anti-CD28 activation demonstrated robust surface expression of SS1-KIRS2 that was comparable to the CD3ζ-based SS1ζ CAR (Fig. 55). SS1-KIRS2/DAP12 co-transduced T cells expanded following polyclonal anti-CD3/anti-CD28 stimulation with kinetics that was comparable to that observed with mock transduced T cells or T cells transduced with a mesothelin-specific CAR containing the CD3ζ cytoplasmic domain (data not shown). The cytotoxic activities of the KIR-based versus CD3ζ (SS1-z) CAR T cells was compared. SS1-KIRS2/DAP12-transduced T cells showed potent cytotoxic activity towards K562 cells that express human mesothelin (K-meso) with similar magnitude to the SS1ζ construct. None of the engineered T cells demonstrate lytic activity towards wild-type K562 (Kwt) supporting the specific activation of the SS1-KIRS2 receptor by the cognate mesothelin target antigen (Fig. 55).

Since expression of KIR2DS2 has been described in T cells in the absence of detectable DAP12 expression, the expression and function of the SS1-KIRS2 receptor with or without co-delivery of DAP12 was evaluated. Using a lentiviral vector that co-expressed DAP12 with the red fluorescent protein, dsRed (DAP12-dsRed) or a dsRed-expressing control vector (dsRed), T cells were transduced with the lentiviral DAP12 or control vector followed by transfection with *in vitro* transcribed RNA expressing SS1-KIRS2. SS1-KIRS2 was expressed at the surface of T cells without the addition of DAP12; however, the surface expression of SS1-KIRS2 increased by ∼1-log with the addition of DAP12 (Fig. 61A). Despite the expression of SS1-KIRS2, T cells without DAP12 do not lyse mesothelin-expressing target cells demonstrating a requirement for DAP12 in SS1-KIRS2-triggered T cell cytotoxic activity (Fig 61B). These data do not preclude the possibility that the chimeric KIR might provide signals independently of its association with DAP12 as previously reported for the natural KIR2DS2 receptor in T cell clones (Snyder *et al*.).

The non-covalent association of natural KIR2DS2 and DAP12 depends upon the electrostatic interactions between an aspartic acid residue in the KIR transmembrane (TM) domain and a lysine residue in the DAP12 TM domain (Feng et al., PLoS biology, 4(5):e142, 2006). Although the configuration of these ionizable amino acid residues in the TM domains of TCR and CD3 subunits are thought to differ from the KIRs and DAP12, providing some specificity for the interactions, the possibility that SS1-KIRS2 might be interacting with components of the CD3 complex in lieu of co-delivered DAP12 was investigated. Since the association between the CD3 complex and TCR chains is required for TCR expression on the cell surface, competition of the KIR for CD3 components would be expected to interfere with normal TCR expression as previously observed with expression of cloned TCRs. The introduction of an ectopic Vβ chain into T cells has been shown to reduce the surface expression of endogenous TCR Vβ due to competition during complex assembly (Varela-Rohena et al., Nature medicine, 14(12):1390-5, 2008). The similar frequency and intensity of TCR Vβ 13.1+ in KIRS2-transduced polyclonal T cells compared with mock-transduced, control T cells supports the absence of a significant interaction between SS1-KIRS2 and members of the endogenous CD3 complex (Fig. 62).

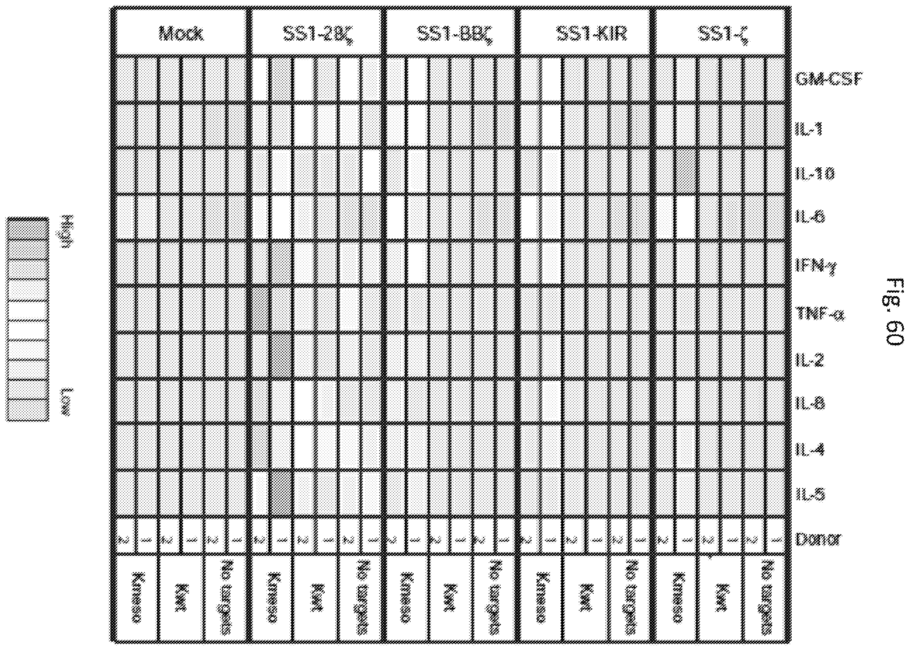

Although cytotoxic activity is an important effector function for in vivo anti-tumor activity of T cells, the ability of an antigen-receptor to trigger cytokine secretion and T cell proliferation are also important characteristics that generally correlate with robust anti-tumor activity in vivo. Therefore, antigen-triggered interferon-y (IFN-γ) and interleukin-2 (IL-2) secretion by SS1-KIRS2/DAP12-modified T cells to T cells bearing a CD3ζ-based CAR without a costimulatory domain (SS1-ζ) or with CD28 or 4-1BB co-stimulatory domains (SS1-28ζ and SS1-BBζ, respectively, was compared. The SS1-ζ construct stimulates the lowest secretion of both IFN-γ and IL-2 (Fig. 59, 60). Interferon-y production is increased and comparable in T cells expressing SS1-KIRS2/DAP12 or SS1-BBζ whereas T cells expressing the SS1-28ζ CAR show significantly greater IL-2 and IFN-γ production (Fig. 59). Analysis of a larger panel of cytokines and chemokines demonstrates that SS1-KIRS2/DAP12 stimulates a pattern of expression that is qualitatively similar across CD3ζ-based CARs with a magnitude of antigen-induced cytokine and chemokine secretion that is comparable to SS1-ζ and SS1-BBζ CARs (Fig. 60).

The SS1-KIRS2/DAP12 receptor was also a potent stimulator of T cell proliferation in response to cognate antigen (Fig. 63). Unlike the standard SS1-ζ CAR that depends upon additional costimulatory signals for robust proliferation, SS1-KIRS2/DAP12 T cells show proliferation that is comparable to SS1ζ with addition of anti-CD28 agonist antibody. The mechanism of the costimulation provided by SS1-KIR2 in the absence of DAP12 might be related to KIR interactions with other adaptor molecules (Synder *et al*.). Additional receptors naturally expressed by T cells may also be capable of utilizing the co-delivered DAP12 further contributing to T cell activation and proliferation. In particular, integrins can provide costimulatory signals to T cells (Brunmark et al. (1996) PNAS USA 93(25): 14736-41; Zuckerman et al. (1998) J. Immunol. 160(7):3259-68). DAP12 appears critical to outside-in signaling by integrins in macrophages and neutrophils (Jakus et al. (2007) Trends in Cell Biol. 17(10):493-501; Mocsai et al. (2006) Nature Immunol. 7(12):1326-33), and may confer unique signaling activity to LFA-1 and other integrins in T cells contributing to SS1-KIRS2/DAP12 activity.

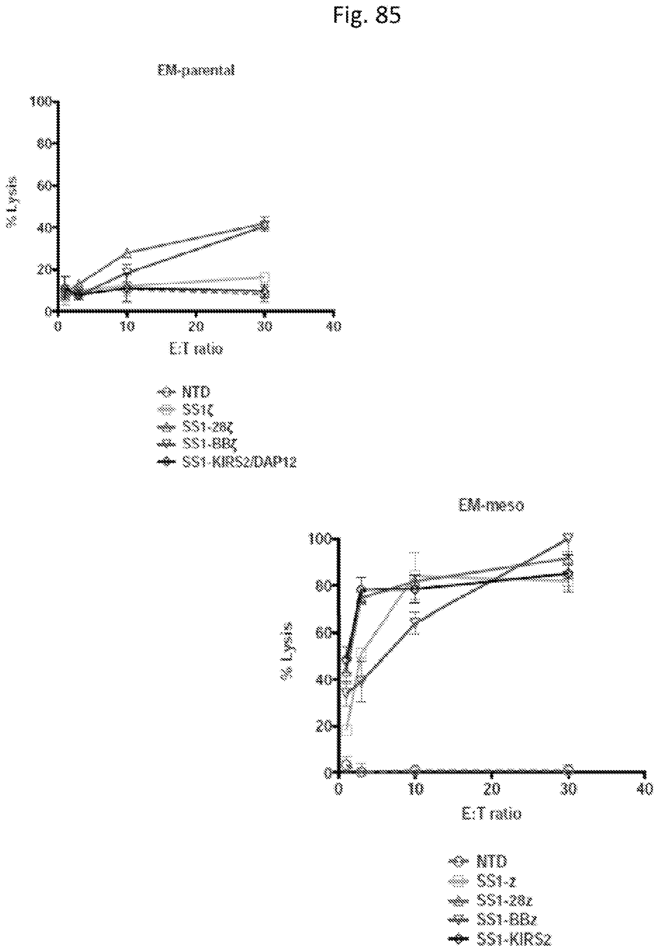

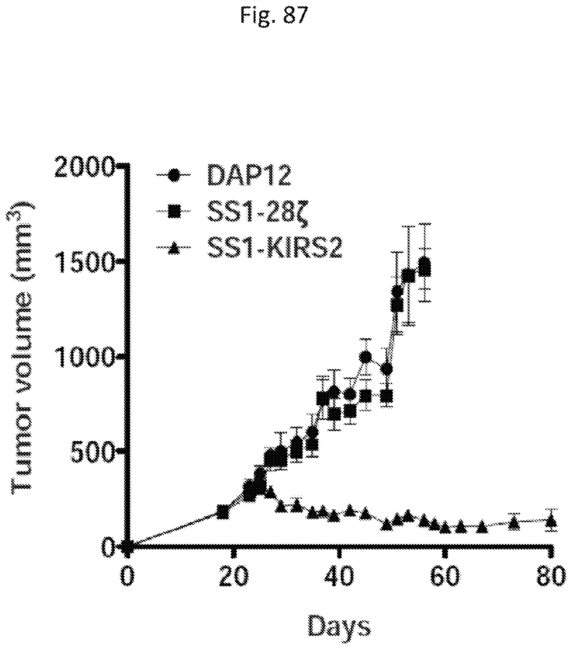

CD28 and 4-1BB have been incorporated into CARs to enhance CAR T cell activity in vivo (Carpenito et al. (2009) PNAS USA 106(9):3360-65); however, costimulation is not always able to overcome the immunosuppressive tumor microenvironment. It was recently reported that SS1-BBζ CAR T cells injected into immunodeficient NOD-SCID-γ_{c}^{-/-} (NSG) mice bearing a xenograft of EM-meso cells (a cell line derived from the pleural effusion of a patient with malignant mesothelioma) expand in vivo, but become hypofunctional within the tumor microenvironment associated with failure to clear tumors (Moon et al. (2014) Clinical Cancer Res. 20:4262-4273). The activity of SS1-KIRS2/DAP12-modified T cells was evaluated in this highly resistant model of mesothelioma. SS1-KIRS2/DAP12 and CD3ζ-based CARs with or without costimulation are able to lyse EM-meso cells in vitro with comparable efficacy. Mock-transduced and DAP12-dsRed-transduced T cells show minimal lytic activity towards EM-meso cells (Fig. 85). A single intravenous injection of mock, SS1ζ, and the SS1BBζ-transduced T cells had no observable anti-tumor effect on established EM-meso xenografts (Fig 64A). Tumor growth was signficantly delayed by SS1-28ζ CAR T cells; however, only SS1-KIRS2/DAP12-modified T cells induced regression of tumors with significant suppression of EM-meso tumor growth at 52 days (p < 0.001, ANOVA with post-hoc Scheffe F-test). A second experiment comparing T cells expressing SS1-KIRS2/DAP12 to DAP12 alone or SS1-28ζ engineered T cells showed similar enhanced anti-tumor activity of SS1-KIRS2/DAP12 T cells (Fig 87). The robust activity of a KIR-based CAR is not unique to the mesothelin specificity. A CD19-specific KIR-based CAR was also constructed with in vitro activity comparable to second generation CD3ζ CARs (Fig. 65B). Testing in a NALM-6 leukemia xenograft model also showed KIR-CAR efficacy superior to a first generation CAR and comparable to a second generation CAR with a 4-1BB costimulatory domain.

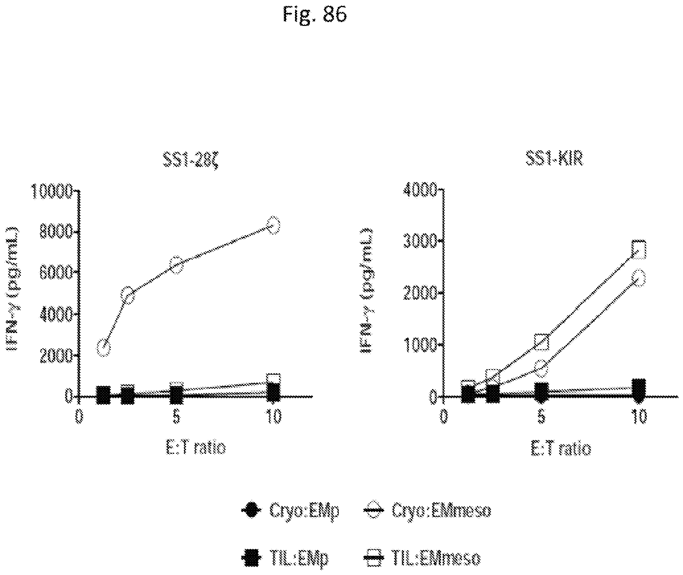

Analysis of T cell engraftment and tumor infiltrating lymphocytes (TILs) was performed to explore the mechanism of the enhanced anti-tumor activity of SS1-KIRS2/DAP12 T cells in the EM-meso xenograft model. Only mice receiving the SS1-BBζ CAR T cells had detectable human CD45 (hCD45) positive cells in the blood and spleen consistent with the previously observed effect of the 4-1BB costimulatory domain on in vivo CAR+ T cell persistence (Milone *et al*.). Few hCD45+ tumor infiltrating lymphocytes (TILs) were detected in mock or SS1ζ-treated mice. In contrast, tumors treated with SS1-KIRS2/DAP12, SS1-28ζ, and SS1-41BBζ CAR T cells had hCD45+ TILs that comprised 2-4% of the total viable cells with comparable frequencies for each group (Fig 64B). Immunohistochemical staining showed both CD8+ and CD4+ TILs (data not shown) within the tumors of SS1-KIRS2/DAP12, SS1-28ζ, and SS1-41BBζ CAR T cell-treated mice confirming the flow cytometric analysis. The increased efficacy of the SS1-KIRS2/DAP12 T cells is therefore unrelated to the TIL frequency within the tumors at late stages of tumor growth. Since comparison of TILs is limited by the large differences in tumor volume at the late time points, earlier time points following T cell injection were evaluated. At 10 days following T cell injection, comparable frequencies of hCD45+ TILs were observed in SS1-28ζ and SS1-KIRS2/DAP12 CAR T cell treated groups, but few CD45+ TILs were present in the SS1-BBζ CAR T cell group (Fig 64B). Limited analysis of these isolated TILs showed that only SS1-KIRS2/DAP12 CAR T cell were capable of in vitro lytic activity towards EM-meso cells (data not shown). These results indicate that delayed accumulation of SS1-BBζ T cells into the tumor along with tumor-induced hypofunction underlies the poor anti-tumor activity of these cells despite their high frequency at late stages of tumor development. A repeat experiment was conducted comparing SS1-28ζ and SS1-KIRS2/DAP12 CAR T cells with TIL isolation at 18 days following T cell injection to obtain greater numbers of TILs for phenotypic and functional analysis. Isolated SS1-28ζ TILs demonstrated markedly reduced cytotoxic activity and antigen-specific IFN-γ production compared to cryopreserved T cells used for treatment. In contrast, the TILs from SS1-KIRS2/DAP12 CAR T cell-treated tumors showed comparable in vitro cytotoxicity and IFN-γ production to cryopreserved cells (Fig 64E and Fig 86). Immunoblotting of protein lysates from the TILs and cryopreserved cells for CAR protein demonstrated loss of CAR expression (data not shown). The absence of CAR in the TILs may be due to downregulation of expression or poor survival of the SS1-28ζ CAR T cells relative to non-transduced T cells within the tumor microenvironment.

In conclusion, the data presented herein demonstrate that the combination of KIR-based CAR and DAP12 provides a highly effective, antigen-specific receptor system for conferring artificial antigen specificity to T cells. Despite relative equivalent *in vitro* activity, it has further been shown that this KIR-based CAR has much improved anti-tumor efficacy compared to CARs based on CD3ζ with one or more costimulatory domains in the model tumor system utilized herein, perhaps due to increased resistance to inactivation. Further exploration into the mechanisms of this increased efficacy and of chimeric receptor designs based upon other DAP12-associated ligand-binding receptors, as well as additional natural ITAM containing receptors systems such as FcRy, will be pursued.

The concept, methods and compositions described in this example are applicable to RNKR-CARs. The concept, methods and compositions described in this example are applicable to RNKR-CARX cells.

### Example 29: A KIR-based CAR can be co-expressed with a natural inhibitory KIR permitting regulation by HLA expression on the target cells

### Generation and characterization of a K562-meso cell line that express the KIR2DL3 ligand HLA-Cw

*Material and Method*: Wild type K562 cells or a K562 line previously engineered to express mesothelin (K562-meso) were transduced with a lentiviral vector encoding the HLA-Cw3 allele. Cells were sorted for uniform expression of mesothelin and HLA-Cw3 by fluorescence-activated cell sorting. HLA-Cw3 expression was confirmed by flow cytometry following staining with the W6/32 anti-HLA A, B, C antibody conjugated to APC.

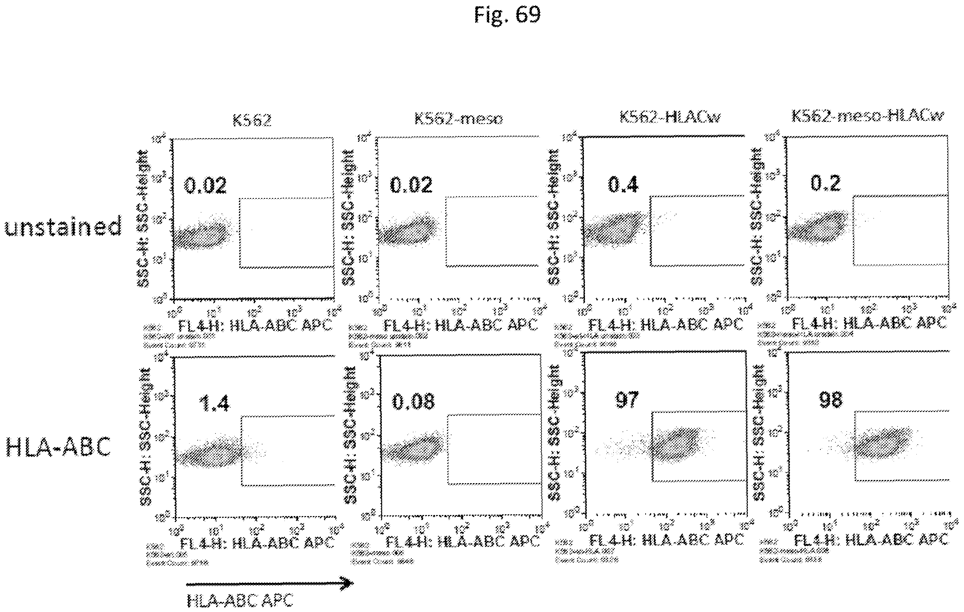

*Result:* K562 cell lines expressing either mesothelin or HLA-Cw3 alone or in combination can be generated (Fig. 69).

### Co-expression of SS1-KIRS2 and KIR2DL3 in primary human T cells

*Material and Method*: Primary human T cells were stimulated with anti-CD3/28 microbeads followed by transduction with either a bicistronic lentiviral vector expressing DAP12 and SS1-KIRS2 alone or in combination with a lentiviral vector expressing KIR2DL3 on day 1 following activation. The expression of the SS1-KIRS2 CAR was assessed by flow cytometry using a biotinylated goat-anti-mouse F(ab)2 polyclonal antibody followed by SA-APC. KIR2DL3 expression was determined using a KIR2D specific monoclonal antibody.

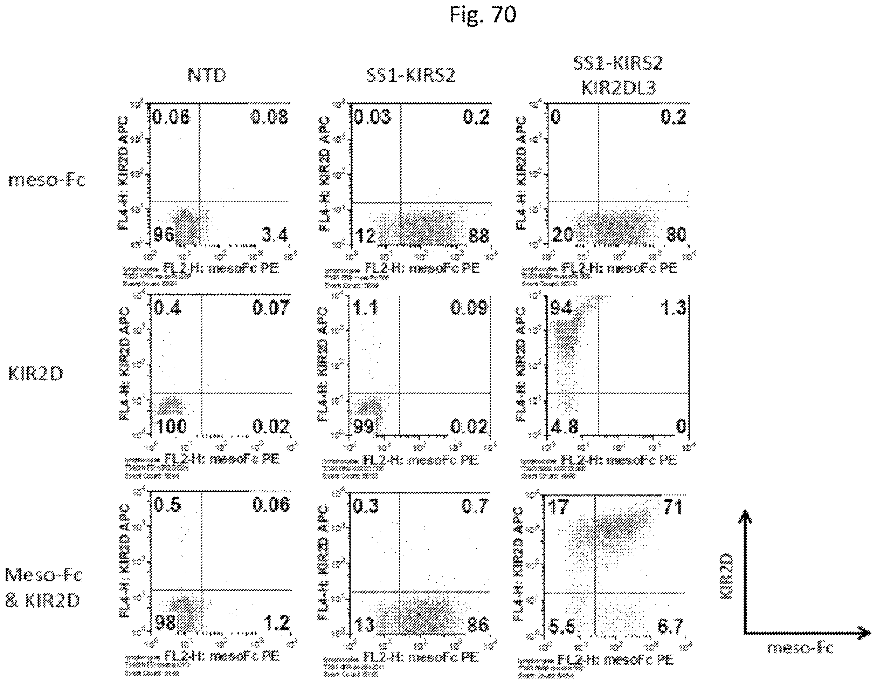

*Result:* Primary human T cells expressing a mesothelin-specific KIR-based CAR with DAP12 (KIRS2) alone, KIR2DL3 alone or a combination of the two receptors can be generated (Fig. 70).

### KIR2DL3 coexpressed with a KIR CAR can suppress antigen specific cytotoxicity in the presence of HLA-Cw on the target cells

*Material and Method:* Primary human T cells were stimulated with anti-CD3/28 microbeads followed by transduction with a bicistronic lentiviral vector expressing DAP12 and SS1-KIRS2. 5 µg of in vitro transcribed mRNA encoding KIR2DL3 was introduced into the lentivirally-transduced T cells by electroporation following 10 days of *ex vivo* expansion. These T cell populations were mixed with ⁵¹Cr-labeled K562 target cells (K562, K562-meso, K562-HLACw and K562-meso/HLACw) as indicated at varying ratios of effector T cells to target K562 cells (E:T ratio). Cytotoxicity was determined by measuring the fraction of ⁵¹Cr released into the supernatant at 4 hours.

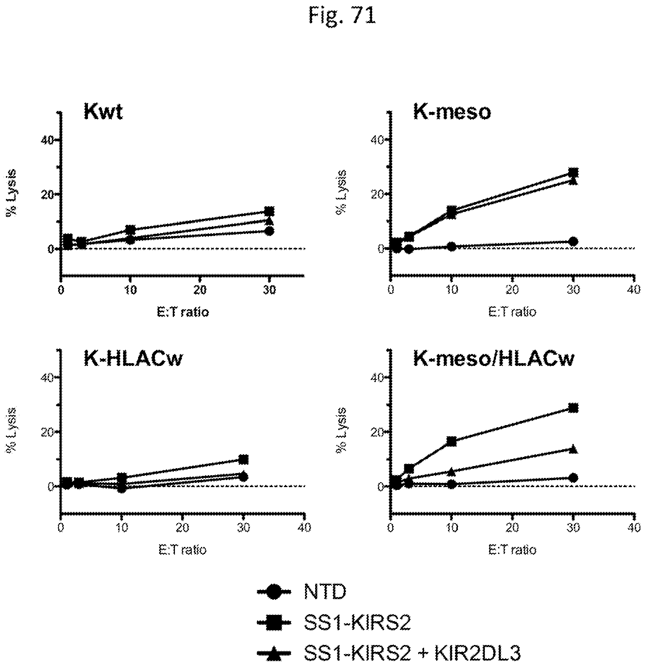

*Result:* SS1-KIRS2/DAP12-expressing T cells were capable of killing target K562 cells that express mesothelin regardless of HLA-Cw3 expression. In contrast, T cells co-expressing the SS1-KIRS2/DAP12 receptor complex and the inhibitory KIR, KIR2DL3 failed to exhibit robust cytotoxicity against K562 expressing mesothelin with HLA-Cw3; however, these cells demonstrated cytotoxic activity towards K562 cells expressing mesothelin alone that was comparable to SS1-KIRS2/DAP12-modified T cells. These results demonstrate the ability of inhibitory KIR receptors to regulate the functional activity of activating KIR-based CARs (Fig. 71).

The concept, methods and compositions described in this example are applicable to RNKR-CARs. The concept, methods and compositions described in this example are applicable to RCAR/NKR-CARX cells and/or RNKR-CARX cells.

### Example 30: A KIR-based CAR with CD19 specificity can trigger antigen-specific target cell cytotoxicity in vitro and in vivo

### A KIR-based CAR with CD19 specificity can trigger antigen-specific target cell cytotoxicity in vitro

*Material and Method*: Following anti-CD3/anti-CD28 bead activation, T cells were transduced with a bicistronic lentiviral vector expressing DAP12 along with either a CD19-specific KIR-based CAR in which the FMC63-derived scFv is fused to full length KIR2DS2 (CD19-KIR2DS2) or a KIR-based CAR generated by fusing the FMC63 scFv to the transmembrane and cytoplasmic domain of KIR2DS2 via a short linker [Gly]₄-Ser linker(CD19-KIRS2). The transduced T cells were cultured until the end of the log phase growth, and the expression of the CD19-specific KIR-based CAR was assessed by flow cytometry using a biotinylated goat-anti-mouse F(ab)₂ polyclonal antibody followed by SA-PE. ⁵¹Cr-labeled K562 target cells with (K562- CD19) or without (K562-wt) CD19 expression were mixed at varying ratios with T cells to target cells (E:T ratio). Cytotoxicity was determined by measuring the fraction of ⁵¹Cr released into the supernatant at 4 hours. Control T cells that were either mock transduced (NTD) or transduced with a CD3ζ-based CAR specific to CD19 (CD19-z) were also included as negative and positive controls, respectively.

*Result:* Flow cytometric analysis demonstrates expression of the CD19-specific scFv on the surface of the T cells transduced with CD19-KIR2DS2, CD19-KIRS2 and CD19-z (Fig. 65A). T cells expressing DAP12 with either CD19-KIR2DS2 or CD19-KIRS2 were capable of killing target cells in an antigen-specific manner (Fig. 65B). Cytotoxicity exhibited by the KIR-based CAR-modified T cells was comparable to or higher than T cells expressing a CD19-specific CD3ζ-based CAR.

### T cells transduced with CD19-KIRS2/DAP12 induce tumor regression in a human leukemia xenograft

*Material and Method*: NOD-SCID-γ_{c}^{-/-}(NSG) mice were engrafted intravenously by tail vein on day 0 with 1 million Nalm-6 CBG tumor cells, a leukemia cell line expressing CD19. In the experiment, T cells were stimulated with anti-CD3/anti-CD28 stimulator beads followed by lentiviral transduction on day 1 with a series of CD3-based CAR with or without a costimulatory domain (CD19-z, CD19-BBz) or the CD19-specific KIR-based CARs, CD19-KIRS2 with DAP12 as indicated in the figure. Mock transduced T cells (NTD) were used as a control. The T cells were expanded until the end of log-phase growth ex vivo and injected intravenously on day 5 post leukemia cell line injection with 2 million CAR T cells per mouse. Tumor burden was assessed via bioluminescent imaging. 5 animals were analyzed for each T cell condition (Fig 66).

*Result:* In the in vivo experiment presented (Fig 66), the NTD T cells had no effect on tumor growth, while CD19**z**, CD19BB**z** and CD19-KIRS2-transduced T cells exhibit various anti-tumor effects. Mice infused with CD19**z** T cells showed a slight reduction in tumor burden but retained detectable levels of luminescence. In contrast, tumor cell luminescence in mice infused with either CD19BBz or CD19KIRS2 T cells dropped to the lower limit of detection (Fig 66B, dotted line) only 7 days post T cell injection, exhibiting complete clearance outside of a small reservoir of leukemia cells in the T cell-inaccessible tooth root. By day 15, tumor burden in the mock T cell group surpassed the endpoint (2x10¹⁰ photons/second) and were sacrificed, while luminescence in the CD19BBz and CD19KIRS2 groups remained at the lower limit of detection.

The concept, methods and compositions described in this example are applicable to RNKR-CARs. The concept, methods and compositions described in this example are applicable to RNKR-CARX cells.

### Example 31: A camelid single VHH domain-based CAR can be expressed on a T cell surface in combination with a scFv-based CAR without appreciable receptor interaction.

*Material and Method*: Jurkat T cells expressing GFP under an NFAT-dependent promoter (NF-GFP) were transduced with either a mesothelin-specific activating CAR (SS1-CAR), CD19-specific activating (19-CAR) or a CAR generated using a camelid VHH domain specific to EGFR (VHH-CAR). Following transduction with the activating CAR, the cells were then transduced with an additional inhibitory CAR recognizing CD19 (19-PD1) to generate cells co-expressing both the activating and inhibitory CAR (SS1+19PD1, 19+19PD1 or VHH+19PD1). The transduced Jurkat T cells were co-cultured for 24 hours with different cell lines that are either 1) devoid of all target antigens (K562), 2) express mesothelin (K-meso), CD19 (K-19) or EGFR (A431) only, 3) express a combination of EGFR and mesothelin (A431-mesothelin) or CD19 (A431-CD19) or 4) express a combination of CD19 and mesothelin (K-19/meso). Additional conditions that include either no stimulator cells (no stim) or K562 with 1 µg/mL of OKT3 (OKT3) were also included as negative and positive controls for NFAT activation, respectively. GFP expression, as a marker of NFAT activation, was assessed by flow cytometry.

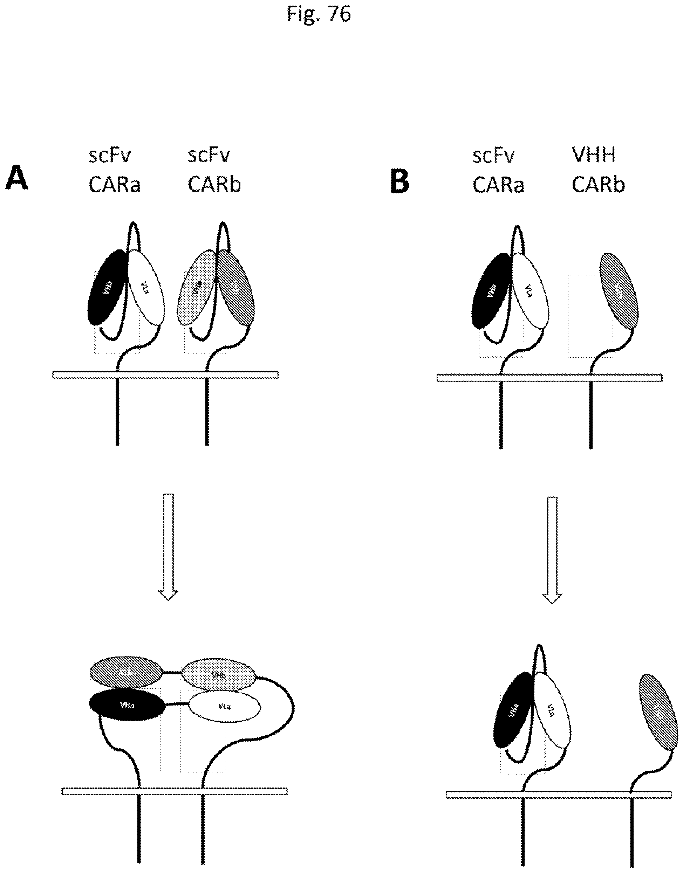

*Result:* Camels and related species (e.g. Llama) naturally produce antibodies that have a single heavy-chain like variable domain. This domain, known as a camelid VHH domain, has evolved to exist without pairing to a light chain variable domain. Fig. 76A shows schematically the possibility that two heterologous scFv molecules can dissociate and re-associate with one another when displayed on the surface of a cell as demonstrated by the observed disruption in scFv binding to cognate ligand during receptor co-expression (Fig 74 and Fig 75). Fig. 76B shows a schematic representation of the expected reduced interaction between a scFv CAR displayed on the surface of a cell in combination with a VHH domain-based CAR. Fig 77 demonstrates that coexpression of two scFv-based CARs (SS1-z activating CAR and CD19-PD1 inhibitory CAR) on the surface of a Jurkat leads to the inability of the activating CAR (SS1-z) to recognize its cognate ligand on the target cell and trigger T cell activation despite the absence of the inhibitory receptor's ligand. This is consistent with the observed reduced ligand binding on the surface (Fig 74). In contrast, the coexpression of the same inhibitory CAR (CD19-PD1) with a camelid VHH-based activating CAR (VHH-z) has no impact on the ability of the VHH-based activating CAR to recognize its cognate EGFR ligand. These data support the model depicted in Fig 76B that a VHH-based activating CAR can be expressed with an scFv-based CAR without significant interaction between the receptors due to the reduced ability of the scFv and VHH domains to interact.

The concept, methods and compositions described in this example are applicable to RNKR-CARs. The concept, methods and compositions described in this example are applicable to RCAR/NKR-CARX cells and/or RNKR-CARX cells.

### Example 32: An NKp46-based NCR CAR with mesothelin specificity triggers antigen specific cytotoxicity

*Material and Method*: Following anti-CD3/anti-CD28 bead activation, T cells were transduced with a bi-cistronic lentiviral vector expressing either DAP12 and SS1-KIRS2 (control), or FcεRγ and a mesothelin specific NKp46-based CAR (SS1-NKp46) or FcεRγ and a mesothelin-specific NKp46 CAR in which the natural NKp46 extracellular domain was truncated (SS1-TNKp46). The expression of the mesothelian-specific CARs was assessed by flow cytometry using a biotinylated goat-anti-mouse F(ab)2 polyclonal antibody followed by SA-PE (Figure 67). The T cells were mixed with ⁵¹Cr-labeled K562 target cells expressing mesothelin at varying ratios of effector T cells to target K562 cells (E:T ratio). Cytotoxicity was determined by measuring the fraction of ⁵¹Cr released into the supernatant at 4 hours compared with spontaneous release.

*Result:* Both the SS1-NKp46 and SS1-sNKp46 receptors exhibit surface expression on T cells. SS1-TNKp46 transduced T cells show robust target cell cytolysis that is comparable to the KIR-based SS1-KIRS2 CAR. SS1-NKp46 exhibited weaker cytotoxic activity that was evident only at high effector to target cell ratios (Fig 67). These data demonstrate that an antigen-specific chimeric immunoreceptor for use in redirecting T cell cytolytic activity can be generated from natural cytotoxicity receptors (NCRs) using a design similar to that used to create a KIR-based CAR.

The concept, methods and compositions described in this example are applicable to RNKR-CARs. The concept, methods and compositions described in this example are applicable to RNKR-CARX cells.

### Example 33: Interaction of scFv domains

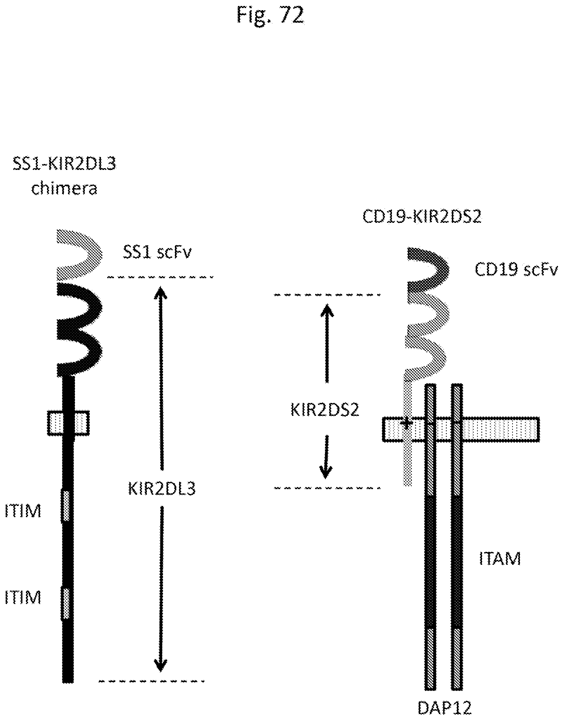

*Material and Method:* In figure 73, Jurkat T cells were transduced with lentiviral vector encoding a mesothelin-specific inhibitory KIR-based CAR (SS1-KIR2DL3). These transduced cells were then transduced with varying dilutions of a lentiviral vector encoding a CD19-specific activating KIR-based CAR (CD19-KIR2DS2). These KIR-CARs are shown schematically in Figure 72. Following transduction with both CARs, the frequency of cells with surface expression of a CAR with an intact scFv capable of binding their target ligand was assessed by flow cytometry following staining with both a mesothelin-Fc fusion protein followed by a secondary anti-Fc antibody labeled with PE and an anti-CD 19-specific (clone FMC63) anti-idiotype monoclonal antibody labeled with APC. In figure 74, anti-CD3/28-activated primary human T cells were transduced with different lentiviral vectors encoding either a mesothelin-specific CD3z-based CAR bearing an mCherry fusion to the C-terminus (SS1z-mCh), a CD19-specific CAR with CD3z and 4-1BB cytoplasmic domain (19bbz) or a combination of both SS1z-mCh and 19bbz. The expression of mCherry and a functional SS1 scFv was assessed by flow cytometry following staining with a mesothelin-Fc fusion protein followed by a secondary anti-Fc antibody labeled with FITC. In figure 75, anti-CD3/28-activated primary human T cells were transduced with different lentiviral vectors encoding either a mesothelin-specific CD3z-based CAR (SS1z), a CD19-specific CAR bearing the FMC63 scFv (19bbz) or a CD19-specific CAR bearing the 21d4 scFv (21d4bbz) or a CD19-specific CAR bearing the BL22 scFv (BL22bbz) in which the scFv was composed of either a heavy chain variable domain (VH) 5' to the light chain variable domain (VL) in the scFv (H2L) or the VL located 5' to the VH (L2H). Following transduction with each of the CD19-specific CAR, the T cells were then co-transduced with SS1z. The binding of the SS1z to mesothelin and the surface expression of the anti-CD19 scFv was assessed by flow cytometry following staining with a mesothelin-Fc fusion protein followed by a secondary anti-Fc antibody labeled with FITC or biotinylated protein L followed by streptavidin-conjugated APC.

*Result:* Fig. 73 shows that coexpression of two intact, ligand-binding scFv-based CARs (SS1-KIR2DL3 and CD19-KIR2DS2) on the cell surface is mutually exclusive. Figure 75 demonstrates the loss of ligand binding occurs despite expression of the CAR in the cell as illustrated by the presence of mCherry expressing cells with reduced mesothelin binding in cells co-transduced with SS1z-mCh and 19bbz. Figure 75 demonstrates that the interaction between scFv leading to loss of scFv binding function can be observed using different scFv-based CARs supporting the universal nature of this effect. These observations are consistent with the model depicted in Fig 76 Panel A in which the variable domain of one scFv can undergo intermolecular pairing with a heterologous scFv-based chimeric receptor leading to loss of binding by the scFv within a single CAR.

The concept, methods and compositions described in this example are applicable to RNKR-CARs. The concept, methods and compositions described in this example are applicable to RCAR/NKR-CARX cells and/or RNKR-CARX cells.

### Example 34: A Chimeric Antigen Receptor (CARs) based upon a Killer Immunoglobulin-like Receptor (KIR) Triggers Cytotoxic Activity in Solid Tumors

Chimeric antigen receptors (CARs) based upon a single chimeric molecule bearing an antigen binding domain linked in *cis* to the cytoplasmic domains of CD3ζ and costimulatory receptors CD28 or 4-1BB provide a potent method for engineering T cell cytotoxicity towards tumors. A chimeric multichain receptor based upon a killer immunoglobulin-like receptors (KIRs) normally expressed by natural killer (NK) cells and T cells was used. Constructed by fusing a single chain variable fragment (scFv) to the transmembrane and cytoplasmic domain of a KIR, it was shown that a KIR-based CAR targeting mesothelin (SS1-KIR) triggers antigen-specific cytotoxic activity and cytokine production that is comparable to CD3ζ-based CARs with antigen-induced proliferation that is independent of additional costimulation. Using a xenograft model of mesothelioma resistant to T cell immunotherapy, it was further demonstrate that a KIR-based CAR targeting mesothelin exhibits more potent anti-tumor activity compared with T cells bearing mesothelin-specific CD3ζ-based CARs with costimulation despite in vivo persistence of the latter CAR-modified T cells. Evaluation of tumor infiltrating lymphocytes demonstrate that KIR-based CAR+ T cells show resistance to acquired hypofunction within the tumor microenivornment compared with CD3ζ-based CARs with costimulatory receptor domains. The ability of a KIR-based CAR to induce regression of a tumor in which second generation CD3ζ-based CARs show limited activity supports the future clinical evaluation of a KIR-based CAR in mesothelioma and other tumors, e.g., other solid tumors.

The concept, methods and compositions described in this example are applicable to RNKR-CARs. The concept, methods and compositions described in this example are applicable to RNKR-CARX cells.

### Example 35: KIR-CAR sequences

**DAP12-T2A-SS1-KIRS2 (SEQ ID NO: 158)**
**1464 bp DNA**

| **FEATURES** | **Location** |
|---|---|
| DAP12 | 1..339 |
| T2A sequence | 352..408 |
| SS1-scFv | 481..1200 |
| GS-linker | 1207..1236 |
| KIR2DS2-derived sequence | 1237..1464 |

**DAP12-T2A-SS1-KIRS2 (SEQ ID NO: 159)**
**488 aa Protein**

| **FEATURES** | **Location** |
|---|---|
| DAP12 | 1..113 |
| T2A seq | 118..136 |
| Signal_peptide from CD8alpha | 138..158 |
| SS1-scFv | 161..400 |
| GS-linker | 403..412 |
| KIR2DS2-derived seq | 413..487 |

### Sequence

**FCERG-T2A-SS1-TNKp46 (SEQ ID NO: 160)**
**1365 bp DNA**

| **FEATURES** | **Location** |
|---|---|
| FCERG | 1..258 |
| T2A | 271..327 |
| Signal peptide from CD8alpha | 331..393 |
| SS1-scFv | 400..1119 |
| GS-linker | 1126..1155 |
| NKp46-derived sequence | 1156..1365 |

### Sequence

**FCERG-T2A-SS1-TNKp46 (SEQ ID NO: 161)**
**455aa Protein**

| FEATURES | Location |
|---|---|
| FCERG | 1..86 |
| T2A | 91..109 |
| Signal peptide from CD8alpha | 111..131 |
| SS1-scFv | 134..373 |
| GS-liner | 376..385 |
| NKp46-derived sequence | 386..454 |

### Sequence

**DAP12-T2A-CD19-KIRS2 (SEQ ID NO: 162)**
**1470 bp DNA**

| **FEATURES** | **Location** |
|---|---|
| DAP12 | 1..339 |
| T2A sequence | 352..408 |
| CD19-scFv | 481.. 481 |
| GS-linker | 1213..1242 |
| KIR2DS2-derived sequence | 1243..1470 |

### Sequence

**DAP12-T2A-CD19-KIRS2 (SEQ ID NO: 163)**
**489 aa Protein**

| **FEATURES** | **Location** |
|---|---|
| DAP12 | 1..113 |
| T2A seq | 118..136 |
| Signal_peptide from CD8alpha | 138..158 |
| CD19-scFv | 161.. 402 |
| GS-linker | 405..414 |
| KIR2DS2-derived seq | 415..489 |

### Sequence

**4-1BB Intracellular domain (amino acid sequence) (SEQ ID NO: 138)**
   KRGRKKLLYIFKQPFMRPVQTTQEEDGCSCRFPEEEEGGCEL
**Cytoplasmic Domain of PD1 (SEQ ID NO: 164)**
   **Amino acids 192-288**
**Cytoplasmic Domain of CTLA-4 (SEQ ID NO: 165)**
   **Amino acids 183-223**
   AVSLSKMLKKRSPLTTGVYVKMPPTEPECEKQFQPYFIPIN
**Human CD8 hinge (SEQ ID NO: 168)**
   43 aa linear UNA 10-FEB-2009
   TTTPAPRPPT PAPTIASQPL SLRPEACRPA AGGAVHTRGL DFA

### Example 36: Regulatable NKR-CAR (RNKR-CAR) using a Dimerization Switch

This example illustrates a general concept underlying embodiments of regulatable NKR-CARs that is based on the separation of an antigen binding member ("binding event") from an intracellular signaling member ("signaling event"). In the presence of a dimerization molecule, e.g., a small molecule, the switch domains of the antigen binding member and the intracellular signaling member associate and trigger signal transduction in the now associated RNKR-CAR molecule.

By way of example, an extracellular antigen binding domain, such as a scFv, is fused to a transmembrane domain and a first switch domain (e.g., a switch domain described herein, e.g., a switch domain from FKBP or FRB) of the dimerization switch (e.g., a heterodimerization switch). The intracellular signaling domain comprises a second switch domain (e.g., FRB or FKBP) of the heterodimerization switch and one or more intracellular signaling domains such as a primary signaling domain described herein, e.g., CD3zeta or DAP12.

Dimerization and initiation of the signaling cascade is achieved by the addition of a small molecule heterodimerizer ("heterodimerization molecule" because the switch domains are not the same) which links the extracellular binding domain to the intracellular signaling domain. For example,the small molecule inducing dimerization can be rapamycin or analogs thereof (termed "rapalogue"). The rapamycin or rapalogues function by binding with high affinity to FKBP and to the FRB domain of mTOR, thereby acting as a heterodimerizer to induce complex formation Choi, J., et al(1996) Structure of the FKBP12-rapamycin complex interacting with the binding domain of human FRAP Science 273: 239-42). Other dimerization molecules, e.g., heterodimerization molecules, described herein, can be used to regulate a RNKR-CAR.

The following examples illustrate that the dimerization switch can be on the inside or the outside of the cell.

For illustrative purposes only, a scFv fragment is used as an extracellular antigen binding domain to generate the RNKR-CARs. To generate a RNKR-CAR, a pair of constructs is generated and co-expressed in the target immune effector cell. The various switch domains of the heterodimerization switch can be linked to different domains of the RNKR-CAR construct.

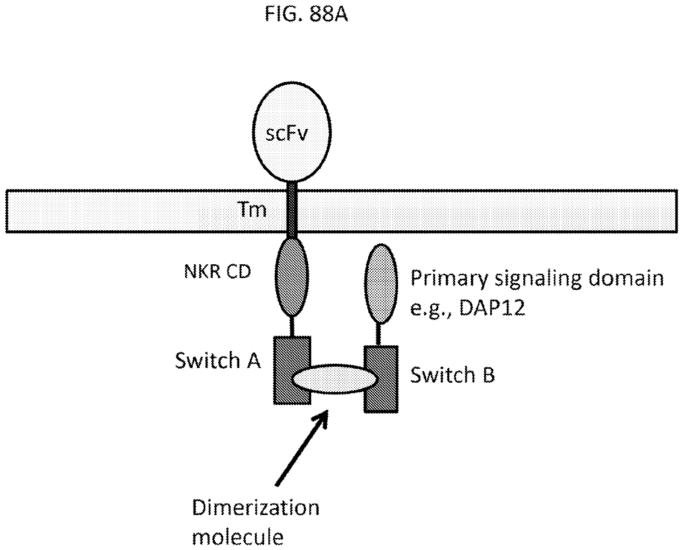

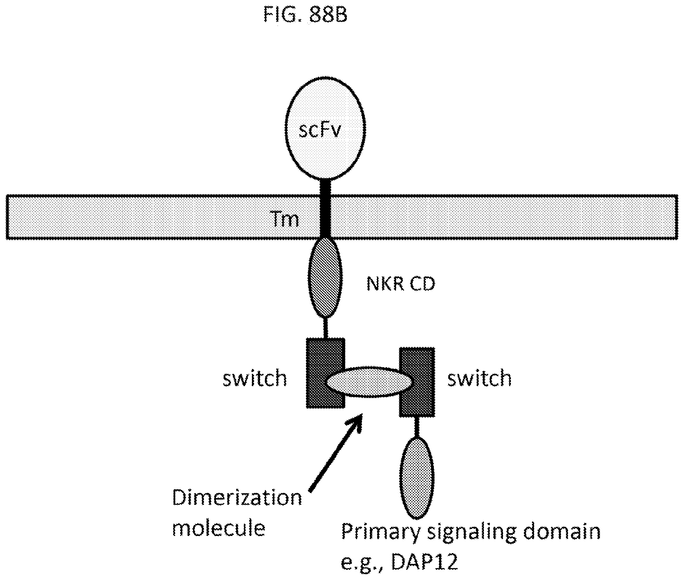

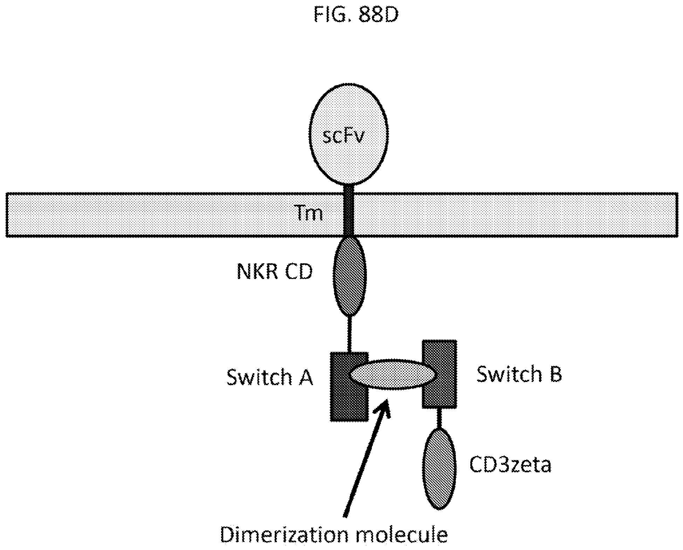

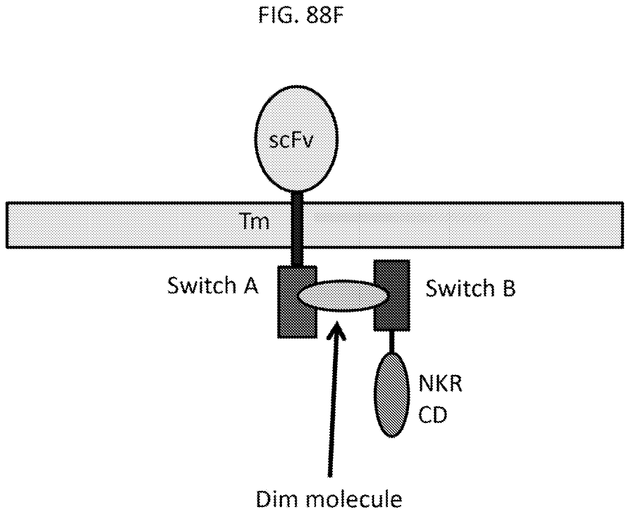

"Switch A" comprises a pair of constructs. The first construct is designed in which the antigen binding member is constructed by fusing a scFv to a transmembrane region, followed by a NKR cytoplasmic domain, and the first intracellular switch domain. In some cases, a hinge region is disposed between the scFv and the transmembrane region, and/or a linker is disposed between NKR cytoplasmic domain and the first switch domain. The corresponding intracellular signaling member/construct is designed by fusing the second switch domain to an intracellular signaling domain, e.g., primary signaling domain (e.g,. DAP12 or CD3zeta). In some cases, a linker is disposed between the second switch domain and the intracellular signaling domain. The constructs can be generated such that the domains as listed in the order above are present in a polypeptide in any orientation, i.e., from the N- to C- terminus, the C- to N- terminus, from extracellular to intracellular, or intracellular to extracellular. In some examples, the intracellular signaling member and the antigen binding member are oriented in a cell such that the NKR cytoplasmic domain is capable of interacting with, e.g., binding, the intracellular signaling domain (e.g., primary signaling domain, e.g., DAP12). See, e.g., Fig. 88A. In some cases, the first construct comprises a leader sequence fused to the N-terminus of the polypeptide, e.g., to the N-terminus of the scFv. See, e.g., Figs. 88A, 88B, and 88D.

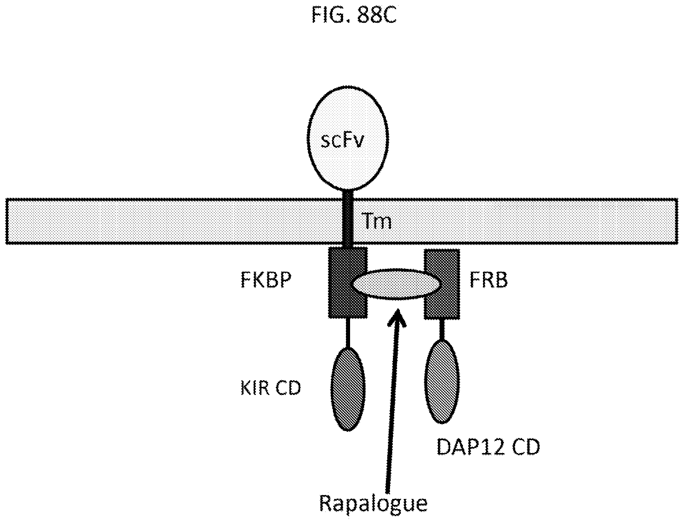

"Switch C" comprises a pair of constructs. The first construct is designed in which the antigen binding member is constructed by fusing a scFv to a transmembrane region, followed by the first intracellular switch domain (e.g., FKBP), and then a NKR cytoplasmic domain (e.g., KIR cytoplasmic domain). In some cases, a hinge region is disposed between the scFv and the transmembrane region, and/or a linker is disposed between the transmembrane region and the first intracellular switch, and/or a linker is disposed between the first switch domain and the NKR cytoplasmic domain. The second construct (intracellular signaling member/construct) is designed by fusing the second switch domain (e.g., FRB) to an intracellular signaling domain (e.g., primary signaling domain, e.g., DAP12). In some cases, a linker is disposed between the second switch domain and the intracellular signaling domain. The constructs can be generated such that the domains as listed in the order above are present in a polypeptide in any orientation, i.e., from the N- to C- terminus, the C- to N- terminus, from extracellular to intracellular, or intracellular to extracellular. In some cases, the first construct comprises a leader sequence fused to the N-terminus of the polypeptide, e.g., to the N-terminus of the scFv. See, e.g., Fig. 88C.

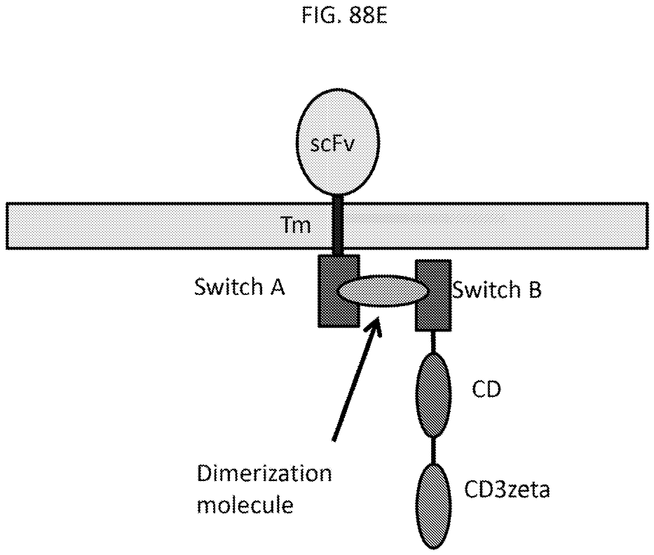

"Switch E" comprises a pair of constructs. The first construct is designed in which the antigen binding member is constructed by fusing a scFv to a transmembrane region, followed by the first intracellular switch domain. In some cases, a hinge is disposed between the scFv and the transmembrane domain. The second construct (intracellular signaling member/construct) is designed by fusing the second switch domain to a NKR cytoplasmic domain, followed by an intracellular signaling domain (e.g., primary signaling domain, e.g., CD3zeta). In some cases, a linker is disposed between the second switch domain and the NKR cytoplasmic domain, and/or the linker is disposed between the NKR cytoplasmic domain and the intracellular signaling domain. The constructs can be generated such that the domains as listed in the order above are present in a polypeptide in any orientation, i.e., from the N- to C- terminus, the C- to N- terminus, from extracellular to intracellular, or intracellular to extracellular. In some cases, the first construct comprises a leader sequence fused to the N-terminus of the polypeptide, e.g., to the N-terminus of the scFv. See, e.g., Fig. 88E.

"Switch F" comprises a pair of constructs. The first construct is designed in which the antigen binding member is constructed by fusing a scFv to a transmembrane region, followed by the first intracellular switch domain. In some cases, a hinge is disposed between the scFv and the transmembrane domain. The second construct (intracellular signaling member/construct) is designed by fusing the second switch domain to a NKR cytoplasmic domain. In some cases, a linker is disposed between the second switch domain and the NKR cytoplasmic domain. The constructs can be generated such that the domains as listed in the order above are present in a polypeptide in any orientation, i.e., from the N- to C- terminus, the C- to N- terminus, from extracellular to intracellular, or intracellularto extracellular. In some cases, the first construct comprises a leader sequence fused to the N-terminus of the polypeptide, e.g., to the N-terminus of the scFv. See, e.g., Fig. 88E.

The RNKR-CAR construct(s) can be introduced into a cell using the methods described herein, and the activity of the RNKR-CAR construct(s) can be assessed using methods described herein.

## Claims

1. A regulatable natural killer receptor CAR (RNKR-CAR), wherein the RNKR-CAR comprises:
(a) an antigen binding member, comprising:
a binding domain element that comprises an antigen binding domain derived from an antibody molecule,
a transmembrane domain, and
a first switch domain; and
(b) an intracellular signaling member comprising a second switch domain, wherein the first and second switch domains comprise a dimerization switch, further wherein either:
(i) the antigen binding member further comprises a cytoplasmic domain, further wherein
1) the transmembrane and/or cytoplasmic domain comprises an NKR transmembrane or cytoplasmic domain, each of which is selected from a killer cell immunoglobulin-like receptor (KIR), a natural cytotoxicity receptor (NCR), the signaling lymphocyte activation molecule (SLAM) family of immune cell receptors, an Fc receptor (FcR), a Ly49 receptor, or an NKR of Table 24; and
2) the intracellular signaling member comprises a NKR cytoplasmic domain selected from Table 24, or a primary intracellular signaling domain selected from Table 1, or a cytoplasmic ITAM domain;
(ii) the transmembrane domain comprises an NKR transmembrane domain from a killer cell immunoglobulin-like receptor (KIR), a natural cytotoxicity receptor (NCR), the signaling lymphocyte activation molecule (SLAM) family of immune cell receptors, an Fc receptor (FcR), a Ly49 receptor, or an NKR of Table 24, and the intracellular signaling member comprises a cytoplasmic ITAM domain; or
(iii) the intracellular signaling member further comprises an NKR cytoplasmic domain selected from Table 24,
further wherein the dimerization switch comprises
a FKBP/FRB-based dimerization switch, optionally wherein the first and second switch domains are dimerized by a rapamycin or a rapamycin analogue;
a GyrB-GyrB based switch, optionally wherein the first and second switch domains are dimerized by coumermycin;
a GAI-GID1 based switch;
a gibberellin-based dimerization switch; or
a Halo-tag/SNAP-tag based switch.

2. The RNKR-CAR of claim 1, wherein the NKR cytoplasmic domain comprises a KIR cytoplasmic domain selected from Table 24.

3. The RNKR-CAR of claim 1 or claim 2, wherein the primary intracellular signaling domain is:
(a) derived from CD3 zeta, common FcR gamma (FCER1G), Fc gamma RIIa, FcR beta (Fc Epsilon R1b), CD3 gamma, CD3 delta, CD3 epsilon, CD79a, CD79b, DAP10, and DAP12; or
(b) a DAP12 signaling domain, a DAP10 signaling domain or a CD3zeta signaling domain.

4. The RNKR-CAR of any one of claims 1 to 3, wherein:
(a) the transmembrane domain comprises a KIR transmembrane domain selected from Table 24;
(b) the RNKR-CAR comprises a mutated NKR transmembrane domain selected from the group consisting of a mutated KIR transmembrane domain, mutated NCR transmembrane domain, mutated FcR transmembrane domain, mutated Ly49 receptor transmembrane domain and mutated SLAMF receptor transmembrane domain; or
(c) the RNKR-CAR comprises a transmembrane domain from a T cell molecule selected from CD8alpha and CD3zeta.

5. The RNKR-CAR of any one of the preceding claims, wherein one of the first and second switch domains of a FKBP-FRB based switch comprises an FKBP-based switch domain and the other comprises an FRB-based switch domain, further wherein the FKBP-based switch domain comprises a FRB binding fragment or analog of FKBP and the FRB-based switch domain comprises an FKBP binding fragment or analog of FRB comprising an E2032 mutation such as E2032I or E2032L and/or a T2098 mutation such as T2098L.

6. The RNKR-CAR of any one of the preceding claims, wherein the antigen binding domain derived from an antibody molecule includes:
(a) an antibody, an antibody fragment, an scFv, a Fv, a Fab, a (Fab')2, a single domain antibody (SDAB), a VH or VL domain, or a camelid VHH domain; or
(b) an scFV, a VH domain, a nanobody or a camelid VHH domain.

7. The RNKR-CAR of any one of the preceding claims wherein the antigen binding domain binds a tumor antigen selected from the group consisting of:
(a) folate receptor (FRa), mesothelin, EGFRvlll, IL-13Ra, CD123, CD19, CD33, BCMA, GD2, CLL-1, CA-IX, MUC1, HER2, and any combination thereof; or
(b) CD19, mesothelin, CD123 and BCMA.

8. The RNKR-CAR of claim 1(iii), wherein the intracellular signaling member comprises a primary intracellular signaling domain selected from Table 1.

9. A nucleic acid encoding a RNKR-CAR of any one of the previous claims.

10. A vector system, e.g., one or more vectors, comprising a nucleic acid of claim 9.

11. A cell comprising a RNKR-CAR of any one of claims 1-8, a nucleic acid encoding a RNKR-CAR of claim 9, or the vector system of claim 10.

12. A method of making a cell of claim 11, comprising introducing a nucleic acid encoding a RNKR-CAR of claim 9, or the vector system of claim 10 into said cell.

13. A RNKR-CAR cell of claim 11, for use in a method of
(a) treating a subject with a disease associated with a tumor antigen; or
(b) treating a subject for cancer,
wherein the method comprises administering to the subject an effective amount of the RNKR-CAR cell.

14. A method of providing an RNKR-CAR cell comprising:
receiving from an entity an immune effector cell from a human;
inserting a nucleic acid encoding an RNKR-CAR of any of claims 1-8 into said immune effector cell, or a daughter cell thereof, to form an RNKR-CAR cell; and,
optionally, providing said RNKR-CAR cell to said entity.

15. A RCAR/NKR-CAR cell comprising:
A) a regulatable CAR (RCAR) and a NKR-CAR;
B) a nucleic acid encoding a RCAR and a NKR-CAR; or
C) a vector system comprising a nucleic acid encoding a RCAR and a NKR-CAR,
wherein the RCAR comprises:
a) an intracellular signaling member comprising:
a primary intracellular signaling domain, and
a first switch domain;
b) an antigen binding member comprising:
an antigen binding domain,
a second switch domain; and
optionally, one or a plurality, of co-stimulatory signaling domain, and
c) a transmembrane domain,
wherein the first and second switch domains form a dimerization switch, comprising:
1) a FKBP/FRB-based dimerization switch, optionally wherein the first and second switch domains are dimerized by a rapamycin or a rapamycin analogue;
2) a GyrB-GyrB based switch, optionally wherein the first and second switch domains are dimerized by coumermycin;
3) a GAI-GID1 based switch;
4) a gibberellin-based dimerization switch; or
5) a Halo-tag/SNAP-tag based switch; and
wherein the NKR-CAR comprises:
a) an antigen binding domain derived from an antibody molecule, e.g. an antibody, an antibody fragment, an scFv, a Fv, a Fab, a (Fab')2, a single domain antibody (SDAB), a VH or VL domain, or a camelid VHH domain,
b) a transmembrane domain, and
c) a cytoplasmic domain,
wherein the NKR-CAR comprises a NKR transmembrane domain and/or a NKR cytoplasmic domain.

## Patentansprüche

1. Regulierbarer natürlicher Killer-Rezeptor CAR (RNKR-CAR), wobei der RNKR-CAR umfasst:
(a) ein antigenbindendes Element, umfassend:
ein Bindungsdomänenelement, das eine antigenbindende Domäne umfasst, die von einem Antikörpermolekül abgeleitet ist,
eine Transmembrandomäne und
eine erste Schalterdomäne; und
(b) ein intrazelluläres Signalisierungselement, das eine zweite Schalterdomäne umfasst, wobei die erste und die zweite Schalterdomäne eine Dimerisationsschalter umfassen, weiterhin wobei entweder:
(i) das antigenbindende Element weiterhin eine zytoplasmatische Domäne umfasst, weiterhin wobei
1) die Transmembran- und/oder die zytoplasmatische Domäne eine NKR-Transmembran- oder zytoplasmatische NKR-Domäne umfasst, die jeweils aus einem immunoglobulinartigen Killerzellrezeptor (KIR), einem natürlichen Zytotoxizitätsrezeptor (NCR), der Signalisierungslymphozytenaktivierungsmolekül-Familie (SLAM-Familie) von Immunzellrezeptoren, einem Fc-Rezeptor (FcR), einem Ly49-Rezeptor oder einem NKR von Tabelle 24 ausgewählt sind; und
2) das intrazelluläre Signalisierungselement eine zytoplasmatische NKR-Domäne, die aus Tabelle 24 ausgewählt ist, oder eine primäre intrazelluläre Signalisierungsdomäne, die aus Tabelle 1 ausgewählt ist, oder eine zytoplasmatische ITAM-Domäne umfasst;
(ii) die Transmembrandomäne eine NKR-Transmembrandomäne von einem immunoglobulinartigen Killerzellrezeptor (KIR), einem natürlichen Zytotoxizitätsrezeptor (NCR), der Signalisierungslymphozytenaktivierungsmolekül-Familie (SLAM-Familie) von Immunzellrezeptoren, einem Fc-Rezeptor (FcR), einem Ly49-Rezeptor oder einem NKR von Tabelle 24 umfasst und das intrazelluläre Signalisierungselement eine zytoplasmatische ITAM-Domäne umfasst; oder
(iii) das intrazelluläre Signalisierungselement weiterhin eine zytoplasmatische NKR-Domäne, die aus Tabelle 24 ausgewählt ist, umfasst,
weiterhin wobei der Dimerisationsschalter umfasst:
einen Dimerisationsschalter auf FKBP/FRB-Basis, optional wobei die erste und die zweite Schalterdomäne durch ein Rapamycin oder ein Rapamycin-Analogon dimerisiert werden;
einen Schalter auf GyrB-GyrB-Basis, optional wobei die erste und die zweite Schalterdomäne durch Coumermycin dimerisiert werden;
einen Schalter auf GAI-GID1-Basis;
einen Dimerisationsschalter auf Gibberellin-Basis oder
einen Schalter auf Halo-Tag/SNAP-Tag-Basis.

2. RNKR-CAR nach Anspruch 1, wobei die zytoplasmatische NKR-Domäne eine zytoplasmatische KIR-Domäne, die aus Tabelle 24 ausgewählt ist, umfasst.

3. RNKR-CAR nach Anspruch 1 oder 2, wobei die primäre intrazelluläre Signalisierungsdomäne:
(a) von CD3-zeta, allgemeinem FcR-gamma (FCER1G), Fc-gamma-RIIa, FcR-beta (Fc-epsilon R1b), CD3-gamma, CD3-delta, CD3-epsilon, CD79a, CD79b, DAP10 und DAP12 abgeleitet ist oder
(b) eine DAP12-Signalisierungsdomäne, eine DAP10-Signalisierungsdomäne oder eine CD3zeta-Signalisierungsdomäne ist.

4. RNKR-CAR nach einem der Ansprüche 1 bis 3, wobei:
(a) die Transmembrandomäne eine KIR-Transmembrandomäne, die aus Tabelle 24 ausgewählt ist, umfasst;
(b) der RNKR-CAR eine mutierte NKR-Transmembrandomäne umfasst, die aus der Gruppe bestehend aus einer mutierten KIR-Transmembrandomäne, mutierten NCR-Transmembrandomäne, mutierten FcR-Transmembrandomäne, mutierten Ly49-Rezeptor-Transmembrandomäne und mutierten SLAMF-Rezeptor-Transmembrandomäne ausgewählt ist; oder
(c) der RNKR-CAR eine Transmembrandomäne von einem T-Zellmolekül umfasst, das aus CD8alpha und CD3zeta ausgewählt ist.

5. RNKR-CAR nach einem der vorhergehenden Ansprüche, wobei eine der ersten und der zweiten Schalterdomäne eines Schalters auf FKBP-FRB-Basis eine Schalterdomäne auf FKBP-Basis umfasst und die andere eine Schalterdomäne auf FRB-Basis umfasst, weiterhin wobei die Schalterdomäne auf FKBP-Basis ein FRB-Bindungsfragment oder FKBP-Analogon umfasst und die Schalterdomäne auf FRB-Basis ein FKBP-Bindungsfragment oder FRB-Analog umfasst, umfassend eine E2032-Mutation, wie E2032I oder E2032L, und/oder eine T2098-Mutation, wie T2098L.

6. RNKR-CAR nach einem der vorhergehenden Ansprüche, wobei die antigenbindende Domäne, die von einem Antikörpermolekül abgeleitet ist, beinhaltet:
(a) einen Antikörper, ein Antikörperfragment, ein scFv, ein Fv, ein Fab, ein (Fab')2, einen Einzeldomänenantikörper (SDAB), eine VH- oder VL-Domäne oder eine Kamelid-VHH-Domäne; oder
(b) einen scFv, eine VH-Domäne, einen Nanokörper oder eine Kamelid-VHH-Domäne.

7. RNKR-CAR nach einem der vorhergehenden Ansprüche, wobei die antigenbindende Domäne ein Tumor-Antigen bindet, das ausgewählt ist aus der Gruppe bestehend aus:
(a) Folatrezeptor (FRa), Mesothelin, EGFRvIII, IL-13Ra, CD123, CD19, CD33, BCMA, GD2, CLL-1, CA-IX, MUC1, HER2 und einer beliebigen Kombination davon; oder
(b) CD19, Mesothelin, CD123 und BCMA.

8. RNKR-CAR nach Anspruch 1 (iii), wobei das intrazelluläre Signalisierungselement eine primäre intrazelluläre Signalisierungsdomäne, die aus Tabelle 1 ausgewählt ist, umfasst.

9. Nukleinsäure, die einen RNKR-CAR nach einem der vorherigen Ansprüche kodiert.

10. Vektorsystem, z. B. ein oder mehrere Vektoren, umfassend eine Nukleinsäure nach Anspruch 9.

11. Zelle, umfassend einen RNKR-CAR nach einem der Ansprüche 1-8, eine Nukleinsäure, die einen RNKR-CAR kodiert, nach Anspruch 9 oder das Vektorsystem nach Anspruch 10.

12. Verfahren zur Herstellung einer Zelle nach Anspruch 11, umfassend ein Einbringen einer Nukleinsäure, die einen RNKR-CAR kodiert, nach Anspruch 9 oder des Vektorsystems nach Anspruch 10 in die Zelle.

13. RNKR-CAR-Zelle nach Anspruch 11 zur Verwendung in einem Verfahren zur
(a) Behandlung eines Probanden mit einer Krankheit, die mit einem Tumor-Antigen assoziiert ist; oder
(b) Behandlung eines Probanden für Krebs,
wobei das Verfahren ein Verabreichen einer wirksamen Menge der RNKR-CAR-Zelle an den Probanden umfasst.

14. Verfahren zur Bereitstellung einer RNKR-CAR-Zelle, umfassend:
Empfangen einer Immuneffektorzelle von einem Menschen von einer Entität;
Insertieren einer Nukleinsäure, die einen RNKR-CAR nach einem der Ansprüche 1-8 kodiert, in die Immuneffektorzelle oder eine Tochterzelle davon, um eine RNKR-CAR-Zelle zu bilden; und
optional Bereitstellen der RNKR-CAR-Zelle an die Entität.

15. RCAR/NKR-CAR-Zelle, umfassend:
A) einen regulierbaren CAR (RCAR) und einen NKR-CAR;
B) eine Nukleinsäure, die einen RCAR und einen NKR-CAR kodiert; oder
C) ein Vektorsystem, umfassend eine Nukleinsäure, die einen RCAR und einen NKR-CAR kodiert,
wobei der RCAR umfasst:
a) ein intrazelluläres Signalisierungselement, umfassend:
eine primäre intrazelluläre Signalisierungsdomäne und
eine erste Schalterdomäne;
b) ein antigenbindendes Element, umfassend:
eine antigenbindende Domäne,
eine zweite Schalterdomäne und
optional eine oder eine Vielzahl von kostimulatorischen Signalisierungsdomänen, und
c) eine Transmembrandomäne,
wobei die erste und die zweite Schalterdomäne einen Dimerisationsschalter bilden, umfassend:
1) einen Dimerisationsschalter auf FKBP/FRB-Basis, optional wobei die erste und die zweite Schalterdomäne durch ein Rapamycin oder ein Rapamycin-Analogon dimerisiert werden;
2) einen Schalter auf GyrB-GyrB-Basis, optional wobei die erste und die zweite Schalterdomäne durch Coumermycin dimerisiert werden;
3) einen Schalter auf GAI-GID1-Basis;
4) einen Dimerisationsschalter auf Gibberellin-Basis oder
5) einen Schalter auf Halo-Tag/SNAP-Tag-Basis; und
wobei der NKR-CAR umfasst:
a) eine antigenbindende Domäne, die von einem Antikörpermolekül abgeleitet ist, z. B. einem Antikörper, einem Antikörperfragment, einem scFv, einem Fv, einem Fab, einem (Fab')2, einem Einzeldomänenantikörper (SDAB), einer VH- oder VL-Domäne oder einer Kamelid-VHH-Domäne,
b) eine Transmembrandomäne und
c) eine zytoplasmatische Domäne,
wobei der NKR-CAR eine NKR-Transmembrandomäne und/oder eine zytoplasmatische NKR-Domäne umfasst.

## Revendications

1. Un récepteur CAR de cellules tueuses naturelles régulable (RNKR-CAR), où le RNKR-CAR comprend :
(a) un membre de liaison à un antigène, comprenant :
un élément de domaine de liaison qui comprend un domaine de liaison à un antigène dérivé d'une molécule d'anticorps,
un domaine transmembranaire, et
un premier domaine de commutation ; et
(b) un membre de signalisation intracellulaire comprenant un deuxième domaine de commutation, dans lequel le premier et le deuxième domaine de commutation comprennent un commutateur de dimérisation, dans lequel en outre soit :
(i) le membre de liaison à un antigène comprend en outre un domaine cytoplasmique, dans lequel en outre
1) le domaine transmembranaire et/ou cytoplasmique comprend un domaine transmembranaire ou cytoplasmique de NKR, chacun desquels est sélectionné parmi un récepteur type immunoglobuline de cellules tueuses (KIR), un récepteur de cytotoxicité naturelle (NCR), la famille des molécules d'activation des lymphocytes de signalisation (SLAM) de récepteurs de cellules immunitaires, un récepteur Fc (FcR), un récepteur Ly49 ou un NKR de la Table 24 ; et
2) le membre de signalisation intracellulaire comprend un domaine cytoplasmique de NKR sélectionné parmi la Table 24, ou bien un domaine de signalisation intracellulaire primaire sélectionné parmi la Table 1, ou bien un domaine à ITAM cytoplasmique ;
(ii) le domaine transmembranaire comprend un domaine transmembranaire de NKR d'un récepteur type immunoglobuline de cellules tueuses (KIR), d'un récepteur de cytotoxicité naturelle (NCR), de la famille des molécules d'activation des lymphocytes de signalisation (SLAM) de récepteurs de cellules immunitaires, d'un récepteur Fc (FcR), d'un récepteur Ly49 ou d'un NKR de la Table 24, et le membre de signalisation intracellulaire comprend un domaine à ITAM cycoplasmique ; soit
(iii) le membre de signalisation intracellulaire comprend en outre un domaine cytoplasmique de NKR sélectionné parmi la Table 24,
dans lequel en outre le commutateur de dimérisation comprend
un commutateur de dimérisation basé sur FKBP/FRB, optionnellement dans lequel le premier et le deuxième domaine de commutation sont dimérisés par une rapamycine ou un analogue de rapamycine ;
un commutateur basé sur GyrB-GyrB, optionnellement dans lequel le premier et le deuxième domaine de commutation sont dimérisés par la coumermycine ;
un commutateur basé sur GAI-GID1 ;
un commutateur de dimérisation basé sur les gibbérellines ; ou bien
un commutateur basé sur Halo-tag/SNAP-tag.

2. Le RNKR-CAR selon la revendication 1, dans lequel le domaine cytoplasmique de NKR comprend un domaine cytoplasmique de KIR sélectionné parmi la Table 24.

3. Le RNKR-CAR selon la revendication 1 ou la revendication 2, dans lequel le domaine de signalisation intracellulaire primaire est :
(a) dérivé de CD3 zeta, FcR gamma commun (FCER1G), Fc gamma RIIa, FcR bêta (Fc Epsilon R1b), CD3 gamma, CD3 delta, CD3 epsilon, CD79a, CD79b, DAP10 et DAP12 ; ou bien
(b) un domaine de signalisation DAP12, un domaine de signalisation DAP10 ou un domaine de signalisation CD3zeta.

4. Le RNKR-CAR selon l'une quelconque des revendications 1 à 3, dans lequel :
(a) le domaine transmembranaire comprend un domaine transmembranaire de KIR sélectionné parmi la Table 24 ;
(b) le RNKR-CAR comprend un domaine transmembranaire de NKR muté sélectionné parmi le groupe consistant en un domaine transmembranaire de KIR muté, un domaine transmembranaire de NCR muté, un domaine transmembranaire de FcR muté, un domaine transmembranaire du récepteur Ly49 muté et un domaine transmembranaire du récepteur SLAMF muté ; ou bien
(c) le RNKR-CAR comprend un domaine transmembranaire d'une molécule de cellule T sélectionnée parmi CD8alpha et CD3zeta.

5. Le RNKR-CAR selon l'une quelconque des revendications précédentes, dans lequel l'un d'entre le premier et le deuxième domaine de commutation d'un commutateur basé sur FKBP-FRB comprend un domaine de commutation basé sur FKBP et l'autre un domaine de commutation basé sur FRB, dans lequel en outre le domaine de commutation basé sur FKBP comprend un fragment de liaison à FRB ou à un analogue de FKBP et le domaine de commutation basé sur FRB comprend un fragment de liaison à FKBP ou à un analogue de FRB comprenant une mutation E2032 telle que E20321 ou E2032L et/ou une mutation T2098 telle que T2098L.

6. Le RNKR-CAR selon l'une quelconque des revendications précédentes, dans lequel le domaine de liaison à un antigène dérivé d'une molécule d'anticorps comprend :
(a) un anticorps, un fragment d'anticorps, un scFv, un Fv, un Fab, un (Fab')2, un anticorps à domaine unique (SDAB), un domaine VH ou VL ou un domaine VHH de camélidés ; ou bien
(b) un scFV, un domaine VH, un nanocorps ou un domaine VHH de camélidés.

7. Le RNKR-CAR selon l'une quelconque des revendications précédentes, dans lequel le domaine de liaison à un antigène se lie à un antigène tumoral sélectionné parmi le groupe consistant en :
(a) récepteur de folate (FRa), mésothéline, EGFRvIII, IL-13Ra, CD123, CD19, CD33, BCMA, GD2, CLL-1, CA-IX, MUC1, HER2 et une combinaison quelconque de ceux-ci ; ou bien
(b) CD19, mésothéline, CD123 et BCMA.

8. Le RNKR-CAR selon la revendication 1(iii), dans lequel le membre de signalisation intracellulaire comprend un domaine de signalisation intracellulaire primaire sélectionné parmi la Table 1.

9. Un acide nucléique codant un RNKR-CAR selon l'une quelconque des revendications précédentes.

10. Un système vectoriel, par ex. un ou plusieurs vecteurs, comprenant un acide nucléique selon la revendication 9.

11. Une cellule comprenant un RNKR-CAR selon l'une quelconque des revendications 1-8, un acide nucléique codant un RNKR-CAR selon la revendication 9 ou le système vectoriel selon la revendication 10.

12. Un procédé de préparation d'une cellule selon la revendication 11, comprenant introduire un acide nucléique codant RNKR-CAR selon la revendication 9 ou le système vectoriel selon la revendication 10 dans ladite cellule.

13. Une cellule RNKR-CAR selon la revendication 11, à utiliser dans une méthode destinée à
(a) traiter un sujet ayant une maladie associée à un antigène tumoral ; ou bien
(b) traiter un sujet pour un cancer,
où la méthode comprend administrer au sujet une quantité efficace de la cellule RNKR-CAR.

14. Un procédé de fourniture d'une cellule RNKR-CAR comprenant :
recevoir d'une entité une cellule effectrice immunitaire d'un être humain ;
insérer un acide nucléique codant un RNKR-CAR selon l'une quelconque des revendications 1-8 dans ladite cellule effectrice immunitaire, ou une cellule fille de celle-ci, pour former une cellule RNKR-CAR ; et
fournir optionnellement ladite cellule RNKR-CAR à ladite entité.

15. Une cellule RCAR/NKR-CAR comprenant :
A) un CAR régulable (RCAR) et un NKR-CAR ;
B) un acide nucléique codant un RCAR et un NKR-CAR ; ou bien
C) un système vectoriel comprenant un acide nucléique codant un RCAR et un NKR-CAR,
dans laquelle le RCAR comprend :
a) un membre de signalisation intracellulaire comprenant :
un domaine de signalisation intracellulaire primaire, et
un premier domaine de commutation;
b) un membre de liaison à un antigène comprenant :
un domaine de liaison à un antigène,
un deuxième domaine de commutation; et
optionnellement, un ou une pluralité de domaines de signalisation co-stimulateurs, et
c) un domaine transmembranaire,
dans laquelle le premier et le deuxième domaine de commutation forment un commutateur de dimérisation, comprenant :
1) un commutateur de dimérisation basé sur FKBP/FRB, optionnellement où le premier et le deuxième domaine de commutation sont dimérisés par une rapamycine ou un analogue de rapamycine ;
2) un commutateur basé sur GyrB-GyrB, optionnellement où le premier et le deuxième domaine de dimérisation sont dimérisés par la coumermycine ;
3) un commutateur basé sur GAI-GID1 ;
4) un commutateur de dimérisation basé sur les gibbérellines ; ou bien
5) un commutateur basé sur Halo-tag/SNAP-tag ; et
où le NKR-CAR comprend :
a) un domaine de liaison à un antigène dérivé d'une molécule d'anticorps, par ex. un anticorps, un fragment d'anticorps, un scFv, un Fv, un Fab, un (Fab')2, un anticorps à domaine unique (SDAB), un domaine VH ou VL ou un domaines VHH de camélidés,
b) un domaine transmembranaire, et
c) un domaine cytoplasmique,
où le NKR-CAR comprend un domaine transmembranaire de NKR et/ou un domaine cytoplasmique de NKR.
